(19) Europäisches Patentamt
European Patent Office
Office européen des brevets

(11) EP 2 017 263 A1

(12) **EUROPEAN PATENT APPLICATION**
published in accordance with Art. 153(4) EPC

(43) Date of publication:
**21.01.2009 Bulletin 2009/04**

(21) Application number: **07743059.3**

(22) Date of filing: **09.05.2007**

(51) Int Cl.:
*C07D 209/08* (2006.01) *A61K 31/404* (2006.01)
*A61K 31/4155* (2006.01) *A61K 31/4709* (2006.01)
*A61K 31/538* (2006.01) *A61K 45/00* (2006.01)
*A61P 37/06* (2006.01) *A61P 37/08* (2006.01)
*A61P 43/00* (2006.01) *C07D 403/12* (2006.01)
*C07D 409/06* (2006.01) *C07D 409/12* (2006.01)
*C07D 409/14* (2006.01) *C07D 413/12* (2006.01)

(86) International application number:
**PCT/JP2007/059624**

(87) International publication number:
**WO 2007/129745 (15.11.2007 Gazette 2007/46)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **09.05.2006 JP 2006130424**

(71) Applicant: **Daiichi Sankyo Company, Limited**
**Chuo-ku**
**Tokyo 103-8426 (JP)**

(72) Inventors:
- **MACHINAGA, Nobuo**
  **Tokyo 134-8630 (JP)**
- **YOSHINO, Toshiharu**
  **Tokyo 134-8630 (JP)**
- **CHIBA, Jun**
  **Tokyo 134-8630 (JP)**
- **WATANABE, Jun**
  **Tokyo 134-8630 (JP)**
- **SUZUKI, Takashi**
  **Tokyo 134-8630 (JP)**
- **KIMURA, Youichi**
  **Tokyo 134-8630 (JP)**

(74) Representative: **Hartz, Nikolai**
**Wächtershäuser & Hartz**
**Patentanwälte**
**Weinstrasse 8**
**80333 München (DE)**

# (54) HETEROARYLAMIDE LOWER CARBOXYLIC ACID DERIVATIVE

(57) To provide a novel compound which has S1P receptor agonistic activity, exhibits excellent immunosuppressing effect, gives less adverse side effects, and can be orally administered.

The invention provides a compound represented by general formula (I) (wherein A is a single bond, -O-, or - $CH_2$-; $R^1$ represents a hydrogen atom or a $C_1$-$C_6$ alkyl group, and V represents any one group selected from among the following groups (1) to (3) : (1) -$G^1$-, (2) -$G^2$-N($R^2$) -$G^3$-, and (3) a group represented by formula 2, wherein each of $Z^1$ and $Z^2$ represents a hydrogen atom or a $C_1$-$C_6$ alkyl group, $Z^3$ represents a hydrogen or the like, Q represents -$CH_2$-O- or the like, and Y represents a group represented by foumula 3, a salt thereof, or a solvate thereof.

EP 2 017 263 A1

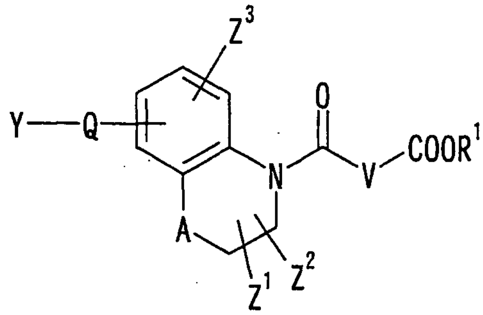

(I)

[Formula 2]

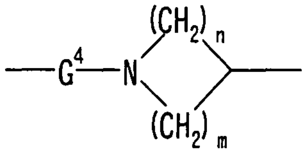

[Formula 3]

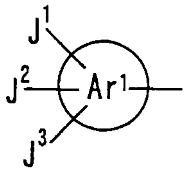

or

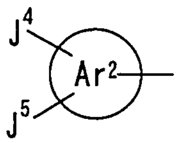

## Description

### Technical Field

The present invention relates to a heteroarylamide lower carboxylic acid derivative which exhibits sphingosine-1-phosphate receptor agonistic activity and which can be used as an immunosuppressor, and to a medicament containing the derivative.

### Background Art

Sphingosine-1-phosphate (hereinafter abbreviated as S1P) receptors belong to the endothelial differentiation gene (EDG) family of G protein-coupled receptors, and include five subtypes; i.e., S1P1, S1P2, S1P3, S1P4, and S1P5 (which are also called EDG-1, EDG-5, EDG-3, EDG-6, and EDG-8, respectively).

Hitherto, FTY720 (2-amino-2-[2-(4-octylphenyl)ethyl]-1,3-propanediol hydrochloride), having a sphingosine-like structure, has been known to exhibit immunosuppressing action (Patent Document 1). In vitro, FTY720 has no inhibitory action on production of cytokines such as IL-2 and, therefore, is thought to exhibit immunosuppressing effect via a mechanism of action different from those of FK506 and cyclosporin, which are known immunosuppressors. However, recent research has revealed that FTY720 is phosphorylated in the living body to act as an S1P receptor agonist, and induce reduction in blood lymphocytes, to thereby exhibit an immunosuppressing action (Non-Patent Document 1). Application of FTY720 has been clinically tested in application to transplant and multiple sclerosis, and bradycardia is reported as an adverse side effect (Non-Patent Document 2). Therefore, there is demand for the development of a new immunosuppressor which solves the aforementioned problem and exhibits higher immunosuppresing effect.

A carboxylic acid derivative having S1P1 (EDG-1) receptor agonistic action and a carboxylic acid derivative having S1P4 (EDG-6) receptor binding ability are also disclosed to express immunosuppressing action (Patent Documents 2, 3, and 4). However, there is also demand for a novel, low-molecular weight S1P receptor agonistic compound which exhibits excellent immunosuppressing effect, gives less adverse side effects, and can be orally administered.

Patent Document 1:
WO 94/008943
Patent Document 2:
WO 2005/000833
Patent Document 3:
WO 2005/020882
Patent Document 4:
WO 2004/058149
Non-Patent Document 1:
Science, 296, 346-349 (2002)
Non-Patent Document 2:
Journal of the American Society of Nephrology, 13(4), 1073-1083 (2002)

### Disclosure of the Invention

### Problems to be Solved by the Invention

An object of the present invention is to provide a novel compound which has S1P receptor agonistic activity, exhibits excellent effect as an immunosuppressor, gives less adverse side effects, and can be orally administered.

### Means for Solving the Problems

In order to solve the aforementioned problems, the present inventors have conducted extensive studies, and have found that novel compounds having a heteroarylamide lower carboxylic acid structure differing from those of existing compounds can be used as an immunosuppressor which has S1P receptor agonistic activity, reduces lymphocytes in peripheral blood of mice in vivo model through oral administration, and gives less adverse side effects such as bradycardia. The present invention has been accomplished on the basis of this finding.

Accordingly, the present invention provides a compound represented by the following general formula (I):

[F1]

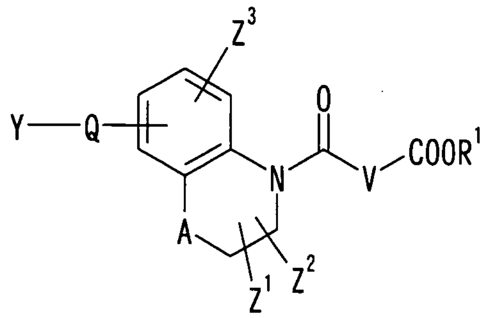

(I)

[wherein A represents a single bond, -O-, or $-CH_2-$;
$R^1$ represents a hydrogen atom or a $C_1$-$C_6$ alkyl group;
V represents any one group selected from the following groups (1) to (3):

(1) $-G^1-$ (wherein $G^1$ represents an optionally substituted straight-chain alkylene group having 1 to 5 carbon atoms),
(2) $-G^2-N(R^2)-G^3-$ (wherein $G^2$ represents a single bond or an optionally substituted straight-chain alkylene group having 1 to 3 carbon atoms, and $G^3$ represents an optionally substituted straight-chain alkylene group having 1 to 4 carbon atoms, and $R^2$ represents a hydrogen atom, a $C_1$-$C_6$ alkyl group, or a 3- to 8-membered cycloalkyl group), and
(3) a group represented by the following group:

[F2]

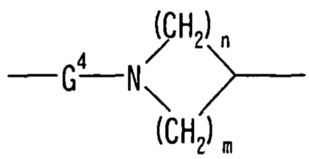

(wherein $G^4$ represents a single bond or an optionally substituted straight-chain alkylene group having 1 to 2 carbon atoms, and each of m and n independently represents 1, 2, or 3);
each of $Z^1$ and $Z^2$ independently represents a hydrogen atom or a $C_1$-$C_6$ alkyl group;
$Z^3$ represents a hydrogen atom, a halogen atom, a cyano group, a $C_1$-$C_6$ alkyl group, a halogeno-$C_1$-$C_6$ alkyl group, a $C_1$-$C_6$ alkoxy group, or a halogeno-$C_1$-$C_6$ alkoxy group;
Q represents a single bond, -O-, $-CH_2-$, $-CH_2-CH_2-$, $-CH_2-CH_2-CH_2-$, $-CH_2-CH_2-CH_2-CH_2-$, $-CH_2-CH_2-CH_2-CH_2-CH_2-$, $-CH_2-O-$, - $CH_2-CH_2-O-$, $-CH_2-O-CH_2-$, $-CH_2-CH_2-O-CH_2-$, $-CH_2-CH_2-CH_2-O-$, $-CH_2-CH_2-CH_2-O-CH_2-$, $-CH_2-CH_2-O-CH_2-CH_2-$, $-CH_2-CH_2-CH_2-CH_2-O-$, CH=CH-, or $-CH=CH-CH_2-O-$ (wherein these groups may each have 1 or 2 $C_1$-$C_6$ alkyl groups or halogen atoms as substituents); and
Y represents the following groups:
[F3]

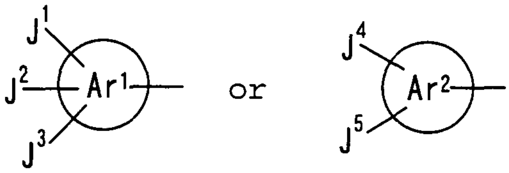

(wherein each of $Ar^1$ and $Ar^2$ independently represents a benzene ring or a 5- or 6-membered aromatic heterocycle; each of $J^1$, $J^2$, $J^3$ , $J^4$ , and $J^5$ independently represents a hydrogen atom, a halogen atom, a hydroxyl group, a nitro group, a cyano group, a $C_1$-$C_6$ alkyl group, a halogeno-$C_1$-$C_6$ alkyl group, a $C_1$-$C_6$ alkoxy-$C_1$-$C_6$ alkyl group, a $C_1$-$C_6$ alkoxy group, a halogeno-$C_1$-$C_6$ alkoxy group, an amino group, a mono-$C_1$-$C_6$ alkylamino group, a di-$C_1$-$C_6$ alkylamino group, a carboxyl group, a $C_1$-$C_6$ alkoxycarbonyl group, a carbamoyl group, a mono-$C_1$-$C_6$ alkylcarbamoyl group, a di-$C_1$-$C_6$ alkylcarbamoyl group, a sulfamoyl group, a mono-$C_1$-$C_6$ alkylsulfamoyl group, a di-$C_1$-$C_6$ alkylsulfamoyl group, an optionally substituted phenyl group, an optionally substituted benzyl group, an optionally substituted phenethyl group, an optionally substituted styryl group, an optionally substituted phenoxy group, an optionally substituted benzyloxy group, an optionally substituted phenoxymethyl group, an optionally substituted 3- to 8-membered cycloalkyl group, an optionally substituted 3- to 8-membered cycloalkenyl group, an optionally substituted 3- to 8-membered cycloalkylmethyl group, an optionally substituted 3- to 8-membered cycloalkyloxy group, an optionally substituted 3- to 8-membered cycloalkylmethoxy group, an optionally substituted 5- or 6-membered aromatic heterocyclic group, an optionally substituted 4- to 6-membered saturated heterocyclic group, or an optionally substituted 5- or 6-membered heteroaryloxy group)], a salt thereof, or a solvate thereof.

The present invention also provides a medicament; an S1P receptor agonist; an immunosuppressor; and a therapeutic and/or preventive agent for rejection upon transplantation, an autoimmune disease, and/or an allergic disease, containing, as an effective ingredient, the aforementioned compound, a salt thereof, or a solvate thereof.

The present invention also provides a medicament containing the aforementioned compound, a salt thereof, or a solvate thereof, in combination with one or more species selected from among an immunosuppressor, an antibody useful for immunosuppression, a rejection-treating agent, an antibiotic, and a steroidal agent.

The present invention also provides use of the aforementioned compound, a salt thereof, or a solvate thereof, for producing a medicament.

The present invention also provides a method for preventing and/or treating an S1P receptor-relating disease, characterized by administering an effective amount of the aforementioned compound, a salt thereof, or a solvate thereof.

Effects of the Invention

The heteroarylamide lower carboxylic acid derivative according to the present invention, a salt thereof, or a solvate thereof, has S1P receptor agonistic activity and reduces, through oral administration, lymphocytes in peripheral blood of mice and rats in mouse in-vivo model and rat in-vivo model. Therefore, the derivative, salt, or solvate is a useful effective ingredient for medicaments such as an immunosuppressor; for example, for a therapeutic and/or preventive agent for rejection upon transplantation, an autoimmune disease, or an allergic disease of mammals, particularly human. Since the medicaments reduce lymphocytes in peripheral blood of mice and rats through oral administration, they could be orally administered. In addition, the medicaments give less adverse side effects such as bradycardia, which is observed when other S1P receptor agonists are used.

Best Modes for Carrying Out the Invention

Hereinafter, the groups given in the specification will be described in detail.

The "$C_1$-$C_6$ alkyl group" refers to a $C_1$-$C_6$ straight-chain or branched-chain saturated hydrocarbon group. Examples of the alkyl group include methyl, ethyl, propyl, isopropyl, n-butyl, isobutyl, tert-butyl, n-pentyl, 1-ethylpropyl, and 2,2-dimethylpropyl.

The "straight-chain alkylene group" refers to a straight-chain alkylene group consisting of methylene groups. The "straight-chain alkylene group having 1 to 2 carbon atoms" include $-CH_2-$ and $-CH_2-CH_2-$. The " straight-chain alkylene group having 1 to 3 carbon atoms" include $-CH_2-$, $-CH_2-CH_2-$, and $-CH_2-CH_2-CH_2-$. The "straight-chain alkylene group having 1 to 4 carbon atoms" include $-CH_2-$, $-CH_2-CH_2-$, $-CH_2-CH_2-CH_2-$, and $-CH_2-CH_2-CH_2-CH_2-$. The "straight-chain alkylene group having 1 to 5 carbon atoms" include $-CH_2-$, $CH_2-CH_2-$, $-CH_2-CH_2-CH_2-$, $-CH_2-CH_2-CH_2-CH_2-$, and $-CH_2-CH_2-CH_2-CH_2-CH_2-$.

The "halogen atom" include a fluorine atom, a chlorine atom, a bromine atom, and an iodine atom.

The "halogeno-$C_1$-$C_6$ alkyl group" refers to said $C_1$-$C_6$ alkyl group having a halogen atom as a substituent. The number of halogen atoms may be one or more. When two or more halogen atoms are included, these halogen atoms may be identical to or different from one another. Examples include chloromethyl, fluoromethyl, difluoromethyl, trifluoromethyl, 2-chloroethyl, 2,2,2-trifluoroethyl, 1,1,2,2-tetrafluoroethyl, pentafluoroethyl, and 1,1-difluoro-2-methylpropyl.

The $C_1$-$C_6$ alkoxy group refers to a straight-chain or branched-chain alkyloxy group having 1 to 6 carbon atoms. Examples include methoxy, ethoxy, propoxy, isopropoxy, n-butoxy, isobutyloxy, tert-butoxy, n-pentyloxy, 1-ethylpropoxy, and 2,2-dimethylpropoxy.

The halogeno-$C_1$-$C_6$ alkoxy group refers to said $C_1$-$C_6$ alkoxy group having a halogen atom as a substituent. The number of halogen atoms may be one or more. When two or more halogen atoms are included, these halogen atoms may be identical to or different from one another. Examples include fluoromethoxy, chloromethoxy, difluoromethoxy, trifluoromethoxy, trichloromethoxy, and pentafluoroethoxy.

The "$C_1$-$C_6$ alkoxy-$C_1$-$C_6$ alkyl group" refers to said $C_1$-$C_6$ alkyl group having said $C_1$-$C_6$ alkoxy group as a substituent. The number of alkoxy groups may be one or more. When two or more alkoxy groups are included, these alkoxy groups may be identical to or different from one another. Examples include methoxyethyl and ethoxymethyl.

The "mono-$C_1$-$C_6$ alkylamino group" refers to an amino group having one said $C_1$-$C_6$ alkyl group as a substituent. Examples include methylamino and ethylamino.

The "di-$C_1$-$C_6$ alkylamino group" refers to an amino group having two said $C_1$-$C_6$ alkyl groups as substituents. The two $C_1$-$C_6$ alkyl groups may be identical to or different from each other. Examples include dimethylamino and N-methyl-N-ethylamino.

The "$C_1$-$C_6$ alkoxycarbonyl group" refers to a group formed from the aforementioned $C_1$-$C_6$ alkoxy group and a carbonyl group. Examples include methoxycarbonyl, ethoxycarbonyl, n-propoxycarbonyl, isopropoxycarbonyl, n-butoxycarbonyl, isobutyloxycarbonyl, tert-butoxycarbonyl, n-pentyloxycarbonyl, and n-hexyloxycarbonyl.

The "mono-$C_1$-$C_6$ alkylcarbamoyl group" refers to a carbamoyl group having one said $C_1$-$C_6$ alkyl group as a substituent. Examples include methylcarbamoyl and ethylcarbamoyl.

The "di-$C_1$-$C_6$ alkylcarbamoyl group" refers to a carbamoyl having two said $C_1$-$C_6$ alkyl groups as substituents. The two $C_1$-$C_6$ alkyl groups may be identical to or different from each other. Examples include dimethylcarbamoyl, diethylcarbamoyl, and N-methyl-N-ethylcarbamoyl.

The "mono-$C_1$-$C_6$ alkylsulfamoyl group" refers to an aminosulfonyl group having one said $C_1$-$C_6$ alkyl group as a substituent. Examples include methylsulfamoyl, ethylsulfamoyl, and isopropyl sulfamoyl.

The "di-$C_1$-$C_6$ alkylsulfamoyl group" refers to an aminosulfonyl group having two said $C_1$-$C_6$ alkyl groups as substituents. The two $C_1$-$C_6$ alkyl groups may be identical to or different from each other. Examples include dimethylsulfamoyl, diethylsulfamoyl, and N-methyl-N-ethylsulfamoyl.

The "3- to 8-membered cycloalkyl group" refers to a 3-to 8-membered saturated monocyclic hydrocarbon group. Examples include cyclopropyl, cyclobutyl, cyclopentyl, and cyclohexyl.

The "3- to 8-membered cycloalkenyl group" refers to a 3- to 8-membered unsaturated monocyclic hydrocarbon group. Examples include cyclopropenyl, cyclobutenyl, cyclopentenyl, and cyclohexenyl.

The "3- to 8-membered cycloalkylmethyl group" refers to a methyl group having one said 3- to 8-membered cycloalkyl groups. Examples include cyclopropylmethyl, cyclobutylmethyl, cyclopentylmethyl, and cyclohexylmethyl.

The "3- to 8-membered cycloalkyloxy group" refers to an alkyloxy group having one said 3- to 8-membered cycloalkyl group. Examples include cyclopropyloxy, cyclobutyloxy, cyclopentyloxy, and cyclohexyloxy.

The "3- to 8-membered cycloalkylmethoxy group" refers to a methoxy group having one said 3- to 8-membered cycloalkyl groups. Examples include cyclopropylmethoxy, cyclobutylmethoxy, cyclopentylmethoxy, and cyclohexylmethoxy.

The "5- or 6-membered aromatic heterocycle" refers to a 5- or 6-membered monocyclic aromatic heterocyclic group having at least one heteroatom selected from a nitrogen atom, an oxygen atom, and a sulfur atom as a ring-forming atom. Examples include a furan ring, a thiophene ring, a pyrrole ring, an oxazole ring, a thiazole ring, an imidazole ring, an isoxazole ring, an isothiazole ring, a pyrazole ring, a triazole ring, a pyridine ring, a pyridazine ring, a pyrimidine ring, and a pyrazine ring.

The "5- or 6-membered aromatic heterocyclic group" refers to a group formed from the aforementioned 5- or 6-membered aromatic heterocycle. Examples include furyl, thienyl, pyrrolyl, oxazolyl, thiazolyl, imidazolyl, isoxazolyl, isothiazolyl, pyrazolyl, triazolyl, pyridyl, pyridazyl, pyrimidyl, and pyrazyl.

The "4- to 6-membered saturated hetericyclic group" refers to a 4- to 6-membered saturated monocyclic heterocyclic group having at least one heteroatom selected from a nitrogen atom, an oxygen atom, and a sulfur atom, as a ring-forming atom. Examples include azetidin-1-yl, pyrrolidin-1-yl, piperidin-1-yl, piperazin-1-yl, tetrahydrofuran-2-yl, and tetrahydropyran-3-yl.

The "5- or 6-membered heteroaryloxy group" refers to a group which is formed from a 5- or 6-membered monocyclic heteroaryl group having at least one heteroatom selected from a nitrogen atom, an oxygen atom, and a sulfur atom, as

a ring-forming atom, and an oxy group. Examples include thienyloxy, furyloxy, pyranyloxy, pyrrolyloxy, imidazolyloxy, pyrazolyloxy, thiazolyloxy, isothiazolyloxy, oxazolyloxy, isoxazolyloxy, pyridyloxy, pyrimidyloxy, pyrazyloxy, and pyridazyloxy.

The "substituent" in the phrase "optionally substituted" include, for example, a halogen atom, a hydroxyl group, a nitro group, a cyano group, a $C_1$-$C_6$ alkyl group, a 3- to 8-membered cycloalkyl group, a halogeno-$C_1$-$C_6$ alkyl group, a $C_1$-$C_6$ alkoxy-$C_1$-$C_6$ alkyl group, a $C_1$-$C_6$ alkoxy group, a halogeno-$C_1$-$C_6$ alkoxy group, an amino group, a mono-$C_1$-$C_6$ alkylamino group, a di-$C_1$-$C_6$ alkylamino group, an oxo group, a carboxyl group, a $C_1$-$C_6$ alkoxycarbonyl group, a carbamoyl group, a mono-$C_1$-$C_6$ alkylcarbamoyl group, a di-$C_1$-$C_6$ alkylcarbamoyl group, a sulfamoyl group, a mono-$C_1$-$C_6$ alkylsulfamoyl group, and a di-$C_1$-$C_6$ alkylsulfamoyl group.

Preferred embodiments of the groups A, $R^1$, $R^2$, V, $G^1$, $G^2$, $G^3$, $G^4$, $Z^1$, $Z^2$, $Z^3$, Q, Y, m, n, $Ar^1$, $Ar^2$, $J^1$, $J^2$ $J^3$, $J^4$, and is will next be described.

The group A in the general formula (I) represents a single bond, -O-, or -$CH_2$-, preferably a single bond or O-, and more preferably a single bond.

The group $R^1$ in the general formula (I) represents a hydrogen atom or a $C_1$-$C_6$ alkyl group. The $C_1$-$C_6$ alkyl group in $R^1$ include methyl, ethyl, propyl, and isopropyl. $R^1$ is preferably a hydrogen atom.

V in formula (I) represents any one group selected from the following (1) to (3):

(1) -$G^1$- (wherein $G^1$ represents an optionally substituted straight-chain alkylene group having 1 to 5 carbon atoms),
(2) -$G^2$-N($R^2$)-$G^3$- (wherein $G^2$ represents a single bond or an optionally substituted straight-chain alkylene group with 1 to 3 carbon atoms, $G^3$ represents an optionally substituted straight-chain alkylene group having 1 to 4 carbon atoms, and $R^2$ represents a hydrogen atom, a $C_1$-$C_6$ alkyl group, or a 3- to 8-membered cycloalkyl group), and
(3) the following group:

[F4]

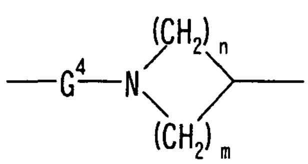

(wherein $G^4$ represents a single bond or an optionally substituted straight-chain alkylene group with 1 to 2 carbon atoms, and each of m and n independently represents 1, 2, or 3) .

Each of the groups represented by (1), (2), and (3) links, on its right side, to -$COOR^1$ in the general formula (I).

Preferred embodiments of the aforementioned (1) will next be described in detail. V represents -$G^1$- (wherein $G^1$ represents an optionally substituted straight-chain alkylene group having 1 to 5 carbon atoms). $G^1$ represents a $C_1$-$C_5$ straight-chain alkylene group having no substituent or a $C_1$-$C_5$ straight-chain alkylene group having a substituent. When a substituent is present, the alkylene group may have one or more substituents. When a plurality of substituents are present, the substituents may be identical to or different from one another. When a plurality of substituents are present, these substituents may be bonded to the same carbon atom of the alkylene group or to different carbon atoms thereof. When $G^1$ has a substituent, the number of the substituent is preferably 1 to 4, more preferably 1 to 2, futher more preferably 1. When $G^1$ has a substituent, examples of the substituent include those exemplified above. The substituent is preferably a halogen atom, a hydroxyl group, a $C_1$-$C_6$ alkyl group, an amino group, a mono-$C_1$-$C_6$ alkylamino group, a di-$C_1$-$C_6$ alkylamino group, and an oxo group, more preferably a hydroxyl group, an amino group, a mono-$C_1$-$C_6$ alkylamino group, and a $C_1$-$C_6$-di-alkylamino group, and futher more preferably an amino group and a mono-$C_1$-$C_6$ alkylamino group.

The optionally substituted $C_1$-$C_5$ straight-chain alkylene group in $G^1$ includes an optionally substituted -$CH_2$-, an optionally substituted -$CH_2$-$CH_2$-, an optionally substituted - $CH_2$-$CH_2$-$CH_2$-, an optionally substituted -$CH_2$-$CH_2$-$CH_2$-$CH_2$-, and an optionally substituted $CH_2$-$CH_2$-$CH_2$-$CH_2$-$CH_2$-, preferably an optionally substituted -$CH_2$-$CH_2$- and an optionally substituted -$CH_2$-$CH_2$-$CH_2$-, more preferably an optionally substituted -$CH_2$-$CH_2$-, futher more preferably a substituted $CH_2$-$CH_2$-.

Specific examples of $G^1$ include -$CH_2$-$CH_2$- which may have as a substituent 1 to 4 groups selected from the

group consisting of a halogen atom, a hydroxyl group, a $C_1$-$C_6$ alkyl group, an amino group, a mono-$C_1$-$C_6$ alkylamino group, and a di-$C_1$-$C_6$ alkylamino group; -$CH_2$-$CH_2$-$CH_2$- which may have as a substituent 1 to 4 groups selected from the group consisting of a halogen atom, a hydroxyl group, a $C_1$-$C_6$ alkyl group, an amino group, a mono-$C_1$-$C_6$ alkylamino group, and a di-$C_1$-$C_6$ alkylamino group; and -$CH_2$-$CH_2$-$CH_2$-$CH_2$- which may have as a substituent 1 to 4 groups selected from the group consisting of a halogen atom, a hydroxyl group, a $C_1$-$C_6$ alkyl group, an amino group, a mono-$C_1$-$C_6$ alkylamino group, and a di-$C_1$-$C_6$ alkylamino group. Of these, -$CH_2$-$CH_2$- which may have as a substituent 1 or 2 groups selected from the group consisting of a halogen atom, a hydroxyl group, a $C_1$-$C_6$ alkyl group, an amino group, a mono-$C_1$-$C_6$ alkylamino group, and a di-$C_1$-$C_6$ alkylamino group is preferred, with -$CH_2$-$CH_2$- which may have as a substituent one group selected from the group consisting of a halogen atom, a hydroxyl group, a $C_1$-$C_6$ alkyl group, an amino group, a mono-$C_1$-$C_6$ alkylamino group, and a di-$C_1$-$C_6$ alkylamino group being more preferred. The halogen atom is preferably a fluorine atom or a chlorine atom, and the $C_1$-$C_6$ alkyl group is preferably a methyl group or an ethyl group. The mono-alkylamino group is preferably a methylamino group or an ethylamino group, and the dialkylamino group is preferably a dimethylamino group or an N-methyl-N-ethylamino group.

More specific examples of group $G^1$ include -$CH_2$-$CH_2$-, - $CH_2$-$CH_2$-$CH_2$-, -$CH_2$-$CH_2$-$CH_2$-$CH_2$-, -$CH_2$-$CH_2$-$CH_2$-$CH_2$-$CH_2$-, -CH(OH)-$CH_2$-, -CH($NH_2$)-$CH_2$-, -CH($NCH_3$)-$CH_2$-, -CH(N($CH_3$)$_2$)-$CH_2$-, - CH($NCH_3$($C_2Hg$))-$CH_2$-, -$CH_2$-CH(OH)-, -$CH_2$-CH($NH_2$)-, -CH($NH_2$)-$CH_2$-$CH_2$-, and -$CH_2$-CH($NH_2$)-$CH_2$-. Preferred examples include -$CH_2$-$CH_2$-, -CH(OH)-$CH_2$-, -CH($NH_2$)-$CH_2$-, -CH($NHCH_3$)-$CH_2$-, -$CH_2$-CH(OH)-, and $CH_2$-CH($NH_2$)-. More preferred examples include - $CH_2$-$CH_2$-, -CH($NH_2$)-$CH_2$-, and CH($NCH_3$)-$CH_2$-. Each of the groups of specific examples links, on its right side, to -$COOR^1$ in the general formula (I).

Preferred embodiments of the aforementioned (2) will next be described in detail. V represents -$G^2$-N($R^2$)-$G^3$-(wherein $G^2$ represents a single bond or an optionally substituted $C_1$-$C_3$ straight-chain alkylene group, $G^3$ represents an optionally substituted $C_1$-$C_4$ straight-chain alkylene group, and $R^2$ represents a hydrogen atom, a $C_1$-$C_6$ alkyl group, or a 3- to 8-membered cycloalkyl group). $G^2$ represents a single bond, a $C_1$-$C_3$ straight-chain alkylene group having no substituent, or a $C_1$-$C_3$ straight-chain alkylene group having a substituent. $G^3$ represents a $C_1$-$C_4$ straight-chain alkylene group having no substituent or a $C_1$-$C_4$ straight-chain alkylene group having a substituent. When $G^2$ or $G^3$ has a substituent, each group may have one or more substituents. When a plurality of substituents are present, the substituents may be identical to or different from one another. When $G^2$ or $G^3$ has a plurality of substituents, these substituents may be bonded to the same carbon atom of the alkylene group or to different carbon atoms thereof. When $G^2$ or $G^3$ has a substituent, $G^2$ or $G^3$ preferably has a 1 to 4 substituents, respectively. When $G^2$ or $G^3$ has a substituent, examples of the substituent include those exemplified above. Examples of preferred substituents include a halogen atom, a hydroxyl group, a $C_1$-$C_6$ alkyl group, and an oxo group. The $C_1$-$C_6$ alkyl group include methyl and ethyl.

When $G^2$ is a $C_1$-$C_3$ straight-chain alkylene group, the straight-chain alkylene group of $G^2$ and $G^3$ preferably has 4 or less carbon atoms in total.

The optionally substituted $C_1$-$C_3$ straight-chain alkylene group in $G^2$ includes an optionally substituted -$CH_2$-, an optionally substituted -$CH_2$-$CH_2$-, and an optionally substituted -$CH_2$-$CH_2$-$CH_2$-. Of these, an optionally substituted -$CH_2$- and an optionally substituted -$CH_2$-$CH_2$- are preferred.

The optionally substituted $C_1$-$C_4$ straight-chain alkylene group in $G^3$ includes an optionally substituted -$CH_2$-, an optionally substituted -$CH_2$-$CH_2$-, an optionally substituted - $CH_2$-$CH_2$-$CH_2$-, and an optionally substituted -$CH_2$-$CH_2$-$CH_2$-$CH_2$-. Of these, an optionally substituted -$CH_2$- and an optionally substituted -$CH_2$-$CH_2$- are preferred.

$G^2$ is preferably a single bond or an optionally substituted -$CH_2$-, more preferably -$CH_2$- having no substituent. Specific examples of $G^2$ include a single bond; -$CH_2$- which may have as a substituent 1 or 2 groups selected from the group consisting of a halogen atom, a hydroxyl group, a $C_1$-$C_6$ alkyl group, and an oxo group; and -$CH_2$-$CH_2$- which may have as a substituent 1 or 2 groups selected from the group consisting of a halogen atom, a hydroxyl group, a $C_1$-$C_6$ alkyl group, and an oxo group. Among them, a single bond and -$CH_2$-which may have as a substituent one group selected from the group consisting of a halogen atom, a hydroxyl group, a $C_1$-$C_6$ alkyl group, and an oxo group are preferred, with -$CH_2$-having no substituent being more preferred.

$G^3$ is preferably an optionally substituted -$CH_2$- and an optionally substituted -$CH_2$-$CH_2$-, more preferably -$CH_2$-$CH_2$-having no substituent. Specific examples of $G^3$ include -$CH_2$-which may have as a substituent 1 or 2 groups selected from the group consisting of a halogen atom, a hydroxyl group, a $C_1$-$C_6$ alkyl group and an oxo group; and -$CH_2$-$CH_2$- which may have as a substituent 1 or 2 groups selected from the group consisting of a halogen atom, a hydroxyl group, a $C_1$-$C_6$ alkyl group and an oxo group. Among them, -$CH_2$-$CH_2$- having no substituent, -$CH_2$-$CH_2$- having one halogen atom as a substituent, and -$CH_2$-$CH_2$- having one hydroxyl group as a substituent are preferred.

In $G^2$ and $G^3$, the halogen atom is preferably a fluorine atom or a chlorine atom, and the $C_1$-$C_6$ alkyl group is preferably methyl group or ethyl group.

$R^2$ represents a hydrogen atom, a $C_1$-$C_6$ alkyl group, or a 3- to 8-membered cycloalkyl group. $R^2$ is preferably a hydrogen atom or a $C_1$-$C_6$ alkyl group. In $R^2$, the $C_1$-$C_6$ alkyl group includes methyl group, ethyl group, propyl group, and isopropyl group, is preferably a methyl group. The 3- to 8-membered cycloalkyl group in $R^2$ includes cyclopropyl group and cyclobutyl group, is preferably a cyclopropyl group.

When V is a group represented by (2), examples of preferred groups (V) include -NH-$CH_2$-$CH_2$-, -NH-$CH_2$-$CH_2$-$CH_2$-, - $CH_2$-NH-$CH_2$-, -$CH_2$-NH-$CH_2$-$CH_2$-, -$CH_2$-N($CH_3$)-$CH_3$-, -$CH_2$-N($CH_3$)-$CH_2$-$CH_2$-, -$CH_2$-N ($C_2H_5$)-$CH_2$-$CH_2$-, -$CH_2$-N(i-Pr)-$CH_2$-$CH_2$-, -$CH_2$-N(c-Pr)-$CH_2$-$CH_2$-, -CH($CH_3$)-NH-$CH_2$-, -$CH_2$-NH-$CH_2$-CH($CH_3$) -, -CH($CH_3$)-NH-$CH_2$-$CH_2$-, -C($CH_3$)$_2$-NH-$CH_2$-$CH_2$-, -CH ($CH_3$)-N($CH_3$)-$CH_2$-$CH_2$-, -C($CH_3$)$_2$-N($CH_3$)-$CH_2$-$CH_2$-, -$CH_2$-$CH_2$-NH-$CH_2$-, -CHF-NH-$CH_2$-$CH_2$-, -CO-NH-$CH_2$-$CH_2$-, -CO-N($CH_3$)-$CH_2$-$CH_2$-, -$CH_2$-NH-$CH_2$-CH(OH) -, and -$CH_2$-NH-$CH_2$-CHF-. Examples of more preferred (V) include -$CH_2$-NH-$CH_2$-, -$CH_2$-N($CH_3$)-$CH_2$-, -$CH_2$-NH-$CH_2$-$CH_2$-, -$CH_2$-N($CH_3$) - $CH_2$-$CH_2$-, -CH($CH_3$)-NH-$CH_2$-$CH_2$-, -$CH_2$-$CH_2$-NH-$CH_2$-, -$CH_2$-NH-$CH_2$-CH(OH)-, and $CH_2$-NH-$CH_2$-CHF- Each of the exemplified groups links, on its right side, to -COOR$^1$ in the general formula (I). The symbol "i-Pr" refers to an isopropyl group, and "c-Pr" a cyclopropyl group.

Preferred embodiments of the aforementioned (3) will next be described in detail. V represents the following group:

[F5]

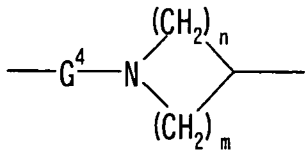

(wherein $G^4$ represents a single bond or an optionally substituted $C_1$-$C_2$ straight-chain alkylene group, and each of m and n is independently 1, 2, or 3). The "m" is 1, 2, or 3, preferably 1. The "n" is 1, 2, or 3. Preferred combinations of m and n are m (1 or 2) and n (1, 2, or 3). More preferably, when m is 1, n is 1, 2, or 3. Still more preferably, m is 1, and n is 2 or 3. $G^4$ represents a single bond, a $C_1$-$C_2$ straight-chain alkylene group having a substituent, or a $C_1$-$C_2$ straight-chain alkylene group having no substituent. When $G^4$ has a substituent, the number of the substituent may be one or more. When a plurality of substituents are present, the substituents may be identical to or different from one another. When $G^4$ has a plurality of substituents, these substituents may be bonded to the same carbon atom of the alkylene group or to different carbon atoms thereof. When $G^4$ has a substituent, the number of the substituent is preferably 1 or 2. When $G^4$ has a substituent, the substituent include those exemplified above, is preferably a halogen atom or a $C_1$-$C_6$ alkyl group, more preferably a $C_1$-$C_6$ alkyl group.

In $G^4$, the optionally substituted $C_1$-$C_2$ straight-chain alkylene group includes an optionally substituted -$CH_2$- and an optionally substituted -$CH_2$-$CH_2$-, is preferably an optionally substituted -$CH_2$-, more preferably -$CH_2$- having no substituent.

$G^4$ is preferably a single bond or an optionally substituted -$CH_2$-, more preferably -$CH_2$- having no substituent. Examples of $G^4$ include a single bond; -$CH_2$-which may have as a substituent 1 or 2 groups selected from the group consisting of a halogen atom and a $C_1$-$C_6$ alkyl group; and -$CH_2$-$CH_2$- which may have as a substituent 1 or 2 groups selected from the group consisting of a halogen atom and a $C_1$-$C_6$ alkyl group. Among them, a single bond and -$CH_2$-which may have as a substituent one group selected from the group consisting of a halogen atom and a $C_1$-$C_6$ alkyl group are preferred, with a single bond and -$CH_2$- having no substituent being more preferred. Of these, -$CH_2$- having no substituent is still more preferred. The $C_1$-$C_6$ alkyl group includes methyl group and ethyl group.

When V is a group represented by (3), specific examples of preferred groups (V) include the following.

[F6]

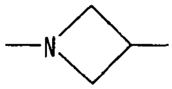, 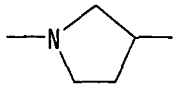, 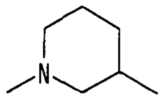, 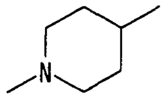

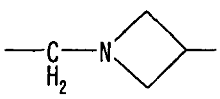, 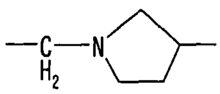, 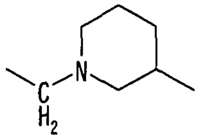, 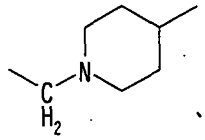,

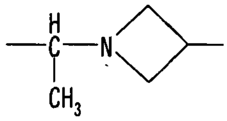, 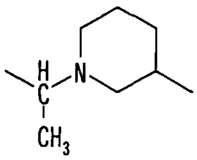

Among them, the following groups are preferred.
[F7]

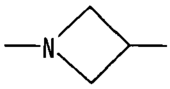, 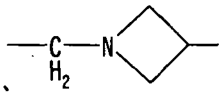, 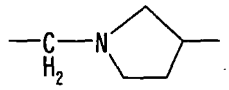,

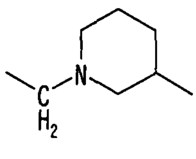 and 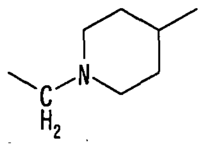

Each of the groups of specific examples links, on its right side, to -COOR$^1$ in the general formula (I).
In the general formula (I), each of $Z^1$ and $Z^2$ represents independently a hydrogen atom or a $C_1$-$C_6$ alkyl group. The $C_1$-$C_6$ alkyl group includes methyl group, ethyl group, and propyl group. Of these, methyl is preferred. Regarding $Z^1$ and $Z^2$, both $Z^1$ and $Z^2$ are preferably hydrogen atoms.
In the general formula (I), $Z^3$ represents a hydrogen atom, a halogen atom, a cyano group, a $C_1$-$C_6$ alkyl group, a halogeno-$C_1$-$C_6$ alkyl group, a $C_1$-$C_6$ alkoxy group, or a halogeno-$C_1$-$C_6$ alkoxy group. The $C_1$-$C_6$ alkyl group, halogeno-$C_1$-$C_6$ alkyl group, $C_1$-$C_6$ alkoxy group, and halogeno-$C_1$-$C_6$ alkoxy group include those species having 1 to 6 carbon atoms. $Z^3$ is preferably a hydrogen atom, a $C_1$-$C_6$ alkyl group, and a $C_1$-$C_6$ alkoxy group, with a hydrogen atom and a methyl group being more preferred. In the present invention, compounds having a $C_1$-$C_6$ alkyl group or a $C_1$-$C_6$ alkoxy group as $Z^3$ are more expectable, since such compounds usually exhibit higher activity and less adverse side effects, as compared with compounds having a hydrogen atom as $Z^3$.
In formula (I), Q represents a single bond, -O-, $-CH_2-$, $-CH_2-CH_2-$, $-CH_2-CH_2-CH_2-$, $-CH_2-CH_2-CH_2-CH_2-$, $-CH_2-CH_2-CH_2-CH_2-CH_2-$, $-CH_2-O-$, $-CH_2-CH_2-O-$, $-CH_2-O-CH_2-$, $-CH_2-CH_2-O-CH_2-$, $-CH_2-CH_2-CH_2-O-$, $-CH_2-CH_2-CH_2-O-CH_2-$, $-CH_2-CH_2-O-CH_2-CH_2-$, $-CH_2-CH_2-CH_2-CH_2-O-$, -CH=CH-, or $-CH=CH-CH_2-O-$ (wherein these group each may have 1 or 2 $C_1$-$C_6$ alkyl groups or halogen atoms as substituents). Each of these groups may be linked, on its right or left side, to Y in formula (I). When any of these groups has a $C_1$-$C_6$ alkyl group as a substituent, the $C_1$-$C_6$

alkyl group includes methyl group and ethyl group. Of these, methyl is preferred. The halogen atom is preferably a fluorine atom or a chlorine atom. When any of these groups has two substituents which are a $C_1$-$C_6$ alkyl group or a halogen atom, the substituents may be identical to or different from each other, and may be bonded to the same carbon atom of the group or to different carbon atoms thereof. When any of these groups has a $C_1$-$C_6$ alkyl group or a halogen atom as a substituent, the number of the $C_1$-$C_6$ alkyl group or the halogen atom is preferably 1 or 2. Q is preferably a single bond, $-CH_2-O-$ optionally having 1 or 2 $C_1$-$C_6$ alkyl groups or halogen atoms as substituents, or $-CH_2-CH_2-CH_2-O-$ optionally having 1 or 2 $C_1$-$C_6$ alkyl groups or halogen atoms as substituents, with $-CH_2-O-$ having no substituent being more preferred. Each of the exemplified groups of Q may be linked, on its right or left side, to Y in formula (I).

In formula (I), Y represents the following groups:

[F8]

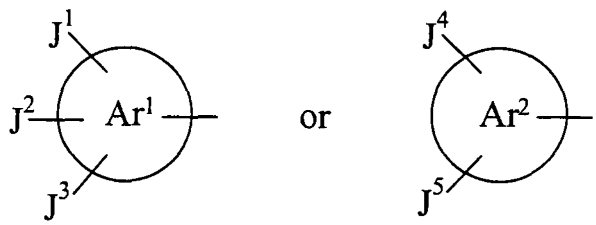

(wherein each of $Ar^1$ and $Ar^2$ independently represents a benzene ring or a 5- or 6-membered aromatic heterocycle; each of $J^1$, $J^2$, $J^3$, $J^4$, and $J^5$ independently represents a hydrogen atom, a halogen atom, a hydroxyl group, a nitro group, a cyano group, a $C_1$-$C_6$ alkyl group, a halogeno-$C_1$-$C_6$ alkyl group, a $C_1$-$C_6$ alkoxy-$C_1$-$C_6$ alkyl group, a $C_1$-$C_6$ alkoxy group, a halogeno-$C_1$-$C_6$ alkoxy group, an amino group, a mono-$C_1$-$C_6$ alkylamino group, a di-$C_1$-$C_6$ alkylamino group, a carboxyl group, a $C_1$-$C_6$ alkoxycarbonyl group, a carbamoyl group, a mono-$C_1$-$C_6$ alkylcarbamoyl group, a di-$C_1$-$C_6$ alkylcarbamoyl group, a sulfamoyl group, a mono-$C_1$-$C_6$ alkylsulfamoyl group, a di-$C_1$-$C_6$ alkylsulfamoyl group, an optionally substituted phenyl group, an optionally substituted benzyl group, an optionally substituted phenethyl group, an optionally substituted styryl group, an optionally substituted phenoxy group, an optionally substituted benzyloxy group, an optionally substituted phenoxymethyl group, an optionally substituted 3- to 8-membered cycloalkyl group, an optionally substituted 3- to 8-membered cycloalkenyl group, an optionally substituted 3- to 8-membered cycloalkylmethyl group, an optionally substituted 3- to 8-membered cycloalkyloxy group, an optionally substituted 3- to 8-membered cycloalkylmethoxy group, an optionally substituted 5- or 6-membered aromatic heterocyclic group, an optionally substituted 4- to 6-membered saturated heterocyclic group, or an optionally substituted 5- or 6-membered heteroaryloxy group).

In Y, each of $Ar^1$ and $Ar^2$ independently represents a benzene ring or a 5- or 6-membered aromatic heterocycle. The 5- or 6-membered aromatic heterocycle include a furan ring, a thiophene ring, a pyrrole ring, an oxazole ring, a thiazole ring, an imidazole ring, an isoxazole ring, an isothiazole ring, a pyrazole ring, a triazole ring, a pyridine ring, a pyridazine ring, a pyrimidine ring, and a pyrazine ring. Of these, a furan ring, a thiophene ring, an oxazole ring, a thiazole ring, an imidazole ring, a pyrazole ring, a triazole ring, a pyridine ring, and a pyridazine ring are preferred. Examples of preferred $Ar^1$ include a benzene ring, a furan ring, a thiophene ring, a pyrrole ring, an imidazole ring, a pyrazole ring, a pyridine ring, a pyridazine ring, a pyrimidine ring, and a pyrazine ring. Of these, a benzene ring, a furan ring, a thiophene ring, an imidazole ring, a pyrazole ring, a pyridine ring, and a pyridazine ring, are more preferred, with a benzene ring, a thiophene ring, and a pyrazole ring being still more preferred. Examples of preferred $Ar^2$ include an oxazole ring, a thiazole ring, an isoxazole ring, an isothiazole ring, and a triazole ring. Of these, an oxazole ring, a thiazole ring, and a triazole ring are more preferred.

In Y, each of $J^1$, $J^2$, $J^3$, $J^4$, and $J^5$ independently represents a hydrogen atom, a halogen atom, a hydroxyl group, a nitro group, a cyano group, a $C_1$-$C_6$ alkyl group, a halogeno-$C_1$-$C_6$ alkyl group, a $C_1$-$C_6$ alkoxy-$C_1$-$C_6$ alkyl group, a $C_1$-$C_6$ alkoxy group, a halogeno-$C_1$-$C_6$ alkoxy group, an amino group, a mono-$C_1$-$C_6$ alkylamino group, a di-$C_1$-$C_6$ alkylamino group, a carboxyl group, a $C_1$-$C_6$ alkoxycarbonyl group, a carbamoyl group, a mono-$C_1$-$C_6$ alkylcarbamoyl group, a di-$C_1$-$C_6$ alkylcarbamoyl group, a sulfamoyl group, a mono-$C_1$-$C_6$ alkylsulfamoyl group, a di-$C_1$-$C_6$ alkylsulfamoyl group, an optionally substituted phenyl group, an optionally substituted benzyl group, an optionally substituted phenethyl group, an optionally substituted styryl group, an optionally substituted phenoxy group, an optionally substituted benzyloxy group, an optionally substituted phenoxymethyl group, an optionally substituted 3- to 8-membered cycloalkyl group, an optionally substituted 3- to 8-membered cycloalkenyl group, an optionally substituted 3- to 8-membered cycloalkylmethyl group, an optionally substituted 3-to 8-membered cycloalkyloxy group, an optionally substituted 3- to 8-membered cycloalkylmethoxy group, an optionally substituted 5 -or 6-membered aromatic heterocyclic group, an optionally substituted 4- to 6-membered saturated heterocyclic group, or an optionally substituted 5- or 6-membered heteroaryloxy group. A hydrogen atom, a halogen atom, a nitro group, a cyano group, a $C_1$-$C_6$ alkyl group, a halogeno-$C_1$-$C_6$ alkyl group, a $C_1$-$C_6$ alkoxy-$C_1$-$C_6$ alkyl group, a $C_1$-$C_6$ alkoxy group, a halogeno-$C_1$-$C_6$ alkoxy group, a di-$C_1$-$C_6$ alkylamino group, an optionally substituted phenyl group, an optionally substituted phenethyl group, an optionally substituted phenoxy group, an optionally substituted benzyloxy group, an optionally substituted 3- to 8-membered cycloalkyl group, an optionally substituted 3- to 8-membered cycloalkenyl group, an optionally substituted 3- to 8-membered cycloalkylmethyl group, an optionally substituted 3-to 8-membered cycloalkylmethoxy group, and an optionally substituted 5- or 6-membered aromatic heterocyclic group are preffered. More preferred are a hydrogen atom, a halogen atom, a nitro group, a cyano group, a $C_1$-$C_6$ alkyl group, a halogeno-$C_1$-$C_6$ alkyl group, a $C_1$-$C_6$ alkoxy-$C_1$-$C_6$ alkyl group, a $C_1$-$C_6$ alkoxy group, a halogeno-$C_1$-$C_6$ alkoxy group, an optionally substituted phenyl group, and an optionally substituted 3- to 8-membered cycloalkyl group.

One preferred embodiment of Y is the following group:

[F9]

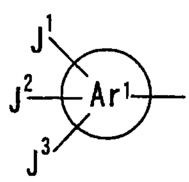

(wherein $Ar^1$ is a benzene ring, a furan ring, a thiophene ring, an imidazole ring, a pyrazole ring, a pyridine ring, or a pyridazine ring, and each of $J^1$, $J^2$, and $J^3$ is independently a hydrogen atom, a halogen atom, a hydroxyl group, a nitro group, a cyano group, a $C_1$-$C_6$ alkyl group, a halogeno-$C_1$-$C_6$ alkyl group, a $C_1$-$C_6$ alkoxy-$C_1$-$C_6$ alkyl group, a $C_1$-$C_6$ alkoxy group, a halogeno-$C_1$-$C_6$ alkoxy group, an amino group, a mono-$C_1$-$C_6$ alkylamino group, a di-$C_1$-$C_6$ alkylamino group, a carboxyl group, a $C_1$-$C_6$ alkoxycarbonyl group, a carbamoyl group, a mono-$C_1$-$C_6$ alkylcarbamoyl group, a di-$C_1$-$C_6$ alkylcarbamoyl group, a sulfamoyl group, a mono-$C_1$-$C_6$ alkylsulfamoyl group, a di-$C_1$-$C_6$ alkylsulfamoyl group, an optionally substituted phenyl group, an optionally substituted benzyl group, an optionally substituted phenethyl group, an optionally substituted styryl group, an optionally substituted phenoxy group, an optionally substituted benzyloxy group, an optionally substituted phenoxymethyl group, an optionally substituted 3- to 8-membered cycloalkyl group, an optionally substituted 3- to 8-membered cycloalkenyl group, an optionally substituted 3- to 8-membered cycloalkylmethyl group, an optionally substituted 3-to 8-membered cycloalkyloxy group, an optionally substituted 3- to 8-membered cycloalkylmethoxy group, an optionally substituted 5- or 6-membered aromatic heterocyclic group, an optionally substituted 4- to 6-membered saturated heterocyclic group, or an optionally substituted 5- or 6-membered heteroaryloxy group).

Another preferred embodiment of Y is the following group:

[F10]

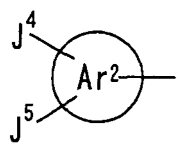

(wherein $Ar^2$ is an oxazole ring, a thiazole ring, or a triazole ring, and each of $J^1$, $J^2$, and $J^3$ is independently a hydrogen atom, a halogen atom, a hydroxyl group, a nitro group, a cyano group, a $C_1$-$C_6$ alkyl group, a halogeno-$C_1$-$C_6$

alkyl group, a $C_1$-$C_6$ alkoxy-$C_1$-$C_6$ alkyl group, a $C_1$-$C_6$ alkoxy group, a halogeno-$C_1$-$C_6$ alkoxy group, an amino group, a mono-$C_1$-$C_6$ alkylamino group, a di-$C_1$-$C_6$ alkylamino group, a carboxyl group, a $C_1$-$C_6$ alkoxycarbonyl group, a carbamoyl group, a mono-$C_1$-$C_6$ alkylcarbamoyl group, a di-$C_1$-$C_6$ alkylcarbamoyl group, a sulfamoyl group, a mono-$C_1$-$C_6$ alkylsulfamoyl group, a di-$C_1$-$C_6$ alkylsulfamoyl group, an optionally substituted phenyl group, an optionally substituted benzyl group, an optionally substituted phenethyl group, an optionally substituted styryl group, an optionally substituted phenoxy group, an optionally substituted benzyloxy group, an optionally, substituted phenoxymethyl group, an optionally substituted 3- to 8-membered cycloalkyl group, an optionally substituted 3- to 8-membered cycloalkenyl group, an optionally substituted 3- to 8-membered cycloalkylmethyl group, an optionally substituted 3-to 8-membered cycloalkyloxy group, an optionally substituted 3- to 8-membered cycloalkylmethoxy group, an optionally substituted 5- or 6-membered aromatic heterocyclic group, an optionally substituted 4- to 6-membered saturated heterocyclic group, or an optionally substituted 5- or 6-membered heteroaryloxy group).

Y is preferably the following group:

[F11]

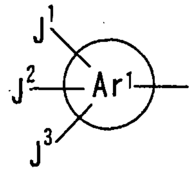

(wherein $Ar^1$ is a benzene ring, a furan ring, a thiophene ring, an imidazole ring, a pyrazole ring, a pyridine ring, or a pyridazine ring, each of $J^1$, $J^2$, and $J^3$ is independently a hydrogen atom, a halogen atom, a nitro group, a cyano group, a $C_1$-$C_6$ alkyl group, a halogeno-$C_1$-$C_6$ alkyl group, a $C_1$-$C_6$ alkoxy-$C_1$-$C_6$ alkyl group, a $C_1$-$C_6$ alkoxy group, a halogeno-$C_{1-}$ $C_6$ alkoxy group, a di-$C_1$-$C_6$ alkylamino group, an optionally substituted phenyl group, an optionally substituted phenethyl group, an optionally substituted phenoxy group, an optionally substituted benzyloxy group, an optionally substituted 3- to 8-membered cycloalkyl group, an optionally substituted 3- to 8-membered cycloalkenyl group, an optionally substituted 3-to 8-membered cycloalkylmethyl group, an optionally substituted 3- to 8-membered cycloalkylmethoxy group, or an optionally substituted 5- or 6-membered aromatic heterocyclic group. More preferred are the following cases: $J^1$ is a halogeno-$C_1$-$C_6$ alkyl group, $J^2$ is a $C_1$-$C_6$ alkyl group, a halogeno-$C_1$-$C_6$ alkyl group, an optionally substituted phenyl group, or an optionally substituted 3- to 8-membered cycloalkyl group, and $J^3$ is a hydrogen atom; $J^1$ is a nitro group, a cyano group, or a $C_1$-$C_6$ alkoxy-$C_1$-$C_6$ alkyl group, $J^2$ is an optionally substituted phenyl group or an optionally substituted 3- to 8-membered cycloalkyl group, and $J^3$ is a hydrogen atom; $J^1$ is a $C_1$-$C_6$ alkoxy group, $J^2$ is a $C_1$-$C_6$ alkyl group, an optionally substituted phenyl group, or an optionally substituted 3- to 8-membered cycloalkyl group, and $J^3$ is a hydrogen atom; and $J^1$ is a halogeno-$C_1$-$C_6$ alkoxy group, $J^2$ is a $C_1$-$C_6$ alkyl group, an optionally substituted phenyl group, or an optionally substituted 3- or 8-membered cycloalkyl group, and $J^3$ is a hydrogen atom. $Ar^1$ is preferably a benzene ring, a thiophene ring, or a pyrazole ring.

More specifically, when $Ar^1$ is a benzene ring, preferably, any one of $J^1$, $J^2$, and $J^3$ is a hydrogen atom, and each of the other two of them is independently a hydrogen atom, a halogen atom, a nitro group, a cyano group, a $C_1$-$C_6$ alkyl group, a halogeno-$C_1$-$C_6$ alkyl group, a $C_1$-$C_6$ alkoxy-$C_1$-$C_6$ alkyl group, a $C_1$-$C_6$ alkoxy group, a halogeno-$C_1$-$C_6$ alkoxy group, an optionally substituted phenyl group, or an optionally substituted 3- to 8-membered cycloalkyl group. More preferred are the following cases: $J^1$ is a halogeno-$C_1$-$C_6$ alkyl group, $J^2$ is a halogeno-$C_1$-$C_6$ alkyl group, an optionally substituted phenyl group, or an optionally substituted 3- to 8-membered cycloalkyl group, and $J^3$ is a hydrogen atom; $J^1$ is a nitro group, a cyano group, or a $C_1$-$C_6$ alkoxy-$C_1$-$C_6$ alkyl group, $J^2$ is an optionally substituted phenyl group or an optionally substituted 3- to 8-membered cycloalkyl group, and $J^3$ is a hydrogen atom; $J^1$ is an optionally substituted phenol group and each of $J^2$ and $J^3$ is a hydrogen atom; $J^1$ is a $C_1$-$C_6$ alkoxy group, $J^2$ is a $C_1$-$C_6$ alkyl group, an optionally substituted phenyl group, or an optionally substituted 3- to 8-membered cycloalkyl group, and $J^3$ is a hydrogen atom; and $J^1$ is a halogeno-$C_1$-$C_6$ alkoxy group, $J^2$ is a $C_1$-$C_6$ alkyl group, an optionally substituted phenyl group, or an optionally substituted 3- to 8-membered cycloalkyl group, and $J^3$ is a hydrogen atom.

When $Ar^1$ is a furan ring, a thiophene ring, an imidazole ring, a pyrazole ring, a pyridine ring, or a pyridazine ring, preferably, any one of $J^1$, $J^2$ , and $J^3$ is a hydrogen atom, and each of the other two of them is independently a hydrogen atom, a halogen atom, a nitro group, a cyano group, a $C_1$-$C_6$ alkyl group, a halogeno-$C_1$-$C_6$ alkyl group, a $C_1$-$C_6$ alkoxy-$C_1$-$C_6$ alkyl group, a $C_1$-$C_6$ alkoxy group, a halogeno-$C_1$-$C_6$ alkoxy group, an optionally substituted phenyl

group, or an optionally substituted 3- to 8-membered cycloalkyl group. More preferred is the following case: $J^1$ is a halogeno-$C_1$-$C_6$ alkyl group, $J^2$ is a $C_1$-$C_6$ alkyl group, an optionally substituted phenyl group, or an optionally substituted 3- to 8-membered cycloalkyl group, and $J^3$ is a hydrogen atom.

When $Ar^1$ is a benzene ring, and only one of $J^1$, $J^2$, and $J^3$ is a hydrogen atom, preferably, Q is linked to 1-position of the benzene ring, the hydrogen atom in $J^1$, $J^2$, or $J^3$ is linked to 5-position of the benzene ring, and the other two groups are linked to 3- and 4-positions; or Q is linked to the 1-position of the benzene ring, the hydrogen atom in $J^1$, $J^2$, or $J^3$ is linked to 3-position of the benzene ring, and the other two groups are linked to 2- and 4-positions. When $Ar^1$ is a benzene ring and each of the other two of $J^1$, $J^2$, and $J^3$ is a hydrogen atom, preferably, Q is linked to 1-position of the benzene ring, and one of $J^1$, $J^2$ , and $J^3$, which is other than a hydrogen atom, is linked to 4-position of the benzene ring.

When $Ar^1$ is a furan ring or a thiophene ring, preferably, Q is linked to the 2-position of the furan or thiophene ring, and $J^1$, $J^2$ , and $J^3$ are linked to 3-, 4-, and 5-positions of the furan or thiophene ring, respectively. When only one of $J^1$, $J^2$, and $J^3$ is a hydrogen atom, the hydrogen atom is preferably linked to 3-position of the furan or thiophene ring.

When $Ar^1$ is an imidazole ring, preferably, Q is linked t 4-position of the imidazole ring, and $J^1$, $J^2$, and $J^3$ are linked to 1-, 2-, and 5-positions of the imidazole ring, respectively. When only one of $J^1$, $J^2$, and $J^3$ is a hydrogen atom, a hydrogen atom is preferably linked to 5-position of the imidazole ring.

When $Ar^1$ is a pyrazole ring, preferably, Q is linked to 3-position of the pyrazole ring, and $J^1$, $J^2$, and $J^3$ are linked to 1-, 4-, and 5-positions of the pyrazole ring, respectively. When only one of $J^1$, $J^2$ , and $J^3$ is a hydrogen atom, a hydrogen atom is preferably linked to 4-position of the pyrazole ring. Also preferably, Q is linked to 4-position of the pyrazole ring, and $J^1$, $J^2$, and $J^3$ are linked to 1-, 3-, and 5-positions of the pyrazole ring, respectively. When only one of $J^1$, $J^2$, and $J^3$ is a hydrogen atom, a hydrogen atom is preferably linked to 5-position of the pyrazole ring.

When $Ar^1$ is a pyridine ring, preferably, Q is linked to 2- or 3-position of the pyridine ring, and $J^1$, $J^2$, and $J^3$ are linked to 4-, 5-, and 6-positions of the pyridine ring, respectively. When only one of $J^1$, $J^2$, and $J^3$ is a hydrogen atom, a hydrogen atom is preferably linked to 4-position of the pyridine ring.

When $Ar^1$ is a pyridazine ring, preferably, Q is linked to 3-position of the pyridazine ring, and $J^1$, $J^2$, and $J^3$ are linked to 4-, 5-, and 6-positions of the pyridazine ring, respectively. When only one of $J^1$, $J^2$, and $J^3$ is a hydrogen atom, the hydrogen atom is preferably linked to 4-position of the pyridazine ring.

Y is preferably the following group:

[F12]

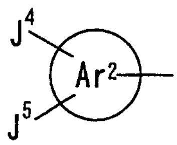

(wherein $Ar^2$ is an oxazole ring, a thiazole ring, or a triazole ring, each of $J^4$ and $J^5$ is independently a hydrogen atom, a halogen atom, a nitro group, a cyano group, a $C_1$-$C_6$ alkyl group, a halogeno-$C_1$-$C_6$ alkyl group, a $C_1$-$C_6$ alkoxy-$C_1$-$C_6$ alkyl group, a $C_1$-$C_6$ alkoxy group, a halogeno-$C_1$-$C_6$ alkoxy group, an optionally substituted phenyl group, or an optionally substituted 3- to 8-membered cycloalkyl group). More preferably, $J^4$ is a halogeno-$C_1$-$C_6$ alkyl group, and $J^5$ is an optionally substituted phenyl group.

When $Ar^2$ is an oxazole ring or a thiazole ring, preferably, Q is linked to 2-position of the oxazole or thiazole ring, and $J^4$ and $J^5$ are linked to 4- and 5-positions of the oxazole or thiazole ring, respectively.

When $Ar^2$ is a triazole ring, an 1,2,4-triazole ring is preferred. Preferably, Q is linked to 3-position of the 1,2,4-triazole ring, and $J^4$ and $J^5$ are linked to 1- and 5-positions of the 1,2,4-triazole ring, respectively.

When each of $J^1$, $J^2$ , $J^3$ , $J^4$ , and $J^5$ in Y is independently a hydrogen atom, a halogen atom, a hydroxyl group, a nitro group, a cyano group, a $C_1$-$C_6$ alkyl group, a halogeno-$C_1$-$C_6$ alkyl group, a $C_1$-$C_6$ alkoxy-$C_1$-$C_6$ alkyl group, a $C_1$-$C_6$ alkoxy group, a halogeno-$C_1$-$C_6$ alkoxy group, an amino group, a mono-$C_1$-$C_6$ alkylamino group, a di-$C_1$-$C_6$ alkylamino group, a carboxyl group, a $C_1$-$C_6$ alkoxycarbonyl group, a carbamoyl group, a mono-$C_1$-$C_6$ alkylcarbamoyl group, a di-$C_1$-$C_6$ alkylcarbamoyl group, a sulfamoyl group, a mono-$C_1$-$C_6$ alkylsulfamoyl group, a di-$C_1$-$C_6$ alkylsulfamoyl group, an optionally substituted phenyl group, an optionally substituted benzyl group, an optionally substituted phenethyl group, an optionally substituted styryl group, an optionally substituted phenoxy group, an optionally substituted benzyloxy group, an optionally substituted phenoxymethyl group, an optionally substituted 3- to 8-membered cycloalkyl group, an optionally substituted 3- to 8-membered cycloalkenyl group, an optionally substituted 3- to 8-membered cycloalkylmethyl

group, an optionally substituted 3-to 8-membered cycloalkyloxy group, an optionally substituted 3- to 8-membered cycloalkylmethoxy group, an optionally substituted 5- or 6-membered aromatic heterocyclic group, an optionally substituted 4- to 6-membered saturated heterocyclic group, or an optionally substituted 5- or 6-membered heteroaryloxy group, specific examples of each substituent include those as exemplified above. Among them, a halogen atom, a $C_1$-$C_6$ alkyl group, a cyano group, and a 3-to 8-membered cycloalkyl group are preferred. The $C_1$-$C_6$ alkyl group is preferably methyl group, and the 3- to 8-membered cycloalkyl group is preferably cyclopropyl group.
Specific examples of $J^1$, $J^2$, $J^3$, $J^4$, and $J^5$ include a hydrogen atom, a fluorine atom, a chlorine atom, a nitro group, a cyano group, a methyl group, an ethyl group, a propyl group, an isopropyl group, an n-butyl group, an isobutyl group, a trifluoromethyl group, a 1,1-difluoro-2-methylpropyl group, a methoxy group, an ethoxy group, a trifluoromethoxy group, a dimethylamino group, a phenyl group, a phenethyl group, a phenoxy group, a benzyloxy group, a cyclopropyl group, a cyclobutyl group, a cyclopentyl group, a cyclohexyl group, a 1-methyl-1-cyclopropyl group, a cyclohexen-1-yl group, a cyclopentylmethyl group, a cyclohexylmethyl group, a cyclopentylmethoxy group, and a cyclohexylmethoxy group. Of these, a hydrogen atom, a fluorine atom, a methyl group, an ethyl group, a propyl group, an isopropyl group, an n-butyl group, an isobutyl group, a trifluoromethyl group, a methoxy group, an ethoxy group, a trifluoromethoxy group, a phenyl group, a phenethyl group, a phenoxy group, a benzyloxy group, a cyclopentyl group, and a cyclohexyl group are preferred.

Next, preferred combinations of A, $R^1$, V, $Z^1$, $Z^2$, $Z^3$, Q, and Y will be described.
One preferred combination of the above groups is the following case; A is a single bond; $R^1$ is a hydrogen atom; V is any one group selected from (1) and (2):

(1) -$G^1$- (wherein $G^1$ represents an optionally substituted $C_1$-$C_5$ straight-chain alkylene group),
(2) -$G^2$-N($R^2$) -$G^3$- (wherein $G^2$ represents a single bond or an optionally substituted $C_1$-$C_3$ straight-chain alkylene group, $G^3$ represents an optionally substituted $C_1$-$C_4$ straight-chain alkylene group, and $R^2$ represents a hydrogen atom, a $C_1$-$C_6$ alkyl group, or a 3- to 8-membered cycloalkyl group)); each of $Z^1$ and $Z^2$ is a hydrogen atom; $Z^3$ is a hydrogen atom or a $C_1$-$C_6$ alkyl group; Q is -$CH_2$-O-; and Y is the following group:

[F13]

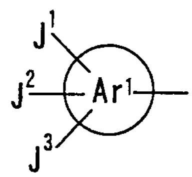

(wherein $Ar^1$ represents a benzene ring, a furan ring, a thiophene ring, an imidazole ring, a pyrazole ring, a pyridine ring, or a pyridazine ring), each of $J^1$, $J^2$, and $J^3$ independently represents a hydrogen atom, a halogen atom, a nitro group, a cyano group, a $C_1$-$C_6$ alkyl group, a halogeno-$C_1$-$C_6$ alkyl group, a $C_1$-$C_6$ alkoxy-$C_1$-$C_6$ alkyl group, a $C_1$-$C_6$ alkoxy group, a halogeno-$C_1$-$C_6$ alkoxy group, a di-$C_1$-$C_6$ alkylamino group, an optionally substituted phenyl group, an optionally substituted phenethyl group, an optionally substituted phenoxy group, an optionally substituted benzyloxy group, an optionally substituted 3- to 8-membered cycloalkyl group, an optionally substituted 3- to 8-membered cycloalkenyl group, an optionally substituted 3- to 8-membered cycloalkylmethyl group, an optionally substituted 3-to 8-membered cycloalkylmethoxy group, or an optionally substituted 5- or 6-membered aromatic heterocyclic group).

Unless otherwise specified, when the compound of the present invention has one or more asymmetric centers in a molecule thereof, the compound encompasses enantiomers of the compound, racemic modifications of the compound, diastereomers of the compound, and mixtures thereof. Also, unless otherwise specified, when the compound of the present invention includes geometrical isomers, the compound encompasses a cis-form of the compound, a trans-form of the compound, and mixtures thereof. Further, unless otherwise specified, when the compound of the present invention includes tautomers, any types of tautomers and mixtures thereof are included.

The present invention encompasses a compound represented by formula (I), a salt thereof, and a solvate thereof. The salt of the compound of the present invention include salts of an alkali metal such as potassium, sodium, or lithium; salts of an alkaline earth metal such as calcium or magnesium; ammonium salts such as tetramethylammonium salts and tetrabutylammonium salts; salts of an organic amine such as triethylamine, methylamine, dimethylamine, N-methylglucamine, or tris(hydroxymethyl)methylamine; inorganic acid salts such as hydrochlorides, phosphates, and nitrates; and organic acid salts such as acetates, lactates, tartrates, oxalates, fumarates, maleates, citrates; methanesulfonates,

ethanesulfonates, and benzenesulfonates.
The solvate of the compound of the present invention include hydrates, methanolates, and ethanolates.

The compound of the present invention, a salt thereof, and a solvate thereof may be present as a pro-drug. The pro-drug includes a compound produced through esterification or amidation of the carboxyl group of the compound represented by formula (I).

The method for producing the compound represented by formula (I) will next be described. However, the production method is not limited to those described below.
The compound represented by formula (I) and intermediates for producing the compound may be produced through any of a variety of known reactions which will be described hereinbelow. In the production, in some cases, a functional group of a starting material or that of an intermediate is protected with an appropriate protective group. Examples of such a functional group include a hydroxyl group, a carboxyl group, an amino group, and a carbonyl group. The type of the protective group and conditions of protection and deprotection may be selected with reference to, for example, "Protective Groups in Organic Synthesis" (T. W. Green and P. G. Wuts, John Wiley & Sons, Inc., New York, 1991).

The compound (I) of the present invention may be produced through [production method 1] described below.

[F14]

[Production method 1]

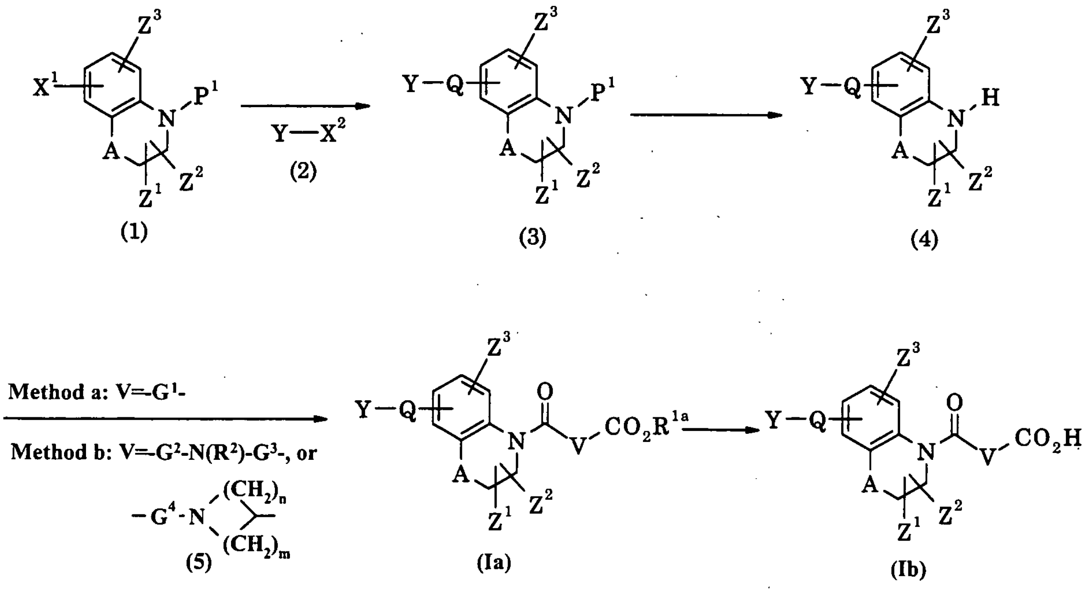


(Wherein, $R^{1a}$ represents $C_1$-$C_6$ alkyl group; $X^1$ and $X^2$ represent groups which react with each other to form Q; $P^1$. represents a protective group; and A, Q, V, $G^1$, $G^2$, $G^3$, $G^4$, $R^2$, m, n, Y, $Z^1$, $Z^2$, and $Z^3$ have the same meanings as defined above in formula (I).

An ester form (Ia) of the compound (I) of the present invention may be produced through the following procedure: a compound (1) is reacted with a compound (2) to thereby yield a compound (3); and subsequently the protective group $P^1$ of the compound (3) is removed to thereby yield a compound (4), followed by performing the method a or b.
The protective group $P^1$ is preferably a carbamate protective group such as a tert-butoxycarbonyl group or a benzyloxycarbonyl group; an arylsulfonyl protective group such as a benzenesulfonyl group or a p-toluenesulfonyl group; or an alkyl nitrogen-atom-protective group such as a methoxymethyl group or a (2-trimethylsilylethoxy)methyl group, particularly preferably a tert-butoxycarbonyl group.

A carboxylic acid derivative (Ib) of the compound (I) of the present invention may be produced through hydrolysis of the ester form (Ia) with an alkali or an acid.

Next will be described [production method A-1] to [production method A-4], which are methods for producing a compound (3) through reaction between the compound (1) and the compound (2) shown in [production method 1].
The compound (3) in which the group Q is $-(CH_2)_p-CH_2-O-$or $-CH_2-O-$ may be produced through the following method.

[F15]

[Production method A-1]

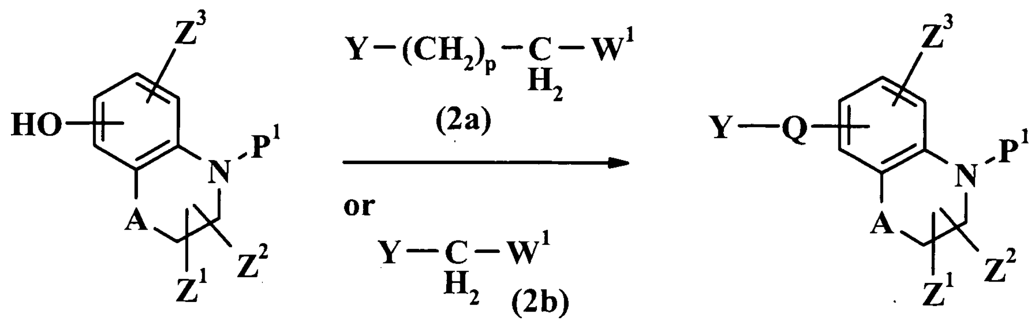


(1a) (3)

Wherein, Q represents $-(CH_2)_p-CH_2-O-$ or $-CH_2-O-$; $W^1$ represents a hydroxyl group or a leaving group; A, $P^1$, Y, $Z^1$, $Z^2$, and $Z^3$ have the same meanings as defined above; and p is 1, 2, or 3.)

The aforementioned [production method A-1] may be roughly classified into two methods as described below.

(1) In the case where $W^1$ is a hydroxyl group

The compound (3) may be produced through treatment of a phenol derivative (1a) and an alcohol derivative (2a or 2b) with an azo reagent and a phosphine compound (Mitsunobu reaction). The azo reagent include azodicarboxylic acid diethyl ester, azodicarboxylic acid diisopropyl ester, 1,1'-(azodicarbonyl)dipiperidine, and 1,1'-azobis(N,N-dimethylformamide). The amount by mole of the azo reagent used is preferably 1.0 to 1.2 times that of the phenol derivative (1a). The phosphine compound include triphenylphosphine, tri-n-butylphosphine, and trimethylphosphine. The amount by mole of the phosphine compound used is preferably 1.0 to 1.3 times that of the phenol derivative (1a) Examples of preferred solvents include ether solvents such as tetrahydrofuran and diethyl ether; aprotic polar solvents such as acetonitrile; hydrocarbon solvents such as toluene and benzene; and halogenated hydrocarbon solvents such as dichloromethane and 1,2-dichloroethane. The reaction temperature is 0°C to the boiling point of the solvent, preferably 0 to 80°C. The reaction time is generally about 1 to about 24 hours.

(2) In the case where $W^1$ is a leaving group

The compound (3) may be produced through alkylation of the phenol derivative (1a) with the compound (2a or 2b) in the presence of a base. The leaving group $W^1$ of the compound (2a) or (2b) is preferably a halogen atom, a methanesulfonyloxy group, a p-toluenesulfonyloxy group, or a benzenesulfonyloxy group. The base include alkali metal hydroxides such as sodium hydroxide, potassium hydroxide, and lithium hydroxide; alkali metal hydrides such as sodium hydride and potassium hydride; and alkali metal carbonates such as sodium carbonate, potassium carbonate, and cesium carbonate. The amount by mole of the base used is preferably 1.0 to 1.5 times that of the compound (2a or 2b). The solvents include inert polar solvents such as N,N-dimethylformamide, dimethyl sulfoxide, N-methylpyrrolidone, and acetonitrile; and ether solvents such as tetrahydrofuran and diethyl ether. The reaction temperature is -20°C to the boiling point of the solvent, preferably 0 to 150°C. The reaction time is generally about 1 to about 48 hours.

The compound (1a) may be a commercially available one, or may be produced through any of the below-described methods.

(1) In the case of compound (la-i) in which A is a single bond

[F16]

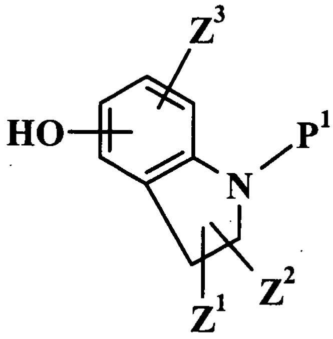

(1a-i)

Wherein, $P^1$, $Z^1$, $Z^2$, and $Z^3$ have the same meanings as defined above.
The compound (Ia-i) may be produced based on, for example, the following procedure: an indole derivative (i.e., an intermediate) is produced based on the method by Christian, G. H., et al. (Org. Lett. 2003, 5, 1899-1902), the method by Michael, E. F., et al. (J. Med. Chem. 1979, 22, 63-69), the method by Joseph, G. C., et al. (J. Heterocycl. Chem. 1990, 27, 2093-2095), the method by Troxler, F., et al. (Helv. Chim. Acta. 1968, 51, 1203-1213), the method by Arnold, L. D., et al. (WO 95/23141), or the method by Pankaj, D. Rege, et al. (Org. Lett. 2006, 8, 3117-3120); and subsequently the indole ring is reduced to the corresponding indoline ring based on the method by Stark, L. M., et al. (J. Org. Chem. 2000, 65, 3227-3230), the method by Kamiya, S., et al. (Chem. Pharm. Bull. 2001, 49, 563-571), or the method by Igarashi, S., et al. (Chem. Pharm. Bull. 2000, 48, 1689-1697).

(2) In the case of compound (Ia-ii) in which A is -O-

[F17]

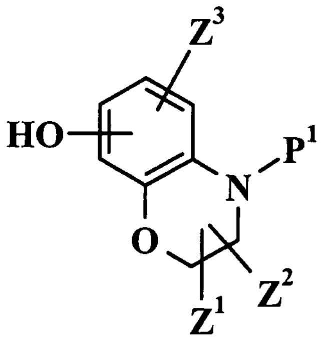

(1a-ii)

Wherein, $P^1$, $Z^1$, $Z^2$ , and $Z^3$ have the same meanings as defined above.
The compound (Ia-ii) may be produced based on, for example, the method by Buon L., et al. (Tetrahedron, 2000, 56, 605-614), the method by Iakovou, K., et al. (Eur. J. Med. Chem. 1999, 34, 903-917), the method by Ilas, J., et al. (Tetrahedron, 2005, 61, 7325-7348), or the method by Francois, C., et al. (EP No. 1,428,514).

(3) In the case of compound (1a-iii) in which A is $-CH_2-$

[F18]

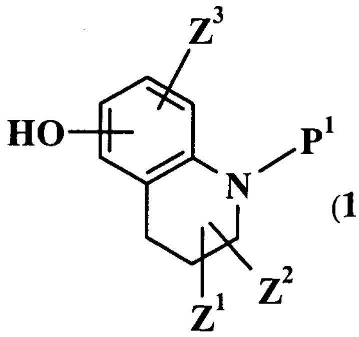

(1a-iii)

Wherein, $P^1$, $Z^1$, $Z^2$, and $Z^3$ have the same meanings as defined above.

The compound (1a-iii) may be produced based on, for example, the method by Atkins, R. L., et al. (J. Org. Chem. 1978, 43 (10), 1975-1980), the method by Michael, H., et al. (J. Chem. Soc., Perkin Trans. 1: Organic and Bio-Organic Chemistry (1972-1999), 1980, 1933-1999), or the method by Selsam, B. L., et al. (WO 2004/026837).

The compound (2a or 2b) may be a commercially available one, or may be produced through any of the below-described methods.

[F19]

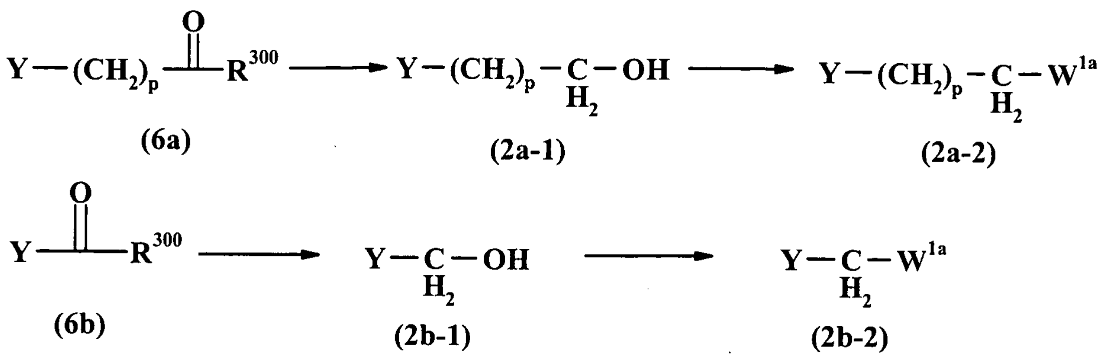

(6a) (2a-1) (2a-2)

(6b) (2b-1) (2b-2)

Wherein $R^{300}$ represents a hydrogen atom, a hydroxyl group, or an alkoxy group; $W^{la}$ represents a leaving group; and p and Y have the same meanings as defined above.

An alcohol form (2a-1), which is a compound (2a) in which $W^1$ is a hydroxyl group, may be produced through reduction of an ester form (6a). Examples of the reference for this procedure include Jikken Kagaku Koza (4th Edition, Vol. 26, edited by The Chemical Society of Japan, Maruzen Co., Ltd.) and "Organic Synthesis VIII: Asymmetric Synthesis·Reduction·Sugar·Labeled Compound, P185-P248."

A compound (2a-2), which is a compound (2a) in which $W^1$ is a leaving group, may be produced by converting the hydroxyl group of the alcohol form (2a-1) into a leaving group such as an alkylsulfonyloxy group, an arylsulfonyloxy group, or a halogen atom through a conventional method. Examples of the reference for this procedure include Jikken Kagaku Koza (4th Edition, Vol. 19, edited by The Chemical Society of Japan, Maruzen Co., Ltd.) and "Organic Synthesis I: Hydrocarbon-Halogen Compound, P438-P446 and P465-470."

An alcohol form (2b-1) and a compound (2b-2) may be produced in a manner similar to that of the aforementioned alcohol form (2a-1) and compound (2a-2).

The aforementioned ester form (6a and 6b) may be a commercially available one, or may be produced based on any of the following methods (a) to (j-3):

(a) the method by Pierre, M., et al. (Tetrahedron Letters, 1985, 26 (33), 3947-3950);
(b) the method by Illig, C. R., et al. (WO 99/40088);

(c) the method by Gattuso, M., et al. (Atti della Societa Peloritana di Science Fische, Matematiche Naturali, 1968, 14 (4), 371-380);
(d) the method by Matsuo, M., et al. (WO 91/19708);
(e) the method by Vicentini, C. B., et al. (Heterocycles, 2000, 53 (6), 1285-1292);
(f) the method by Tensmeyer, L. G., et al. (J. Org. Chem., 1966, 31, 1878-1883);
(g) the method by Padwa, A., et al. (J. Org. Chem., 1982, 47, 786-791);
(h) the method by Capuano, L., et al. (Liebigs Annalen der Chemie, 1985, 12, 2305-2312);
(i) the method by Rafferty, M. F., et al. (J. Med. Chem. 1982, 25, 1204-1208);
(j) the method by Wright, S. W., et al. (J. Org. Chem. 1994, 59, 6095-6097);
(j-1) the method by Liselotte, O., et al. (Synlett, 2001, 1893-1896);
(j-2) the method by Fletcher, S. R., et al. (Bioorg. Med. Chem. Lett., 1992, 2, 627-630); and
(j-3) the method by Ana, B. B., et al. (Tetrahedron Lett. 2005, 46, 7769-7771).

A compound (3) in which the group Q has one or two $C_1$-$C_6$ alkyl groups or fluorine atoms as substituents may be produced in a manner similar to that described above by using, in place of the compound (2a or 2b), a reagent (2c or 2d) having one or two $C_1$-$C_6$ alkyl groups or fluorine atoms as substituents. The compound (2c and 2d) may be a commercially available one, or may be produced in a manner similar to that of the aforementioned compound (2a or 2b).
[F20]

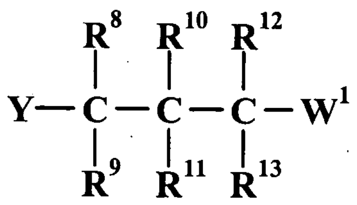
(2c)

or

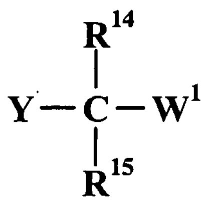
(2d)

Wherein, any one or two of $R^8$ to $R^{13}$ represent $C_1$-$C_6$ alkyl groups or fluorine atoms, and the remaining groups represents hydrogen atoms; one of $R^{14}$ and $R^{15}$ represents an alkyl group or a fluorine atom and the other represents a hydrogen atom, or both of $R^{14}$ and $R^{15}$ represent alkyl groups or fluorine atoms; and $W^1$ and Y have the same meanings as defined above.
A compound (2d-1), which is a compound (2d) in which $W^1$ is a chlorine atom, a bromine atom, or an iodine atom, may be produced through the following method.
[F21]

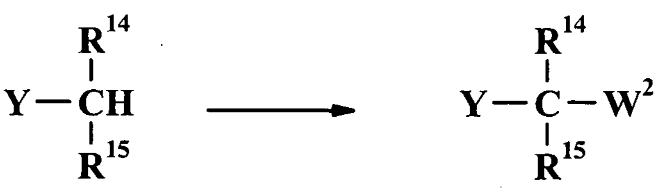
(7) (2d-1)

Wherein, $W^2$ represents a chlorine atom, a bromine atom, or an iodine atom; and $R^{14}$, $R^{15}$ , and Y have the same meanings as defined above.
The compound (2d-1) may be produced through halogenation of a compound (7) with a halogenating reagent such as chlorine, bromine, sulfuryl chloride, N-bromosuccinimide, N-chlorosuccinimide, N-iodosuccinimide, or tert-butyl

hypochlorite. This halogenation reaction may be carried out under light irradiation, or in the presence of a catalyst such as perbenzoic acid. Examples of the reference for this halogenation reaction include Jikken Kagaku Koza (4th Edition, Vol. 19, edited by The Chemical Society of Japan, Maruzen Co., Ltd.), and "Organic Synthesis I: Hydrocarbon-Halogen Compound, P427-P429."

The aforementioned compound (7) may be a commercially available one, or may be produced with reference to the method described in any of the following references (k) to (P) :

(k) the method by Gupta, A. K., et al. (Synlett, 2004, 12, 2227-2229);
(1) the method by Casalnuovo, A. L., et al. (J. Am. Chem. Soc. 1990, 112, 4324-4330);
(m) the method by Schlosser, M., et al. (Eur. J. Org. Chem. 2002, 2913-2920);
(n) the method by Kotone, A., et al. (JP-A-1976-093999);
(o) the method by Lyga, J. W., et al. (Journal of Heterocyclic Chemistry 1990, 27 (4), 9191-921); and
(p) the method by Shridhar, D. R., et al. (Indian Journal of Chemistry, Section B: Organic Chemistry Including Medicinal Chemistry 1983, 22B (12), 1187-1190).

A compound (3) in which the group Q is $-CH_2-O-CH_2-$ may be produced through the following method.

[F22]

[Production method A-2]

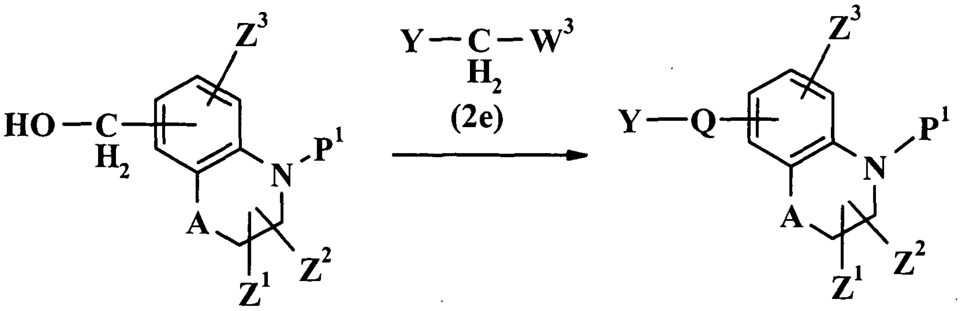


(1b) (3)

In the above scheme, Q represents $-CH_2-O-CH_2-$; $W^3$ represents a leaving group; and A, $P^1$, Y, $Z^1$, $Z^2$, and $Z^3$ have the same meanings as defined above.

The compound (3) may be produced through alkylation of a compound (1b), which is an an alcohol derivative, with a compound (2e) in the presence of a base. The leaving group $W^3$ of the compound (2e) is preferably, for example, a halogen atom, a methanesulfonyloxy group, a toluenesulfonyloxy group, or a benzenesulfonyloxy group. Examples of the base include alkali metal hydroxides such as sodium hydroxide, potassium hydroxide, and lithium hydroxide; alkali metal hydrides such as sodium hydride and potassium hydride; and alkali metal carbonates such as sodium carbonate, potassium carbonate, and cesium carbonate. Examples of usable solvents include aprotic polar solvents such as N,N-dimethylformamide, dimethyl sulfoxide, N-methylpyrrolidone, and acetonitrile; and ether solvents such as tetrahydrofuran and diethyl ether. The reaction temperature is -20°C to the boiling point of a solvent used, preferably 0 to 100°C. The reaction time is generally 1 to 48 hours.

The compound (1b) may be a commercially available one, or may be produced through any of the below-described methods.

(1) In the case of compound (1b-i) in which A is a single bond

[F23]

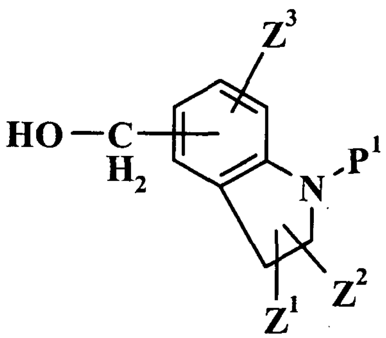

(1b-i)

In the above formula, $P^1$, $Z^1$, $Z^2$, and $Z^3$ have the same meanings as defined above.
The compound (1b-i) may be produced through a method similar to that described in any of the references for production of the compound (1a-i), or based on the method by Kamiya, S., et al. (Chem. Pharm. Bull. 2001, 49 (5), 563-571).

(2) Compound (1b-ii) in which A is -O-

[F24]

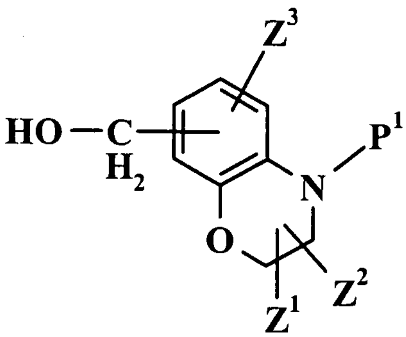

(1b-ii)

In the above formula, $P^1$, $Z^1$, $Z^2$, and $Z^3$ have the same meanings as defined above.
The compound (1b-ii) may be produced through a method similar to that described in any of the references for production of the aforementioned compound (la-ii).

(3) In the case of compound (1b-iii) in which A is $-CH_2-$

[F25]

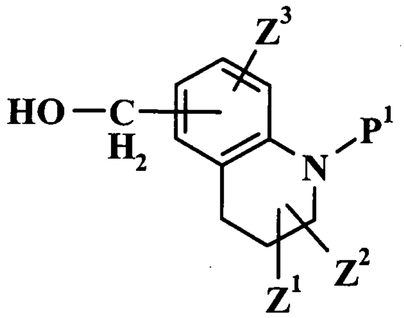

(1b-iii)

In the above formula, $P^1$, $Z^1$, $Z^2$, and $Z^3$ have the same meanings as defined above.
The compound (1b-iii) may be produced through a method similar to that for producing the aforementioned compound (1a-iii); the method by Timothy, G., et al. (US No. 6,362,188); or based on the method by Oku, T., et al. (WO 97/11069). The compound (2e) may be a commercially available one, or may be produced through the following method.

[F26]

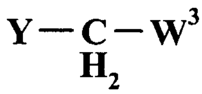

(2e)

In the above formula, $W^3$ and Y have the same meanings as defined above.
The compound (2e) may be produced through a method similar to that for producing the aforementioned compound (2b) or (2b-2).

The compound (3) in which the group Q has one or two $C_1$-$C_6$ alkyl groups or fluorine atoms as substituents may be produced in a manner similar to that described above by using, in place of the compound (1b) and/or the compound (2e), a reagent (1b-2) and/or (2f) having one or two $C_1$-$C_6$ alkyl groups or fluorine atoms as substituents.

[F27]

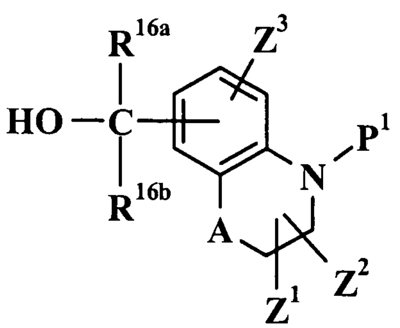

(1b-2)

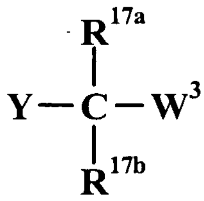

(2f)

In the above formulas, one of $R^{16a}$ and $R^{16b}$ is a $C_1$-$C_6$ alkyl group or a fluorine atom and the other is a hydrogen atom, or both of $R^{16a}$ and $R^{16b}$ are alkyl groups or fluorine atoms; one of $R^{17a}$ and $R^{17b}$ is a $C_1$-$C_6$ alkyl group or a fluorine atom and the other is a hydrogen atom, or both of $R^{17a}$ and $R^{17b}$ are alkyl groups or fluorine atoms; and A, $P^1$, $W^3$, $Z^1$, $Z^2$, and $Z^3$ have the same meanings as defined above.

The compounds (1b-2 and 2f) may be a commercially available one, or may be produced through a method similar to that for producing the aforementioned compound (2d or 2d-1).

.

A compound (3) in which the group Q is -CH=CH- or $-CH_2-CH_2-$ may be produced through the following method.

[F28]

[Production method A-3]

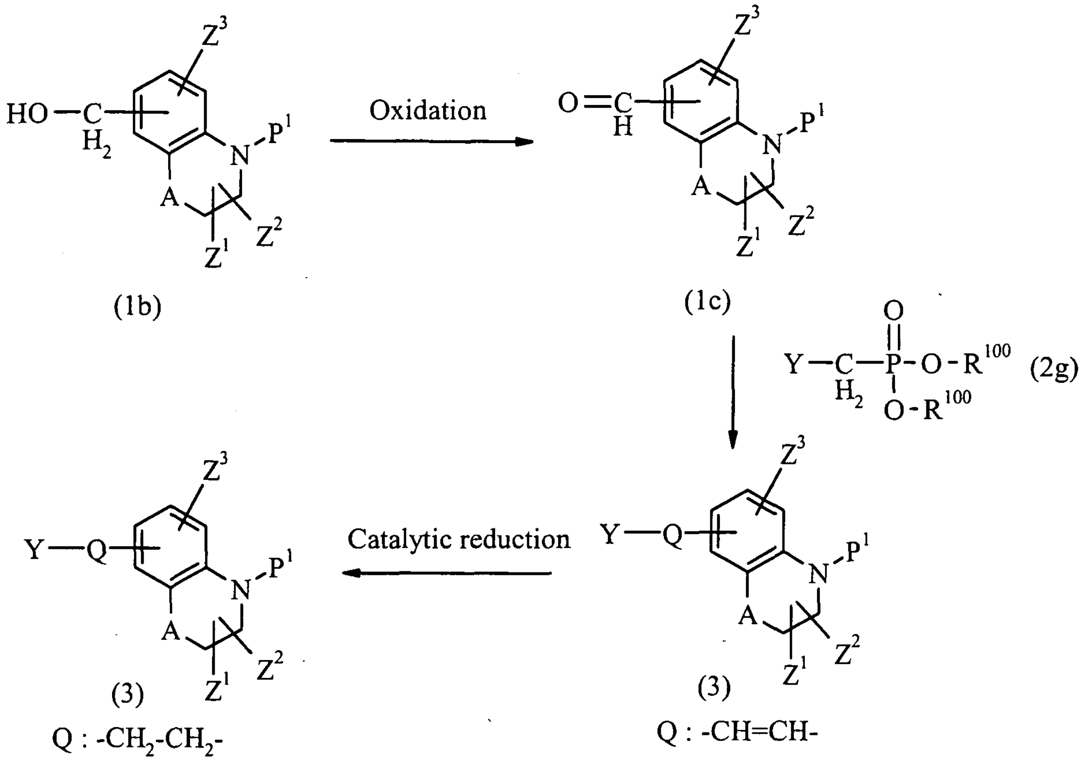


(1b) (1c) (2g) (3) Q : $-CH_2-CH_2-$ (3) Q : -CH=CH-

In the above scheme, Q represents -CH=CH- or $-CH_2-CH_2-$; $R^{100}$ represents a $C_1$-$C_6$ alkyl group; and A, $P^1$, Y, $Z^1$, $Z^2$, and $Z^3$ have the same meanings as defined above.

The compound (3) in which the group Q is -CH=CH- may be produced through the Wadsworth-Emmons reaction how the aforementioned compound (1b) is oxidized to an aldehyde form (1c), and the aldehyde form (1c) is treated with a compound (2g) in the presence of a base.

The oxidizing agent used for oxidation of the aforementioned compound (1b) to the aldehyde form (1c) may be commercially available manganese dioxide. The amount by mole of the oxidizing agent used is 1 to 20 times, preferably 1 to 1.5 times, that of the compound (1b). Examples of usable reaction solvents include hydrocarbon solvents such as toluene, benzene, and hexane; ether solvents such as diethyl ether; and halogenated hydrocarbon solvents such as chloroform and carbon tetrachloride. The reaction temperature is 0°C to the boiling point of a solvent used, preferably room temperature to the boiling point of the solvent. The reaction time is generally about 4 hours to about 48 hours. The Swern oxidation may be carried out based on the method by Bailey, P. S., et al. (Org. Synth., Collective Volume, 1973,

5, 489).

Examples of the base used in the Wadsworth-Emmons reaction for producing the compound (3) from the aldehyde form (1c) include alkali metal hydrides such as sodium hydride and potassium hydride; sodium bis(trimethylsilyl) amide; and potassium bis(trimethylsilyl)amide. The amount by mole of the base used is preferably 1 to 1.2 times that of the compound (2g). Examples of usable solvents include ether solvents such as tetrahydrofuran and tert-butyl methyl ether; and hydrocarbon solvents such as toluene. The reaction temperature is -20°C to the boiling point of a solvent used, preferably 0 to 80°C. The reaction time is generally about 1 to about 24 hours.

The compound (3) in which the group Q is $-CH_2-CH_2-$ may be produced through catalytic reduction of the compound (3) in which the group Q is -CH=CH-. The catalyst used for catalytic reduction may be, for example, palladium (Pd)/carbon, palladium hydroxide $[Pd(OH)_2]$/carbon, or platinum (Pt). Examples of usable solvents include alcohol solvents such as methanol and ethanol; aprotic polar solvents such as N,N-dimethylformamide; ester solvents such as ethyl acetate; ether solvents such as tetrahydrofuran and dioxane; and acetic acid. The reaction temperature is 0°C to the boiling point of a solvent used, preferably room temperature to 80°C. The reaction time is generally about 1 to about 48 hours.

The compound (2g) used in the aforementioned production method may also be produced through the following method.

[F29]

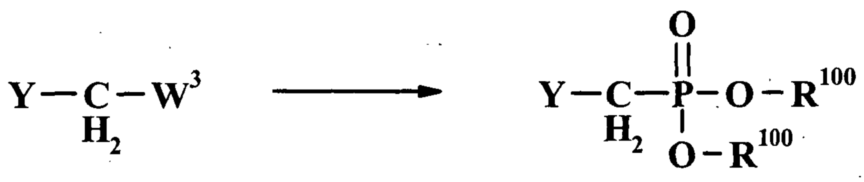

(2e) (2g)

In the above scheme, $R^{100}$, $W^3$, and Y have the same meanings as defined above.
The compound (2g) may be produced by subjecting the compound (2e) shown in [production method A-2] to the Arbuzow reaction. Examples of the reference for the Arbuzow reaction include the method by Gronowitz, S., et al. (Heterocylces 1981, 15 (2), 947-959).

The compound (3) in which the group Q has one or two $C_1$-$C_6$ alkyl groups or fluorine atoms as substituents may also be produced in a manner similar by using, in place of the compound (1c) and/or (2g), a compound (1c-1) and/or (2h) having one $C_1$-$C_6$ alkyl group or fluorine atom as a substituent.

[F30]

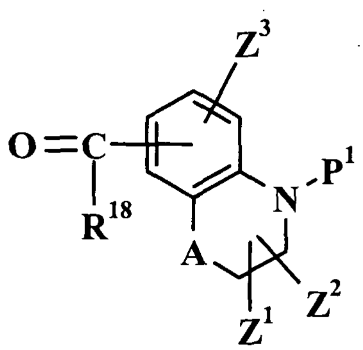

(1c-1)

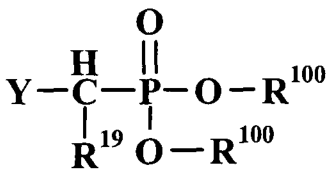

(2h)

In the above formulas, one of $R^{18}$ and $R^{19}$ is an alkyl group or a fluorine atom and the other is a hydrogen atom, or both of $R^{18}$ and $R^{19}$ are alkyl groups or fluorine atoms; and A, $P^1$, $R^{100}$, Y, $Z^1$, $Z^2$, and $Z^3$ have the same meanings as defined above.

The aforementioned compound (1c-1) may be produced through the following method.

[F31]

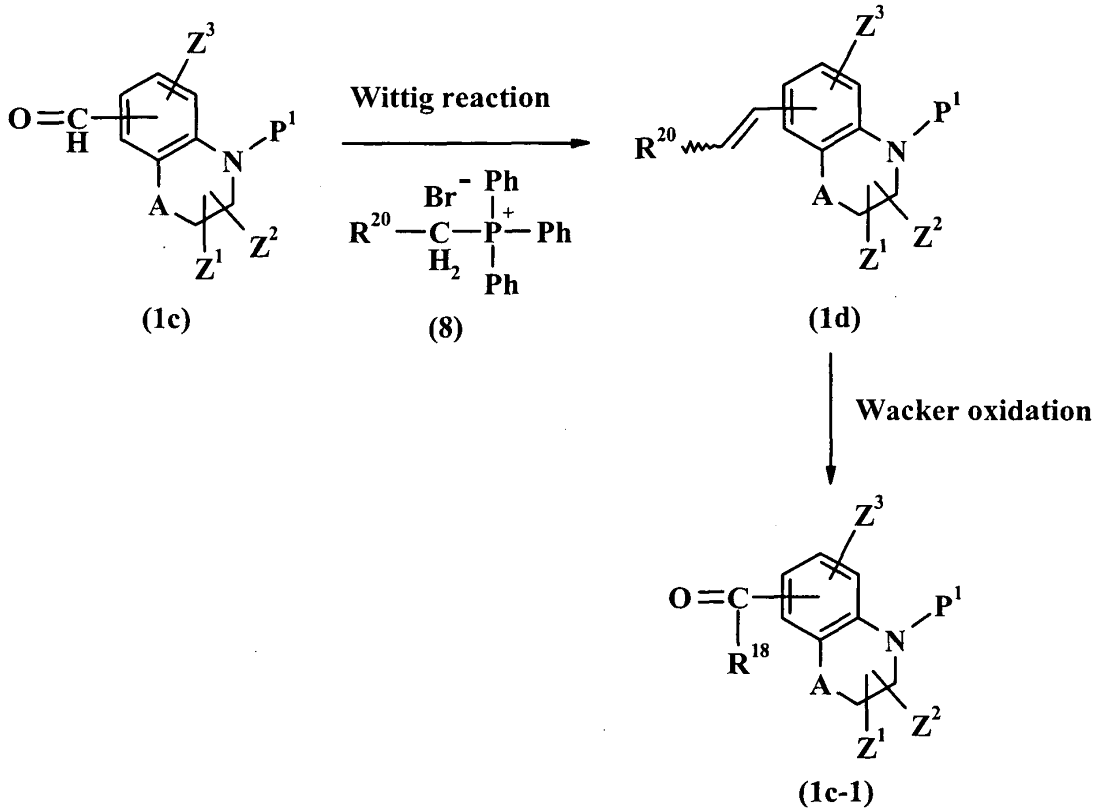


(1c) (8) (1d) (1c-1)

In the above scheme, $R^{20}$ represents a hydrogen atom or a $C_1$-$C_6$ alkyl group; and A, $P^1$, $R^{18}$, $Z^1$, $Z^2$, and $Z^3$ have the same meanings as defined above.

The compound (1c-1) may be produced through the following procedure: the aforementioned compound (1c) is treated with a commercially available Wittig reagent (8) in the presence of a base, to thereby yield an olefin form (1d); and the olefin form (1d) is subjected to the Wacker oxidation.

Examples of the base used for the Wittig reaction include alkali metal hydrides such as sodium hydride and potassium hydride; alkali metal alkoxides such as sodium tert-butoxide and potassium tert-butoxide; sodium bis(trimethylsilyl)amide; and potassium bis(trimethylsilyl)amide. The amount by mole of the base used is preferably 1 to 1.2 times that of the compound (8). Examples of usable solvents include aprotic polar solvents such as dimethyl sulfoxide; ether solvents such as tetrahydrofuran and tert-butyl methyl ether; and hydrocarbon solvents such as toluene. The reaction-temperature is -20°C to the boiling point of a solvent used, preferably 0 to 80°C. The reaction time is generally about 1 to about 24 hours.

The Wacker oxidation may be carried out based on the method by Hegedus, L. S., et al. (Comp. Org. Syn. 1991, 4, 552-559), or the method by Gaunt, M. J., et al. (Chem. Commun. 2001, 18, 1844-1845).

The aforementioned compound (2h) may be produced through the following method.

[F32]

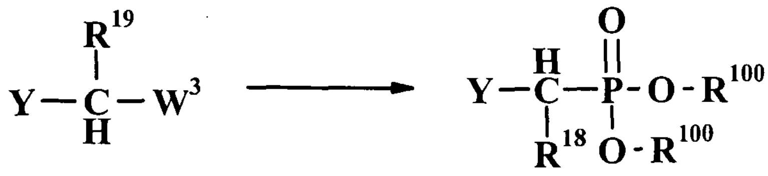

(2i) (2h)

In the above scheme, $R^{19}$, $R^{100}$, $W^3$, and Y have the same meanings as defined above.

The compound (2h) may be produced by subjecting a compound (2i) in which either $R^{17a}$ or $R^{17b}$ in the compound (2f) shown in [production method A-2] is a hydrogen atom to the Arbuzow reaction in a manner similar to that in the case of production of the aforementioned compound (2g).

A compound (3) in which the group Q is $-CH_2-CH_2-CH_2-$ may be produced through the following method.

[F33]

[Production method A-4]

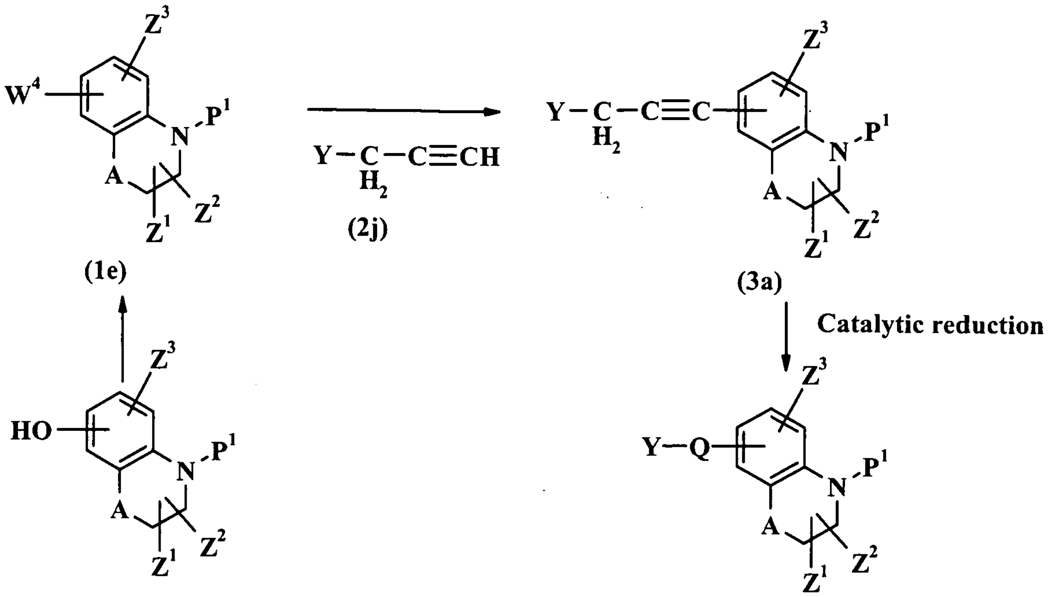


(1e) (2j) (3a) (1a) (3) Q : $-CH_2-CH_2-CH_2-$

In the above scheme, Q represents $-CH_2-CH_2-CH_2-$; $W^4$ represents a leaving group; and A, $P^1$, Y, $Z^1$, $Z^2$, and $Z^3$ have the same meanings as defined above.

The compound (3) in which the group Q is $-CH_2-CH_2-CH_2-$may be produced through the following procedure: a compound (1e) and an acetylene derivative (2j) are subjected to coupling reaction using a metal catalyst, to thereby yield a compound (3a); and subsequently the compound (3a) is subjected to catalytic reduction. Catalytic reduction is carried out under conditions similar to those described in [production method A-3].

The aforementioned coupling reaction is known as the Sonogashira reaction. Examples of the reference for this coupling reaction include the review by K. C. Nicolaou, et al. "Angew. Chem. Int. Ed. 2005, 44, 4442-4489."

The compound (1e) may be a commercially available one, or may be produced from the phenol derivative (1a) based on the method of converting the alcohol form (2a-1 or 2b-1) into the compound (2a-2 or 2b-2) described above in [production method A-1], or through the method by Thompson, L. S. A., et al. (Synthesis, 1994, 2, 107-108).

The compound (2j) used in the aforementioned production method may be produced through the following method.

[F34]

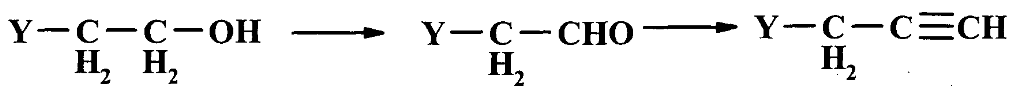

(2a-1) (9) (2j)

In the above scheme, Y has the same meaning as defined above.

The compound (2j) may be produced through the following procedure: the compound (2a-1) is oxidized with manganese dioxide or subjected to the Swern oxidation, to thereby yield a compound (9); and subsequently the compound (9) is subjected to the method by Miwa, K., et al. (Synlett, 1994, 2, 107-108) or the method by Corey, E., et al. (Tetrahedron Lett. 1972, 3769-3772).

The compound (3) in which the group Q has one or two $C_1$-$C_6$ alkyl groups or fluorine atoms as substituents may also be produced in a manner similar to that described above by using, in place of the compound (2j), a reagent (2k) having one or two $C_1$-$C_6$ alkyl groups or fluorine atoms as substituents.

[F35]

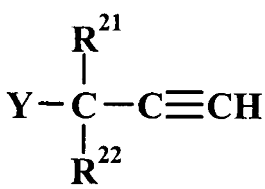

(2k)

In the above formula, one of $R^{21}$ and $R^{22}$ is an alkyl group or a fluorine atom and the other is a hydrogen atom, or both of $R^{21}$ and $R^{22}$ are alkyl groups or fluorine atoms; and Y has the same meaning as defined above.

The aforementioned compound (2k) may be produced through the following method.

[F36]

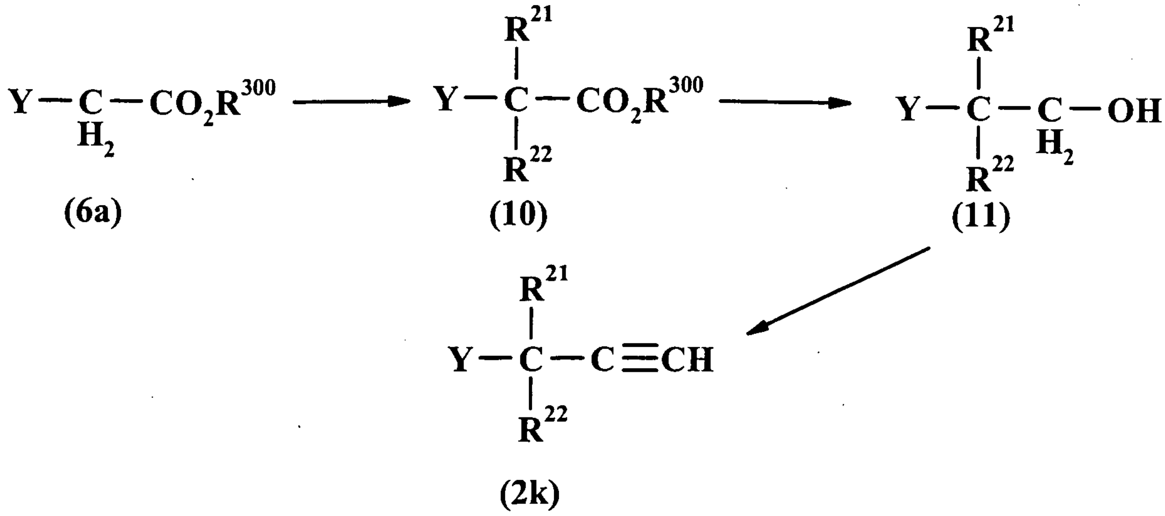

(6a) (10) (11)

(2k)

In the above scheme, $R^{21}$, $R^{22}$, $R^{300}$, and Y have the same meanings as defined above.
The compound (2k) may be produced through the following procedure: a compound (6a) is converted into a compound (10) through alkylation in the presence of a base; subsequently the compound (10) is reduced to an alcohol form (11) with a metal hydride complex compound (e.g., lithium aluminum hydride); and then the alcohol form (11) is subjected to a method similar to the method for producing the aforementioned compound (2j).
The aforementioned alkylation reaction may be carried out based on the method by Prasad, G., et al. (J. Org. Chem. 1991, 56, 7188-7190), or the method by Suzuki, S., et al. (Can. J. Chem. 1994, 72, 357-361).
A compound (3) in which each of the groups Q and A is a single bond may be produced through the following method.
[F37]

[Production method A-5]

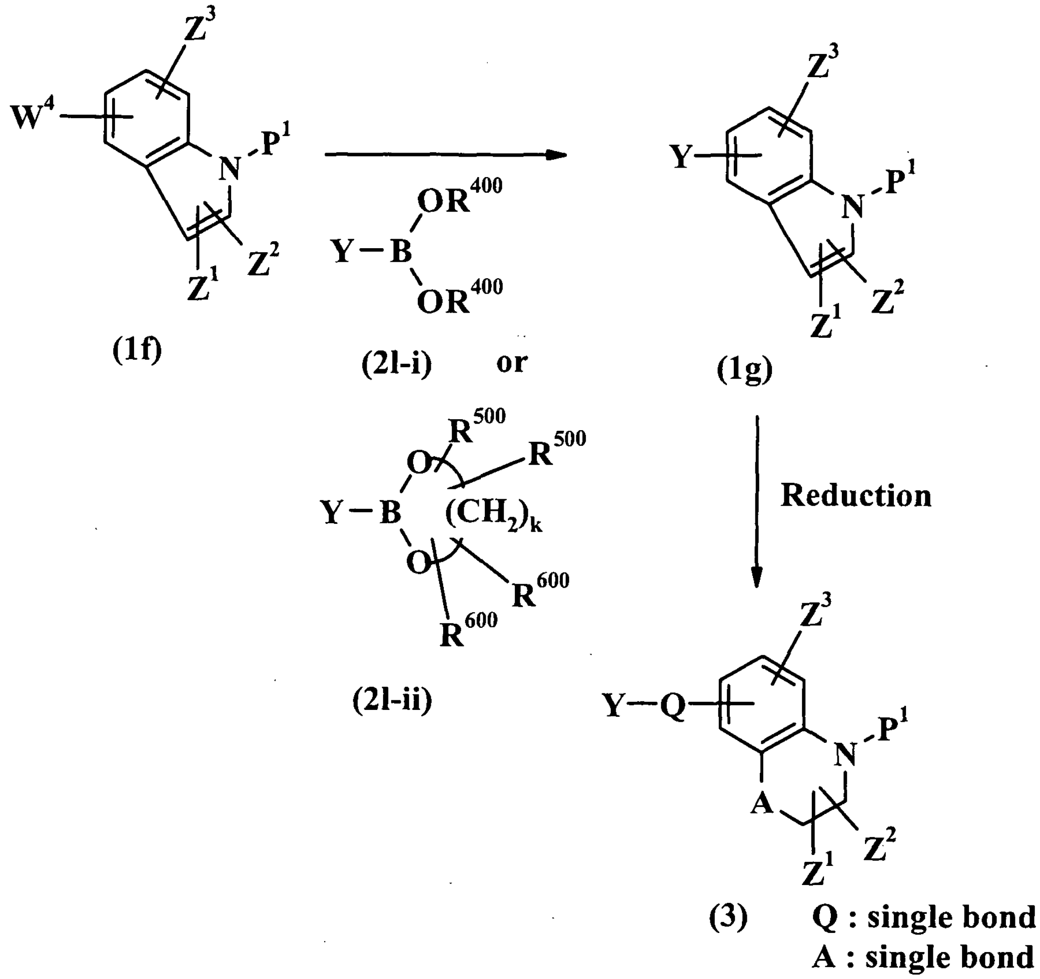


(1f) (2l-i) or (1g)

(2l-ii)

(3) Q : single bond
A : single bond

In the above scheme, each of Q and A represents a single bond; $W^4$ represents a leaving group; $P^1$, Y, $Z^1$, $Z^2$, and $Z^3$ have the same meanings as defined above; k is 2 or 3; and each of $R^{400}$, $R^{500}$, and $R^{600}$ independently represents a hydrogen atom or a $C_1$-$C_6$ alkyl group.

The compound (3) in which each of the groups Q and A is a single bond may be produced through the following procedure: the aforementioned compound (1f) and a boric acid derivative (21-i or 21-ii) are subjected to coupling reaction using a metal catalyst, to thereby yield a compound (1g); and subsequently the compound (1g) is subjected to the reduction described in the method for producing the compound (la-i) in [production method A-1].

Examples of the aforementioned coupling reaction include the method by Wang, W., et al. (Tetrahedron 2002, 58, 3101-3110) and the method by Lebouvier, N., et al. (Tetrahedron Lett., 2006, 47, 6479-6483).

The compound (1f) may be a commercially available one, or may be produced based on the method for producing the compound (la-i) shown in [production method A-1] or the method for producing the compound (1e) shown in [production method A-4].

The compounds (21-i and 21-ii) may be a commercially available one, or may be produced based on, for example, the method by Murata, M., et al. (Synlett, 2006, 1867-1870) or the method by Moleele, S., et al. (Tetrahedron, 2006, 62, 2831-2844).

Next will be described [production method B], which is a method for producing a compound (4) or a salt (e.g., hydrochloride) thereof by deprotecting the protective group $P^1$ of a compound (3) produced through any of the aforementioned [production method A-1] to [production method A-5].

[F38]

[Production method B]

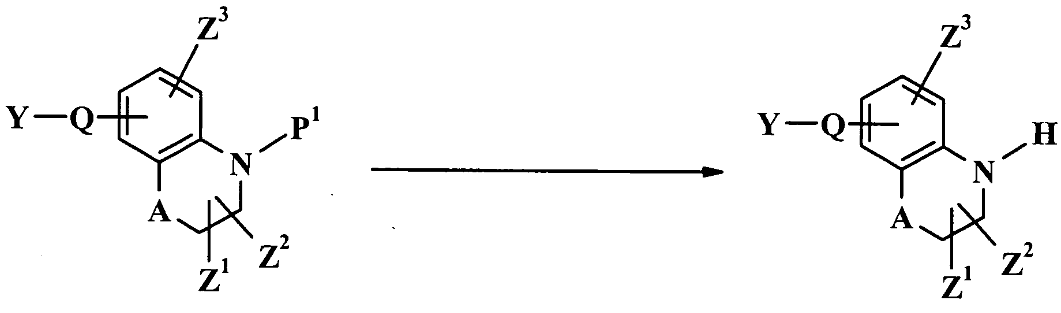

(3) (4)

In the above scheme, Q represents any of the aforementioned groups; and A, $P^1$, Q, Y, $Z^1$, $Z^2$, and $Z^3$ have the same meanings as defined above.

When the protective group $P^1$ of the compound (3) is a tert-butoxycarbonyl group, deprotection may be carried out by causing an inorganic acid such as hydrochloric acid or sulfuric acid, or trifluoroacetic acid to act on the protective group. Examples of preferred reaction solvents include halogenated hydrocarbon solvents such as dichloromethane and chloroform; ether solvents such as tetrahydrofuran and 1,4-dioxane; ester solvents such as ethyl acetate; aprotic polar solvents such as acetonitrile; and alcohol solvents such as methanol and ethanol. The reaction temperature is 0°C to the boiling point of a solvent used, preferably room temperature to 80°C. The reaction time is generally about 1 hour to about 24 hours.

The thus-obtained compound (4) is in the form of a salt corresponding to the acid used in the aforementioned reaction. After completion of the reaction, the resultant reaction mixture may be maintained under weakly basic conditions by use of an alkali metal bicarbonate (e.g., sodium bicarbonate), an alkali metal carbonate (e.g., sodium carbonate), or an alkali metal hydroxide (e.g., sodium hydroxide), to thereby yield a compound (4) which is in the form of free amine.

When the protective group $P^1$ of the compound (3) is a protective group other than the aforementioned one, deprotection may be carried out based on, for example, Protective Groups in Organic Synthesis (T. W. Green and P. G. Wuts, John Wiley & Sons, Inc., New York, 1991).

Next will be specifically described [production method C-1] to [production method C-3], which correspond to the methods a and b shown in [production method 1] for producing an ester form (Ia) from the compound (4).

A compound (Ia-1), which is a compound (Ia) in which V is -$G^1$-, may be produced through the following method (method a in [production method 1]).

[F39]

[Production method C-1]

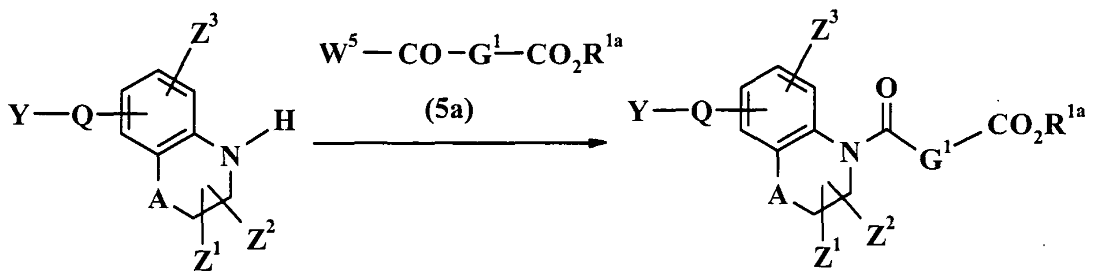


(4)

(Ia-1)

In the above scheme, Q represents any of the aforementioned groups; $W^5$ represents a hydroxyl group or a leaving group; Q represents any of the aforementioned groups; and A, $G^1$, $R^{1a}$, Y, $Z^1$, $Z^2$, and $Z^3$ have the same meanings as defined above.
The compound (Ia-1) may be produced through reaction between the compound (4) or a salt (e.g., hydrochloride) thereof and a compound (5a) for formation of an amide bond.

(1) In the case where $W^5$ is a hydroxyl group

The compound (Ia-1) may be produced through condensation between a commercially available compound (5a) and the compound (4) or a salt (e.g., hydrochloride) thereof by use of a condensing agent which is commonly used for peptide production. Examples of such a common condensing agent include DCC (N,N-dicyclohexylcarbodiimide), N,N-carbonyldiimidazole, DCC/HOBt (1-hydroxybenzotriazole), and water-soluble carbodiimide (e.g., 1-ethyl-3-(3-dimethylaminopropyl)carbodiimide hydrochloride). Preferred are, for example, 1-ethyl-3-(3-dimethylaminopropyl)carbodiimide hydrochloride and N,N-dicyclohexylcarbodiimide. Examples of reaction solvents used in the invention include halogenated hydrocarbon solvents such as methylene chloride; hydrocarbon solvents such as toluene; ether solvents such as tetrahydrofuran; and aprotic polar solvents such as N,N-dimethylformamide. Preferred are, for example, methylene chloride, tetrahydrofuran, and N,N-dimethylformamide. The reaction temperature is -20°C to the boiling point of a solvent used, preferably 0°C to room temperature. The reaction time is generally about 1 to about 24 hours.
Preferably, the aforementioned condensation reaction employs an organic amine base such as triethylamine, diisopropylethylamine, N-methylmorpholine, pyridine, or 4-(N,N-dimethylamino)pyridine in an amount of 1 to 30 equivalents. Particularly when a salt of the compound (4) is used, any of the aforementioned bases must be used in a stoichiometrically equivalent amount or more, so as to neutralize the salt. Preferably, an active esterification reagent such as 1-hydroxybenzotriazole is used in an amount of 0.2 to 1.5 equivalents to the carboxylic acid derivative (5a) .
(2) In the case where $W^5$ is a leaving group
The compound (Ia-1) may be produced through reaction, in the presence of a base, between the compound (4) or a salt (e.g., hydrochloride) thereof, and a compound obtained by converting the carboxyl group, into an acid chloride, acid azide, or similar moiety, of a commercially available carboxylic acid derivative (5a) or the carboxylic acid derivative (5a) described above in (1) through a known method. The base may be an organic amine base such as triethylamine, diisopropylethylamine, N-methylmorpholine, pyridine, or 4-(N,N-dimethylamino)pyridine. The base is used in an amount of 1 to 30 equivalents, preferably 1 to 10 equivalents. Particularly when a salt of the compound (4) is used, any of the aforementioned bases must be used in a stoichiometrically equivalent amount or more, so as to neutralize the salt. Examples of usable reaction solvents include halogenated hydrocarbon solvents such as methylene chloride; hydrocarbon solvents such as toluene; or ether solvents such as tetrahydrofuran. The reaction temperature is -20°C to the boiling point of a solvent used, preferably 0°C to room temperature. The reaction time is generally about 1 to about 24 hours.
Examples of the reference for the condensation reaction described above in (1) and (2) include "Peptide Synthesis" authored by Bodanszky, M., Klausner, Y. S., and Ondetti, A. (A Wiley-interscience publication, New York, 1976); "Synthetic Peptide" authored by Pettit, G. (Elsevier Scientific Publication Company, New York, 1976); and "Jikken Kagaku Koza, 4th Edition, Vol. 22, Organic Synthesis IV" edited by The Chemical Society of Japan (Maruzen Co., Ltd., 1991).

A compound (Ia-2), which is a compound (Ia) in which V is $-G^2-N(R^2)-G^3-$, may be produced through the following method (method b in [production method 1]).

[F40]

[Production method C-2]

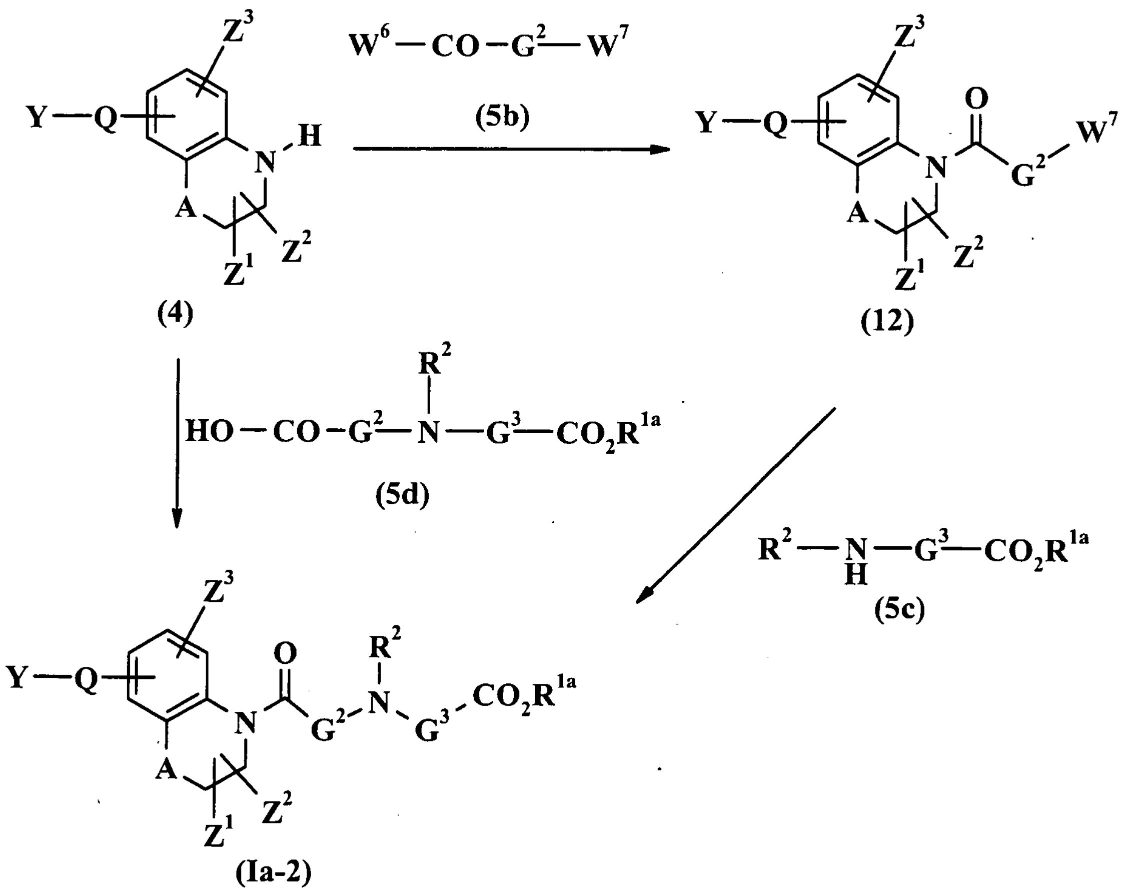
(4)
(5b)
(12)
(5d)
(5c)
(Ia-2)

In the above scheme, Q represents any of the aforementioned groups; each of $W^6$ and $W^7$ independently represents a chlorine atom, a bromine atom, or an iodine atom; and A, $G^2$, $G^3$, $R^{1a}$, $R^2$, Y, $Z^1$, $Z^2$, and $Z^3$ have the same meanings as defined above.

The compound (Ia-2) may be produced through the following procedure: the compound (4) is amidated with a commercially available compound (5b) in the presence of a base, to thereby yield a compound (12); and subsequently the compound (12) is condensed with a commercially available compound (5c) or a salt (e.g., hydrochloride) thereof in the presence of a base.

The aforementioned amidation reaction may be carried out based on the method described above in (2) of [production method C-1]. The base used in condensation between the compound (12) and the compound (5c) or a salt (e.g., hydrochloride) thereof may be an organic amine base such as triethylamine, diisopropylethylamine, N-methylmorpholine, pyridine, or 4-(N,N-dimethylamino)pyridine; or an inorganic base such as potassium carbonate or cesium carbonate. The base is used in an amount of 1 to 30 equivalents, preferably 1 to 10 equivalents. Particularly when a salt of the compound (5c) is used, any of the aforementioned bases must be used in a stoichiometrically equivalent amount or more, so as to neutralize the salt. Examples of usable reaction solvents include halogenated hydrocarbon solvents such as methylene chloride; hydrocarbon solvents such as toluene; ether solvents such as tetrahydrofuran; and aprotic polar solvents such as acetonitrile and N,N-dimethylformamide. The reaction temperature is -20°C to the boiling point of a solvent used, preferably room temperature to 80°C. The reaction time is generally about 1 to about 48 hours. This condensation reaction may be carried out according to, for example, the method by Zhao, H., et al. (Bioorg. Med. Chem.

Lett. 2002, 12, 3105-3109); the method by Jiang, X. -H., et al. (Tetrahedron, 2005, 61, 1281-1288); the method by Nam, J., et al. (Tetrahedron Lett. 2003, 44, 7727-7730); or the method by Hayashi, K., et al. (J. Med. Chem. 1989, 32, 289-297). When $G^2$ is a single bond, the compound (Ia-2) may be produced by sequentially carrying out the aforementioned amidation reaction and condensation reaction in a single container, thereby forming an amide bond (a urea bond in this case). Even when $G^2$ is a $C_1$-$C_3$ straight-chain alkylene group, the compound (Ia-2) may be produced in a similar manner. The aforementioned urea bond formation reaction may be carried out according to, for example, the method by Bermudez, J., et al. (J. Med. Chem. 1990, 33, 1932-1935).

The compound (Ia-2) may also be produced through formation of an amide bond between the compound (4) and the compound (5d) based on the method described above in (1) of [production method C-1].

The aforementioned compound (5d) may be produced through, for example, the following method.
The compound (5d) may be produced through the following procedure: commercially available compounds (12) and (5c) are reacted based on the method described in [production method C-2] for producing the compound. (Ia-2) through reaction between the compound (12) and the compound (5c), to thereby yield a compound (13); and subsequently the benzyl group of the compound (13) is deprotected through catalytic reduction. This catalytic reduction may be carried out through the catalytic reduction method shown in [production method A-3].

[F41]

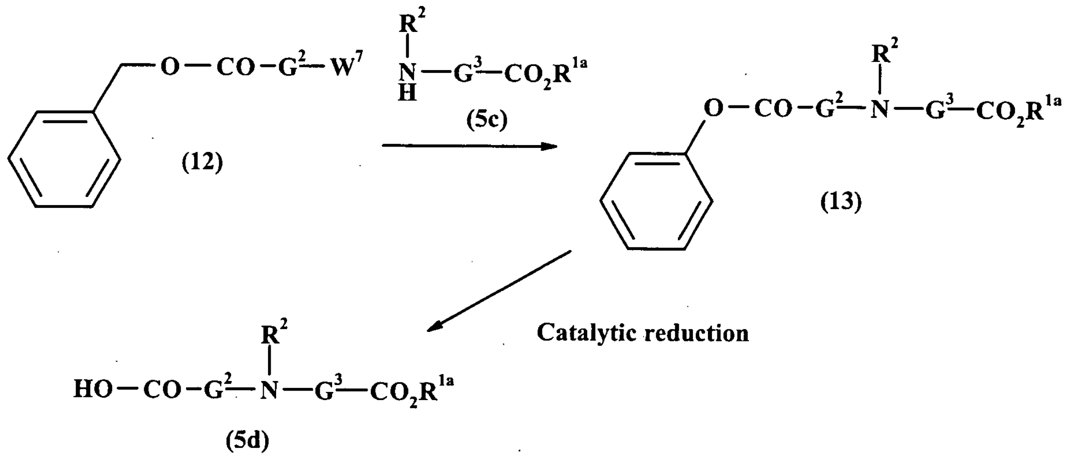


In the above scheme, $W^7$, $G^2$, $G^3$, $R^{1a}$, and $R^2$ have the same meanings as defined above.

A compound (Ia-3), which is a compound (Ia) in which V is the group (5) shown in [production method 1], may be produced through the following method (method b in [production method 1]).

[F42]

[Production method C-3]

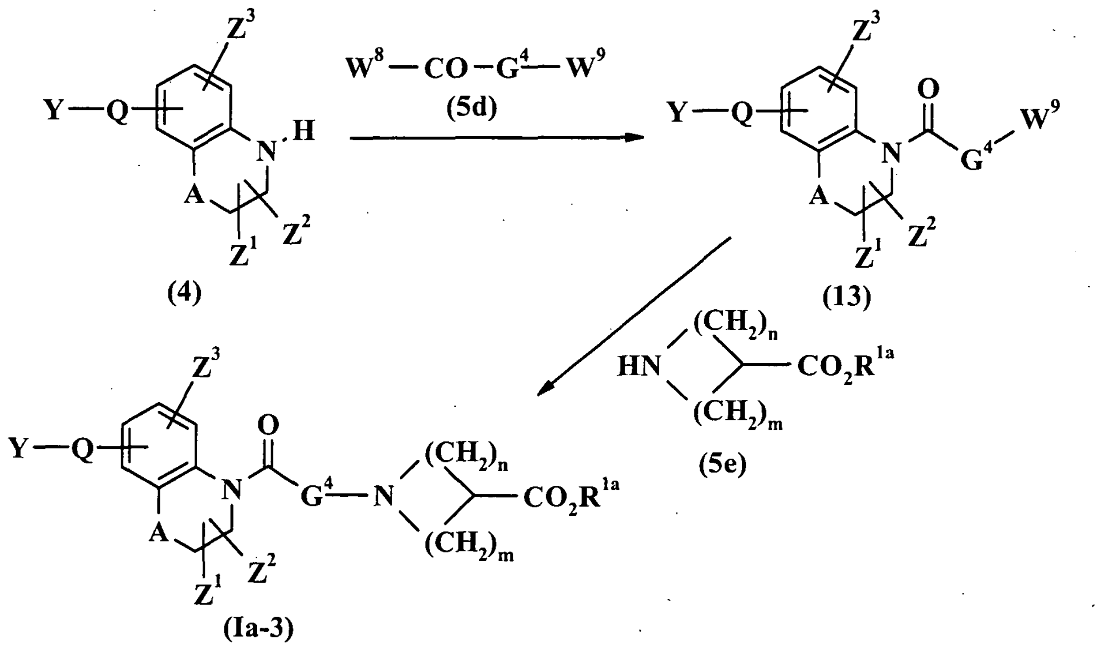

In the above scheme, Q represents any of the aforementioned groups; each of $W^8$ and $W^9$ represents a chlorine atom, a bromine atom, or an iodine atom; and A, $G^4$, m, n, $R^{1a}$, Y, $Z^1$, $Z^2$, and $Z^3$ have the same meanings as defined above. The compound (Ia-3) may be produced by carrying out amidation reaction between the compound (4) and a commercially available compound (5d) based on the method described in [production method C-2] for producing the compound (Ia-2), followed by condensation reaction between the compound (13) and a commercially available compound (5e).

In another synthesis method for the compound (I) of the present invention, a compound (Ib-1); i.e., a carboxylic acid derivative (Ib) described in [production method 1] in which V is optionally substituted $-CH_2-CH_2-$, optionally substituted $-CH_2-CH_2-CH_2-$, or optionally substituted $-CH_2-CH_2-CH_2-CH_2-$, may be produced through reaction between the compound (4) and a cyclic acid anhydride (5f) without forming the ester form (Ia).

[F43]

[Production method D]

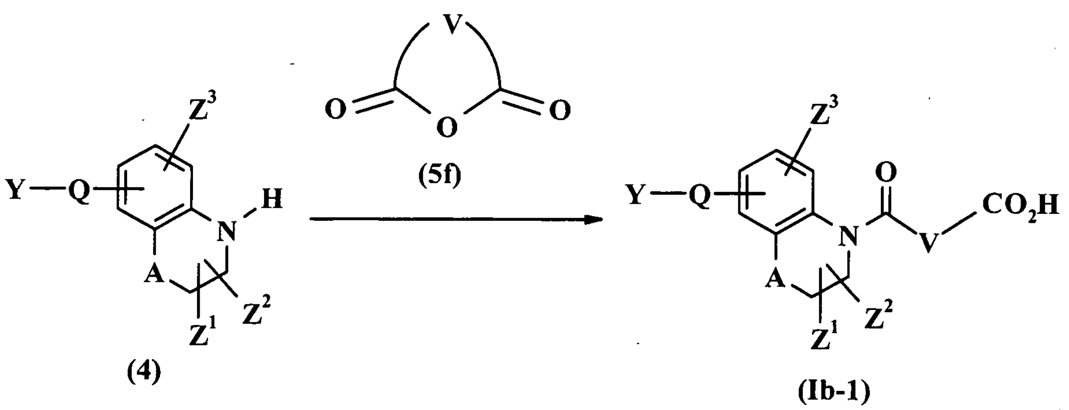

In the above scheme, V represents optionally substituted $-CH_2-CH_2-$, optionally substituted $-CH_2-CH_2-CH_2-$,

or optionally substituted $-CH_2-CH_2-CH_2-CH_2-$; Q represents any of the aforementioned groups; and A, Y, $Z^1$, $Z^2$, and $Z^3$ have the same meanings as defined above.

The compound (Ib-1) may be produced through reaction between the compound (4) or a salt (e.g., hydrochloride) thereof and the cyclic acid anhydride (5f) for formation of an amide bond. Examples of usable reaction solvents include halogenated hydrocarbon solvents such as methylene chloride; hydrocarbon solvents such as toluene; ether solvents such as tetrahydrofuran; and aprotic polar solvents such as acetonitrile and N,N-dimethylformamide. The reaction temperature is -20°C to the boiling point of a solvent used, preferably 0°C to the boiling point of the solvent. The reaction time is generally about 1 to about 24 hours.

This reaction may be carried out in the presence of a base, and, in this case, the base is preferably an organic amine base such as triethylamine, diisopropylethylamine, N-methylmorpholine, pyridine, or 4-(N,N-dimethylamino)pyridine, or an alkali metal carbonate such as potassium carbonate or cesium carbonate. The base is used in an amount of 1 to 30 equivalents, preferably 1 to 10 equivalents. Particularly when a salt (e.g., hydrochloride) of the compound (4) is used, preferably, any of the aforementioned bases is used in a stoichiometrically equivalent amount or more, so as to neutralize the salt. When a mineral acid such as hydrochloric acid is added after completion of reaction, so as to attain weakly acidic conditions, the compound (Ib-1) can be obtained in the form of free carboxylic acid.

The aforementioned reaction may be carried out according to any of the following methods (q) to (u):

(q) the method by Rajashekhar, B., et al. (J. Org. Chem. 1985, 50, 5480-5484);
(r) the method by Kubo, Y., et al. (J. Org. Chem. 1985, 50, 5485-5487);
(s) the method by Sun, W. S., et al. (J. Med. Chem. 2003, 46, 5619-5627);
(t) the method by Lherbet, C., et al. (Bioorg. Med. Chem. Lett. 2003, 13, 997-1000); and
(u) the method by Shultes, C. M., et al. (Bioorg. Med. Chem. Lett. 2004, 14, 4347-4351).

The cyclic acid anhydride (5f) described in [production method D] may be a commercially available one, or may be produced through the method described in the aforementioned reference (q) or (r), or a method similar thereto.

[F44]

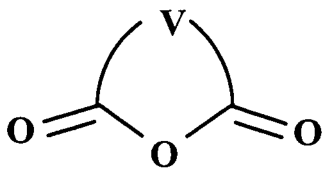

(5f)

Among the compounds used in the aforementioned production methods, the compounds (2a-1), (2a-2), (2b-1), (2b-2), and (6b) include novel compounds, which are useful as intermediates for the production of various compounds. Of these compounds, an alcohol form of the compound (2b-1), a halide of the compound (2b-2) ($W^{1a}$ = halogen), and the compound (6b) are particularly useful.

The compound (I) of the present invention produced through any of the aforementioned methods may be isolated and purified through a known technique such as extraction, precipitation, fractionation, chromatography, fractional recrystallization, or recrystallization.

When having an asymmetric carbon atom, the compound (I) of the present invention has optical isomers. These optical isomers may be isolated from one another and purified through a customary technique such as fractional recrystallization with an appropriate salt (salt separation) or column chromatography.

As described above, S1P receptor agonists are useful as immunosuppressors. The compound of the present invention represented by formula (I), a salt thereof, or a solvate thereof has an agonistic effect on an S1P receptor (in particular, S1P1 receptor), and thus is a useful effective ingredient for an immunosuppressor, and also for a therapeutic and/or preventive agent for, for example, rejection upon transplantation, an autoimmune disease, and an allergic disease of mammals (in particular, human). The compound of the present invention, a salt thereof, or a solvate thereof reduces, through oral administration, lymphocytes in peripheral blood of mice in vivo models, and therefore could be used as an effective ingredient of a medicament (e.g., an immunosuppressor) which can be orally administered. Such a medicament provides less adverse side effects such as bradycardia, which are observed when other S1P receptor agonists are used.

As used herein, "rejection upon transplantation" refers to acute rejection which occurs within three months after transplantation of a graft (e.g., liver, kidney, heart, lung, small intestine, skin, cornea, bone, fetal tissue, bone marrow cells, hematopoietic stem cells, peripheral blood stem cells, cord blood stem cells, pancreatic islet cells, liver cells, nerve cells, or intestinal epithelial cells); chronic rejection which occurs three months or more after such transplantation; and graft-versus-host disease.

Examples of the autoimmune disease include collagen disease, systemic lupus erythematosus, rheumatoid arthritis, multiple sclerosis, nephrotic syndrome, lupus nephritis, Sjogren's syndrome, scleroderma, polymyositis, psoriasis, inflammatory bowel disease, Crohn's disease, mixed connective tissue disease, primary myxedema, Addison's disease, aplastic anemia, autoimmune hemolytic anemia, autoimmune thrombocytopenia, autoimmune diabetes, uveitis, antireceptor disease, myasthenia gravis, thyrotoxicosis, thyroiditis, and Hashimoto's disease.

Examples of the allergic disease include atopic dermatitis, asthma, rhinitis, conjunctivitis, and pollinosis.

The compound of the present invention represented by formula (I), a salt thereof, or a solvate thereof may be administered to a mammal (in particular, human) systemically or locally via an oral or parenteral route.

The medicament of the present invention may be prepared in any dosage form suitable for the administration way through a commonly used medicament preparation method.

Examples of oral medicament forms include tablet, pill, powder, granule, capsule, liquid, suspension, emulsion, syrup, and elixir. Such a medicament form may be prepared through a customary method by use of an optional additive(s) appropriately selected from among an excipient, a binder, a disintegrant, a lubricant, a swelling agent, a swelling aid, a coating agent, a plasticizer, a stabilizer, an antiseptic, an antioxidant, a colorant, a dissolution aid, a suspending agent, an emulsifier, a sweetening agent, a preservative, a buffer, a diluent, a humectant, and the like, which are commonly used as additives.

Examples of parenteral medicament forms include injection, ointment, gel, cream, plaster, patch, aerosol, inhalant, spray, eye drop, nasal drop, suppository, and inhalant. Such a medicament form may be prepared through a customary method by use of an optional additive(s) appropriately selected from among a stabilizer, an antiseptic, a dissolution aid, a moisturizer, a preservative, an antioxidant, an aromatizing agent, a gelatinizing agent, a neutralizer, a dissolution aid, a buffer, an isotonizing agent, a surfactant, a colorant, a buffering agent, a thickener, a humectant, a filler, an absorption promoter, a suspending agent, a binder, and the like, which are commonly used as additives.

The medicament of the present invention may be a medicament containing the compound of formula (I), a salt thereof, or a solvate thereof, in combination with one or more species selected from among an immunosuppressor, an antibody useful for immunosuppression, a rejection-treating agent, an antibiotic, and a steroidal agent. This medicament is administered in the form of a combination medicament containing the compound of the present invention represented by formula (I), a salt thereof, or a solvate thereof and containing one or more of other agents. The combination with the compound of the present invention represented by formula (I), a salt thereof and the pharmaceutical agent may be prepared as a mixture containing both of these ingredients in a formulation, or may be administered in the form of separate formulations. When administered separately, the formulations may be administered at the same time or at different times. The method for administrating these formulations may be same or different. These medicament may be provided in the form of a kit containing the compound of formula (I), a salt thereof, or a solvate thereof, in combination with other agents; for example, one or more species selected from among an immunosuppressor, an antibody useful for immunosuppression, a rejection-treating agent, an antibiotic, and a steroidal agent.

Specific examples of the immunosuppressor, the antibody useful for immunosuppression, and the rejection-treating agent include cyclosporin A, tacrolimus (FK506), azathioprine, mizoribine, methotrexate, mycophenolate mofetil, cyclophosphamide, sirolimus, everolimus, prednisolone, methylprednisolone, orthoclone OKT3, anti-human lymphocyte globulin, and deoxyspergualin.

Specific examples of the antibiotic include cefuroxime sodium, meropenem trihydrate, netilmicin sulfate, sisomicin sulfate, ceftibuten, PA-1806, IB-367, tobramycin, PA-1420, doxorubicin, astromicin sulfate, and cefetamet pivoxil hydrochloride.

Specific examples of the steroidal agent include clobetasol propionate, diflorasone acetate, fluocinonide, mometasone furoate, betamethasone dipropionate, betamethasone butyrate propionate, betamethasone valerate, difluprednate, budesonide, diflucortolone valerate, amcinonide, halcinonide, dexamethasone, dexamethasone propionate, dexamethasone valerate, dexamethasone acetate, hydrocortisone acetate, hydrocortisone butyrate, hydrocortisone butyrate propionate, deprodone propionate, prednisolone valerate acetate, fluocinolone acetonide, beclomethasone propionate, triamcinolone acetonide, flumethasone pivalate, alclometasone propionate, clobetasone butyrate, prednisolone, beclomethasone propionate, fludroxycortide, cortisone acetate, hydrocortisone, hydrocortisone sodium phosphate, hydrocortisone sodium succinate, fludrocortisone acetate, prednisolone acetate, prednisolone sodium succinate, prednisolone butylacetate, prednisolone sodium phosphate, halopredone acetate, methylprednisolone, methylprednisolone acetate, methylprednisolone sodium succinate, triamcinolone, triamcinolone acetate, dexamethasone sodium phosphate, dexamethasone palmitate, paramethasone acetate, betamethasone, fluticasone propionate, flunisolide, ST-126P, ciclesonide, dexamethasone palomithionate, mometasone furancarbonate, prasterone sulfonate, deflazacort, methylprednisolone suleptanate, and methylprednisolone sodium succinate.

The dose of the compound of the present invention represented by formula (I), a salt thereof, or a solvate thereof may vary depending on, for example, the symptom, age, or body weight of a subject in need thereof, or the type or dose of a pharmaceutical agent which is administered in combination therewith. Generally, the dose of the compound

(I) for an adult is 0.001 mg to 1,000 mg per administration. Preferably, the compound (I) is systemically or locally administered via an oral or parenteral route once to several times a day, or continuously administered intravenously for 1 to 24 hours a day.

Examples

The present invention will next be described in more detail by way of example, which should not be construed as limiting the invention thereto.

In the Examples, "IR," "NMR," and "MS" denote infrared absorption spectrum, nuclear magnetic resonance spectrum, and mass analysis, respectively.

"IR" was measured by means of a Hitachi 270-30 spectrometer or Horiba FT-720 (S. T. Japan Durascope (Diamond/KRS-5)) through the ATR method or the KBr tablet method.

Element analysis was performed by means of Perkin-Elmer CHNS/O 2400II.

The following mass analyzers were used in the Examples: JEOL JMS-AX505W (EI, CI), JEOL JMS-HX110 (FD, FAB) spectrometer, Thermoquest Finning AQA (ESI), Agilent Technologies Agilent 1100 series LC/MSD, and PE SCIEX API150EX (ESI) or JMS-T100LP AccuTOF LC-plus.

Unless otherwise specified, "NMR" refers to "$^1$H-NMR." Measurement was performed by means of JEOL JNM-EX400. The species enclosed by parentheses is a solvent for measurement, and TMS (tetramethysilane) was used as an internal standard. Multiplicity in $^1$H-NMR was denoted by the following codes: s = singlet, d = doublet, t = triplet, q = quintet, m = multiplet, and br s = broad singlet. "Anal. Calcd for (molecular formula)" refers to calculated element analysis values, whereas "Found" refers to measured element analysis values.

In column chromatography, a silica gel "Kiesel-gel 60" (product of E-Merck, particle size: 0.060 to 0.200 mm or 0.040 to 0.063 mm) was used. In thin-layer chromatography (TLC), a plate "Kieselgel 60 $F_{254}$" (product of E-Merck) was used.

In the present specification, the following abbreviations are used.

Asp: aspartic acid
$BH_3$·THF: borane-ditetrahydrofuran complex
Boc or boc: tert-butoxycarbonyl
$Boc_2O$: di-tert-butyl dicarbonate
Bn: benzyl
$CDCl_3$: heavy chloroform
DEAD: diethyl azodicarboxylate.
DIEA: diisopropylethylamine
DMAP: 4-(N,N-dimethylamino)pyridine
DMF: N,N-dimethylformamide
DMSO: dimethyl sulfoxide
EDC: 1-ethyl-3-(3-dimethylaminopropyl)carbodiimide hydrochloride
HOAt: 1-hydroxy-7-azabenzotriazole
HOBt: 1-hydroxybenzotriazole
Ms: methanesulfonyl
Pd/C: palladium/carbon
tBu: tert-butyl
TEA: triethylamine
THF: tetrahydrofuran

[Example 1]

4-Oxo-4-[5-[(4-phenyl-5-trifluoromethyl-2-thienyl)methoxy]indolin-1-yl]butyric acid

(1) 1-tert-Butoxycarbonyl-5-hydroxyindoline

[F45]

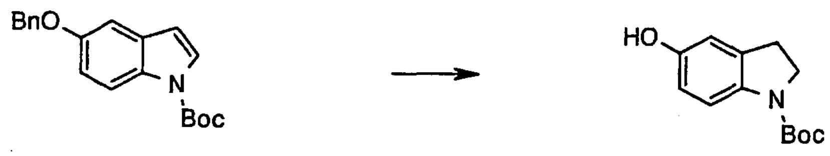

5-Benzyloxy-1-tert-butoxycarbonyl-1H-indole (3.14 g) was dissolved in 10% THF/methanol (80 mL), and 10% palladium hydroxide/carbon (50 mg) was added to the solution at room temperature. The mixture was stirred for 30 minutes under a stream of hydrogen at ambient pressure. The reaction mixture was filtered through a Celite pad, and the catalyst was washed with methanol. The filtrate was concentrated under reduced pressure, and ethyl acetate (50 mL) was added to the residue. The precipitated solid was collected by filtration, whereby the title compound (1.85 g) was yielded.
NMR (DMSO-$d_6$) δ:
1.47(9H,s),2.95(2H,t,J=8.4Hz),3.83(2H,t,J=8.5Hz)6.51(1H,d,J=8 5Hz),6.60(1H,s),7.53-7.22(1H,m),9.00(1H,s).

(2) 4-Phenyl-5-trifluoromethylthiophene-2-methanol

[F46]

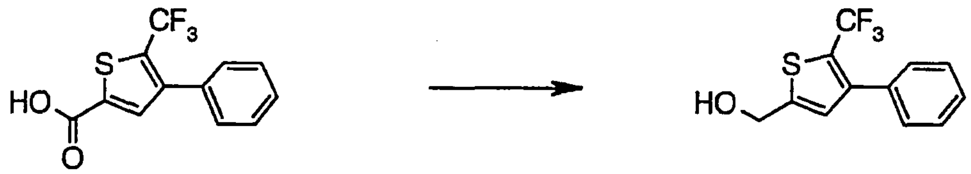

4-Phenyl-5-trifluoromethylthiophene-2-carboxylic acid (3.52 g) was dissolved in THF (60 mL), and a 1M THF solution (26 mL) of $BH_3$·THF complex was added dropwise to the solution at room temperature. The reaction mixture was refluxed for 3 hours and then cooled to 0°C. Through addition of water to the reaction mixture, the reaction was terminated. The reaction mixture was extracted with ethyl acetate, and the extract was washed with saturated brine, followed by drying over sodium sulfate anhydrate, whereby the title compound (3.42 g) was yielded.
NMR ($CDCl_3$) δ :
1.99(1H,t,J=6.0Hz),4.87(2H,d,J=5.6Hz),6.98-7.00(1H,m),7.37-7.42(5H,m).
(3) 5-Chloromethyl-3-phenyl-2-trifluoromethylthiophene
[F47]

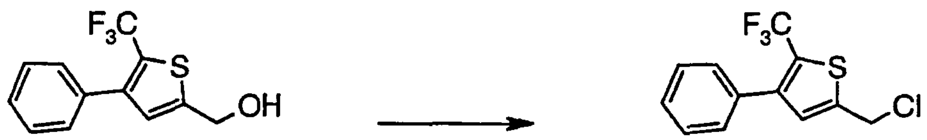

To a solution (7.0 mL) of 4-phenyl-5-trifluoromethylthiophene-2-methanol (482 mg) in dichloromethane, thionyl chloride (708 μL) was added at room temperature, and the mixture was stirred at 50°C for 16 hours. The reaction mixture was cooled to room temperature and concentrated under reduced pressure. The residue was subjected to silica gel flash column chromatography with hexane as an eluent, whereby the title compound (434 mg) was yielded.
NMR($CDCl_3$)δ:
4.77(2H,d,J=0.7Hz),7.07(1H,br s),7.35-7.44(5H,m).

(4) 1-tert-Butoxycarbonyl-5-[(4-phenyl-5-trifluoromethyl-2-thienyl)methoxy]indoline

[F48]

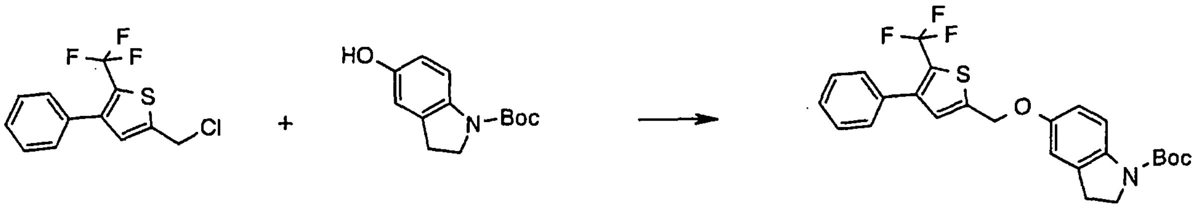

To a solution of 2-chloromethyl-4-phenyl-5-trifluoromethylthiophene (692 mg) and 1-tert-butoxycarbonyl-5-hydroxyindoline (588 mg) in DMF (10 mL), potassium carbonate (691 mg) was added at room temperature, and the mixture was stirred at 60°C for 3.5 hours. The reaction mixture was concentrated under reduced pressure. Ethyl acetate (15 mL) and water (10 mL) were added to the concentrate for phase separation, and the aqueous layer was extracted with ethyl acetate (50 mL). The extract was added to the ethyl acetate layer, and the combined liquid was dried over sodium sulfate anhydrate. Solvent was evaporated under reduced pressure, and the residue was purified by silica gel flash column chromatography (Biotage 40S), whereby the title compound (1.13 g) was yielded.
NMR ($CDCl_3$) δ:
1.60-1.52(9,Hm)3.07(2H,t,J=8.7Hz),3.97(2H,s),5.18(2H,s),6.78(1H,d, J=8.5Hz),6.81(1H,s),7.05(1H,s),7.43-7.39(5H, m),7.77(1H,br s).

(5) 5-[(4-Phenyl-5-trifluoromethyl-2-thienyl)methoxy]indoline hydrochloride

[F49]

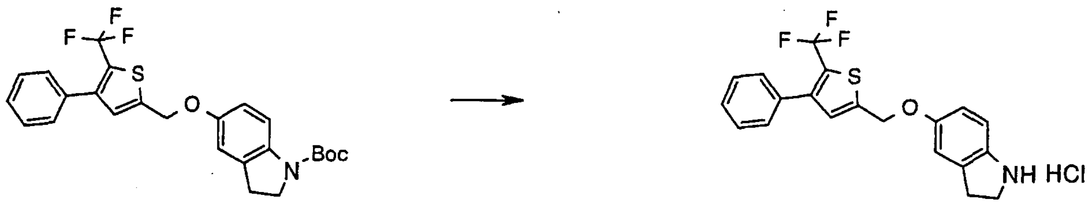

To a solution of 1-tert-butoxycarbonyl-5-[(4-phenyl-5-trifluoromethyl-2-thienyl)methoxy]indoline (1.13 g) in 1,4-dioxane (5.0 mL), 4N HCl/1,4-dioxane (5 mL) was added at room temperature. The mixture was stirred for 14 hours, and solvent was evaporated under reduced pressure. Ethyl acetate (5 mL) was added to the residue, and the precipitated

solid was collected by filtration, followed by washing with diethyl ether and drying, whereby the title compound (895 mg) was yielded.
NMR ($CD_3OD$) δ : 3.33(3H,t,J=7.8Hz), 3.87(2H,t,J=7.7Hz), 5.40(2H,s), 7.10(1H,dd,J =8.7,2.6Hz),7.21-7.19(1H,m),7.23 (1H,br s),7.44-7.40(6H,m).

(6) 4-Oxo-4-[5-[(4-phenyl-5-trifluoromethyl-2-thienyl)methoxy]indolin-1-yl]butyric acid ethyl ester

[F50]

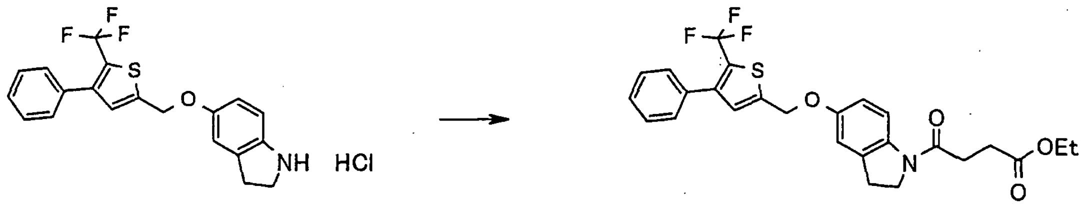

To a suspension of 5-[(4-phenyl-5-trifluoromethyl-2-thienyl)methoxy]indoline hydrochloride (148 mg) in dichloromethane (3 mL), DIEA (188 μL) was added at room temperature, and the mixture was stirred at room temperature for 30 minutes. Subsequently, succinyl monoethylchloride (53.9 μL) was added thereto. The resultant mixture was further stirred for 5 hours. The reaction mixture was concentrated under reduced pressure, whereby the title compound (21 mg) was yielded. This compound was employed in a subsequent reaction without performing isolation or purification.
MS (ESI) m/z : 514 $(M+H)^+$.

(7) 4-Oxo-4-[5-[(4-phenyl-5-trifluoromethyl-2-thienyl)methoxy]indolin-1-yl]butyric acid

[F51]

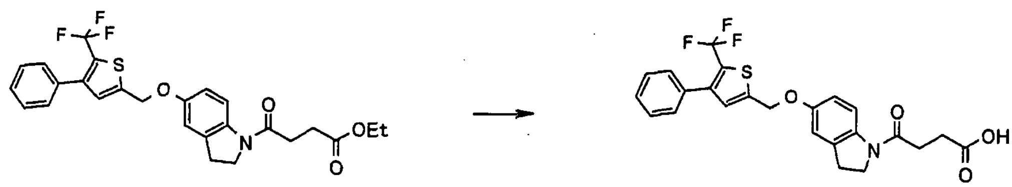

To a solution of 4-oxo-4-[5-[(4-phenyl-5-trifluoromethyl-2-thienyl)methoxy]indolin-1-yl]butyric acid ethyl ester (221 mg) in a 33% methanol/THF mixture (1.5 mL), 1N aqueous sodium hydroxide solution (0.5 mL) was added, and the resultant mixture was stirred at room temperature for 14 hours. Water (2 mL) was added to the reaction mixture, and 1N aqueous hydrochloric acid solution (0.6 mL) was added thereto so as to make the mixture weekly acidic. Subsequently, a 10% methanol/chloroform mixture (3 mL) was further added to extract an organic substance. The suspension of the combined organic layer was concentrated until the volume of the solvent was reduced to half the volume. The precipitated solid was collected by filtration, followed by washing with chloroform and drying under reduced pressure, whereby the title compound (128 mg) was yielded.
NMR(DMSO-$d_6$)δ: 2.64(2H,t,J=6.3Hz),3.13(2H,t,J=8.4Hz),3.29(2H,d,J=9.0Hz),4.09 (2H,t,J=8.5Hz),5.34(2H,s),6.84(1H,dd,J=8.8,2.2Hz),6.97(1H,d,J =2.2Hz),7.35(1H,s),7.50-7.41(5H,m),7.96(1H,d,J=8.8Hz).
MS (EIS)m/z:476 $(M+H)^+$.
Anal. Calcd for
$C_{24}H_{20}O_4NF_3S$:C,60.62;H,4.24;F,11.99;N,2.95;S,6.74. Found:C,60.43 ;H,4.24;F,12.07;N,3.05;S,6.88.

[Example 2]

4-Oxo-4-[7-[(4-phenyl-5-trifluoromethyl-2-thienyl)methoxy]-2,3-dihydrobenzo[1,4]oxazin-4-yl]butyric acid

(1) 4-Oxo-4-(7-hydroxy-2,3-dihydrobenzo[1,4]oxazin-4-yl)butyric acid methyl ester

[F52]

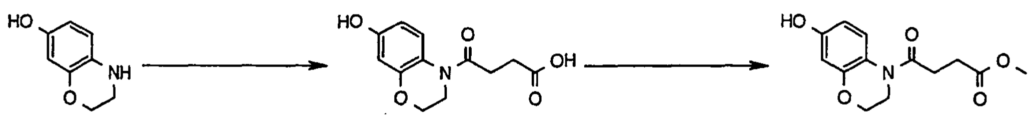

To a THF solution (10 mL) of 3,4-dihydro-2H-benzo[1,4]oxazin-7-ol (Eur. J. Med. Chem., 1999, 34, 903-917) (151 mg), succinic anhydride (200 mg) was added, and the mixture was stirred for 6 hours at room temperature. The reaction mixture was diluted with THF (10 mL), and methanol (405 μL) and EDC·HCl (958 mg) were added thereto, followed by stirring at room temperature for 4 days. To the resultant mixture, 1N aqueous hydrochloric acid solution was added, followed by extracting twice, each with ethyl acetate. The extracts were combined and washed with saturated brine, followed by drying over sodium sulfate anhydrate. Solvent was evaporated under reduced pressure, and the residue was purified by silica gel column chromatography (Yamazen Hi-Flash column L), to thereby yield the title compound (97 mg).
MS(ESI)m/z:266$(M+H)^+$.

(2) 4-Oxo-4-[7-[(4-phenyl-5-trifluoromethyl-2-thienyl)methoxy]-2,3-dihydrobenzo[1,4]oxazin-4-yl]butyric acid methyl ester

[F53]

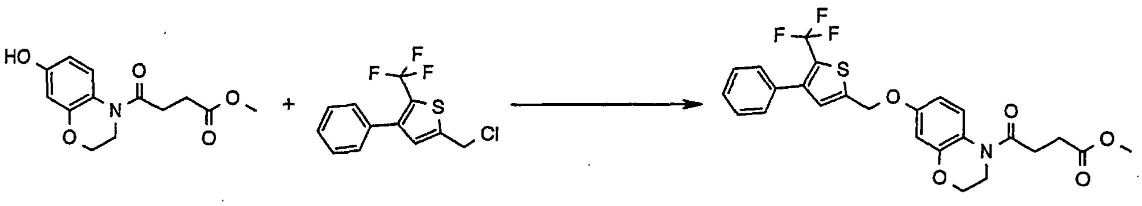

To a DMF solution (5 mL) of 4-oxo-4-(7-hydroxy-2,3-dihydrobenzo[1,4]oxazin-4-yl)butyric acid methyl ester (97 mg), 55% sodium hydride (17 mg) was added under cooling on ice, and the mixture was stirred for 1 hour under cooling on ice. 5-chloromethyl-3-phenyl-2-trifluoromethylthiophene (111 mg) was added thereto, followed by stirring at 55°C overnight. Saturated brine was added thereto, and the resultant mixture was extracted twice, each with ethyl acetate. The extracts were combined and washed with saturated brine, followed by drying over sodium sulfate anhydrate. After filtration, solvent was evaporated under reduced pressure, and the residue was purified by flash column chromatography (Yamazen Hi-Flash column L), to thereby yield the title compound (130 mg).
NMR($CDCl_3$)δ:
2.70-2.86 (4H, m), 3.68 (3H, s), 3.93 (2H, s), 4.29 (2H, t, J=4.3Hz), 5.18 (2H, s ), 6.55-6.57 (2H, m), 7.08 (1H, s), 7.26 (1H, s), 7.43-7.40(5H,m). MS(ESI)m/z:506(M+H)$^+$.

(3) 4-Oxo-4-[7-[(4-phenyl-5-trifluoromethyl-2-thienyl)methoxy]-2,3-dihydrobenzo[1,4]oxazin-4-yl]butyric acid

[F54]

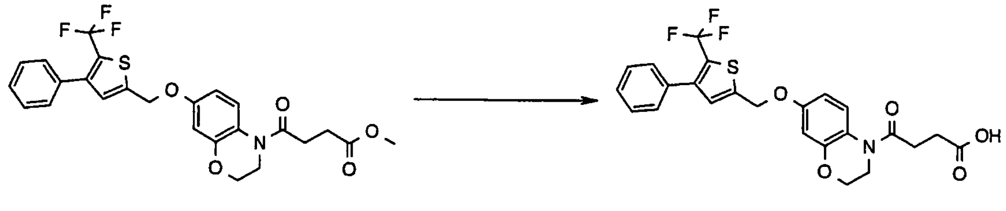

To a THF solution (1 mL) of 4-oxo-4-[7-[(4-phenyl-5-trifluoromethyl-2-thienyl)methoxy]-2,3-dihydrobenzo[1,4]oxazin-4-yl]butyric acid methyl ester (130 mg), methanol (0.50 mL) and 1N aqueous sodium hydroxide solution (0.51 mL) were added, and the mixture was stirred overnight at room temperature. To the reaction mixture, 1N hydrochloric acid was added, followed by extracting twice, each with ethyl acetate. The extracts were combined and washed with saturated brine, followed by drying over sodium sulfate anhydrate. Solvent was evaporated under reduced pressure. The residue was purified by flash column chromatography (Yamazen Hi-Flash column L). The obtained product was freeze-dried by use of water and 1,4-dioxane, to thereby yield the title compound (100 mg).
NMR($CDCl_3$)δ:
2.75-2.86(4H,m),3.94(2H,s),4.27(2H,s),5.18(2H,s),6.55-6.58(2H,m),7.07(2H,s),7.37-7.43(5H,m).
IR(ATR)cm$^{-1}$:3060,1710,1654,1619,1504,1378,1286,1164,1110.
MS(ESI)m/z:492(M+H)$^+$.
HRMS (FAB) calcd for $C_{24}H_{21}F_3NO_5S$(M+H)$^+$:492.1
093;Found 492.1084.
Anal. Calcd for
$C_{24}H_{20}F_3NO_5S\cdot0.5H_2O$:C,57.60 ;H,4.23;F,11.39;N,2.80;S,6.41.Found: C,57.27;H,4.09;F,11.07;N,2.76;S,6.40.

[Example 3]

4-Oxo-4-[6-[(4-phenyl-5-trifluoromethyl-2-thienyl)methoxy]-1,2,3,4-tetrahydroquinolin-1-yl]butyric acid

(1) 4-(6-Hydroxy-1,2,3,4-tetrahydroquinolin-1-yl)-4-oxobutyric acid

[F55]

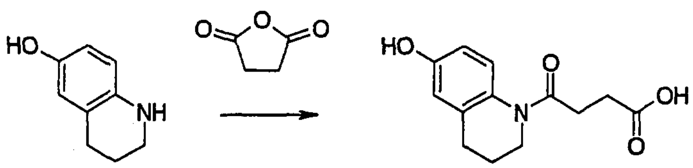

To a THF solution (30 mL) of 6-hydroxy-1,2,3,4-tetrahydroquinoline (2.00 g), succinic anhydride (1.34 g) was added, and the mixture was stirred for 17 hours at room temperature. The reaction mixture was concentrated under reduced pressure, and the residue was dissolved in dichloromethane, followed by extraction with 1N aqueous sodium hydroxide solution. The extracts were combined, and the pH of the combined extract was adjusted to 2 by use of 10% aqueous hydrochloric acid solution, followed by extraction with ethyl acetate. The extract was washed with saturated brine and dried over sodium sulfate anhydrate. Sodium sulfate anhydrate was removed by filtration, and the filtrate was concentrated under reduced pressure, to thereby yield the title compound (2.25 g).
NMR(DMSO-$d_6$)δ:
1.81(2H,t,J=7.1Hz),2.33-2.75(5H,m),3.27-3.40(1H,m),3.62(1H,t,J=6.4Hz),6.577(3H,s),7.12(1H,s),9.29(1H,s ),12.05 (1H,s).
MS(ESI)m/z:250(M+H)$^+$.

(2) 4-(6-Hydroxy-1,2,3,4-tetrahydroquinolin-1-yl)-4-oxobutyric acid methyl ester

[F56]

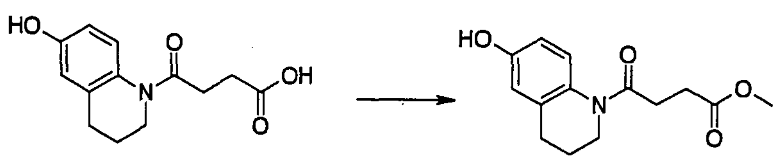

Thionyl chloride (1.0 mL) was added dropwise to methanol (10 mL) at 0°C in a nitrogen atmosphere, and the mixture was stirred for 10 minutes. A methanol solution (20 mL) of 4-(6-hydroxy-1,2,3,4-tetrahydroquinolin-1-yl)-4-oxobutyric acid (540 mg) was added dropwise to the reaction mixture at 0°C over 20 minutes, and the resultant mixture was stirred for 3 days during which the mixture was allowed to warm to room temperature, followed by concentration under reduced pressure. The residue was purified by silica gel flash column chromatography (Biotage 25S), to thereby yield the title compound (275 mg).
NMR($CDCl_3$)δ:
1.86-1.97(2H,m),2.58-
2.82(6H,m),3.65(3H,s),3.74(2H,t,J=6.1Hz),6.58-6.68(2H,m),7.15-6.88(1H,m).
MS(ESI)m/z:264(M+H).

(3) 4-Oxo-4-[6-[(4-phenyl-5-trifluoromethyl-2-thienyl)methoxy]-1,2,3,4-tetrahydroquinolin-1-yl]butyric acid methyl ester

[F57]

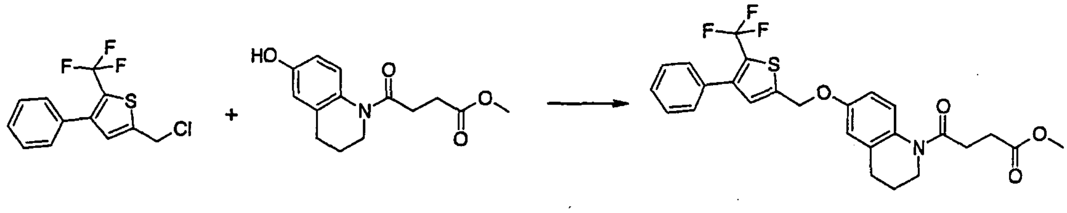

To a DMF solution (5 mL) of 5-chloromethyl-3-phenyl-2-trifluoromethylthiophene (347 mg) and 4-(6-hydroxy-1,2,3,4-tetrahydroquinolin-1-yl)-4-oxobutyric acid methyl ester (275 mg), potassium carbonate (288 mg) was added, and the mixture was heated to 60°C, followed by stirring for 14 hours. The reaction mixture was cooled to room temperature, and ice water was added thereto, followed by extraction with ethyl acetate. The extracts were combined and washed sequentially with ice water and saturated brine, followed by drying over sodium sulfate anhydrate. Sodium sulfate anhydrate was removed by filtration, and the filtrate was concentrated under reduced pressure. The residue was purified by silica gel flash column chromatography (Biotage 25S), to thereby yield the title compound (479 mg).
NMR($CDCl_3$)δ:
1.84-2.04(4H,m),2.50-2.99(6H,m),3.65(3H,s),3.76(1H,t,J=5.4Hz),5.18(2H,s),6.74-6.86(2H,m),7.06(1H,s),7.34-7.43 (5H,m).
MS(ESI)m/z:504(M+H).

(4) 4-Oxo-4-[6-[(4-phenyl-5-trifluoromethyl-2-thienyl)methoxy]-1,2,3,4-tetrahydroquinolin-1-yl]butyric acid

[F58]

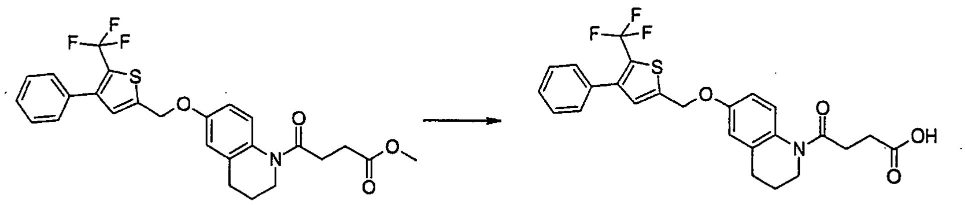

To a solution of 4-oxo-4-[6-[(4-phenyl-5-trifluoromethyl-2-thienyl)methoxy]-1,2,3,4-tetrahydroquinolin-1-yl]butyric acid methyl ester (479 mg) in a methanol/THF (1:2) mixture (6 mL), 1N sodium hydroxide solution (2 mL) was added at room temperature, and the resultant mixture was stirred for 17 hours. The reaction mixture was concentrated under reduced pressure, to thereby yield a residue. The pH of the residue was adjusted to 2 by use of 1N hydrochloric acid, followed by extraction with a 10% methanol/chloroform solution. The extracts were combined and washed with saturated brine, followed by drying over sodium sulfate anhydrate. Sodium sulfate anhydrate was removed by filtration, and the filtrate was concentrated under reduced pressure. The residue was purified by silica gel flash column chromatography (Yamazen Hi-Flash L), to thereby yield the title compound (444 mg).
NMR($CDCl_3$)δ:
1.98(2H,t,J=6.4Hz),2.61-2.87(6H,m),3.81(2H,s),5.22(2H,s),6.80-6.86(3H,m),7.08(1H,s),7.39-7.45(6H,m).
IR(ATR)cm-1:2943,1728,1711,1644,1608,1496,1473.
MS(ESI)m/z:490$(M+H)^+$.
HRMS(FAB)Calcd for $C_{26}H_{23}F_3NO_4S$:490.1300.Found:490.1293.

[Example 4]

5-Oxo-5-[5-[(4-phenyl-5-trifluoromethyl-2-thienyl)methoxy]indolin-1-yl]valeric acid

(1) 5-Oxo-5-[5-[(4-phenyl-5-trifluoromethyl-2-thienyl)methoxy]indolin-1-yl]valeric acid ethyl ester

[F59]

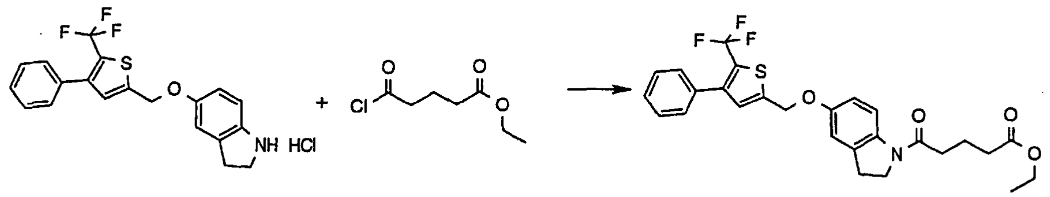

To a solution of 5-(4-phenyl-5-trifluoromethyl-2-thienyl)methoxy]indoline hydrochloride (67.4 mg) in dichloromethane (2.0 mL), DIEA (0.0855 mL) and 4-chloroformyl butyric acid ethyl ester (38.5 mg) were added at room temperature, and the mixture was stirred for 2 hours. Saturated aqueous sodium hydrogencarbonate solution (10 mL) and chloroform (20 mL) were added to the reaction mixture for phase separation, and the aqueous layer was extracted with chloroform (10 mL). The extracts were combined and dried over sodium sulfate anhydrate, and solvent was evaporated under reduced pressure. The residue was purified by silica gel flash column chromatography (Biotage 25M), to thereby yield the title compound (81.8 mg).
NMR($CDCl_3$)δ:
1.27(3H,t,J=7.1Hz)2.02-2.11(2H,m),2.43-2.53(4H,m),3.19(2H,t,J=8.4Hz),4.07(2H,t,J=8.4Hz),4.15(2H,q,J= 7.1Hz), 5.20(2H,s),6.82(1H,dd,J=8.6,2.6Hz),6.84(1H,s),7.07(1H, s),7.35-7.47(5H,m),8.18(1H,d,J=8.6Hz).
MS(ESI)m/z:517 ($M^+$)

(2) 5-Oxo-5-[5-[(4-phenyl-5-trifluoromethyl-2-thienyl)methoxy]indolin-1-yl]valeric acid

[F60]

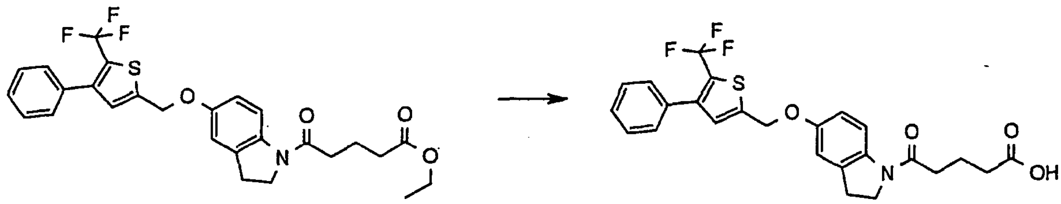

To a solution of 5-oxo-5-[5-[(4-phenyl-5-trifluoromethyl-2-thienyl)methoxy]indolin-1-yl]valeric acid ethyl ester (80.0 mg) in THF (3.0 mL), methanol (1.5 mL) and 1N aqueous sodium hydroxide solution (0.470 mL) were added at room temperature, and the mixture was stirred for 15 hours. To the reaction mixture, 1N hydrochloric acid (0.470 mL) and water (20 mL) were added, and the resultant mixture was concentrated to about 20 mL under reduced pressure. To the formed precipitates, 1N hydrochloric acid (10 mL) and ethyl acetate (20 mL) were added for phase separation, and the aqueous layer was extracted with ethyl acetate (20 mL). The extracts were combined and dried over sodium sulfate anhydrate, and solvent was removed under reduced pressure. The residue was slurried with hexane. The solid matter was collected by filtration, and dried (in vacuum, for 3 hours, 40°C), to thereby yield the title compound (67.3 mg).
NMR($CDCl_3$)δ:
2.04-2.14(2H,m),2.55(4H,t,J=7.0Hz),3.20(2H,t,J=8.4Hz),4.07(2H,t,J= 8.4Hz),5.21(2H,s),6.82(1H,dd,J=8.7,2.7Hz),6.85 (1H,s),7.07(1H, s),7.37-7.46(5H,m),8.18(1H,d,J=8.7Hz).
IR(ATR)$cm^{-1}$:2906,1698,1652,1488,1290,1269,1099,1014,698.
MS(ESI)m/z:490$(M+H)^+$.
Anal. Calcd for
$C_{25}H_{22}F_3NO_4S$:C, 61.34;H,4.53 ;F, 11. 64;N, 2.86;S, 6.55.Found:C, 61.28 ;H,4.52;F,11.28;N,2.90;S,6.61.

[Example 5]

6-Oxo-6-[5-[(4-phenyl-5-trifluoromethyl-2-thienyl)methoxy]indolin-1-yl]hexanoic acid

(1) 6-Oxo-6-[5-[(4-phenyl-5-trifluoromethyl-2-thienyl)methoxy]indolin-1-yl]hexanoic acid ethyl ester

[F61]

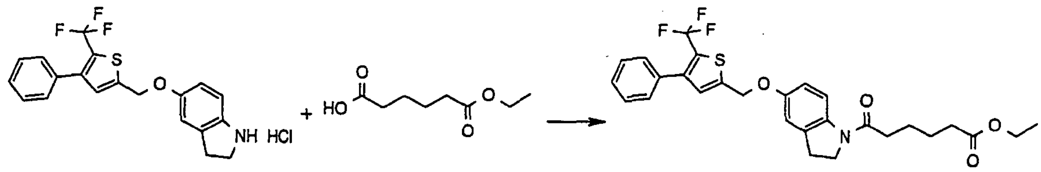

To a suspension containing 5-[(4-phenyl-5-trifluoromethyl-2-thienyl)methoxy]indoline hydrochloride (100 mg) and adipic acid monoethyl ester (46.5 mg) in DMF (3.0 mL), EDC·HCl (58.0 mg), HOBt (41.0 mg), and TEA (0.104 mL) were added at room temperature, and the mixture was stirred for 4 days. The reaction mixture was concentrated under reduced pressure, and ethyl acetate (30 mL), saturated aqueous solution of sodium hydrogencarbonate (20 mL), and water (40 mL) were added to the concentrate for phase separation. The aqueous layer was extracted with chloroform (20 mL). The extracts were combined and dried over sodium sulfate anhydrate, and solvent was removed under reduced pressure. The residue was purified by silica gel flash column chromatography (Biotage 25M), to thereby yield the title compound (116 mg). NMR($CDCl_3$)δ:
1.26(3H,t,J=7.1Hz)1.67-1.73(4H,m),2.37(2H,t,J=6.8Hz),2.44(2H,t,J=6.8Hz),3.19(2H,t,J= 8.4Hz),4.06(2H,t,J=8.4Hz), 4.14(2H,q,J=7.1Hz),5.20(2H,s),6.78-6.86(2H,m),7.07(1H,s),7.36-7.46(5H,m),8.18(1H,d,J=8.8Hz).
MS(ESI)m/z:532($M^+H$).

(2) 6-Oxo-6-[5-[(4-phenyl-5-trifluoromethyl-2-thienyl)methoxy]indolin-1-yl]hexanoic acid

[F62]

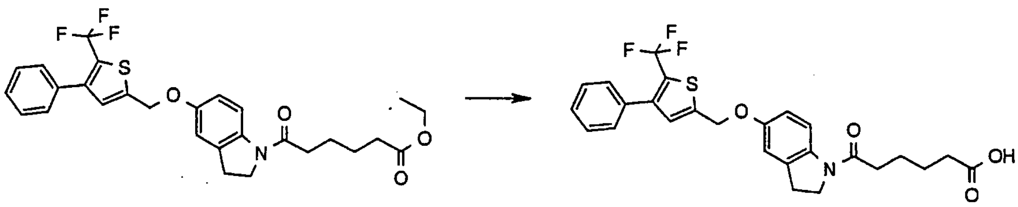

To a solution of 6-oxo-6-[5-[(4-phenyl-5-trifluoromethyl-2-thienyl)methoxy]indolin-1-yl]hexanoic acid ethyl ester (115 mg) in THF (4.0 mL), methanol (2.0 mL) and 1N aqueous sodium hydroxide solution (0.650 mL) were added at room temperature, and the mixture was stirred for 15 hours. To the reaction mixture, 1N hydrochloric acid (0.650 mL) and water (20 mL) were added, and the resultant mixture was concentrated to about 20 mL under reduced pressure. The formed precipitates were collected by filtration and dried (in vacuo, for 3 hours, 40°C), to thereby yield the title compound (103 mg). NMR($CDCl_3$)δ:
1.70-1.90(4H,m),2.40-2.52(4H,m),3.19(2H,t,J=8.4Hz),4.06(2H,t,J=8.4Hz),5.20(2H,s),6 .82(1H,dd,J=8.6,2.6Hz),6.84(1H,s),7.07(1H,s), 7.38-7.46(5H,m),8.18(1H,d,J=8.4Hz). $CO_2H$ not observed.

IR(ATR)$cm^{-1}$:2943,1701,1649,1487,1383,1288,1263,1109,1014,766,698.
MS(ESI)m/z:504$(M+H)^+$
Anal. Calcd for $C_{26}H_{24}F_3NO_4S$:C,62.02;H,4.80;F,11.32;N,2.78;S,6.37.Found:C,61.84 ;H,4.70;F,11.40;N,2.78;S,6.47.

[Example 6]

7-Oxo-7-[5-[(4-phenyl-5-trifluoromethyl-2-thienyl)methoxy]indolin-1-yl]heptanoic acid

(1) 7-Oxo-7-[5-[(4-phenyl-5-trifluoromethyl-2-thienyl)methoxy]indolin-1-yl]heptanoic acid ethyl ester

[F63]

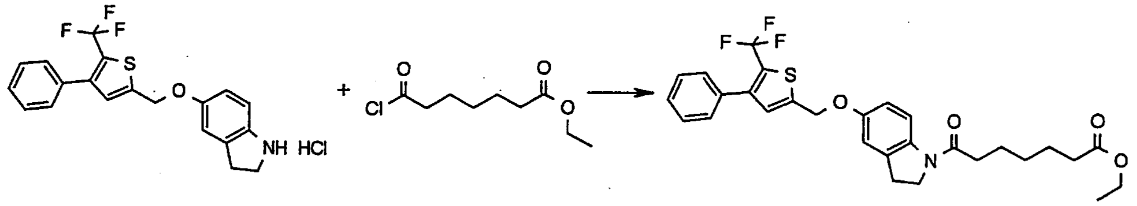

To a solution of 5-(4-phenyl-5-trifluoromethyl-2-thienyl)methoxy]indoline hydrochloride (51.9 mg) in dichloromethane (2.0 mL), DIEA (0.0660 mL) and 6-chloroformylhexanoic acid ethyl ester (36.0 mg) were added at room temperature, and the mixture was stirred for 2 hours. Saturated aqueous solution of sodium hydrogencarbonate (10 mL) and chloroform (20 mL) were added to the reaction mixture for phase separation, and the aqueous layer was extracted with chloroform (10 mL). The extracts were combined and dried over sodium sulfate anhydrate, and solvent was evaporated under reduced pressure. The residue was purified by silica gel flash column chromatography (Biotage 25M), to thereby yield the title compound (68.4 mg). NMR($CDCl_3$)δ: 1.26(3H,t,J=7.2Hz)1.38-1.50(2H,m),1.63-1.80(4H,m),2.32(2H,t,J=7.5Hz),2.41(2H,t,J=7.5Hz),3.18(2H,t,J= 8.4Hz),4.06 (2H,t,J=8.4Hz),4.12(2H,q,J=7.2Hz),5.20(2H,s),6.78-6.86(2H,m),7.06(1H,s),7.36-7.41(5H,m),8.18(1H,d,J=8.8Hz).
MS(ESI)m/z:546$(M+H)^+$

(2) 7-Oxo-7-[5-[(4-phenyl-5-trifluoromethyl-2-thienyl)methoxy]indolin-1-yl]heptanoic acid

[F64]

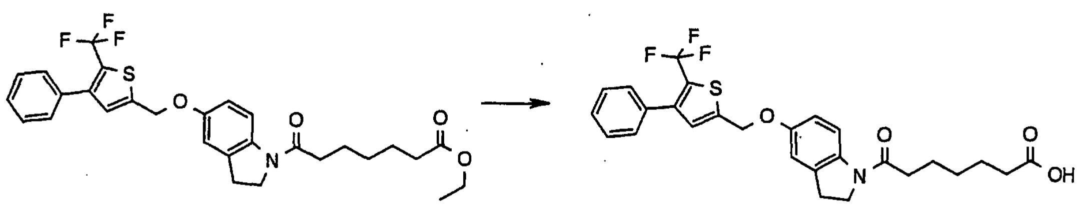

To a solution of 7-oxo-7-[5-[(4-phenyl-5-trifluoromethyl-2-thienyl)methoxy]indolin-1-yl]heptanoic acid ethyl ester (67.0 mg) in THF (3.0 mL), methanol (1.5 mL) and 1N aqueous sodium hydroxide solution (0.370 mL) were added at room temperature, and the mixture was stirred for 20 hours. To the reaction mixture, 1N hydrochloric acid (0.370 mL) and water (20 mL) were added, and the resultant mixture was concentrated to about 20 mL under reduced pressure.

The formed precipitates were collected by filtration and dried (in vacuo, for 3 hours, 40°C), to thereby yield the title compound (56.7 mg). NMR(DMSO-$d_6$)δ:
1.27-1.37(2H,m),1.47-1.61(4H,m),2.19(2H,t,J=7.3Hz),3.39(2H,t,J=7.3Hz),3.10(2H,t,J= 8.4Hz),4.06(2H,t,J=8.4Hz), 5.33(2H,s),6.83(1H,dd,J=8.8,2.3Hz), 6.96(1H,d,J=2.3Hz),7.35(1H,s),7.40-7.51(5H,m),7.99(1H,d,J=8.8Hz). $CO_2H$ not observed.
IR(ATR)$cm^{-1}$:2945,1705,1583,1489,1408,1387,1269,1201,1173,1120,1097,1020 ,768,700.
MS(ESI)m/z:518$(M+H)^+$.
HRMS (ESI) Calcd for $C_{27}H_{27}F_3NO_4S(M+H)^+$: 518.1613.Found:518.1604. Anal. Calcd for $C_{27}H_{26}F_3NO_4S\cdot 0.25H_2O$:C,62.12;H,5.12;F,10.92;N,2.68;S,6.14.Found:C,62.48;H, 5.08;F,11.30;N,2.20;S,6.11.

[Example 7]

(S)-2-Hydroxy-4-oxo-4-[5-[(4-phenyl-5-trifluoromethyl-2-thienyl)methoxy]indolin-1-yl]butyric acid

(1) (S)-2-Hydroxy-4-oxo-4-[5-[(4-phenyl-5-trifluoromethyl-2-thienyl)methoxy]indolin-1-yl]butyric acid methyl ester

[F65]

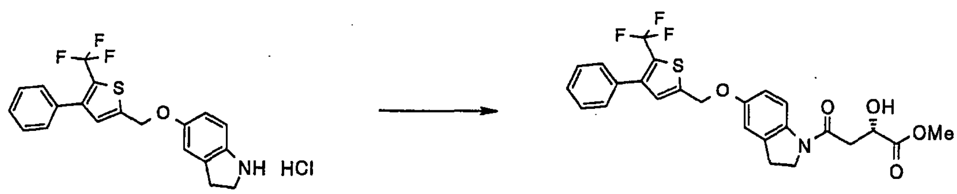

(S)-(2,5-Dioxotetrahydrofuran-3-yl) trifluoroacetate (106 mg) was dissolved in methanol (2 mL), and the mixture was stirred at room temperature for 3.5 hours. The reaction mixture was concentrated, and THF (1 mL) was added to the residue to dissolve the residue. TEA (60 μL), HOBt (67.6 mg) and EDC·HCl (95.9 mg) were added to the solution at room temperature, followed by stirring for 10 minutes. 5-[(4-phenyl-5-trifluoromethyl-2-thienyl)methoxy]indoline hydrochloride (206 mg), TEA (80.0 μL), and DMF (1 mL) were added thereto at room temperature. The reaction mixture was further stirred for 14 hours and concentrated under reduced pressure. Chloroform (3 mL) and water (1.5 mL) were added to the concentrate for phase separation, and the aqueous layer was extracted with chloroform (1 mL × 3). The extracts were combined, and solvent was removed under reduced pressure. DMSO (3 mL) was added to the residue, and insoluble matter was removed. The resultant product was purified by high performance liquid chromatography (NOMURA Develosil Combi-RP-5), to thereby yield the title compound (58.7 mg).
NMR($CDCl_3$)δ:
2.96(2H,d,J=4.9Hz),3.18(2H,t,J=8.3Hz),3.82(3H,s),4.05(2H,t,J=8.5Hz),4.60(1H,t,J=4.8Hz),5.19(2H,s),6.80(1H,dd,J=8.8,2.4Hz), 6.84(1H,d,J=2.2Hz),7.06(1H,s),7.42-7.38(5H,m),8.13 (1H,d,J=8.8Hz).
MS(EIS)m/z:506$(M+H)^+$

(2) (S)-2-Hydroxy-4-oxo-4-[5-[(4-phenyl-5-trifluoromethyl-2-thienyl)methoxy]indolin-1-yl]butyric acid

[F66]

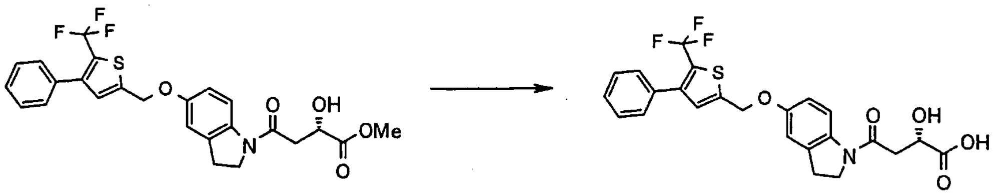

To a solution of (S)-2-hydroxy-4-oxo-4-[5-[(4-phenyl-5-trifluoromethyl-2-thienyl)methoxy]indolin-1-yl]butyric acid methyl ester in a 33% methanol/THF mixture (1.5 mL), 1N aueous sodium hydroxide solution (0.5 mL) was added, and the mixture was stirred at room temperature for 14 hours. Water (2 mL) was added to the reaction mixture, and 1N hydrochloric acid (0.6 mL) was added thereto so as to make the resultant mixture weekly acidic. Subsequently, a 10% methanol/chloroform mixture (3 mL) was further added thereto to extract an organic substance. The extracts were combined and dried over sodium sulfate anhydrate, and solvent was removed under reduced pressure. DMSO (2 mL) was added to the residue, and insoluble matter was removed by filtration. The filtrate was purified by high performance liquid chromatography (NOMURA Develosil Combi-RP-5), to thereby yield the title compound (22.0 mg).
NMR($CDCl_3$)δ:
3.14-2.92(2H,m),3.24(2H,t,J=8.1Hz),4.17-4.02(2H,m),4.56(1H,br s),5.21(2H,s),6.95-6.81(2H,m),7.07(1H,s),7.41(5H,br s),8.12(1H,d,J=8.5Hz).
MS (EIS)m/z:491 (M+H)+
Anal. Calcd for $C_{24}H_{20}O_5NF_3S$.
$0.25H_2O$:C,58.12;H,4.17;N,2.82;S,6.47.Found:C,58.06;H,4.12;N,2 .93;S,6.58.

[Example 8]

(S)-3-Hydroxy-4-oxo-4-[5-[(4-phenyl-5-trifluoromethyl-2-thienyl)methoxy]indolin-1-yl]butyric acid

[F67]

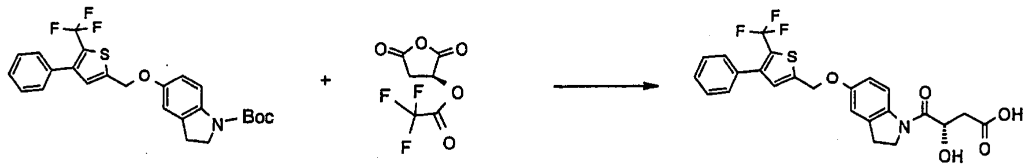

1-tert-Butoxycarbonyl-5-[(4-phenyl-5-trifluoromethyl-2-thienyl)methoxy]indoline (61 mg) was dissolved in dichloromethane (5 mL), and trifluoroacetic acid (5 mL) was added to the solution, followed by stirring for 4 hours. The reaction mixture was concentrated, to thereby form 5-[(4-phenyl-5-trifluoromethyl-2-thienyl)methoxy]indoline trifluoroacetic acid salt. The salt was dissolved in THF (2 mL), and DIEA (48 μL) and (S)-2-trifluoroacetoxytartaric anhydride (27.4 mg) were added to the soution, followed by stirring for 18 hours. The reaction mixture was concentrated under reduced pressure, and the concentrate was dissolved in DMSO (1 mL), followed by purification by high performance liquid chromatography (NOMURA Develosil Combi-RP-5), to thereby yield the title compound (38.1 mg).
MS(EIS)m/z:492(M+H)$^+$.

[Example 9]

(R)-2-Amino-4-oxo-4-[5-[(4-phenyl-5-trifluoromethyl-2-thienyl)methoxy]indolin-1-yl]butyric acid, and

[Example 10]

(R)-3-Amino-4-oxo-4-[5-[(4-phenyl-5-trifluoromethyl-2-thienyl)methoxy]indolin-1-yl]butyric acid

(1) (R)-4-Oxo-4-[5-[(4-phenyl-5-trifluoromethyl-2-thienyl)methoxy]indolin-1-yl]-2-(trifluoroacetylamino)butyric acid and (R)-4-oxo-4-[5-[(4-phenyl-5-trifluoromethyl-2-thienyl)methoxy]indolin-1-yl]-3-(trifluoroacetylamino)butyric acid

[F68]

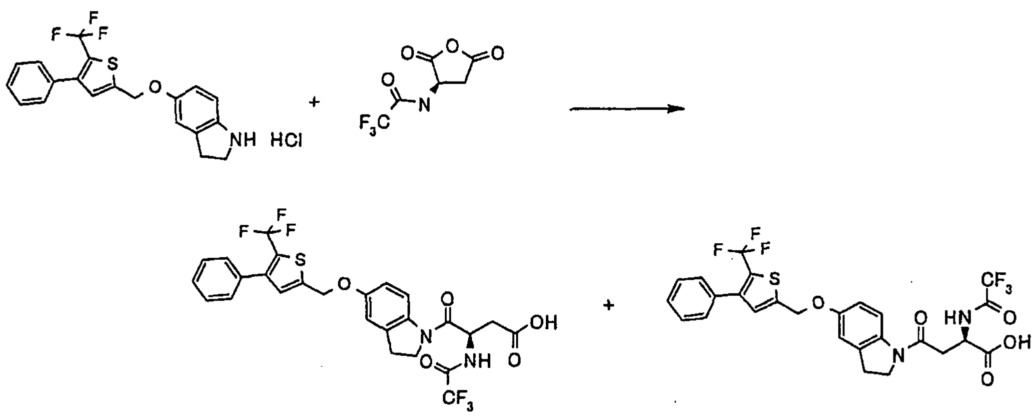

To a solution of 5-[(4-phenyl-5-trifluoromethyl-2-thienyl)methoxy]indoline hydrochloride (206 mg) and TEA (69.7 μL) dissolved in a 25% acetonitrile/dichloromethane mixture (3 mL), (R)-2-trifluoroacetylaminotartaric acid anhydride (106 mg) was added at room temperature. The reaction mixture was stirred for 14 hours and concentrated under reduced pressure, to thereby yield a mixture (302 mg) of the title compounds as a pale brown amorphous solid. The mixture was employed in a subsequent reaction without performing further isolation/purification.
MS (ESI)m/z:587 $(M+H)^+$.

(2) (R)-2-Amino-4-oxo-4-[5-[(4-phenyl-5-trifluoromethyl-2-thienyl)methoxy]indolin-1-yl]butyric acid and (R)-3-amino-4-oxo-4-[5-[(4-phenyl-5-trifluoromethyl-2-thienyl)methoxy]indolin-1-yl]butyric acid

[F69]

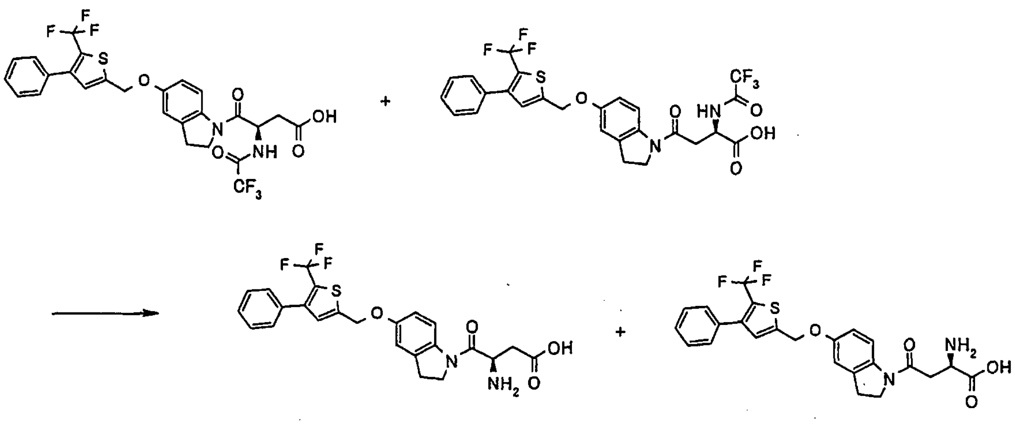

To a solution of (R)-4-oxo-4-[5-[(4-phenyl-5-trifluoromethyl-2-thienyl)methoxy]indolin-1-yl]-2-(trifluoroacetylamino)butyric acid and (R)-4-oxo-4-[5-[(4-phenyl-5-trifluoromethyl-2-thienyl)methoxy]indolin-1-yl]-3-(trifluoroacetylamino)butyric acid (302 mg) in a 33% methanol/THF mixture (1.5 mL), 1N aqueous sodium hydroxide solution (0.5 mL) was added, and the resultant mixture was stirred at room temperature for 14 hours. Water (2 mL) was added to the reaction mixture, and then, 1N aqueous hydrochloric acid solution was added thereto for neutralization, followed by extraction with a 10% methanol/chloroform mixture (3 mL). The extracts were combined and concentrated under reduced pressure. DMSO (6 mL) was added to the residue, and insoluble matter was removed by filtration. The mixture was purified by high performance liquid chromatography (NOMURA Develosil Combi-RP-5). A solid, which precipitated from a fraction containing a compound having a long retention time, was collected by filtration, followed by washing with water and drying, to thereby yield (R)-2-amino-4-oxo-4-[5-[(4-phenyl-5-trifluoromethyl-2-thienyl)methoxy]indolin-1-yl] butyric acid (21.3 mg). Separately, fractions containing a compound having a short retention time were collected, followed by lyophilization, to thereby yield (R)-3-amino-4-oxo-4-[5-[(4-phenyl-5-trifluoromethyl-2-thienyl)methoxy]indolin-1-yl]butyric acid (36.5 mg).

(R)-2-Amino-4-oxo-4-[5-[(4-phenyl-5-trifluoromethyl-2-thienyl)methoxy]indolin-1-yl]butyric acid:
NMR(DMSO-$d_6$)δ:
2.70(1H,dd,J=17.3,9.0Hz),3.05(1H,dd,J=17.1,3.1Hz),3.13(2H,t,J =8.5Hz),3.54(1H,dd,J=8.9,3.1Hz),4.06(2H,dt, J=11.2,8.5Hz),5.34 (2H,s),6.86(1H,dd,J=8.8,2.4Hz),6.99(1H,d,J=2.4Hz),7.35(1H,br s),7.50-7.41(5H,m),8.00(1H,d, J=8.8Hz).
MS(EIS)m/z:491$(M+H)^+$.
Anal. Calcd for
$C_{24}H_{21}O_4N_2F_3S\cdot1.25H_2O$:C,56.19;H,4.62;N,5.46;S,6.25.Found:C,56.3 5;H,4.56;N,5.58;5,6.38.
(R)-3-Amino-4-oxo-4-[5-[(4-phenyl-5-trifluoromethyl-2-thienyl)methoxy]indolin-1-yl]butyric acid:
NMR(DMSO-$d_6$)δ:
2.25(1H,dd,J=16.2,8.7Hz),2.59(1H,dd,J=16.1,5.6Hz),3.15(2H,t,J =8.5Hz),4.00(1H,dd,J=5.9,8.8Hz),4.13(1H,dt, J=8.3,9.3Hz),4.28( 1H,dt,J=7.6,9.3Hz),5.36(2H,s),6.88(1H,dd,J=8.8,2.7Hz),7.01(1H ,d,J=2.4Hz),7.37(1H,br s), 7.51-7.42(5H,m),8.02(1H,d,J=8.8Hz).
MS(EIS)m/z:491$(M+H)^+$.
Anal. Calcd for
$C_{24}H_{21}O_4N_2F_3S\cdot1.5H_2O$:C,55.70;H,4.67;F,11.00;N,5.41;S,6.20.Found :C,55.85;H,4.35;F,11.12;N,5.38;S,6.35.

[Example 11]

(R)-3-Amino-4-oxo-4-[5-[3,5-bis-(trifluoromethyl)benzyloxy]indolin-1-yl]butyric acid hydrochloride

(1) 5-[3,5-Bis-(trifluoromethyl)benzyloxy]-1-tert-butoxycarbonylindoline

[F70]

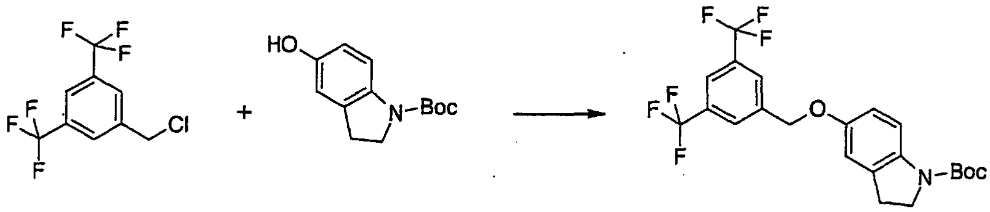

To a DMF solution (5 mL) of 3,5-bis-(trifluoromethyl)benzyl chloride (341 mg) and 1-(tert-butoxycarbonyl)-5-hydroxyindoline (235 mg), potassium carbonate (415 mg) was added, and the mixture was heated to 50°C, followed by stirring for 14 hours. The reaction mixture was cooled to room temperature, and the residue was removed by filtration. The filtrate was concentrated under reduced pressure, to thereby yield the title compound (470 mg).
NMR($CDCl_3$)δ:
1.54(9H,s),3.07(2H,t,J=8.6Hz),3.93-4.04(2H,m),5.12(2H,s),6.74-6.84(2H,m),7.31-7.21(1H,m),7.84(1H,s),7.89(2H,s).

(2) 5-[3,5-Bis-(trifluoromethyl)benzyloxy]indoline hydrochloride

[F71]

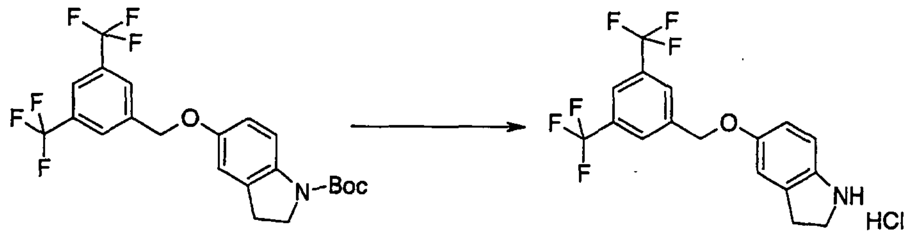

5-[3,5-Bis-(trifluoromethyl)benzyloxy]-1-tert-butoxycarbonylindoline (461 mg) was dissolved in a 4N hydrochloric acid/1,4-dioxane solution (10 mL), followed by stirring at room temperature for 15 hours. The reaction mixture was concentrated under reduced pressure, to thereby yield the title compound (412 mg).
NMR(DMSO-$d_6$)δ:
3.16(2H,t,J=7.4Hz),3.70(2H,t,J=7.8Hz),5.33(2H,s),7.02(1H,d,J= 8.6Hz),7.16(1H,s),7.35-7.28(1H,m),8.11(1H,s),8.16(2H,s).
MS(ESI)m/z:362 $(M+H)^+$.

(3) (R)-3-tert-Butoxycarbonylamino-4-oxo-4-[5-[3,5-bis-(trifluoromethyl)benzyloxy]indolin-1-yl]butyric acid tert-butyl ester

[F72]

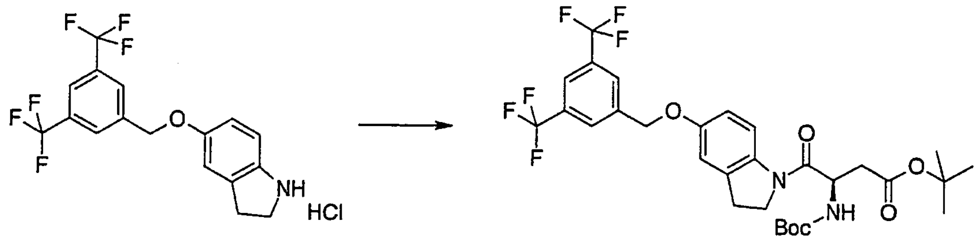

TEA (0.697 mL) was added at room temperature to a DMF solution (4 mL) of 5-[3,5-bis-(trifluoromethyl)benzyloxy]indoline hydrochloride (398 mg), Boc-D-Asp(OtBu)-OH (289 mg), EDC·HCl (288 mg), and HOBt (203 mg), and the mixture was stirred at room temperature 14 hours. The reaction mixture was concentrated under reduced pressure, and the residue was purified by silica gel flash column chromatography (Biotage 25S), to thereby yield the title compound (566 mg).
NMR($CDCl_3$)δ:
1.42(9H,s),1.43(9H,s),2.58(1H,dd,J=15.7,5.9Hz),2.83(1H,dd,J=1 5.7,7.8Hz),3.20(2H,t,J=7.8Hz),4.25-4.43(2H,m),4.92 (1H,dd,J=15.2,6.6Hz),5.13(2H,s),5.28(1H,d,J=9. 6Hz),6.80(1H,dd,J=8.8,2.7Hz),6.85(1H,d,J=2.5Hz),7.84(2H,s),7. 89 (1H,s),8.15(1H,d,J=8.8Hz).
MS(ESI)m/z:633(M+H)$^+$.

(4) (R)-3-Amino-4-oxo-4-[5-[3,5-bis-(trifluoromethyl)benzyloxy]indolin-1-yl]butyric acid hydrochloride

[F73]

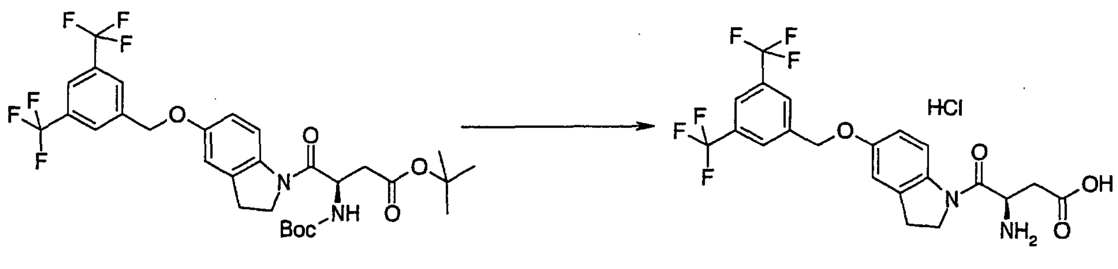

4N HCl/1,4-dioxane (10 mL) and (R)-3-tert-butylcarbonylamino-4-oxo-4-[5-[3,5-bis-(trifluoromethyl)benzyloxy] indolin-1-yl]butyric acid tert-butyl ester (566 mg) was combined and the solution was stirred at room temperature for 14 hours. The reaction mixture was concentrated under reduced pressure, and hexane was added to the residue. The precipitated solid was collected by filtration and dried under reduced pressure, to thereby yield the title compound (440 mg).
NMR(DMSO-$d_6$)δ:
2.54-2.86(1H,m),3.04(1H,dd,J=17.8,4.8Hz),3.17(1H,t,J=7.8Hz),3.57(2 H,d,J=0.5Hz),4.07-4.31(2H,m),4.41-4.47(1H,m),5.30(2H,s),6.91(1H,dd,J=8.8,2.2Hz),7.04-7.07(1H,m),8.01(1H,d,J=8.8Hz),8.10(1H,s),8.15(2H,s).
IR(ATR)cm$^{-1}$:2999,2914,2600,2305,1732,1653,1599,1572. MS(ESI)m/z:477(M+H)$^+$.
HRMS(FAB)Calcd for $C_{21}H_{19}F_6N_2O_4$:477.1249.Found:477.1248. Anal. Calcd for $C_{21}H_{18}F_6N_2O_4$·1.0HCl·0.25EtOH·0.5$H_2O$:C,48.42;H,4.06;Cl,6.65;F,21. 37;N,5.25.Found:C,48.63;H,3.87;Cl,6.91;F, 21.11;N,5.27.

[Example 12]

(R)-3-Amino-4-oxo-4-[5-(4-biphenylmethoxy)indolin-1-yl]butyric acid hydrochloride

(1) 5-(4-Biphenylmethoxy)indoline-1-carboxylic acid tert-butyl ester

[F74]

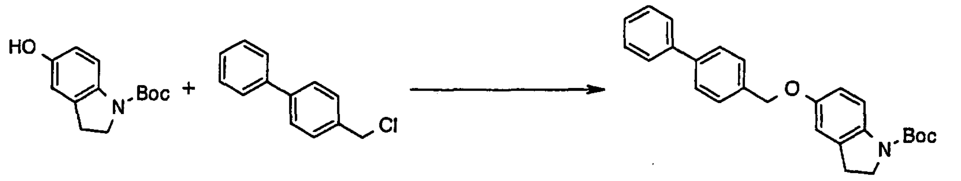

To a DMF solution (5 mL) of 1-Boc-5-hydroxyindoline (150 mg), 4-chloromethylbiphenyl (155 mg) and potassium carbonate (132 mg) were added. The mixture was stirred at 70°C overnight and left to stand to cool to room temperature. Saturated aqueous sodium bicarbonate solution was added to the reaction mixture, followed by extracting twice, each with ethyl acetate. The extracts were combined and washed with saturated brine, followed by drying with sodium sulfate anhydrate and concentrating. The residue was purified by flash column chromatography (Yamazen Hi-Flash column L), to thereby yield the title compound (240 mg).
NMR($CDCl_3$)δ:
1.56(9H,s),3.06(2H,t,J=8.6Hz),3.97(2H,s),5.06(2H,s),6.78-6.83(2H,m),7.26-7.76(10H,m).

(2) 5-(4-Biphenylmethoxy)indoline hydrochloride

[F75]

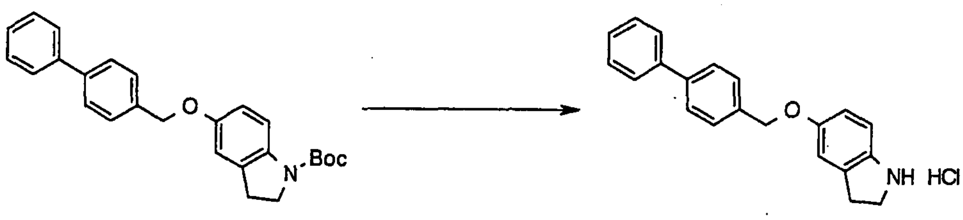

To 1-(tert-butoxycarbonyl)-5-(4-biphenylmethoxy)indoline (230 mg), 4N HCl/1,4-dioxane (10 mL) was added, and the mixture was stirred at room temperature for 4 hours. Diethyl ether was added to the reaction mixture, and the precipitated solid was collected by filtration and dried, to thereby yield the title compound (198 mg).
NMR(DMSO-$d_6$)δ:
3.15-3.19(2H,m),3.69-3.72(2H,m),5.19(2H,s),7.01(1H,dd,J=2.6,8.7Hz),7.15(1H,d,J=2.6 Hz),7.33-7.39(2H,m), 7.45-7.54(4H,m),7.66-7.71(4H,m),11.06(1H,s).
MS(ESI)m/z:302$(M+H)^+$.

(3) (R)-3-tert-Butoxycarbonylamino-4-oxo-4-[5-(4-biphenylmethoxy)indolin-1-yl]butyric acid tert-butyl ester

[F76]

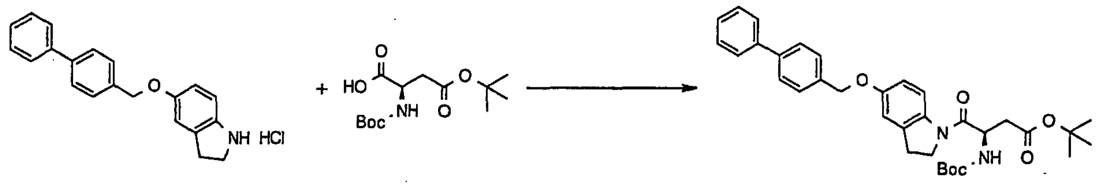

Boc-D-Asp(OtBu)-OH (195 mg), HOBt (114 mg), EDC·HCl (161 mg), and TEA (392 μL) were added to a DMF solution (10 mL) of 5-(4-biphenylmethoxy)indoline hydrochloride (190 mg), and the mixture was stirred overnight. To the reaction mixture, saturated aqueous sodium bicarbonate solution was added, followed by extracting twice, each with ethyl acetate.
The extracts were combined and washed with saturated brine, followed by drying over sodium sulfate anhydrate and concentrating. The residue was purified by flash column chromatography (Yamazen Hi-Flash column L), to thereby yield the title compound (321 mg).
NMR($CDCl_3$)δ:
1.42(9H,s),1.43(9H,s),2.57(1H,dd,J=6.1,15.7Hz),2.79-2.85(1H,m),3.19(2H,t,J=8.3Hz),4.26-4.40(2H,m),4.89-4.95(1H, m),5.08(2H,s),5.28(1H,d,J=9.6Hz),6.81-6.85(2H,m),7.33-7.50(5H,m),7.58-7.62(4H,m),8.12(1H,d,J=8.6Hz).
MS (ESI) m/z : 573 $(M+H)^+$.

(4) (R)-3-Amino-4-oxo-4-[5-(4-biphenylmethoxy)indolin-1-yl]butyric acid hydrochloride

[F77]

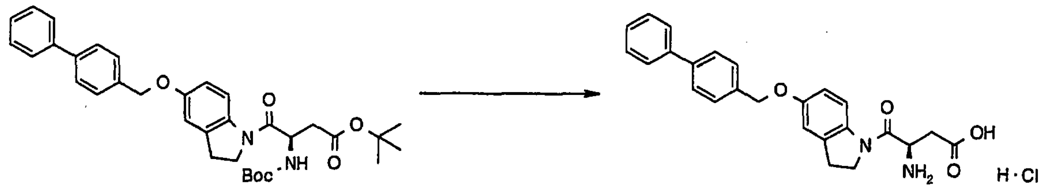

To (R)-3-tert-butoxycarbonylamino-4-oxo-4-[5-(4-biphenylmethoxy)indolin-1-yl]butyric acid tert-butyl ester (315 mg), 4N HCl/1,4-dioxane (5 mL) was added, and the mixture was stirred overnight at room temperature. The reaction mixture was concentrated under reduced pressure, and the residue was suspended with diethyl ether. The solid matter was collected by filtration and dried, to thereby yield the title compound (219 mg). NMR(DMSO-$d_6$)δ:
2.73-2.80(1H,m),3.01(1H,dd,J=5.8,17.3Hz),3.15(2H,t,J=8.2Hz),4.14-4.42(3H,m),5.12(2H,s),6.87(1H,dd,J=2.4,8.4Hz), 7.00(1H,s),7.34 -7.38(1H,m),7.44-7.52(4H,m),7.65-7.68(4H,m),7.99(1H,d,J=8.8Hz). IR(ATR)$cm^{-1}$:2898,1650,1484, 1265,1189.
MS (ESI)m/z:417 $(M+H)^+$.
HRMS(FAB)calcd for $C_{25}H_{25}N_2O_4(M+H)^+$:417.1814. Found 417.1819. Anal. Calcd for $C_{25}H_{25}ClN_2O_4 \cdot 0.5H_2O$:C,65.00;H,5.67;Cl,7.67;N,6.06.Found:C,64.9 4;H,5.62;Cl,7.54;N,5.99.

[Example 13]

(R)-3-Amino-4-oxo-4-[5-(2-cyano-4-biphenylmethoxy)indolin-1-yl]butyric acid hydrochloride

(1) 4-Chloromethylbiphenyl-2-carbonitrile

[F78]

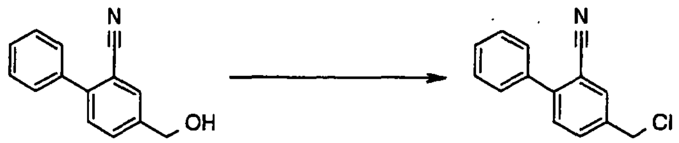

To a dichloroethane solution (10 mL) of 4-hydroxymethylbiphenyl-2-carbonitrile (120 mg), thionyl chloride (208 μL) and a drop of DMF (by means of a Pasteur pipette) were added. The mixture was stirred at 50°C overnight and left to stand to cool to room temperature, followed by concentration. The residue was purified by flash column chromatography (Yamazen Hi-Flash column L), to thereby yield the title compound (136 mg).
NMR($CDCl_3$)δ:
4.63(2H,s),7.46-7.57(6H,m),7.67(1H,dd,J=8.1,2.0Hz),7.79(1H,d,J=2.0Hz).
MS(ESI)m/z:228(M+H)$^+$.

(2) 1-(tert-Butoxycarbonyl)-5-(2-cyano-4-biphenylmethoxy)indoline

[F79]

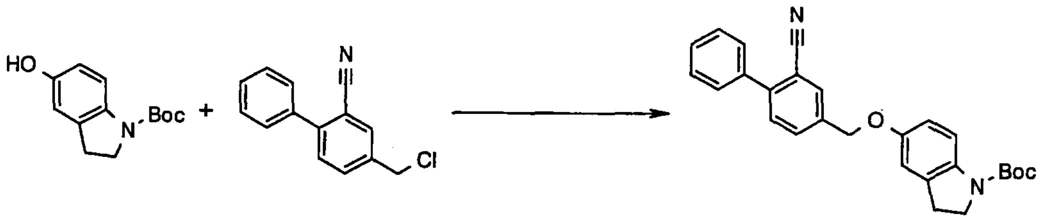

To a DMF solution (5 mL) of 1-(tert-butoxycarbonyl)-5-hydroxyindoline (150 mg), 4-chloromethylbiphenyl-2-carbonitrile (174 mg) and potassium carbonate (132 mg) were added. The mixture was stirred at 70°C overnight and left to stand to cool to room temperature. Saturated aqueous sodium bicarbonate solution was added to the reaction mixture, followed by extracting twice, each with ethyl acetate. The extracts were combined and washed with saturated brine. The organic layer was dried over sodium sulfate anhydrate and concentrated. The residue was purified by flash column chromatography (Yamazen Hi-Flash column L), to thereby yield the title compound (264 mg).
NMR($CDCl_3$)δ:
1.55(9H,s),3.07(2H,t,J=8.7Hz),3.98(2H,s),5.08(2H,s),6.76-6.82(2H,m),7.43-7.83(9H,m).
MS (ESI)m/z:327 (M-Boc)$^+$.

(3) 5-(2-Cyano-4-biphenylmethoxy)indoline hydrochloride

[F80]

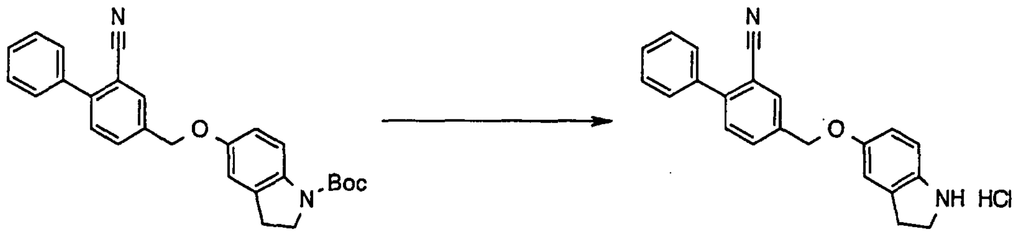

To 1-(tert-butoxycarbonyl)-5-(2-cyano-4-biphenylmethoxy)indoline (255 mg), 4N HCl/1,4-dioxane (10 mL) was added, and the mixture was stirred at room temperature for 4 hours. To the reaction mixture, diethyl ether was added. The precipitated solid was collected by filtration and dried, to thereby yield the title compound (222 mg). NMR(DMSO-$d_6$)δ: 3.16-3.20(2H,m),3.70-3.73(2H,m),5.25(2H,s),7.05(1H,dd,J=2.6,8.7Hz),7.18(1H,d,J=2.6 Hz),7.37(1H,d,J=8.7Hz), 7.48-7.61(5H,m),7.67(1H,d,J=8.1Hz),7.85(1H,dd,J=2.0,8.1Hz),8.03(1H ,d,J=1.5Hz),11.20(1H,br s).
MS(ESI)m/z:327$(M+H)^+$.

(4) (R)-3-tert-Butoxycarbonylamino-4-oxo-4-[5-(2-cyano-4-biphenylmethoxy)indolin-1-yl]butyric acid tert-butyl ester

[F81]

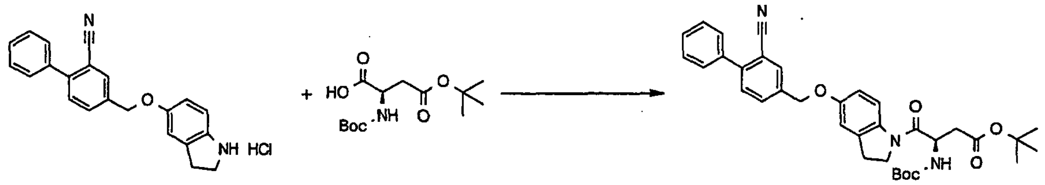

Boc-D-Asp(OtBu)-OH (205 mg), HOBt (120 mg), EDC·HCl (170 mg), and TEA (413 μL) were added to a DMF solution (10 mL) of 5-(2-cyanobiphenyl-4-ylmethoxy)indoline hydrochloride (215 mg), and the mixture was stirred overnight. To the reaction mixture, saturated aqueous sodium bicarbonate solution was added, followed by extracting twice, each with ethyl acetate. The extracts were combined and washed with saturated brine, followed by drying over sodium sulfate anhydrate and concentrating. The residue was purified by flash column chromatography (Yamazen Hi-Flash column L), to thereby yield the title compound (327 mg).
NMR($CDCl_3$)δ:
1.42(9H,s),1.43(9H,s),2.58(1H,dd,J=6.1,15.7),2.80-2.86(1H,m),3.21(2H,t,J=8.3Hz),4.28-4.41(2H,m),4.89-4.95(1H,m), 5.10(2H,s),5.27(1H,d,J=9.8Hz),6.78-6.85(2H,m),7.43-7.58(6H,m),7.69(1H,dd,J=1.7,8.1Hz),7.83(1H,d,J=1.5Hz),8.14 (1H ,d,J=8.8Hz).
MS(ESI)m/z:598$(M+H)^+$.

(5) (R)-3-Amino-4-oxo-4-[5-(2-cyano-4-biphenylmethoxy)indolin-1-yl]butyric acid hydrochloride

[F82]

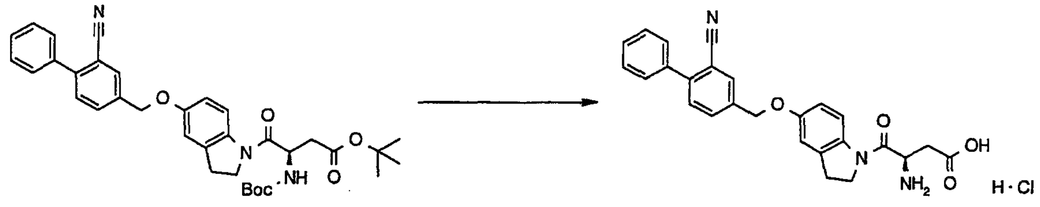

To (R)-3-tert-butoxycarbonylamino-4-oxo-4-[5-(2-cyano-4-biphenylmethoxy)indolin-1-yl]butyric acid tert-butyl ester (320 mg), 4N HCl/1,4-dioxa (5 mL) was added, and the mixture was stirred overnight at room temperature, followed by stirring at 50°C for 8 hours and further stirring overnight at room temperature. The reaction mixture was concentrated under reduced pressure, and the residue was suspended in diethyl ether. The solid matter was collected by filtration, followed by drying, to thereby yield the title compound (213 mg).
NMR(DMSO-$d_6$)δ:
2.77-2.83(1H,m),3.00-3.07(1H,m),3.18(2H,t,J=8.2Hz),4.19-4.44(3H,m),5.20(2H,s),6.91(1H,d,J=8.6Hz),7.05(1H,s), 7.48-7.67(6H,m),7.84(1H,dd,J=1.5,8.1Hz),8.01-8.03(2H,m). IR(ATR)$cm^{-1}$:3239,2219,1643,1486,1419,1180.
MS(ESI)m/z:442$(M+H)^+$.
HRMS (FAB) calcd for $C_{26}H_{24}N_3O_4$ $(M+H)^+$: 442.1767; Found 442.1774. Anal. Calcd for $C_{26}H_{24}ClN_3O_4 \cdot 0.25H_2O$:C,64.73;H,5.12;Cl,7.35;N,8.71.Found:C,64. 42;H,5.05;Cl,7.41;N,8.72.

[Example 14]

(R)-3-Amino-4-oxo-4-[5-[(2-trifluoromethyl-4-biphenyl)methoxy]indolin-1-yl]butyric acid

(1) 4-Trifluoromethanesulfonyloxy-3-trifluoromethylbenzoic acid methyl ester

[F83]

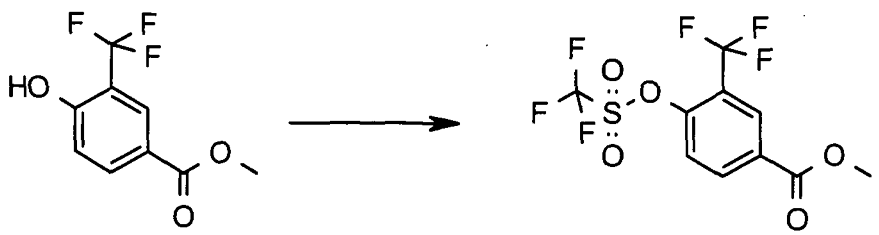

To a methylene chloride solution (50 mL) of 4-hydroxy-3-trifluoromethylbenzoic acid methyl ester (1.05 g, 4.77 mmol), triethylamine (1.00 mL, 7.15 mmol) and 4-dimethylaminopyridine (58 mg, 0.48 mmol) were added. After cooling to 0°C, trifluoromethanesulfonic anhydride (962 μL, 5.72 mmol) was added thereto, and the mixture was stirred overnight at room temperature. To the reaction mixture, saturated aqueous sodium bicarbonate solution was added, followed by extraction twice, each with chloroform. The organic layer was washed sequentially with saturated aqueous ammonium chloride solution and saturated brine, dried over sodium sulfate anhydrate, and concentrated. The residue was purified by flash column chromatography (Yamazen Hi-Flash column 2L, ethyl acetate/n-hexane = 3% to 23%), to thereby yield the title compound (1.60 g, 95%) as pale brown oil.
$^1$H-NMR($CDCl_3$)δ:
3.99(3H,s),7.62(1H,d,J=8.8Hz),8.34(1H,dd,J=2.0,8.8Hz),8.44(1H ,d,J=2.0Hz).
MS(ESI)m/z:353$(M+H)^+$.

(2) 2-Trifluoromethyl-4-biphenylcarboxylic acid methyl ester

[F84]

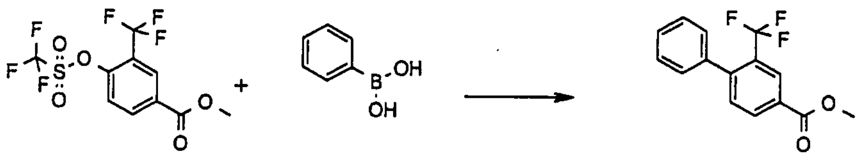

To a solution of 4-trifluoromethanesulfonyloxy-3-trifluoromethylbenzoic acid methyl ester (361 mg) in toluene (10 mL), phenyl borate (250 mg), cesium carbonate (1.00 g), and water (2.0 mL) were added at room temperature. Nitrogen was bubbled through the reaction mixture for 3 minutes, and tetrakis(triphenylphosphine)palladium (236 mg) was added thereto. The reaction mixture was stirred at 90°C for 90 minutes, followed by standing to cool to room temperature. Ethyl acetate (20 mL) and saturated brine (20 mL) were added thereto for phase separation. The organic layer was dried over sodium sulfate anhydrate, and solvent was evaporated under reduced pressure. The residue was purified by silica gel flash column chromatography (Biotage 25M), to thereby yield the title compound (278 mg).
NMR($CDCl_3$)δ:
3.98(3H,s),7.29-7.45(6H,m),8.21(1H,dd,J=8.0,1.4Hz),8.43(1H,d,J=1.4Hz).

(3) (2-Trifluoromethyl-4-biphenyl)methanol

[F85]

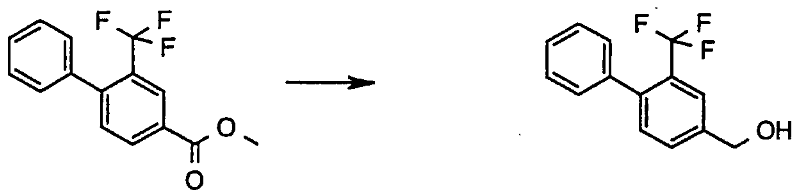

To a solution of 2-trifluoromethyl-4-biphenylcarboxylic acid methyl ester (215 mg) in THF (10 mL), lithium borohydride (50.0 mg) was added at room temperature. The reaction mixture was refluxed for 15 hours and left to stand to cool to room temperature. Water (30 mL) and 1N hydrochloric acid (30 mL) were added thereto, and the mixture was extracted with ethyl acetate (2 x 30 mL). The extracts were combined and washed with saturated aqueous sodium hydrogencarbonate solution (30 mL), followed by drying over sodium sulfate anhydrate. Solvent was evaporated under reduced pressure. The residue was purified by silica gel flash column chromatography (Biotage 25M), to thereby yield the title compound (182 mg, 94%).
NMR($CDCl_3$)δ:
1.85(1H,t,J=5.9Hz),4.81(2H,d,J=5.9Hz),7.27-7.48(6H,m),7.56(1H,br d,J=8.3Hz),7.76(1H,br s).

(4) 1-(tert-Butoxycarbonyl)-5-[(2-trifluoromethyl-4-biphenyl)methoxy]indoline

[F86]

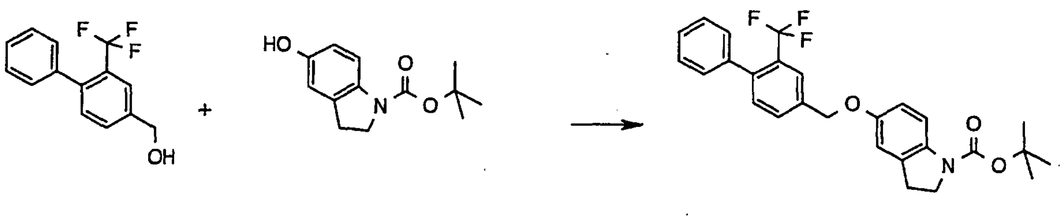

To a solution of (2-trifluoromethyl-4-biphenyl)methanol (303 mg) in THF (6.0 mL), 1-(tert-butoxycarbonyl)-5-hydroxyindoline (340 mg), triphenylphosphine (380 mg), and DEAD (0.230 mL) were added at room temperature. The mixture was stirred at room temperature for 2 days and concentrated under reduced pressure. The residue was purified by silica gel flash column chromatography (Biotage 40M), to thereby yield the title compound (398 mg).
NMR($CDCl_3$)δ:
1.54(9H,s),3.08(2H,t,J=8.7Hz),3.99(2H,br s),5.11(2H,s),6.80(1H,d,J=8.8Hz),6.84(1H,s),7.30-7.43(6H,m),7.62(1H,d, J=7.8Hz),7.70 7.86(1H,br),7.81(1H,s).MS(ESI)m/z:514(M+H-isobutene)$^+$.

(5) 5-[(2-Trifluoromethyl-4-biphenyl)methoxy]indoline hydrochloride

[F87]

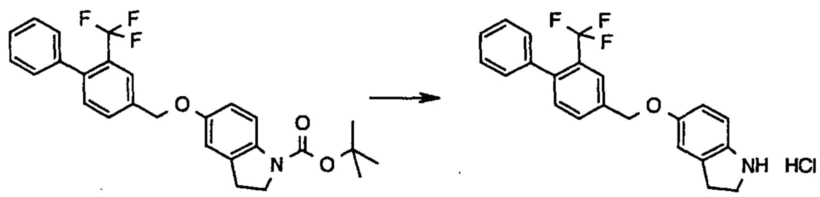

To 1-(tert-butoxycarbonyl)-5-[(2-trifluoromethyl-4-biphenyl)methoxy]indoline (395 mg), 4N HCl/1,4-dioxane (5.0 mL) was added at room temperature. The reaction mixture was stirred at room temperature for 2 hours and concentrated under reduced pressure. The residue was slurried with hexane over 15 hours. The slurry was filtered, and the collected solid matter was dried (in vacuo, 40°C, 2 hours), to thereby yield the title compound (334 mg).
NMR(DMSO-$d_6$)δ:
3.16(2H,t,J=7.8Hz),3.69(2H,t,J=7.8Hz),5.27(2H,s),7.01(1H,dd,J =8.6,2.2Hz),7.16(1H,d,J=2.2Hz),7.28-7.35(3H,m), 7.40-7.48(4H,m),7.76(1H,d,J=7.8Hz),7.89(1H,s).
MS (ESI) m/z : 370 (M+H) +.

(6) (R)-3-(tert-Butoxycarbonylamino)-4-oxo-4-[5-[(2-trifluoromethyl-4-biphenyl)methoxy]indolin-1-yl]butyric acid tert-butyl ester

[F88]

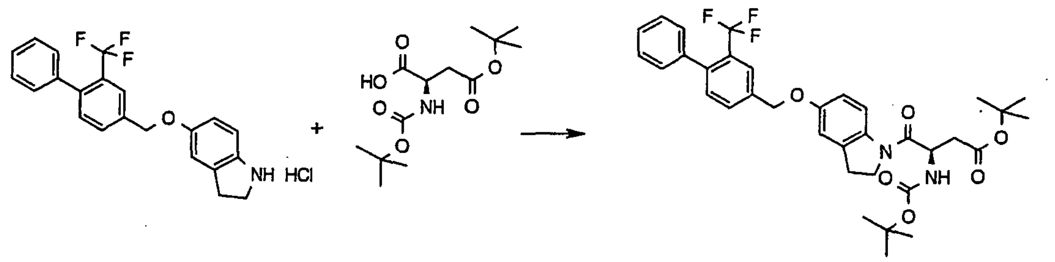

EDC·HCl (243 mg), HOBt (101 mg), and TEA (0.350 mL) were added at room temperature to a suspension of 5-[(2-trifluoromethyl-4-biphenyl)methoxy]indoline hydrochloride (342 mg) and Boc-D-Asp(OtBu)-OH (244 mg) in DMF (3.0 mL), and the mixture was stirred for 3 days. The reaction mixture was concentrated under reduced pressure. To the concentrate, ethyl acetate (30 mL), saturated aqueous sodium hydrogencarbonate solution (20 mL), and water (40 mL) were added for phase separation, and the aqueous layer was extracted with ethyl acetate (20 mL). The extracts were combined and dried over sodium sulfate anhydrate. Solvent was removed under reduced pressure. The residue was purified by silica gel flash column chromatography (Biotage 40M), to thereby yield the title compound (473 mg).
NMR($CDCl_3$)δ:
1.42(9H,s)1.43(9H,s),2.58(1H,dd,J=15.6,6.1Hz),2.83(1H,dd,J=15 .6,7.6Hz),3.21(2H,t,J=8.4Hz),4.26-4.42(2H,m), 4.88-4.98(1H,m),5.12(2H,s),5.27(1H,d,J=10.0Hz),6.83(1H,dd,J=8.7,2. 7Hz),6.87(1H,s),7.29-7.43(6H,m),7.62(1H,d, J=8.3Hz),7.80(1H,s)8.14(1H,d,J=8.8Hz).
MS(ESI)m/z:641$(M+H)^+$.

(7) (R)-3-Amino-4-oxo-4-[5-[(2-trifluoromethyl-4-biphenyl)methoxy]indolin-1-yl]butyric acid

[F89]

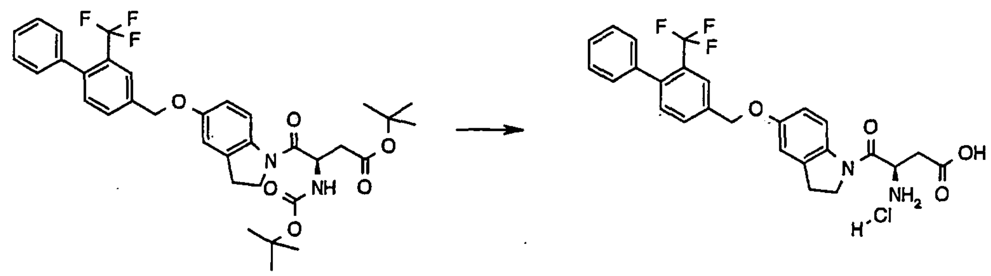

To (R)-3-(tert-butoxycarbonylamino)-4-oxo-4-[5-[(2-trifluoromethyl-4-biphenyl)methoxy]indolin-1-yl]butyric acid tert-butyl ester (470 mg), 4N HCl/1,4-dioxane (10.0 mL) was added at room temperature, and the mixture was stirred at room temperature for 16 hours. The reaction mixture was concentrated under reduced pressure, and the concentrate was slurried with hexane. The solid was collected by filtration and dried (in vacuo, 40°C, 3 hours), to thereby yield the title compound (378 mg, 97%). NMR(DMSO-$d_6$)δ:
2.71(1H,dd,J=17.5,7.5Hz),3.02(1H,dd,J=17.5,5.1Hz),3.18(2H,t,J =8.1Hz),4.14-4.30(2H,m),4.43(1H,dd,J=7.5,5.1Hz), 5.24(2H,s),6.92(1H,dd,J=9. 0,1.8Hz),7.05(1H,d,J=1.8Hz),7.29-7.34(2H,m),7.42-7.48(4H,m),7.76(1H,d,J=8.1Hz),7.89 (1H,s),8.01(1H,d,J=8.8Hz).
$CO_2H$ and $NH_2$ (3H) not observed.
IR(ATR)$cm^{-1}$:3028,1722,1655,1597,1487,1423,1317,1120,1068,700.
MS(ESI)m/z:485$(M+H)^+$.
HRMS (ESI) Calcd for $C_{26}H_{24}F_3N_2O_4(M+H)^+$:485.1688.Found:485.1673. Anal. Calcd for $C_{26}H_{23}F_3N_2O_4\cdot1.0HCl\cdot0.5H_2O$:C,58.93;H,4.75;Cl,6.69;F,10.75;N,5. 29.Found:C,58.85;H,4.65;Cl,6.65;F,10.75;N,5.19.

[Example 15]

(R)-3-Amino-4-oxo-4-[5-[(1-phenyl-5-trifluoromethyl-1H-pyrazol-3-yl)methoxyindolin-l-yl]butyric acid hydrochloride

(1) 3-Methyl-1-phenyl-5-trifluoromethyl-1H-pyrazole

[F90]

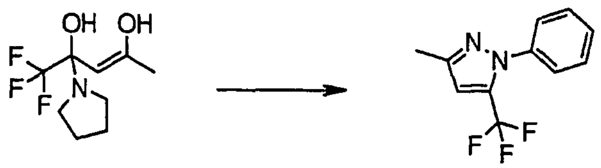

To a tetrahydrofuran solution (25 mL) of phenylhydrazine (20 mmol), 5,5,5-trifluoro-4-(1-pyrrolidinyl)-2-pentene-2,4-diol (4.50 g) was added, and the mixture was stirred at room temperature for 19 hours. The reaction mixture was concentrated under reduced pressure, and the residue was purified by silica gel column chromatography. The obtained compound was dissolved in dichloromethane (50 mL), and concentrated hydrochloric acid (100 μL) was added thereto, followed by stirring at room temperature for 1 hour. The reaction mixture was washed with saturated aqueous sodium bicarbonate solution (50 mL), and the aqueous layer was extracted with chloroform (20 mL x 2). The extracts were combined and dried over magnesium sulfate anhydrate, followed by concentration under reduced pressure. The residue was subjected to silica gel column chromatography, to thereby yield the title compound (2.02 g).
NMR($CDCl_3$)δ:
2.36(3H,s),6.60(1H,s),7.44-7.48(5H,m).
MS(ESI)m/z:227(M+H)$^+$.
(2) 3-Bromomethyl-1-phenyl-5-trifluoromethyl-1H-pyrazole
[F91]

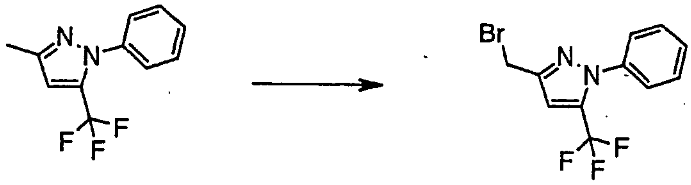

To a carbon tetrachloride solution (25 mL) of 3-methyl-1-phenyl-5-trifluoromethyl-1H-pyrazole (679 mg), N-bromosuccinimide (641 mg) and benzoyl peroxide (19 mg) were added, and the mixture was refluxed for 21 hours and left to stand to cool to room temperature. Insoluble matter was removed by filtration, and the filtrate was subjected to silica gel column chromatography, to thereby yield the title compound (315 mg).
NMR($CDCl_3$)δ:
4.52(2H,s),6.88(1H,s),7.47-7.50(5H,m).
MS(ESI)m/z:305(M+H)$^+$.

(3) 1-tert-Butoxycarbonyl-5-[(1-phenyl-5-trifluoromethyl-1H-pyrazol-3-yl)methoxy]indoline

[F92]

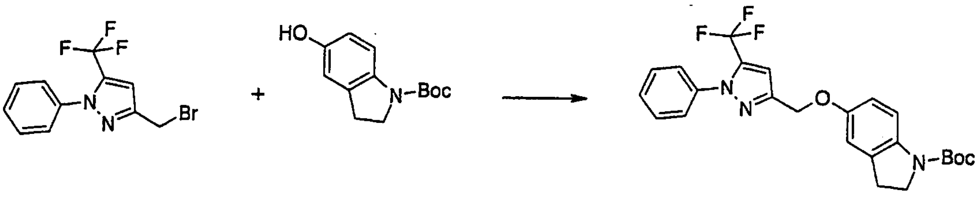

To a DMF solution (2 mL) of 3-bromomethyl-1-phenyl-5-trifluoromethyl-1H-pyrazole (305 mg) and 1-(tert-butoxycarbonyl)-5-hydroxyindoline (141 mg), potassium carbonate (276 mg) was added, and the mixture was heated to 60°C, followed by stirring for 22 hours. The reaction mixture was cooled to room temperature, and then, insoluble matter was removed by filtration. The filtrate was concentrated under reduced pressure. The residue was purified by silica gel flash column chromatography (Biotage 25S), to thereby yield the title compound (128 mg).
NMR($CDCl_3$)δ:
1.55(9H,s),3.07(2H,t,J=8.6Hz),3.91-4.03(2H,m),5.10(2H,s),6.81(1H,d,J=8.3Hz),6.84(1H,s),6.91(1H,s ),7.47-7.51(5H, m),7.84-7.70(1H,m).
MS(ESI)m/z:460$(M+H)^+$.

(4) 5-[(1-Phenyl-5-trifluoromethyl-1H-pyrazol-3-yl)methoxy]indoline hydrochloride

[F93]

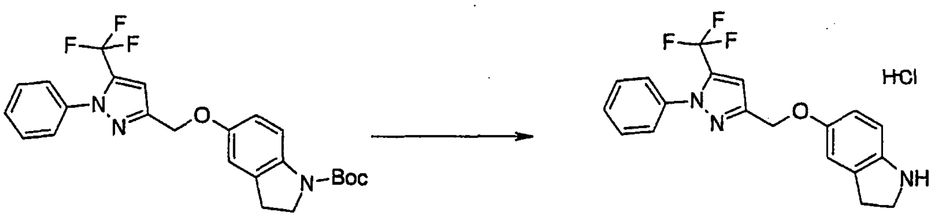

1-tert-Butoxycarbonyl-5-[(1-phenyl-5-trifluoromethyl-1H-pyrazol-3-yl)methoxy]indoline (128 mg) was dissolved in 4N HCl/1,4-dioxane (3 mL), and the mixture was stirred at room temperature for 18 hours. The reaction mixture was concentrated under reduced pressure, and the concentrate was subjected to recrystallization by use of ether/hexane/ethyl acetate, to thereby yield the title compound (89 mg).
NMR(DMSO-$d_6$)δ:
3.17(2H,t,J=7.7Hz),3.71(2H,t,J=7.8Hz),5.18(2H,s),7.03(1H,dd,J =8.8,2.5Hz),7.17(1H,d,J=2.5Hz),7.26(1H,s),7.33(1H, d,J=8.8Hz), 7.51-7.55(2H,m),7.61-7.57(3H,m).
MS(ESI)m/z:360$(M+H)^+$.

(5) (R)-3-tert-Butoxycarbonylamino-4-oxo-4-[5-[(1-phenyl-5-trifluoromethyl-1H-pyrazol-3-yl)methoxy]indolin-1-yl]butyric acid tert-butyl ester

[F94]

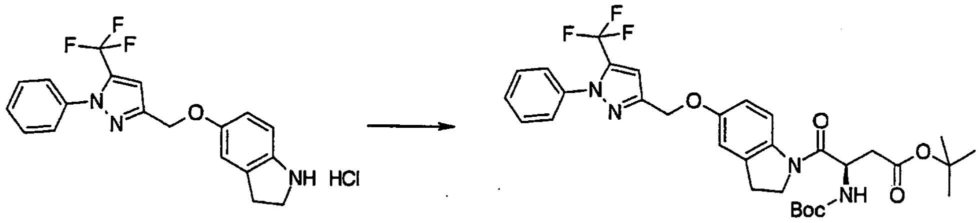

TEA (0.157 mL) was added at room temperature to a DMF solution (2 mL) of 5-[(1-phenyl-5-trifluoromethyl-1H-pyrazol-3-yl)methoxy]indoline hydrochloride (89 mg), Boc-D-Asp(OtBu)-OH (65.1 mg), EDC·HCl (64.7 mg), and HOBt (45.6 mg), and the mixture was stirred at room temperature for 14 hours. The resultant mixture was concentrated under reduced pressure. The residue was purified by silica gel flash column chromatography (Biotage 25S), to thereby yield the title compound (85.0 mg).
NMR($CDCl_3$)δ:
1.42(9H,s),1.43(9H,s),2.58(1H,dd,J=15.6,5.9Hz),2.83(1H,q,J=7. 7Hz),3.20(2H,t,J=8.4Hz),4.26-4.47(1H,m),4.88-4.98 (1H,m),5.12(3H,s),5.28(1H,d,J=9.0Hz),6.78-6.96(3H,m),7.47-7.51(5H,m),8.13(1H,d,J=8.8Hz).
MS (ESI) m/z : 631 $(M+H)^+$.

(6) (R)-3-Amino-4-oxo-4-[5-[(1-phenyl-5-trifluoromethyl-1H-pyrazol-3-yl)methoxy]indolin-1-yl]butyric acid hydrochloride

[F95]

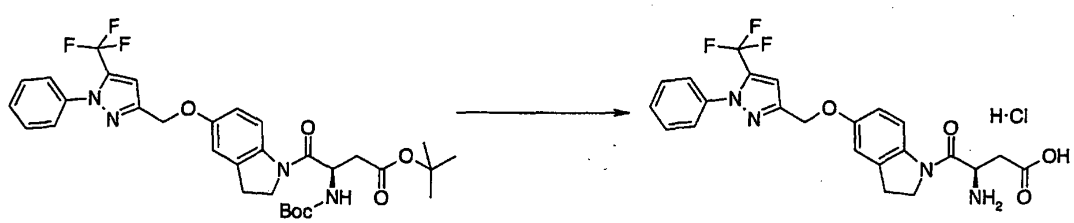

4N HCl/1,4-dioxane (10 mL) and (R)-3-tert-butoxycarbonylamino-4-oxo-4-[5-[(1-phenyl-5-trifluoromethyl-1H-pyrazol-3-yl)methoxyindolin-1-yl]butyric acid tert-butyl ester (85 mg) were combined and the solution was stirred at room temperature for 24 hours. The reaction mixture was concentrated under reduced pressure, and ether was added to the residue. The precipitated solid was collected by filtration and dried under reduced pressure, to thereby yield the title compound (74 mg). NMR(DMSO-$d_6$)δ:
2.74(1H,dd,J=17.3,7.7Hz),3.06(1H,dd,J=17.4,4.9Hz),3.18(1H,t,J =8.3Hz),3.38(1H,d,J=6.6Hz),4.14-4.31(2H,m),4.46 (1H,dd,J=7.4,5.1Hz),5.13(2H,s),6.92(1H,dd,J=8. 8,2.7Hz),7.05(1H,d,J=2.5Hz),7.25(1H,s),7.51-7.62(5H,m),8.02(1H,d, J=8.8Hz).
IR(ATR)$cm^{-1}$:2881,1724,1651,1595,1487,1429,1390,1365,1290.
MS(ESI)m/z:475$(M^++H)^+$.
HRMS (FAB) Calcd for $C_{23}H_{22}F_3N_4O$:475.1593.Found:475.1605. Anal. Calcd for $C_{23}H_{21}F_3N_4O \cdot 1HCl \cdot 0.75H_2O$:C,52.68;H,4.52;Cl,6.76;F,10.87;N,10.6 8.Found:C,52.61;H,4.47;Cl,7.16;F,10.68;N, 10.70.

[Example 16]

(R)-3-Amino-4-oxo-4-[5-(2-nitro-4-biphenylmethoxy)indolin-1-yl]butyric acid hydrochloride

(1) (2-Nitrobiphenyl-4-yl)methanol

[F96]

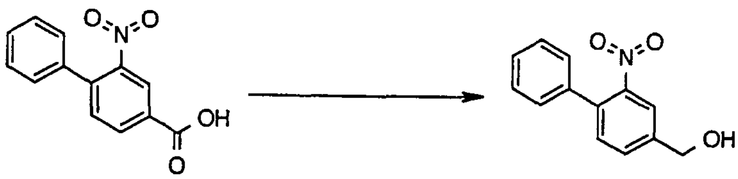

To a THF solution (15 mL) of 2-nitrobiphenyl-4-carboxylic acid (350 mg), TEA (301 μL) was added, and the mixture was cooled on ice. Ethyl chlorocarbonate (165 μL) was added thereto, and the resultant mixture was stirred under cooling on ice for 2 hours. The formed precipitates were removed by filtration, whereby a filtrate was obtained. Separately, sodium boron hydride (327 mg) was suspended in ethanol (4.3 mL), followed by cooling to 0°C. The thus-obtained filtrate was added dropwise to the suspension over 10 minutes, followed by stirring at room temperature for 50 minutes. To the reaction mixture, 1N hydrochloric acid was added, followed by extracting twice, each with ethyl acetate. The extracts were washed sequentially with 1N aqueous sodium hydroxide solution and saturated brine, followed by drying over sodium sulfate anhydrate and concentration. The residue was purified by flash column chromatography (Yamazen Hi-Flash column L), to thereby yield the title compound (295 mg). NMR ($CDCl_3$) δ:
1.95 (1H, s), 4.83(2H,s), 7.30-7.45(6H,m), 7. 60-7.63(1H,m),7.87(1H,d,J=0.7Hz).
MS (ESI) m/z :230 $(M+H)^+$.

(2) 4-Chloromethyl-2-nitrobiphenyl

[F97]

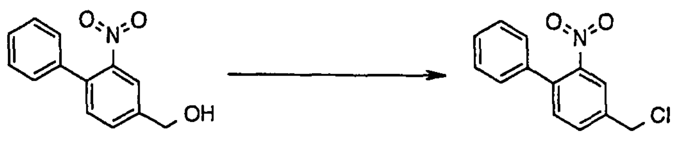

To a dichloroethane solution (15 mL) of 2-nitro-4-biphenylmethanol (290 mg), thionyl chloride (458 μL) was added. The mixture was stirred at 50°C for 3.5 hours and left to stand to cool to room temperature. The reaction mixture was concentrated, and the residue was purified by flash column chromatography (Yamazen Hi-Flash column L), to thereby yield the title compound (299 mg).
NMR ($CDCl_3$) δ:

4.66(2H,s),7.30-7.32(2H,m),7.41-7.46(4H,m), 7.64(1H,dd,J=1.7,7.8Hz),7.89(1H,d,J=1.9Hz).

(3) 1-(tert-Butoxycarbonyl)-5-(2-nitrobiphenyl-4-ylmethoxy)indoline

[F98]

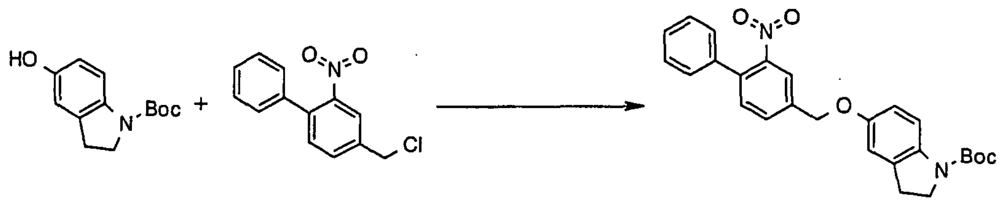

To a DMF solution (5 mL) of 1-tert-butoxycarbonyl-5-hydroxyindoline (150 mg), 4-chloromethyl-2-nitrobiphenyl (189 mg) and potassium carbonate (132 mg) were added. The mixture was stirred at 70°C overnight and left to stand to cool to room temperature. To the reaction mixture, saturated aqueous sodium bicarbonate solution was added, followed by extracting twice, each with ethyl acetate. The extracts were combined and washed with saturated brine, followed by drying over sodium sulfate anhydrate and concentrating. The residue was purified by flash column chromatography (Yamazen Hi-Flash column L), to thereby yield the title compound (287 mg).
NMR ($CDCl_3$) δ:
1.55(9H, s), 3.07(2H, t, J=8.7Hz), 3.98(2H, s), 5.12(2H, s), 6.77-6.83(2H,m),7.31-7.46(7H,m),7.65-7.93(2H,m).

(4) 5-(2-Nitrobiphenyl-4-ylmethoxy)indoline hydrochloride

[F99]

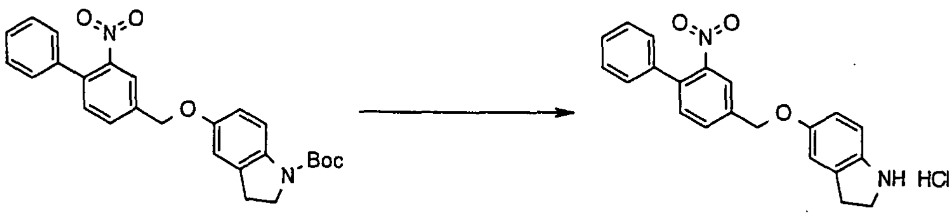

To 1-tert-butoxycarbonyl-5-(2-nitro-4-biphenylmethoxy)indoline (280 mg), 4N HCl/1,4-dioxane (10 mL) was added, and the mixture was stirred at room temperature for 1 hour. Diethyl ether was added to the reaction mixture, and the precipitated solid was collected by filtration, followed by drying, to thereby yield the title compound (209 mg).
NMR (DMSO-$d_6$) δ:
3.18(2H,t,J=7.8Hz), 3.71(2H,t,J=7.8Hz), 5.30(2H,s), 7.05(1H,dd,J =2.6,8.7Hz), 7.18(1H,d,J=2.6Hz), 7.33-7.49(6H,m), 7.60(1H,d,J=8.1Hz),7.83(1H,dd,J=1.7,7.8Hz),8.07(1H ,d,J=1.7Hz), 11.16(1H,s).
MS (ESI) m/z : 347 $(M+H)^+$.

(5) (R)-3-tert-Butoxycarbonylamino-4-[5-(2-nitro-4-biphenylmethoxy)indolin-1-yl]-4-oxobutyric acid tert-butyl ester

[F100]

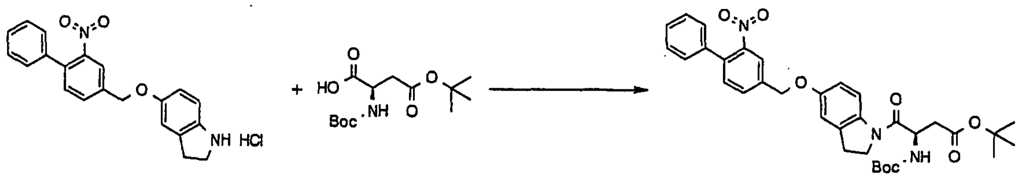

Boc-D-Asp(OtBu)-OH (181 mg), HOBt (106 mg), EDC-HCl (150 mg), and TEA (364 μL) were added to a DMF solution (10 mL) of 5-(2-nitro-4-biphenylmethoxy)indoline hydrochloride (200 mg), and the mixture was stirred overnight. To the reaction mixture, saturated aqueous sodium bicarbonate solution was added, followed by extraction twice, each with ethyl acetate. The extracts were combined and washed with saturated brine, followed by drying over sodium sulfate anhydrate and concentrating. The residue was purified by flash column chromatography (Yamazen Hi-Flash column L), to thereby yield the title compound (309 mg).
NMR ($CDCl_3$ ) δ :
1.42(9H,s), 1.43(9H,s), 2.58(1H,dd,J=6.1,15.7Hz), 2.83(1H,dd,J=7 , 8,15.7Hz), 3.21(2H,t,J=8.3Hz), 4.30-4.39(2H,m), 4.89-4.95(1H,m), 5.14(2H,s), 5.27(1H,d,J=9.3Hz), 6.79-6.86(2H,m),7.26-7.47(6H,m), 7.66(1H,dd,J=1.3,8.0Hz), 7.93 (1H,d,J=1.3Hz), 8.14(1H ,d,J=8.8Hz).
MS (ESI) m/z : 618 $(M+H)^+$.

[0414] (6) (R)-3-Amino-4-[5-(2-nitro-4-biphenylmethoxy)indolin-1-yl)-4-oxobutyric acid hydrochloride

[F101]

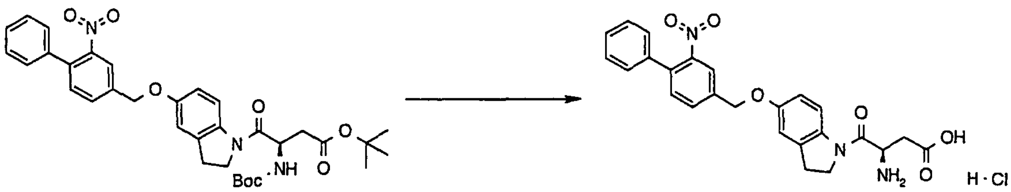

To (R)-3-tert-butoxycarbonylamino-4-[5-(2-nitrobiphenyl-4-ylmethoxy)indolin-1-yl]-4-oxobutyric acid tert-butyl ester (300 mg), 4N HCl/1,4-dioxane (10 mL) was added, and the mixture was stirred overnight at room temperature. The reaction mixture was concentrated under reduced pressure, and the residue was suspended in diethyl ether. The suspended precipitates were collected by filtration and dried, to thereby yield the title compound (205 mg).
NMR (DMSO-$d_6$) δ:
2.76-2.87(1H,m), 3.04(1H,dd,J=5.8,17.5Hz), 3.18(2H,t,J=8.3Hz), 4.17-4.45(3H,m), 5.25(2H,s), 6.92(1H,dd, J=2.5,8.8Hz), 7.06(1H,d,J=2.5 Hz),7.34-7.49(5H,m), 7.59(1H,d,J=7.8Hz), 7.81(1H,dd,J=1.6,8.0Hz), 8.01-8.05(2H,m), 8.52(2H, s).
IR(ATR)$cm^{-1}$:2861,1724,1643,1527,1484,1184.MS(ESI)m/z:462$(M+H)^+$.
HRMS (FAB) calcd for $C_{25}H_{24}N_3O_6$ $(M+H)^+$: 462. 1665; Found 462.1662.

Anal. Calcd for $C_{25}H_{24}ClN_3O_6$·
$0.5H_2O$:C,59.23; H,4.97; Cl,6.99; N,8.29. Found:C,59.25; H,5.05;C1, 7.55;N,8.26.

[Example 17]

(R)-3-Amino-4-[6-[2-(4-biphenyl)ethoxy]indolin-1-yl] -4-oxobutyric acid

(1) 6-Benzyloxy-1-(tert-butoxycarbonyl)-1H-indole

[F102]

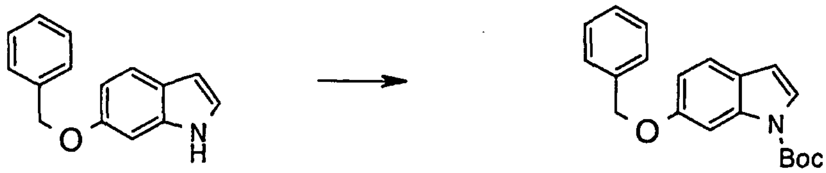

To a solution of 6-benzyloxy-1H-indole (1.00 g) in THF (50 mL), $Boc_2O$ (1.45 g) and DMAP (50 mg) were added, and the mixture was stirred at room temperature for 3 days. The reaction mixture was concentrated, and the residue was purified by silica gel flash column chromatography (Yamazen Hi-Flash L), to thereby yield the title compound (1.43 g).
NMR ($CDCl_3$) δ:
1.65 (9H, s), 5.13(2H,s ), 6.49(1H, dd, J=3.8,0.6Hz), 6.95(1H, dd, J=8. 7,2.3Hz), 7.28-7.35 (1H,m), 7.37-7.42(3H,m), 7.43-7.49(4H,m).
MS (ESI) m/z : 324 $(M+H)^+$.

[0421] (2) 1-(tert-Butoxycarbonyl)-6-hydroxyindoline

[F103]

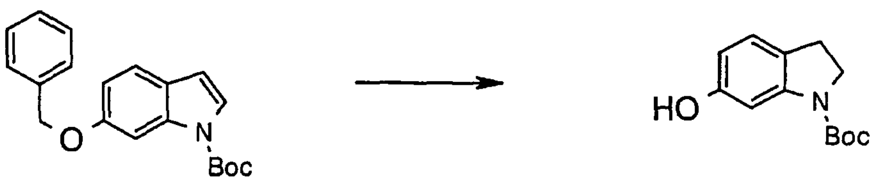

6-Benzyloxy-1-(tert-butoxycarbonyl)-1H-indole (1.43 g) was dissolved in ethanol (20 mL), and 5% Pd/C was added to the solution. The reaction mixture was subjected to catalytic hydrogenation with stirring for 1 day. The catalyst was removed by filtration, and the filtrate was concentrated. The residue was purified by silica gel flash column chromatography (Yamazen Hi-Flash L), to thereby yield the title compound (1.15 g).
NMR ($CDCl_3$) δ:
1.55(9H,s), 2.98(2H,t,J=8.8Hz), 3.97(2H,t,J=8.3Hz),5.20-5.71(1H,m), 6.43(1H,dd,J=8.1,2.2Hz), 6.96(1H,d,J=8.5Hz), 7.55-7.34(1H,m).
MS (ESI) m/z : 236 $(M+H)^+$.

(3) 6-[2-(4-Biphenyl)-2-oxo]ethoxy-1-(tert-butoxycarbonyl)indoline

[F104]

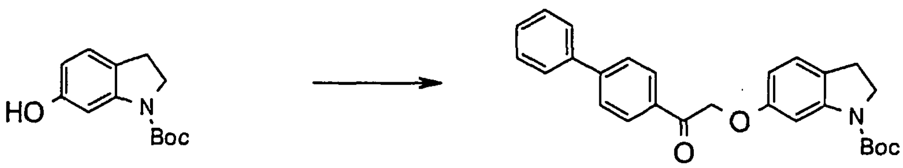

1-(tert-Butoxycarbonyl)-6-hydroxyindoline (400 mg), 2-bromo-4'-phenylacetophenone (515 mg), potassium carbonate (470 mg), and DMF (10 mL) were mixed, and the mixture was stirred at 70°C for 12 hours. The reaction mixture was diluted with ethyl acetate (200 mL), and the organic layer was washed with saturated brine. The washed layer was dried over sodium sulfate anhydrate, and solvent was removed by evaporation. The residue was purified by silica gel flash column chromatography (Yamazen Hi-Flash L), to thereby yield the title compound (487 mg).
NMR ($CDCl_3$) δ:
1.54(9H,s),3.01(2H,t,J=8.7Hz),3.98(2H,t,J=8.7Hz),5.27(2H,s),6 .56(2H,br s),7.02(1H,d,J=8.3Hz), 7.38-7.43(1H,m), 7.45-7.51(2H,m),7.61-7.65(2H,m), 7.71(2H,d,J=8.5Hz), 8.08(2H,d,J=8.3Hz).
MS (ESI) m/z :430 $(M+H)^+$.

(4) 6-[2-(4-Biphenyl)-2-hydroxy]ethoxy-1-(tert-butoxycarbonyl)indoline

[F105]

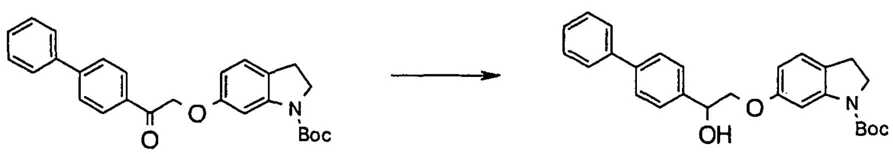

6-[2-(4-Biphenyl)-2-oxoethoxy]-1-(tert-butoxycarbonyl)indoline (478 mg) was dissolved in ethanol (20 mL), and 5% Pd/C (500 mg) was added to the solution. The reaction mixture was subjected to catalytic hydrogenation with stirring for 1.5 hours. The catalyst was removed by filtration, and the filtrate was concentrated. The residue was purified by silica gel flash column chromatography (Yamazen Hi-Flash L), to thereby yield the title compound (402 mg).
NMR ($CDCl_3$) δ :
1.54(9H,br s), 2.83(1H,s), 3.01(2H,t,J=8.5Hz), 3.93-4.20(4H,m),5.15(1H,dt,J=8.1,2.9Hz), 6.52(1H,dd,J=8.1,2.2Hz), 7. 01(1H,d,J=8.1Hz),7.35(1H,tt,J=7.07,1.2Hz),7.42-7.47(2H,m),7.52(3H,d,J=8.3Hz),7.63-7.58(4H,m).
MS (ESI) m/z : 432 $(M+H)^+$.

(5) 6-[2-(4-Biphenyl)ethoxy]-1-(tert-butoxycarbonyl)indoline

[F106]

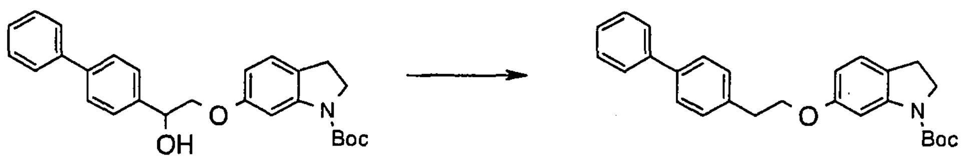

6-[2-(4-Biphenyl)-2-hydroxy]ethoxy-1-(tert-butoxycarbonyl)indoline (402 mg) was dissolved in dichloromethane (10 mL), and to the solution, TEA (313 μL) and trifluoroacetic acid anhydride (156 μL) were added sequentially with stirring at 0°C, followed by stirring at the same temperature for 12 hours. The reaction mixture was concentrated. To the concentrate, ethyl acetate (20 mL) and 10% Pd/C (500 mg) were added, and catalytic hydrogenation was conducted for 3 days. The catalyst was removed by filtration, and the filtrate was concentrated. The residue was purified by silica gel flash column chromatography (Yamazen Hi-Flash 2L), to thereby yield the title compound (198 mg).
MS (ESI) m/z : 416 $(M+H)^+$.

(6) 6-[2-(4-Biphenyl)ethoxy]-indoline hydrochloride

[F107]

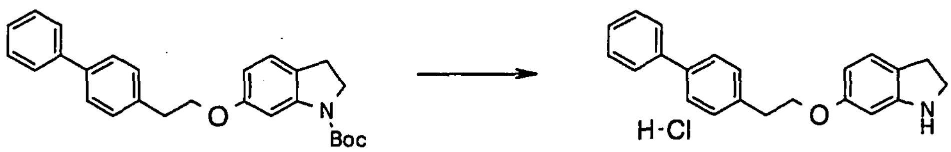

To 6-[2-(4-biphenyl)ethoxy]-1-(tert-butoxycarbonyl)indoline (198 mg), 4N HCl/1,4-dioxane (10 mL) was added, and the mixture was stirred for 4 hours. The reaction mixture was concentrated, and the solid was dried in vacuo for 1 day, to thereby yield the title compound (167 mg).
MS (ESI) m/z : 316 $(M+H)^+$.

(7) (R)-4-[6-[2-(4-Biphenyl)ethoxy]indolin-1-yl]-3-(tert-butoxycarbonylamino)-4-oxobutyric acid tert-butyl ester

[F108]

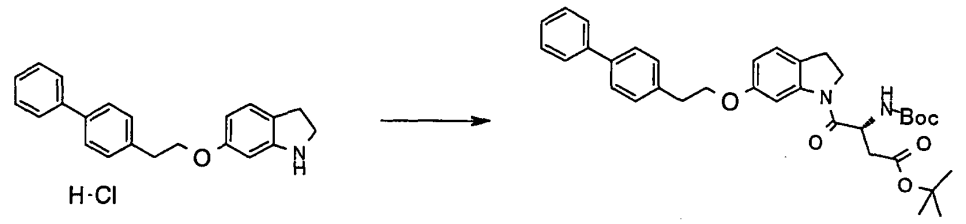

6-[2-(4-Biphenyl)ethoxy]indoline hydrochloride (216 mg) and Boc-D-Asp(OtBu)-OH (213 mg) were dissolved in DMF (10 mL), and EDC·HCl (177 mg), HOBt (126 mg), and TEA (428 μL) were added to the solution, followed by stirring for 12 hours. The reaction mixture was diluted with ethyl acetate (200 mL). The organic layer was washed with saturated brine and dried over sodium sulfate anhydrate, and solvent was removed by evaporation. The residue was purified by silica gel flash column chromatography (Yamazen Hi-Flash L), to thereby yield the title compound (198 mg).
NMR ($CDCl_3$) δ:
1.37-1.46(18H,m),2.56 (1H,dd,J=15.62,6.10Hz), 2.82(1H,dd,J=15.87,8.06Hz), 3.07-3.17(4H,m),4.15-4.42(4H,m),4.92 (1H,q,J=7.3Hz), 5.23(1H,d,J=9.0Hz), 6.62(1H,dd,J =8.3,2.4Hz), 7.05(1H,d,J=8.3Hz), 7.32-7.37(3H,m), 7.40-7.46(2H, m), 7.51-7.55(2H,m), 7.60-7.56(2H,m),7.91(1H,d,J=2.2Hz).
MS (ESI) m/z : 587 $(M+H)^+$.

(8) (R)-3-Amino-4-[6-[2-(4-biphenyl) ethoxy]-indolin-1-yl]-4-oxobutyric acid hydrochloride

[F109]

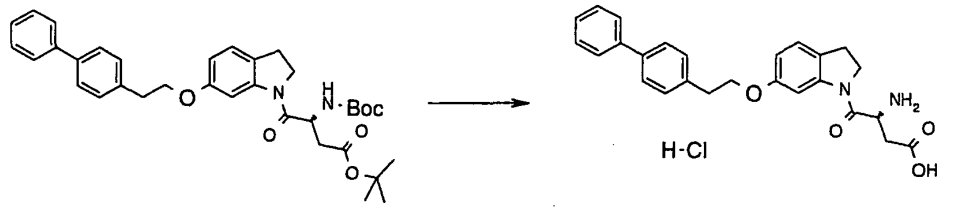

(R)-4-[6-[2-(4-Biphenyl)ethoxy]-indolin-1-yl]-3-(tert-butoxycarbonylamino)-4-oxobutyric acid tert-butyl ester (190 mg) and 4N HCl/1,4-dioxane (10 mL) were mixed, and the mixture was stirred for 1 day. The reaction mixture was concentrated, and the concentrate was triturated with dichloromethane and hexane, to thereby yield the title compound (98 mg).
NMR ($CDCl_3$) δ:
2.71-3.22(6H,m), 3.61-4.32(4H,m), 4.77-4.97(1H,m), 6.40-6.53(1H,m), 6.79-6.93(1H,m), 7.15-7.49(9H,m), 7.64-7.77 (1H,m),8.45-8.11(4H,m).
MS (ESI) m/z:431 $(M+H)^+$.
HRMS (ESI) Calcd for $C_{26}H_{27}N_2O_4$ $M^+$+H:431. 19708.Found 431. 19449. Anal. Calcd for $C_{26}H_{26}N_2O_4$·HCl·1.5$H_2O$:C,63.22; H,6.12; N,5.67.Found:C,63.51;H,5.86;N,5.57.

[Example 18]

(R)-3-Amino-4-[4-[2-(4-biphenyl)ethoxy]-indolin-1-yl]-4-oxobutyric acid

(1) 4-[2-(4-Biphenyl)-2-oxo]ethoxy-1-(tert-butoxycarbonyl)indoline

[F110]

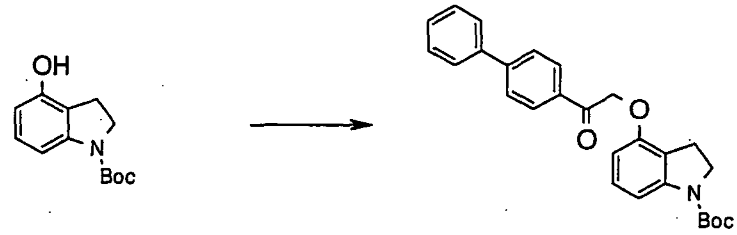

1-(tert-Butoxycarbonyl)-4-hydroxyindoline (482 mg), 2-bromo-4'-phenylacetophenone (620 mg), potassium carbonate (566 mg), and DMF (10 mL) were mixed, and the mixture was stirred at 70°C for 12 hours. The reaction mixture was diluted with ethyl acetate (200 mL). The organic layer was washed with saturated brine and dried over sodium sulfate anhydrate, and solvent was removed by evaporation. The residue was purified by silica gel flash column chromatography (Yamazen Hi-Flash L), to thereby yield the title compound (621 mg).
NMR ($CDCl_3$) δ :
1.55(9H,s), 3.11(2H,t,J=8.8Hz), 4.00(2H,t,J=8.8Hz), 5.30 (2H,s), 6.42(1H,d,J=8.5Hz), 7.09(1H,t,J=7.9Hz), 7.39-7.44 (1H,m), 7.45-7.51(2H,m), 7.61-7.65(2H,m),7.71(2H,d,J=8.3Hz),8.07(2H,d,J=8.3Hz).
MS (ESI) m/z :430 $(M+H)^+$.

(2) 4-[2-(4-Biphenyl)ethoxy]-1-(tert-butoxycarbonyl)indoline

[F111]

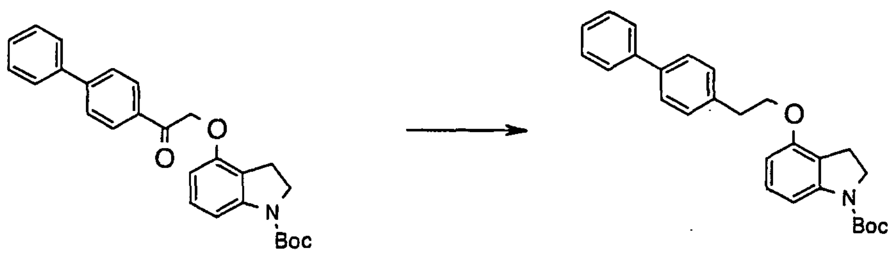

4-[2-(4-Biphenyl)-2-oxo]ethoxy-1-(tert-butoxycarbonyl)indoline (621 mg) was dissolved in ethanol (20 mL), and 5% Pd/C (620 mg) was added to the solution. The mixture was subjected to catalytic hydrogenation with stirring for 20 hours. The catalyst was removed by filtration, and the filtrate was concentrated. The residue was purified by silica gel flash column chromatography (Yamazen Hi-Flash L), to thereby yield the title compound (411 mg).
NMR ($CDCl_3$) δ:
1.55(9H,s),2.98(2H,t,J=8.7Hz), 3.12(2H,t,J=6.8Hz),3.97(2H,t,J= 8.5Hz),4.23(2H,t,J=6.8Hz),6.49(1H,d,J=8.8H,m), 7.46-7.40(2H,m),7.60-7.52(4H,m).
MS (ESI)m/z:416 $(M+H)^+$.

(3) 4-[2-(4-Biphenyl)ethoxy]indoline hydrochloride

[F112]

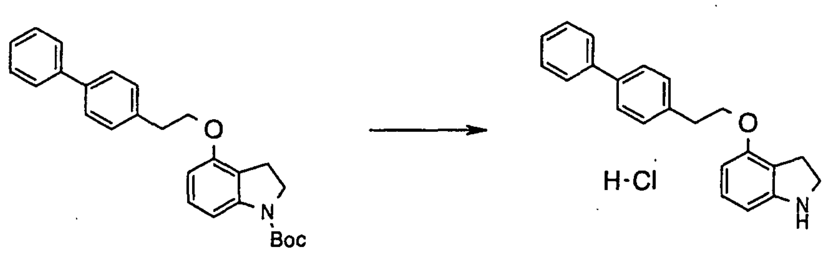

To 4-[2-(4-biphenyl)ethoxy]-1-(tert-butoxycarbonyl)indoline (411 mg), 4N HCl/1,4-dioxane (10 mL) was added, and the mixture was stirred for 12 hours. The reaction mixture was concentrated. The formed solid was dried in vacuo for 1 day, to thereby yield the title compound (320 mg).
NMR (DMSO-$d_6$) δ:
3.03(2H,t,J=7.8Hz),3.09(2H,t,J=6.6Hz),3.68(2H,t,J=7.9Hz),4.30 (2H,t,J=6.7Hz),6.94(1H,d,J=7.8Hz),7.02(1H,d, J=8.3Hz),7.28-7.37(2H,m),7.39-7.48(4H,m),7.68-7.59(4H,m).
MS(ESI) m/z : 316 $(M+H)^+$.

(4) (R)-4-[4-[2-(4-Biphenyl)ethoxy]indolin-1-yl]-3-(tert-butoxycarbonylamino)-4-oxobutyric acid tert-butyl ester

[F113]

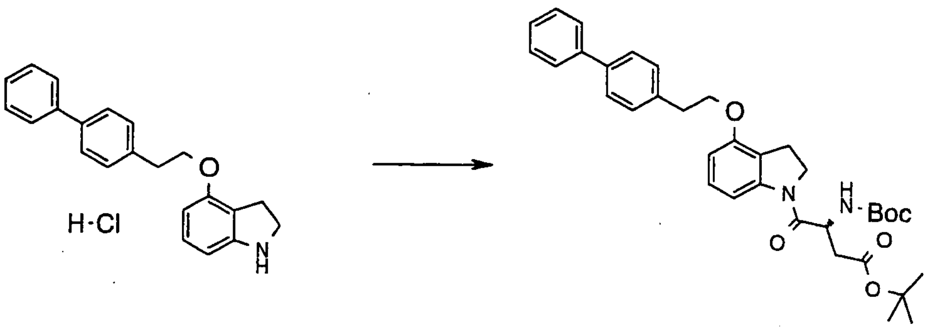

4-[2-(4-Biphenyl)ethoxy]indoline hydrochloride (320 mg) and Boc-D-Asp(O-tBu)-OH (316 mg) were dissolved in DMF (10 mL), and EDC·HCl (262 mg), HOBt (184 mg), and TEA (380 μL) were added to the solution, followed by stirring for 12 hours. The reaction mixture was diluted with ethyl acetate (200 mL). The organic layer was washed with saturated brine and dried over sodium sulfate anhydrate, and solvent was removed by evaporation. The residue was purified by silica gel flash column chromatography (Yamazen Hi-Flash L), to thereby yield the title compound (198 mg).
NMR ($CDCl_3$) δ:
1.41(9H,s),1.43(9H,s),2.56(1H,dd,J=15.9,5.9Hz),2.82(1H,dd,J=1 5.9,7.6Hz),3.07-3.15(4H,m),4.20-4.41(4H,m), 4.87-4.97 (1H, m), 5.27-5.34 (1H, m), 6.59 (1H, d, J=8.3Hz), 7.14 (1H, t, J=8.2Hz), 7.31-7.37 (3H, m), 7.43 (2H, t, J=7.3Hz), 7.60-7.52(4H,m),7.81(1H,d,J=8.1Hz).
MS (ESI) m/z : 587 $(M+H)^+$.

(5) (R)-3-Amino-4-[4-[2-(4-biphenyl)ethoxy]indolin-1-yl]-4-oxobutyric acid hydrochloride

[F114]

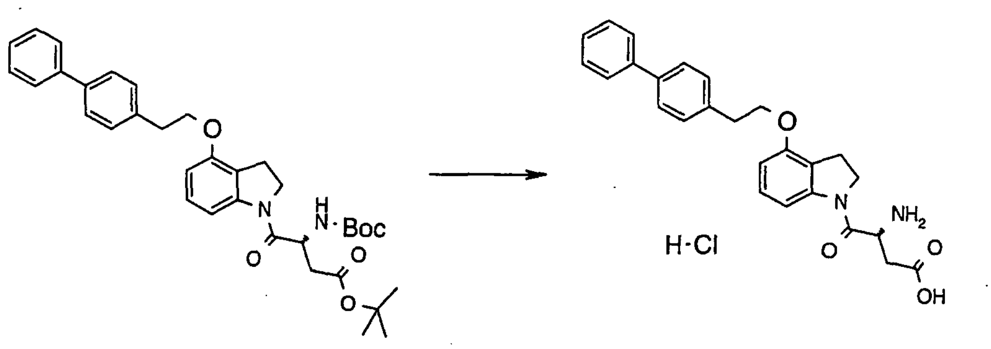

(R) -4- [4- [2- (4-Biphenyl) ethoxy] -indolin-1-yl] -3- (tert-butoxycarbonylamino)-4-oxobutyric acid tert-butyl ester (170 mg) and 4N HCl/1,4-dioxane (10 mL) were mixed, and the mixture was stirred for 1 day. The reaction mixture was concentrated, and dichloromethane and hexane were added thereto. The precipitated solid was collected by filtration and dried, to thereby yield the title compound (18 mg).
MS (ESI) m/z :431 $(M+H)^+$.

[Example 19]

(R)-3-Amino-4-oxo-4-[5-[2-(4-phenyl-5-trifluormethyl-2-thienyl)ethyl]indolin-1-yl]butyric acid hydrochloride

(1) 1-(tert-Butoxycarbonyl)-5-methoxycarbonyl-1H-indole

[F115]

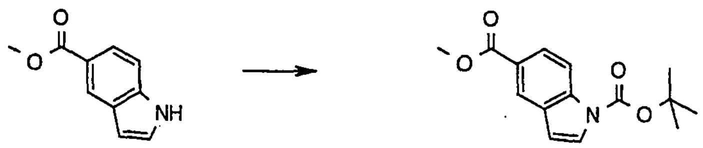

To a solution of 5-methoxycarbonyl-1H-indole (2.00 g) in THF (50 mL), DMAP (140 mg) and $Boc_2O$ (2.74 g) were added at room temperature. The mixture was stirred at room temperature for 2 hours, and the reaction mixture was concentrated under reduced pressure. The residue was purified by silica gel flash column chromatography (Biotage 40M), to thereby yield the title compound (3.10 g).
NMR($CDCl_3$) δ:
1.68(9H,s),3.94(3H,s),6.64(1H,d,J=3.8Hz),7.64(1H,d,J=3.8Hz),8 .01(1H,dd,J=8.8,1.7Hz),8.18(1H,d,J=8.8Hz),8.30 (1H,d,J=1.7Hz).
MS (ESI) m/z : 276 $(M+H)^+$

(2) 1-(tert-Butoxycarbonyl)-5-methoxycarbonylindoline

[F116]

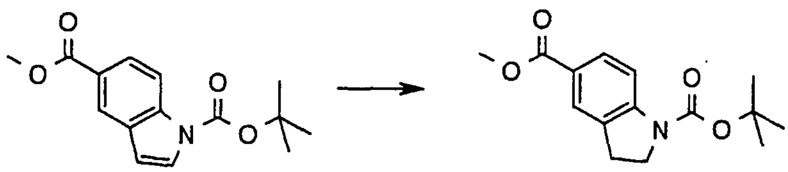

To a solution of 1-(tert-butoxycarbonyl)-5-methoxycarbonyl-1H-indole (3.10 g) in ethanol (50 mL), 5% Pd/C (1.00 g) was added at room temperature. In a hydrogen atmosphere, the mixture was stirred at room temperature for 15 hours. The reaction mixture was filtered, and the filtrate was concentrated under reduced pressure. The residue was purified by silica gel flash column chromatography (Biotage 40M), to thereby yield the title compound (1.64 g).
NMR ($CDCl_3$) δ:
1.58(9H,s), 3.12(2H,t,J=8.5Hz), 3.89(3H,s), 4.03(2H,t,J=8.5Hz), 7 .80-7.83(1H,m), 7.89(1H,d,J=6.8Hz
MS (ESI) m/z :278 $(M+H)^+$

(3) 1-(tert-Butoxycarbonyl)-5-hydroxymethylindoline

[F117]

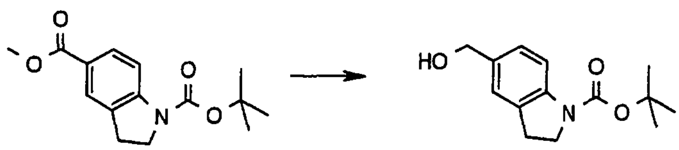

To a solution of 1-(tert-butoxycarbonyl)-5-methoxycarbonylindoline (627 mg) in dichloromethane (10 mL), diisobutylaluminum hydride (1.0N toluene solution) (5.65 mL) was added at -78°C, and the mixture was stirred at -78°C for 30 minutes. To the reaction mixture, saturated aqueous ammonium chloride solution (0.80 mL) was added, and the resultant mixture was allowed to warm to room temperature. Diethyl ether (60 mL) was added thereto, followed by stirring for 30 minutes. To the mixture, magnesium sulfate anhydrate was added, followed by stirring for 30 minutes. The resultant mixture was filtered through a Celite pad, and the filtrate was concentrated under reduced pressure. The residue was purified by silica gel flash column chromatography (Biotage 40M), to thereby yield the title compound (504 mg).
NMR ($CDCl_3$) δ :
1.52(1H,t,J=5.9Hz), 1.56(9H,s), 3.08(2H,t,J=8.8Hz), 3.98(2H,t,J= 8.8Hz), 4.61(2H,d,J=5.9Hz),7.14(1H,d,J=8.3Hz),7.17 (1H,s),7.23-8.00(1H,br).
MS (ESI) m/z : 232 $(M-OH)^+$.

(4) 1-(tert-Butoxycarbonyl)-5-formylindoline

[F118]

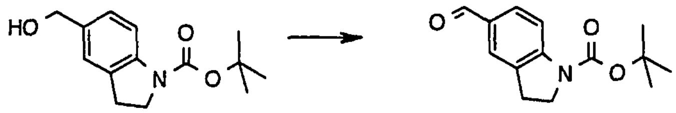

To a solution of 1-(tert-butoxycarbonyl)-5-hydroxymethylindoline (390 mg) in THF (10 mL), sodium chloride (360 mg) and manganese dioxide (820 mg) were added at room temperature, and the reaction mixture was stirred at room temperature for 15 hours. Manganese dioxide (820 mg) was further added thereto, followed by stirring for 24 hours. The reaction mixture was filtered through a Celite pad, and the filtrate was concentrated under reduced pressure. The residue was purified by silica gel flash column chromatography (Biotage 25M), to thereby yield the title compound (318 mg). NMR ($CDCl_3$ ) δ :
1.59(9H,s), 3.15(2H,t,J=8.8Hz), 4.06(2H,t,J=8.8Hz), 7.60-8.10(1H,br), 7.68(1H,s), 7.69(1H,d,J=6.8Hz), 9.86(1H,s).
MS (ESI) m/z:248(M+H)$^+$

(5) 1-(tert-Butoxycarbonyl)-5-[(E)-2-(4-phenyl-5-trifluormethyl-2-thienyl)ethenyl]indoline

[F119]

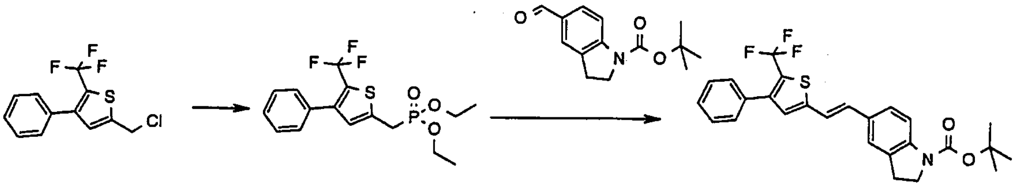

To 5-(chloromethyl)-3-phenyl-2-(trifluormethyl)thiophene (500 mg), triethyl phosphite (650 mL) was added at room temperature, and the mixture was stirred at 150°C for 5 hours. The reaction mixture was cooled to room temperature, and concentrated under reduced pressure. The residue was purified by silica gel flash column chromatography (Biotage 40M), whereby a 3:1 mixture (1.48 g) of triethyl phosphite and (4-phenyl-5-trifluormethyl-2-thienyl)methyl phosphonic acid diethyl ester was obtained. A solution of the mixture (545 mg) in THF (3.0 mL) was added dropwise at 0°C to a 55% suspension of sodium hydride (65.0 mg) in THF (3.0 mL). The resultant mixture was stirred at 0°C for 30 minutes, and 1-(tert-butoxycarbonyl)-5-formylindoline (310 mg) in THF (3.0 mL) was added thereto, followed by stirring at room temperature for 2 hours. To the reaction mixture, water (10 mL), saturated aqueous ammonium chloride solution (20 mL), and ethyl acetate (30 mL) were added for phase separation. The aqueous layer was extracted with ethyl acetate (20 mL). The extracts were combined and washed with saturated brine (30 mL), followed by drying over sodium sulfate anhydrate. Solvent was removed under reduced pressure. The residue was purified by silica gel flash column chromatography (Biotage 40M), to thereby yield the title compound (545 mg).
NMR ($CDCl_3$) δ:
1.58(9H,s), 3.12(2H,t,J=8.8Hz), 4.01(2H,t,J=8.8Hz), 6.97(1H,d,J= 15.9Hz), 6.99(1H,s), 7.05(1H,d,J=15.9Hz), 7.22-7.33 (2H,m), 7.36-7.47 (5H,m), 7.50-8.03 (1H,br).
MS(ESI)m/z:372(M+H-Boc)$^+$

(6) 1-(tert-Butoxycarbonyl)-5-[2-(4-phenyl-5-trifluormethyl-2-thienyl)ethyl]indoline

[F120]

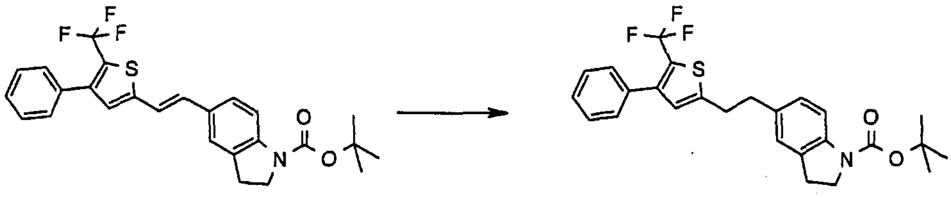

To a solution of 1-(tert-butoxycarbonyl)-5-[(E)-2-(4-phenyl-5-trifluormethyl-2-thienyl)ethenyl]indoline (103 mg) in ethyl acetate (3.0 mL), 5% Pd/C (100 mg) was added at room temperature. The reaction mixture was stirred in a hydrogen atmosphere at room temperature for 3 hours, followed by filtration. The filtrate was concentrated under reduced pressure, to thereby yield the title compound (106 mg). The compound was employed in a subsequent reaction without further purification.
NMR ($CDCl_3$) δ :
1.54(9H,s), 2.94(2H,t,J=8.6Hz), 3.06(2H,t,J=8.6Hz), 3.10(2H,t,J= 7.1Hz),3.97(2H,br t,J=7.1Hz),6.75(1H,d,J=1.2Hz), 6.95-7.05(2H,m),7.33-7.43(5H,m), 7.50-7.95(1H,br).

(7) 5-[2-(4-Phenyl-5-trifluormethyl-2-thienyl)ethyl]indoline hydrochloride

[F121]

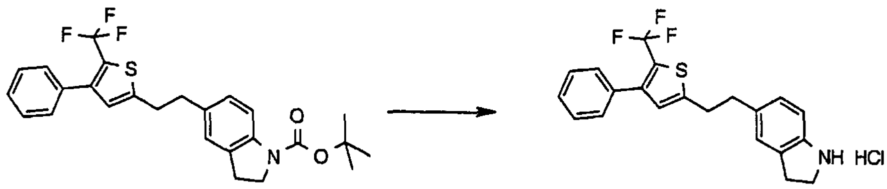

To 1-(tert-butoxycarbonyl)-5-[2-(4-phenyl-5-trifluormethyl-2-thienyl)ethyl]indoline (106 mg), 4N HCl/1,4-dioxane (5.0 mL) was added at room temperature. The reaction mixture was stirred at room temperature for 2 hours and concentrated under reduced pressure, to thereby yield the title compound (100 mg). The compound was employed in a subsequent reaction without further purification.
NMR (DMSO-$d_6$) δ:
3.01(2H,t,J=8.1Hz), 3.14(2H,t,J=8.1Hz), 3.18(2H,t,J=7.4Hz), 3.67 (2H,t,J=7.4Hz),7.08(1H,s), 7.18-7.30(2H,m), 7.30-7.51(5H,m), 10.00-11.00(1H,br).
MS (ESI)m/z:374 $(M+H)^+$.

(8) (R)-3-[(tert-Butoxycarbonyl)amino]-4-oxo-4-[5-[2-(4-phenyl-5-trifluormethyl-2-thienyl)ethyl]indolin-1-yl]butyric acid tert-butyl ester

[F122]

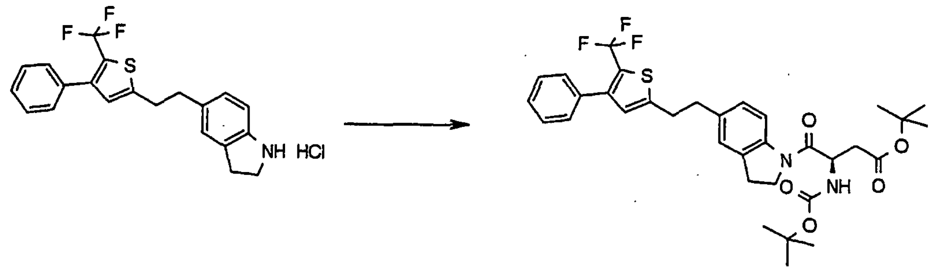

EDC·HCl (63.0 mg), HOBt (44.5 mg), and TEA (0.152 mL) were added at room temperature to a suspension of 5-[2-(4-phenyl-5-trifluormethyl-2-thienyl)ethyl]indoline hydrochloride (100 mg) and Boc-D-Asp(OtBu)-OH (70.0 mg) in DMF (3.0 mL), and the mixture was stirred for 15 hours. The reaction mixture was concentrated under reduced pressure, and to the concentrate, ethyl acetate (30 mL), saturated aqueous sodium hydrogencarbonate solution (20 mL), and water (40 mL) were added for phase separation. The aqueous layer was extracted with ethyl acetate (20 mL). The extracts were combined and dried with sodium sulfate anhydrate. Solvent was removed under reduced pressure. The residue was purified by preparative silica gel thin-layer chromatography, to thereby yield the title compound (33.7 mg). The compound was employed in a subsequent reaction without further purification.
NMR ($CDCl_3$) δ:
1.42(9H,s), 1.43(9H,s), 2.58(1H,dd,J=15.6,5.9Hz), 2.83(1H,dd,J=1 5.6,7.3Hz), 2.97(2H,t,J=7.6Hz), 3.11(2H,t,J=7.6Hz), 3.20(2H,t,J= 8.3Hz), 4.26-4.42(2H,m), 4.87-4.98(1H,m),5.27(1H,d,J=9.5Hz), 6.67(1H,s), 7.01-7.07 (2H,m), 7.30-7.42 (5H,m) , 8.12 (1H,d,J=7.8Hz).
MS(ESI)m/z:533(M+H-isobutene×2)$^+$.

(9) (R)-3-Amino-4-oxo-4-[5-[2-(4-phenyl-5-trifluormethyl-2-thienyl)ethyl]indolin-1-yl]butyric acid hydrochloride

[F123]

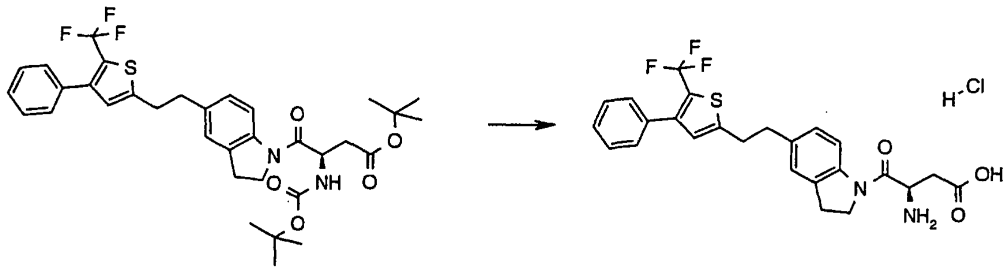

To (R)-3-[(tert-butoxycarbonyl)amino]-4-oxo-4-[5-[2-(4-phenyl-5-trifluormethyl-2-thienyl)ethyl]indolin-1-yl]butyric acid tert-butyl ester (33.7 mg), 4N HCl/1,4-dioxane (5.0 mL) was added at room temperature. The mixture was stirred at room temperature for 15 hours, and the reaction mixture was concentrated under reduced pressure. The solid matter was slurried with diethyl ether-hexane. The slurry was filtered, and the collected solid matter was dried, to thereby yield the title compound (20.5 mg).
NMR (DMSO-$d_6$) δ:
2.73(1H,dd,J=17.6,7.8Hz),2 .97(2H,t,J=7.7Hz),3.05(1H,dd,J=17.6,5.3Hz),3.16(4H,t,J=7.9Hz) ,4.13-4.32(2H,m), 4.45 (1H,t,J=6.2Hz), 7.07(1H,s), 7.12(1H,d,J=7.8Hz),7 .23(1H,s), 7.36-7.52(5H,m), 7.99(1H,d,J=8.3Hz).
MS (ESI) m/z :489 (M+H)$^+$
HRMS(FAB)Calcd for $C_{25}H_{22}F_3N_2O_3$ (M+H)$^+$:489.1460. Found: 489.1462.

[Example 20]

(R)-3-(N,N-Dimethylamino)-4-oxo-4-[5-(2-trifluormethyl-4-biphenyl)methoxy]indolin-1-yl]butyric acid hydrochloride

[F124]

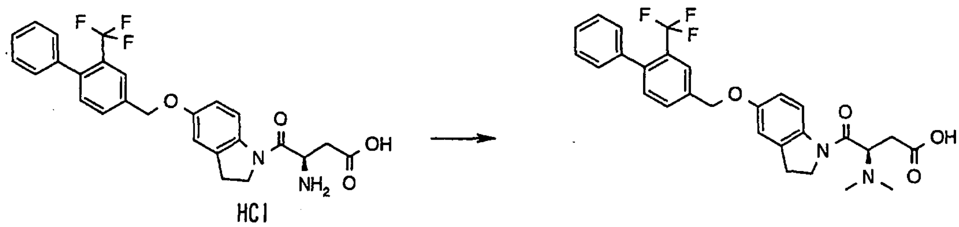

HCl

A 37% aqueous formalin solution (0.144 mL), acetic acid (0.5 mL), and sodium cyanoborohydride (24.1 mg) were added at room temperature to a methanol solution of (R)-3-amino-4-oxo-4-[5-(2-trifluormethyl-4-biphenyl)methoxy] indolin-1-yl]butyric acid hydrochloride (100 mg), and the mixture was stirred for 14 hours. To the reaction mixture, water was added, followed by stirring for 30 minutes. The resultant mixture was extracted with a methanol/chloroform mixture (10%). The extracts were combined and washed with saturated brine, followed by drying over sodium sulfate anhydrate. Sodium sulfate anhydrate was removed by filtration, and the filtrate was concentrated under reduced pressure. The residue was purified by thin-layer chromatography, to thereby yield (R)-3-(N,N-dimethylamino)-4-oxo-4-[5-(2-trifluormethyl-4-biphenyl)methoxy]indolin-1-yl]butyric acid (83 mg).
To (R)-3-(N,N-dimethylamino)-4-oxo-4-[5-(2-trifluormethyl-4-biphenyl)methoxy]indolin-1-yl]butyric acid (83 mg), 4N HCl/ 1,4-dioxane (2 mL) was added at room temperature, and the mixture was stirred for 30 minutes, followed by concentration under reduced pressure. Ether was added to the residue, and the precipitated solid was collected by filtration, to thereby yield the title compound (78 mg).
NMR (DMSO-$d_6$) δ:
2.82(6H,s), 3.09(2H,d,J=6.6Hz), 3.19(2H,t,J=7.4Hz), 4.21(1H,dd,J =18.0,9.7Hz),4.42-4.49(1H,m), 4.60(1H,t,J=6.1Hz), 5.23(2H,s),6.91(1H,dd,J=8.8,2.7 Hz), 7.07-7.03(1H,m), 7.34-7.28(2H,m), 7.39-7.46(5H,m), 7.76(1H,d,J=7.8Hz), 7.88 (1H,d,J=1.2Hz), 8.03(1H,d,J= 8.8Hz).
IR(ATR)$cm^{-1}$:297,2929,2669,2598,1720,1645,1597.
MS (ESI) m/z : 513 $(M+H)^+$.
Anal. Calcd for $C_{28}H_{27}F_3N_2O_4$·1.25HCl· 1.5$H_2O$:C,57.48; H,5.38; Cl, 7.57; F,9.74; N,4.79.Found:C,57.32; H,4. .99; Cl, 7.85; F,9.54; N,4.99.

[Example 21]

(R)-3-Amino-4-oxo-4-[7-[(4-phenyl-5-trifluoromethyl-2-thienyl)methoxy]-2,3-dihydrobenzo[1,4]oxazin-4-yl]butyric acid hydrochloride

(1) 4-(tert-Butoxycarbonyl)-7-hydroxy-2,3-dihydrobenzo[1,4]oxazine

[F125]

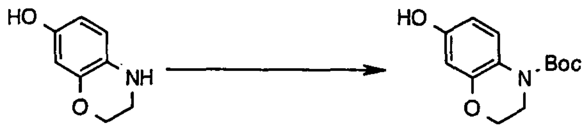

Water (2.5 mL), sodium hydrogencarbonate (252 mg), and $Boc_2O$ (327 mg) were added to a 1,4-dioxane solution (2.5 mL) of 2,3-dihydro-7-hydroxy-4H-benzo[1,4]oxazine (Eur. J. Med. Chem., 1999, 34, 903-917) (151 mg), and the mixture was stirred overnight. To the reaction mixture, water was added for dilution, and the resultant mixture was extracted twice, each with ethyl acetate. The extracts were combined and washed with saturated brine, followed by drying over sodium sulfate anhydrate and concentrating. The residue was purified by flash column chromatography (Yamazen Hi-Flash column L), to thereby yield the title compound (126 mg).
NMR($CDCl_3$) δ:
1.53(9H,s), 3.80-3.82(2H,m), 4.21-4.23(2H,m),4.88(1H,s), 6.36-6.39(2H,m),7.58(1H,s).
MS (ESI)m/z:252 $(M+H)^+$.

(2) 4-tert-Butoxycarbonyl-7-[(4-phenyl-5-trifluoromethyl-2-thienyl)methoxy]-2,3-dihydrobenzo[1,4]oxazine

[F126]

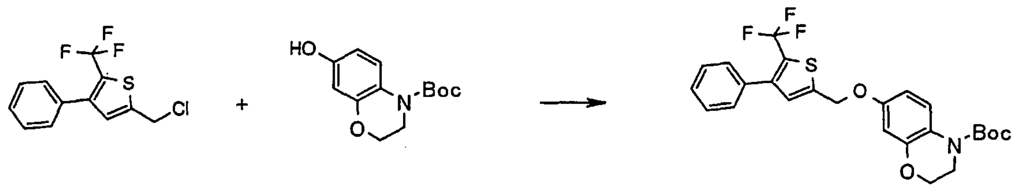

To a solution of 2-chloromethyl-4-phenyl-5-trifluoromethylthiophene (129 mg) and 4-tert-butoxycarbonyl-7-hydroxy-2,3-dihydrobenzo[1,4]oxazine (117 mg) in DMF (5 mL), potassium carbonate (161 mg) was added at room temperature, and the mixture was stirred at 60°C for 6 hours. The reaction mixture was concentrated under reduced pressure, and chloroform (10 mL) and water (10 mL) were added to the concentrate for phase separation. The aqueous layer was extracted with chloroform (5 mL x 2). The extracts were combined and dried over sodium sulfate anhydrate. Solvent was removed under reduced pressure. The residue was purified by silica gel flash column chromatography (Biotage 25S), to thereby yield the title compound (226 mg).
NMR ($CDCl_3$) δ:
1.53(9H,s), 3.83(2H,t,J=4.5Hz), 4.24(2H,t,J=4.5Hz), 5.17(2H,s), 6 .51(1H,d,J=2.9Hz), 6.55(1H,dd,J=9.3,2.9Hz), 7.06 (1H,br s),7.43-7.38 (5H,m), 7.68 (1H,br s).

(3) 7-[(4-Phenyl-5-trifluoromethyl-2-thienyl)methoxy]-2,3-dihydrobenzo[1,4]oxazine hydrochloride

[F127]

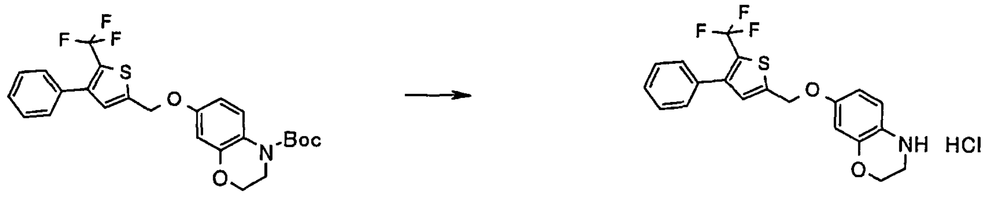

To a solution of 4-tert-butoxycarbonyl-[7-[(4-phenyl-5-trifluoromethyl-2-thienyl)methoxy]-2,3-dihydrobenzo[1,4]oxazine (226 mg) in 1,4-dioxane (1.0 mL), a 4N HCl/1,4-dioxane (2 mL) was added at room temperature, and the mixture was stirred for 14 hours. Solvent was removed under reduced pressure, and to the residue, diethyl ether (3 mL) was added. The precipitated solid was collected by filtration followed by washing with diethyl ether and drying, to thereby yield the title compound (156 mg).
NMR (DMSO-$d_6$) δ :
3.45(2H,t,J=4.64Hz),4.29(2H,t,J=4.64Hz),5.35(2H,s),6.68-6.64(2H,m),7.05(1H,d,J=8.79Hz),7.36(1H,s),7.50-7.41(5H,m).

(4) (R)-3-(tert-Butoxycarbonyl)amino-4-oxo-4-[7-[(4-phenyl-5-trifluoromethyl-2-thienyl)methoxy]-2,3-dihydrobenzo[1,4]oxazin-4-yl]butyric acid tert-butyl ester

[F128]

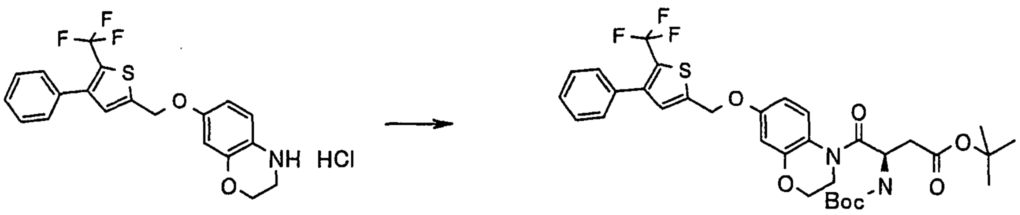

To a solution of Boc-D-Asp(OtBu)-OH (106 mg) in DMF (2 mL), DIEA (59 μL), HOBt (64.1 mg), and EDC·HCl (90.9 mg) were added at room temperature, and the reaction mixture was stirred for 10 minutes. A solution of 7-[(4-phenyl-5-trifluoromethyl-2-thienyl)methoxy]-2,3-dihydrobenzo[1,4]oxazine hydrochloride (156 mg) and DIEA (100 μL) in DMF (1 mL) was added thereto at room temperature, followed by stirring for 14 hours. The reaction mixture was concentrated under reduced pressure, and chloroform (10 mL) and water (5 mL) were added for phase separation. The aqueous layer was extracted with chloroform (5 mL x 3). The extracts were combined and dried over sodium sulfate anhydrate. Solvent was removed under reduced pressure. The residue was purified by silica gel flash column chromatography (Biotage 25S), to thereby yield the title compound (73.4 mg).
NMR ($CDCl_3$) δ :
1.46-1.39(20H,m),3.70(1H,s),4.40-4.22(3H,m),5.18(2H,s),5.37(1H,d,J=9.02Hz),6.60-6.51(2H,m),7.07(1H,s),7.44-7.38(6H,m).

(5) (R)-3-Amino-4-oxo-4-[7-[(4-phenyl-5-trifluoromethyl-2-thienyl)methoxy]-2,3-dihydrobenzo[1,4]oxazin-4-yl]butyric acid hydrochloride

[F129]

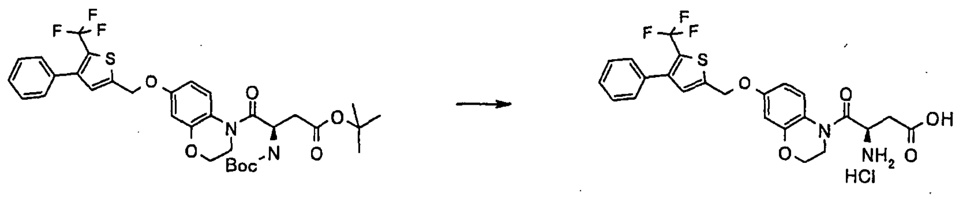

To a solution of (R)-3-(tert-butoxycarbonyl)amino-4-oxo-4-[7-[(4-phenyl-5-trifluoromethyl-2-thienyl)methoxy]-2,3-dihydrobenzo[1,4]oxazin-4-yl]butyric acid tert-butyl ester in 1,4-dioxane (0.5 mL), 4N HCl/1,4-dioxane (2.0 mL) was added at room temperature, and the reaction mixture was stirred for 24 hours. 4N HCl /1,4-dioxane (2.0 mL) was added thereto at room temperature, and the resultant mixture was further stirred for 24 hours. Solvent was removed under reduced pressure. To the residue, DMSO (5 mL) was added, and insoluble matter was removed. The solution was purified by high-performance liquid column chromatography (NOMURA Develosil Combi-RP-5). The thus-obtained solution was freeze-dried, to thereby yield the title compound (13.9 mg).
NMR ($CD_3OD$) δ;
2.37(1H,d,J=13.2Hz),2.61(1H,d,J=13.2Hz),4.41-3.66(5H,br m),5.30(2H,s),6.57(2H,s),7.31(1H,s),7.45-7.35(6H,m), 7.81(6H,br s).
MS (ESI)m/z:507 $(M+H)^+$.
Anal. Calcd for $C_{24}H_{22}O_5N_2F_3S\cdot$
HCl:C,53.09;H,4.08;N,5.16;S,5.91.Found:C,53.19;H,4.36;N,5.01; S,5.95.

[Example 22]

(R)-3-(N,N-Dimethylamino)-4-oxo-4-[5-[(4-phenyl-5-trifluoromethyl-2-thienyl)methoxy]-indolin-1-yl]butyric acid

[F130]

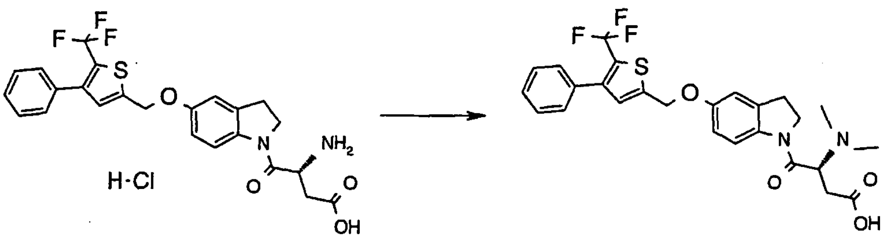

(R)-3-Amino-4-oxo-4-[5-[(4-phenyl-5-trifluoromethyl-2-thienyl)methoxy]-indolin-1-yl]butyric acid hydrochloride (100 mg) was dissolved in THF (10 mL). 37% Aqueous formaldehyde (77 μL) was added to the solution with stirring, and then, sodium triacetoxyborohydride (127 mg) was added to the mixture, followed by stirring for 6 hours. To the reaction mixture, water (50 mL) was added, and the resultant mixture was neutralized with saturated aqueous sodium hydrogencarbonate solution (50 mL), followed by extraction with 20% methanol/chloroform (2 x 200 mL). The extracts were combined and dried over sodium sulfate anhydrate, and solvent was removed by filtration. The residue was added to dichloromethane and hexane, and the precipitated solid was collected by filtration and dried, to thereby yield the title compound (99 mg).
NMR ($CDCl_3$) δ:
2.61(6H,s),2.75-2.84(2H,m),3.18(2H,t,J=8.3Hz),4.12-4.24 (2H,m), 4.29-4 .42 (1H,m), 5.19 (2H, s), 6.76-6.88(2H,m), 7.05(1H,s),7.33-7.43 (5H,m), 8.12 (1H,dd,J=25.9, 17.1Hz).
MS (ESI) m/z : 519 $(M+1)^+$.
HRMS(ESI) Calcd for $C_{26}H_{26}F_3N_2O_4S$ $M^++H$: 519.15654. Found 519.15394.
Anal . Calcd for $C_{26}H_{25}F_3N_2O_2S\cdot$

$0.5H_2O$:C,59.19;H,4.59;F,10.80;N,5.31;S,6.08.Found:C,59.07;H,4 .59;F,10.70;N,5.22;S,6.11.

[Example 23]

3-[(N-Ethyl-N-methyl)amino]-4-oxo-4-[5-[(4-phenyl-5-trifluoromethyl-2-thienyl)methoxy]indolin-1-yl]butyric acid hydrochloride

(1) 3-[(N-Benzyloxycarbonyl-N-methyl)amino]-4-oxo-4-[5-[(4-phenyl-5-trifluoromethyl-2-thienyl)methoxy]indolin-1-yl] butyric acid tert-butyl ester

[F131]

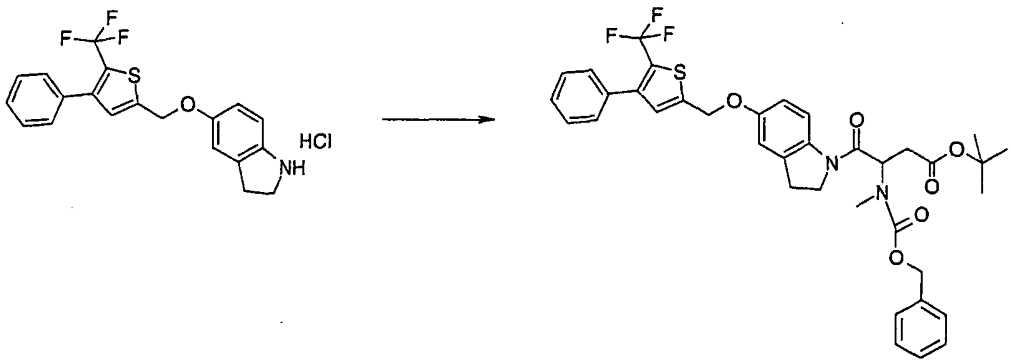

To a DMF solution (3 mL) of 5-[(4-phenyl-5-trifluoromethyl-2-thienyl)methoxy]indoline hydrochloride (100 mg), benzyloxycarbonyl-MeAsp(OtBu) (126 mg), EDC·HCl (70.0 mg), and HOBt (49.2 mg), TEA (0.169 mL) was added at room temperature, and the mixture was stirred at room temperature for 23 hours. The reaction mixture was concentrated under reduced pressure. The residue was purified by silica gel flash column chromatography (Biotage 25S), to thereby yield the title compound (184 mg).
NMR ($CDCl_3$) δ:
1.40 and 1.43(total 9H,each s,amide isomers),2.45-2.56(1H,m),2.87 and 2.89(total 3H,each s,amide isomers),2.94-3.29 (4H,m),3.65-4.33(2H,m),5.02-5.53(4H,m),6.76-6.85 (2H,m), 7.06(1H,s), 7.33-7.43 (10H,m), 8.17-8.08 (1H,m).
MS (ESI) m/z : 695 $(M+H)^+$.

(2) 3-[(N-Ethyl-N-methyl)amino]-4-oxo-4-[5-[(4-phenyl-5-trifluoromethyl-2-thienyl)methoxy]indolin-1-yl]butyric acid tert-butyl ester

[F132]

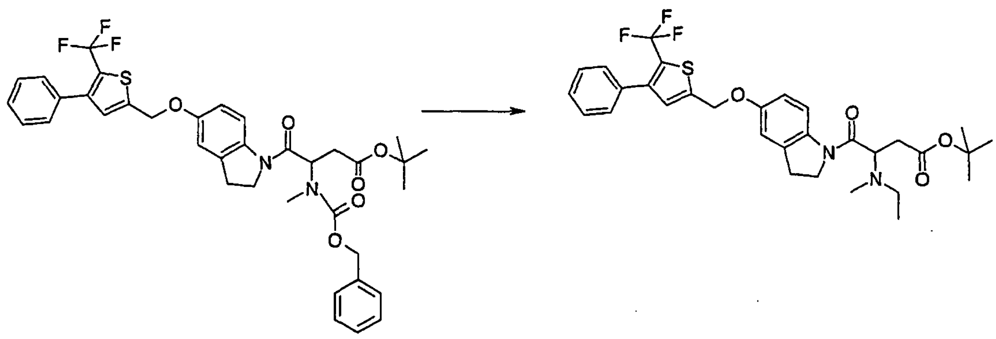

To an ethyl acetate solution (5 mL) of 3-[(N-benzyloxycarbonyl-N-methyl)amino]-4-oxo-4-[5-[(4-phenyl-5-trifluoromethyl-2-thienyl)methoxy]indolin-1-yl]butyric acid tert-butyl ester (92 mg), 5% Pd/C (20 mg) was added, and the mixture was stirred under a hydrogen atmosphere for 18 hours. The catalyst was removed by filtration, and to the residue, ethanol (5 mL) and 5% Pd/C (20 mg) were added, followed by further stirring for 4 days under a stream of hydrogen gas. The catalyst was removed by filtration, and the filtrate was concentrated under reduced pressure. The residue obtained after concentration was purified by silica gel flash column chromatography (Biotage 25S), to thereby yield the title compound (26 mg).
NMR($CDCl_3$) δ:
1.06(3H,t,J=7.6Hz),1.42(9H,s),2.25(3H,s),2.34-2.67(3H,m),2.89-3.21(2H,m),3.97(1H,dd,J=10.0,3.7Hz),4.05-4.19(1H,m),4.61(2H,q,J=9.1Hz),5.19(2H,s),6.79(1H,dd,J=9.1,2.5 Hz),6.84(1H,d,J=2.0Hz),7.06(1H,s),7.34-7.44(5H,m),8.17(1H,d,J=8.8Hz).
MS (ESI) m/z : 589 $(M+H)^+$.

(3) 3-[(N-Ethyl-N-methyl)amino]-4-oxo-4-[5-[(4-phenyl-5-trifluoromethyl-2-thienyl)methoxy]indolin-1-yl]butyric acid hydrochloride

[F133]

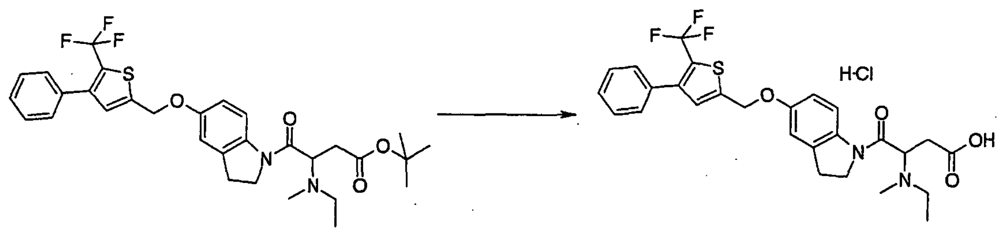

4N HCl/1,4-dioxane (2 mL) of 3-[(N-ethyl-N-methyl)amino]-4-oxo-4-[5-[(4-phenyl-5-trifluoromethyl-2-thienyl)methoxy]indolin-1-yl]butyric acid tert-butyl ester (26 mg) was stirred at room temperature for 2 days. The reaction mixture was concentrated under reduced pressure, and to the residue, ether was added. The precipitated solid was collected by filtration and dried under reduced pressure, to thereby yield the title compound (2.12 mg).
NMR (DMSO-$d_6$) δ:
1.09 and 1.91(total3H,t and s,J=8.3Hz,amide isomers),2.68-2.88(2H,m),3.01-3.27(5H,m),4.15-4.67(4H,m),5.39(2H,s),6.93(1H,dd,J=8.8,2.2Hz),7.06(1H,d,J=1.7 Hz),7.36-7.52(7H,m),8.04(1H,d,J=9.1Hz).
MS (ESI) m/z : 533 $(M+H)^+$.

[Example 24]

3-(N-Methylamino)-4-oxo-4-[5-[(4-phenyl-5-trifluoromethyl-2-thienyl)methoxy]indolin-1-yl]butyric acid

(1) 3-[(N-Benzyloxycarbonyl-N-methyl)amino]-4-oxo-4-[5-[(4-phenyl-5-trifluoromethyl-2-thienyl)methoxy]indolin-1-yl] butyric acid

[F134]

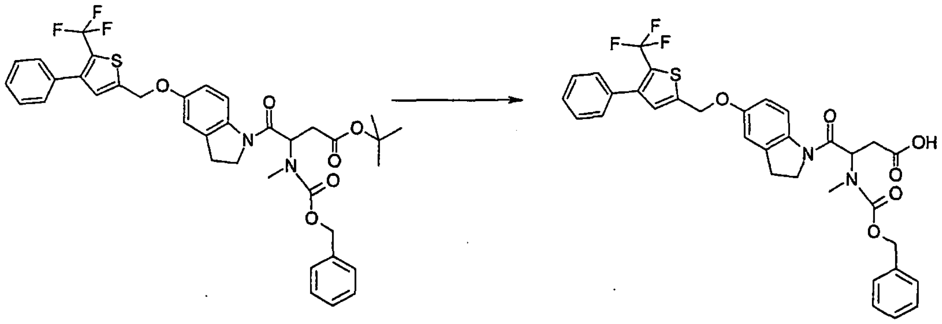

4N HCl/1,4-dioxane (3 mL) and 3-[(N-benzyloxycarbonyl-N-methyl)amino]-4-oxo-4-[5-[(4-phenyl-5-trifluoromethyl-2-thienyl)methoxy]indolin-1-yl]butyric acid tert-butyl ester (92 mg) were combined and the solution was stirred at room temperature for 18 hours. The reaction mixture was concentrated, and the residue was purified by thin-layer chromatography, to thereby yield the title compound (67 mg). NMR ($CDCl_3$) δ:
2.48-2.65(1H,m),2.84 and 2.86(total 3H,each s,amide isomers),2.95-3.27(3H,m),3.50-4.29(3H,m),5.02-5.55(6H,m), 6.74-6.85(2H,m),7.05(1H,s),7.28-7.47(10H,m),8.14-8.05(1H,m).
MS (ESI) m/z : 639 $(M+H)^+$.

(2) 3-(N-Methylamino)-4-oxo-4-[5-[(4-phenyl-5-trifluoromethyl-2-thienyl)methoxy]indolin-1-yl]butyric acid

[F135]

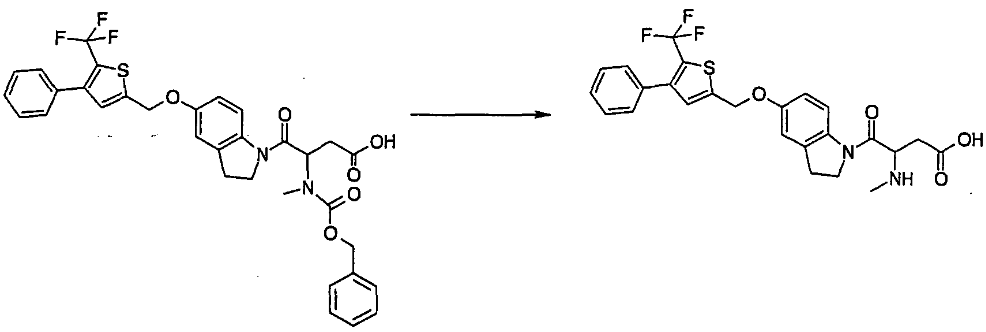

To a ethanol solution (3 mL) of 3-[(N-benzyloxycarbonyl-N-methyl)amino]-4-oxo-4-[5-[(4-phenyl-5-trifluoromethyl-2-thienyl)methoxy]indolin-1-yl]butyric acid (52 mg), 5% Pd/C (20 mg) was added, and the mixture was stirred under a hydrogen atmosphere for 15 hours. The catalyst was removed by filtration, and to the residue, ethanol (3 mL) and 5%

Pd/C (20 mg) were added, followed by further stirring for 3 days. The catalyst was removed by filtration, and the reaction mixture was concentrated under reduced pressure. To the residue, TEA (0.169 mL) was added at room temperature, followed by stirring at room temperature for 23 hours. The reaction mixture was concentrated under reduced pressure, and the residue was purified by thin-layer chromatography, to thereby yield the title compound (3.45 mg).
NMR ($CDCl_3$) δ:
2.37-2.73 (4H, m), 3.26 (2H, t, J=8.3Hz), 3.62-3.74 (1H, m), 3.99-4.23 (2H, m), 5.22 (2H, s), 6.82-6.91 (2H, m), 7.07 (1H, s), 7.44-7.37(7H,m),8.16(1H,d,J=8.3Hz).
MS (ESI) m/z : 505 $(M+H)^+$.

[Example 25]

1-[5-[(4-Phenyl-5-trifluoromethyl-2-thienyl)methoxy]indolin-1-ylcarbonyl]azetidine-3-carboxylic acid

(1) 1-[5-[(4-Phenyl-5-trifluoromethyl-2-thienyl)methoxy]indolin-1-ylcarbonyl]azetidine-3-carboxylic acid methyl ester

[F136]

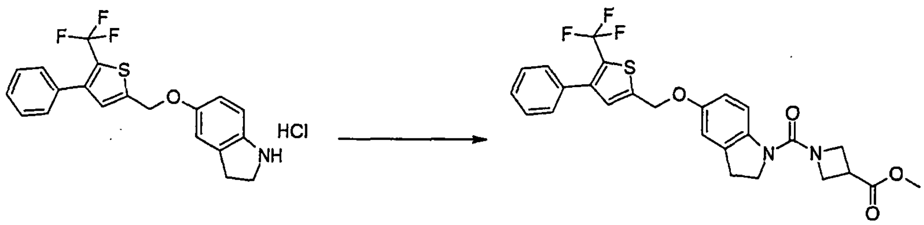

To a solution of 5-[(4-phenyl-5-trifluoromethyl-2-thienyl)methoxy]indoline hydrochloride (100 mg) in a dichloromethane/pyridine (10:1) mixture (2.2 mL), triphosgene (23.7 mg) was added at 0°C in a nitrogen atmosphere, and the mixture was stirred for 14 hours. To the reaction mixture, azetidine-3-carboxylic acid methyl ester hydrochloride (36.4 mg) was added, followed by stirring for 7 hours. An equiamount of azetidine-3-carboxylic acid methyl ester hydrochloride (36.4 mg) was added thereto, followed by further stirring for 17 hours. The reaction mixture was diluted with ethyl acetate, and 1N hydrochloric acid was added thereto, followed by stirring for 30 minutes. The resultant mixture was extracted with ethyl acetate. The extracts were combined and washed sequentially with saturated aqueous sodium bicarbonate solution and saturated brine, followed by drying over sodium sulfate anhydrate. Sodium sulfate anhydrate was removed by filtration, and solvent was removed under reduced pressure. The residue was subjected to silica gel flash column chromatography (Biotage 25S), to thereby yield the title compound (103 mg). NMR ($CDCl_3$) δ :
3.11(2H,t,J=8.8Hz),3.40-3.49(1H,m),3.77(3H,s),3.92(2H,t,J=8.6Hz),4.30(2H,s),4.32(2H,s ),5.18(2H,s),6.77-6.83(2H, m),7.04-7.06(1H,m),7.35-7.44(5H,m),7.61(1H,d,J=8.8Hz).
MS (ESI)m/z: 517 $(M+H)^+$.

(2) 1-[5-[(4-Phenyl-5-trifluoromethyl-2-thienyl)methoxy]indolin-1-ylcarbonyl]azetidine-3-carboxylic acid

[F137]

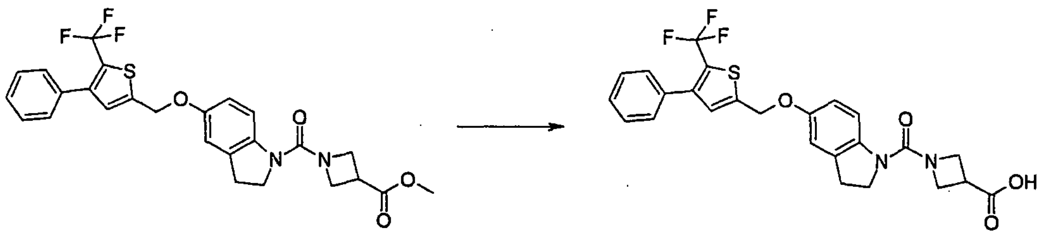

To a solution of 1-[5-[(4-phenyl-5-trifluoromethyl-2-thienyl)methoxy]indolin-1-ylcarbonyl]azetidine-3-carboxylic acid methyl ester (103 mg) in a methanol/THF (1:2) mixture (3 mL), 1N aqueous sodium hydroxide solution (1 mL) was added at room temperature, and the mixture was stirred for 14 hours. The reaction mixture was concentrated under reduced pressure, and to the residue, 1N hydrochloric acid was added so as to adjust the pH of the solution to 2. The resultant mixture was extracted with a 10% methanol/chloroform solution. The extracts were combined and washed with saturated brine, followed by drying over sodium sulfate anhydrate. Sodium sulfate anhydrate was removed by filtration, and the filtrate was concentrated under reduced pressure, to thereby yield the title compound (81 mg).
NMR ($CDCl_3$) δ:
3.11(2H,t,J=8.8Hz),3.40-3.49(1H,m),3.77(3H,s),3.92(2H,t,J=8.6Hz),4.30(2H,s),4.32(2H,s ),5.18(2H,s),6.77-6.83(2H, m),7.04-7.06(1H,m),7.35-7.44(5H,m),7.61(1H,d,J=8.8Hz).
IR(ATR)$cm^{-1}$:2960,2900,2521,1732,1606,1568,1491.
MS (ESI) m/z : 503 $(M+H)^+$.
HRMS(FAB)Calcd for $C_{25}H_{22}F_3N_2O_4S$: 503.1252. Found: 503.1250.

[Example 26]

1-[5-[(4-Phenyl-5-trifluoromethyl-2-thienyl)methoxy]indolin-1-yl]carbonylmethyl]-3-azetidinecarboxylic acid

(1) 1-[5-[(4-Phenyl-5-trifluoromethyl-2-thienyl)methoxy]indolin-1-yl]carbonylmethyl]-3-azetidinecarboxylic acid methyl ester

[F138]

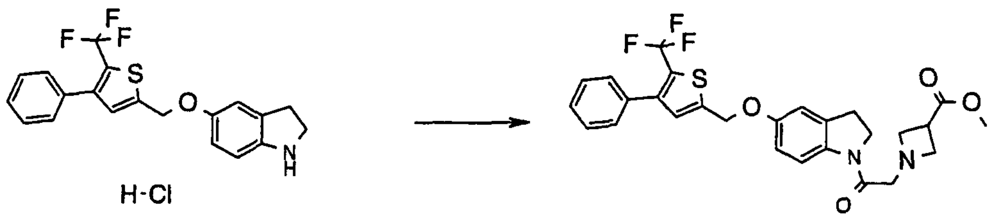

[5-[(4-Phenyl-5-trifluoromethyl-2-thienyl)methoxy]indoline] hydrochloride (100 mg) was dissolved in acetonitrile (5 mL), and to the solution, DIEA (85 μL) and chloroacetyl chloride (21 μL) were sequentially added at 0°C with stirring, and the mixture was stirred for 1 hour. To the reaction mixture, azetidinecarboxylic acid methyl ester hydrochloride (73 mg) and DIEA (169 μL) were added, followed by stirring at 50°C for 2 hours. The resultant mixture was concentrated, and the residue was purified by silica gel flash column chromatography (Yamazen Hi-Flash L), to thereby yield the title

compound (109 mg).
NMR ($CDCl_3$) δ:
3.16(2H,t,J=8.5Hz),3.36(2H,s),3.39-3.48(2H,m),3.67-3.81(6H,m),4.01(2H,t,J=8.4Hz),5.18(2H,s),6.77-6.84(2H,m),7.05(1H,s),7.35-7.43(5H,m),8.14(1H,d,J=8.8Hz).
MS (ESI) m/z 531 $(M+H)^+$.

(2) 1-[5-[(4-Phenyl-5-trifluoromethyl-2-thienyl)methoxy]indolin-1-yl]carbonylmethyl]-3-azetidinecarboxylic acid

[F139]

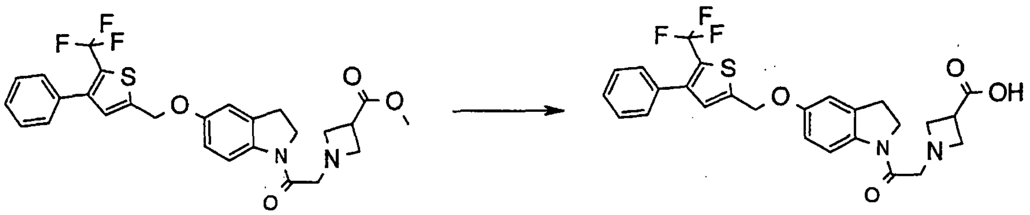

To 1-[5-[(4-phenyl-5-trifluoromethyl-2-thienyl)methoxy]indolin-1-yl]carbonylmethyl]-3-azetidinecarboxylic acid methyl ester (109 mg), THF (5 mL) and 0.25N aqueous sodium hydroxide solution (1.64 mL) were added, and the mixture was stirred for 1.5 hours. The reaction mixture was neutralized with 1N hydrochloric acid, and the resultant mixture was diluted with water (50 mL), followed by extraction with 10% methanol/chloroform (2 x 100 mL). The extracts were combined and dried (sodium sulfate anhydrate). Solvent was removed by evaporation, and to the residue, dichloromethane and hexane were added to form solid. The formed solid was collected by filtration, followed by drying in vacuo for 1 day, to thereby yield the title compound (106 mg).
NMR ($CDCl_3$) δ :
2.93-3.03(2H,m),3.53-3.64(1H,m),3.81-4.31(8H,m),5.09(2H,s),6.67-6.72(2H,m),7.01(1H,s),7.35-7.41(5H,m),7.98(1H,d,J=8.8Hz).
MS (ESI) m/z: 517$(M+H)^+$.
Anal. Calcd for $C_{26}H_{23}F_3N_2O_4S\cdot$
$0.25H_2O$:C,59.93;H,4.55;F,10.94;N,5.38;S,6.15.Found:C,59.69;H, 4.34;F,10.76;N,5.34;S,6.14.

[Example 27]

4-[N-[5-[(4-Phenyl-5-trifluoromethyl-2-thienyl)methoxy]indolin-1-ylcarbonyl]amino]butyric acid

(1) 4-[N-[5-[(4-Phenyl-5-trifluoromethyl-2-thienyl)methoxy]indolin-1-ylcarbonyl]amino]butyric acid ethyl ester

[F140]

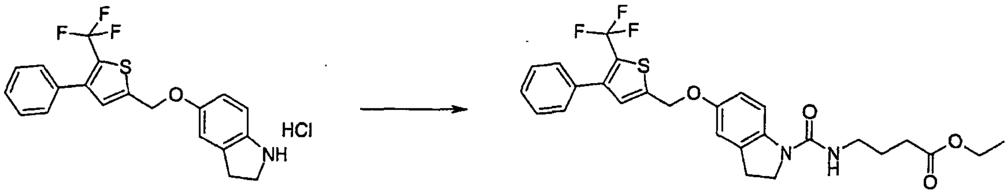

To a dichloromethane solution (2 mL) of 5-[(4-phenyl-5-trifluoromethyl-2-thienyl)methoxy]indoline hydrochloride (62 mg), pyridine (0.50 mL) and triphosgene (14.7 mg) were added at 0°C, and the mixture was stirred for 23 hours during which the mixture was allowed to warm to room temperature. To the reaction mixture, 4-aminobutyric acid ethyl ester (30.3 mg) was added, followed by further stirring for 23 hours. To the resultant mixture, water was added, and the mixture was extracted with ethyl acetate. The extracts were combined and washed sequentially with 1N hydrochloric acid, saturated aqueous sodium bicarbonate solution, and saturated brine, followed by drying over sodium sulfate anhydrate. Sodium sulfate anhydrate was removed by filtration, and solvent was evaporated under reduced pressure. The residue was subjected to silica gel flash column chromatography (Biotage 25S), to thereby yield the title compound (2 mg).
NMR ($CDCl_3$ ) δ :
1.25(3H,t,J=7.2Hz),1.88-1.95(2H,m),2.42(2H,t,J=6.9Hz),3.16(2H,t,J=8.6Hz),3.38(2H,q,J= 6.2Hz),3.90(2H,t,J=8.6Hz),4.13(2H,q,J=7.2Hz),4.87-4.93(1H,m),5.18(2H,s),6.75-6.83(2H,m),7.05(1H,s),7.37-7.44(5H,m),7.84(1H,d,J=8.6Hz).
MS (ESI) m/z : 532 $(M+H)^+$.

(2) 4-[N-[5-[(4-Phenyl-5-trifluoromethyl-2-thienyl)methoxy]indoline-1-carbonyl]amino]butyric acid

[F141]

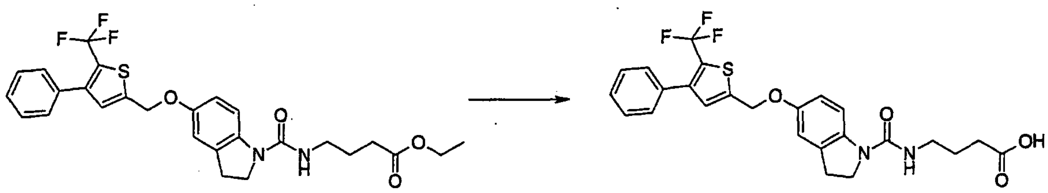

To a solution of 4-[N-[5-[(4-phenyl-5-trifluoromethyl-2-thienyl)methoxy]indolin-1-ylcarbonyl]amino]butyric acid ethyl ester (12 mg) in methanol/THF (1:2) (1.5 mL), 1N sodium hydroxide solution (0.5 mL) was added, and the mixture was stirred for 3 days. The reaction mixture was concentrated under reduced pressure, and to the residue, 1N hydrochloric acid was added. The precipitated solid was collected by filtration, to thereby yield the title compound (8 mg). NMR ($DMSO-d_6$) δ :
1.63-1.73(2H,m),2.24(2H,t,J=7.4Hz),3.02-3.14(4H,m),3.84(2H,t,J=8.7Hz),5.26-5.37(3H,m),6.55(1H,t,J=5.6Hz),6.77(1H,dd,J=8.6,2.7Hz),6.89(1H ,d,J=2.7Hz),7.31-7.51(6H,m),7.72(1H,d,J=8.8Hz).
MS (ESI) m/z : 505 $(M+H)^+$.

[Example 28]

2-[N-[2-Oxo-2-[5-[(4-phenyl-5-trifluoromethyl-2-thienyl)methoxy]indolin-1-yl]ethyl]amino]acetic acid

(1) 1-(Chloroacetyl)-5-[(4-phenyl-5-trifluoromethyl-2-thienyl)methoxy]indoline

[F142]

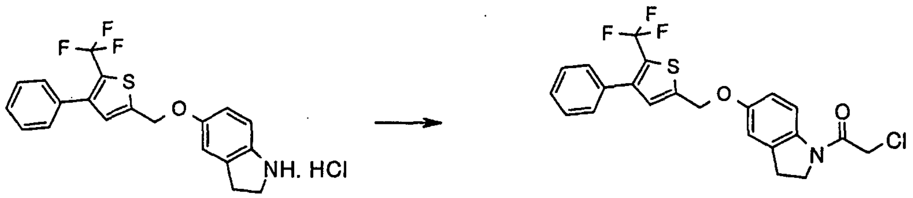

To a solution of 5-[(4-phenyl-5-trifluoromethyl-2-thienyl)methoxy]indoline hydrochloride (234 mg) and TEA (167μ L) in dichloromethane (3.5 mL), chloroacetyl chloride (47.6 μL) was added at room temperature. The reaction mixture was stirred at room temperature for 3 hours and concentrated under reduced pressure. Ethyl acetate (15 mL) and water (10 mL) were added thereto for phase separation, and the aqueous layer was extracted with ethyl acetate (10 mL x 3). The extracts were combined and dried over sodium sulfate anhydrate. Solvent was removed under reduced pressure, and the residue was dried under reduced pressure, to thereby yield the title compound (240 mg). The compound was employed in a subsequent reaction without performing further isolation/purification.
MS (ESI)m/z: 452 $(M+H)^+$.

(2) 2-[N-[2-Oxo-2-[5-[(4-phenyl-5-trifluoromethyl-2-thienyl)methoxy]indolin-1-yl]ethyl]amino]acetic acid ethyl ester

[F143]

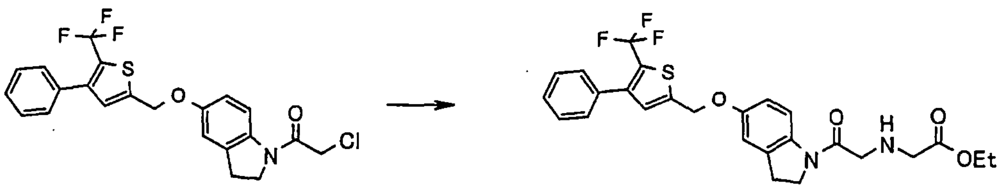

To a suspension of 1-(chloroacetyl)-5-[(4-phenyl-5-trifluoromethyl-2-thienyl)methoxy]indoline (120 mg) and glycine hydrochloride methyl ester (74.3 mg) in acetonitrile (2 mL), DIEA (232 μL) was added at room temperature. The reaction mixture was stirred at 70°C for 14 hours and left to stand to cool to room temperature. The resultant mixture was concentrated under reduced pressure, and chloroform (10 mL) and water (10 mL) were added to the concentrate for phase separation. The aqueous layer was extracted with chloroform (5 mL x 3). The extracts were combined and washed with saturated brine, followed by drying over sodium sulfate anhydrate. Solvent was removed under reduced pressure, and the residue was purified by silica gel flash column chromatography (Biotage 25M), to thereby yield the title compound (90.8 mg).
NMR ($CDCl_3$) δ:
1.28(3H,t,J=7.1Hz),3.20(2H,t,J=8.1Hz),3.54(2H,s),3.56(2H,s),4 00(2H,t,J=8.1Hz),4.20(2H,q,J=7.1Hz),5.20(2H,s), 6.86-6.80(2H,m),7.06(1H,s),7.43-7.39(5H,m),8.17(1H,d,J=8.8Hz).

(3) 2-[N-[2-Oxo-2-[5-[(4-phenyl-5-trifluoromethyl-2-thienyl)methoxy]indolin-1-yl]ethyl]amino]acetic acid

[F144]

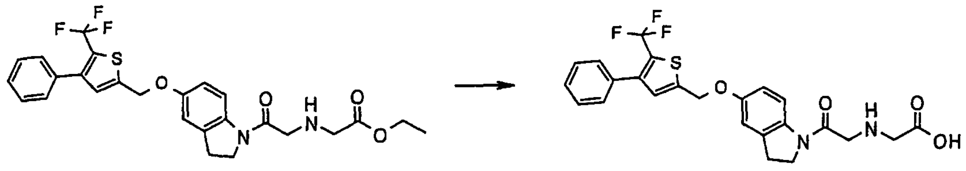

A solution of 2-[N-[2-oxo-2-[5-[(4-phenyl-5-trifluoromethyl-2-thienyl)methoxy]indolin-1-yl] ethyl] amino] acetic acid ethyl ester (90.8 mg) in a 33% methanol/THF mixture (1.5 mL), 1N aqueous sodium hydroxide solution (0.5 mL) was added, and the mixture was stirred at room temperature for 14 hours. To the reaction mixture, water (5 mL) was added, and then, 1N hydrochloric acid was added thereto so as to make the mixture weekly acidic (pH: 4). In addition, a 20% methanol/chloroform mixture (10 mL) was added to the resultant mixture, and organic matter was extracted. The extracts were combined, and methanol (5 mL) was added to the combined extract. The solution was dried over sodium sulfate anhydrate, and solvent was removed under reduced pressure. To the residue, 2% methanol/water (2 mL) was added. The precipitated solid was collected by filtration, followed by washing with water and drying under reduced pressure, to thereby yield the title compound (44.7 mg).
NMR (DMSO-$d_6$) δ:
3.13(2H,t,J=8.1Hz),3.25(2H,s),3.70(2H,s),4.01(2H,t,J=8.1Hz),5 .35(2H,s),6.88(1H,dd,J=8.8,2.0Hz),6.99(1H,s),7.36 (1H,s),7.50-7.41(5H, m), 7.98(1H, d, J=8.8Hz).
MS (ESI) m/z : 491 $(M+H)^+$.

[Example 29]

3-[N-[2-Oxo-2-[5-[(4-phenyl-5-trifluoromethyl-2-thienyl)methoxy]indolin-1-yl]ethyl]amino]propionic acid

(1) 3-[N-[2-Oxo-2-[5-[(4-phenyl-5-trifluoromethyl-2-thienyl)methoxy]indolin-1-yl]ethyl]amino]propionic acid ethyl ester

[F145]

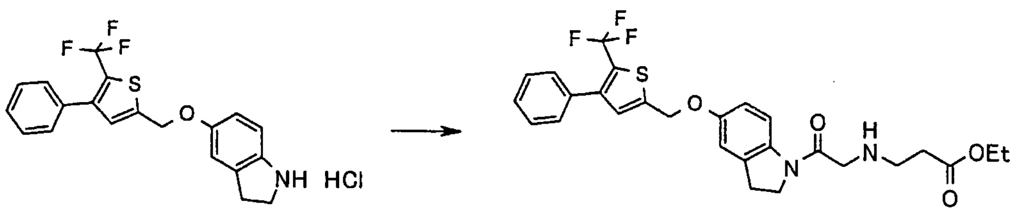

To a solution of 5-[(4-phenyl-5-trifluoromethyl-2-thienyl)methoxy]indoline hydrochloride (165 mg) and DIEA (146 μL) in acetonitrile (4 mL), chloroacetyl chloride (33.5 μL) was added at room temperature, and the mixture was stirred at room temperature for 1.5 hours. DIEA (73.2 μL) and 3-aminopropionic acid hydrochloride ethyl ester (61.4 mg) were added thereto, followed by stirring at 80°C for 20 hours. The reaction mixture was concentrated under reduced pressure, and to the concentrate, a 10% methanol/chloroform mixture (10 mL) and water (5 mL) were added for phase separation. The aqueous layer was extracted with 10% methanol/chloroform (5 mL x 3). The extracts were combined and dried over sodium sulfate anhydrate. Solvent was removed under reduced pressure. To the residue, DMSO (3 mL) was added, and insoluble matter was removed by filtration. The filtrate was purified by high-performance liquid chromatography (NOMURA Develosil Combi-RP-5). The eluates were combined and freeze-dried, to thereby yield the title compound (37.4 mg).
NMR ($CDCl_3$) δ:

1.25(3H, t, J=6.8Hz), 2.87(2H, t, J=6.1Hz), 3.13(2H, t, J=7.8Hz), 3.32 (2H, t, J=6.1Hz), 3.95(2H,s), 3.98(2H, t, J=7.8Hz), 4.15(2H, q, J=7.0H z), 5.15(2H,s), 6.75(1H,d,J=8.8Hz), 6.81(1H,s), 7.05(1H,s), 7.41-7.38(5H,br m),8.08(1H,d,J=8.8Hz), 8.31(1H,br s), 8.54(1H,br s).
MS (ESI) m/z : 533 $(M+H)^+$.

(2) 3-[N-[2-Oxo-2-[5-[(4-phenyl-5-trifluoromethyl-2-thienyl)methoxy]indolin-1-yl]ethyl]amino]propionic acid

[F146]

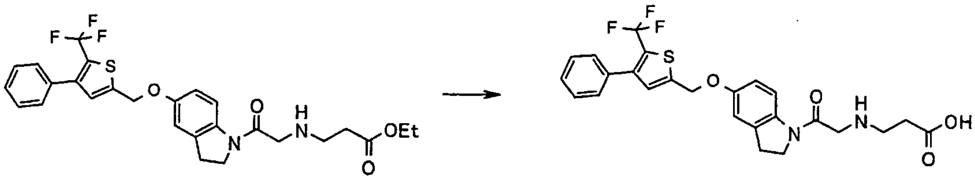

To a solution of 3-[N-[2-oxo-2-[5-[(4-phenyl-5-trifluoromethyl-2-thienyl)methoxy]indolin-1-yl]ethyl]amino]propionic acid ethyl ester (37.4 mg) in a 33% methanol/THF mixture (1.5 mL), 1N aqueous sodium hydroxide solution (0.5 mL) was added, and the mixture was stirred at room temperature for 14 hours. To the reaction mixture, water (5 mL) was added, and 1N aqueous hydrochloric acid solution was added so as to make the mixture weekly acidic (pH: 4). In addition, a 10% methanol/chloroform mixture (7.5 mL) was added to the resultant mixture, and organic matter was extracted. The extracts were combined and dried over sodium sulfate anhydrate. Solvent was removed under reduced pressure, and to the residue, diethyl ether (2 mL) was added to form solid. The formed solid was collected by filtration and washed with ethyl acetate and diethyl ether. The washed solid was dried under reduced pressure, to thereby yield the title compound (21.0 mg).
NMR (DMSO-$d_6$) δ:
2.73(2H, t, J=7.6Hz), 3.19(4H, t, J=7.6Hz), 4.06(2H, t, J=7.6Hz), 4.13 (2H,s), 5.37(2H, s), 6.92(1H, dd, J=8.5, 2.0Hz), 7.05(1H,s), 7.37(1H, s), 7.51-7.40(5H,m), 7.98(1H, d, J=8.5Hz).
MS(EI)m/z:505$(M+H)^+$
HRMS(FAB)Calcd for $C_{25}H_{24}O_4N_2F_3S$ $(M+H)^+$: 505.5378. Found: 505.1409 .

[Example 30]

3-[N-Methyl-N-[2-oxo-2-[5-[(4-phenyl-5-trifluoromethyl-2-thienyl)methoxy]indolin-1-yl]ethyl]amino]propionic acid

(1) 3-[N-Methyl-N-[2-oxo-2-[5-[(4-phenyl-5-trifluoromethyl-2-thienyl)methoxy]indolin-1-yl]ethyl]amino]propionic acid ethyl ester

[F147]

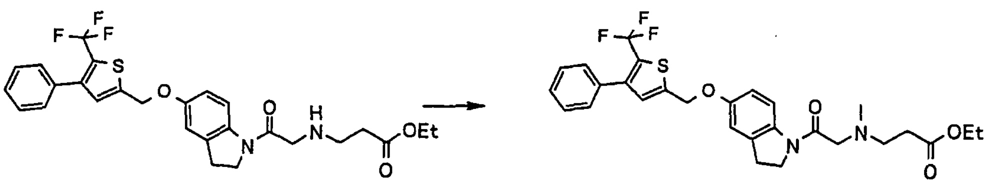

To a solution of 3-[N-[2-oxo-2-[5-[(4-phenyl-5-trifluoromethyl-2-thienyl)methoxy]indolin-1-yl] ethyl] amino] propionic acid ethyl ester (94.1 mg) in THF (5 mL), a 37% aqueous formaldehyde solution (32.9 μL) was added at room temperature, and the mixture was stirred for 1 hour. Sodium triacetoxyborohydride (56.2 mg) was added thereto, and the resultant mixture was stirred at room temperature for 18 hours. To the reaction mixture, chloroform (10 mL) and saturated sodium hydrogencarbonate solution (5 mL) were added for phase separation. The aqueous layer was extracted with chloroform (5 mL x 3). The extracts were combined and washed with saturated brine, followed by drying over sodium sulfate anhydrate. Solvent was removed under reduced pressure, to thereby yield the title compound (89.2 mg). The compound was employed in a subsequent reaction without performing further isolation/purification.
NMR($CDCl_3$) δ:
1.22(3H,t,J=7.1Hz), 2.39(3H, s), 2.51(2H, t, J=6.6Hz), 2.89(2H, t, J= 6.6Hz), 3.14(2H, t, J=8.5Hz),3.30(2H,s), 4.10(3H, q, J=7.1Hz), 4.14( 1H, t, J=8.5Hz), 5.19(2H,s), 6.80(1H, dd, J=8.8,2.0Hz), 6.83(1H,br s), 7.05(1H,s), 7.43-7.37(5H, m), 8.17(1H, d, J=8.8Hz).

(2) 3-[N-Methyl-N-[2-oxo-2-[5-[(4-phenyl-5-trifluoromethyl-2-thienyl)methoxy]indolin-1-yl]ethyl]amino]propionic acid hydrochloride

[F148]

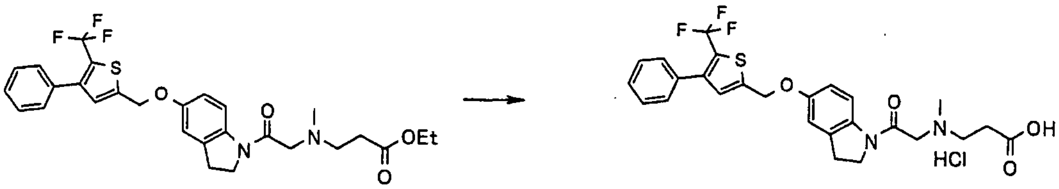

To a solution of 3-[N-methyl-N-[2-oxo-2-[5-[(4-phenyl-5-trifluoromethyl-2-thienyl)methoxy]indolin-1-yl]ethyl] amino]propionic acid ethyl ester in a 33% methanol/THF mixture (3 mL), 1N aqueous sodium hydroxide solution (1 mL) was added, and the mixture was stirred at room temperature for 14 hours. To the reaction mixture, water (5 mL) was added, and then, 1N hydrochloric acid was added thereto so as to make the mixture weekly acidic (pH: 4). In addition, a 20% methanol/chloroform mixture (15 mL) was added to the resultant mixture, and organic matter was extracted. The extracts were combined, and methanol (10 mL) was added to the combined extract. The solution was dried over sodium sulfate anhydrate, and solvent was removed under reduced pressure. To the residue, DMSO (3 mL) was added, and insoluble matter was removed by filtration. The filtrate was purified by high-performance liquid chromatography (NOMURA Develosil Combi-RP-5). The eluates were combined and concentrated. To the residue, 4N HCl/1,4-dioxane (2 mL) was added, followed by stirring for 10 minutes. Solvent was removed under reduced pressure, and a 25% diethyl ether/ hexane mixture (3 mL) was added to the residue so as to form solid. The formed solid was recovered through filtration and washed with a 25% diethyl ether/hexane mixture, followed by drying under reduced pressure, to thereby yield the title compound (36.0 mg).
NMR (DMSO-$d_6$) δ:
2.84 (2H, t, J=7.3Hz), 2.87 (3H, s), 3.19 (2H, t, J=8.2Hz), 3.33 (2H, br s),4.04(2H,t,J=8.2Hz),4.36(2H,br s), 5.37(2H,s), 6.93(1H, dd, J=8.8, 2.0Hz), 7.05(1H, d, J=2.0Hz),7.37 (1H,s), 7.41-7.51(5H, m), 7.98(1H, d, J=8.8Hz).
MS (ESI) m/z : 519 $(M+H)^+$

[Example 31]

3-[N-[2-Oxo-2-[5-[(2-trifluoromethyl-4-biphenyl)-methoxy]indolin-1-yl]ethyl]amino]propionic acid

(1) 1-Chloroacetyl-5-[(2-trifluoromethyl-4-biphenyl)methoxy]indoline

[F149]

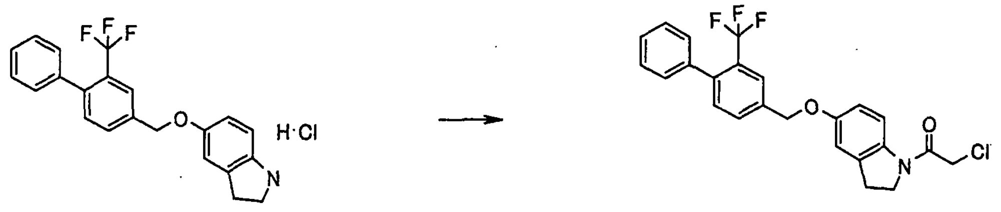

To a solution of 5-[(2-trifluoromethyl-4-biphenyl)methoxy]indoline hydrochloride (146 mg) and TEA (105µ L) in dichloromethane (3 mL), chloroacetyl chloride (30.0 µL) was added at room temperature, and the reaction mixture was stirred at room temperature for 2 hours. The reaction mixture was concentrated under reduced pressure, and chloroform (10 mL) and water (7 mL) were added thereto for phase separation. The aqueous layer was extracted with chloroform (5 mL x 2). The extracts were combined and dried over sodium sulfate anhydrate. Solvent was removed under reduced pressure, to thereby yield the title compound (154 mg). The compound was employed in a subsequent reaction without performing further isolation/purification.
NMR ($CDCl_3$) δ:
3.23 (2H, t, J=8.1Hz), 4.15 (2H, s), 4.17 (2H, t, J=8.1Hz), 5.12 (2H, s), 6 .89-6.83(2H,m),7.42-7.30(6H,m),7.61(1H,d, J=7.8Hz), 7.81(1H,s), 8.16(1H,d,J=9.0Hz).
MS (ESI) m/z : 446 $(M+H)^+$

(2) 3-[N-[2-Oxo-2-[5-[5-[(2-trifluoromethyl-4-biphenyl)methoxy]indolin-1-yl]ethyl]amino]propionic acid tert-butyl ester

[F150]

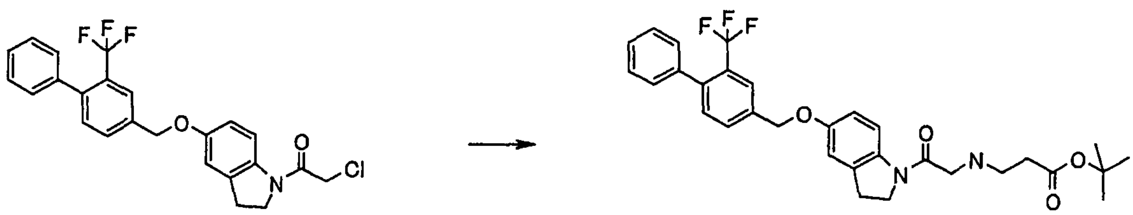

To a suspension of 1-chloroacetyl-5-[(2-trifluoromethyl-4-biphenyl)methoxy]indoline (154 mg) and β-alanine tert-butyl ester (107 mg) in acetonitrile (3 mL), DIEA (205 µL) was added at room temperature. The reaction mixture was stirred at 70°C for 11 hours and left to stand to cool to room temperature. The reaction mixture was concentrated under reduced pressure, and chloroform (10 mL) and saturated aqueous sodium hydrogencarbonate solution (5 mL) were added to the concentrate for phase separation. The aqueous layer was extracted with chloroform (5 mL x 3). The extracts were combined and washed with saturated brine, followed by drying over sodium sulfate anhydrate. Solvent was removed under reduced pressure, and the residue was purified by silica gel flash column chromatography (Biotage 25M), to thereby yield the title compound (109 mg).
NMR ($CDCl_3$) δ:
1.46(9H,s), 2.47(2H,t,J=6.7Hz), 2.93(2H,t,J=6.7Hz), 3.20(2H, t, J= 8.4Hz), 3.50(2H,s), 4.02(2H, t, J=8.4Hz), 5.12(2H, s), 6.83(1H, dd, J= 8.8,2.7Hz), 6.86(1H,s), 7.42-7.31(6H,m), 7.62(1H, d, J=7.8Hz), 7.80(1H,s), 8.17(1H,d,J=8.8Hz).
MS (ESI) m/z : 555 $(M+H)^+$.

(3) 3-[N-[2-Oxo-2-[5-[(2-trifluoromethyl-4-biphenyl)methoxy]indolin-1-yl]ethyl]amino]propionic acid

[F151]

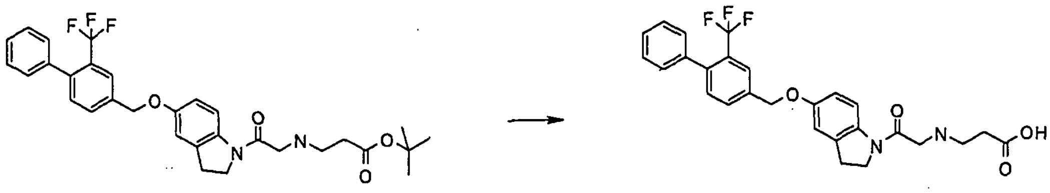

To 3-[N-[2-oxo-2-[5-[5-[(2-trifluoromethyl-4-biphenyl)methoxy]indolin-1-yl]ethyl]amino]propionic acid tert-butyl ester, trifluoroacetic acid (1 mL) was added at room temperature, and the reaction mixture was stirred for 1 hour. To the reaction mixture, water (5 mL) was added, and then, saturated aqueous sodium hydrogencarbonate solution was added thereto for neutralization. In addition, a 20% methanol/chloroform mixture (15 mL) was added to the resultant mixture, and organic matter was extracted. The extracts were combined, and methanol (10 mL) was added to the combined extract. The solution was dried over sodium sulfate anhydrate, and solvent was removed under reduced pressure. To the residue, DMSO (3 mL) was added, and insoluble matter was removed by filtration. The filtrate was purified by high-performance liquid chromatography (NOMURA Develosil Combi-RP-5). The eluates were combined and freeze-dried. To a solution of the residue dissolved in a 33% methanol/THF mixture (1.5 mL), 1N aqueous sodium hydroxide solution (0.5 mL) was added, followed by stirring at room temperature for 14 hours. To the reaction mixture, water (5 mL) was added, and then, 1N hydrochloric acid was added so as to make the mixture weekly acidic (pH: 4). In addition, a 20% methanol/chloroform mixture (15 mL) was added to the resultant mixture, and organic matter was extracted. The extracts were combined, and methanol (10 mL) was further added to the combined extract. The solution was dried over sodium sulfate anhydrate, and solvent was removed under reduced pressure. To the residue, a 20% ethyl acetate/hexane mixture (2 mL) was added. The precipitated solid was collected by filtration, followed by washing with a 20% ethyl acetate/hexane mixture and drying under reduced pressure, to thereby yield the title compound (48.9 mg). NMR (DMSO-$d_6$) δ: 2.37(2H, t, J=6.7Hz), 2.83(2H, t, J=6.7Hz), 3.13(2H, t, J=8.0Hz), 3.56 (2H,s), 4.04(2H, t, J=8.0Hz), 5.21(2H,s), 6.86(1H, dd, J=8.5,2.0Hz), 6.99(1H,br s),7.33-7.29(2H,m),7.45-7.41 (5H,m), 7.76 (1H, d, J=8.0Hz), 7.88 (1H, s),7.99 (1H, d, J=8.5Hz) .
MS (ESI) m/z=499 $(M+H)^+$.

[Example 32]

(3R)-Amino-4-[5-[(4-cyclohexyl-2-trifluoromethylphenyl)methoxy]indolin-1-yl]-4-oxobutyric acid hydrochloride

(1) (3R)-(tert-Butoxycarbonylamino)-4-[5-[(4-cyclohexyl-2-trifluoromethylphenyl)methoxy]indolin-1-yl]-4-oxobutyric acid tert-butyl ester

[F152]

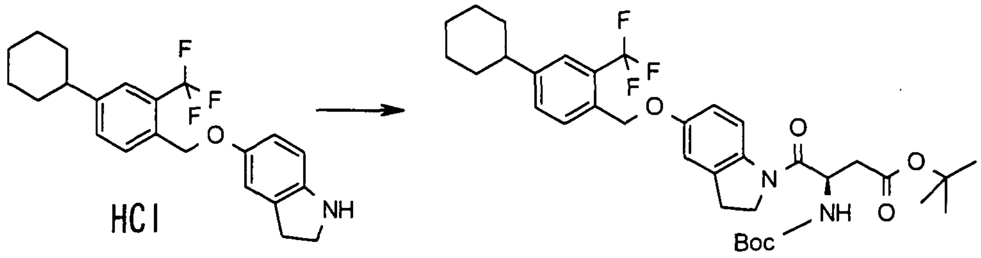

TEA (0.35 mL) was added at room temperature to a DMF solution (5 mL) of 5-[(4-cyclohexyl-2-trifluoromethylphenyl)methoxy]indoline hydrochloride (0.21 g), Boc-D-Asp(OtBu)-OH (Watanabe Chemical Industries, Ltd.) (0.14 g), EDC-HCl (0.14 g), and HOBt (0.10 g), and the mixture was stirred for 2 days hours. The reaction mixture was concentrated, and the residue was purified by silica gel flash column chromatography (Biotage 25S), to thereby yield the title compound (0.37 g).
$^1$H-NMR ($CDCl_3$) δ:
1.11-1.48(20H,m), 1.59-1.69(2H,m), 1.71-1.80(1H,m), 1.81-1.93 (5H,m), 2.47-2.67 (2H,m), 2.75-2.88 (1H,m), 3.10-3.26 (2H,m),4.19-4.40(2H,m),4.81-4.99(1H,m), 5.18(2H,s), 5.29(1H, d, J=10.0Hz), 6.91-6.64(2H,m), 7.38(1H,d,J=8.1Hz), 7.45-7.54(1H,m), 7.60-7.73(1H,m), 8.11(1H, d, J=8.8Hz).
MS (ESI) m/z : 647 $(M+H)^+$.

(2) (3R)-Amino-4-[5-[(4-cyclohexyl-2-trifluoromethylphenyl)methoxy]indolin-1-yl]-4-oxobutyric acid hydrochloride

[F153]

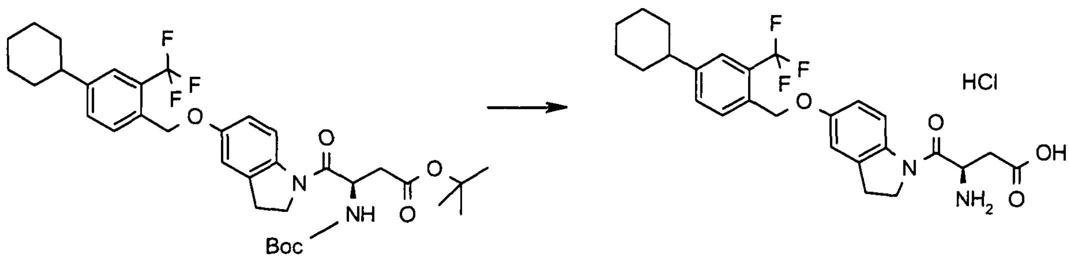

To (3R)-(tert-butoxycarbonylamino)-4-[5-[(4-cyclohexyl-2-trifluoromethylphenyl)methoxy]indolin-1-yl]-4-oxo] butyric acid tert-butyl ester (0.32 g), 4N HCl/1,4-dioxane (10 mL) was added at room temperature, and the mixture was stirred for 3 days, followed by concentration under reduced pressure. To the residue, diethyl ether was added, and the precipitated solid was collected by filtration, to thereby yield the title compound (0.21 g).
$^1$H-NMR (DMSO-$d_6$) δ :
1.03-1.48(6H,m),1.68-1.90(4H,m),2.52-2.93(2H,m),2.94-3.23(3H,m),3.58-3.78(1H,m),4.06-4.35(1H,m),5.08-5.21(2H, m),6.84(1H, dd, J=8.8,2.7Hz), 6.98(1H,d,J=2.5Hz), 7.09(1H ,s), 7.51-7.61(3H,m), 7.64(1H, d, J=7.6Hz), 7.99(1H, d, J=8.8Hz), 8.37(2H,s).
IR(ATR)$cm^{-1}$:2924, 2850, 1724, 1655, 1597, 1489, 1448.
MS (ESI) m/z : 491 $(M+H)^+$.
Anal. Calcd for $C_{26}H_{29}F_3N_2O_4$ ·1.25HCl
$H_2O$:C, 56.36; H, 5.87; Cl, 8.00;F, 10.29; N, 5.06.Found: C, 56.52; H, 5.8 8;Cl, 8.17; F, 10.20; N, 4.91.

[Example 33]

(3R)-Amino-4-[5-(4-cyclohexylmethoxyphenyl)indolin-1-yl]-4-oxobutyric acid

(1) 5-(4-Benzyloxyphenyl)-1H-indole

[F154]

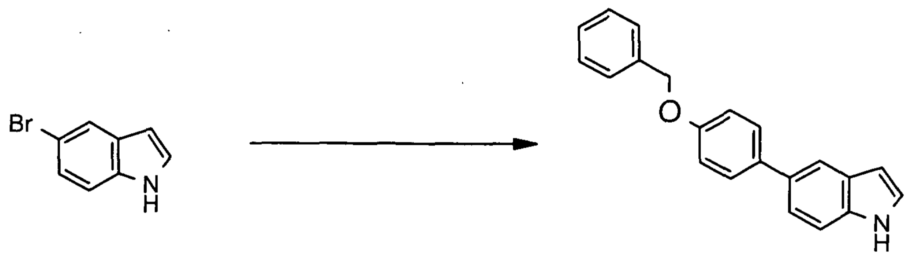

5-Bromoindole (1.00 g), 4-benzyloxyphenyl borate (2.33 g), tetrakis(triphenylphosphine)palladium (0.290 g), 2N aqueous sodium carbonate solution (12.8 mL), toluene (7 mL), and ethanol (7 mL) were mixed together, and the mixture was refluxed for 13 hours with stirring. The reaction mixture was left to stand to cool to room temperature and extracted with ethyl acetate (200 mL). The extract was washed with saturated brine (100 mL) and dried over sodium sulfate anhydrate. Solvent was evaporated, and the residue was purified by silica gel column chromatography (Yamazen Hi-Flash column 3L), to thereby yield the title compound (1.05 g) .
1H-NMR ($CDCl_3$) δ :
5.12 (2H, s), 6.58-6.60 (1H,m), 7.03-7.08(2H,m), 7.23(1H, t, J=2.8Hz), 7.31-7.49(7H, m), 7.55-7.59(2H,m), 8.14(1H,br s).
MS (ESI) m/z:300 $(M+H)^+$.

(2) 5-(4-Benzyloxyphenyl)-1-(tert-butoxycarbonyl)-1H-indole

[F155]

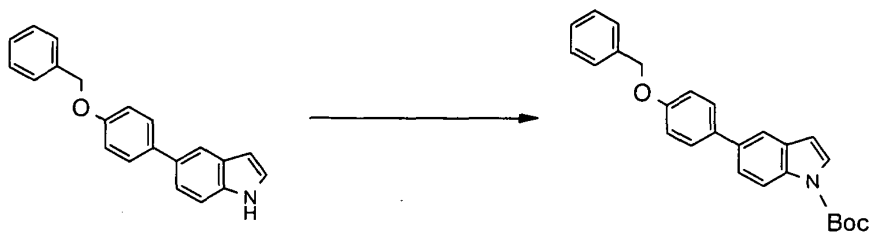

5-(4-Benzyloxyphenyl)-1H-indole (1.05 g) was dissolved in THF (10 mL), and to the solution, $Boc_2O$ (1.15 g) and DMAP (0.040 g) were added, followed by stirring for 17 hours. The reaction mixture was concentrated, and the residue was purified by silica gel column chromatography (Yamazen Hi-Flash column 3L), to thereby yield the title compound (1.29 g).
$^1$H-NMR ($CDCl_3$) δ :
1.69(9H,s), 5.12(2H,s), 6.59(1H,d,J=3.7Hz), 7.03-7.08(2H,m),7.30-7.42(3H,m),7.44-7.53(3H,m),7.54-7.62(3H,m), 7.72-7.70(1H,m), 8.15(1H,d,J=8.3Hz).
MS (ESI) m/z :400 $(M+H)^+$.
(3) 1-(tert-Butoxycarbonyl)-5-(4-hydroxyphenyl)indoline
[F156]

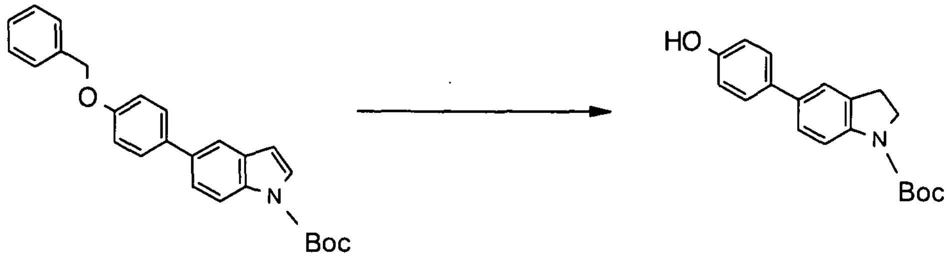

5-(4-Benzyloxyphenyl)-1-(tert-butoxycarbonyl)-1H-indole (1.29 g) was dissolved in a mixed solvent of THF (50 mL) and ethanol (50 mL), and 5% Pd/C (1.29 g) was added to the solution, followed by catalytic hydrogenation at normal pressure for 12 hours with stirring. The reaction vessel was purged with nitrogen, and then the catalyst was removed by filtration. The filtrate was concentrated, and the residue was purified by silica gel column chromatography (Yamazen Hi-Flash column 3L), to thereby yield the title compound (925 mg).
$^1$H-NMR ($CDCl_3$) δ :
1.58(9H,s), 3.13(2H,t,J=8.7Hz), 3.96-4.06(2H,m), 4.93(1H,s), 6.85-6.91(2H,m), 7.30-7.36(2H,m), 7.45-7.40(2H,m). 1H on the indoline ring not observed.
MS (ESI) m/z : 312 $(M+H)^+$.

(4) 1-(tert-Butoxycarbonyl)-5-(4-cyclohexylmethoxyphenyl)indoline

[F157]

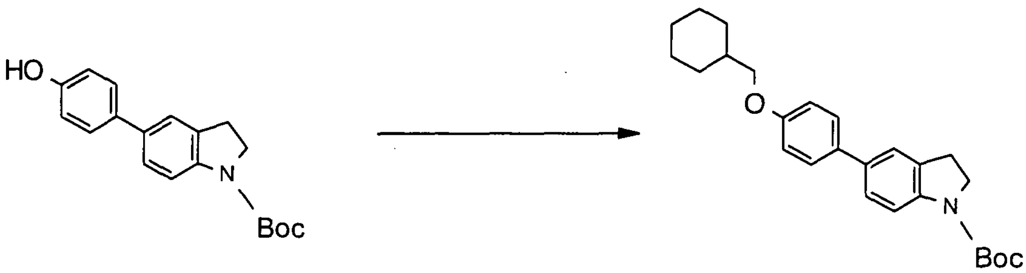

5-(4-Hydroxyphenyl)-1-(tert-butoxycarbonyl)-1H-indole (500 mg), cyclohexylmethyl bromide (453 μL), $K_2CO_3$ (333 mg), and DMF (16 mL) were mixed together, and the mixture was stirred at 80°C for 12 hours. The reaction mixture was left to stand to cool to room temperature and extracted with ethyl acetate (200 mL). The extract was washed with saturated brine (2 x 100 mL) and dried over sodium sulfate anhydrate, and then solvent was evaporated. The residue was purified by silica gel column chromatography (Yamazen Hi-Flash column 2L), to thereby yield the title compound (459 mg).
$^1$H-NMR ($CDCl_3$ ) δ :
0.93-1.37(6H,m), 1.53-1.93(13H,m), 3.13(2H, t, J=8.7Hz), 3.27(1H,d,J=6.4Hz), 3.78(2H,d,J =6.1Hz), 3.95-4.05(2H,m), 6.91-6.95(2H,m),7.34(2H,d,J=9.3Hz), 7.43-7.48(2H,m), 7.86(1H,br s).
MS (ESI) m/z : 352 $(M-tBu)^+$.

(5) (3R)-(tert-Butoxycarbonylamino)-4-[5-(4-cyclohexylmethoxyphenyl)indolin-1-yl]-4-oxobutyric acid tert-butyl ester

[F158]

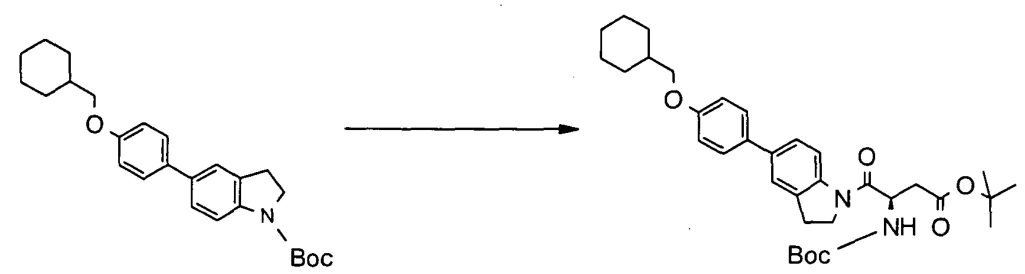

To 1-(tert-butoxycarbonyl)-5-(4-cyclohexylmethoxyphenyl)indoline (459 mg), 4N HCl/1,4-dioxane (10 mL) was added, and the mixture was stirred for 3 hours, followed by concentration. Subsequently, Boc-D-Asp(OBu-t)-OH (326 mg), EDC·HCl (324 mg), HOBt (228 mg), TEA (471 μL), and DMF (10 mL) were added thereto, followed by stirring for 12 hours. The reaction mixture was extracted with ethyl acetate (200 mL), and the extract was washed with saturated brine (2 x 100 mL), followed by drying over sodium sulfate anhydrate. Solvent was evaporated, and the residue was purified by silica gel column chromatography (Yamazen Hi-Flash column 3L), to thereby yield the title compound (400 mg).
$^1$H-NMR ($CDCl_3$) δ :
1.06(2H, ddd, J=23.62,12.15,2.99Hz), 1.15-1.38(4H,m), 1.41-1.45(18H,m),1.66-1.93(4H,m), 2.59(1H, dd, J=15.8,6.0Hz),2.85(1H, dd, J=15.9,7.6Hz), 3.26(2H,t,J=8.3Hz), 3.79(2H, d, J=6.8Hz), 4.29-4.48(2H,m), 4.95(1H, q, J=7.6Hz), 5.27-5.33 (1H,m), 6.92-6.96(2H,m), 7.36-7.40(2H,m), 7.44-7.49(2H,m), 8.24-8.19(1H,m). 1H on the indoline benzene ring not observed.

(6) (3R)-Amino-4-[5-(4-cyclohexylmethoxyphenyl)indolin-1-yl]-4-oxobutyric acid hydrochloride

[F159]

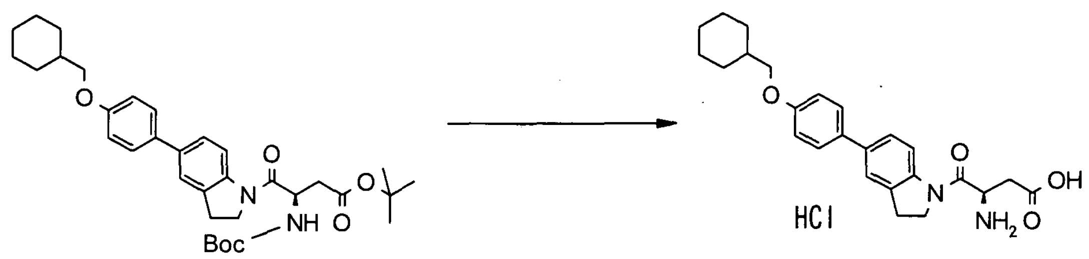

To (3R) - (tert-butoxycarbonylamino) -4- [5- (4-cyclohexylmethoxyphenyl)indolin-1-yl]-4-oxobutyric acid tert-butyl ester (400 mg), 4N HCl/1,4-dioxane (10 mL) was added, and the mixture was stirred for 3 hours, followed by concentration. Subsequently, the concentrate was slurried with diethyl ether. The slurry was filtered, and the collected solid was dried, to thereby yield the title compound (198 mg).
$^1$H-NMR (DMSO-$d_6$) δ:
0.98-1.33(5H,m),1.62-1.86(5H,m),2.78(1H, dd, J=17.33, 7.32Hz), 3.07(1H, dd, J=17.46,5.49 Hz), 3.25(2H, t, J=8.18Hz), 3.81(2H, d, J=6.35Hz), 4.18-4.37(3H,m),4.46-4.52(1H,m), 6.99(2H, d, J=8.79Hz), 7.47(1H, d, J=8.54Hz), 7.59-7.54 (3H, m), 8.11(1H, d, J=8.54Hz). $CO_2H$ or $NH_2HCl$ not observed.
MS (ESI) m/z :423 $(M+H)^+$.
Anal. Calcd for $C_{25}H_{30}N_2O_4$·HCl· $0.5H_2O$:C, 64.16;H, 6.89; Cl, 7.58;N, 5.99. Found:C,63.76;H,7.00;Cl, 7.38;N,6.07.

[Example 34]

(3R)-Amino-4-[5-(4-benzyloxyphenyl)indolin-1-yl]-4-oxobutyric acid

(1) 5-(4-Benzyloxyphenyl)-1-(tert-butoxycarbonyl)indoline

[F160]

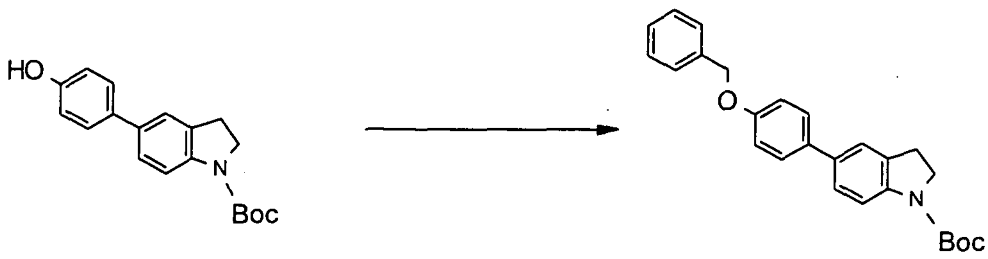

5-(4-Hydroxyphenyl)-1-(tert-butoxycarbonyl)-1H-indole (406 mg), benzyl bromide (310 μL), $K_2CO_3$ (270 mg) , and DMF (16 mL) were mixed together, and the mixture was stirred at 80°C for 12 hours. The reaction mixture was left to stand to cool to room temperature and extracted with ethyl acetate (200 mL). The extract was washed with saturated brine (2 x 100 mL) and dried over sodium sulfate anhydrate. Solvent was evaporated, and the residue was purified by silica gel column chromatography (Yamazen Hi-Flash column 2L), to thereby yield the title compound (446 mg).
$^1$H-NMR ($CDCl_3$) δ :
1.58(9H,br s),3.13(2H,t,J=8.67Hz),3.96-4.05(2H,m),5.10(2H,s),6.99-7.04(2H,m),7.30-7.50(9H,m), 7.87(0.5H,br s).
1/2H on the indoline benzene ring not observed.
MS (ESI) m/z : 402 (M+H)$^+$.

(2) (3R) - (tert-Butoxycarbonylamino) -4- [5- (4-cyclohexylmethoxyphenyl)indolin-1-yl]-4-oxobutyric acid tert-butyl ester

[F161]

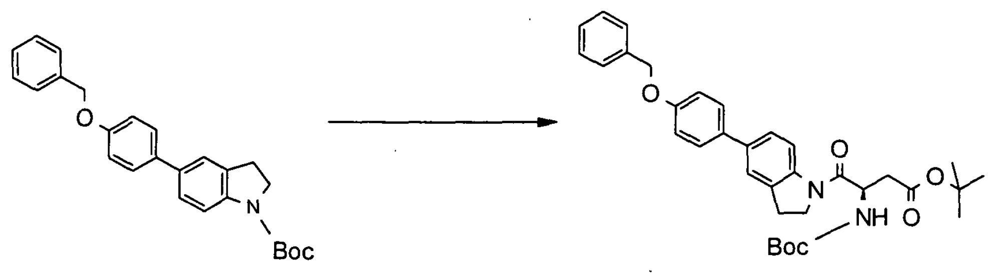

To 5-(4-benzyloxyphenyl)-1-(tert-butoxycarbonyl)indoline (446 mg), 4N HCl/1,4-dioxane (10 mL) was added, and the mixture was stirred for 3 hours, followed by concentration. Subsequently, Boc-D-Asp(OBu-t)-OH (387 mg), EDC·HCl (319 mg), HOBt (225 mg), TEA (464 μL), and DMF (10 mL) were added thereto, followed by stirring for 12 hours. The reaction mixture was extracted with ethyl acetate (200 mL). The extract was washed with saturated brine (2 x 100 mL) and dried over sodium sulfate anhydrate. Solvent was evaporated, and the residue was purified by silica gel column chromatography (Yamazen Hi-Flash column 3L), to thereby yield the title compound (198 mg).
$^1$H-NMR ($CDCl_3$) δ:
1.41-1.45(18H,m), 2.59(1H, dd, J=16.1,6.3Hz), 2.85(1H, dd, J=15.9, 7.6Hz) 3.26(2H, t, J=8.5Hz), 4.28-4.45(2H,m), 4.90-5.00(1H,m), 5.08-5.14(2H,m), 5.26-5.33(1H,m), 7.05-7.00(2H,m), 7.50-7.30(9H,m),8.22(1H, d, J=8.8Hz).

MS (ESI) m/z : 573 $(M+H)^+$.

(3) (3R) -Amino-4- [5- (4-benzyloxyphenyl) indolin-1-yl] -4-oxobutyric acid hydrochloride

[F162]

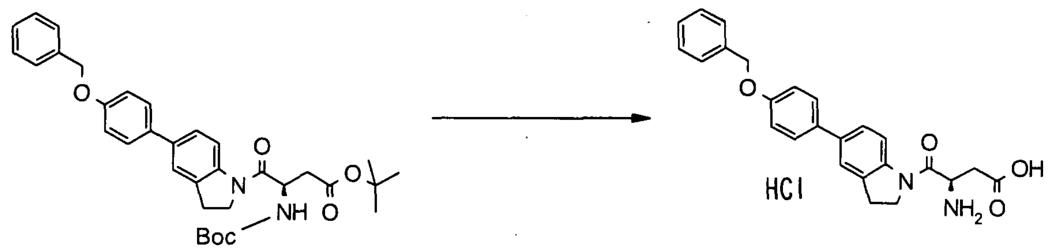

To (3R) - (tert-butoxycarbonylamino) -4- [5- (4-cyclohexylmethoxyphenyl)indolin-1-yl]-4-oxobutyric acid tert-butyl ester (446 mg), 4N HCl/1,4-dioxane (10 mL) was added, and the mixture was stirred for 3 hours, followed by concentration. The residue was slurried with diethyl ether. The slurry was filtered, and the collected solid was dried, to thereby yield the title compound (198 mg).
$^1$H-NMR(DMSO-$d_6$)δ:
2.47-2.54(1H,m),2.72-2.85(1H,m),3.25(2H,t,J=8.3Hz),4.17-4.37(1H,m),4.49(2H,t,J=5.9Hz),5.15(2H,s),7.09(2H,d, J=9.3Hz),7 .61-7.31(9H,m),8.11(1H,d,J=8.3Hz). $CO_2H$ or $NH_2HCl$ not observed.
MS (ESI) m/z : 417 $(M+H)^+$.
Anal. Calcd for $C_{25}H_{24}N_2O_4$. HCl:C,66.29;H,5.56;Cl,7.83;N,6.18.Found:C,66.96;H, 5.99;Cl, 7.8 2;N,5.90.

[Example 35]

(3R)-Amino-4-[5-[3-(2,4-difluorophenyl)propoxy]-1-indolinyl] - 4-oxobutyric acid

(1) 3-(2,4-Difluorophenyl)propanol

[F163]

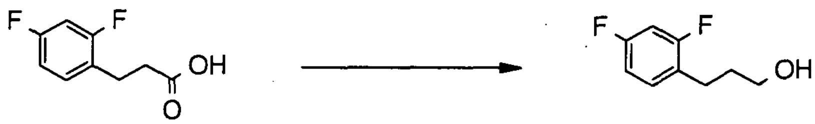

$LiAlH_4$ (0.430 g) was added to THF (10 mL) with stirring, and to the mixture, a solution of 3-(2,4-difluorophenyl) propionic acid (1.00 g) in THF (5 mL) was added dropwise at 0°C, and the mixture was stirred for 5 hours. To the reaction mixture, water (0.34 mL), 4N aqueous sodium hydroxide solution (0.34 mL), and then water (1.36 mL) were added, followed by stirring for 12 hours. The mixture was filtered off, and the filtrate was concentrated, to thereby yield the title compound (938 mg).
$^1$H-NMR ($CDCl_3$) δ:
1.43(1H,br s),1.80-1.89(2H,m),2.71(2H,t,J=7.7Hz),3.67(2H,t,J=6.3Hz),6.83-6.74(2H,m),7.19-7.12(1H,m).
MS (ESI)m/z:173 $(M+H)^+$.

(2) 3-(2,4-Difluorophenyl)-1-methanesulfonyloxypropane

[F164]

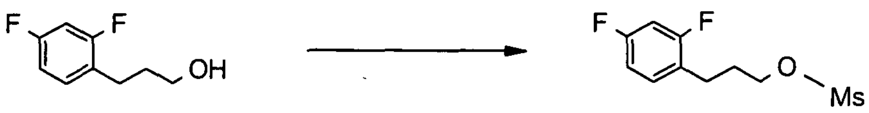

To a solution of 3-(2,4-difluorophenyl)propanol (938 mg) in dichloromethane (10 mL), TEA (1.52 mL) and MsCl (0.633 mL) were added with stirring at 0°C, and the mixture was further stirred for 2 hours. The reaction mixture was concentrated, and the residue was purified by silica gel column chromatography (Yamazen Hi-Flash column 3L), to thereby yield the title compound (1.19 g).
$^1$H-NMR($CDCl_3$)δ:
2.01-2.09(2H,m),2.75(2H,t,J=7.6Hz),3.01(3H,s),4.23(2H,t,J=6.3Hz),6 .76-6.85(2H,m),7.20-7.12(1H,m).
MS (ESI) m/z : 273 (M+Na)$^+$.

(3) 1- (tert-Butoxycarbonyl)-5-[3-(2,4-difluorophenyl)propoxy]indoline hydrochloride

[F165]

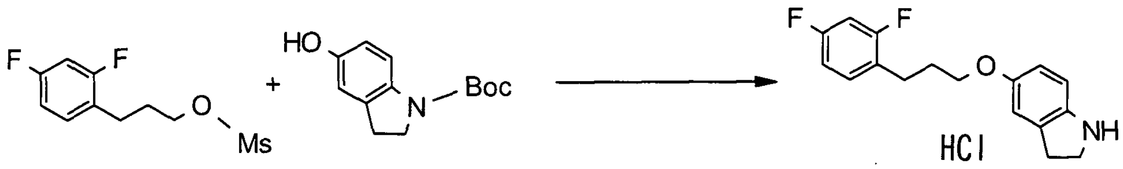

3-(2,4-Difluorophenyl)-1-methanesulfonyloxypropane (1.19 g), 1-(tert-butoxycarbonyl)-5-hydroxyindoline (1.12 g), potassium carbonate (0.990 g), and DMF (20 mL) were mixed together, and the mixture was stirred at 90°C for 16 hours. The reaction mixture was left to stand to cool to room temperature and extracted with ethyl acetate (200 mL). The extract was washed with saturated brine (2 x 100 mL) and dried over sodium sulfate anhydrate. Solvent was evaporated, and the residue was purified by silica gel column chromatography (Yamazen Hi-Flash column 3L), whereby an ether-form intermediate was yielded. Subsequently, the ether form was mixed with 4N HCl/1,4-dioxane (10 mL), and the mixture was stirred for 4 hours. The reaction mixture was concentrated, and the solid matter was collected by filtration, followed by drying, to thereby yield the title compound (1.16 g).
$^1$H-NMR ($CDCl_3$) δ:
2.03-2.11(2H,m),2.80(2H,t,J=7.6Hz),3.27(2H,t,J=7.7Hz),3.99-3.90(4H,m),6.86-6.75(4H,m),7.18-7.10(1H,m),7.55-7.50 (1H,m),11.59(2H,s).
MS (ESI) m/z:290(M+H)$^+$.

(4) (3R)-(tert-Butoxycarbonylamino)-4-[5-[3-(2,4-difluorophenyl)propoxy]-1-indolinyl]-4-oxobutyric acid tert-butyl ester

[F166]

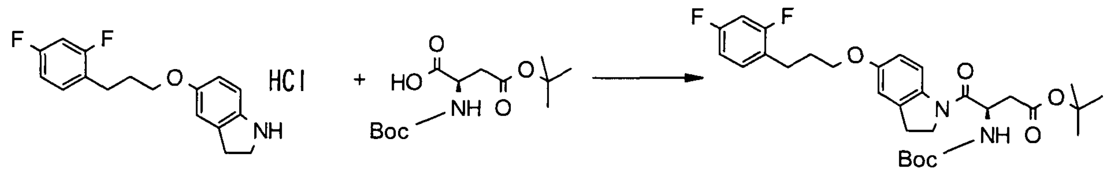

1- (tert-Butoxycarbonyl) -5- [3- (2, 4-difluorophenyl)propoxy]indoline hydrochloride (300 mg), Boc-D-Asp(O-tert-Bu)-OH (279 mg), EDC·HCl ( 265 mg), HOBt (187 mg), TEA (642 μL), and DMF (10 mL) were mixed together, and the mixture was stirred for 14 hours. The reaction mixture was extracted with ethyl acetate (200 mL). The extract was washed with saturated brine (2 x 100 mL) and dried over sodium sulfate anhydrate. Solvent was evaporated, and the residue was purified by silica gel column chromatography (Yamazen Hi-Flash column 2L), to thereby yield the title compound (493 mg).
$^1$H-NMR ($CDCl_3$) δ:
1.41-1.46(18H,m), 2.00-2.09(2H,m), 2.57(1H, dd, J=15.6,6.1Hz),2.75-2.86(3H,m), 3.18(2H, t, J=8.3Hz), 3.92(2H, t, J=6.2Hz), 4.25-4.40(2H,m),4.87-4.96(1H,m), 5.25-5.30(1H,m), 6.68-6.83(4H,m), 7.18-7.11(1H,m), 8.10(1H,d,J=8.8Hz).
MS (ESI) m/z : 561 $(M+H)^+$.

(5) (3R)-Amino-4-[5-[3-(2,4-difluorophenyl)propoxy] -1-indolinyl]-4-oxobutyric acid hydrochloride

[F167]

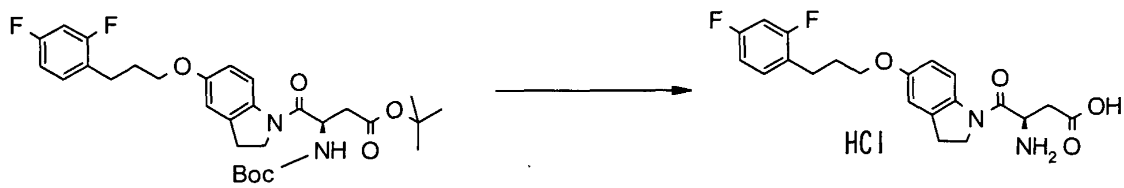

To (3R)-(tert-butoxycarbonylamino)-4-[5-[3-(2,4-difluorophenyl)propoxy]-1-indolinyl]-4-oxobutyric acid tert-butyl ester (493 mg), 4N HCl/1,4-dioxane (10 mL) was added, and the mixture was stirred for 1 day. The reaction mixture was concentrated, and the formed solid was dried, to thereby yield the title compound (266 mg).
$^1$H-NMR (DMSO-$d_6$) δ:
1.93-2.03(2H,m),2.71-4.48(11H,m),6.74-6.79(1H,m),6.88-6.90(1H,m),6.99-7.05(1H,m),7.14-7.21(1H,m), 7.32-7.39 (1H,m),7.98(1H, d, J=8.8Hz), 8.52(3H,br s). No proton peak attributed to NH or $CO_2H$ observed.
MS(ESI)m/z:405 $(M+H)^+$.
Anal. Calcd for $C_{21}H_{22}F_2N_2O_4$.0.5$H_2O$.
1.25HCl:C,54.95;H,5.33;Cl,9.66;F,8.28;N,6.10.Found:C,55.16;H, 5.57;Cl,9.26;F,7.69;N,5.96.

[Example 36]

(3R)-Amino-4-[5-(4-benzyloxy-3-trifluoromethylbenzyloxy)indolin-1-yl]-4-oxobutyric acid hydrochloride

(1) (4-Benzyloxy-3-trifluoromethylphenyl)methanol (WO 2003045925)

[F168]

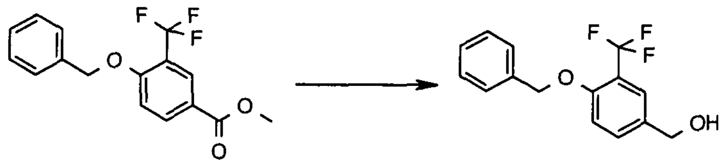

To a THF solution (40 mL) of 4-benzyloxy-3-trifluoromethylbenzoic acid methyl ester (Huang, Weiyuan; Qian, Zhaohui; Huaxue Xuebao (1987), 45(12), 1175-9) (1.24 g), lithium borohydride (261 mg) was added, and the mixture was stirred at reflux for 10 hours. The reaction mixture was left to stand to cool to room temperature, and 1N hydrochloric acid was added thereto. The resultant mixture was extracted twice with ethyl acetate. The extracts were combined and washed with saturated brine, followed by drying over sodium sulfate anhydrate. Insoluble matter was filtered off, and the filtrate was concentrated under reduced pressure. The residue was purified by flash column chromatography (Yamazen Hi-Flash column 2L), to thereby yield the title compound (977 mg).
$^1$H-NMR ($CDCl_3$) δ :
1.69(1H,t,J=5.9Hz),4.66(2H,d,J=5.9Hz), 5.20(2H,s),7.02(1H, d, J= 8.3Hz), 7.30-7.47(6H,m),7.61(1H, d, J=2.0Hz).
MS (ESI) m/z : 265 $(M-OH)^+$.

(2) 5-(4-Benzyloxy-3-trifluoromethylbenzyloxy)-1-(tert-butoxycarbonyl)indoline

[F169]

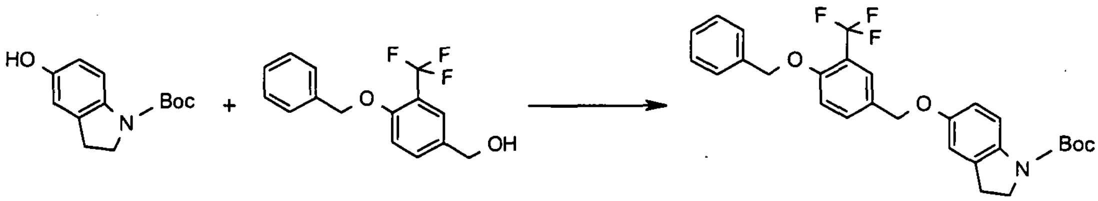

(4-Benzyloxy-3-trifluoromethylphenyl)methanol (870 mg), triphenylphosphine (808 mg), and DEAD (2.2M toluene solution) (1.40 mL) were added to a THF solution (20 mL) of 1-(tert-butoxycarbonyl)-5-hydroxyindoline (725 mg), and the mixture was stirred overnight at room temperature. The reaction mixture was concentrated under reduced pressure, and the residue was purified by flash column chromatography (Yamazen Hi-Flash column L), to thereby yield the title compound (981 mg).
$^1$H-NMR ($CDCl_3$) δ :
1.56 (9H, s), 3.0 (2H, t, J=8.7Hz), 3.96 (2H, s), 4.95 (2H, s), 5.20 (2H, s) ,6.73-6.78(2H,m), 7.03(1H, d, J=8.6Hz), 7.29-7.52 (6H,m), 7.65-7.75 (2H, m) .

(3) 4-[5-(4-Benzyloxy-3-trifluoromethylbenzyloxy)indolin-1-yl]-(3R)-[N-(tert-butoxycarbonyl)amino]-4-oxobutyric acid tert-butyl ester

[F170]

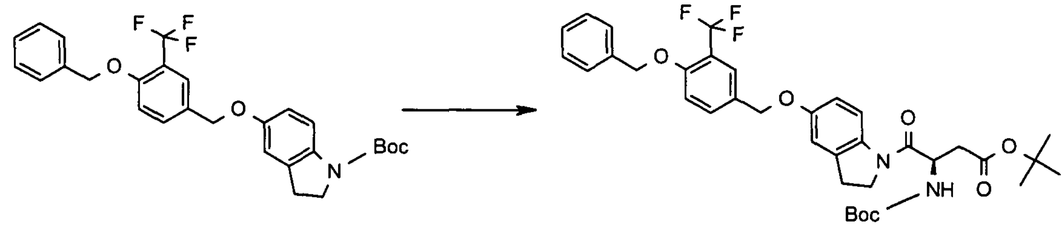

To 5-(4-benzyloxy-3-trifluoromethylbenzyloxy)-1-(tert-butoxycarbonyl)indoline (970 mg), 4N HCl/1,4-dioxane (20 mL) was added, and the mixture was stirred at room temperature for 1 hour. The reaction mixture was concentrated under reduced pressure, and the residue was dissolved in DMF (20 mL). To the solution, (2R)-[(tert-butoxycarbonyl) amino]succinic acid 4-tert-butyl ester (674 mg), HOBt (394 mg), EDC·HCl (558 mg), and TEA (1.35 mL) were added, followed by stirring overnight. To the reaction mixture, saturated aqueous sodium bicarbonate solution was added, and the resultant mixture was extracted twice with ethyl acetate. The extracts were combined and washed with saturated brine, followed by drying over sodium sulfate anhydrate. Insoluble matter was filtered off, and the filtrate was concentrated under reduced pressure. The residue was purified by flash column chromatography (Yamazen Hi-Flash column 2L), to thereby yield the title compound (1.22 g).

$^1$H-NMR ($CDCl_3$) δ :

1.42(9H,s), 1.43(9H,s), 2.57(1H, dd, J=6.0,15.6Hz), 2.82(1H, dd, J=7 .5,15.6Hz),3.18(2H,t,J=8.3Hz),4.26-4.39(2H,m), 4.90-4.97(3H,m),5.20-5.28(3H,m),6.76-6.81(2H,m), 7.03(1H, d, J=8.3Hz), 7.30-7.52(6H,m), 7.65(1H, d, J=2.0Hz), 8.12 (1H, d, J=8.8Hz).

MS (ESI) m/z : 671 $(M+H)^+$.

(4) (3R)-Amino-4-[5-(4-benzyloxy-3-trifluoromethylbenzyloxy)indolin-1-yl]-4-oxobutyric acid hydrochloride

[F171]

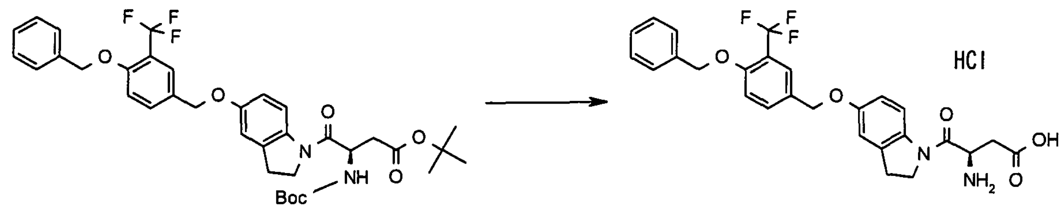

To 4-[5-(4-benzyloxy-3-trifluoromethylbenzyloxy)indolin-1-yl]-(3R)-[(tert-butoxycarbonyl)amino]-4-oxobutyric acid tert-butyl ester (100 mg), 4N HCl/1,4-dioxane (5.0 mL) was added, and the mixture was stirred overnight at room temperature. The reaction mixture was concentrated under reduced pressure, and the residue was purified by flash column chromatography (Yamazen Hi-Flash column L), to thereby yield the title compound (35 mg).

$^1$H-NMR (DMSO-$d_6$) δ:

2.24(1H, dd, J=8.9, 16.3Hz), 2.58(1H, dd, J=5.6,16.3Hz), 3.13(2H, t, J =8.5Hz), 4.00(1H,s), 4.09-4.15(1H,m),4.24-4.30 (1H,m), 5.05(2H,s), 5.29(2H,s), 6.80-6.83(1H,m), 6.96(1H,s), 7.31-7.46(6H,m), 7.67-7.71 (2H, m), 7.99 (1H, d, J=8.8Hz) .

IR(ATR)$cm^{-1}$:2913,1594,1488,1257,1120.

MS (ESI)m/z:515 $(M+H)^+$.

HR-MS(FAB)calcd for $C_{27}H_{26}F_3N_2O_5$ $(M+H)^+$: 515.1794 ;found 515.1810.

[Example 37]

(3R)-Amino-4-oxo-4-[5-(4-phenethylbenzyloxy)indolin-1-yl]butyric acid hydrochloride

(1) (4-Phenethylphenyl)methanol (WO 9616980)

[F172]

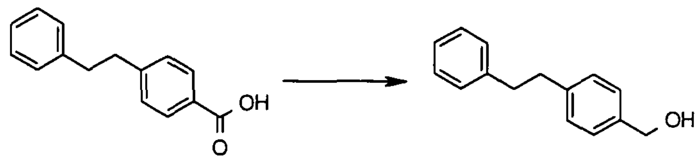

To a THF solution (10 mL) of 4-phenethylbenzoic acid (452 mg), TEA (419 μL) was added, and the mixture was cooled on ice. Ethyl chlorocarbonate (229 μL) was added thereto, and the resultant mixture was stirred under cooling on ice for 15 minutes. The formed precipitates were removed by filtration, and a filtrate was yielded. Separately, sodium borohydrate (454 mg) was suspended in ethanol (6.0 mL), followed by cooling to 0°C. To the cooled suspension, the thus-obtained filtrate was added dropwise over 10 minutes, followed by stirring at room temperature for 1 hour. To the reaction mixture, 1N hydrochloric acid was added, followed by extraction with ethyl acetate twice. The extracts were combined and washed sequentially with 1N aqueous sodium hydroxide solution and saturated brine. The extract was dried over sodium sulfate anhydrate and concentrated. The residue was purified by flash column chromatography (Yamazen Hi-Flash column L), to thereby yield the title compound (425 mg).
$^1$H-NMR ($CDCl_3$) δ:
2.89-3.02(4H,m), 4.64(2H,s), 7.16-7.37(9H,m).
MS (ESI) m/z : 195 (M-OH)$^+$.

(2) 1-(tert-Butoxycarbonyl)-5-(4-phenethylbenzyloxy)indoline

[F173]

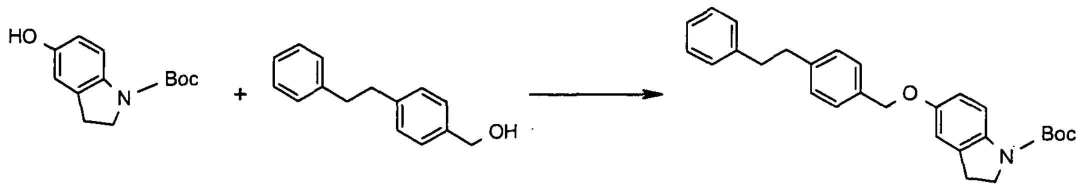

(4-Phenethylphenyl)methanol (271 mg), triphenylphosphine (334 mg) and DEAD (2.2 mol/L toluene solution) (580 μL) were added to a THF solution (10 mL) of 1-(tert-butoxycarbonyl)-5-hydroxyindoline (200 mg), and the mixture was stirred overnight at room temperature. The reaction mixture was concentrated under reduced pressure, and the residue was purified by flash column chromatography (Yamazen Hi-Flash column L), to thereby yield the title compound (300 mg).
$^1$H - NMR ($CDCl_3$) δ:
1.57(9H,s),2.91-3.07(6H,m),3.97(2H,s),4.92(2H,s),6.74-6.78(2H,m),7.13-7.41(9H,m),7.76(1H,s).

(3) (3R)-[N-(tert-Butoxycarbonyl)amino]-4-oxo-4-[5-(4-phenethylbenzyloxy)indolin-1-yl]butyric acid tert-butyl ester

[F174]

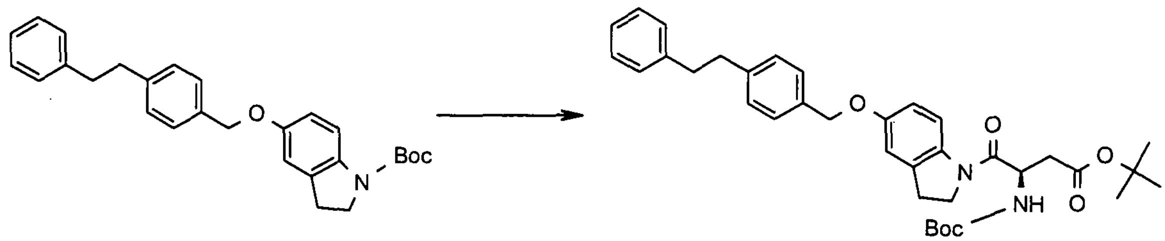

To 1-(tert-butoxycarbonyl)-5-(4-phenethylbenzyloxy)indoline (290 mg, 0.675 mmol), 4N HCl/1,4-dioxane (15 mL) was added, and the mixture was stirred at room temperature for 1 hour. The reaction mixture was concentrated under reduced pressure, and the residue was dissolved in DMF (15 mL). To the solution, (2R)-[(tert-butoxycarbonyl) amino]succinic acid 4-tert-butyl ester (194 mg), HOBt (137 mg), EDC·HCl (194 mg), and TEA (471 μL) were added, and the mixture was stirred overnight at room temperature. To the reaction mixture, saturated aqueous sodium bicarbonate solution was added, and the mixture was extracted twice with ethyl acetate. The extracts were combined and washed with saturated brine, followed by drying over sodium sulfate anhydrate. After filtration and concentration under reduced pressure, the residue was purified by flash column chromatography (Yamazen Hi-Flash column L), to thereby yield the title compound (365 mg).
$^1$H-NMR ($CDCl_3$ ) δ :
1.42(9H,s), 1.43(9H,s), 2.58(1H,dd,J=6.1,15.7Hz),2.83(1H,dd,J=7 .5,15.7Hz),2.91-3.00(4H,m),3.18(2H,t,J=8.3Hz), 4.26-4.40(2H,m),4.93(3H,s), 5.29(1H,d,J=9.3Hz),6.76-6.81(2H,m),7.12-7.40(9H,m),8.12(1H,d,J=8.8Hz).
MS (ESI)m/z:602 $(M+2)^+$.

(4) (3R)-Amino-4-oxo-4-[5-(4-phenethylbenzyloxy)indol-1-yl]butyric acid hydrochloride

[F175]

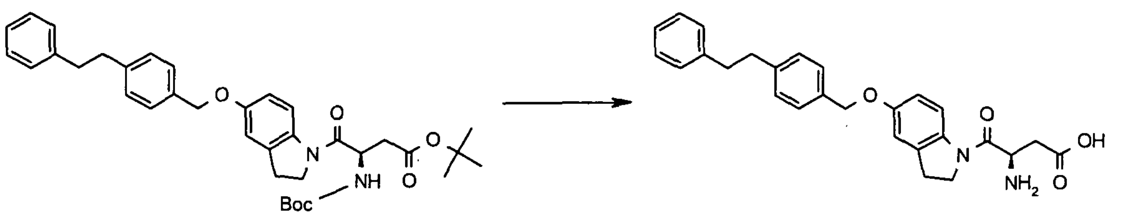

To (3R)-[N-(tert-butoxycarbonyl)amino]-4-oxo-4-[5-(4-phenethylbenzyloxy)indolin-1-yl]butyric acid tert-butyl ester (355 mg), 4N HCl/1,4-dioxane (10 mL) was added, and the mixture was stirred overnight at room temperature. The reaction mixture was concentrated under reduced pressure, and the residue was purified by flash column chromatography (Yamazen Hi-Flash column L), to thereby yield the title compound (205 mg).
$^1$H-NMR (DMSO-$d_6$) δ :
2.43-2.49(1H,m), 2.78(1H,dd,J=5.4,16.7Hz),2.85-2.95 (4H,m), 3.15 (2H, t, J=8.3Hz) , 4.12-4.31(3H,m), 5.03(2H,s), 6.84 (1H, dd, J=2.5,8.8Hz), 6.97(1H,d,J=2.5 Hz), 7.15-7.30(8H,m), 7.41(1H, d, J=7.4Hz), 8.00(1H,d,J=8.8Hz).
IR(ATR)$cm^{-1}$:2923,1650,1592,1486,1371,1263.

MS (ESI) m/z : 445 $(M+H)^+$.
HR-MS(FAB)calcd for $C_{27}H_{29}N_2O_4$ $(M+H)^+$: 445.2127; found 445.2127.

[Example 38]

(3R)-Amino-4-[5-[[2,4-bis (trifluoromethyl)benzyl]oxy]-2,3-dihydro-1H-indol-1-yl]-4-oxobutyric acid

(1) 5-Indolinol hydrochloride

[F176]

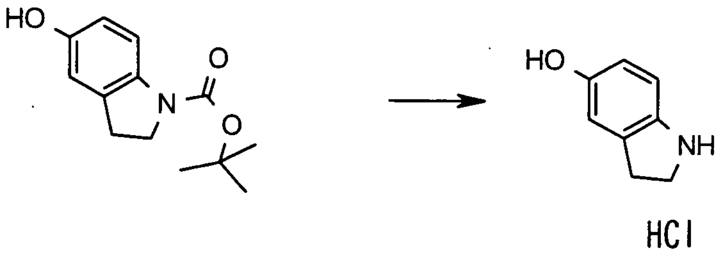

To 5-hydroxy-1-indoline carboxylic acid tert-butyl ester (500 mg), 4N HCl/1,4-dioxane (5.3 mL) was added at room temperature, and the mixture was stirred at room temperature for 3 hours. The reaction mixture was concentrated under reduced pressure, and the concentrate was slurried with hexane over 1 hour. The slurry was filtered, and the collected solid was dried (in vacuo at 40°C for 1 hour), to thereby yield the title compound (413 mg) in the form of a mixture containing 1,4-dioxane.
$^1$H-NMR(DMSO-$d_6$) δ:
3.12(2H,t,J=7.7Hz), 3.67(2H, t, J=7.7Hz), 6.75(1H, d, J=8.5Hz), 6.82 (1H,s),7.23(1H,d,J=8.5Hz),9.87(1H,s),10.84(1H, brs).
MS (ESI)m/z:136 $(M+H)^+$.

(2) (3R)-[N-(tert-Butoxycarbonyl)amino]-4-(5-hydroxy-2,3-dihydro-1H-indol-1-yl)-4-oxobutyric acid tert-butyl ester

[F177]

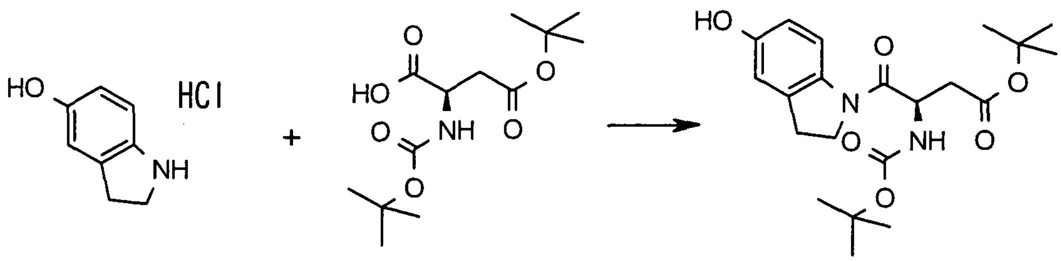

EDC·HCl (490 mg), HOBt (345 mg), and TEA (1.48 mL) were added at room temperature to a suspension of 5-indolinol hydrochloride (413 mg) and Boc-D-Asp(OBu-tert)-OH (commercially available) (616 mg) in DMF (10 mL), and the mixture was stirred for 15 hours. The reaction mixture was concentrated under reduced pressure, and the residue was purified by silica gel flash column chromatography (Biotage 40M), to thereby yield the title compound (406 mg).
$^1$H-NMR ($CDCl_3$) δ:

1.41(9H,s), 1.43(9H,s), 2.56(1H, dd, J=15.7,6.1Hz), 2.81(1H, dd, J=1 5.7,7.4Hz), 3.11(2H, t, J=8.3Hz), 4.20-4.36(2H, m), 4.85-4.93(1H,m), 5.04(1H,s), 5.29(1H, d, J=9.3Hz), 6.63(1H, d, J=8.5Hz), 6 .66(1H,s), 8.04 (1H, d, J=8.5Hz).
MS (ESI) m/z : 407 $(M+H)^+$.

(3) 4-[5-[[2,4-Bis(trifluoromethyl)benzyl]oxy]-2,3-dihydro-1H-indol-1-yl]-(3R)-[N-(tert-butoxycarbonyl)amino]-4-oxobutyric acid tert-butyl ester

[F178]

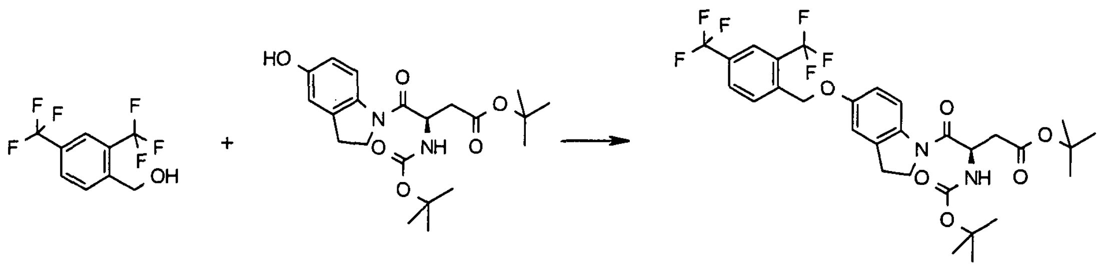

(3R)-[N-(tert-Butoxycarbonyl)amino]-4-(5-hydroxy-2,3-dihydro-1H-indol-1-yl)-4-oxobutyric acid tert-butyl ester (121 mg), triphenylphosphine (93.8 mg), and DEAD (2.2M toluene solution) (0.163 mL) were added at room temperature to a solution of [2,4-bis(trifluoromethyl)phenyl]methanol (commercially available) (80.0 mg) in THF (3.0 mL), and the mixture was stirred at room temperature for 15 hours. The reaction mixture was concentrated under reduced pressure, and the residue was purified by silica gel column flash chromatography (Biotage 25M), to thereby yield the title compound (47.5 mg).
$^1$H-NMR ($CDCl_3$ ) δ :
1.42(9H,s),1.43(9H,s),2.58
(1H, dd, J=15.8,6.1Hz), 2.83(1H, dd, J=15.8, 7.4Hz), 3.20(2H, t, J=8.3 Hz), 4.24-4.42(2H,m), 4.86-4.96(1H,m),5.23-5.33 (1H,m), 5.29(2H,s), 6.78(1H, dd, J=8.8,2.4Hz), 6.81(1H,s), 7.83 (1H, d, J=8.0Hz), 7.93(1H, d, J=8.0Hz), 7.94(1H,s), 8.13(1H, d, J=8.8H z) .

(4) (3R)-Amino-4-[5-[[2,4-bis (trifluoromethyl) benzyl] oxy] - 2,3-dihydro-1H-indol-1-yl]-4-oxobutyric acid TFA salt

[F179]

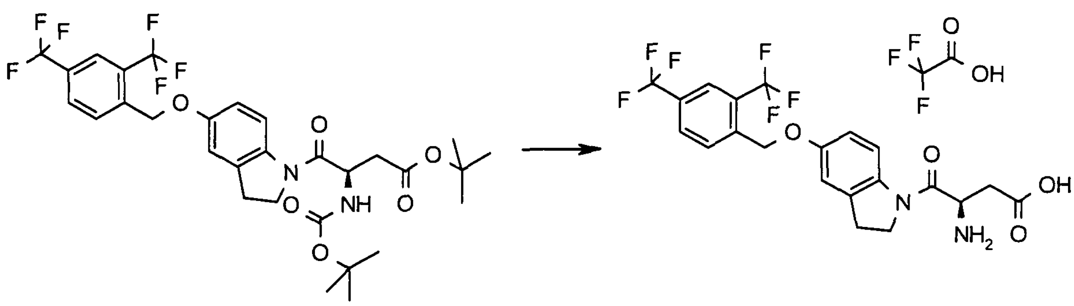

To a solution of 4-[5-[[2,4-bis(trifluoromethyl)benzyl]oxy]-2,3-dihydro-1H-indol-1-yl]-(3R)-[N-(tert-butoxycarbonyl)amino]-4-oxobutyric acid tert-butyl ester (47.0 mg) in dichloromethane (4.0 mL), TFA (1.0 mL) was added at room temperature. The reaction mixture was stirred at room temperature for 4 hours, followed by concentration under reduced pressure. Diethyl ether-hexane was added to the residue to form solid, and the formed solid matter was collected by filtration, followed by drying (in vacuo at room temperature for 1 hour), to thereby yield the title compound (23.0 mg).

$^1$H-NMR(DMSO-$d_6$) δ:
2.68(1H,dd,J=17.5,7.7Hz), 3.01(1H, dd, J=17.5, 5.0Hz), 3.16(2H,t,J =8.2Hz),4.12-4.28(2H,m),4.44(1H, dd, J=7.7, 5.0Hz), 5.31(2H,s), 6.87(1H,dd,J=8. 8,2.4Hz), 7.01(1H,s),7.96-8.04(2H,m),8.09(1H,s),8.14(1H,d,J=8.3Hz). $CO_2H$ or $NH_2$ not observed.
IR (ATR) $cm^{-1}$:2941,1732,1653,1597,1489,1346,1273,1176,1119,1084,1043,721.
MS (ESI) m/z : 477 $(M+H)^+$.
HR-MS(ESI)Calcd for $C_{21}H_{19}F_6N_2O_4$ $(M+H)^+$:477.12490. Found: 477.12219.
Anal. Calcd for $C_{21}H_{18}F_6N_2O_4$.1.0TFA·0.75H2O:C,47.74;H,3.42;F,28.31;N,4.64.Found:C,46.36;H,3.57;F, 27.75;N,4.53.

[Example 39]

(3S)-Amino-4-oxo-4-[5-[[4-phenyl-3-(trifluoromethyl)phenyl]methoxy]indolin-1-yl]butyric acid hydrochloride

(1) (3S)-[N-(tert-Butoxycarbonyl)amino]-4-oxo-4-[5-[[4-phenyl-3-(trifluoromethyl)phenyl]methoxy]indolin-1-yl]butyric acid tert-butyl ester

[F180]

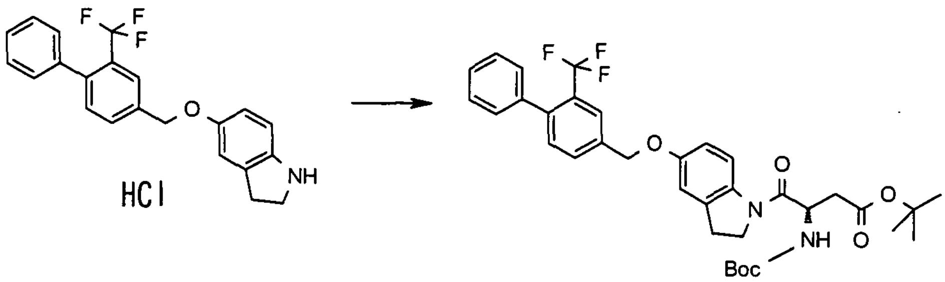

In a manner similar to that employed in step (1) of Example 32, 5-[(4-phenyl-3-trifluoromethylphenyl)methoxy] indoline hydrochloride (0.12 g) and Boc-L-Asp(OBu-tert)-OH (Watanabe Chemical Industries, Ltd.) (87 mg) were employed, to thereby yield the title compound (0.17 g).
$^1$H-NMR ($CDCl_3$) δ :
1.37-1.50(18H,m), 2.52-2.64(1H,m), 2.76-2.91(1H,m), 3.13-3.25(2H,m), 4.28-4.41(2H,m), 4.86-4.97(1H,m), 5.10(2H,s), 5.26-5.42(1H,m), 6.82(1H, dd, J=8.8,2.5Hz), 6.87(1H,s),7.29-7.43(5H,m),7.61(1H,d,J=8.1Hz),7.80(1H,s),8.14(1H,d, J=9.3Hz).
MS (ESI) m/z : 641 $(M+H)^+$.

(2) (3S)-Amino-4-oxo-4-[5-[(4-phenyl-3-trifluoromethylphenyl)methoxy]indolin-1-yl]butyric acid hydrochloride

[F181]

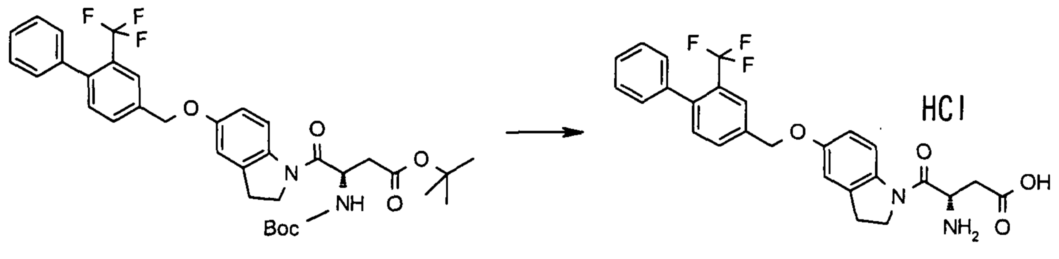

In a manner similar to that employed in step (2) of Example 32, (3S)-[N-(tert-butoxycarbonyl)-N-methylamino]-4-oxo-4-[5-[(4-phenyl-3-trifluoromethylphenyl)methoxy]indolin-1-yl]butyric acid tert-butyl ester (0.17g) was employed, to thereby yield the title compound (0.10 g).
$^1$H-NMR (DMSO-$d_6$) δ :
2.74(1H,dd,J=17.6,7.6Hz), 3.04(1H,dd,J=17.5,5.0Hz), 3.17(2H,t,J =8.2Hz), 4.08-4.31(2H,m), 4.40-4.49(1H,m),5.23(2H, s),6.91(1H,dd,J=8.9,2.6Hz),7.05(1H,d,J=2.5 Hz),7.28-7.33(2H,m),7.48-7.39(4H,m),7.76(1H,d,J=8.1Hz), 7.88(1H,s), 8.01(1H,d,J=8.8Hz), 8 .41 (1H, s) .
MS (ESI) m/z : 485 $(M+H)^+$.
IR(ATR)$cm^{-1}$:2883,1720,1651,1597,1485,1423.

[Example 40]

(3R)-(N-Methylamino)-4-oxo-4-[5-[(4-phenyl-3-trifluoromethylphenyl)methoxy]indolin-1-yl]butyric acid hydrochloride

(1) (2R)-[N-(tert-Butoxycarbonyl)-N-methylamino]succinic acid 4-methyl ester

[F182]

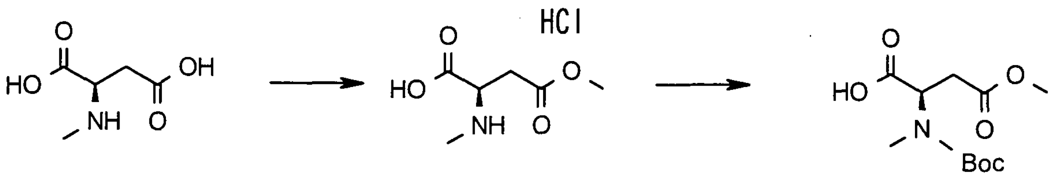

In a nitrogen atmosphere, methanol (10 mL) was cooled to -10°C, and thionyl chloride (0.62 mL) was added dropwise thereto. To the reaction mixture, D-Me-Asp(OH) monohydrate (Watanabe Chemical Industries, Ltd.) (1.0 g) was added, followed by stirring for 18 hours during which the mixture was allowed to warm to room temperature. The reaction mixture was concentrated, whereby (2R)-(N-methylamino)succinic acid 4-methyl ester hydrochloride was yielded. The substance was employed in the subsequent reaction without further purification.
$^1$H-NMR ($CD_3OD$) δ :
2.71(3H,s), 3.11-3.19(2H,m), 3.27-3.34(1H,m), 3.76(3H,s).
To a dioxane solution (10 mL) of (2R)-(N-methylamino)succinic acid 4-methyl ester hydrochloride, saturated aqueous sodium bicarbonate solution (10 mL) and $Boc_2O$ (1.6 g) were added, and the mixture was stirred for 14 hours. The reaction mixture was poured into 1N hydrochloric acid, and the resultant mixture was extracted with ethyl acetate. The extracts were combined and washed with saturated brine, followed by drying over sodium sulfate anhydrate. Solvent was evaporated, and the residue was purified by silica gel flash column chromatography (Yamazen Hi-Flash column L), to thereby yield the title compound (0.46 g).
$^1$H-NMR ($CD_3OD$) δ:
1.43(9H,s), 1.53(3H,s), 2.97-3.10(2H,m),3.64(3H,s), 4.86(1H,s).

(2) (3R)-[N-(tert-Butoxycarbonyl)-N-methylamino]-4-oxo-4-[5-[(4-phenyl-3-trifluoromethylphenyl)methoxy]indolin-1-yl] butyric acid methyl ester

[F183]

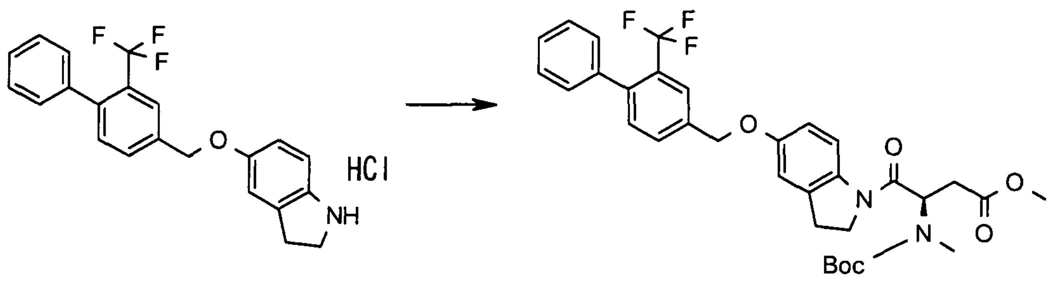

TEA (0.19 mL) was added at room temperature to a DMF solution (5 mL) of 5-[(4-phenyl-3-trifluoromethylphenyl) methoxy]indoline hydrochloride (0.11 g), (2R)-[N-(tert-butoxycarbonyl)-N-methyl]amino]succinic acid 4-methyl ester (0.95 mg), EDC·HCl (80 mg), and HOBt (56 mg), and the mixture was stirred for 3 days hours. The reaction mixture was concentrated, and the residue was purified by silica gel flash column chromatography (Biotage 25S), to thereby yield the title compound (0.13 g).
$^1$H-NMR ($CDCl_3$) δ :
1.49(9H,s),2.41-2.60(1H,m),2.73-2.85(3H,m),3.09-3.27(3H,m),3.48-3.78(3H,m),3.97-4.15(2H,m),4.20-4.34(1H,m), 5.11(2H,s),5.14-5.55(1H,m),6.82(1H,dd,J=9.6,2.2Hz),6.87(1H,s),7.30-7.43(5H,m),7.61(1H,d,J=7.1Hz),7.80(1H,s), 8.13(1H,d,J=8.6Hz).
MS(ESI)m/z:613(M+H)$^+$.

(3) (3R)-[N-(tert-Butoxycarbonyl)-N-methylamino]-4-oxo-4-[5-[(4-phenyl-3-trifluoromethylphenyl)methoxy]indolin-1-yl] butyric acid

[F184]

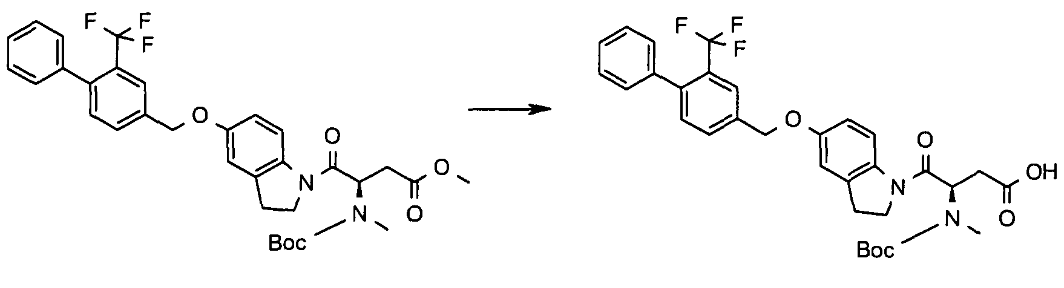

To a solution of (3R)-[N-(tert-butoxycarbonyl)-N-methylamino]-4-oxo-4-[5-[(4-phenyl-3-trifluoromethylphenyl) methoxy]indolin-1-yl]butyric acid methyl ester in a methanol/THF (1/2 mL) mixture, 1N aqueous sodium hydroxide solution (1 mL) was added at room temperature, and the mixture was stirred for 1 hour. The reaction mixture was concentrated, and 1N hydrochloric acid was added to the concentrate so as to adjust the pH thereof to 2, followed by extraction with a chloroform/methanol (10/1, v/v) mixture. The extracts were combined and washed with saturated brine, followed by drying over sodium sulfate anhydrate. Solvent was evaporated, whereby (3R)-[N-(tert-butoxycarbonyl)-N-methylamino]-4-oxo-4-[5-[(4-phenyl-3-trifluoromethylphenyl)methoxy]indolin-1-yl]butyric acid was yielded. The substance was employed in a subsequent step without further purification.
$^1$H-NMR ($CDCl_3$) δ :

1.48(9H,s),2.49-2.67(1H,m),2.75-2.88(3H,m),3.08-3.34(2H,m), 3.94-4.27(3H,m), 5.12(2H,s), 5.14-5.57(1H,m), 6.77-6.92(2H,m),7.27-7.47(6H,m),7.66-7.56(1H,m), 7.80(1H,s), 8.14(1H,d,J=8.8Hz).
MS (ESI) m/z : 599 (M+H)$^+$.

(4) (3R)-(Methylamino)-4-oxo-4-[5-[(4-phenyl-3-trifluoromethylphenyl)methoxy]indolin-1-yl]butyric acid hydrochloride

[F185]

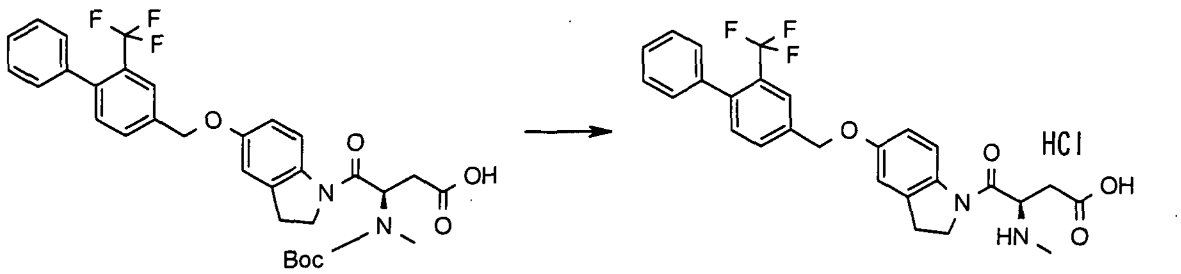

To (3R)-[N-(tert-butoxycarbonyl)-N-methylamino]-4-oxo-4-[5-[(4-phenyl-3-trifluoromethylphenyl)methoxy]indolin-1-yl]butyric acid (0.12 g), 4N HCl/1,4-dioxane (3 mL) was added at room temperature, and the mixture was stirred for 6 hours. The reaction mixture was concentrated, and diethyl ether was added to the concentrate to form solid. The formed solid was collected by filtration and dried, to thereby yield the title compound (64 mg).
$^1$H-NMR(DMSO-d$_6$)δ:
2.49(3H,s),2.80-3.57(4H,m),4.09(1H,q,J=9.0Hz),4.28(1H,dd,J=18.1,8.3Hz),4.41(1 H,t,J=6.3Hz),5.16(2H,s),6.83(1H, dd, J=8.8,2.2Hz), 6.96-6.98 (1H, m), 7.20-7.25 (2H, m), 7.38-7.31 (5H, m), 7.68 (1H, d, J=8.1Hz), 7.80 (1H, s), 7.94 (1H, d, J=8.8Hz).
MS (ESI) m/z : 499 (M+H)$^+$.
HR-MS (FAB) calcd for $C_{27}H_{26}F_3N_2O_4$:499.1845. Found: 499.1813.
IR (ATR)cm$^{-1}$: 3031, 2924, 1724, 1649, 1597, 1570 .

[Example 41]

(3R)-(Methylamino)-4-oxo-4-[5-[(4-phenyl-5-trifluoromethyl-2-thienyl)methoxy]indolin-1-yl]butyric acid hydrochloride

(1) (3R)-[N-(tert-Butoxycarbonyl)-N-methylamino]-4-oxo-4-[5-[(4-phenyl-5-trifluoromethyl-2-thienyl)methoxy]indolin-1-yl]butyric acid methyl ester

[F186]

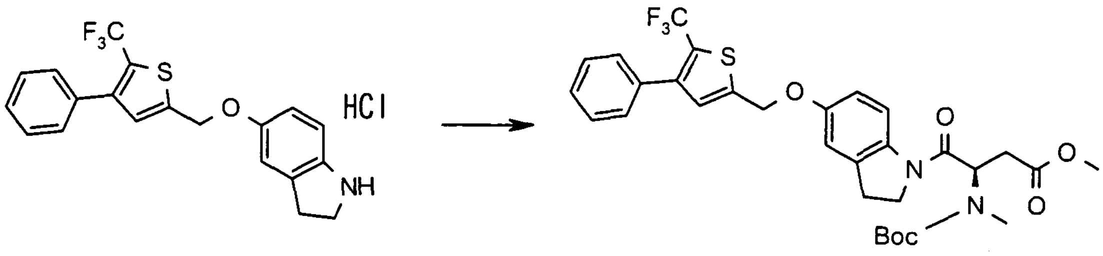

In a manner similar to that employed in step (4) of Example 40, 5-[(4-phenyl-5-trifluoromethyl-2-thienyl)methoxy] indoline hydrochloride (0.13 g) was employed, whereby the title compound (0.16 g) was yielded.
$^1$H -NMR ($CDCl_3$) δ :
1.48(9H,s),2.41-2.63(1H,m),2.75-2.87(3H,m),3.03-3.26(3H,m),3.62-3.74(3H,m),3.95-4.34(2H,m),5.01-5.24(2H,m), 5.38-5.52(1H,m),6.71-6.91(2H,m),7.07(1H,s),7.36-7.45(5H,m),8.21-8.07(1H,m).
MS (ESI) m/z : 619 $(M+H)^+$.

(2) (3R)-[N-(tert-Butoxycarbonyl)-N-methylamino]-4-oxo-4-[5-[[4-phenyl-5-(trifluoromethyl)-2-thienyl]methoxy]indolin-1-yl]butyric acid

[F187]

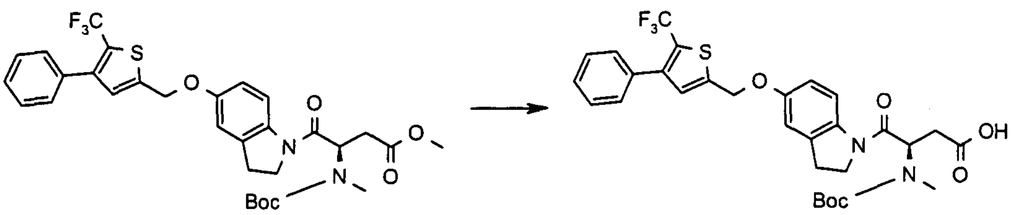

In a manner similar to that employed in step (3) of Example 40, (R)-3-[N-(tert-butoxycarbonyl)-N-methylamino]-4-oxo-4-[5-[(4-phenyl-5-trifluoromethyl-2-thienyl)methoxy]indolin-1-yl]butyric acid methyl ester (0.16 g) was employed, whereby the title compound (0.19 g) was yielded.
$^1$H-NMR ($CDCl_3$) δ:
1.47(9H,s),2.56(1H,dd,J=15.9,5.6Hz),2.77 and 2.81(total 3H,each s),3.06-3.28(3H,m),3.98-4.30(2H,m),5.20(3H,s), 5.45-5.52(1H,m),6.81(1H,d,J=9.6Hz),6.85(1H,s),7.06(1H,s),7.37-7.45(5H,m),8.13(1H,d,J=9.8Hz).
MS (ESI) m/z : 605 $(M+H)^+$.

(3) (3R)-(Methylamino)-4-oxo-4-[5-[(4-phenyl-5-trifluoromethyl-2-thienyl)methoxy]indolin-1-yl]butyric acid hydrochloride

[F188]

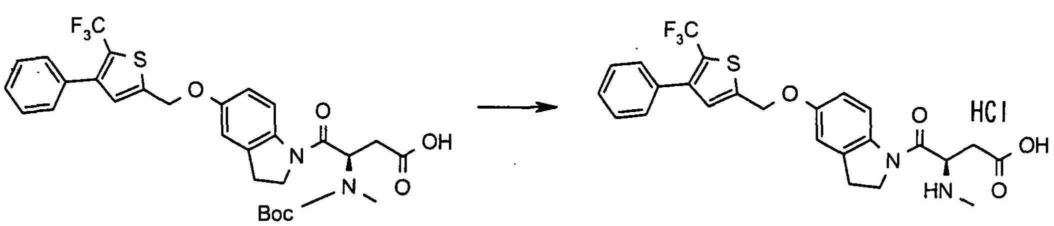

In a manner similar to that employed in step (4) of Example 40, (3R)-[N-(tert-butoxycarbonyl)-N-methylamino]-4-oxo-4-[5-[(4-phenyl-5-trifluoromethyl-2-thienyl)methoxy]indolin-1-yl]butyric acid was employed, whereby the title compound (60 mg) was yielded.
$^1$H-NMR (DMSO-$d_6$) δ :
2.57(3H,s), 2.88(1H,dd,J=17.4,6.1Hz), 3.06(1H,dd,J=17.2,6.6Hz), 3.17(2H,t,J=7.6Hz), 4.17(1H,q,J=8.6Hz), 4.34(1H, dd,J=18.3,8.2Hz ),4.49(1H,t,J=6.0Hz),5.38(2H,s),6.92(1H,dd,J=8.8,2.7Hz),7.05( 1H,d,J=2.5Hz),7.41-7.51(5H,m),8.02 (1H,d,J=8.8Hz),9.26(1H,s).
MS(ESI)m/z:505(M+H)$^+$.

[Example 42]

(3R)-(N-Methylamino)-4-oxo-4-[5-[[2,4-bis(trifluoromethyl)phenyl]methoxy]indolin-1-yl]butyric acid

(1) 5-[2,4-Bis(trifluoromethyl)phenyl]methoxyindoline hydrochloride

[F189]

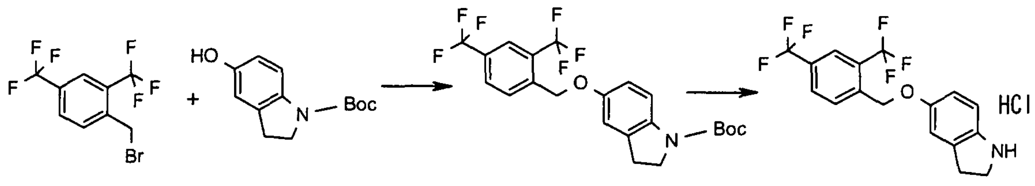

Potassium carbonate (9.0 g) was added at room temperature to a solution (100 mL) of 2,4-bis(trifluoromethyl) benzyl bromide (Aldrich) (4.86 g) and 1-(tert-butoxycarbonyl)-5-hydroxyindoline (3.8 g) in DMF, and the resultant mixture was heated to 50°C, followed by stirring for 4 days. The reaction mixture was cooled to room temperature, and insoluble matter was removed through filtration, and the filtrate was concentrated, to thereby yield 1-(tert-butoxycarbonyl)-5-[2,4-bis(trifluoromethyl)phenyl]methoxyindoline, which was employed in a subsequent step without further purification.
1-(tert-Butoxycarbonyl)-5-[2,4-bis(trifluoromethyl)phenyl]methoxyindoline was dissolved in 4N HCl/1,4-dioxane (50 mL), and the resultant solution was stirred for 1 day at room temperature. The reaction mixture was concentrated, and diethyl ether was added to the residue, whereby the title compound (5.6 g) was yielded.
$^1$H-NMR(DMSO-$d_6$)δ:
3.10-3.22(2H,m),3.60-3.77(2H,m),5.35(2H,s),6.99(1H,d,J=8.6Hz),7.14(1H,s),7.33(1H,d ,J=8.3Hz), 8.02(1H,d, J=8.3Hz), 8.10(1H,s), 8.15(1H,d,J=10.0Hz), 1 0.89(1H,s).

(2) 4-[5-[[2,4-Bis(trifluoromethyl)phenyl]methoxy] indolin-1-yl]-(3R)-[(N-tert-butoxycarbonyl-N-methyl)amino]-4-oxobutyric acid methyl ester

[F190]

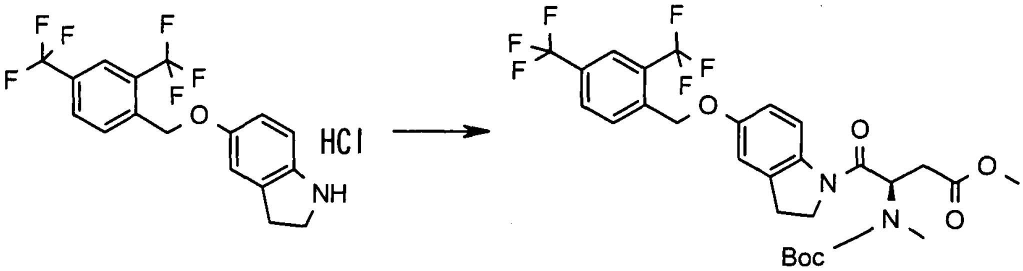

DIEA (0.44 mL) was added at room temperature to a solution (5 mL) of 5-[2,4-bis(trifluoromethyl)phenyl] methoxyindoline hydrochloride (0.20 g), Boc-D-Me-Asp(OMe)-OH (0.13 g), EDC·HCl (0.14 g), and HOBt (0.10 g) in DMF, and the resultant mixture was stirred for 2 days hours. The reaction mixture was concentrated, and the residue was purified by silica gel flash column chromatography (Biotage 25S), whereby the title compound (0.18 g) was yielded.
$^1$H-NMR ($CDCl_3$) δ:
1.22-1.48(9H,m),2.45-2.87(4H,m),2.88-3.25(3H,m),3.58-3.78(3H,m),3.98-4.37(2H,m),5.29(2H,s),5.44-5.55(1H,m), 6.63-6.86(2H,m),7.83(1H,d,J=7.8Hz),7.93(2H,d,J=9.8Hz),8.12(1H,d,J= 8.6Hz).
MS(ESI)m/z:605(M+H)$^+$.

(3) 4-[5-[[2,4-Bis(trifluoromethyl)phenyl]methoxy] indolin-1-yl]-(3R)-[N-(tert-butoxycarbonyl)-N-methylamino]-4-oxobutyric acid

[F191]

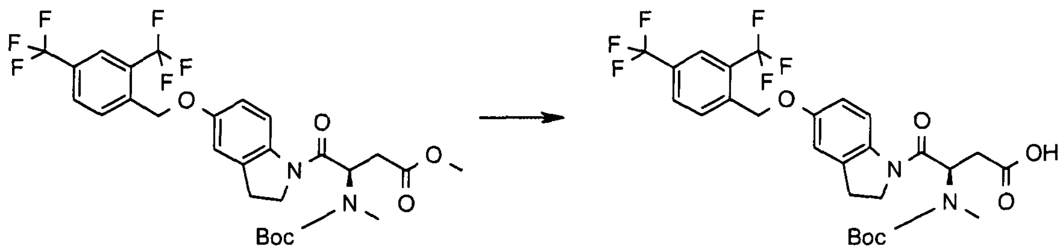

A 1N aqueous sodium hydroxide solution (1 mL) was added at room temperature to a methanol/THF mixture (1/2 mL) of 4-[5-[[2,4-bis(trifluoromethyl)phenyl]methoxy]indolin-1-yl]-(3R)-[(N-tert-butoxycarbonyl-N-methyl)amino]-4-oxobutyric acid methyl ester, and the resultant mixture was stirred for 1 hour. The reaction mixture was concentrated, and the pH of the resultant mixture was adjusted to 2 with 1N hydrochloric acid, followed by extraction with a chloroform/ methanol mixture (10/1, v/v). The extracts were combined and washed with saturated brine, followed by drying over sodium sulfate anhydrate. Solvent was evaporated, and the residue was purified by thin-layer chromatography, whereby the title compound (0.16 g) was yielded.
$^1$H-NMR ($cDCl_3$)δ:
1.40-1.54(9H,m),2.48-2.62(1H,m),2.70-2.85(3H,m),3.06-3.29(2H,m),3.95-4.33(2H,m),5.03-5.15 and 5.42-5.51(total 1H,each m,amide isomers),5.29(2H,s),6.67-7.00(2H,m),7.82(1H,d,J=8.3Hz),7.85-8.00(2H,m),8.12(1H,d,J=8.8Hz).
MS(ESI)m/z:591(M+H)$^+$.

(4) 4-[5-[[2,4-Bis(trifluoromethyl)phenyl]methoxy]indolin-1-yl]-(3R)-(N-methylamino)-4-oxobutyric acid

[F192]

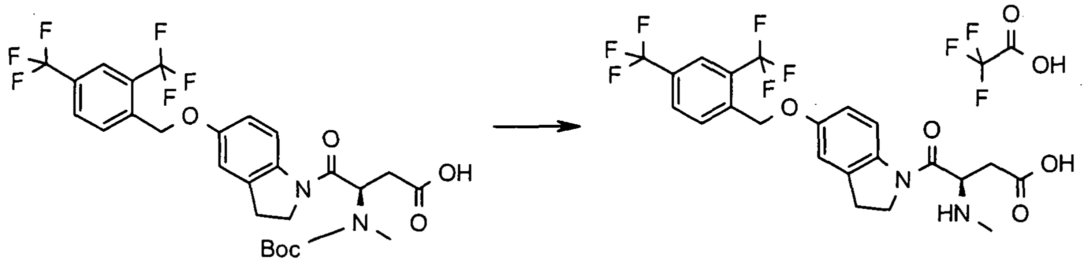

TFA (2 mL) was added at room temperature to a solution (10 mL) of 4-[5-[[2,4-bis(trifluoromethyl)phenyl] methoxy]indolin-1-yl]-(3R)-[N-(tert-butoxycarbonyl)-N-methylamino]-4-oxobutyric acid (0.16 g) in dichloromethane, and the resultant mixture was stirred for 5 hours. The reaction mixture was concentrated, and n-hexane was added to the residue. Precipitated solid was collected by filtration and dried, whereby the title compound (0.11 g) was yielded.
$^1$H-NMR (DMSO-$d_6$) δ:
2.60(3H,d,J=6.6Hz),2.75-3.26(4H,m),3.47-3.65(1H,m),3.96-4.38(2H,m),4.51(1H,t,J=6.1Hz),5.31(2H,s),6.78-6.92(1H, m),7.01(1H,d,J=13.0Hz),8.19-7.92(4H,m).
IR(ATR)cm$^{-1}$:3079,2875,1641,1579,1491,1435,1390.

[Example 43]

4-[5-[[4-(Cyclohexyl)-2-(trifluoromethyl)phenyl] methoxy] indolin-1-yl]-(3R) - (methylamino)-4-oxobutyric acid

(1) (3R)-[N-(tert-Butoxycarbonyl)-N-methylamino]-4-[5-[(4-cyclohexyl-2-trifluoromethylphenyl)methoxy]indolin-1-yl]-4-oxobutyric acid methyl ester

[F193]

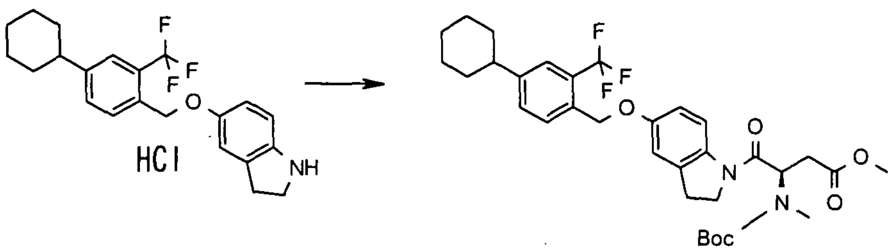

TEA (0.35 mL) was added at room temperature to a solution (5 mL) of 5-[(4-cyclohexyl-2-trifluoromethylphenyl) methoxyindoline hydrochloride (0.21 g), Boc-D-Me-Asp(OMe)-OH (0.13 g), EDC·HCl (0.14 g), and HOBt (0.10 g) in DMF, and the resultant mixture was stirred for 2 days hours. The reaction mixture was concentrated, and the residue was purified by silica gel flash column chromatography (Biotage 25S), whereby the title compound (176 mg) was yielded.
$^1$H-NMR ($CDCl_3$) δ:
1.19-1.52(13H,m),1.67-1.96(6H,m),2.42-2.62(2H,m),2.71-2.87(3H,m),3.01-3.27(3H,m),3.62-3.73(3H,m),3.88-4.35 (2H,m),5.19(2H,s),5.39-5.55(1H,m),6.74-6.87(2H,m), 7.38(1H,d,J=7.8Hz), 7.51(1H,s), 7.60(1H,d, J=7.8Hz),8 .10(1H,d, J=8.6Hz).
MS(ESI)m/z:619(M+H)$^+$.

(2) (3R)-[N-(tert-Butoxycarbonyl)-N-methylamino]-4-[5-[[4-(cyclohexyl)-2-(trifluoromethyl)phenyl]methoxy]indolin-1-yl]-4-oxobutyric acid

[F194]

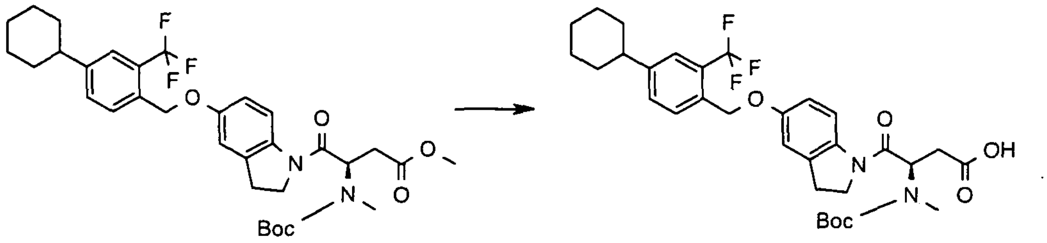

A 1N aqueous sodium hydroxide solution (1 mL) was added at room temperature to a solution of (3R)-[N-(tert-butoxycarbonyl)-N-methylamino]-4-[5-[(4-cyclohexyl-2-trifluoromethylphenyl)methoxy]indolin-1-yl]-4-oxobutyric acid methyl ester (176 mg) in a methanol/THF mixture (1/2 mL), and the resultant mixture was stirred for 1 hour. The reaction mixture was concentrated, and 1N hydrochloric acid was added to the residue. Precipitated solid was collected by filtration and dried under reduced pressure, whereby the title compound (170 mg) was yielded.
$^1$H-NMR ($CDCl_3$) δ:
1.22-1.45(15H,m),1.59-1.91(6H,m),2.48-2.63(2H,m),2.70-2.81(3H,m),2.95-3.22(2H,m),3.81-4.31(1H,m),5.16(2H,s), 5.45(1H,d,J=9.3Hz),6.75-6.79(2H,m), 7.00(1H,s), 7.38(1H,d,J=7.6Hz), 7.51(1H,d,J=4.7Hz), 7 .59(1H,d,J=8.1Hz), 8.09 (1H,d,J=8.8Hz).
MS (ESI)m/z:605 $(M+H)^+$.

(3) 4-[5-[(4-Cyclohexyl-2-trifluoromethylphenyl)methoxy]indolin-1-yl]-(3R)-(methylamino)-4-oxobutyric acid

[F195]

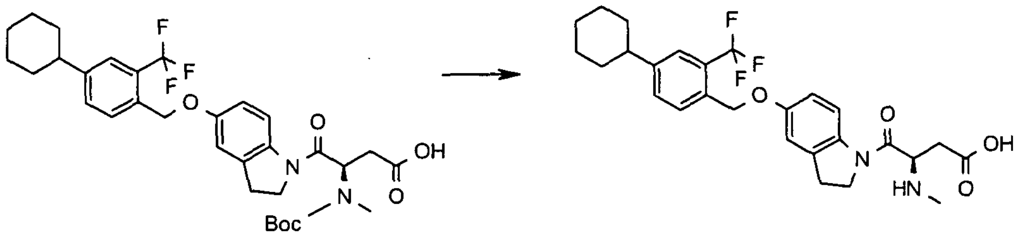

TFA (2 mL) was added at room temperature to a solution (10 mL) of (3R)-[N-(tert-butoxycarbonyl)-N-methylamino]-4-[5-[(4-cyclohexyl-2-trifluoromethylphenyl)methoxy]indolin-1-yl]-4-oxobutyric acid (190 mg) in dichloromethane, and the resultant mixture was stirred for 23 hours. The reaction mixture was concentrated, and the residue was diluted with water. The pH of the resultant mixture was adjusted to 7 with 1N sodium hydroxide. The mixture was extracted with a chloroform/methanol mixture (10/1, v/v). The extracts were combined and washed with saturated brine, followed by drying over sodium sulfate anhydrate. Solvent was evaporated, and the residue was purified by thin-layer chromatography. The oily product was freeze-dried from dioxane, whereby the title compound (25 mg) was yielded.
$^1$H-NMR (DMSO-$d_6$) δ:
0.82-1.27(4H,m),1.35-1.85(6H,m),2.26-2.38(3H,m),2.81(2H,t,J=6.5Hz),3.10(2H,t,J=8.3Hz),3.35-3.72(2H,m),3.96-4.07 (4H,m),4.94(2H,s),6.78(1H,dd,J=8.7,2.6Hz),6.89(1H,s),7.96 (1H,d,J=8.8Hz),8.04(1H,s).
IR(ATR)$cm^{-1}$:2922,2850,1728,1643,1620,1595,1493.
MS(ESI)m/z:505$(M+H)^+$.
Anal. Calcd for
$C_{26}H_{29}F_3N_2O_4 \cdot 1.25HCl \cdot H_2O$:C,56.36;H,5.87;Cl,8.00;F,10.29;N,5.06.
Found:C,56.52;H,5.88;Cl,8.17;F,10.20;N,4.91.

[Example 44]

4-[5-[(4-Cyclohexyl-3-trifluoromethylphenyl)methoxy]indolin-1-yl]-(3R)-(N-methylamino)-4-oxobutyric acid

(1) (3R)-[N-(tert-Butoxycarbonyl)-N-methylamino]-4-[5-[(4-cyclohexyl-3-trifluoromethylphenyl)methoxy]indolin-1-yl]-4-oxobutyric acid methyl ester

[F196]

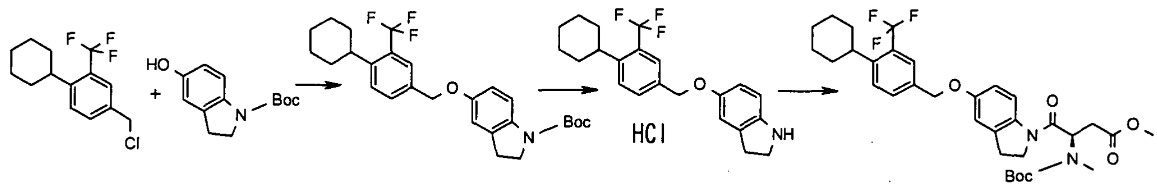

Potassium carbonate (1.48 g) was added at room temperature to a solution (20 mL) of 4-cyclohexyl-3-trifluoromethylbenzyl chloride (0.99 g) and 1-(tert-butoxycarbonyl)-5-hydroxyindoline (0.84 g) in DMF, and the resultant mixture was heated to 60°C, followed by stirring for 4 days. The reaction mixture was cooled to room temperature. Insoluble matter was removed by filtration, and the filtrate was concentrated, to thereby yield 1-(tert-butoxycarbonyl)-5-[(4-cyclohexyl-3-trifluoromethylphenyl)methoxy]indoline (340 mg), which was employed in a subsequent step without further purification.
1-(tert-Butoxycarbonyl)-5-[(4-cyclohexyl-3-trifluoromethylphenyl)methoxy]indoline (340 mg) was dissolved in 4N HCl/1,4-dioxa (20 mL), and the resultant solution was stirred for 14 hours at room temperature. The reaction mixture was concentrated, to thereby yield 5-[(4-cyclohexyl-3-trifluoromethylphenyl)methoxy]indoline hydrochloride (460 mg), which was employed in a subsequent step without further purification.
DIEA (0.39 mL) was added at room temperature to a solution (5 mL) of the above 5-[(4-cyclohexyl-3-trifluoromethylphenyl)methoxy]indoline hydrochloride (185 mg), Boc-D-Me-Asp(OMe)-OH (0.24 g), EDC·HCl (0.17 g), and HOAt (0.13 g) in DMF, and the resultant mixture was stirred for 18 hours. The reaction mixture was concentrated, and the residue was purified by silica gel flash column chromatography (Biotage 25S), whereby the title compound (227 mg) was yielded.
$^1$H-NMR($CDCl_3$)δ:
1.19-1.54(12H,m),1.73-1.89(2H,m),2.45-3.36(8H,m),3.48-3.81(6H,m),3.92-4.41(3H,m),4.52-4.88(1H,m),5.01(2H,s),5.08-5.56(1H,m),6.79(1H,d,J=9.1Hz),6.84(1H,s),7.41-7.59(2H,m),7.65(1H,s),8.11(1H,d,J=8.8Hz).
MS (ESI) m/z : 619 (M+H) .

(2) 4-[5-[(4-Cyclohexyl-3-trifluoromethylphenyl)methoxy]indolin-1-yl]-(3R)-(N-methylamino)-4-oxobutyric acid

[F197]

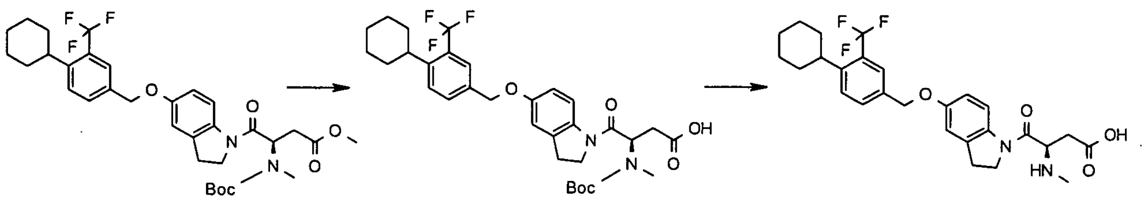

A 1N aqueous sodium hydroxide solution (1 mL) was added at room temperature to a solution of (3R)-[N-(tert-butoxycarbonyl)-N-methylamino]-4-[5-[(4-cyclohexyl-3-trifluoromethylphenyl)methoxy]indolin-1-yl]-4-oxobutyric acid methyl ester (0.23 g) in a methanol/THF mixture (1 mL/2 mL). The resultant mixture was stirred for 14 hours. The reaction

mixture was concentrated, and 1N hydrochloric acid was added to the residue. Precipitated solid was collected by filtration and dried under reduced pressure, to thereby yield (3R)-[N-(tert-butoxycarbonyl)-N-methylaminol-4-[5-[(4-cyclohexyl-3-trifluoromethylphenyl)methoxy]indolin-1-yl]-4-oxobutyric acid, which was employed in a subsequent step. TFA (1 mL) was added at room temperature to a solution (10 mL) of the above (3R)-[N-(tert-butoxycarbonyl)-N-methylamino]-4-[5-[(4-cyclohexyl-3-trifluoromethylphenyl)methoxy]indolin-1-yl]-4-oxobutyric acid in dichloromethane. The resultant mixture was stirred for 4 days. The reaction mixture was concentrated, and the pH of the concentrate was adjusted to 6 with 1N aqueous sodium hydroxide solution, followed by extraction with a chloroform/methanol mixture (10/1, v/v). The extract was dried over sodium sulfate anhydrate. Solvent was removed under reduced pressure, and the residue was purified by thin-layer chromatography, and then freeze-dried from dioxane, whereby the title compound (28 mg) was yielded.

$^1$H-NMR ($CDCl_3$) δ:
1.10-1.54(6H,m),1.68-1.93(SH,m),2.48-2.80(5H,m),2.82-2.99(1H,m),3.06-3.27(2H,m),3.93-4.37(2H,m),4.98(2H,s), 6.64-6.85(2H,m),7.45(1H,d,J=7.8Hz),7.51(1H,d,J=8.1Hz),7.63(1H,s),8 .11(lH,d,J=8.3Hz).
MS (ESI) m/z : 505 (M+H) .

[Example 45]

4-[5-(4-Cyclopentyl-3-trifluoromethylphenyl)methoxy]indolin-1-yl]-(3R)-(N-methylamino)-4-oxobutyric acid

(1) 1-(tert-Butoxycarbonyl)-5-[(4-cyclohexyl-3-trifluoromethylphenyl)methoxy]indoline

[F198]

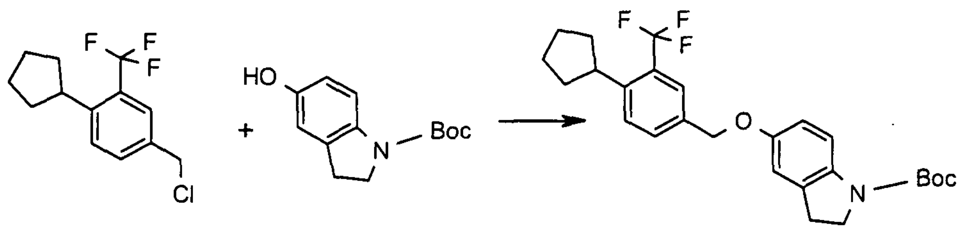

Potassium carbonate (1.14 g) was added at room temperature to a solution (15 mL) of 4-cyclopentyl-3-trifluoromethylbenzyl chloride (0.72 g) and 1-(tert-butoxycarbonyl)-5-hydroxyindoline (0.78 g) in DMF, and the resultant mixture was heated to 80°C, followed by stirring for 1 day. The reaction mixture was cooled to room temperature, and insoluble matter was removed by filtration. The filtrate was concentrated, and the residue was purified by silica gel flash column chromatography (Biotage 40M), whereby the title compound (0.97 g) was yielded as colorless solid.

$^1$H-NMR ($CDCl_3$) δ:
2.17-1.21(20H,m),3.06(2H,t,J=8.7Hz),3.37(1H,t,J=8.7Hz),3.97(1H,s), 5.00(2H,s),6.74-6.81(2H,m),7.47(1H,d, J=8.3Hz),7.54(1H,d,J=6.5Hz),7.64(1H,s).

(2) 5-[(4-Cyclohexyl-3-trifluoromethylphenyl)methoxy]indoline hydrochloride

[F199]

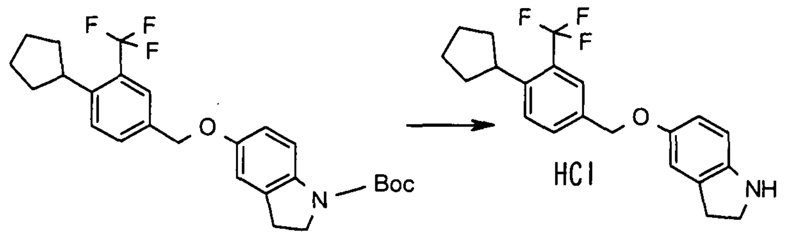

1-(tert-Butoxycarbonyl)-5-[(4-cyclohexyl-3-trifluoromethylphenyl)methoxy]indoline (0.97 g) was dissolved in 4N HCl/1,4-dioxane (20 mL), and the solution was stirred for 3 days at room temperature. The reaction mixture was concentrated, and diethyl ether was added to the residue to form solid. The solid was collected by filtration and dried, whereby the title compound (0.76 g) was yielded.
$^1$H-NMR (DMSO-$d_6$) δ:
1.48-1.76(4H,m),1.75-1.89(2H,m),1.89-2.09(2H,m),3.10-3.29(2H,m),3.32-3.60(2H,m),3.69(2H,t,J=7.7Hz),5.17(-2H,s), 6.98(1H,dd,J=8.7,2.6 Hz),7.12(1H,d,J=2.5Hz),7.31(1H,d,J=8.6Hz),7.55-7.75(3H,m),10.64(1H,s).

(3) (3R)-[N-(tert-Butoxycarbonyl)-N-methylamino]-4-[5-[(4-cyclopentyl-3-trifluoromethylphenyl)methoxy]indolin-1-yl]-4-oxobutyric acid methyl ester

[F200]

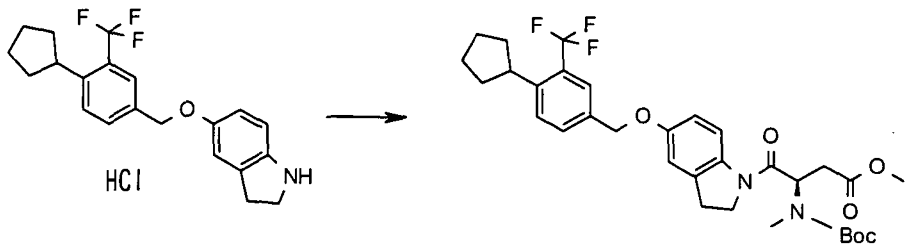

DIEA (0.87 mL) was added at room temperature to a solution (5 mL) of 5-[(4-cyclopentyl-3-trifluoromethylphenyl) methoxy]indoline hydrochloride (0.20 g), Boc-D-Me-Asp(OMe)-OH (0.13 g), EDC·HCl (0.14 g), and HOAt (0.10 g) in DMF. The resultant mixture was stirred for 14 hours. The reaction mixture was concentrated, and the residue was purified by silica gel flash column chromatography (Biotage 25S), whereby the title compound (211 mg) was yielded.
$^1$H-NMR ($CDCl_3$) δ :
1.32-1.91(15H,m),2.01-2.15(2H,m),2.40-2.62(1H,m),2.66-2.85(3H,m),3.04-3.44(3H,m),3.60-3.78(4H,m),3.98-4. 34 (2H,m), 5. 02 (2H, s),5.08-5.58(1H,m),6.78(1H,d,J=9.3Hz),6.83(1H,s),7.47(1H,d,J=8.6Hz),7 .54(1H,d,J=8.3Hz),7.65 (1H,s),8.11(1H,d,J=8.8Hz).
MS (ESI) m/z : 605 (M+H) .

(4) 4-[5-[(4-Cyclopentyl-3-trifluoromethylphenyl)methoxy]indolin-1-yl]-(3R)-(N-methylamino)-4-oxobutyric acid

[F201]

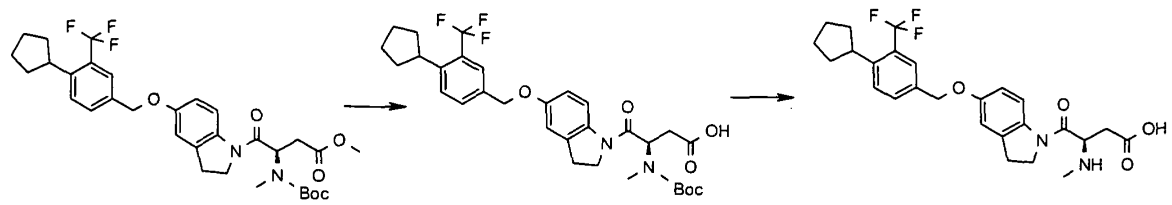

A 1N aqueous sodium hydroxide solution (0.70 mL) was added at room temperature to a solution of (3R)-[N-(tert-butoxycarbonyl)-N-methylamino]-4-[5-[(4-cyclopentyl-3-trifluoromethylphenyl)methoxy]indolin-1-yl]-4-oxobutyric acid methyl ester (0.21 g) in a methanol/THF mixture (1/2 mL). The resultant mixture was stirred for 14 hours. The reaction mixture was concentrated, and the pH of the concentrate was adjusted to 2 with 1N hydrochloric acid, followed by extraction with a chloroform/methanol mixture (10/1, v/v). The extracts were combined and washed with saturated brine, followed by drying over sodium sulfate anhydrate, to thereby yield (3R)-[N-(tert-butoxycarbonyl)-N-methylamino]-4-[5-(4-cyclopentyl-3-trifluoromethylphenyl)methoxy]indolin-1-yl]-4-oxobutyric acid, which was employed in a subsequent step without further purification.
TFA (0.5 mL) was added at room temperature to a solution (10 mL) of (3R)-[N-(tert-butoxycarbonyl)-N-methylamino]-4-[5-[(4-cyclopentyl-3-trifluoromethylphenyl)methoxy]indolin-1-yl]-4-oxobutyric acid in dichloromethane, and the resultant mixture was stirred for 3 hours. The reaction mixture was concentrated, and the pH of the concentrate was adjusted to 6 with 1N aqueous sodium hydroxide solution and 1N hydrochloric acid, followed by extraction with chloroform/methanol (10/1, v/v). Solvent of the extract was evaporated, and the residue was purified by thin-layer chromatography. n-Hexane was added to the residue to form solid, and the solid was collected by filtration and dried, whereby the title compound (67 mg) was yielded.
$^1$H-NMR($CDCl_3$)δ:
1.34-2.05(9H,m),2.37-3.42(8H,m),3.77-4.31(3H,m),4.92(2H,s),5.87(1H,br s),6.46-6-.78(2H,m),7.49-7.29(2H,m),7.57 (1H,s),8.04(1H,s).
MS(ESI)m/z:491(M+H).
IR(ATR)cm$^{-1}$:2954,2871,1716,1653,1616,1593,1489,1377.
Anal. Calcd for
$C_{26}H_{29}F_3N_2O_4$·0.05HCl·$H_2O$:C,61.19;H, 6.13 ; Cl, 0.35; F,11.17;N, 5.49.
Found:C,60.97;H,5.93;C1,0.40;F,11.14;N,5.45.

[Example 46]

(3R)-(N-Methylamino)-4-[5-(3-cyano-4-cyclohexylphenyl)methoxy]-4-methylindolin-1-yl]-4-oxobutyric acid

(1) 1-(tert-Butoxycarbonyl)-5-(3-cyano-4-cyclohexylphenylmethoxy)indoline

[F202]

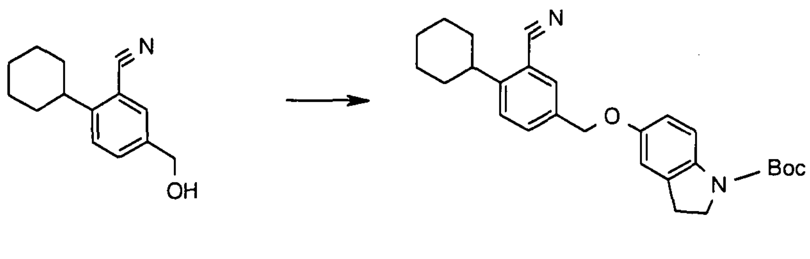

Thionyl chloride (10 mL) was added to 3-cyano-4-cyclohexylbenzyl alcohol (0.80 g) at room temperature, and the mixture was heated to 70°C, followed by stirring for 4 hours. The reaction mixture was cooled to room temperature, and then concentrated. The residue was dissolved in DMF (20 mL). 1-(tert-Butoxycarbonyl)-5-hydroxyindoline (0.66 g) and potassium carbonate (1.2 g) were added to the solution at room temperature, and the resultant mixture was heated to 70°C, followed by stirring for 14 hours. The reaction mixture was cooled to room temperature, and insoluble matter was removed by filtration. The filtrate was concentrated, and the residue was purified by silica gel flash column chromatography (Biotage 40S), whereby the title compound (0.91 g) was yielded.
$^1$H-NMR($CDCl_3$)δ:
1.16-1.65(15H,m),1.67-1.97(5H,m),2.88-3.15(3H,m),3.90-4.07(2H,m),4.99(2H,s),6.73(1H,d,J=9.6Hz),6.78(1H,s),7.37 (1H,d ,J=8.3Hz),7.57(1H,d,J=8.1Hz),7.66(1H,s).

(2) 5-(3-Cyano-4-cyclohexylphenylmethoxy)indoline hydrochloride

[F203]

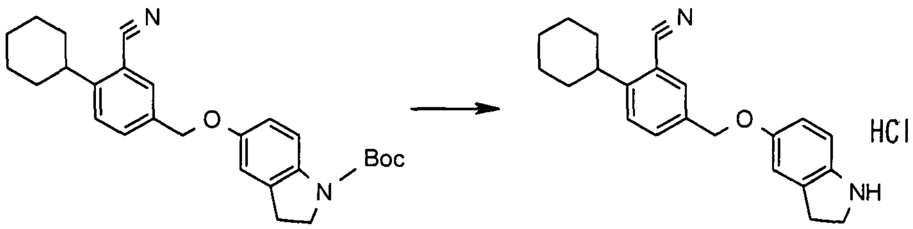

1-(tert-Butoxycarbonyl)-5-(3-cyano-4-cyclohexylphenyl)methoxyindoline (0.91 g) was dissolved in 4N HCl/1,4-dioxa (30 mL), and the solution was stirred for 13 hours at room temperature. The reaction mixture was concentrated, and Precipitated solid was collected by filtration, washed with diethyl ether, and dried, whereby the title compound (0.82 g) was yielded.
$^1$H-NMR(DMSO-$d_6$)δ:
1.23-1.53(6H,m),1.65-1.87(4H,m),2.74-2.88(1H,m),2.97-3.17(2H,m),3.60-3.32(1H,m),3.61-3.73(2H,m),5.12(2H,s), 6.87-6.99(1H,m),7.10(1H,s),7.25(1H,s),7.45-7.61(1H,m),7.71(1H,dd,J=8.3,1.7Hz),7.82(1H,d,J=1.5Hz).

(3) (3R)-[N-(tert-Butoxycarbonyl)-N-methylamino]-4-[5-[(3-cyano-4-cyclohexylphenyl)methoxy]indolin-1-yl]-4-oxobutyric acid methyl ester

[F204]

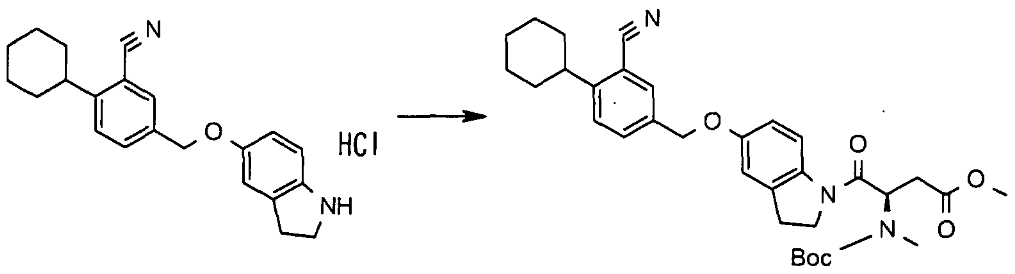

DIEA (0.87 mL) was added at room temperature to a solution (5 mL) of 5-(3-cyano-4-cyclohexylphenylmethoxy) indoline hydrochloride (0.21 g), Boc-D-Me-Asp(OMe)-OH (0.13 g), EDC-HCl (0.19 g), and HOAt (0.14 g) in DMF. The resultant mixture was stirred for 14 hours. The reaction mixture was concentrated, and the residue was purified by silica gel flash column chromatography (Biotage 25S), whereby the title compound (216 mg) was yielded.
$^1$H-NMR ($CDCl_3$)δ:
1.10-1.32(2H,m),1.34-1.62(12H,m),1.68-1.99(4H,m),2.48-2.59(1H,m),2.71-2.88(3H,m),2.88-3.04(1H,m),3.03-3.30 (3H,m),3.57-3.77(4H,m),3.98-4.31(2H,m),5.01(2H,s),5.07-5.57(1H,m),6.76(1H,d,J=8.8Hz),6.82(1H,s),7.37(1H,d, J=8.1Hz),7 .56(1H,dd,J=8.3,2.4Hz),7.66(1H,s),8.11(1H,d,J=8.8Hz).

(4) 4-[5-[(3-Cyano-4-cyclohexylphenyl)methoxy]indolin-1-yl]-(3R)-(N-methylamino)-4-oxobutyric acid

[F205]

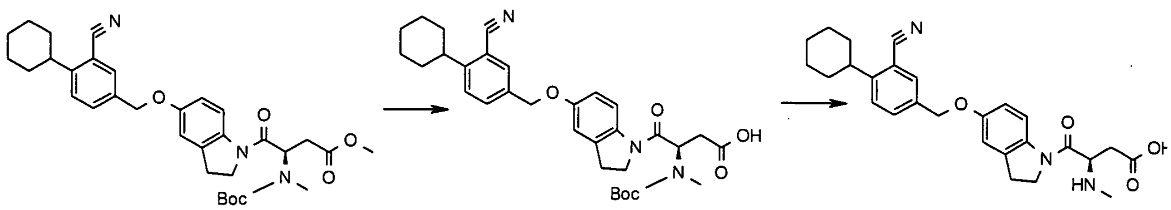

A 1N aqueous sodium hydroxide solution (0.76 mL) was added at room temperature to a solution of (3R)-[N-(tert-butoxycarbonyl)-N-methylamino]-4-[5-[(3-cyano-4-cyclohexylphenyl)methoxy]indolin-1-yl]-4-oxobutyric acid methyl ester (0.22 g) in a methanol/THF mixture (1/2 mL). The resultant mixture was stirred for 20 hours. The reaction mixture was concentrated, and the pH of the concentrate was adjusted to 2 with 1N hydrochloric acid, followed by extraction with a chloroform/methanol mixture (10/1, v/v). The extracts were combined and washed with saturated brine, followed by drying over sodium sulfate anhydrate. Solvent was evaporated, to thereby yield (3R)-[N-(tert-butoxycarbonyl)-N-methylamino]-4-[5-(3-cyano-4-cyclohexylphenyl)methoxy]indolin-1-yl]-4-oxobutyric acid, which was employed in a subsequent step without further purification.
TFA (0.5 mL) was added at room temperature to a solution (10 mL) of (3R)-[N-(tert-butoxycarbonyl)-N-methylamino]-4-[5-(3-cyano-4-cyclohexylphenyl)methoxy]indolin-1-yl]-4-oxobutyric acid in dichloromethane. The resultant mixture was stirred for 8 hours. The reaction mixture was concentrated, and the pH of the concentrate was adjusted to 6 with 1N aqueous sodium hydroxide solution and 1N hydrochloric acid solution, followed by extraction with a chloroform/methanol mixture (10/1, v/v). The extract was dried over sodium sulfate anhydrate, and solvent was removed under reduced pressure. The residue was purified by thin-layer chromatography, and diethyl ether was added to the residue to form solid. The solid was collected by filtration and dried, whereby the title compound (39 mg) was yielded.
$^1$H-NMR (DMSO-$d_6$) δ:
1.17-1.57(6H,m),1.63-1.89(5H,m), 2.49(3H,s), 2.60(1H,dd,J=16.5,5.5Hz), 2.84(1H,t,J=11 .6Hz), 3.13(2H,t,J=8.2Hz),

3.37(1H,q,J=6.9Hz), 3.86(1H,t,J=6.9Hz ),4.00-4.38(2H,m), 5.08(2H,s), 6.81(1H,d,J=9.1Hz), 6.95(1H,s), 7.53(1H,d , J=8.3Hz), 7.70(1H,d,J=7.8Hz), 7.81(1H,s), 8.00(1H,d,J=8.8Hz).
MS (ESI) m/z :462 (M+H) .
IR(ATR)$cm^{-1}$:3032,2925,2852,2224,1720,1653,1595,1489.
Anal. Calcd for
$C_{27}H_{31}N_3O_4 \cdot 1.75H_2O$:C,65.77;H,7.05;N,8.52.Found:C,65.62;H,6.79;N ,8.22.

[Example 47]

4-[5-[(4-Cyclopropyl-3-trifluoromethylphenyl)methoxy]indolin-1-yl]-(3R)-(N-methylamino)-4-oxobutyric acid

(1) (3R)-[N-(tert-Butoxycarbonyl)-N-methylamino]-4-[5-[(4-cyclopropyl-3-trifluoromethylphenyl)methoxy]indolin-1-yl]-4-oxobutyric acid methyl ester

[F206]

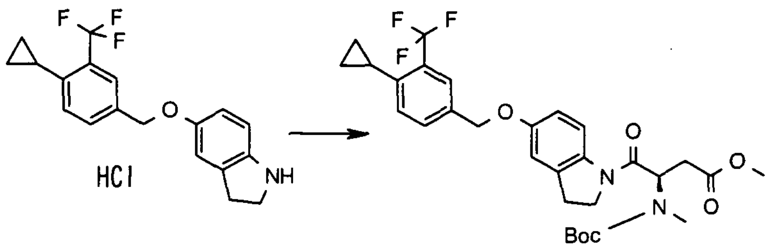

DIEA (0.29 mL) was added at room temperature to a solution (5 mL) of 5-[(4-cyclopropyl-3-trifluoromethylphenyl) methoxy]indoline hydrochloride (123 mg), Boc-D-Me-Asp(OMe)-OH (87 mg), EDC·HCl (96 mg), and HOAt (67 mg) in DMF. The resultant mixture was stirred for 21 hours. The reaction mixture was poured to ice-water, and then extracted with ethyl acetate. The extracts were combined and washed with ice-water and saturated brine, followed by drying over sodium sulfate anhydrate. Solvent was evaporated, and the residue was purified by silica gel flash column chromatography (Biotage 25S), whereby the title compound (99 mg) was yielded.
$^1$H-NMR ($CDCl_3$)δ:
0.68-0.81(2H,m),0.97-1.11(2H,m),1.48(9H,s),2.10-2.28(1H,m),2.47-2.59(1H,m),2.69-2.87(3H,m),3.01-3.27(3H,m), 3.51-3.77(3H,m),3.94-4.36(2H,m),5.01(2H,s),5.04-5.55(1H,m),6.77(1H,d,J=8.8Hz),6.77(1H,s),7.04(1H,d,J=7.4Hz),7 . 47(1H,d,J=7.8Hz),7.66(1H,s),8.11(1H,d,J=9.3Hz).
MS(ESI)m/z:577(M+H).

(2) 4-[5-[(4-Cyclopropyl-3-trifluoromethylphenyl)methoxy] indolin-1-yl] - (3R) - (N-methylamino)-4-oxobutyric acid

[F207]

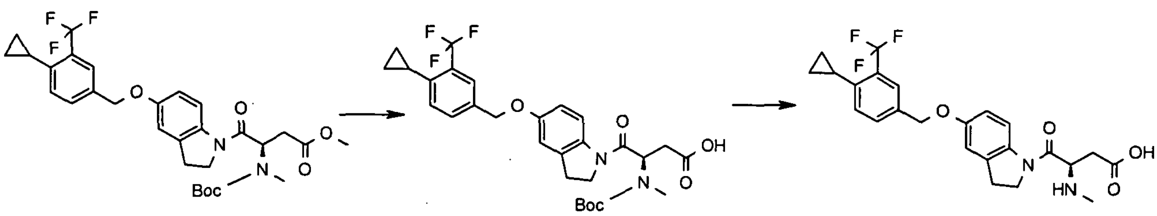

A 1N aqueous sodium hydroxide solution (0.60 mL) was added at room temperature to a solution of (3R)-[N-(tert-butoxycarbonyl)-N-methylamino]-4-[5-[(4-cyclopropyl-3-trifluoromethylphenyl)methoxy]indolin-1-yl]-4-oxobutyric acid

methyl ester (88 mg) in a methanol/THF mixture (0.3 mL/0.6 mL). The resultant mixture was stirred for 3 days. The reaction mixture was concentrated, and the pH of the concentrate was adjusted to 2 with 1N hydrochloric acid, followed by extraction with a chloroform/methanol mixture (10/1, v/v). The extracts were combined and washed with saturated brine, followed by drying over sodium sulfate anhydrate. Solvent was evaporated, to thereby yield (3R)-[N-(tert-butoxycarbonyl)-N-methylamino]-4-[5-[(4-cyclopropyl-3-trifluoromethylphenyl)methoxy]indolin-1-yl]-4-oxobutyric acid, which was employed in a subsequent step without further purification.

TFA (0.5 mL) was added at room temperature to a dichloromethane solution (5 mL) of the above (3R)-[N-(tert-butoxycarbonyl)-N-methylamino]-4-[5-[(4-cyclopropyl-3-trifluoromethylphenyl)methoxy]indolin-1-yl]-4-oxobutyric acid. The resultant mixture was stirred for 8 hours. The reaction mixture was concentrated, and the pH of the concentrate was adjusted to 6 with 1N aqueous sodium hydroxide solution and 1N hydrochloric acid, followed by extraction with a chloroform/methanol mixture (10/1, v/v). The extract was dried over sodium sulfate anhydrate, and solvent was removed under reduced pressure. The residue was recrystallized from ethyl acetate/n-hexane, whereby the title compound (126 mg) was yielded. $^1$H-NMR ($CD_3OD$)δ:

2.42(2H,t,J=7.0Hz), 2.89(2H,t,J=7.0Hz), 3.34(2H,s), 3.53(2H,s), 4 .07(2H,t,J=8.4Hz),5.07(2H,s),6.82(1H,s),6.92(1H,s), 6.95(1H,d, J=8.3Hz),7.02(2H,d,J=7.1Hz),7.19(1H,d,J=7.3Hz),7.39(2H,t,J=8. 0Hz),7.61(1H,d,J=9.0Hz),7.78(1H,s),8.05 (1H,d,J=9.0Hz).

MS(ESI)m/z:463 (M+H).

[Example 48]

(3R)-(N-Methylamino)-4-oxo-4-[5-[(1-phenyl-5-trifluoromethyl-1H-pyrazol-3-yl)methoxy]-2,3-dihydro-1H-indol-1-yl]butyric acid

(1) (3R)-[N-(tert-Butoxycarbonyl)-N-methylamino]-4-oxo-4-[5-[(1-phenyl-5-trifluoromethyl-1H-pyrazol-3-yl)methoxy]-2,3-dihydro-1H-indol-1-yl]butyric acid methyl ester

[F208]

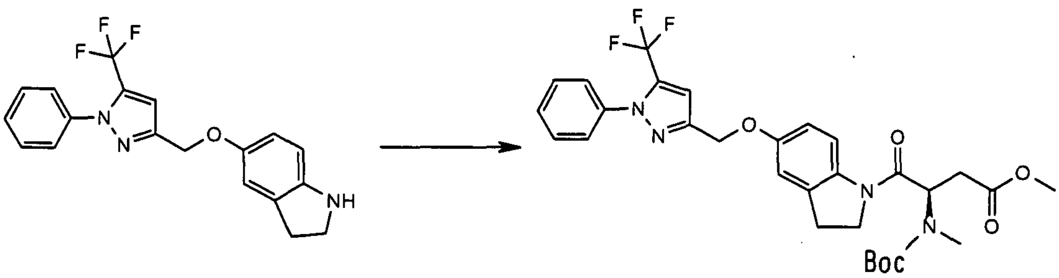

Boc-D-Me-Asp(OMe)-OH (159 mg), EDC-HCl (117 mg), HOBt (82.5 mg), and DIEA (259 μL) were added to a solution (10 mL) of 5-[(1-phenyl-5-trifluoromethyl-1H-pyrazol-3-yl)methoxy]indoline (183 mg) in DMF. The mixture was stirred at room temperature. Saturated aqueous sodium hydrogencarbonate solution was added to the reaction mixture, and the aqueous layer was extracted with ethyl acetate. The extracts were combined and washed with saturated brine, followed by drying over magnesium sulfate anhydrate. Solvent was removed under reduced pressure, and the residue was purified by silica gel column flash chromatography, whereby the title compound (225 mg) was yielded.

$^1$H-NMR ($CDCl_3$) δ:

1.48(9H,s),2.52-2.56(1H,m),2.78-2.81(3H,m),3.09-3.21(3H,m),3.66-3.70(3H,m),4.03-4.15(2H,m),4.24-4.26(1H,m), 5.11-5.12(2H,m),6.83-6.86(2H,m),6.91(1H,s),7.48-7.49(5H,m),8.12(1H,d,J=8.8Hz).

MS (ESI)m/z:603 (M+H)$^+$.

(2) (3R)-[N-(tert-Butoxycarbonyl)-N-methylamino]-4-oxo-4-[5-[(1-phenyl-5-trifluoromethyl-1H-pyrazol-3-yl)methoxy]-2,3-dihydro-1H-indol-1-yl]butyric acid

[F209]

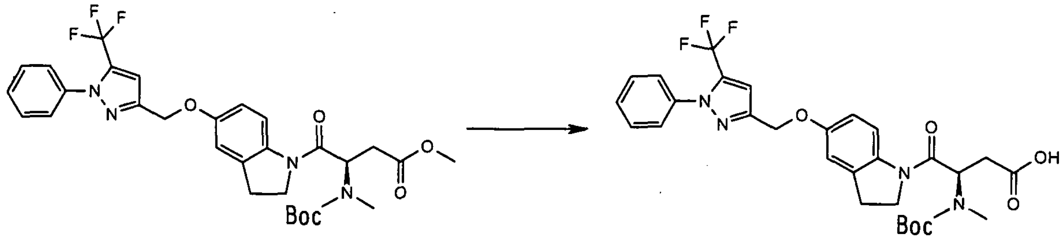

(3R)-[N-(tert-Butoxycarbonyl)-N-methylamino]-4-oxo-4-[5-[(1-phenyl-5-trifluoromethyl-1H-pyrazol-3-yl)methoxy]-2,3-dihydro-1H-indol-1-yl]butyric acid methyl ester (225 mg) was dissolved in a 50% methanol/THF solvent (16 mL). A 1N aqueous sodium hydroxide solution (8.0 mL) was added to the solution, and the resultant mixture was stirred overnight at room temperature. The pH of the reaction mixture was adjusted to about 6 with 1N hydrochloric acid. Water was added to the mixture, and the aqueous layer was extracted with a 10% methanol/chloroform solvent. The extracts were combined and washed with saturated brine, followed by drying over magnesium sulfate anhydrate. Solvent was removed under reduced pressure, whereby the title compound (152 mg) was yielded.
$^1$H-NMR ($CDCl_3$) δ:
1.47(9H,s), 2.56(1H,d,J=16.1Hz), 2.79(4H,d,J=10.7Hz), 3.12-3.19(2H,m), 3.98-4.06(1H,m), 4.22-4.23(1H,m), 5.12(2H, s), 5.46-5.47(1H,m),6.85-6.89(2H,m),6.91(1H,s),7.48-7.49(5H,m),8.12(1H,d,J=8.5Hz).
MS(ESI)m/z:589(M+H)$^+$.

(3) (3R)-(N-Methylamino)-4-oxo-4-[5-[(1-phenyl-5-trifluoromethyl-1H-pyrazol-3-yl)methoxy]-2,3-dihydro-1H-indol-1-yl] butyric acid

[F210]

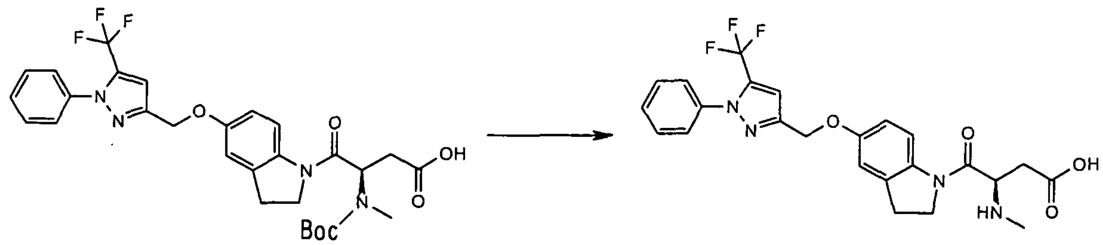

(3R)-[N-(tert-Butoxycarbonyl)-N-methylamino]-4-oxo-4-[5-[(1-phenyl-5-trifluoromethyl-1H-pyrazol-3-yl)methoxy]-2,3-dihydro-1H-indol-1-yl]butyric acid (152 mg) was dissolved in a 10% TFA/dichloromethane solution (10 mL), and the resultant solution was stirred for 4 hours at room temperature. Solvent was evaporated, and the residue was purified by reverse phase preparative HPLC, whereby the title compound (51 mg) was yielded.
$^1$H-NMR ($CD_3OD$) δ :
2.55-2.59(1H,m),2.73(3H,s),2.78-2.82(1H,m),3.25(2H,t,J=8.3Hz),4.13-4.20(1H,m),4.25-4.32(1H,m),4.41-4.44(1H,m), 5.14(2H,s),6.87-6.90(1H,m),6.99-7.01(1H,m),7.04(1H,s),7.48-7.50(2H,m),7.55-7.56(3H,m),8.10(1H,d,J=8.8Hz).
IR(ATR) cm$^-$
$^1$:1655,1595,1489,1389,1290,1228,1184,1128,1086,987,690.
MS (ESI)m/z:489 (M+H)$^+$.
HR-MS(ESI) :Calcd for
$C_{24}H_{24}F_3N_4O_4$ (M+H)$^+$: 489.17496. Found: 489.17519.
Anal. Calcd for
$C_{24}H_{23}F_3N_4O_4 \cdot 0.074CF_3COOH \cdot 1.25H_2O$:C, 55.84;H,4.96;F,11.78;N,10.79 .Found:C,56.12;H,4.66;F,11.48;N,10.67.

[Example 49]

4-[5-[3-(2,4-Difluorophenyl)propoxy]-1-indolinyl]-(3R)-(N-methylamino)-4-oxobutyric acid

(1) 4-[5-[3-(2,4-Difluorophenyl)propoxy]-1-indolinyl]-(3R)-(N-methylamino)-4-oxobutyric acid methyl ester

[F211]

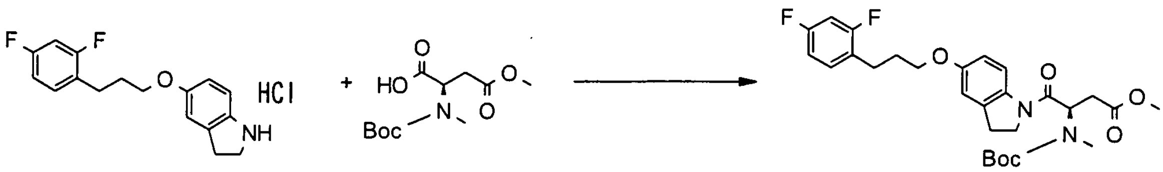

1-(tert-Butoxycarbonyl)-5-[3-(2,4-difluorophenyl)propoxy]indoline hydrochloride (269 mg), Boc-D-Me-Asp (OMe)-OH (216 mg), EDC·HCl (237 mg), HOBt (167 mg), TEA (575 μL), and DMF (10 mL)were mixed together, and the resultant mixture was stirred for 14 hours. The reaction mixture was diluted with ethyl acetate (200 mL), and then washed with saturated brine (2 x 100 mL) and dried over sodium sulfate anhydrate. Solvent was evaporated, and the residue was purified by silica gel column chromatography (Yamazen flash column L), whereby the title compound (302 mg) was yielded.
$^1$H-NMR($CDCl_3$) δ:
1.48(9H,s),2.00-2.09(2H,m),2.54(1H,dd,J=15.7,5.5Hz),2.76-2.82(5H,m),3.05-3.27(3H,m),3.64-3.75(3H,m),3.92(2H,t, J=6.1Hz),3.98-4.31(2H,m),5.15(0.5H,br s),5.50(0.5H,dd,J=8.9,5.5Hz),6.70(1H,dd,J=8.9,2.3Hz),6.73-6.82(3H,m), 7.18-7.10(1H,m), 8.09(1H,d,J=8.5Hz).
MS (ESI) m/z : 533 $(M+H)^+$.

(2) 4-[5-[3-(2,4-Difluorophenyl)propoxy]-1-indolinyl]-(3R)-(N-methylamino)-4-oxobutyric acid hydrochloride

[F212]

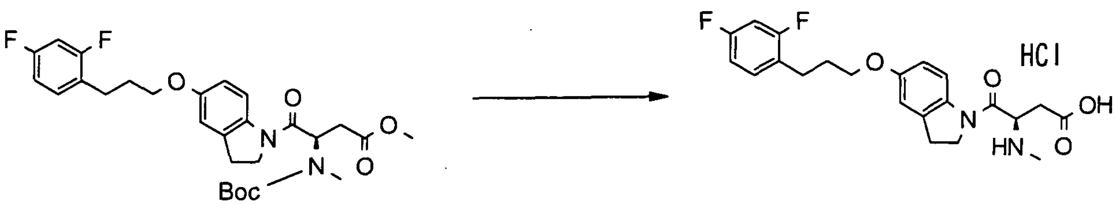

A 0.25N aqueous sodium hydroxide solution (4.56 mL) and THF (4.56 mL) were added to 4-[5-[3-(2, 4-difluorophenyl)propoxy]-1-indolinyl]-(3R)-(N-methylamino)-4-oxobutyric acid methyl ester (302 mg), and the resultant mixture was stirred for 17 hours. The reaction mixture was acidified with 1N hydrochloric acid, and then extracted with 20% methanol/chloroform (2 x 200 mL). The extracts were combined and dried with sodium sulfate anhydrate, and solvent was evaporated. 4N HCl/1,4-dioxane (10 mL) was added to the residue, and the resultant mixture was stirred for 5 hours. The reaction mixture was concentrated and then slurried with diethyl ether. The precipitate was collected by filtration and dried, whereby the title compound (150 mg) was yielded.
$^1$H-NMR (DMSO-$d_6$) δ:
1.93-2.02(2H,m),2.57(3H,s),2.75(2H,t,J=7.4Hz),2.85-3.98(6H,m), 4.18(1H,q,J=8.8Hz), 4.35(1H,q,J=9.3Hz), 4.47(1H,t, J= 6.2Hz),6.77(1H,dd,J=8.8,2.7Hz),6.88-6.91(1H,m),6.98-7.05(1H,m), 7.14-7.21(1H,m), 7.39-7.32(1H,m), 8.00(1H,d, J=8.8Hz).
No proton peak attributed to $CO_2H$ or NHHCl was observed.
MS (ESI) m/z :419 $(M+H)^+$.
Anal. Calcdfor$C_{22}H_{24}F_2N_2O_4$·0.25$H_2O$·1.25HCl:C,56.40;H,5.54;Cl,9.46;F,8 .11;N,5.98.Found:C,56.04;H,5.48;F,7.75;

N,5.83.

[Example 50]

3-[[2-[5-[[2,4-Bis(trifluoromethyl)benzyl]oxy]-2,3-dihydro-1H-indol-1-yl]-2-oxoethyl]amino]propionic acid TFA salt

(1) 3-[[2-(Benzyloxy)-2-oxoethyl]amino]propionic acid tert-butyl ester (Kim, Jin Mi; Roy, Rene, Carbohydrate Research (1997), 298 (3),173-179.)

[F213]

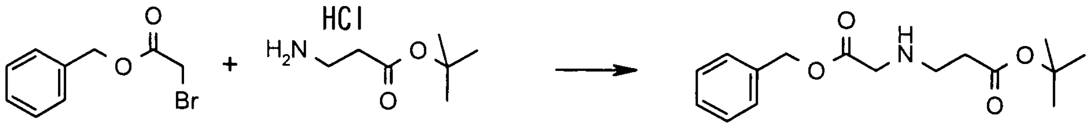

2-Bromoacetic acid benzyl ester (commercially available) (1.05 mL) was added at room temperature to a solution of 3-aminopropionic acid tert-butyl ester (commercially available) (3.00 g) in acetonitrile (130 mL). The resultant mixture was stirred for 15 hours at 80°C. The reaction mixture was left to stand to cool to room temperature and concentrated under reduced pressure, and the residue was purified by silica gel flash column chromatography (Biotage 40M), whereby the title compound (1.74 g) was yielded.
$^1$H-NMR($CDCl_3$)δ:
1.45(9H,s), 2.42(2H,t,J=6.7Hz), 2.86(2H,t,J=6.7Hz), 3.46(2H,s), 5 .17(2H,s), 7.29-7.40(5H,m). No NH was observed.
MS(ESI)m/z:294(M+H)$^+$

(2) 3-[N-[2-(Benzyloxy)-2-oxoethyl]-N-(tert-butoxycarbonyl)amino]propionic acid tert-butyl ester

[F214]

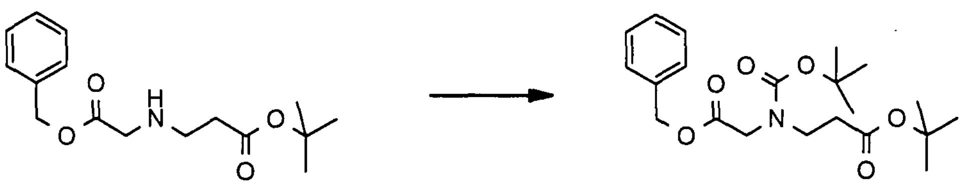

Saturated aqueous sodium hydrogencarbonate solution (5.0 mL) and $Boc_2O$ (90.0 g) were added to a solution of 3-[[2-(benzyloxy)-2-oxoethyl]amino]propionic acid tert-butyl ester (100 mg) in dichloromethane (5.0 mL) at room temperature, and the resultant mixture was stirred for 15 hours at room temperature. The reaction mixture was partitioned, and the aqueous layer was extracted with chloroform (20 mL). The organic layers were combined and dried over sodium sulfate anhydrate, and solvent was removed under reduced pressure. The residue was purified by silica gel flash column chromatography (Biotage 25M), whereby the title compound (128 mg) was yielded.
$^1$H-NMR ($CDCl_3$)δ:
1.34(1/2 of 9H, s), 1.42 (1/2 of 9H,s),1.43(1/2 of 9H,s),1.55(1/2 of 9H,s),2.50(1/2 of 2H,t,J=6.7Hz),2.55(1/2 of 2H,t, J=6.7Hz),3.49(1/2 of 2H,t,J=6.7Hz),3.54(1/2 of 2H,t,J=6.7Hz),4.00(1/2 of 2H,s),4.09(1/2 of 2H,s),5.15(2H,s),7.28-7.40 (5H,m).
MS (ESI)m/z:394 (M+H)$^+$

(3) 2-[N-(tert-Butoxycarbonyl)-N-[3-(tert-butoxy)-3-oxopropyl] amino] acetic acid

[F215]

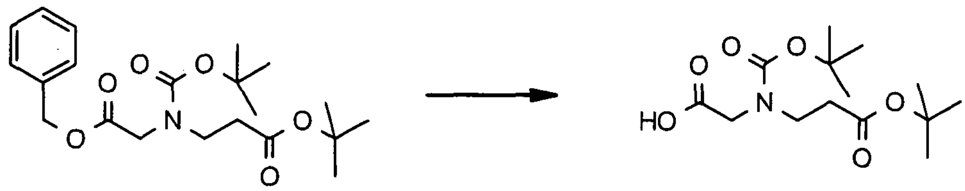

5% Pd/C (90.0 mg) was added to a solution of 3-[N-[2-(benzyloxy)-2-oxoethyl]-N-(tert-butoxycarbonyl)amino] propionic acid tert-butyl ester (125 mg) in methanol (5.0 mL). The resultant mixture was stirred for 4 hours under a hydrogen atmosphere at room temperature. The reaction mixture was filtered, and the filtrate was concentrated under reduced pressure, whereby the title compound (96.1 mg) was yielded.
1H-NMR ($CDCl_3$ )δ :
1.44(18H,s),2.52(2H,br s),3.52(2H,br s),3.99(2H,br s). No COOH was observed.
HR-MS(ESI): Calcd for $C_6H_{10}NO_6$(M+H-isobutene(57)-isobutene(57))$^+$:192.05081. Found:192.04972.

(4) 5-(Benzyloxy)-1-indolinecarboxylic acid tert-butyl ester

[F216]

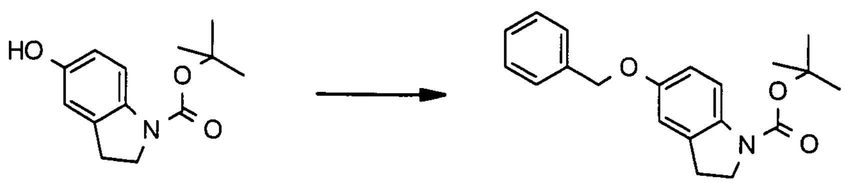

Potassium carbonate (6.34 g) and benzyl bromide (2.73 mL) were added at room temperature to a solution of 5-hydroxy-1-indolinecarboxylic acid tert-butyl ester (3.60 g) in acetonitrile (70 mL), and the resultant mixture was stirred for 3 hours at 90°C. The reaction mixture was left to stand to cool to room temperature and then filtered. The filtrate was concentrated under reduced pressure, and the residue was purified by silica gel flash column chromatography (Biotage 40M), whereby the title compound (4.50 g) was yielded.
$^1$H-NMR ($CDCl_3$) δ:
1.55(9H,s),3.05(2H,t,J=7.5Hz),3.96(2H,br s),5.02(2H,s),6.77(1H,d,J=8.8Hz),6.80(1H,s),7.22(1/2 of 1H,br s), 7.28-7.44 (5H,m),7.74(1/2 of 1H,br s).
MS(ESI)m/z:326(M+H)$^+$.

(5) 5-(Benzyloxy)indoline hydrochloride

[F217]

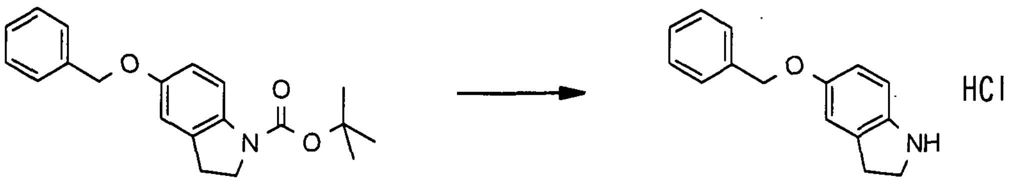


4N HCl/1,4-dioxa (75 mL) was added to 5-(benzyloxy)-1-indolinecarboxylic acid tert-butyl ester (4.50 g) at room temperature. Thirty minutes later (precipitation was observed), diethyl ether (200 mL) was added to the mixture, and the resultant mixture was stirred for 15 hours at room temperature. The resulting precipitated solid was collected by filtration and dried (in vacuo, 40°C, 2 hours), whereby the title compound (3.31 g) was yielded.
$^1$H-NMR (DMSO-$d_6$) δ:
3.15(2H,t,J=7.8Hz),3.69(2H,t,J=7.8Hz),5.12(2H,s),6.97(1H,dd,J =8.8,2.4Hz),7.11(1H,d,J=2.4Hz),7.29-7.45(6H,m). No NH was observed.
MS(ESI)m/z:226(M+H)$^+$.

(6) 3-[N-[2-[5-(Benzyloxy)-2,3-dihydro-1H-indol-1-yl]-2-oxoethyl]-N-(tert-butoxycarbonyl)amino]propionic acid tert-butyl ester

[F218]

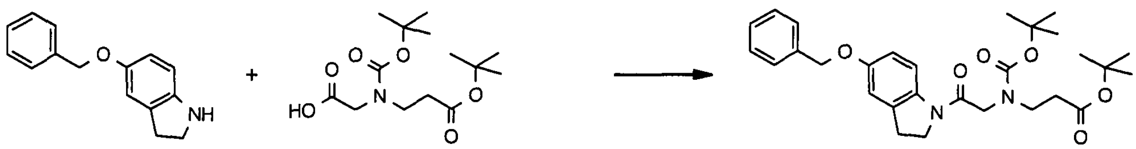

EDC·HCl (2.20 g), HOBt (1.55 g), and TEA (5.32 mL) were added at room temperature to a suspension of 5-(benzyloxy)indoline hydrochloride (2.00 g) and 2-[N-(tert-butoxycarbonyl)-N-[3-(tert-butoxy)-3-oxopropyl] amino] acetic acid (2.78 g) in DMF (50 mL). The resultant mixture was stirred for 15 hours. The reaction mixture was concentrated under reduced pressure. Ethyl acetate (70 mL), saturated aqueous sodium hydrogencarbonate solution (70 mL), and water (50 mL) were added to the concentrated product for partitioning, and the aqueous layer was extracted with ethyl acetate (50 mL). The organic layers were combined and then dried over sodium sulfate anhydrate. Solvent was removed under reduced pressure, and the residue was purified by silica gel flash column chromatography (Biotage 40M), whereby the title compound (3.70 g) was yielded.
$^1$H-NMR (cDCl$_3$ ) δ :
1.38-1.57 (18H, m), 2.54-2.64 (2H, m), 3.12-3.22 (2H, m), 3.54-3.63 (2H, m), 3.96-4.14 (2H, m), 4.17 (2H, s), 5.03 (2H, s), 6.75-6.88(2H,m),7.25-7.48(5H,m),8.08-8.16(1H,m).
MS(ESI)m/z:511(M+H)$^+$.

(7) 3-[N-(tert-Butoxycarbonyl)-N-[2-(5-hydroxy-2,3-dihydro-1H-indol-1-yl)-2-oxoethyl]amino]propionic acid tert-butyl ester

[F219]

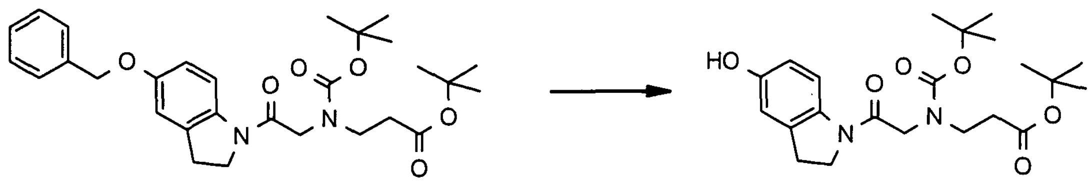

5% Pd/C (1.00 g) was added to a solution of 3-[N-[2-[5-(benzyloxy)-2,3-dihydro-1H-indol-1-yl]-2-oxoethyl]-N-(tert-butoxycarbonyl)amino]propionic acid tert-butyl ester (3.54 g) in a mixture of methanol (20 mL) and THF (20 mL). The resultant mixture was stirred for 2 hours under a hydrogen atmosphere at room temperature. Insoluble matter in the reaction mixture was separated by filtration, and the filtrate was concentrated under reduced pressure. The residue was purified by silica gel flash column chromatography (Biotage 40M), whereby the title compound (2.57 g) was yielded.
$^1$H-NMR ($CDCl_3$)δ:
1.44(9H,s),1.53(9H,s),2.58(2H,t,J=6.6Hz),2.85(2H,t,J=8.3Hz),3 .58(2H,t,J=6.6Hz),3.68(2H,t,J=8.3Hz),4.06(2H,s), 6.50-6.60(2H,m),7.44(1H,s),8.02(1H,d,J=8.8Hz).
MS(ESI)m/z:421(M+H)$^+$.

(8) 3-[N-[2-[5-[[2,4-Bis(trifluoromethyl)benzyl]oxy]-2,3-dihydro-1H-indol-1-yl]-2-oxoethyl]-N-(tert-butoxycarbonyl)amino] propionic acid tert-butyl ester

[F220]

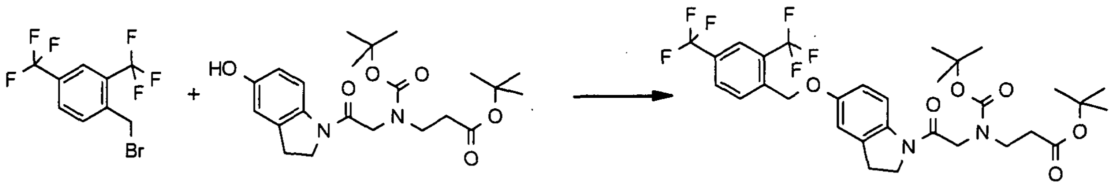

Potassium carbonate (111 mg) was added at room temperature to a solution of 2,4-bis(trifluoromethyl)benzyl bromide (commercially available) (0.140 mL) and 3-[N-(tert-butoxycarbonyl)-N-[2-(5-hydroxy-2,3-dihydro-1H-indol-1-yl)-2-oxoethyl]amino]propionic acid tert-butyl ester (260 mg) in DMF (5.0 mL). The reaction mixture was stirred for 15 hours at 50°C and then left to stand to cool to room temperature, and the precipitate was removed by filtration. Water (100 mL) and saturated aqueous ammonium chloride solution (50 mL) were added to the filtrate, and the resultant mixture was extracted with ethyl acetate (50 mL). The extracts were combined and washed with saturated brine (50 mL), followed by drying over sodium sulfate anhydrate. Solvent was removed under reduced pressure, and the residue was purified by silica gel flash column chromatography (Biotage 25M), whereby the title compound (327 mg) was yielded.
$^1$H-NMR ($CDCl_3$) δ:
1.38-1.58 (18H, m), 2.54-2.63 (2H, m), 3.15-3.23 (2H, m), 3.54-3.62 (2H, m), 3.98-4.12 (2H, m), 4.18 (2H, s), 5.28 (2H, s), 6.70-6.83(2H,m), 7.80-8.00(3H,m), 8.10-8.18(1H,m).
MS(ESI)m/z:669(M+Na)$^+$.

(9) 3-[N-[2-[5-[[2,4-Bis(trifluoromethyl)benzyl]oxy]-2,3-dihydro-1H-indol-1-yl]-2-oxoethyl]amino]propionic acid TFA salt

[F221]

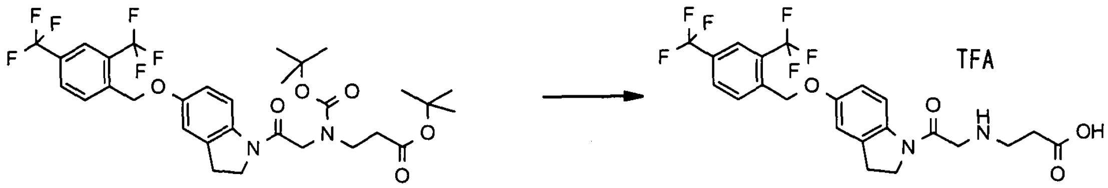

TFA (1.0 mL) was added at room temperature to a solution of 3-[N-[2-[5-[[2,4-bis(trifluoromethyl)benzyl]oxy]-2,3-dihydro-1H-indol-1-yl]-2-oxoethyl]-N-(tert-butoxycarbonyl)amino]propionic acid tert-butyl ester (84.8 mg) in dichloromethane (2.0 mL). The reaction mixture was stirred for 2 hours at room temperature, and then concentrated under reduced pressure. Diethyl ether-hexane was added to the residue to form solid, and solvent was removed under reduced pressure, followed by drying (in vacuo, room temperature, 1 hour), whereby the title compound (77.1 mg) was yielded.
$^1$H-NMR (DMSO-$d_6$) δ:
2.72(2H,t,J=7.4Hz),3.14-3.23(4H,m),4.05(2H,t,J=8.4Hz),4.14(2H,s),5.31(2H,s),6.88(1H,d d,J=8.8,2.5Hz),7.02(1H,d, J=2.5Hz),7.97(1H,d,J=8.8Hz),8.01(1H, d,J=8.2Hz),8.09(1H,s),8.14(1H,d,J=8.2Hz),8.94(2H,br s). No $CO_2H$ was observed.
IR (ATR) $cm^{-1}$:3163,1720,1674,1491,1348,1279,1173,1161,1113,1043,721.
MS (ESI) m/z:491 $(M+H)^+$.
HR-MS(ESI)Calcd for $C_{22}H_{21}F_6N_2O_4(M+H)^+$:491.14055. Found: 491.13682.
Anal. Calcd for
$C_{22}H_{20}F_6N_2O_4\cdot1.0TFA$:C,47.69;H,3.50;F,28.29;N,4.63. Found: C,47.42 ;H,3.46;F,27.99;N,4.50.

[Example 51]

3-[N-[2-Oxo-2-[5-[2-[4-phenyl-5-(trifluoromethyl)-2-thienyl]ethyl]-2,3-dihydro-1H-indol-1-yl] ethyl] amino] propionic acid TFA salt

(1) 3-[N-(tert-Butoxycarbonyl)-N-[2-oxo-2-[5-[2-[4-phenyl-5-(trifluoromethyl)-2-thienyl]ethyl]-2,3-dihydro-1H-indol-1-yl] ethyl]amino]propionic acid tert-butyl ester

[F222]

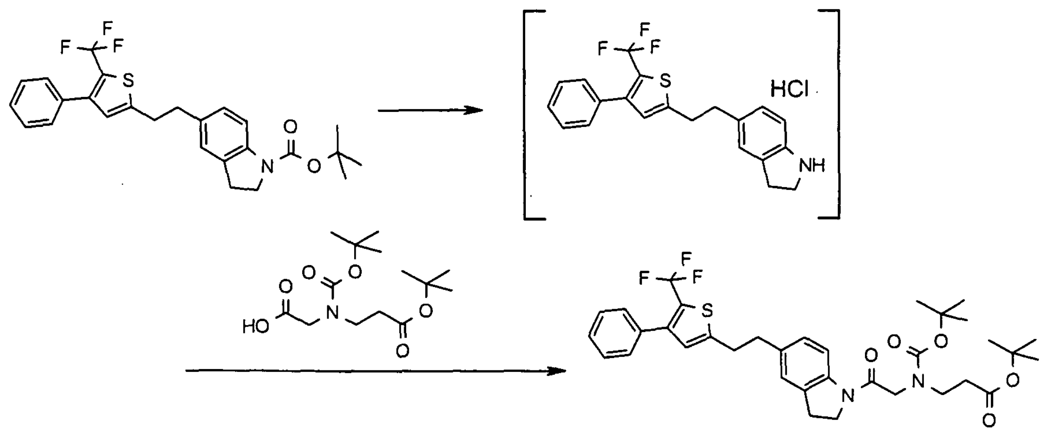

4N HCl/1,4-dioxane (5.0 mL) was added to 1-(tert-butoxycarbonyl)-5-[2-[4-phenyl-5-(trifluoromethyl)-2-thienyl]ethyl]indoline (115 mg) at room temperature. The reaction mixture was stirred at room temperature for 3 hours and concentrated under reduced pressure, whereby 5-[2-[4-phenyl-5-(trifluoromethyl)-2-thienyl]ethyl]indoline hydrochloride was yielded. This hydrochloride was dissolved in DMF (5.0 mL), and 2-[N-(tert-butoxycarbonyl)-N-[3-(tert-butoxy)-3-oxopropyl]amino]acetic acid (100 mg), EDC·HCl (70.2 mg), HOBt (49.5 mg), and TEA (0.170 mL) were added to the resultant solution at room temperature, followed by stirring for 15 hours. The reaction mixture was concentrated under reduced pressure. Ethyl acetate (30 mL), saturated aqueous sodium hydrogencarbonate solution (20 mL), and water (50 mL) were added to the concentrate for phase separation, and the aqueous layer was extracted with ethyl acetate (20 mL). The extracts were combined and dried over sodium sulfate anhydrate. Thereafter, solvent was evaporated under reduced pressure, and the residue was purified by silica gel flash column chromatography (Biotage 25M), whereby the title compound (133 mg) was yielded.
$^1$H-NMR ($CDCl_3$) δ:
1.38-1.58(18H,m),2.50-2.65(2H,m),2.92-3.00(2H,m),3.05-3.25(4H,m), 3.45-3.80(4H,m), 3.95-4.32(4H,m), 6.75(1H,s), 7.33-7.43(5H,m),8.08-8.17(1H,m).
MS (ESI) m/z : 659 $(M+H)^+$.

(2) 3-[N-[2-Oxo-2-[5-[2-[4-phenyl-5-(trifluoromethyl)-2-thienyl]ethyl]-2,3-dihydro-1H-indol-1-yl]ethyl]amino]propionic acid TFA salt

[F223]

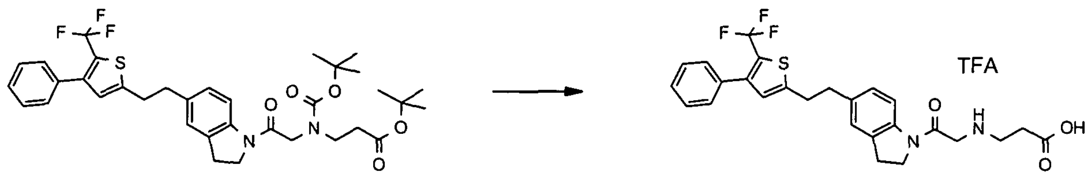


To a solution of 3-[N-(tert-butoxycarbonyl)-N-[2-oxo-2-[5-[2-[4-phenyl-5-(trifluoromethyl)-2-thienyl]ethyl]-2,3-dihydro-1H-indol-1-yl]ethyl]amino]propionic acid tert-butyl ester (130 mg) in dichloromethane (2.0 mL), TFA (1.0 mL) was added at room temperature. The reaction mixture was stirred at room temperature for 2 hours and concentrated under reduced pressure. The concentrate was dissolved in 1,4-dioxane, followed by freeze-drying, whereby the title compound (120 mg) was yielded.
$^1$H-NMR (DMSO-$d_6$) δ:
2.70(2H,t,J=7.7Hz),2.95(2H,t,J=8.3Hz),3.10-3.23(6H,m),4.04(2H,t,J=7.7Hz),4.14(2H,s),7.05(1H,s),7.09-7.15(1H,m), 7.22(1H,s),7.34-7.59(5H,m), 7.94(1H,d,J=8.3Hz), 8.95(1H,br s). $CO_2H$ was not observed.
MS(ESI) m/z: 503 $(M+H)^+$.
HR-MS(ESI)Calcd for
$C_{26}H_{25}F_3N_2O_3S(M+H)^+$:503.16162. Found:503.16004.

[Example 52]

3-[N-[2-[5-[[3,5-Bis(trifluoromethyl)benzyl]oxy]-2,3-dihydro-1H-indol-1-yl]-2-oxoethyl]amino]propionic acid TFA salt

(1) 3-[N-[2-[5-[[3,5-Bis(trifluoromethyl)benzyl]oxy]-2,3-dihydro-1H-indol-1-yl]-2-oxoethyl]-N-(tert-butoxycarbonyl)amino]propionic acid tert-butyl ester

[F224]

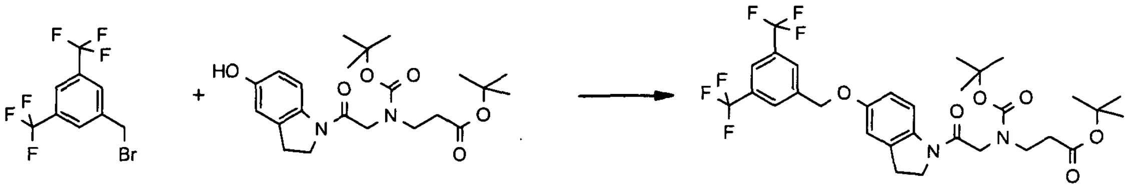

To a solution of 3,5-bis(trifluoromethyl)benzyl bromide (commercially available) (0.052 mL) and 3-[N-(tert-butoxycarbonyl)-N-[2-(5-hydroxy-2,3-dihydro-1H-indol-1-yl)-2-oxoethyl]amino]propionic acid tert-butyl ester (100 mg) in DMF (2.0 mL), potassium carbonate (42.8 mg) was added at room temperature. The reaction mixture was stirred at 50°C for 20 hours and cooled to room temperature, followed by removal of precipitated matter by filtration. Water (30 mL) and saturated aqueous ammonium chloride solution (20 mL) were added to the filtrate, and the mixture was extracted with ethyl acetate (2 x 20 mL). The extracts were combined and washed with saturated brine (30 mL), followed by drying over sodium sulfate anhydrate. Solvent was evaporated under reduced pressure, and the residue was purified by silica gel flash column chromatography (Biotage 25M), whereby the title compound (127 mg) was yielded.
$^1$H-NMR ($CDCl_3$) δ:
1.38-1.62(18H,m),2.53-2.63(2H,m),3.14-3.25(2H,m),3.53-3.62(2H,m),3.98-4.22(4H,m),5.12(2H,s),6.73-6.90(2H,m), 7.84(1H,s),7.89(2H,s),8.10-8.20(1H,m).
MS(ESI)m/z:647$(M+H)^+$.

(2) 3-[N-[2-[5-[[3,5-Bis(trifluoromethyl)benzyl]oxy]-2,3-dihydro-1H-indol-1-yl]-2-oxoethyl]amino]propionic acid TFA salt

[F225]

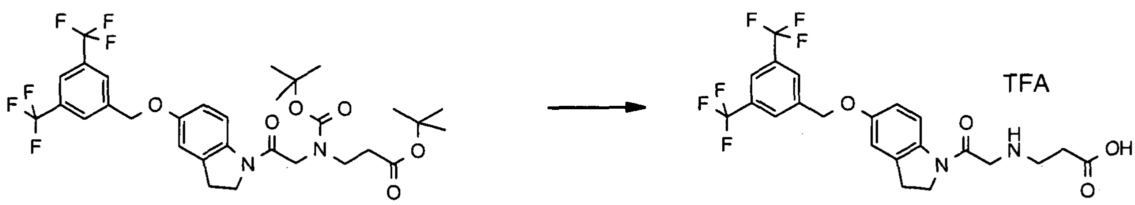

To a solution of 3-[N-[2-[5-[[3,5-bis(trifluoromethyl)benzyl]oxy]-2,3-dihydro-1H-indol-1-yl]-2-oxoethyl]-N-(tert-butoxycarbonyl)amino]propionic acid tert-butyl ester (126 mg) in dichloromethane (2.0 mL), TFA (1.0 mL) was added at room temperature. The reaction mixture was stirred at room temperature for 2 hours and concentrated under reduced pressure. Diethyl ether was added to the concentrate to form solid, and solvent was evaporated under reduced pressure, followed by drying (in vacuo, room temperature, 1 hour), whereby the title compound (94.2 mg) was yielded.
$^1$H-NMR (DMSO-$d_6$) δ:
2.72(2H,t,J=7.3Hz),3.14-3.23(4H,m), 4.05(2H,t,J=8.3Hz), 4.14(2H,s), 5.29(2H,s), 6.91(1H,d d,J=8.8,2.2Hz),7.05(1H,d, J=2.2Hz),7.97(1H,d,J=8.8Hz),8.09(1H, s),8.14(2H,s),8.94(2H,br s),12.63(1H,br s). The peak attributed to $CF_3COOH$ was included in the peak at 8.94.
IR(ATR)$cm^{-1}$:3109,1655,1550,1491,1369,1288,1161,1119,885,684.
MS(ESI)m/z:491$(M+H)^+$.
HR-MS (ESI) Calcd for $C_{22}H_{21}F_6N_2O_4$ $(M+H)^+$: 491.14055 . Found: 491.1363 . Anal. Calcd for $C_{22}H_{20}F_6N_2O_4$· 1.0TFA:C,47.69;H,3.50;F,28.29;N,4.63.Found:C,47.48;H,3.41;F,2 8.24;N,4.39.

[Example 53]

3-[N-[2-[5-[[(E)-3-[2,4-Bis(trifluoromethyl)phenyl]-2-propenyl]oxy]-2,3-dihydro-1H-indol-1-yl]-2-oxoethyl] amino] propionic acid

(1) 2,4-Bis(trifluoromethyl)benzaldehyde (WO 2002/096849)

[F226]

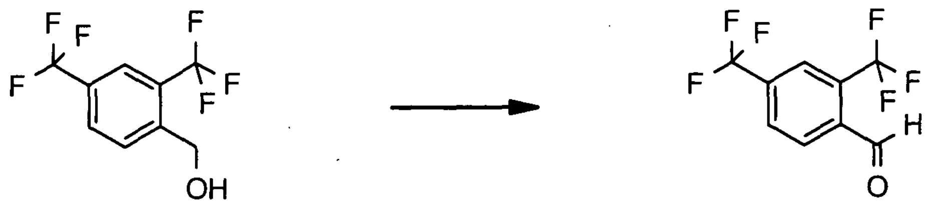

To a solution of [2,4-bis(trifluoromethyl)phenyl]methanol (commercially available) (2.04 g) in THF (40 mL), NaCl (2.00 g) and manganese dioxide (4.36 g) were added at room temperature. The reaction mixture was stirred for 2 days and insoluble matter was removed by filtration through a Celite pad. Thereafter, the filtrate was concentrated under reduced pressure, and the concentrate was purified by silica gel flash column chromatography (Biotage 40M), whereby the title compound (1.23 g) was yielded.
$^1$H-NMR ($CDCl_3$) δ :
7.99(1H,d,J=8.2Hz),8.05(1H,s),8.27(1H,d,J=8.2Hz),10.43-10.46(1H,m).

(2) (E)-3-[2,4-Bis(trifluoromethyl)phenyl]-2-propenoic acid ethyl ester

[F227]

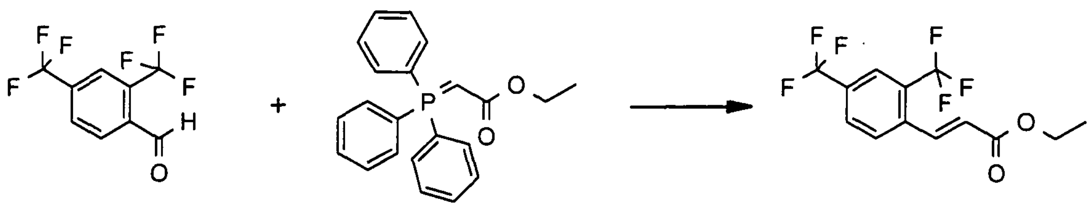

To a solution of 2,4-bis(trifluoromethyl)benzaldehyde (1.22 g) in toluene (30 mL), 2-(triphenylphosphoranylidene)acetic acid ethyl ester (1.93 g) was added at room temperature. The reaction mixture was refluxed for 15 hours and left to stand to cool to room temperature, followed by concentration under reduced pressure. The residue was purified by silica gel flash column chromatography (Biotage 40M), whereby the title compound (1.25 g) was yielded.
$^1$H-NMR($CDCl_3$)δ:
1.36(3H,t,J=7.1Hz),4.30(2H,q,J=7.1Hz),6.47(1H,d,J=15.7Hz),7.7 9-7.86(2H,m),7.96(1H,s),8.03(1H,dd,J=15.7,2.2Hz).
MS(ESI)m/z:313(M+H)$^+$.

(3) (E)-3-[2,4-Bis(trifluoromethyl)phenyl]-2-propen-1-ol

[F228]

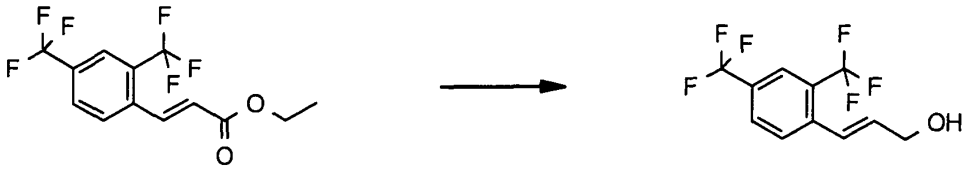

To a solution of (E)-3-[2,4-bis(trifluoromethyl)phenyl]-2-propenoic acid ethyl ester (1.25 g) in THF (20 mL), diisobutylaluminum hydride (0.99M toluene solution) (10.0 mL) was added at 0°C. The reaction mixture was stirred at 0°C for 1 hour, and saturated aqueous ammonium chloride solution (3.0 mL) was added thereto, followed by allowing to warm to room temperature. The reaction mixture was diluted with diethyl ether (100 mL), and stirred at room temperature for 1 hour. Magnesium sulfate anhydrate was added to the reaction mixture, and the mixture was stirred for 30 minutes, followed by removal of insoluble matter by filtration. The filtrate was concentrated under reduced pressure, and the residue was purified by silica gel flash column chromatography (Biotage 25M), whereby the title compound (1.06 g) was yielded.
$^1$H-NMR ($CDCl_3$) δ:
1.60(1H,t,J=5.9Hz),4.38-4.44(2H,m),6.44(1H,dt,J=15.7,5.1Hz),7.02(1H,dd,J=15.7,2.2Hz), 7.72-7.79(2H,m),7.89(1H, s).

(4) 3-[N-[2-[5-[[(E)-3-[2,4-Bis(trifluoromethyl)phenyl]-2-propenyl]oxy]-2,3-dihydro-1H-indol-1-yl]-2-oxoethyl]-N-(tert-butoxycarbonyl)amino]propionic acid tert-butyl ester

[F229]

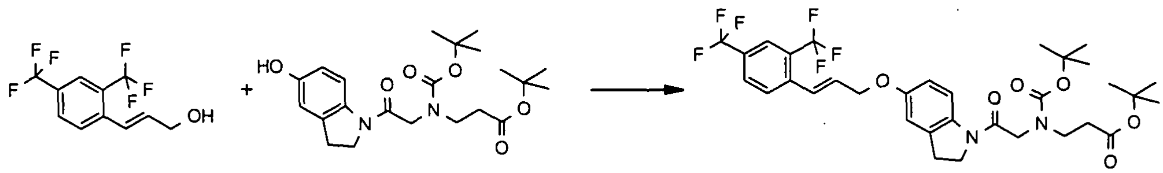

To a solution of (E)-3-[2,4-bis(trifluoromethyl)phenyl]-2-propen-1-ol (146 mg) in THF (6.0 mL), 3-[N-(tert-butoxycarbonyl)-N-[2-(5-hydroxy-2,3-dihydro-1H-indol-1-yl)-2-oxoethyl]amino]propionic acid tert-butyl ester (200 mg), triphenylphosphine (155 mg), and DEAD (2.2M toluene solution) (0.269 mL) were added at room temperature. The reaction mixture was stirred at room temperature for 3 days and concentrated under reduced pressure. The residue was purified by silica gel column flash chromatography (Biotage 25M), whereby the title compound (215 mg) was yielded.
$^1$H-NMR($CDCl_3$)δ:
1.33-1.60(18H,m),2.54-2.63(2H,m),3.15-3.23(2H,m),3.55-3.62(2H,m),3.95-4.25(4H,m),4.73(2H,s),6.40-6.50(1H,m), 6.71-6.83(1H,m),7.11(1H,d,J=15.4Hz),7.43-7.58(1H,m),7.64-7.80(2H,m),7.89(1H,s),8.20-8.32(1H,m).
MS (ESI) m/z : 673 (M+H)$^+$.

(5) 3-[N-[2-[5-[[(E)-3-[2,4-Bis(trifluoromethyl)phenyl]-2-propenyl] oxy] -2, 3-dihydro-1H-indol-1-yl] -2-oxoethyl]amino]propionic acid

[F230]

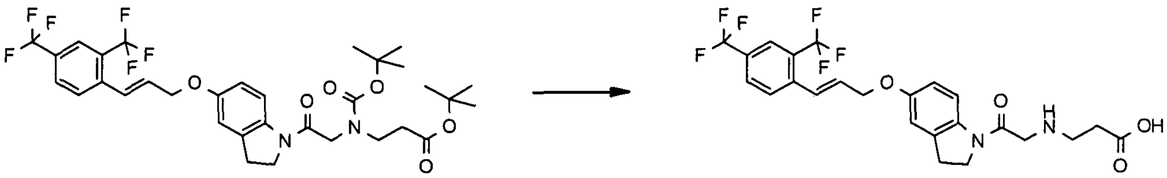

To a solution of 3-[N-[2-[5-[[(E)-3-[2, 4-bis(trifluoromethyl)phenyl]-2-propenyl]oxy]-2,3-dihydro-1H-indol-1-yl]-2-oxoethyl]-N-(tert-butoxycarbonyl)amino]propionic acid tert-butyl ester (215 mg) in dichloromethane (5.0 mL), TFA (1.0 mL) was added at room temperature. The reaction mixture was stirred at room temperature for 1.5 hours and concentrated under reduced pressure. The residue was purified by reverse phase high-performance liquid chromatography (column: Develosil Combi-RP-5 (10 cm), product of Nomura Chemical Co., Ltd.), followed by freeze-drying, whereby the title compound (116 mg) was yielded.
$^1$H-NMR(DMSO-$d_6$)δ:
2.41(2H,t,J=6.8Hz),2.88(2H,t,J=6.8Hz),3.13(2H,t,J=8.5Hz),3.64 (2H,s),4.04(2H,t,J=8.5Hz),4.80(2H,d,J=3.7Hz), 6.72-6.85(2H,m), 6.94(1H,s), 7.03(1H,d,J=15.4Hz), 7.94-8.14(4H,m).
Neither COOH nor NH was observed.
IR(ATR)cm$^{-1}$:1643,1493,1346,1282,1269,1171,1115.
MS(ESI)m/z:517(M+H)$^+$.
HR-MS(ESI)Calcd for
$C_{24}H_{23}F_6N_2O_4$ (M+H) $^+$: 517 15620 . Found: 517.15459.
Anal. Calcd for $C_{24}H_{22}F_6N_2O_4 \cdot$ 1.0$H_2O$:C,53.94;H,4.53;F,21.33;N,5.24.Found:C,53.72;H,4.09;F,2 1.92;N,5.10.

[Example 54]

3-[N-[2-[5-[3-[2,4-Bis(trifluoromethyl)phenyl]propoxy]-2,3-dihydro-1H-indol-1-yl]-2-oxoethyl]amino]propionic acid

(1) 3-[2,4-Bis(trifluoromethyl)phenyl]-1-propanol

[F231]

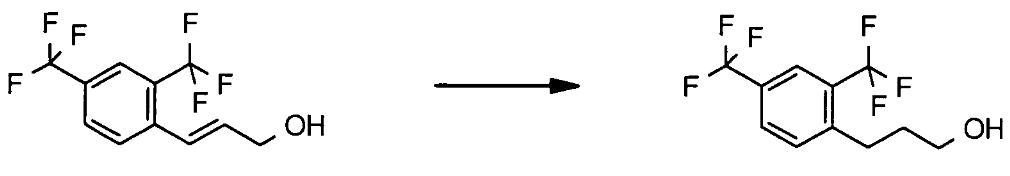

To a solution of (E)-3-[2,4-bis(trifluoromethyl)phenyl]-2-propen-1-ol (620 mg) in methanol (15 mL), 5% Pd/C (300 mg) was added at room temperature, and the mixture was stirred under a hydrogen atmosphere at room temperature for 2 hours. Insoluble matter was removed from the reaction mixture by filtration, and then the filtrate was concentrated under reduced pressure, whereby the title compound (611 mg) was yielded.
$^1$H-NMR ($CDCl_3$) δ :
1.38(1H,t,J=5.1Hz), 1.86-1.96(2H,m), 2.96(2H,t,J=7.9Hz), 3.70-3.78(2H,m), 7.52(1H,d,J=8.3Hz), 7.74(1H,d,J=8.3Hz), 7.88(1H,s).

(2) 3-[N-[2-[5-[3-[2,4-Bis(trifluoromethyl)phenyl]propoxy] - 2,3-dihydro-1H-indol-1-yl]-2-oxoethyl]-N-(tert-butoxycarbonyl)amino]propionic acid tert-butyl ester

[F232]

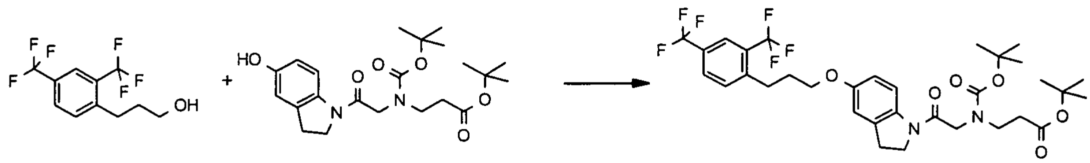

To a solution of 3-[2,4-bis(trifluoromethyl)phenyl]-1-propanol (148 mg) in THF (6.0 mL), 3-[N-(tert-butoxycarbonyl)-N-[2-(5-hydroxy-2,3-dihydro-1H-indol-1-yl)-2-oxoethyl]amino]propionic acid tert-butyl ester (200 mg), triphenylphosphine (155 mg), and DEAD (2.2M toluene solution) (0.269 mL) were added at room temperature. The reaction mixture was stirred at room temperature for 3 days and concentrated under reduced pressure. The residue was purified by silica gel column flash chromatography (Biotage 25M), whereby the title compound (208 mg) was yielded.
$^1$H-NMR ($CDCl_3$) δ:
1.45-1.60(18H,m), 1.87-1.95(2H,m), 2.10(2H,br s),2.54-2.63(2H, m), 2.96 (2H, t, J=8.2Hz), 3.04 (2H, t, J=7.7Hz), 3.13-3.23(2H,m), 3.55-3.62(2H,m), 3.95-4.20(2H,m), 7.65-7.78 (2H,m), 7.43-8.15 (4H,m) .
MS (ESI) m/z : 675 $(M+H)^+$.

(3) 3-[N-[2-[5-[3-[2,4-Bis(trifluoromethyl)phenyl]propoxy]-2,3-dihydro-1H-indol-1-yl]-2-oxoethyl]amino]propionic acid

[F233]

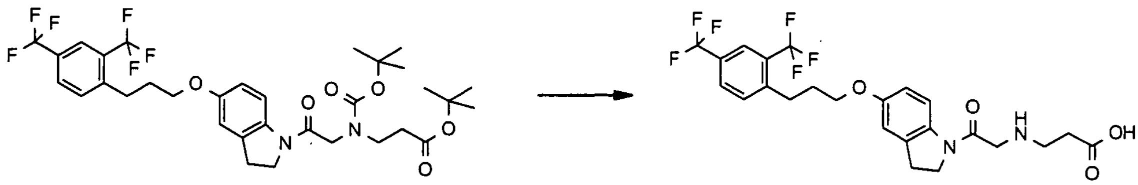

To a solution of 3-[N-[2-[5-[3-[2,4-bis(trifluoromethyl)phenyl]propoxy]-2,3-dihydro-1H-indol-1-yl]-2-oxoethyl]-N-(tert-butoxycarbonyl)amino]propionic acid tert-butyl ester (208 mg) in dichloromethane (5.0 mL), TFA (1.0 mL) was added at room temperature. The reaction mixture was stirred at room temperature for 1.5 hours and concentrated under reduced pressure. The residue was purified by reverse phase high-performance liquid chromatography (column: Develosil Combi-RP-5 (10 cm), product of Nomura Chemical Co., Ltd.), followed by freeze-drying of a target fraction, whereby the title compound (71.0 mg) was yielded.
$^1$H-NMR (DMSO-$d_6$) δ:
1.99-2.08(2H,m), 2.38(2H,t,J=6.7Hz), 2.85(2H,t,J=6.7Hz), 2.99(2H,t,J= 7.5Hz), 3.11(2H,t,J=8.4Hz), 3.59(2H,s), 2.96-3.07(4H,m), 6.72(1H,dd,J=8.8, 2.5Hz), 6.83(1H,d,J=2.5Hz), 7.79(1H ,t,J=8.0Hz), 7.95(1H,d,J=8.8Hz), 7.96(1H,s), 8.01(1H,d,J=8.0Hz).
Neither COOH nor NH was observed.
IR(ATR)cm$^{-1}$:1645,1493,1348,1279,1113.
MS (ESI)m/z:519 $(M+H)^+$.
HR-MS(ESI)Calcd for
$C_{24}H_{25}F_6N_2O_4(M+H)^+$: 519.17185. Found : 519.17069.
Anal. Calcd for $C_{24}H_{24}F_6N_2O_4 \cdot 0.5H_2O$:C,54.65;H,4.78;F,21.61;N,5.31.Found:C,54.31;H,4.47;F,2 2.12;N,5.17.

[Example 55]

3-[N-[2-[5-[[4-(Cyclohexyl)-3-(trifluoromethyl)phenyl]methoxy]indolin-1-yl]-2-oxoethyl]amino]propionic acid hydrochloride

(1) 4-(Cyclohexyl)-3-(trifluoromethyl)benzoic acid (WO 2006/131336)

[F234]

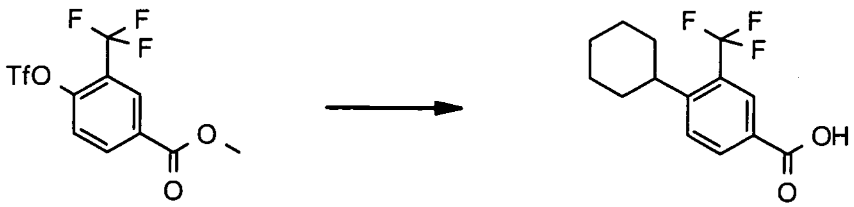

To a THF solution (100 mL) of 4-(trifluoromethanesulfonyloxy)-3-(trifluoromethyl)benzoic acid methyl ester (3.0 g) and 0.5M cyclohexylzinc bromide (51 mL), $Pd(tert\text{-}Bu_3P)_2$ (0.44 g) was added under a nitrogen atmosphere at room temperature, and the mixture was refluxed with stirring for 2 hours. The reaction mixture was cooled to room temperature, and then saturated aqueous sodium bicarbonate solution was added thereto. The mixture was stirred for a while, and then insoluble matter was removed by filtration through a Celite pad. The filtrate was extracted with ethyl acetate, and the extracts were combined. The combined extract was washed with saturated brine and dried over sodium sulfate anhydrate. Solvent was evaporated under reduced pressure, and the residue was purified by silica gel flash column chromatography (Biotage 40M), whereby 4-(cyclohexyl)-3-(trifluoromethyl)benzoic acid methyl ester was yielded, the product containing impurities difficult to remove. MS(ESI)m/z:309$(M+Na)^+$.
To a methanol/THF mixture solution (10 mL/20 mL) of the aforementioned mixture, 1N aqueous sodium hydroxide solution (10 mL) was added at room temperature, and the mixture was stirred for 20 hours. The reaction mixture was concentrated, and the residue was extracted with ether. 10% HCl was added to the aqueous layer, and the resulting precipitates were collected by filtration. The solid was dried under reduced pressure, whereby the title compound [1.4 g (2 steps)] was yielded.
$^1$H-NMR (DMSO-$d_6$) δ:
1.24-1.41(3H,m),1.48-1.86 (7H, m), 2.86 (1H, t, J=11.5Hz), 3.30 (1H, s), 7.77 (1H, d, J=8.1Hz), 8.11-8.17(2H,m).
MS (ESI) m/z :273 $(M+H)^+$.

(2) 4-(Cyclohexyl)-3-(trifluoromethyl)benzyl alcohol

[F235]

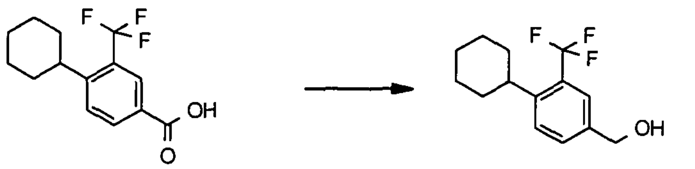

To a THF solution (20 mL) of 4-(cyclohexyl)-3-(trifluoromethyl)benzoic acid (1.2 g), 10M borane-dimethyl sulfide complex (1.3 mL) was added at room temperature, and the mixture was stirred for 17 hours. Saturated aqueous sodium bicarbonate solution was added to the reaction mixture, and the mixture was stirred for a while, followed by extraction with ethyl acetate. The extracts were combined and washed with saturated brine, followed by drying over sodium sulfate

anhydrate. Solvent was evaporated under reduced pressure, and then the residue was purified by silica gel flash column chromatography (Biotage 40S), whereby the title compound (1.2 g) was yielded.
$^1$H-NMR ($CDCl_3$) δ:
1.21-1.51(7H,m),1.73-1.90(4H,m),2.83-2.99(1H,m), 4.71(2H,s), 7.45(1H,d,J=7.4Hz), 7.50(1H,d,J=8.1Hz), 7 .60 (1H, s).

(3) 4-(cyclohexyl)-3-(trifluoromethyl)benzyl chloride

[F236]

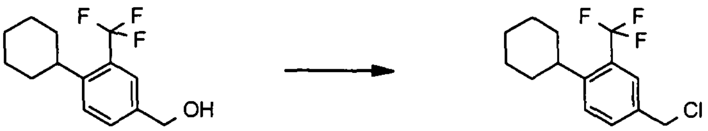

To a 1,2-dichloroethane solution (50 mL) of 4-(cyclohexyl)-3-(trifluoromethyl)benzyl alcohol (1.1 g), thionyl chloride (10 mL) was added at room temperature, and the mixture was stirred for 1 hour. The reaction mixture was concentrated, and the residue was purified by silica gel flash column chromatography (Biotage 40S), whereby the title compound (1.1 g) was yielded.
$^1$H-NMR ($CDCl_3$) δ :
1.26-1.49(6H,m), 1.78-1.92(5H,m), 4.58(2H,s), 7.45(1H,d,J=8.3Hz), 7.52(1H,d,J=8.1Hz), 7 .61(1H,s).

(4) 3-[N-(tert-Butoxycarbonyl)-N-[2-[5-[[4-(cyclohexyl)-3-(trifluoromethyl)phenyl]methoxy]indolin-1-yl]-2-oxoethyl]amino]propionic acid tert-butyl ester

[F237]

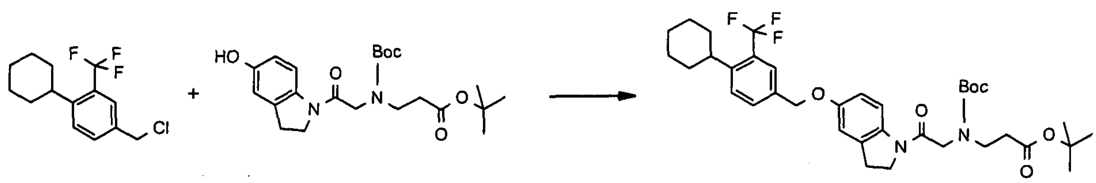

To a DMF solution (5 mL) of 4-(cyclohexyl)-3-(trifluoromethyl)benzyl chloride (99 mg) and 3-[N-(tert-butoxycarbonyl)-N-[[2-(5-hydroxyindolin-1-yl)-2-oxoethyl]amino]propionic acid tert-butyl ester (0.10 g), potassium carbonate (99 mg) was added at room temperature, and the mixture was stirred at 50°C for 14 hours. The reaction mixture was cooled to room temperature, and then insoluble matter was removed by filtration. The filtrate was concentrated, and the residue was purified by silica gel flash column chromatography (Biotage 25S), whereby the title compound (0.17 g) was yielded.
$^1$H-NMR ($CDCl_3$) δ:
1.22-1.52(23H,m),1.71-19.7(5H,m),2.54-2.66(2H,m),2.87-3.01(1H,m), 3.17-3.21(2H,m), 3.57-3.61(2H,m), 3.97-4.22 (4H,m), 5.00(2H,s), 6.77(1H,d,J=9.8Hz), 6.82(1H,d,J=9.3Hz), 7 .47(1H,d,J=7.4Hz), 7.54(1H,d,J=8.1Hz), 7.66(1H,s), 8.13(1H,t,J=9 . 8Hz).
MS (ESI) m/z : 661 (M+H)$^+$.

(5) 3-[N-[2-[5-[[4-(Cyclohexyl)-3-(trifluoromethyl)phenyl]methoxy]indolin-1-yl]-2-oxoethyl]amino]propionic acid hydrochloride

[F238]

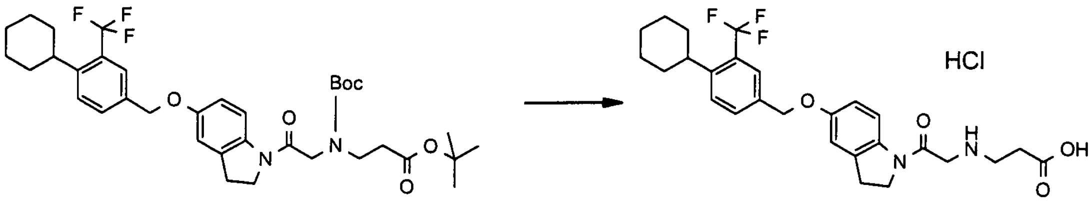

To 3-[N-(tert-butoxycarbonyl)-N-[2-[5-[[4-(cyclohexyl)-3-(trifluoromethyl)phenyl]methoxy]indolin-1-yl] -2-oxoethyl]amino]propionic acid tert-butyl ester (0.16 g, 0.24 mmol), 4N HCl/1,4-dioxane (5 mL) was added at room temperature, and the mixture was stirred for 23 hours, followed by concentration under reduced pressure. Diethyl ether was added to the residue, and the precipitated solid was collected by filtration, whereby the title compound (93 mg) was yielded.
$^1$H-NMR (DMSO-$d_6$) δ:
1.25-1.85(10H,m), 2.69-2.87(3H,m),3.18(4H,q,J=7.4Hz),4.05(2H,t,J=8.3Hz),4.13(2H,s),5 .11(2H,s), 6.87(1H,dd, J=8.7,2.6Hz), 7.01(1H,d,J=2.5Hz), 7.58-7.71(3H,m), 7.95(1H,d,J=8.8Hz).
IR(ATR)cm$^{-1}$:2925,2854,1720,1655,1556,1491,1419,1375.
MS(ESI)m/z:505(M+H).
Anal. Calcd for $C_{27}H_{31}F_3N_2O_4$·HCl· 1.25$H_2O$:C,57.55;H,6.17;C1,6.29;F,10.11;N,4.79.Found:C,57.44;H ,5.94;Cl,6.66; F,10.07;N,5.18.

[Example 56]

3-[N-[2-[5-[(1-(Cyclohexylmethyl)-3-(trifluoromethyl)-1H-pyrazol-4-ylmethoxy)indolin-1-yl]-2-oxoethyl]amino]propionic acid

(1) 1-(Cyclohexylmethyl)-3-(trifluoromethyl)-1H-pyrazole-4-carboxylic acid methyl ester

[F239]

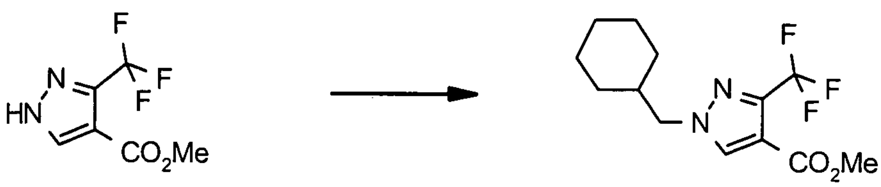

To a DMF solution (20 mL) of 3-(trifluoromethyl)-1H-pyrazole-4-carboxylic acid methyl ester (Lancaster) (1.0 g), cyclohexylmethyl bromide (1.0 mL) was added at room temperature, followed by cooling to 0°C. 60% Sodium hydride (0.42 g, 9.6 mmol) was added to the reaction mixture, and the mixture was stirred for 15 hours during which the mixture was allowed to warm to room temperature. The reaction mixture was added to ice water, and the mixture was extracted with ethyl acetate. The extracts were combined and washed with saturated brine, followed by drying over sodium sulfate anhydrate. Solvent was evaporated, and the residue was purified by silica gel flash column chromatography (Biotage 25S), whereby the title compound (0.25 g) was yielded.
$^1$H-NMR ($CDCl_3$) δ:
0.76-1.05(5H,m),1.14-1.45(4H,m),1.52-1.80(4H,m),1.81-1.99 (1H,m), 3.97 (2H,d,J=7.4Hz), 7.92 (1H, s).

MS (ESI) m/z :291 $(M+H)^+$.

(2) 1-(Cyclohexylmethyl)-3-(trifluoromethyl)-1H-pyrazole-4-carboxylic acid

[F240]

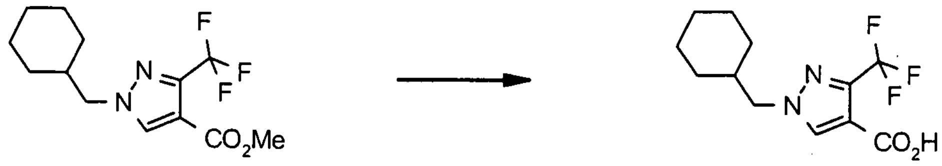

To a methanol/THF solution (2 mL/4 mL) of 1-(cyclohexylmethyl)-3-(trifluoromethyl)-1H-pyrazole-4-carboxylic acid methyl ester, 1N aqueous sodium hydroxide solution (2 mL) was added at room temperature, and the mixture was stirred for 21 hours. The reaction mixture was concentrated, and 1N HCl was added to the residue. The precipitated solid was collected by filtration, and the solid was dried under reduced pressure, whereby the title compound (0.16 g) was yielded.
$^1$H-NMR (DMSO-$d_6$) δ:
0.85-1.01(2H,m),1.07-1.25(2H,m),1.42-1.52(2H,m),1.54-1.71 (4H,m), 1.89-1.74 (1H,m), 4.04 (2H, d, J=7.1Hz), 8.45 (1H, s) .
MS (ESI) m/z : 277 $(M+H)^+$.

(3) 1-(Cyclohexylmethyl)-4-hydroxymethyl-3-(trifluoromethyl)-1H-pyrazole

[F241]

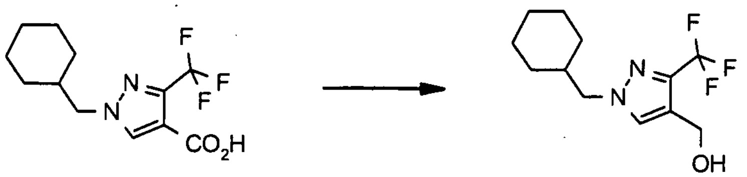

To a THF solution (3 mL) of 1-(cyclohexylmethyl)-3-(trifluoromethyl)-1H-pyrazole-4-carboxylic acid (0.16 g), 10M borane-dimethyl sulfide complex (0.61 mL) was added at room temperature, and the mixture was stirred for 19 hours. Saturated aqueous sodium bicarbonate solution was added to the reaction mixture, and the mixture was stirred for a while, followed by extraction with ethyl acetate. The extracts were combined and washed with saturated brine, followed by drying over sodium sulfate anhydrate. Solvent was evaporated under reduced pressure, and the residue was purified by silica gel flash column chromatography (Biotage 25S), whereby the title compound (0.16 g) was yielded.
$^1$H-NMR ($CDCl_3$) δ:
0.89-1.02(2H,m),1.09-1.32(3H,m),1.55-1.94(6H,m),2.06-2.21(1H,m), 3.93(2H,d,J=6.6Hz), 4.66(2H,s), 7.43(1H,s).
MS (ESI) m/z :263 $(M+H)^+$.

(4) 3-[N-(tert-Butoxycarbonyl)-N-[2-[5-[(1-(cyclohexylmethyl)-3-(trifluoromethyl)-1H-pyrazol-4-ylmethoxy)indolin-1-yl]-2-oxoethyl]amino]propionic acid tert-butyl ester

[F242]

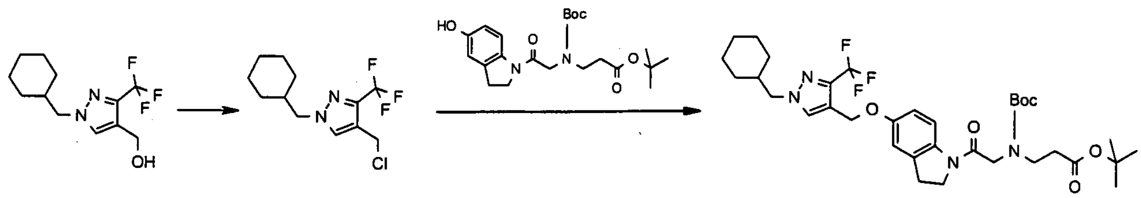

The title compound (0.37 g) was yielded from 1-(cyclohexylmethyl)-4-hydroxymethyl-3-(trifluoromethyl)-1H-pyrazole (0.16 g) in a manner similar to those of the steps (3) and (4) in Example 55.
$^1$H-NMR ($CDCl_3$) δ :
0.87-1.34(8H,m),1.40-1.91(23H,m),2.50-2.69(2H,m),3.12-3.27(2H,m), 3.50-3.68(2H,m), 3.88-4.24(4H,m), 5.00(2H,s), 6.67-6.87(2H,m), 7.41-7.51(1H,m), 8.00-8.19(1H,m).
MS (ESI)m/z:665 $(M+H)^+$.

(5) 3-[N-[2-[5-[(1-(Cyclohexylmethyl)-3-(trifluoromethyl)-1H-pyrazol-4-ylmethoxy]indolin-1-yl]-2-oxoethyl]amino]propionic acid

[F243]

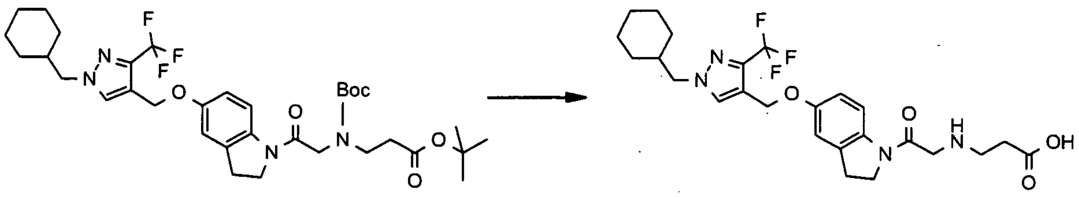

The title compound (53 mg) was yielded from 3-[N-(tert-butoxycarbonyl)-N-[2-[5-(1-(cyclohexylmethyl)-3-(trifluoromethyl)-1H-pyrazol-4-ylmethoxy]indolin-1-yl]-2-oxoethyl]amino]propionic acid tert-butyl ester (0.37 g) in a manner similar to that of the step (5) in Example 55.
$^1$H-NMR (DMSO-$d_6$) δ:
0.82-1.22(4H,m),1.36-1.84(6H,m), 2.34(3H,t,J=6.6Hz), 2.81(2H,t,J=6.5Hz), 3.10(2H,t,J= 8.3Hz),3.52(2H,s),4.08-3.95 (4H,m), 4.94(2H,s), 6.78(1H,dd,J=8.7,2.6Hz), 6.89(1H,s), 7.96 (1H,d,J=8.8Hz), 8.04(1H,s).
MS (ESI) m/z : 509 $(M+H)^+$.
HR-MS(AqTOF)Calcd for
$C_{25}H_{32}F_3N_4O_4$ (M+H) $^+$: 509.2376. Found: 509.2350.
IR(ATR)$cm^{-1}$:2922,2850,1728,1643,1620,1595,1493,1403.
Anal. Calcd for $C_{25}H_{31}F_3N_4O_4 \cdot$ 1.25$H_2O$:C,56.54;H,6.36;F,10.73;N,10.55.Found:C,56.42;H,6.13;F ,10.40;N,10.40.

[Example 57]

3-[N-[2-[5-[[4-(Cyclopentyl)-2-fluorobenzyl]oxy]indolin-1-yl]-2-oxoethyl]amino]propionic acid

(1) 2-Fluoro-4-hyroxybenzoic acid methyl ester (Husain, Mazhar, *et al.*, Journal of Biological Chemistry (1980), 255 (9), 4189-97)

[F244]

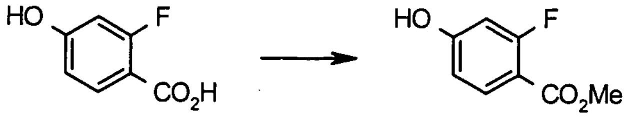

To a methanol solution (50 mL) of 2-fluoro-4-hydroxybenzoic acid (Matrix) (5.0 g), sulfuric acid (1 mL) was added, and the mixture was refluxed with stirring for 3 hours. The reaction mixture was cooled to room temperature and concentrated. Water was added to the residue, followed by extraction with dichloromethane. The extracts were combined and washed with saturated brine, followed by drying over sodium sulfate anhydrate. Solvent was evaporated, whereby the title compound (3.2 g) was yielded.
$^1$H-NMR (DMSO-$d_6$) δ:
3.77(3H,s), 6.62(1H,dd,J=13.1,2.3Hz), 6.69(1H,dd,J=8.8,2.5Hz), 7 .75(1H,t,J=8.8Hz), 10.79(1H,s).
MS (ESI) m/z : 171 $(M+H)^+$.

(2) 4-(Trifluoromethanesulfonyloxy)-2-fluorobenzoic acid methyl ester

[F245]

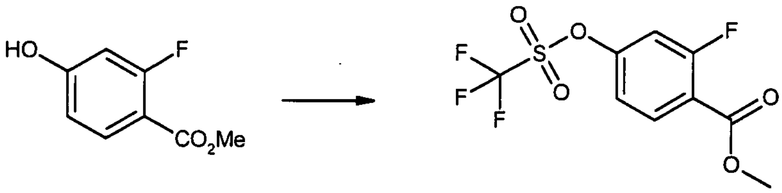

To a dichloromethane solution (50 mL) of 2-fluoro-4-hydroxybenzoic acid methyl ester (3.2 g) and pyridine (10 mL), trifluoromethanesulfonic anhydride (4.6 mL) was added at room temperature, and the mixture was stirred for 18 hours. The reaction mixture was concentrated, and 1N hydrochloric acid was added to the residue, followed by extraction with ethyl acetate. The extracts were combined and the combined extract was sequentially washed with saturated aqueous sodium bicarbonate solution and saturated brine, followed by drying over sodium sulfate anhydrate. Solvent was evaporated, and the residue was purified by silica gel flash column chromatography (Biotage 25S), whereby the title compound (5.6 g) was yielded.
$^1$H-NMR ($CDCl_3$) δ :
3.96(3H,s), 7.14(1H,dd,J=9.6,2.5Hz), 7.18(1H,dd,J=8.3,3.4Hz), 8. 08(1H,t,J=8.3Hz).

(3) 4-(Cyclopentyl)-2-fluorobenzoic acid (WO 2006/047195)

[F246]

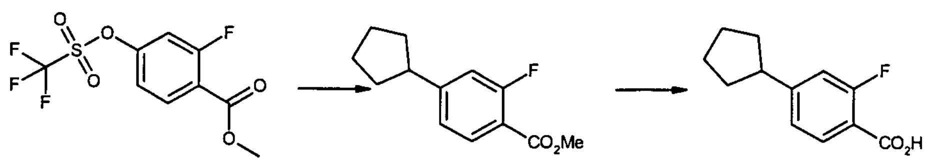

The title compound (0.76 g) was yielded from 4-(trifluoromethanesulfonyloxy)-2-(fluoromethyl)benzoic acid

methyl ester (1.5 g) in a manner similar to that of the step (1) in Example 55.
$^1$H-NMR (DMSO-$d_6$) δ:
1.43-1.84(6H,m), 1.94-2.09(2H,m), 2.91-3.08(1H,m), 7.15(1H,dd,J=5.9,1.5Hz), 7.17(1H,s), 7.76(1H,t,J=8.1 Hz).
MS (ESI) m/z : 209 $(M+H)^+$.

(4) 4-(Cyclopentyl)-2-fluorobenzyl alcohol

[F247]

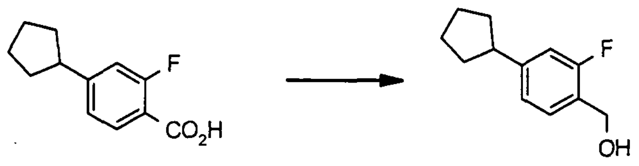

The title compound (0.71 g) was yielded from 4-(cyclopentyl)-2-fluorobenzoic acid (0.76 g) in a manner similar to that of the step (2) in Example 55.
$^1$H-NMR ($CDCl_3$) δ:
1.51-1.85(7H,m),1.99-2.14(2H,m),2.89-3.05(1H,m),4.72(2H,d,J=6.4Hz),6.94(1H,dd,J=11.6,1.6Hz), 7.02(1 H,dd,J=7.8, 1.7Hz), 7.30(1H,t,J=7.8Hz).

(5) 4-(Cyclopentyl)-2-fluorobenzyl chloride

[F248]

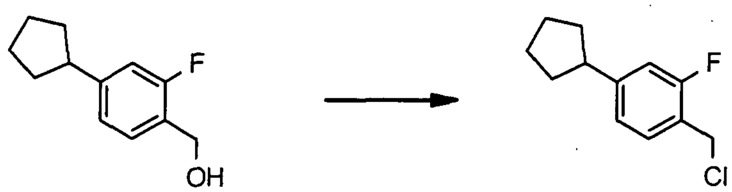

Thionyl chloride (10 mL) was added to 4-(cyclopentyl)-2-fluorobenzyl alcohol (0.71 g) at room temperature, and the mixture was heated to 60°C, followed by stirring for 15 hours. The reaction mixture was concentrated, and the residue was purified by silica gel flash column chromatography (Biotage 25S), whereby the title compound (0.56 g) was yielded.
$^1$H-NMR ($CDCl_3$) δ:
1.38-2.17(8H,m),2.82-3.08(1H,m), 4.62(2H,s), 6.95(1H,d,J=12.3Hz), 7.01(1H,dd, J=7.8,1. 5Hz), 7.30(1H,t,J=8.0Hz).

(6) 3-[N-(tert-Butoxycarbonyl)-N-[2-[5-[[4-(cyclopentyl)-2-fluorobenzyl]oxy]indolin-1-yl]-2-oxoethyl]amino]propionic acid tert-butyl ester

[F249]

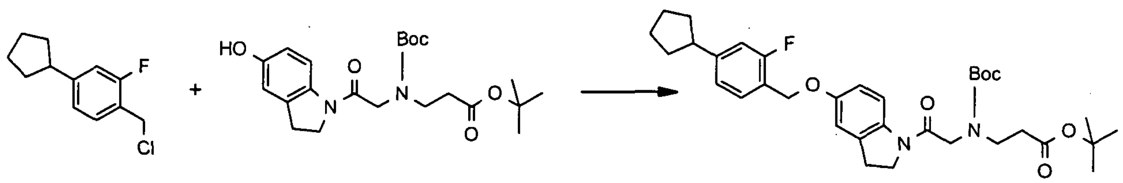

To a DMF solution (5 mL) of 4-(cyclopentyl)-2-(trifluoromethyl)benzyl chloride (0.10 g) and 3-[N-(tert-butoxycarbonyl)-N-[2-(5-hydroxyindolin-1-yl)-2-oxoethyl]amino]propionic acid tert-butyl ester (0.21 mg), potassium carbonate (0.21 g) was added at room temperature, and the mixture was stirred at 60°C for 18 hours. The reaction mixture was cooled to room temperature, and then insoluble matter was removed by filtration. The filtrate was concentrated, and the residue was purified by silica gel flash column chromatography (Biotage 25S), whereby the title compound (0.25 g) was yielded.
$^1$H-NMR ($CDCl_3$) $\delta$ :
1.06-1.89(23H,m),1.95-2.14(2H,m),2.51-2.65(2H,m),2.89-3.25 (3H,m), 3.51-3.64 (2H,m), 3.92-4.29 (4H,m), 5.06 (2H, s), 6.74-7.05(5H,m), 7.36(1H,t,J=6.8Hz), 8.12(1H,t,J=9.6Hz).
MS (ESI) m/z : 597 $(M+H)^+$.

(7) 3-[[2-[5-[[4-(Cyclopentyl)-2-fluorobenzyl]oxy]indolin-1-yl]-2-oxoethyl]amino]propionic acid

[F250]

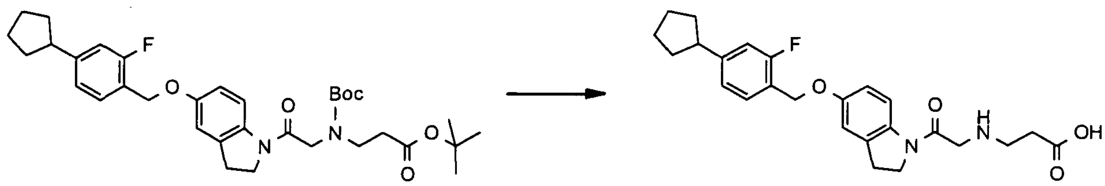

To a dichloromethane solution (10 mL) of 3-[N-(tert-butoxycarbonyl)-N-[2-[5-[[4-(cyclopentyl)-2-fluorobenzyl]oxy]indolin-1-yl]-2-oxoethyl]amino]propionic acid tert-butyl ester (0.25 g), TFA (2.0 mL) was added, and the mixture was stirred for 13 hours. The reaction mixture was concentrated, and the pH of the residue was adjusted to 7 by use of 1N HCl and 1N aqueous sodium hydroxide solution, followed by extraction with a chloroform/methanol mixture. The extracts were combined and washed with saturated brine, followed by drying over sodium sulfate anhydrate. Solvent was evaporated, and the residue was purified by reverse phase chromatography (Gilson, NOMURA Develosil Combi-RP5), followed by freeze-drying from 1,4-dioxane, whereby the title compound (0.75 mg) was yielded.
$^1$H-NMR (DMSO-$d_6$) $\delta$ :
1.45-2.06(8H,m),2.17-2.44(2H,m),2.74-3.18(6H,m), 3.62(1H,s), 4.03(2H,t,J=8.2Hz), 5.02(2H,s), 6.82(1H,d d, J=9.1,3.4Hz), 6.94(1H,s), 7.02-7.15(2H,m), 7.31-7.48(1H,m), 7.86-8.03(1H,m).
MS (ESI) m/z :441 $(M+H)^+$.

[Example 58]

3-[N-[2-[5-[[4-(Cyclopentyl)-3-(trifluoromethyl)benzyl]oxy]indolin-1-yl]-2-oxoethyl]amino]propionic acid

(1) 4-(Cyclopentyl)-3-(trifluoromethyl)benzyl alcohol

[F251]

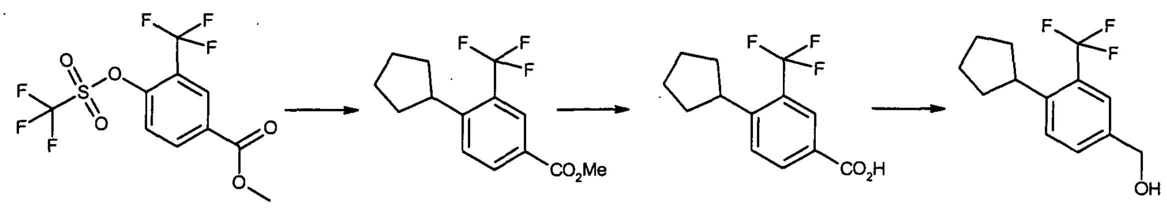

The title compound (0.29 g) was yielded from 4-(trifluoromethanesulfonyloxy)-3-(trifluoromethyl)benzoic acid methyl ester (1.8 g) in a manner similar to those of the steps (1) and (2) in Example 55.
$^1$H-NMR ($CDCl_3$) $\delta$ :
1.45-1.92(6H,m), 1.99-2.17(2H,m), 3.32-3.40(1H,m), 4.71(2H,d,J=5.9Hz), 7.41-7.70(3H,m).

(2) 3-[N-(tert-Butoxycarbonyl)-N-[2-[5-[[4-(cyclopentyl)-3-(trifluoromethyl)benzyl]oxy]indolin-1-yl]-2-oxoethyl]amino]propionic acid tert-butyl ester

[F252]

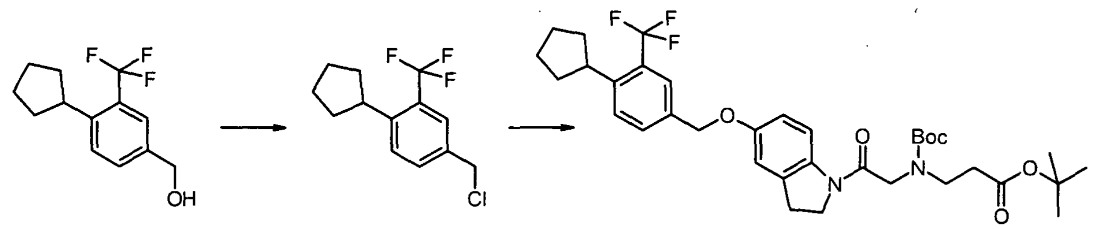

The title compound (0.47 g) was yielded from 4-(cyclopentyl)-3-(trifluoromethyl)benzyl alcohol (0.29 g) in a manner similar to those of the steps (3) and (4) in Example 55.
$^1$H-NMR ($CDCl_3$) $\delta$ :
1.34-1.91(23H,m), 1.98-2.16(3H,m), 2.53-2.70(2H,m), 3.09-3.25(2H,m), 3.30-3.48(1H,m), 3.51-3.64(2H,m), 3.90-4.26 (4H,m), 5.02 (2H, s), 6.70-6.87(2H,m), 7.48(1H,d,J=8.8Hz), 7.54(1H,d,J=8.1Hz), 7.65(1H,s),8 .06-8.23 (1H,m).

(3) 3-[N-[2-[5-[[4-(Cyclopentyl)-3-(trifluoromethyl)benzyl]oxy]indolin-1-yl]-2-oxoethyl]amino]propionic acid

[F253]

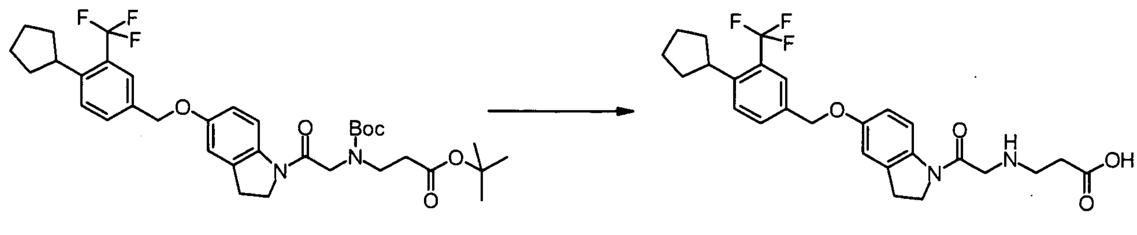

To a dichloromethane solution (20 mL) of 3-[N-(tert-butoxycarbonyl)-N-[2-[5-[[4-(cyclopentyl)-3-(trifluoromethyl) benzyl]oxy]indolin-1-yl]-2-oxoethyl]amino]propionic acid tert-butyl ester (0.47 g), TFA (1.0 mL) was added, and the mixture was stirred at room temperature for 3 days. The reaction mixture was concentrated, and 1N aqueous sodium hydroxide solution was added to the residue, followed by extraction with a chloroform/methanol mixture. The extracts

were combined and washed with saturated brine, followed by drying over sodium sulfate anhydrate. Solvent was evaporated, and the residue was purified by silica gel flash column chromatography (Yamazen Hi-Flash L), followed by formation of solid through addition of diethyl ether, whereby the title compound (0.31 g) was yielded.
$^1$H-NMR (DMSO-$d_6$) δ:
1.51-1.74(4H,m),1.77-2.02(4H,m),2.31-2.43(2H,m),2.75-2.92(2H,m),3.03-3.21(2H,m),3.49-3.64(2H,m),3.95-4.09(2H, m), 5.10(2H,s), 6.82(1H,d,J=8.3Hz), 6.95(1H,s),7.57-7.71(4H,m), 7.96(1H,d,J=8.8Hz).
IR(ATR)cm$^{-1}$:2951,2870,1658,1595,1491,1415,1377.

[Example 59]

3-[N-[2-Oxo-2-[5-[[4-phenoxy-3-(trifluoromethyl)benzyl]oxy]indolin-1-yl] ethyl] amino] propionic acid

(1) 4-(Phenoxy)-3-(trifluoromethyl)benzaldehyde

[F254]

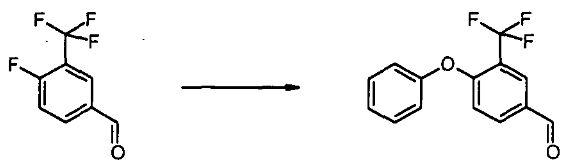

To a DMF solution (15 mL) of 4-fluoro-3-(trifluoromethyl)benzaldehyde (Aldrich) (0.96 g, 5.0 mmol) and phenol (0.47 g), potassium carbonate (2.1 g) was added at room temperature, and the mixture was heated to 70°C, followed by stirring for 7 hours. The reaction mixture was cooled to room temperature and added to ice water, followed by extraction with ethyl acetate. The extracts were combined and washed with saturated brine, followed by drying over sodium sulfate anhydrate. Solvent was evaporated, and the residue was purified by silica gel flash column chromatography (Biotage 40S), whereby the title compound (0.13 g) was yielded.
$^1$H=NMR ($CDCl_3$) δ :
6.96(1H,d,J=8.1Hz), 7.12(2H,d,J=8.1Hz), 7.29(1H,d,J=7.4Hz), 7.41 -7.48(2H,m), 7.94(1H,dd,J=8.6,2.0Hz), 8.21(1H,s), 9.96(1H,s).
MS(ESI)m/z:267(M+H)$^+$.

(2) 4-(Phenoxy)-3-(trifluoromethyl)benzyl alcohol

[F255]

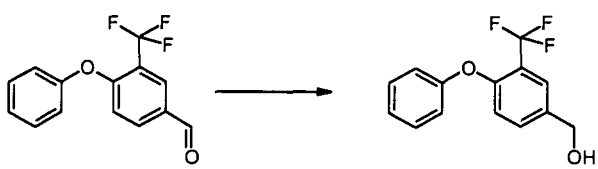

To a methanol solution (1.0 mL) of 4-(phenoxy)-3-(trifluoromethyl)benzaldehyde (0.13 g), sodium borohydride (18 mg) was added at room temperature, and the mixture was stirred for 2 days. Water was added to the reaction mixture, and the mixture was extracted with ethyl acetate. The extracts were combined and washed with saturated brine, followed by drying over sodium sulfate anhydrate. Solvent was evaporated, and the residue was purified by silica gel flash column chromatography (Biotage 40S), whereby the title compound (88 mg) was yielded.
$^1$H-NMR ($CDCl_3$) δ:
1.74(1H,t,J=5.5Hz), 4.71(2H,d,J=5.1Hz), 6.92(1H,d,J=8.6Hz), 7.02 -7.04(2H,m), 7.21-7.08(1H,m), 7.30-7.54(3H,m),

7.68(1H,d,J=1.7Hz).

(3) 3-[N-(tert-Butoxycarbonyl)-N-[2-oxo-2-[5-[[4-phenoxy-3-(trifluoromethyl)benzyl]oxy]indolin-1-yl]ethyl]amino]propionic acid tert-butyl ester

[F256]

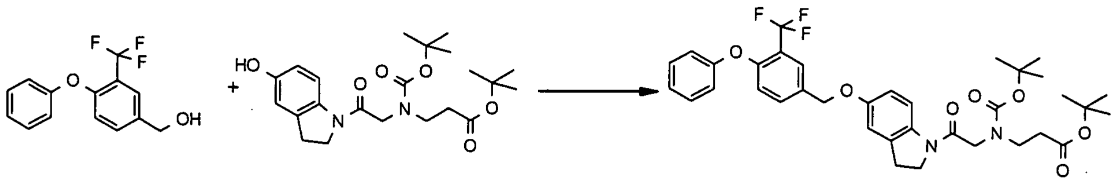

To a solution of [4-phenoxy-3-(trifluoromethyl)phenyl]methanol (88.0 mg) in THF (4.0 mL), 3-[N-(tert-butoxycarbonyl)-N-[2-(5-hydroxy-2,3-dihydro-1H-indol-1-yl)-2-oxoethyl]amino]propionic acid tert-butyl ester (152 mg), triphenylphosphine (103 mg), and DEAD (2.2M toluene solution) (0.178 mL) were added at room temperature. The reaction mixture was stirred at room temperature for 18 hours and concentrated under reduced pressure. The residue was purified by preparative silica gel thin-layer chromatography, whereby the title compound (136 mg) was yielded.
$^1$H-NMR ($CDCl_3$) $\delta$ :
8.10-8.17(1H,m), 7.68-7.76(1H,m), 7.43-7.53(1H,m), 7.33-7.41(2H,m), 7.13-7.20(1H,m), 7.00-7.08(2H,m), 6.90-6.96 (1H,m), 6.74-6.85(2H,m), 5.00(2H,s), 3.99-4.82(4H,m), 3.54-3.62(2H,m), 3.14-3.23(2H,m),2.54-2.63(2H,m), 1.37-1.58 (18H,m).
MS (ESI) m/z : 671 $(M+H)^+$.

(4) 3-[N-[2-Oxo-2-[5-[[4-phenoxy-3-(trifluoromethyl)benzyl]oxy]indolin-1-yl] ethyl] amino] propionic acid

[F257]

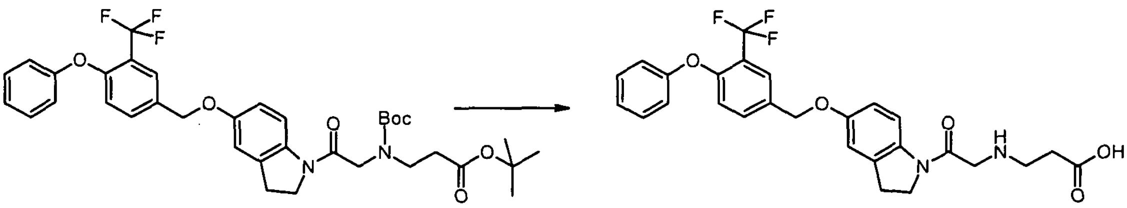

To a dichloromethane solution (10 mL) of 3-[N-(tert-butoxycarbonyl)-N-[2-oxo-2-[5-[[4-phenoxy-3-(trifluoromethyl)benzyl]oxy]indolin-1-yl]ethyl]amino]propionic acid tert-butyl ester (135 mg), TFA (1 mL) was added, and the mixture was stirred for 6 hours. The reaction mixture was concentrated, and saturated aqueous sodium bicarbonate solution was added to the residue, followed by extraction with chloroform/methanol (3:1, v/v) mixture. The extracts were combined and dried over sodium sulfate anhydrate. Thereafter, solvent was evaporated, and the residue was purified by thin-layer chromatography on silica gel, whereby the title compound (16 mg) was yielded.
$^1$H-NMR ($CDCl_3$) $\delta$ :
0.77(2H,s),0.82-0.95(1H,m), 1.05(3H,d,J=6.6Hz), 1.27(2H,s), 2.19-2.22(1H,m), 2.37-2.71(4H,m), 3.09-3.30(2H,m), 3.57-4.27 (2H,m), 5.02 (2H, s), 6.75-6.89 (2H,m), 7.05 (1H, s), 7.41-7.53(1H,m), 7.66(1H,s).
MS (ESI) m/z : 515 $(M+H)^+$.

[Example 60]

3-[N-[2-[5-(4-Isopropyl-3-trifluoromethylbenzyloxy)indolin-1-yl]-2-oxoethyl]amino]propionic acid TFA salt Synthesis method for isopropenylboronic acid (reference: US 2007/3539 and US 2006/472845)

(1) 4-Isopropenyl-3-trifluoromethylbenzoic acid methyl ester

[F258]

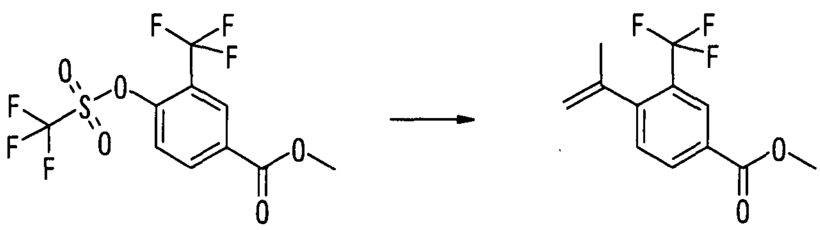

To a toluene solution (14 mL) of 4-trifluoromethanesulfonyloxy-3-trifluoromethylbenzoic acid methyl ester (1.58 g), isopropenylboronic acid (US 2007/3539 and US 2006/472845) (770 mg), cesium carbonate (4.40 g), water (7.0 mL), and tetrakis(triphenylphosphine)palladium(0) (520 mg) were added, and the mixture was stirred at reflux overnight. The reaction mixture was left to stand to cool to room temperature, and then water was added to the mixture, followed by extraction thrice, each with diethyl ether. The extracts were combined and washed with saturated brine, followed by drying over sodium sulfate anhydrate. Insoluble matter was removed by filtration. Thereafter, the filtrate was concentrated under reduced pressure, and the residue was purified by flash column chromatography (Yamazen Hi-Flash Column 2L), whereby the title compound (1.05 g) was yielded as a pale yellow oily substance.
$^1$H-NMR ($CDCl_3$) $\delta$ :
2.09(3H,s), 3.95(3H,s), 4.92(1H,s),5.27(1H,t,J=1.5Hz), 7.34(1H,d ,J=7.8Hz), 8.14(1H,dd,J=1.2,7.8Hz), 8.32(1H,d, J=1.2Hz).
MS (ESI) m/z : 245 $(M+H)^+$.

(2) 4-Isopropyl-3-trifluoromethylbenzoic acid methyl ester

[F259]

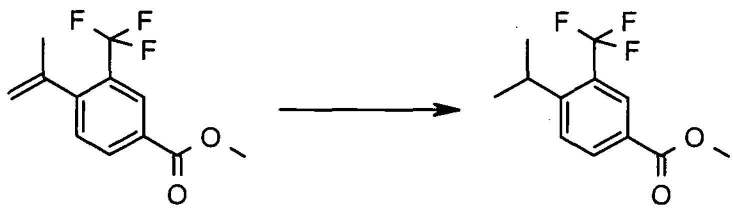

To an ethyl acetate solution (20 mL) of 4-isopropyl-3-trifluoromethylbenzoic acid methyl ester (1.05 g), 5% Pd/C (hydrous) (300 mg) was added, and the mixture was stirred under a hydrogen atmosphere at room temperature for 1.5 hours. The reaction mixture was filtered, and the filtrate was concentrated under reduced pressure, whereby the title compound (1.02 g) was yielded.
$^1$H-NMR ($CDCl_3$) $\delta$ :
1.29(6H,d,J=6.9Hz), 3.37-3.44(1H,m), 3.94(3H,s), 7.56(1H,d,J=8.3Hz), 8.16(1H,dd,J=1.5,8.3 Hz), 8.28 (1H, d, J=1.5Hz).
MS (ESI) m/z : 247 $(M+H)^+$.

(3) (4-Isopropyl-3-trifluoromethylphenyl)methanol

[F260]

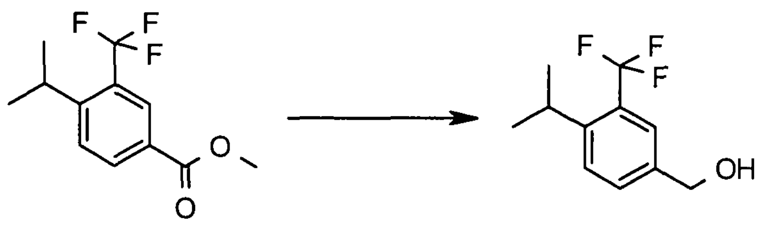

To a THF solution (30 mL) of 4-isopropyl-3-trifluoromethylbenzoic acid methyl ester (1.00 g), lithium borohydride (177 mg) was added, and the mixture was stirred at reflux overnight. The reaction mixture was left to stand to cool to room temperature, and then 1N hydrochloric acid was added thereto, followed by extraction thrice, each with ethyl acetate. The extracts were combined and washed with saturated brine, followed by drying over sodium sulfate anhydrate. Insoluble matter was removed by filtration. Thereafter, the filtrate was concentrated under reduced pressure, and the residue was purified by flash column chromatography (Yamazen Hi-Flash Column L), whereby the title compound (840 mg) was yielded.
$^1$H-NMR ($CDCl_3$) $\delta$ :
1.26(6H,d,J=6.9Hz), 1.72(1H,s), 3.32-3.38(1H,m), 4.71(2H,s), 7.46-7.52(2H,m), 7.60(1H,s).
MS (ESI)m/z:201 $(M-OH)^+$.

(4) 4-Chloromethyl-1-isopropyl-2-trifluoromethylbenzene

[F261]

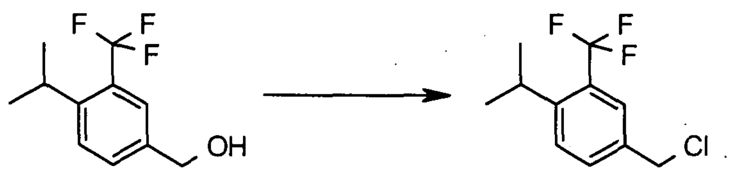

To a 1,2-dichloroethane solution (20 mL) of (4-isobutyl-3-trifluoromethylphenyl)methanol (830 mg), thionyl chloride (1.38 mL) and DMF (2 drops by means of a Pasteur pipette) were added, and the mixture was stirred at 50°C for 1 hour. The reaction mixture was left to stand to cool to room temperature and concentrated under reduced pressure. The residue was purified by flash column chromatography (Yamazen Hi-Flash Column L), whereby the title compound (810 mg) was yielded.
$^1$H-NMR ($CDCl_3$) $\delta$ :
1.26(6H,d,J=6.9Hz), 3.32-3.39(1H,m), 4.59(2H,s), 7.46-7.61(3H,m).

(5) 3-[N-tert-Butoxycarbonyl-N-[2-[5-(4-isopropyl-3-trifluoromethylbenzyloxy)indolin-1-yl]-2-oxoethyl]amino]propionic acid tert-butyl ester

[F262]

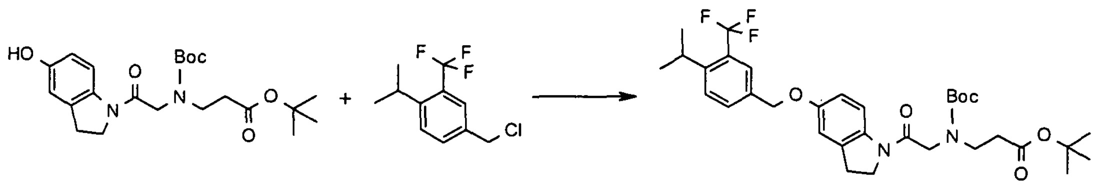

To a DMF solution (10 mL) of 3-[N-(tert-butoxycarbonyl)-N-[2-(5-hydroxyindolin-1-yl)-2-oxoethyl]amino]propionic acid tert-butyl ester (231 mg), 4-chloromethyl-1-isopropyl-2-trifluoromethylbenzene (118 mg) and potassium carbonate (104 mg) were added, and the mixture was stirred at 70°C overnight. The reaction mixture was left to stand to cool to room temperature, and then water was added thereto, followed by extraction thrice, each with ethyl acetate. The extracts were combined and washed with saturated brine, followed by drying over sodium sulfate anhydrate. Insoluble matter was removed by filtration. Thereafter, the filtrate was concentrated under reduced pressure, and the residue was purified by flash column chromatography (Yamazen Hi-Flash Column L), whereby the title compound (338 mg) was yielded.
$^1$H-NMR ($CDCl_3$) δ:
1.27(6H,d,J=6.9Hz), 1.40-1.50(18H,m), 2.55-2.63(2H,m), 3.16-3.22(2H,m), 3.33-3.39(1H,m), 3.56-3.61(2H,m), 3.99-4.18(4H,m),5.01(2H,s),6.76-6.84(2H,m), 7.49 (1H, d, J=8.1Hz), 7.56 (1H, d, J=8.1Hz), 7.65 (1H, s), 8 .10-8.15(1H, m).
MS (ESI) m/z : 621 $(M+H)^+$.

(6) 3-[N-[2-[5-(4-Isopropyl-3-trifluoromethylbenzyloxy)indolin-1-yl]-2-oxoethyl]amino]propionic acid TFA salt

[F263]

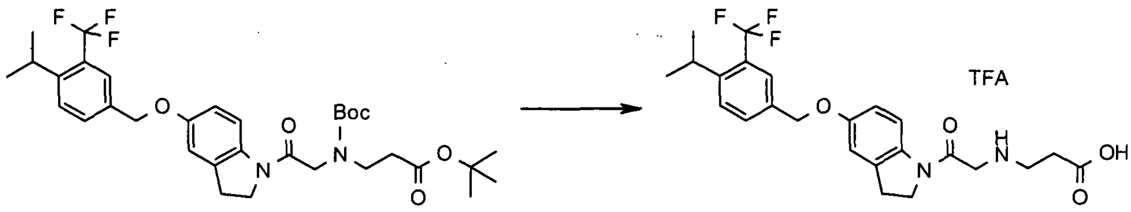

To a dichloromethane solution (8.0 mL) of 3-[N-(tert-butoxycarbonyl)-N-[2-[5-(4-isopropyl-3-trifluoromethylbenzyloxy)indolin-1-yl]-2-oxoethyl]amino]propionic acid tert-butyl ester (310 mg), TFA (2.0 mL) was added under cooling on ice, and the mixture was stirred at room temperature for 4 hours. Thereafter, TFA (1.0 mL) was added to the reaction mixture, followed by further stirring at room temperature for 1 hour. The reaction mixture was concentrated under reduced pressure, and the residue was suspended in diethyl ether, followed by collecting by filtration, whereby the title compound (271 mg) was yielded.
$^1$H-NMR (DMSO-$d_6$) δ:
1.24(6H,d,J=6.6Hz),2.73(2H,t,J=7.4Hz),3.16-3.27(5H,m),4.04-4.14(4H,m), 5.13(2H,s), 6.88(1H,dd,J=2.7,8.8Hz), 7.02 (1H,d,J=2.7 Hz), 7.66-7.71(3H,m), 7.97(1H,d,J=8.8Hz).
IR(ATR)$cm^{-1}$:2962,1641,1182,1133,1116.
MS (ESI) m/z : 465 $(M+H)^+$.
HR-MS (ESI) calcd for $C_{24}H_{28}F_3N_2O_4$ $(M+H)^+$:465.20012; found 465.20006.
Anal. Calcd for $C_{24}H_{27}F_3N_2O_4$.
$CF_3CO_2H$:C,53.98;H,4.88;F,19.70;N,4.84.Found:C,53.98;H,4.96;F, 19.44;N,4.63.

[Example 61]

3-[N-[2-[5-[4-(1-Methyl-1-cyclopropyl)-3-(trifluoromethyl)benzyloxy]-2,3-dihydro-1H-indol-1-yl]-2-oxoethyl]amino]propionic acid

(1) [4-Isopropenyl-3-(trifluoromethyl)phenyl]methanol

[F264]

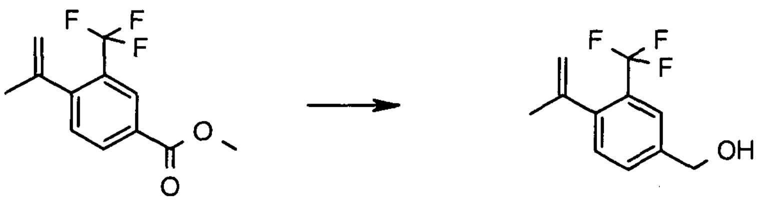

To a solution of 4-isopropenyl-3-(trifluoromethyl)benzoic acid methyl ester (878 mg) in THF (20 mL), diisobutylaluminum hydride (0.99M toluene solution) (9.10 mL) was added at 0°C. The reaction mixture was stirred at 0°C for 1 hour, and then saturated aqueous ammonium chloride solution (3.0 mL) was added thereto, followed by allowing to warm to room temperature. The reaction mixture was diluted with diethyl ether (100 mL) and stirred at room temperature for 1 hour. Magnesium sulfate anhydrate was added to the reaction mixture, and the mixture was stirred for 30 minutes, followed by removal of insoluble matter by filtration. The filtrate was concentrated under reduced pressure, and the residue was purified by silica gel flash column chromatography (Biotage 40M), whereby the title compound (738 mg) was yielded.
$^1$H-NMR ($CDCl_3$) δ :
7.65(1H,d,J=0.5Hz), 7.49(1H,dd,J=8.3,0.5Hz), 7.25(1H,d,J=8.3Hz) , 5.22 (1H,t, J=1.5Hz), 4.88 (1H, s), 4.75 (2H, d, J=5.9Hz), 2.07 (3H, s), 1.73(1H,t,J=5.9Hz).

(2) tert-Butyl[[4-isopropenyl-3-(trifluoromethyl)benzyl]oxy]diphenylsilane

[F265]

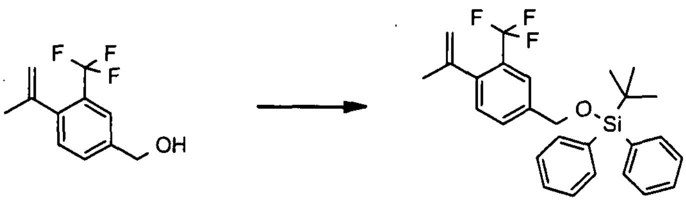

To a solution of [4-isopropenyl-3-(trifluoromethyl)phenyl]methanol (735 mg) in dichloromethane (10 mL), TEA (0.711 mL), DMAP (42.0 mg), and tert-butyldiphenylsilyl chloride (1.06 mL) were added at room temperature. The reaction mixture was stirred at room temperature for 2 days and concentrated under reduced pressure. The residue was purified by silica gel flash column chromatography (Biotage 40M), whereby the title compound (1.47 g) was yielded.
$^1$H-NMR ($CDCl_3$ ) δ :
7.65-7.70(4H,m), 7.58(1H,s), 7.35-7.47(7H,m), 7.20(1H,d,J=7.8Hz), 5.21(1H,t,J=1.6Hz), 4.88(1H,s),4 .78 (2H, s), 2.07 (3H, s), 1.10 (9H, s).

(3) tert-Butyl[[4-(1-methyl-1-cyclopropyl)-3-(trifluoromethyl)benzyl]oxy]diphenylsilane (Tetrahedron Lett. 1998, 39, 8621)

[F266]

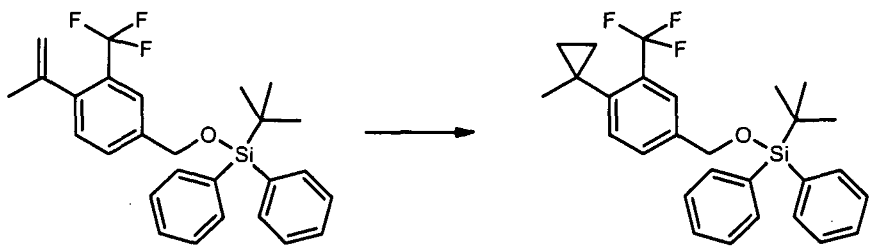

Dichloromethane (4.0 mL) was added to diethylzinc (1.0M hexane solution) (2.23 mL), and then a solution of TFA (172 μL) in dichloromethane (4.0 mL) was slowly added dropwise thereto at 0°C. The reaction mixture was stirred for 20 minutes, and then a solution of diiodomethane (180 μL) in dichloromethane (2.0 mL) was added dropwise thereto. The reaction mixture was stirred for 20 minutes, and then a solution of tert-butyl[(4-isopropenyl-3-(trifluoromethyl)benzyloxy]diphenylsilane (490 mg) in dichloromethane (4.0 mL) was added thereto. The reaction mixture was allowed to warm to room temperature, and stirred for 15 hours. Thereafter, dichloromethane (15 mL) and saturated aqueous ammonium chloride solution (20 mL) were added to the reaction mixture for phase separation, and the aqueous layer was extracted with ethyl acetate (20 mL). The extracts were combined and dried over sodium sulfate anhydrate. Thereafter, solvent was evaporated under reduced pressure, and the residue was purified by silica gel column flash chromatography (Biotage 25M), whereby the title compound (445 mg) was yielded.
$^1$H-NMR ($CDCl_3$ ) δ :
7.64-7.70(4H,m), 7.54(1H,s), 7.50(1H,d,J=8.1Hz), 7.34-7.46(7H,m),4.75(2H,s),1.35(3H,s),1.10(9H,s)0.87-0.93(2H,m), 0.74-0.78(2H,m).

(4) [4-(1-Methyl-1-cyclopropyl)-3-(trifluoromethyl)phenyl]methanol (Tetrahedron Lett. 1998, 39,8621)

[F267]

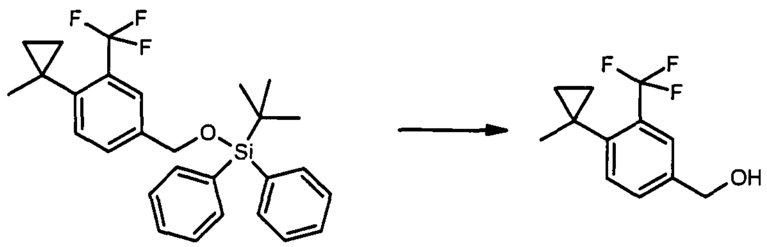

To a solution of tert-butyl[[4-(1-methyl-1-cyclopropyl)-3-(trifluoromethyl)benzyloxy]diphenylsilane (442 mg) in pyridine (4.0 mL), a hydrogen fluoride-pyridine mixture (0.500 mL) was added at 0°C, and then the mixture was stirred at room temperature for 16 hours. The reaction mixture was added to stirred ice water (30 mL), followed by extraction with ethyl acetate (2 x 20 mL). The extracts were combined and washed with saturated aqueous sodium hydrogencarbonate solution (20 mL), followed by drying over sodium sulfate anhydrate. Thereafter, solvent was evaporated under reduced pressure, and the residue was purified by silica gel flash column chromatography (Biotage 25M), whereby the title compound (175 mg) was yielded.
$^1$H-NMR ($CDCl_3$) δ:
7.60(1H,d,J=1.5Hz), 7.55(1H,d,J=7.9Hz), 7.46(1H,d,J=7.9Hz), 4.71 (2H,s), 1.69(1H,br s),1.35(3H,s),0.88-0.93(2H,m), 0.75-0.80(2H,m).
MS (ESI) m/z : 213 (M-OH)$^+$.

(5) 3-[N-(tert-Butoxycarbonyl)-N-[2-[5-[4-(1-methyl-1-cyclopropyl)-3-(trifluoromethyl)benzyloxy]indolin-1-yl]-2-oxoethyl] amino]propionic acid ethyl ester

[F268]

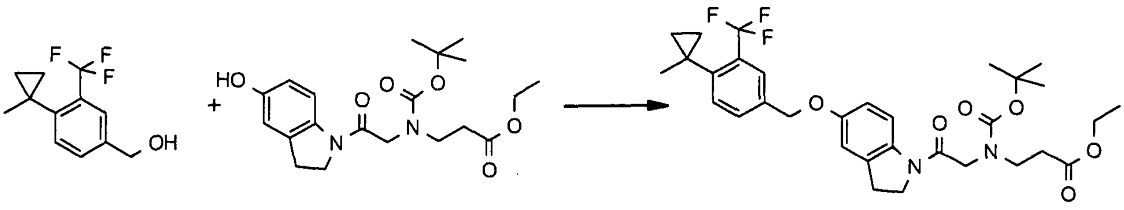

To a solution of [4-(1-methyl-1-cyclopropyl)-3-(trifluoromethyl)phenyl]methanol (172 mg) in THF (8.0 mL), 3-[N-(tert-butoxycarbonyl)-N-[2-(5-hydroxy-2,3-dihydro-1H-indol-1-yl)-2-oxoethyl]amino]propionic acid ethyl ester (323 mg), triphenylphosphine (235 mg), and DEAD (2.2M toluene solution) (0.407 mL) were added at room temperature. The reaction mixture was stirred at room temperature for 17 hours and concentrated under reduced pressure. The residue was purified by silica gel column flash chromatography (Biotage 25M), whereby the title compound (488 mg) was yielded.
$^1$H-NMR ($CDCl_3$) δ :
7.35-8.16(4H,m),6.73-6.85(2H,m),5.01(2H,s),3.97-4.30(6H,m), 3.58-3.68(2H,m), 3.13-3.23(2H,m), 2.63-2.73(2H,m), 1.20-1.60(15H,m), 0.88-0.94(2H,m), 0.75-0.81(2H,m).
MS (ESI) m/z : 605 $(M+H)^+$.

(6) 3-[N-[2-[5-[4-(1-Methyl-1-cyclopropyl)-3-(trifluoromethyl)benzyloxy]-2,3-dihydro-1H-indol-1-yl]-2-oxoethyl] amino] propionic acid

[F269]

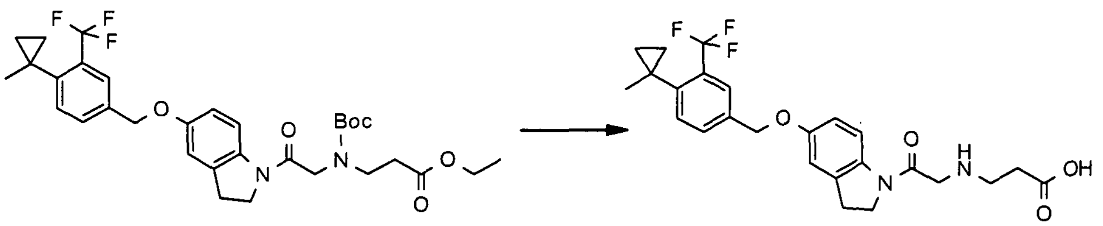

To a THF/methanol mixture solution (4 mL/2 mL) of 3-[N-(tert-butoxycarbonyl)-N-[2-[5-[4-(1-methyl-1-cyclopropyl)-3-(trifluoromethyl)benzyloxy]indolin-1-yl]-2-oxoethyl]amino]propionic acid ethyl ester (0.49 g), 1N aqueous sodium hydroxide solution (2 mL) was added at room temperature, and the mixture was stirred for 2 days. The reaction mixture was concentrated, and 1N hydrochloric acid was added thereto, followed by extraction with chloroform/methanol (10/1, v/v) mixture. The extracts were combined and washed with saturated brine, followed by drying over sodium sulfate anhydrate. Solvent was evaporated, whereby a crude product of 3-[N-(tert-butoxycarbonyl)-N-[2-[5-[4-(1-methyl-1-cyclopropyl)-3-(trifluoromethyl)benzyloxy]indolin-1-yl]-2-oxoethyl]amino]propionic acid was yielded. This crude product was employed in the subsequent reaction without further purification.
To a dichloromethane solution (10 mL) of the above-yielded 3-[N-(tert-butoxycarbonyl)-N-[2-[5-[4- (1-methyl-1-cyclopropyl)-3-(trifluoromethyl)benzyloxy]indolin-1-yl]-2-oxoethyl]amino]propionic acid, TFA (1 mL) was added, and the mixture was stirred for 3 hours. The reaction mixture was concentrated, and saturated aqueous sodium bicarbonate solution was added to the residue, followed by extraction with chloroform/methanol (10:1, v/v) mixture. The extracts were combined and dried over sodium sulfate anhydrate. Solvent was evaporated, whereby the title compound (0.48 g) was yielded.
$^1$H-NMR ($CD_3OD$) δ:
0.66-1.01(4H,m),1.33-1.34 (3H, m), 2.41(2H, t, J=7.1Hz), 2.88 (2H, t, J=7.2Hz), 3.17 (1H, d, J= 8.0Hz), 3.34(2H,d, J=0.7Hz), 3.53(1H,s), 4.07(2H,t,J=8.2Hz), 5.07( 2H,s), 6.84-6.67(1H,m), 6.90(1H,s), 7.49-7.70(3H,m), 8.04(1H,d, J=9.0Hz).
IR(ATR)cm$^{-1}$:2925,2854,1720,1655,1556,1491,1419,1375.
MS (ESI) m/z : 477 (M+H)$^+$.

[Example 62]

3-[N-[2-[5-[[1-Isopropyl-3-(trifluoromethyl)-1H-pyrazol-4-yl]methoxy]-2,3-dihydro-1H-indol-1-yl]-2-oxoethyl]amino]propionic acid

(1) 1-Isopropyl-3-(trifluoromethyl)-1H-pyrazole-4-carboxylic acid ethyl ester

[F270]

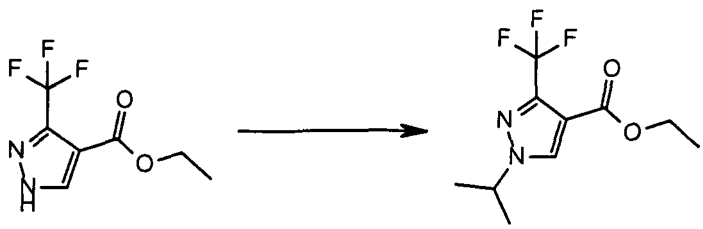

To a DMF solution (15 mL) of 3-(trifluoromethyl)pyrazole-4-carboxylic acid ethyl ester (WO 93/25535; 620 mg), sodium hydride (190 mg) was added, and the mixture was stirred at 80°C for 1.1 hours. The reaction mixture was left to stand to cool to room temperature, and then isopropyl iodide (440 μL) was added thereto, followed by stirring at 80°C for 1 day. The reaction mixture was left to stand to cool to room temperature, and water was added thereto, followed by extraction with a 75% ethyl acetate/hexane mixture. The extracts were combined and washed with saturated brine, followed by drying over magnesium sulfate anhydrate. Insoluble matter was removed by filtration. Thereafter, the filtrate was concentrated, and the residue was purified by silica gel flash column chromatography, whereby the title compound (620 mg) was yielded.
$^1$H-NMR ($CDCl_3$) δ :
1.35 (3H, t, J=7.2Hz), 1.53 (3H, s), 1.55 (3H, s), 4.31 (2H, q, J=7.2Hz), 4 .50-4.60(1H,m), 8.01(1H,s).
MS (ESI)m/z:251 (M+H)$^+$.

(2) [1-Isopropyl-3-(trifluoromethyl)-1H-pyrazol-4-yl]methanol

[F271]

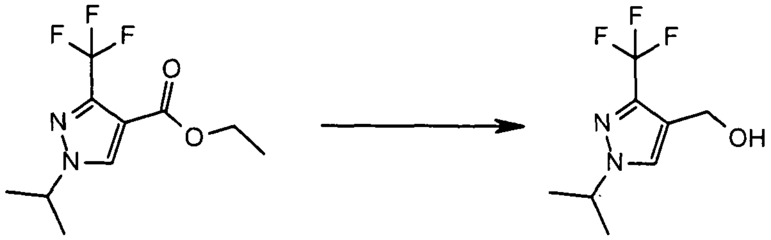

To a THF solution (16 mL) of 1-isopropyl-3-(trifluoromethyl)-1H-pyrazole-4-carboxylic acid ethyl ester (620 mg), lithium aluminum hydride (112 mg) was added, and the mixture was refluxed under stirring for 40 minutes. The reaction mixture was left to stand to cool to room temperature, and then water (110 μL), 1N aqueous sodium hydroxide solution (110 μL), and water (330 μL) were sequentially added thereto, followed by drying over magnesium sulfate anhydrate. Insoluble matter was removed by filtration, and the filtrate was concentrated, whereby the title compound (508 mg) was yielded.
$^1$H-NMR ($CDCl_3$) δ:
1.51(3H,s), 1.52(3H,s), 4.47-4.57(1H,m), 4.67(2H,s), 7.52(1H,s).
MS (ESI) m/z :209 $(M+H)^+$.

(3) 3-[N-(tert-Butoxycarbonyl)-N-[2-oxo-2-[5-[[1-isopropyl-3-(trifluoromethyl)-1H-pyrazol-4-yl]methoxy]-2,3-dihydro-1H-indol-1-yl]ethyl]amino]propionic acid tert-butyl ester

[F272]

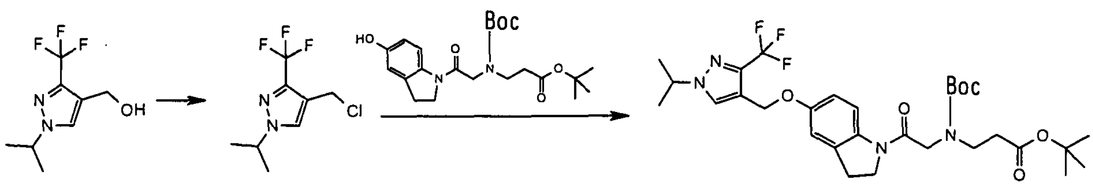

To a dichloromethane solution (10 mL) of [1-isopropyl-3-(trifluoromethyl)-1H-pyrazol-4-yl]methanol (200 mg), thionyl chloride (210 μL) was added, and the mixture was stirred at 50°C overnight. The reaction mixture was left to stand to cool to room temperature and concentrated. The residue was dissolved in DMF (20 mL), and potassium carbonate (398 mg) and 3-[N-(tert-butoxycarbonyl)-N-[2-oxo-2-(5-hydroxy-2,3-dihydro-1H-indol-1-yl)ethyl]amino]propionic acid tert-butyl ester (606 mg) was added to the solution, followed by stirring at room temperature for 1 day. The reaction mixture was diluted with ethyl acetate and water, and then the aqueous layer was extracted with ethyl acetate. The extracts were combined and washed with saturated brine, followed by drying over magnesium sulfate anhydrate. Insoluble matter was removed by filtration. The filtrate was concentrated, and the residue was purified by silica gel column flash chromatography, whereby the title compound (463 mg) was yielded.
$^1$H-NMR ($CDCl_3$) δ :
1.40(3H,s), 1.41(3H,s), 1.43(6H,s), 1.49(6H,s), 1.52(6H,d,J=6.8Hz ),2.57-2.61(2H,m),3.19(2H,q,J=8.7Hz),3.58(2H,m), 4.01-4.07(2H,m), 4.18(2H,s), 4.52(1H,m), 4.98(2H,s), 6.76-6.80(2H,m), 7.55(1H,m), 8.13(1H,m).
MS (ESI) m/z : 611 $(M+H)^+$.

(4) 3-[N-[2-[5-[[1-Isopropyl-3-(trifluoromethyl)-1H-pyrazol-4-yl]methoxy]-2,3-dihydro-1H-indol-1-yl]-2-oxoethyl]amino] propionic acid

[F273]

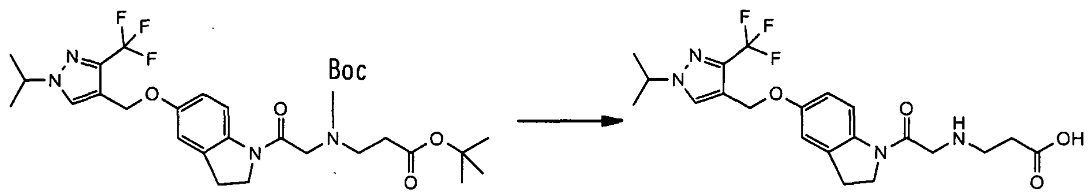

3-[N-(tert-Butoxycarbonyl)-N-[2-oxo-2-[5-[[1-isopropyl-3-(trifluoromethyl)-1H-pyrazol-4-yl]methoxy]-2,3-dihydro-1H-indol-1-yl]ethyl]amino]propionic acid tert-butyl ester (448 mg) was dissolved in a 20% TFA/dichloromethane mixture (16 mL), and the solution was stirred at room temperature for 5 hours. Solvent was evaporated from the reaction mixture, and then the residue was purified by reverse phase preparative HPLC, whereby the title compound (101 mg) was yielded.
$^1$H-NMR ($CD_3OD$) δ:
1.49(3H,s),1.51(3H,s),2.64-2.67(4H,m), 3.24(2H,t,J=7.8Hz), 4.06-4.11(4H,m), 4.53-4.60(1H,m), 4.98(2H,s), 6.81(1H,d, J=8.8Hz), 6.91(1H,s), 7.89(1H,s ), 8.04(1H,d,J=8.8Hz).
IR (ATR) $cm^{-1}$:1641,1493,1363,1325,1286,1267,1172,1153,1122,1068,1012,845, 798.
MS (ESI) m/z : 455 $(M+H)^+$.
Anal. Calcd for $C_{21}H_{25}F_3N_4O_4$·0.1$CF_3COOH$· $H_2O$:C,52.62;H,5.65;F,12.96;N,11.58.Found:C,52.56;H,5.45;F,12. 62;N, 11.15.

[Example 63]

3-[N-[2-Oxo-2-[5-[[1-cyclohexyl-3-(trifluoromethyl)-1H-pyrazol-4-yl]methoxy]-2,3-dihydro-1H-indol-1-yl] ethyl] amino] propionic acid

(1) 1-Cyclohexyl-3-(trifluoromethyl)-1H-pyrazole-4-carboxylic acid ethyl ester

[F274]

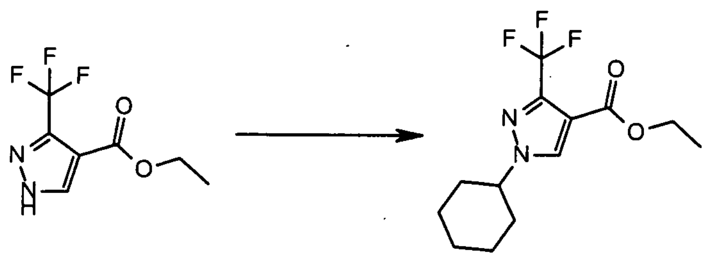

To a DMF solution (15 mL) of 3-(trifluoromethyl)pyrazole-4-carboxylic acid ethyl ester (620 mg), sodium hydride (195 mg) was added, and the mixture was stirred at 80°C for 1 hour. The reaction mixture was left to stand to cool to room temperature, and then cyclohexyl bromide (548 μL) was added thereto, followed by stirring at 80°C for 1 day. The reaction mixture was left to stand to cool to room temperature, and water was added thereto, followed by extraction with 75% ethyl acetate/hexane solution. The extracts were combined and washed with saturated brine, followed by drying over magnesium sulfate anhydrate, filtration, and concentration. The residue was purified by silica gel column flash

chromatography, whereby the title compound (150 mg) was yielded.
$^1$H-NMR ($CDCl_3$) δ :
1.23-1.28(2H,m), 1.35(3H,t,J=7.2Hz), 1.42-1.47(2H,m), 1.64-1.77(2H,m),1.90-1.94(2H,m),2.17-2.21(2H,m), 4.16(1H, m), 4.31(2H,q,J=7.2Hz), 8.00(1H,s).
MS (ESI) m/z : 291 $(M+H)^+$.

(2) [1-Cyclohexyl-3-(trifluoromethyl)-1H-pyrazol-4-yl]methanol

[F275]

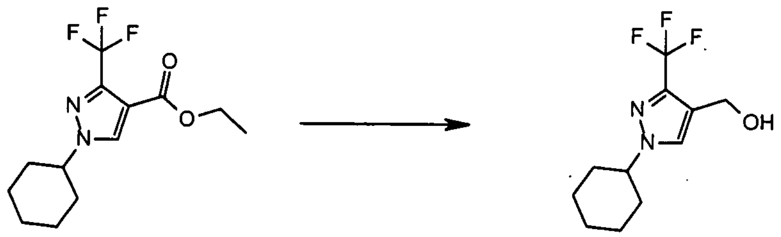

To a THF solution (5.0 mL) of 1-cyclohexyl-3-(trifluoromethyl)-1H-pyrazole-4-carboxylic acid ethyl ester (150 mg), lithium aluminum hydride (27.2 mg) was added, and the mixture was refluxed with stirring for 35 minutes. The reaction mixture was left to stand to cool to room temperature, and then water (110 μL), 1N aqueous sodium hydroxide solution (110 μL), and water (330 μL) were sequentially added thereto, followed by drying over magnesium sulfate anhydrate. Thereafter, insoluble matter was removed by filtration, and the filtrate was concentrated, whereby the title compound (127 mg) was yielded.
$^1$H-NMR ($CDCl_3$) δ :
1.20-1.30 (2H,m), 1.40-1.43 (2H,m),1.65-1.7 (2H,m), 1.89-1.92(2H,m),2.15-2.17(2H,m),4.10-4.16(1H,m), 4.67(2H,s), 7.51(1H,s).
MS (ESI) m/z : 249 $(M+H)^+$.

(3) 5-[[1-Cyclohexyl-3-(trifluoromethyl)-1H-pyrazol-4-yl]methoxy]indoline-1-carboxylic acid tert-butyl ester

[F276]

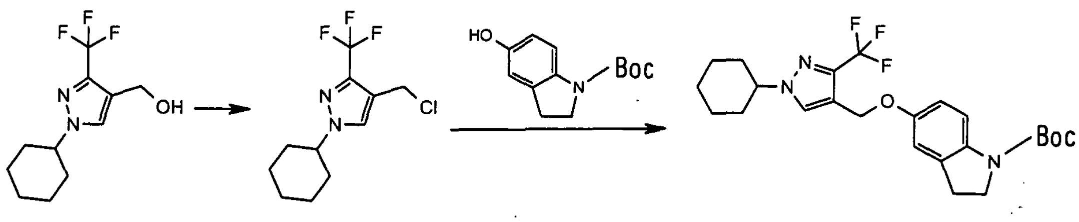

To a dichloromethane solution (10 mL) of [1-cyclohexyl-3-(trifluoromethyl)-1H-pyrazol-4-yl]methanol (125 mg), thionyl chloride (110 μL) was added, and the mixture was stirred at 50°C overnight. The reaction mixture was left to stand to cool to room temperature, and then solvent was evaporated. The residue was dissolved in DMF (20 mL), and potassium carbonate (209 mg) and 5-hydroxyindoline-1-carboxylic acid tert-butyl ester (318 mg) were added to the solution, followed by stirring at 70°C for 1 day. The reaction mixture was diluted with ethyl acetate and water, and then the aqueous layer was extracted with ethyl acetate. The extracts were combined and washed with saturated brine, followed by drying with magnesium sulfate anhydrate, filtration, and concentration. The residue was purified by silica gel column flash chromatography, whereby the title compound (177 mg) was yielded.
$^1$H-NMR ($CDCl_3$) δ:
1.23-1.28(2H,m), 1.39-1.43(2H,m), 1.58(9H,s), 1.65-1.75(2H,m),1.89-1.92(2H,m),2.16-2.17(2H,m), 3.06(2H,t,J=8.7Hz),

3.97(2H,br s),4.12-4.15(1H,m), 4.97(2H,s), 6.75-6.77(2H,m), 7.26(1H,s), 7.54(1H,s).
MS (ESI) m/z : 466 $(M+H)^+$.

(4) 5-[[1-Cyclohexyl-3-(trifluoromethyl)-1H-pyrazol-4-yl] methoxy] indoline

[F277]

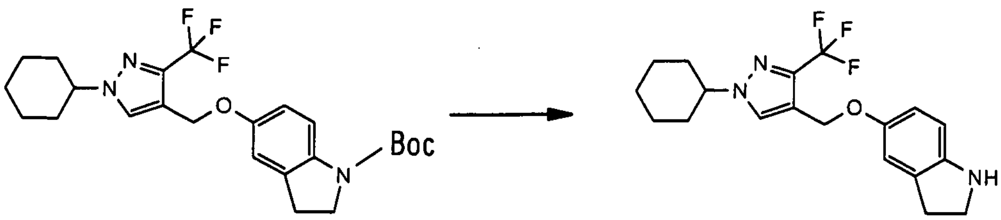

5-[[1-Cyclohexyl-3-(trifluoromethyl)-1H-pyrazol-4-yl]methoxy]indoline-1-carboxylic acid tert-butyl ester (165 mg) was dissolved in 4N HCl/dioxane (4.0 mL), and the solution was stirred at room temperature for 2.4 hours. Solvent was evaporated from the reaction mixture, and then the residue was diluted with saturated aqueous sodium hydrogencarbonate solution. The aqueous layer was extracted with chloroform, and the extracts were combined. The combined extract was washed with saturated brine, and dried over magnesium sulfate anhydrate, followed by filtration and concentration, whereby the title compound (50 mg) was yielded.
MS (ESI) m/z : 366 $(M+H)^+$.

(5) 3-[N-(tert-Butoxycarbonyl)-N-[2-oxo-2-[[5-[[1-cyclohexyl-3-(trifluoromethyl)-1H-pyrazol-4-yl]methoxy]-2,3-dihydro-1H-indol-1-yl]ethyl]amino]propionic acid tert-butyl ester

[F278]

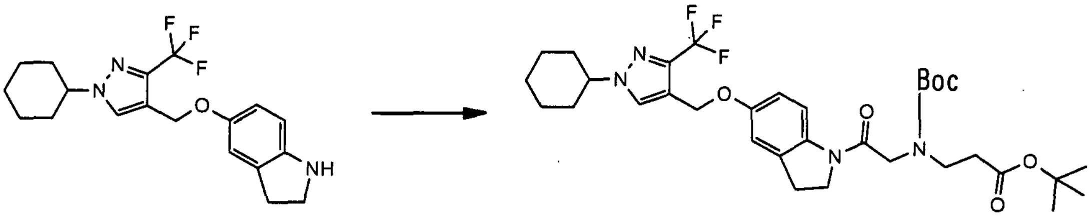

To a DMF solution (2.0 mL) of 5-[[1-cyclohexyl-3-(trifluoromethyl)-1H-pyrazol-4-yl]methoxy]indoline (45 mg), 3-[N-(tert-butoxycarbonyl)-N-[2-oxo-2-(5-hydroxy-2,3-dihydro-1H-indol-1-yl)ethyl]amino]propionic acid tert-butyl ester (45 mg), EDC·HCl (28 mg), HOBt (20 mg), and DIEA (63 μL) were added, and the mixture was stirred at room temperature overnight. Saturated aqueous sodium hydrogencarbonate solution was added to the reaction mixture, and the aqueous layer was extracted with ethyl acetate. The extracts were combined and washed with saturated brine, followed by drying over magnesium sulfate anhydrate, filtration, and concentration. The residue was purified by silica gel column flash chromatography, whereby the title compound (60 mg) was yielded.
$^1$H-NMR ($CDCl_3$) δ:
1.25-1.28(2H,m), 1.40-1.42(12H,m), 1.49(6H,s), 1.66-1.72(4H,m),1.88-1.91(2H,m),2.14-2.17(2H,m),2.59(2H,m), 3.17-3.19(2H,m),3.56-3.58(2H,m),3.98-4.06(2H,m),4.09-4.17(3H,m), 4.98(2H,s), 6.75-6.79(2H,m), 7.54(1H,d, J=8.3Hz), 8.11-8.13(1H,m).
MS (ESI) m/z : 651 $(M+H)^+$.

(6) 3-[N-[2-Oxo-2-[5-[[1-cyclohexyl-3-(trifluoromethyl) -1H-pyrazol-4-yl]methoxy]-2,3-dihydro-1H-indol-1-yl]ethyl]amino] propionic acid

[F279]

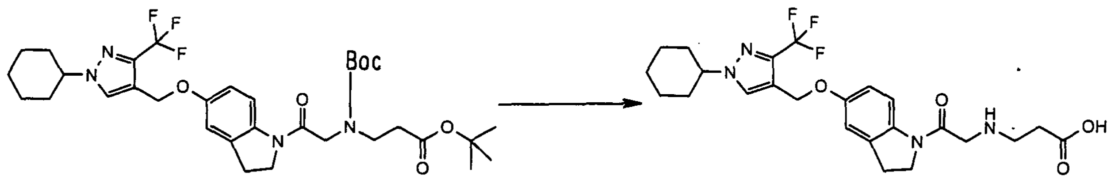

3-[N-(tert-Butoxycarbonyl)-N-[2-oxo-2-[5-[[1-cyclohexyl-3-(trifluoromethyl)-1H-pyrazol-4-yl]methoxy]-2,3-dihydro-1H-indol-1-yl]ethyl]amino]propionic acid tert-butyl ester (60 mg) was dissolved in a 20% TFA/dichloromethane mixture (2.0 mL), and the solution was stirred at room temperature for 6 hours. Solvent was evaporated from the reaction mixture, and then the residue was purified by reverse phase preparative HPLC, whereby the title compound (22 mg) was yielded. $^1$H-NMR ($CD_3OD$) δ :
1.30-1.33(2H,m), 1.46-1.48(2H,m), 1.73-1.82(2H,m), 1.90-1.93(2H,m),2.09-2.12(2H,m),2.56-2.57(2H,m),4.08-4.10(2H, m),4.16-4.20(2H,m),4.98(3H,m),6.80-6.81(1H,m), 6.90(1H,s), 7.88(1H,s), 8.04(1H,d,J=8.8Hz). (4H peaks were not observed due to overlapping with the $CD_3OD$ peak.)
MS (ESI)m/z:495 $(M+H)^+$.

[Example 64]

3-[N-[2-Oxo-2-[[5-[[1-cyclopentyl-3-(trifluoromethyl)-1H-pyrazol-4-yl]methoxy]-2,3-dihydro-1H-indol-1-yl] ethyl] amino] propionic acid

(1) 1-Cyclopentyl-3-(trifluoromethyl)-1H-pyrazole-4-carboxylic acid ethyl ester

[F280]

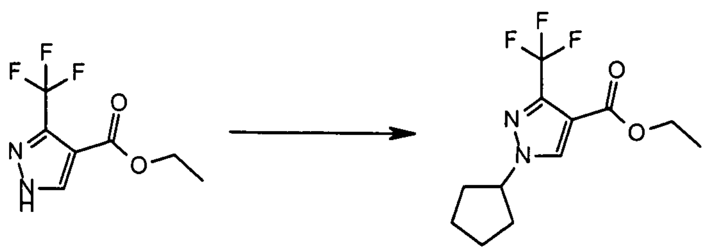

To a DMF solution (15 mL) of 3-(trifluoromethyl)pyrazole-4-carboxylic acid ethyl ester (620 mg), sodium hydride (190 mg) was added, and the mixture was stirred at 80°C for 1.5 hours. The reaction mixture was left to stand to cool to room temperature, and then cyclopentyl iodide (480 μL) was added thereto, followed by stirring at 80°C for 21 hours. The reaction mixture was left to stand to cool to room temperature, and water was added thereto, followed by extraction with a 75% ethyl acetate/hexane mixture. The extracts were combined and washed with saturated brine, followed by drying over magnesium sulfate anhydrate, filtration, and concentration. The residue was purified by silica gel flash column

chromatography, whereby the title compound (857 mg) was yielded.
MS (ESI) m/z : 277 $(M+H)^+$.

(2) [1-Cyclopentyl-3-(trifluoromethyl)-1H-pyrazol-4-yl] methanol

[F281]

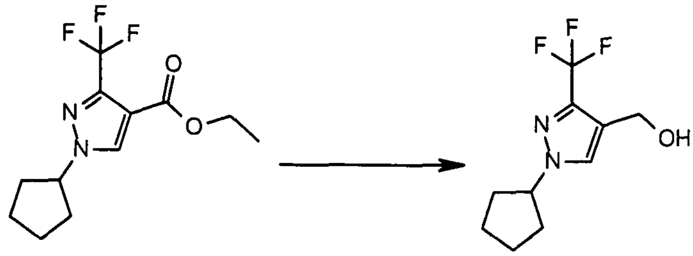

To a THF solution (20 mL) of 1-cyclopentyl-3-(trifluoromethyl)-1H-pyrazole-4-carboxylic acid ethyl ester (850 mg), lithium aluminum hydride (140 mg) was added, and the mixture was refluxed with stirring for 1 hour. The reaction mixture was left to stand to cool to room temperature, and then water (110 μL), 1N aqueous sodium hydroxide solution (110 μL), and water (330 μL) were sequentially added thereto. The reaction mixture was dried over magnesium sulfate anhydrate, followed by filtration. The filtrate was concentrated, whereby the title compound (709 mg) was yielded.
$^1$H-NMR ($CDCl_3$) δ :
0.85-0.88(H,m),1.66-1.72(2H,m),1.81-1.90(2H,m),1.99(2H,m),2.13-2.19(2H,m),4.65-4.66 (3H,m), 7.50 (1H, s) .
MS (ESI) m/z : 235 $(M+H)^+$.

(3) 3-[N-(tert-Butoxycarbonyl)-N-[2-oxo-2-[5-[[1-cyclopentyl-3-(trifluoromethyl)-1H-pyrazol-4-yl]methoxy]-2,3-dihydro-1H-indol-1-yl]ethyl]amino]propionic acid tert-butyl ester

[F282]

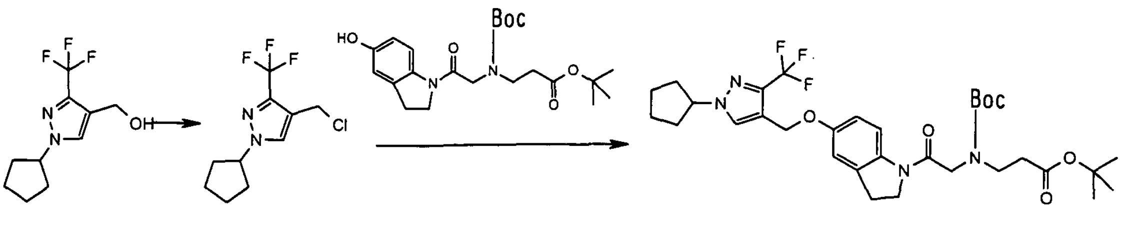

To a dichloromethane solution (10 mL) of [1-cyclopentyl-3-(trifluoromethyl)-1H-pyrazol-4-yl]methanol (200 mg), thionyl chloride (312 μL) was added, and the mixture was stirred at 50°C overnight. The reaction mixture was left to stand to cool to room temperature, and then solvent was evaporated. The residue was dissolved in DMF (20 mL), and potassium carbonate (590 mg) and 3-[N-(tert-butoxycarbonyl)-N-[2-oxo-2-(5-hydroxy-2,3-dihydro-1H-indol-1-yl)ethyl] amino]propionic acid tert-butyl ester (395 mg) were added to the solution, followed by stirring at room temperature. The reaction mixture was diluted with ethyl acetate and water, and then the aqueous layer was extracted with ethyl acetate. The extracts were combined and washed with saturated brine, followed by drying over magnesium sulfate anhydrate,

filtration, and concentration. The residue was purified by silica gel column flash chromatography, whereby the title compound (407 mg) was yielded.
$^1$H-NMR ($CDCl_3$) δ:
1.41-1.43(12H,m),1.50(6H,s),1.70-1.76(2H,m),1.85-1.90(2H,m),1.97-2.03(2H,m),2.14-2.20(2H,m),2.55-2.63(2H,m), 3.18-3.20(2H,m),3.57-3.59(2H,m),3.99-4.06(2H,m), 4.09(1H,s), 4.18(1H,s), 4.62-4.69(1H,m), 8.14(1H,m).
MS (ESI) m/z : 637 $(M+H)^+$.

(4) 3-[N-[2-Oxo-2-[5-[[1-cyclopentyl-3-(trifluoromethyl)-1H-pyrazol-4-yl]methoxy]-2,3-dihydro-1H-indol-1-yl] ethyl] amino] propionic acid

[F283]

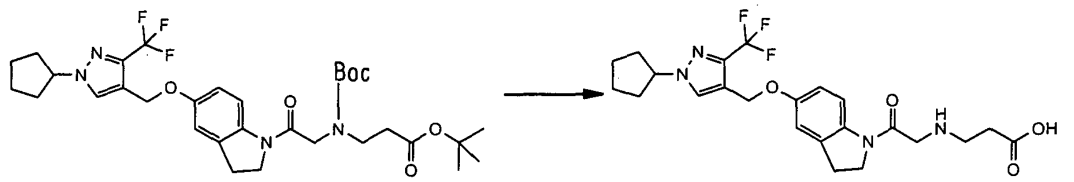

3-[N-(tert-Butoxycarbonyl)-N-[2-oxo-2-[[5-[[1-cyclopentyl-3-(trifluoromethyl)-1H-pyrazol-4-yl]methoxy]-2,3-dihydro-1H-indol-1-yl]ethyl]amino]propionic acid tert-butyl ester (407 mg) was dissolved in 10% TFA/dichloromethane solution (15 mL), and the solution was stirred at room temperature for 1 day. TFA (5.0 mL) was further added to the reaction mixture, and the mixture was stirred at room temperature for 1.5 hours. Solvent was evaporated from the reaction mixture, and then the residue was purified by reverse phase preparative HPLC, whereby the title compound (133 mg) was yielded.
$^1$H-NMR ($CD_3OD$) δ :
1.72-1.75(2H,m),1.87-1.90(2H,m),1.99-2.01(2H,m),2.16-2.18(2H,m), 2.66-2.68(2H,m), 3.23-3.26(2H,m), 4.08-4.12 (4H,m), 4.73-4.77 (1H,m), 4.98 (2H, s), 6.81-6.83(1H,m), 6.91(1H,s), 7.87(1H,s), 8.04-8.06(1H,m). (2H peaks were not observed due to overlapping with the $CD_3OD$ peak.)
IR (ATR) $cm^-$
$^1$:1643,1493,1363,1286,1269,1167,1122,1070,1018,845,800.
MS (ESI) m/z : 481 $(M+H)^+$.
Anal. Calcd for $C_{23}H_{27}F_3N_4O_4 \cdot 0.1CF_3COOH \cdot H_2O$:C,54.65;H,5.75;F,12.30;N,10.99.Found:C,54.54;H,5.45;F,12. 39;N, 10.97.

[Example 65]

3-[N-[2-Oxo-2-[5-[[1-phenyl-5-(trifluoromethyl)-1H-pyrazol-3-yl]methoxy]-2,3-dihydro-1H-indol-1-yl]ethyl]amino]propionic acid

(1) 3-[N-(tert-Butoxycarbonyl)-N-[2-Oxo-2-[5-[[1-phenyl-5-(trifluoromethyl)-1H-pyrazol-3-yl]methoxy]-2,3-dihydro-1H-indol-1-yl]ethyl]amino]propionic acid tert-butyl ester

[F284]

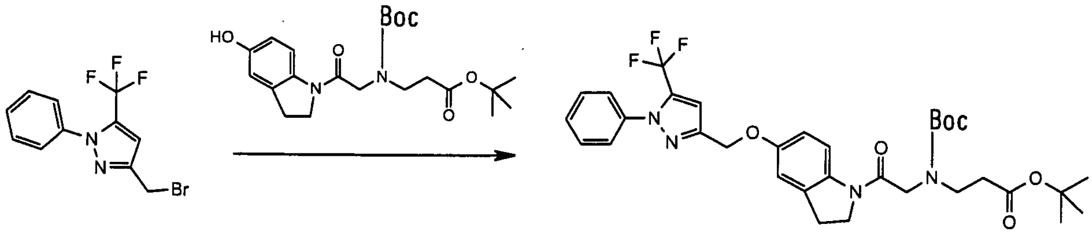

3-(Bromomethyl)-1-phenyl-5-(trifluoromethyl)-1H-pyrazole (200 mg) was dissolved in DMF (20 mL), and potassium carbonate (668 mg) and 3-[N-(tert-butoxycarbonyl)-N-[2-oxo-2-(5-hydroxy-2,3-dihydro-1H-indol-1-yl)ethyl]amino]propionic acid tert-butyl ester (373 mg) were added to the solution, followed by stirring at room temperature. Subsequently, the reaction mixture was heated to 70°C, and stirred for 1 day. The reaction mixture was left to stand to cool to room temperature, and diluted with ethyl acetate and water. Thereafter, the aqueous layer was extracted with ethyl acetate, and the extracts were combined. The combined extract was washed with saturated brine, and dried over magnesium sulfate anhydrate, followed by filtration and concentration. The residue was purified by silica gel column flash chromatography, whereby the title compound (184 mg) was yielded.
$^1$H-NMR ($CDCl_3$) δ:
1.41(6H,s),1.43(6H,s),1.50(6H,s),2.56-2.63(2H,m),3.17-3.20(2H,m),3.58-3.60(2H,m),4.03-4.15(2H,m),4.18(2H,s),5.12(2H,s),6.82-6.84(2H,m), 6.91(1H,s), 7.49(5H,s), 8.12-8.15(1H,m).
MS (ESI) m/z : 645 $(M+H)^+$.

(2) 3-[N-[2-Oxo-2-[[5-[[1-phenyl-5-(trifluoromethyl)-1H-pyrazol-3-yl]methoxy]-2,3-dihydro-1H-indol-1-yl] ethyl] amino] propionic acid

[F285]

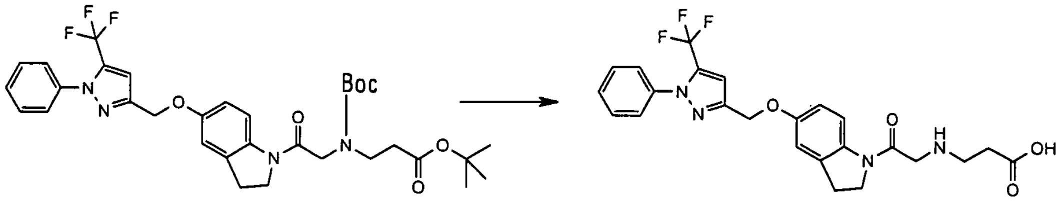

3-[N-(tert-Butoxycarbonyl)-N-[2-oxo-2-[5-[[1-phenyl-5-(trifluoromethyl)-1H-pyrazol-3-yl]methoxy]-2,3-dihydro-1H-indol-1-yl]ethyl]amino]propionic acid tert-butyl ester (170 mg) was dissolved in 20% TFA/dichloromethane solution (10 mL), and the solution was stirred at room temperature for 2.5 hours. Solvent was evaporated from the reaction mixture, and then the residue was purified by reverse phase preparative HPLC, whereby the title compound (98 mg) was yielded.
$^1$H-NMR ($CD_3OD$) δ :
2.57(2H,t,J=6.1Hz), 3.23-3.26(4H,m), 4.07-4.09(4H,m), 5.13(2H,s), 6.87-6.89(1H,m), 6.97-6.99(1H,m), 7.03-7.05(1H, m), 7.50-7.55(5H,m), 8.05(1H, d, J=8.8Hz).
IR (ATR) $cm^{-1}$:1653,1597,1489,1348,1290,1265,1232,1184,1126,1107,1082,1039 ,987,812.
MS (ESI) m/z : 489 $(M+H)^+$.
Anal. Calcd for $C_{24}H_{23}F_3N_4O_4 \cdot 0.1CF_3COOH \cdot H_2O$:C,56.13;H,4.89;F,12.11;N,10.82.Found:C,56.08;H,4.72;F,12. 02;N,10.85.

[Example 66]

3-[N-[2-Oxo-2-[5-[[1-phenyl-5-(trifluoromethyl)-1H-pyrazol-4-yl]methoxy]-2,3-dihydro-1H-indol-1-yl]ethyl]amino]propionic acid

(1) [1-Phenyl-5-(trifluoromethyl)-1H-pyrazol-4-yl]methanol

[F286]

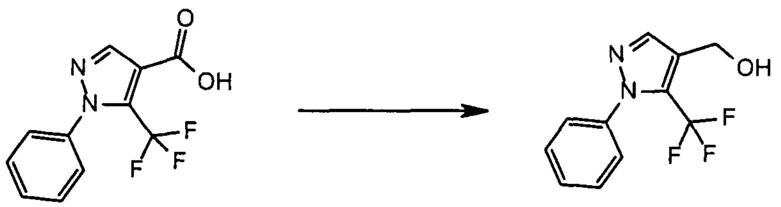

To a THF solution (16 mL) of 1-phenyl-5-(trifluoromethyl)-1H-pyrazole-4-carboxylic acid (GB 2149402, 500 mg), lithium aluminum hydride (89 mg) was added, and the mixture was refluxed with stirring for 2.3 hours. The reaction mixture was left to stand to cool to room temperature, and then water (110 μL), 1N aqueous sodium hydroxide solution (110 μL), and water (330 μL) were sequentially added thereto, followed by drying over magnesium sulfate anhydrate. Thereafter, insoluble matter was removed by filtration, and the filtrate was concentrated, whereby the title compound (399 mg) was yielded.
$^1$H-NMR ($CDCl_3$) δ :
1.84-1.86(1H,m), 4.78(2H,d,J=4.6Hz), 7.44-7.48(5H,m), 7.77(1H,s).
MS (ESI) m/z : 243 $(M+H)^+$.

(2) 3-[N-(tert-Butoxycarbonyl)-N-[2-oxo-2-[5-[[1-phenyl-5-(trifluoromethyl)-1H-pyrazol-4-yl]methoxy]-2,3-dihydro-1H-indol-1-yl]ethyl]amino]propionic acid tert-butyl ester

[F287]

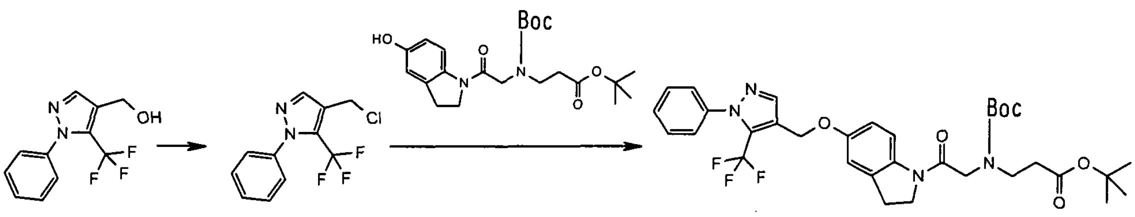

To a dichloromethane solution (5.0 mL) of [1-phenyl-5-(trifluoromethyl)-1H-pyrazol-4-yl]methanol (150 mg), thionyl chloride (226 μL) and DMF (catalytic amount) were added, and the mixture was stirred at 50°C for 2 hours. The reaction mixture was left to stand to cool to room temperature and concentrated. The residue was dissolved in DMF (5.0 mL), and potassium carbonate (514 mg) and 3-[N-(tert-butoxycarbonyl)-N-[2-(5-hydroxy-2,3-dihydro-1H-indol-1-yl)-2-oxoethyl]amino]propionic acid tert-butyl ester (286 mg) were added to the solution, followed by stirring at 70°C overnight. The reaction mixture was left to stand to cool to room temperature, and diluted with ethyl acetate and water. Thereafter, the aqueous layer was extracted with ethyl acetate, and the extracts were combined. The combined extract was washed with saturated brine, and dried over magnesium sulfate anhydrate, followed by filtration. Thereafter, the filtrate was concentrated, and the residue was purified by silica gel column flash chromatography, whereby the title compound (272 mg) was yielded.
$^1$H-NMR ($CDCl_3$) δ :

1.41(6H,s), 1.45(6H,d,J=8.5Hz), 1.50(6H,s), 2.56-2.63(2H,m),3.18-3.20(2H,m),3.58-3.59(2H,m),4.05-4.14 (4H,m), 5.08 (2H, s), 6.79-6.84 (2H,m), 7.48-7.49(5H,m), 7.81(1H,s), 8.14-8.16(1H,m).
MS (ESI)m/z:645 $(M+H)^+$.

(3) 3-[N-[2-Oxo-2-[5-[[1-phenyl-5-(trifluoromethyl)-1H-pyrazol-4-yl]methoxy]-2,3-dihydro-1H-indol-1-yl] ethyl] amino] propionic acid

[F288]

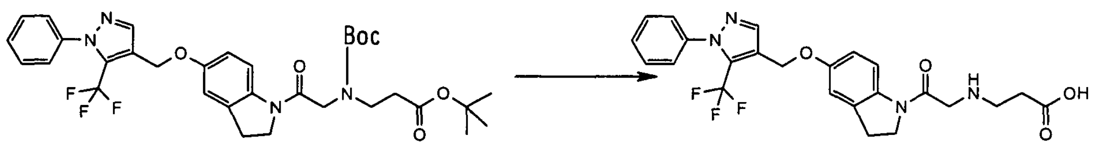

3-[N-(tert-Butoxycarbonyl)-N-[2-oxo-2-[5-[[1-phenyl-5-(trifluoromethyl)-1H-pyrazol-4-yl]methoxy]-2,3-dihydro-1H-indol-1-yl]ethyl]amino]propionic acid tert-butyl ester (170 mg) was dissolved in 20% TFA/dichloromethane solution (10 mL), and the solution was stirred at room temperature for 2.5 hours. Solvent was evaporated from the reaction mixture, and then the residue was purified by reverse phase preparative HPLC, whereby the title compound (78 mg) was yielded.
$^1$H-NMR (DMSO-$d_6$) δ:
2.35-2.36(2H,m),2.82-2.84(2H,m),3.13-3.15(4H,m), 4.04(2H,t,J=8.5Hz), 5.09(2H,s), 6.84-6.86(1H,m), 6.98(1H,s), 7.50-7.51(2H,m), 7.57-7.58(3H,m), 7.99-8.01 (2H, m) .
IR (ATR) $cm^-$
$^1$:1643,1595,1493,1363,1313,1267,1223,1184,1111,1063,1022,974, 847,798,764,692,606.
MS (ESI) m/z :489 $(M+H)^+$.
Anal. Calcd for $C_{24}H_{23}F_3N_4O_4 \cdot 0.08CF_3COOH \cdot$
$0.5H_2O$:C,57.28;H,4.79;F,12.15;N,11.06.Found:C,57.37;H,4.67;F, 11.94;N,11.01.

[Example 67]

3-[5-[3-(2,4-Difluorophenyl)-2-methylpropoxy]-1-indolinylcarbonylamino]propionic acid

(1) 3-(2,4-Difluorophenyl)-2-methylpropionic acid ethyl ester

[F289]

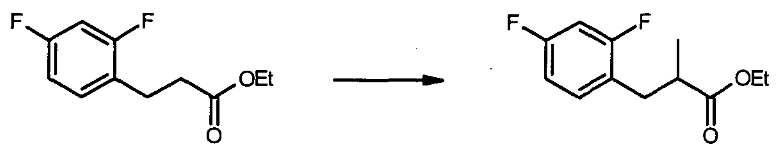

A solution of 3-(2,4-difluorophenyl)propionic acid ethyl ester (EP 401166; 2.38 g) in THF (20 mL) was cooled to -78°C, and a THF solution (13.3 mL) of 1.0M LiHMDS was added dropwise thereto under stirring. The reaction mixture was stirred at the same temperature for 1 hour, and then iodomethane (1.11 mL) was added thereto. The reaction mixture was stirred for 20 hours during which the mixture was allowed to warm to room temperature, and then saturated aqueous ammonium chloride solution was added thereto, followed by extraction with ethyl acetate (200 mL). The extract was dried over sodium sulfate anhydrate, and solvent was evaporated. Thereafter, the residue was purified by silica gel column chromatography (Yamazen Hi-Flash Column L), whereby the title compound (414 mg) was yielded.
$^1$H-NMR ($CDCl_3$) δ :

1.17(3H,d,J=6.3Hz), 1.19(3H,t,J=7.1Hz), 2.68-2.78(2H,m), 2.90-2.99(1H,m), 4.08(2H,q,J=7.1Hz),6.74-6.81(2H,m), 7.16-7.09(1H,m).
MS (ESI) m/z : 229 $(M+H)^+$.

(2) 3-(2,4-Difluorophenyl)-2-methylpropanol

[F290]

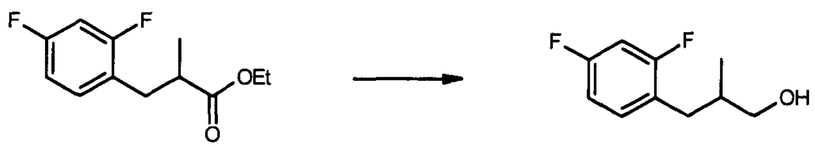

To a solution of 3-(2,4-difluorophenyl)-2-methylpropionic acid ethyl ester (441 mg) in DCM (10 mL), a toluene solution (2.90 mL) of 0.99M-DIBAH was added dropwise at 0°C under stirring. After stirring for 4 hours, 1N hydrochloric acid (50 mL) was added to the reaction mixture, followed by extraction with chloroform (2 x 100 mL). The extract was dried over sodium sulfate anhydrate, and solvent was evaporated. Thereafter, the residue was purified by silica gel column chromatography (Yamazen Hi-Flash Column L), whereby the title compound (250 mg) was yielded.

(3) 1-(tert-Butoxycarbonyl)-5-[3-(2,4-difluorophenyl)-2-methylpropoxy]indoline

[F291]

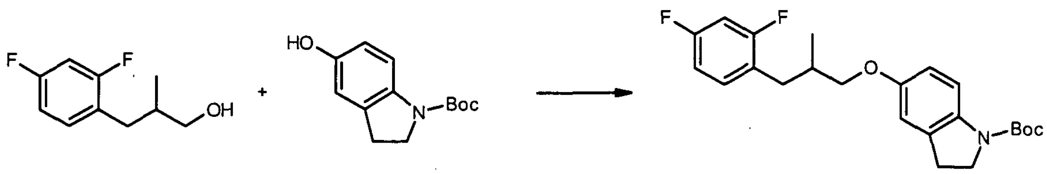

3-(2,4-Difluorophenyl)-2-methylpropanol (250 mg), 1-(tert-butoxycarbonyl)-5-hydroxyindoline (318 mg), and triphenylphosphine (531 mg) were dissolved in THF (10 mL), and DIAD (416 μL) was added dropwise to the solution under stirring. The reaction mixture was stirred for 17 hours and concentrated. The residue was purified by silica gel column chromatography (Yamazen Hi-Flash Column 2L), whereby the title compound (105 mg) was yielded.
$^1$H-NMR ($CDCl_3$) δ:
1.00(3H,d,J=6.6Hz),1.55(9H,br s),2.15-2.27(1H,m), 2.56(1H,dd,J=13.7, 7.8Hz), 2.84(1H,dd,J=13.9,6.6Hz), 3.04(2H,t, J=8.8Hz),3.70-3.77(2H,m),3.96(2H,br s),6.64-6.81(4H,m),7.07-7.15(1H,m),7.80-7.27(1H,m).
MS (ESI) m/z : 186 $(M+H)^+$.

(4) 5-[3-(2,4-Difluorophenyl)-2-methylpropoxy]indoline hydrochloride

[F292]

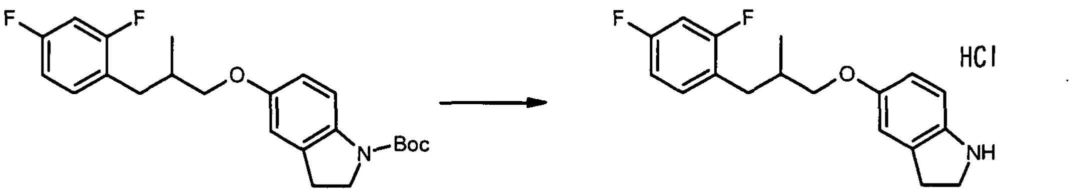

To 1-(tert-butoxycarbonyl)-5-[3-(2,4-difluorophenyl)-2-methylpropoxy]indoline (105 mg), 4N HCl/1,4-dioxane (10 mL) was added, and the mixture was stirred for 2 hours. The reaction mixture was concentrated, and diethyl ether was added to the concentrate to form solid. Thereafter, the solid was collected by filtration, and dried, whereby the title compound (58 mg) was yielded.
$^1$H-NMR ($CDCl_3$) δ:
1.03(3H,d,J=6.6Hz), 2.25(1H,td,J=13.1,6.4Hz), 2.59(1H,dd,J=13.3 ,7.7Hz), 2.83(1H,dd,J=13.7,6.6Hz), 3.27(2H,t, J=7.2Hz), 3.77(2H,d ,J=5.9Hz), 3.96 (2H, t, J=7.4Hz), 6.75-6.85 (4H, m), 7.06-7.14(1H,m),7.52(1H,d,J=9.3Hz), 11.60 (2H,br s).
MS (ESI) m/z : 304 $(M+H)^+$.

(5) 3-[N-(tert-Butoxycarbonyl)-N-[2-[5-[3-(2,4-difluorophenyl)-2-methylpropoxy]-1-indolinyl]-2-oxoethyl]amino]propionic acid tert-butyl ester

[F293]

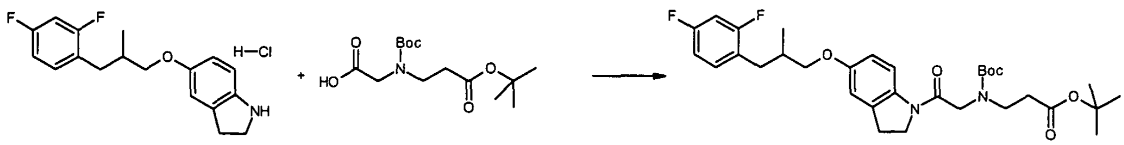

5-[3-(2,4-Difluorophenyl)-2-methylpropoxy]indoline hydrochloride (55 mg), N-(tert-butoxycarbonyl)-N-(tert-butoxycarbonylethyl)aminoacetic acid (49 mg), EDC·HCl (47 mg), HOBt (33 mg), and TEA (113 μL) were added to DMF (5 mL), and the mixture was stirred for 14 hours. The reaction mixture was diluted with ethyl acetate (100 mL), and washed with saturated brine (2 x 50 mL), followed by drying over sodium sulfate anhydrate. Solvent was evaporated, and the residue was purified by silica gel column chromatography (Yamazen Hi-Flash Column 2L), whereby the title compound (68 mg) was yielded.
$^1$H-NMR ($CDCl_3$ ) δ :
1.01 (3H, d, J=6.8Hz), 1.37-1.51 (18H,m), 2.15-2.28 (1H, m), 2.52-2.65(3H,m), 2.84(1H,dd,J=13.4,6.6Hz), 3.18(2H,q, J=8.5Hz), 3.59(2 H,q,J=6.7Hz),3.72-3.77(2H,m),3.96-4.19(4H,m),6.81-6.64(4H,m), 7.16-7.07(1H,m), 8.10(1H,t, J=9.8Hz).
MS (ESI) m/z : 589 $(M+H)^+$.

(6) 3-[N-(tert-Butoxycarbonyl)-N-[2-[5-[3-(2,4-difluorophenyl)-2-methylpropoxy]-1-indolinyl]-2-oxoethyl]amino]propionic acid hydrochloride

[F294]

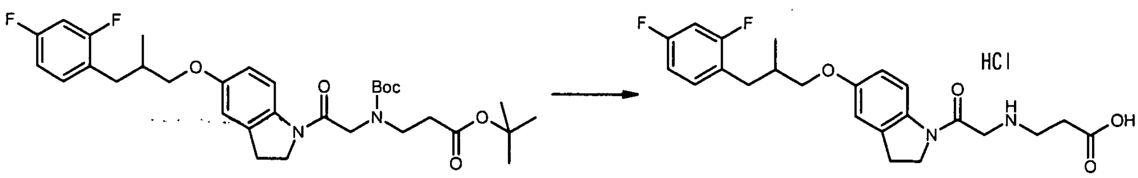

To N-(tert-Butoxycarbonyl)3-[5-[3-(2,4-difluorophenyl) - 2-methylpropoxy]-1-indolinylcarbonylamino]propionic acid tert-butyl ester (66 mg), 4N HCl/1,4-dioxane (10 mL) was added, and the mixture was stirred for 1 day. The reaction mixture was concentrated, and diethyl ether was added to the concentrate to form solid. Thereafter, the solid was collected by filtration, and dried under reduced pressure, whereby the title compound (15 mg) was yielded.
$^1$H-NMR (DMSO-$d_6$) δ:
0.94(3H,d,J=6.8Hz), 2.10-2.21(1H,m), 2.48-2.57(2H,m), 2.73-2.85(3H,m),3.13-3.30(3H,m), 3.79(2H,d,J=5.9Hz), 4.06 (2H,t,J=8.2Hz), 4.13(2H,s), 6 .76(1H,dd,J=8.7,2.1Hz), 6.87-6.90(1H,m), 7.02(1H,td,J=8.5,2.5Hz), 7.14-7.21(1H,m), 7.36-7.29(1H,m), 7.95(1H,d,J=8.8Hz). Proton peaks corresponding to $CO_2H$ and NHHCl were not observed.
MS (ESI) m/z : 433 $(M+H)^+$.

[Example 68]

3-[2-[5-(3-Methyl-4-trifluoromethylbenzyloxy)-2,3-dihydroindol-1-yl]-2-oxoethylamino]propionic acid

(1) 4-Iodo-3-methylbenzoic acid methyl ester

[F295]

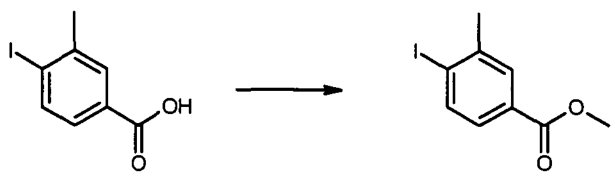

Methanol (120 mL) was added to 4-iodo-3-methylbenzoic acid (3.00 g), and thionyl chloride (4.18 mL) was added dropwise to the mixture under stirring at 0°C. After completion of dropwise addition, the reaction mixture was heated to 60°C while stirring overnight. The reaction mixture was cooled to room temperature, and concentrated, whereby the title compound (3.16 g) was yielded.
$^1$H-NMR ($CDCl_3$) δ :
2.48(3H,s),3.91(3H,s),7.50(1H,dd,J=8.1,2.2Hz), 7.88-7.90(2H,m).
MS(ESI);m/z:277$(M+H)^+$.

(2) 3-Methyl-4-trifluoromethylbenzoic acid methyl ester

[F296]

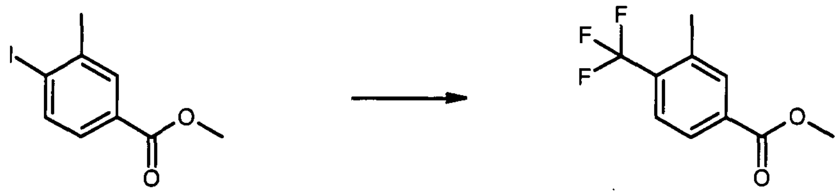

DMF (110 mL) was added to 4-iodo-3-methylbenzoic acid methyl ester (3.16 g), methyl 2,2-difluoro-2-(fluorosulfonyl)acetate (14.5 mL), and copper(I) iodide (2.18 g), and the mixture was stirred with heating at 90°C for 4 hours. The reaction mixture was cooled to room temperature, and saturated brine was added thereto. The mixture was extracted thrice, each with ethyl acetate, and the extracts were combined. The combined extract was washed with saturated brine, and dried over sodium sulfate. Insoluble matter was removed by filtration, and then the filtrate was concentrated. The residue was purified by silica gel column chromatography, whereby the title compound (2.66 g) was yielded.
$^1$H-NMR ($CDCl_3$) δ :
2.56(3H,s), 3.95(3H,s), 7.68(1H,d, J=8.1Hz), 7.93(1H,d,J=8.1Hz),7 .96 (1H, s).

(3) (3-Methyl-4-trifluoromethylphenyl)methanol

[F297]

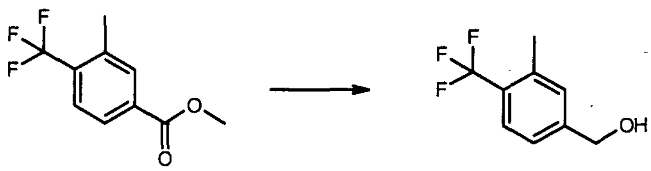

3-Methyl-4-trifluoromethylbenzoic acid methyl ester (0.20 g) was dissolved in THF (10 mL), and lithium borohydride (0.060 g) was added to the solution. The reaction mixture was refluxed with stirring for 3 hours and cooled to room temperature. 1N hydrochloric acid was added to the reaction mixture, followed by extraction thrice, each with ethyl acetate. The extracts were combined and washed with saturated brine, followed by drying over sodium sulfate. Insoluble matter was removed by filtration, and then the filtrate was concentrated. The residue was purified by silica gel column chromatography, whereby the title compound (0.16 g) was yielded.
$^1$H-NMR ($CDCl_3$) δ :
1.72(1H,t,J=5.9Hz), 2.48-2.50(3H,m), 4.73(2H,d,J=5.6Hz), 7.26-7.26(1H,m), 7.29(1H,s), 7.59(1H,d,J=7.8Hz).

(4) 3-[N-[2-[5-(3-Methyl-4-trifluoromethylbenzyloxy)-2,3-dihydroindol-1-yl]-2-oxoethyl]amino]propionic acid

[F298]

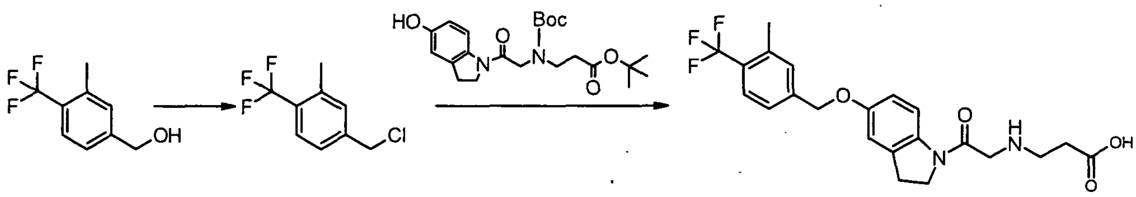

To a 1,2-dichloroethane solution (8 mL) of (3-methyl-4-trifluoromethylphenyl)methanol (0.16 g), thionyl chloride (0.30 mL) and DMF (trace) were added, and the mixture was stirred under heating at 60°C for 1 hour. The reaction mixture was cooled to room temperature and concentrated. To the residue (0.070 g), potassium carbonate (0.12 g), 3-[N-tert-butoxycarbonyl-N-[2-(5-hydroxy-2,3-dihydroindol-1-yl)-2-oxoethyl]amino]propionic acid tert-butyl ester (0.12 g), and DMF (3 mL) were added, and the resultant suspension was stirred at 80°C overnight. The reaction mixture was cooled to room temperature, and concentrated. The residue was purified by silica gel column chromatography, whereby a colorless oily substance (0.16 g, 0.27 mmol) was yielded. 4N HCl/1,4-dioxane (3 mL) was added to this substance, and the mixture was stirred at room temperature for 3 hours. The reaction mixture was concentrated, and purified by reverse phase preparative HPLC, whereby the title compound (0.041 g) was yielded.
$^1$H-NMR ($CD_3OD$) δ:
2.43-2.48(3H,m),2.53-2.56(2H,m),3.21-3.23(2H,m),3.64-3.77(2H,m),4.04-4.13(4H,m),5.11(2H,s),6.81-6.84(1H,m), 6.94(1H,s),7.40-7.43(2H,m), 7.62(1H,d,J=8.1Hz), 8.01-8.06(1H,m).
MS (ESI) ;m/z:437 $(M+H)^+$.
Anal. Calcd for $C_{22}H_{23}F_3N_2O_4\cdot$
$0.25H_2O$:C,59.93;H,5.37;N,6.35;F,12.93.Found:C,60.03;H,5.20;N, 6.33;F,12.63.
IR(ATR)$cm^{-1}$:493,1362,1115,1045,843,607.

[Example 69]

3-[2-[5-(3-Isobutyl-4-trifluoromethylbenzyloxy)-2,3-dihydroindol-1-yl]-2-oxoethylamino]propionic acid

(1) 3-Hydroxy-4-iodobenzoic acid methyl ester

[F299]

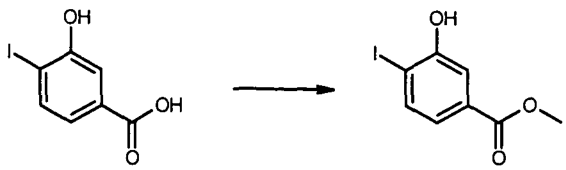

Methanol (100 mL) was added to 3-hydroxy-4-iodobenzoic acid (5.00 g), and thionyl chloride (6.91 mL) was added dropwise to the mixture with stirring at 0°C. After completion of dropwise addition, the reaction mixture was heated to 60°C, and stirred overnight. The reaction mixture was cooled to room temperature, and concentrated, whereby the title compound (5.27 g) was yielded.
$^1$H-NMR ($CDCl_3$) δ :
3.91(3H,s), 7.33(1H,dd,J=8.3,2.0Hz), 7.62(1H,d,J=2.0Hz), 7.75(1H , d, J=8.3Hz).
MS (ESI) ;m/z:279 $(M+H)^+$.

(2) 3-Benzyloxy-4-iodobenzoic acid methyl ester

[F300]

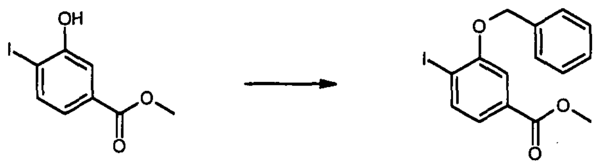

3-Hydroxy-4-iodobenzoic acid methyl ester (5.27 g), benzyl bromide (2.70 mL), and potassium carbonate (7.85 g) were added to acetonitrile (200 mL), and the mixture was refluxed with stirring for 1.5 hours. The reaction mixture was cooled to room temperature, and insoluble matter was removed by filtration. The filtrate was concentrated, and the residue was purified by silica gel column chromatography, whereby the title compound (7.77 g) was yielded.
$^1$H-NMR ($CDCl_3$) δ:
3.91(3H,s),5.21(2H,s),7.34-7.43(4H,m),7.52-7.54(3H,m), 7.88(1H,d,J=8.1Hz).
MS (ESI) ;m/z:369 $(M+H)^+$.

(3) 3-Benzyloxy-4-trifluoromethylbenzoic acid methyl ester

[F301]

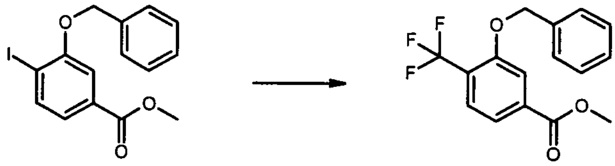

DMF (190 mL) was added to 3-benzyloxy-4-iodobenzoic acid methyl ester (6.97 g), methyl 2,2-difluoro-2-(fluorosulfonyl)acetate (23.9 mL), and copper(I) iodide (3.61 g), and the mixture was stirred at 90°C for 4 hours. The reaction mixture was cooled to room temperature, and saturated brine was added thereto, followed by extraction thrice, each with ethyl acetate. The extracts were combined and washed with saturated brine, followed by drying over sodium sulfate. Insoluble matter was removed by filtration, and then the filtrate was concentrated. The residue was purified by silica gel column chromatography, whereby the title compound (5.88 g) was yielded.
$^1$H-NMR ($CDCl_3$) δ:
3.95(3H,s),5.25(2H,s),7.34-7.35(1H,m),7.38-7.42 (2H,m), 7.46 (2H,d,J=7.1Hz), 7.66-7.69 (2H,m), 7.73 (1H, s).

(4) 3-Hydroxy-4-trifluoromethylbenzoic acid methyl ester

[F302]

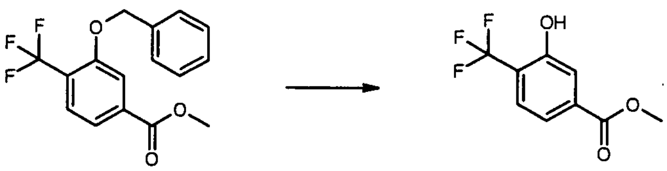

Methanol (200 mL) was added to 3-benzyloxy-4-trifluoromethylbenzoic acid methyl ester (5.88 g) and a 5% Pd/C catalyst (wet) (1.20 g), and the mixture was stirred under a hydrogen atmosphere at room temperature overnight. The reaction mixture was filtered, and the filtrate was concentrated. The residue was purified by silica gel column

chromatography, whereby the title compound (3.94 g) was yielded.
$^1$H-NMR ($CDCl_3$) δ :
3.95(3H,s),6.03(1H,s),7.60(1H,d,J=8.0Hz),7.66(1H,d,J=8.0Hz),7 .69(1H,s).

(5) 3-Trifluoromethanesulfonyloxy-4-trifluoromethylbenzoic acid methyl ester

[F303]

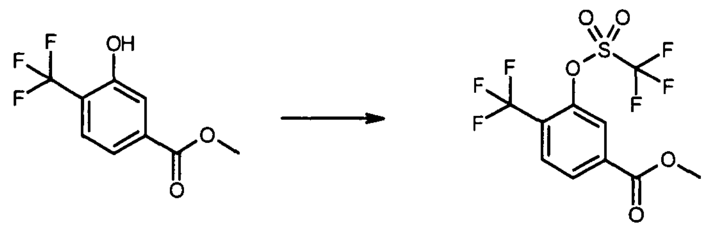

Dichloromethane was added to 3-hydroxy-4-trifluoromethylbenzoic acid methyl ester (3.00 g), TEA (2.85 mL), and DMAP (0.170 g), and trifluoromethanesulfonic anhydride (2.68 mL) was added dropwise to the mixture with stirring at 0°C. The reaction mixture was heated to room temperature, and stirred for 1 hour. Saturated aqueous sodium bicarbonate solution was added to the reaction mixture, followed by extraction thrice, each with chloroform. The extracts were combined and washed with saturated brine, followed by drying over sodium sulfate. Insoluble matter was removed by filtration, and then the filtrate was concentrated. The residue was purified by silica gel column chromatography, whereby the title compound (4.71 g) was yielded.
$^1$H-NMR ($CDCl_3$) δ :
4.00(3H,s),7.86(1H,d,J=8.1Hz),8.15-8.17(2H,m).

(6) 3-Isobutyl-4-trifluoromethylbenzoic acid methyl ester

[F304]

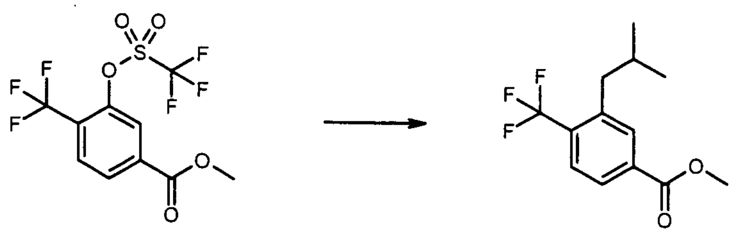

Water (2.5 mL) and toluene (5 mL) were added to 3-trifluoromethanesulfonyloxy-4-trifluoromethylbenzoic acid methyl ester (0.40 g), tetrakis(triphenylphosphine)palladium(0) (0.13 g), 2-methylpropylboric acid (0.35 g), and cesium carbonate (1.9 g), and the mixture was stirred under a stream of nitrogen at 80°C overnight. The reaction mixture was cooled to room temperature, and water was added thereto, followed by extraction thrice, each with diethyl ether. The extracts were combined and washed with saturated brine, followed by drying over sodium sulfate. Insoluble matter was removed through filtration, and then the filtrate was concentrated. The residue was purified by silica gel column chromatography, whereby the title compound (0.24 g) was yielded.
$^1$H-NMR ($CDCl_3$) δ :

0.94(6H,d,J=6.6Hz), 1.97-2.04(1H,m), 2.70(2H,d,J=6.9Hz), 3.95(3H,s), 7.69-7.70(1H,m),7.93-7.94(1H,m),7.98(1H,s).

(7) (3-Isobutyl-4-trifluoromethylphenyl)methanol

[F305]

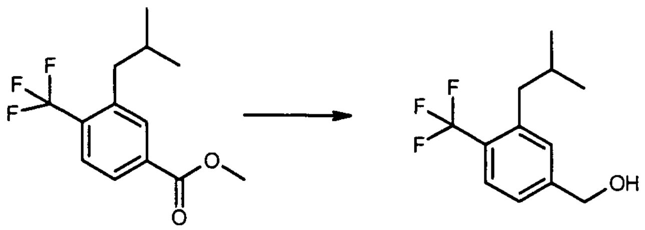

3-Isobutyl-4-trifluoromethylbenzoic acid methyl ester (0.24 g) was dissolved in THF (10 mL), and lithium borohydride (0.060 g) was added to the solution, followed by reflux with stirring for 3 hours. The reaction mixture was cooled to room temperature, and 1N hydrochloric acid was added thereto, followed by extraction thrice, each with ethyl acetate. The extracts were combined and washed with saturated brine, followed by drying over sodium sulfate. Insoluble matter was removed by filtration, and then the filtrate was concentrated. The residue was purified by silica gel column chromatography, whereby the title compound (0.16 g) was yielded.
$^1$H-NMR ($CDCl_3$) $\delta$ :
0.93(6H,d,J=6.6Hz),1.72(1H,t,J=5.9Hz),1.92-2.02(1H,m),2.66(2H,d,J=7.4Hz),4.74(2H,d,J=5.9Hz),7.28-7.31(2H,m), 7.61(1H,d,J=8.1Hz).

(8) 3-[N-[2-[5-(3-Isobutyl-4-trifluoromethylbenzyloxy)-2,3-dihydroindol-1-yl]-2-oxoethyl]amino]propionic acid

[F306]

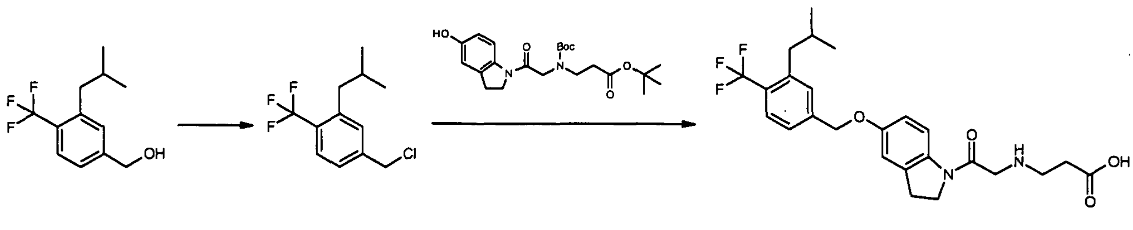

To a 1,2-dichloroethane solution (7 mL) of (3-isobutyl-4-trifluoromethylphenyl)methanol (0.16 g), thionyl chloride (0.26 mL) and DMF (trace) were added, and the mixture was stirred with heating at 60°C for 1 hour. The reaction mixture was cooled to room temperature, and concentrated. To the residue (0.09 g), potassium carbonate (0.12 g), 3-[N-tert-butoxycarbonyl-N-[2-(5-hydroxy-2,3-dihydroindol-1-yl)-2-oxoethyl]amino]propionic acid tert-butyl ester (0.12 g), and DMF (3 mL) were added, and the resultant suspension was stirred at 80°C overnight. The reaction mixture was cooled to room temperature, and concentrated. The residue was purified by silica gel column chromatography, whereby an oily

substance (0.18 g) was yielded. 4N HCl/1,4-dioxane (3 mL) was added to the oily substance, and the mixture was stirred at room temperature for 3 hours. The reaction mixture was concentrated, and purified by reverse phase preparative HPLC, whereby the title compound (0.040 g) was yielded.
$^1$H-NMR ($CD_3OD$) δ:
0.90(6H,d,J=6.6Hz),1.91-1.98(1H,m),2.66-2.70(4H,m),3.21(2H,t,J=8.3Hz),3.33-3.35 (2H,m), 4.06(2H,t,J=8.3Hz), 4.12 (2H,s), 5.13(2H,s), 6.83(1H,d ,J=8.9Hz), 6.93(1H,s), 7.41(1H,d,J=8.1Hz), 7.45(1H,s), 7.64(1H,d, J=8.1Hz),8.02(1H,d, J=8.9Hz).
MS (ESI) ; m/z : 479 $(M+H)^+$.
Anal. Calcd for $C_{25}H_{29}F_3N_2O_4\cdot$ $1.75H_2O$:C,58.87;H,6.42;N,5.49.Found:C,58.62;H,6.00;N,5.26.
IR(ATR)$cm^{-1}$:2960,1651,1597,1491,1309,1113.

[Example 70]

3-[N-[2-[5-(3-Cyclopropyl-4-trifluoromethylbenzyloxy)-2,3-dihydroindol-1-yl]-2-oxoethyl]amino]propionic acid

(1) 3-Cyclopropyl-4-trifluoromethylbenzoic acid methyl ester

[F307]

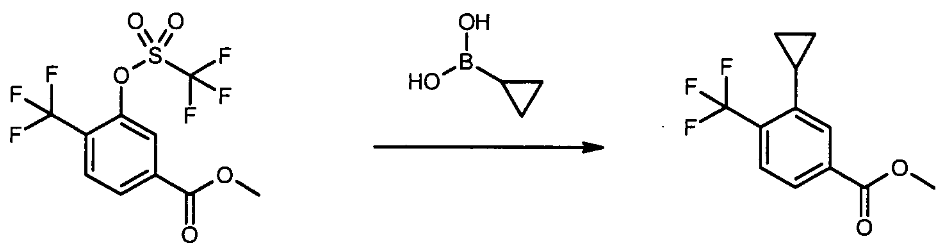

Water (2.5 mL) and toluene (5 mL) were added to 3-trifluoromethanesulfonyloxy-4-trifluoromethylbenzoic acid methyl ester (0.40 g), tetrakis(triphenylphosphine)palladium(0) (0.13 g), cyclopropylboric acid (0.29 g), and cesium carbonate (1.9 g), and the mixture was stirred under a stream of nitrogen at 80°C overnight. The reaction mixture was cooled to room temperature, and water was added thereto, followed by extraction thrice, each with diethyl ether. The extracts were combined and washed with saturated brine, followed by drying over sodium sulfate. Insoluble matter was removed by filtration, and then the filtrate was concentrated. The residue was purified by silica gel column chromatography, whereby the title compound (0.27 g) was yielded.
$^1$H-NMR ($CDCl_3$) δ:
0.84-0.86(2H,m),1.06-1.11(2H,m),2.21-2.25(1H,m), 3.93(3H,s), 7.67-7.69(2H,m), 7.89(1H,t,J=4.4Hz).

(2) (3-Cyclopropyl-4-trifluoromethylphenyl)methanol

[F308]

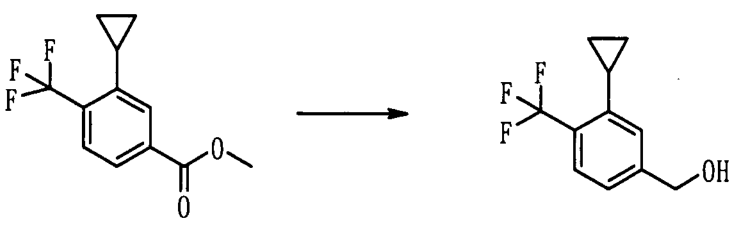

3-Cyclopropyl-4-trifluoromethylbenzoic acid methyl ester (0.27 g) was dissolved in THF (10 mL), and lithium

borohydride (0.070 g) was added to the solution, followed by stirring at 60°C for 3 hours. The reaction mixture was cooled to room temperature, and 1N hydrochloric acid was added thereto, followed by extraction thrice, each with ethyl acetate. The extracts were combined and washed with saturated brine, followed by drying over sodium sulfate. Insoluble matter was removed by filtration, and then the filtrate was concentrated. The residue was purified by silica gel column chromatography, whereby the title compound (0.22 g) was yielded.
$^1$H-NMR ($CDCl_3$) δ:
0.78-0.82(2H,m),1.01-1:05(2H,m),1.70(1H,t,J=5.8Hz),2.21-2.22(1H,m),4.71(2H,d,J=5.8Hz),7.04(1H,s),7.23(1H,d, J=8.0Hz),7 .60(1H,d,J=8.0Hz).

(3) 3-[N-[2-[5-(3-Cyclopropyl-4-trifluoromethylbenzyloxy) - 2,3-dihydroindol-1-yl]-2-oxoethyl]amino]propionic acid

[F309]

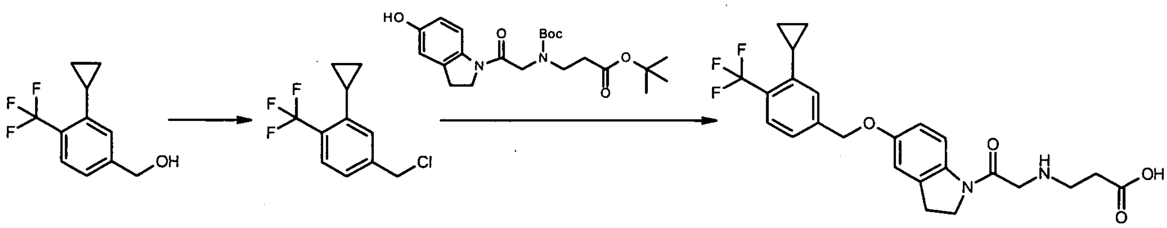

To a 1,2-dichloroethane solution (10 mL) of (3-cyclopropyl-4-trifluoromethylphenyl)methanol (0.22 g), thionyl chloride (0.37 mL) and DMF (trace) were added, and the mixture was stirred with heating at 60°C for 1 hour. The reaction mixture was cooled to room temperature, and concentrated. To the residue (0.080 g), potassium carbonate (0.12 g), 3-[N-tert-butoxycarbonyl-N-[2-(5-hydroxy-2,3-dihydroindol-1-yl)-2-oxoethyl]amino]propionic acid tert-butyl ester (0.12 g), and DMF (3 mL) were added, and the resultant suspension was stirred at 80°C overnight. The reaction mixture was cooled to room temperature, and concentrated. The residue was purified by silica gel column chromatography, whereby an oily substance (0.16 g) was yielded. 4N HCl/1,4-dioxane (3 mL) was added to the oily substance, and the mixture was stirred at room temperature for 3 hours. The reaction mixture was concentrated, and purified by reverse phase preparative HPLC, whereby the title compound (0.038 g) was yielded.
$^1$H-NMR ($CD_3OD$) δ :
0.66-0.70(2H,m),0.92-0.97(2H,m),2.06-2.09(1H,m), 2.52(2H,t,J=6.4Hz), 3.12(2H,t,J=8.1Hz), 3.54-3.66(2H,m), 3.96-4.00(4H,m), 4.99(2H,s), 6.72(1H,dd,J=8.8,2.5Hz), 6.82(1H,d,J=2.5 Hz), 7.06(1H,s), 7.26(1H,d,J=8.0Hz), 7.52(1H, d,J=8.0Hz), 7.93(1H, d,J=8.8Hz).
MS (ESI) ; m/z : 463 $(M+H)^+$.
Anal. Calcd for $C_{24}H_{25}F_3N_2O_4$· $1.5H_2O$:C,58.89;H,5.77;N,5.72.Found:C,58.92;H,5.40;N,6.06.
IR(ATR)$cm^{-1}$:1652,1489,1311,1107,1039,827.

[Example 71]

3-[N-[2-[5-(3-Isopropyl-4-trifluoromethylbenzyloxy)-2,3-dihydroindol-1-yl]-2-oxoethyl]amino]propionic acid

(1) 3-Isopropenyl-4-trifluoromethylbenzoic acid methyl ester

[F310]

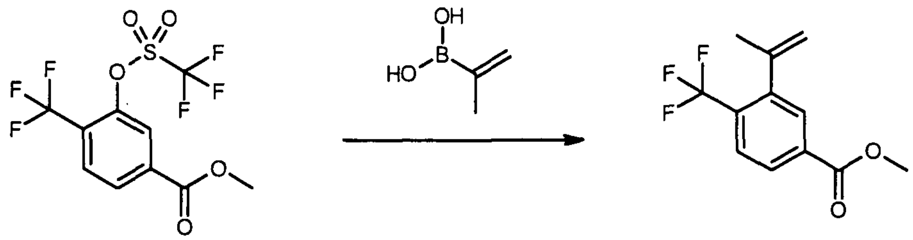

Water (2.5 mL) and toluene (5 mL) were added to 3-trifluoromethanesulfonyloxy-4-trifluoromethylbenzoic acid methyl ester (0.40 g), tetrakis(triphenylphosphine)palladium(0) (0.13 g), isopropenylboric acid (0.43 mL), and cesium carbonate (1.9 g). Under a stream of nitrogen gas, the resultant mixture was stirred overnight at 80°C. The reaction mixture was cooled to room temperature, and water was added thereto, followed by extraction thrice, each with diethyl ether. The extracts were combined and washed with saturated brine, followed by drying over sodium sulfate. Insoluble matter was removed by filtration, and the filtrate was concentrated. The residue was purified by silica gel column chromatography, whereby the title compound (0.28 g) was yielded.
$^1$H-NMR ($CDCl_3$) δ :
2.09(3H,s), 3.95(3H,s), 4.91(1H,s), 5.27-5.2(1H,m), 7.72(1H,d,J=8.2Hz), 7.92(1H,s), 8.01(1H,d,J=8.2Hz).

(2) 3-Isopropyl-4-trifluoromethylbenzoic acid methyl ester

[F311]

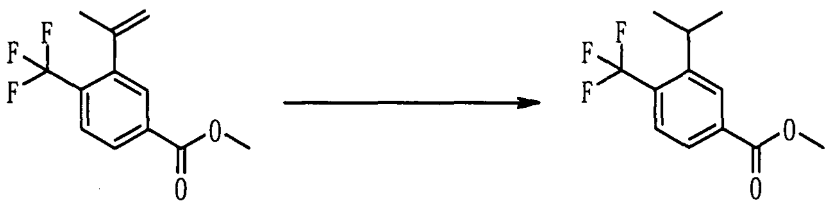

Methanol (10 mL) was added to 3-isopropenyl-4-trifluoromethylbenzoic acid methyl ester (0.28g) and 5% Pd/C catalyst (wet) (0.060 g), and the resultant mixture was stirred overnight under a hydrogen atmosphere at room temperature. The reaction mixture was filtered, and the filtrate was concentrated, whereby the title compound (0.28 g) was yielded.
$^1$H-NMR ($CDCl_3$) δ :
1.30(6H,d,J=6.9Hz),3.35-3.42(1H,m), 3.95(3H,s), 7.67(1H,d,J=8.1Hz), 7.91(1H,d,J=8.3Hz),8 .14(1H,s).

(3) (3-Isopropyl-4-trifluoromethylphenyl)methanol

[F312]

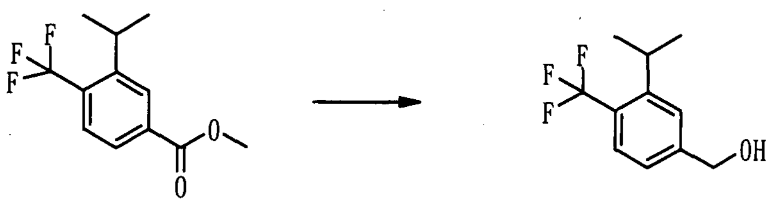

(3-Isopropyl-4-trifluoromethylbenzoic acid methyl ester (0.28 g) was dissolved in THF (10 mL). Lithium borohydride (0.070 g) was added to the solution, and the resultant mixture was refluxed for 3 hours. The reaction mixture was cooled to room temperature, and 1N hydrochloric acid was added to the mixture, followed by extraction thrice, each with ethyl acetate. The extracts were combined and washed with saturated brine, followed by drying over sodium sulfate. Insoluble matter was removed by filtration, and the filtrate was concentrated. The residue was purified by silica gel column chromatography, whereby the title compound (0.19 g) was yielded.
$^1$H-NMR ($CDCl_3$) δ :
1.27-1.28(6H,m), 1.74(1H,t,J=5.9Hz), 3.35-3.38(1H,m), 4.76(2H,d,J=5.9Hz), 7.23-7.26(1H,m), 7.46(1H,s), 7.59(1H,d, J=8.1Hz).

(4) 3-[N-[2-[5-(3-Isopropyl-4-trifluoromethylbenzyloxy)-2,3-dihydroindol-1-yl]-2-oxoethyl]amino]propionic acid

[F313]

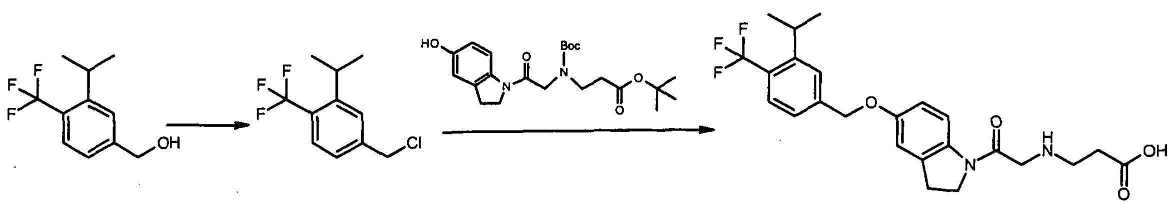

Thionyl chloride (0.32 mL) and DMF (trace) were added to a solution (10 mL) of (3-isopropyl-4-trifluoromethylphenyl)methanol (0.19 g) in 1,2-dichloroethane. The resultant mixture was stirred for 1 hour at 60°C. The reaction mixture was cooled to room temperature, and then concentrated. To an aliquot (0.07 g) of the residue, potassium carbonate (0.12 g), 3-[N-tert-butoxycarbonyl-N-[2-(5-hydroxy-2,3-dihydroindol-1-yl)-2-oxoethyl]amino]propionic acid tert-butyl ester (0.12 g), and DMF (3 mL) were added, and the resultant suspension was stirred overnight at 80°C. The reaction mixture was cooled to room temperature and then concentrated. The residue was purified by silica gel column chromatography, to thereby yield an oily product (0.19 g, 0.31 mmol, quant.). 4N HCl/1,4-dioxane (3 mL) was added to the oily product, and the resultant mixture was stirred for 3 hours at room temperature. The reaction mixture was concentrated, and the residue was purified by reverse phase preparative HPLC, whereby the title compound (0.050 g) was yielded.
$^1$H-NMR ($CDCl_3$) δ :
1.26(6H,d,J=6.6Hz),2.64-2.66(2H,m),3.12-3.19(4H,m),3.36-3.37(1H,m),3.75-3.78 (2H,m), 3.96 (2H, t, J=8.2Hz), 5.03 (2H, s), 6.77-6.79(2H,m),7.29-7.30(1H,m), 7.49(1H,s), 7.58(1H,d,J=8.2Hz), 8.07(1H,d,J=8.2Hz).
MS (ESI) ; m/z : 465 $(M+H)^+$.
Anal. Calcd for
$C_{24}H_{27}F_3N_2O_4\cdot1.5H_2O$:C,58.65;H,6.15;N,5.70.Found:C,58.41;H,5.79; N,5.43.
IR (ATR) $cm^{-1}$: 2962, 1643, 1491, 1309, 1109, 829.

[Example 72]

3-[N-[2-[5-(3-Cyclobutyl-4-trifluoromethylbenzyloxy)-2,3-dihydroindol-1-yl]-2-oxoethyl]amino]propionic acid

(1) 3-Cyclobutyl-4-trifluoromethylbenzoic acid methyl ester

[F314]

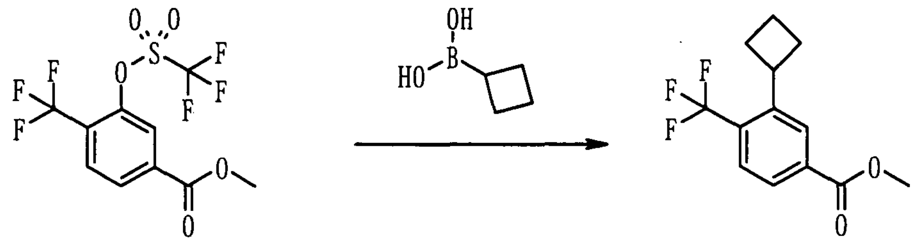

Water (2.5 mL) and toluene (5 mL) were added to 3-trifluoromethanesulfonyloxy-4-trifluoromethylbenzoic acid methyl ester (0.60 g), tetrakis(triphenylphosphine)palladium(0) (0.20 g), cyclobutylboric acid (0.51 g), and cesium carbonate (2.8 g). The resultant mixture was stirred overnight under a stream of nitrogen gas at 80°C. The reaction mixture was cooled to room temperature, and water was added to the mixture, followed by extraction thrice, each with diethyl ether. The extracts were combined and washed with saturated brine, followed by drying over sodium sulfate. Insoluble matter was removed by filtration, and the filtrate was concentrated, whereby a mixture (0.23 g) of the title compound and impurities was yielded.

(2) (3-Cyclobutyl-4-trifluoromethylphenyl)methanol

[F315]

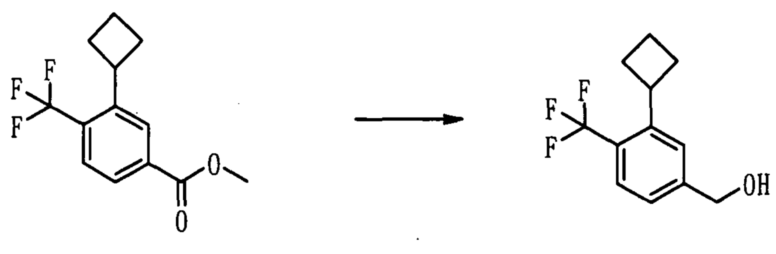

The above mixture (0.23 g) of 3-cyclobutyl-4-trifluoromethylbenzoic acid methyl ester and impurities was dissolved in THF (10 mL), and lithium borohydride (0.060 g) was added to the solution, followed by reflux for 3 hours under stirring. The reaction mixture was cooled to room temperature and then to 0°C, and 1N hydrochloric acid was added to the reaction mixture, followed by extraction thrice, each with ethyl acetate. The extracts were combined and washed with saturated brine, followed by drying over sodium sulfate. Insoluble matter was removed by filtration, and the filtrate was concentrated. The residue was purified by silica gel column chromatography, whereby the title compound (0.040 g) was yielded.
$^1$H-NMR ($CDCl_3$) δ :
1.75-1.77 (1H, m), 1.85-1.89 (1H, m), 1.99-2.09 (1H, m), 2.18-2.26 (2H, m), 2.34-2.36 (2H, m), 3.89-3.91 (1H, m), 4.78 (2H, d,J=5.6Hz), 7.27(1H,d,J=5.6Hz), 7.58-7.62 (2H,m) .

(3) 3-[N-[2-[5-(3-Cyclobutyl-4-trifluoromethylbenzyloxy)-2,3-dihydroindol-1-yl]-2-oxoethyl]amino]propionic acid

[F316]

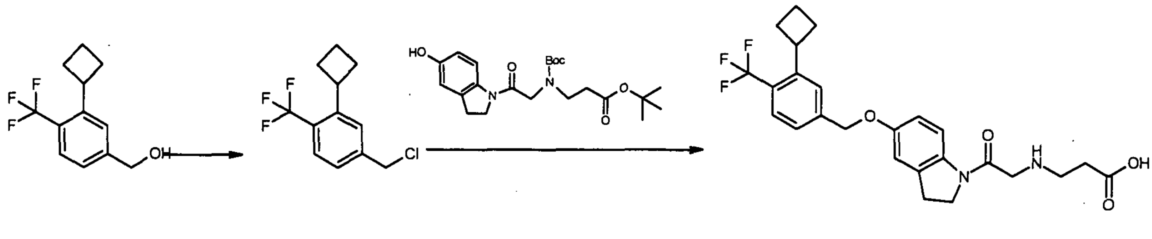

Thionyl chloride (0.070 mL) and DMF (catalytic amount) were added to a solution (5 mL) of (3-cyclobutyl-4-trifluoromethylphenyl)methanol (0.040 g) in 1,2-dichloroethane, and the resultant mixture was stirred for 1 hour at 60°C. The reaction mixture was cooled to room temperature and then concentrated. Potassium carbonate (0.070 g), 3-[N-tert-butoxycarbonyl-N-[2-(5-hydroxy-2,3-dihydroindol-1-yl)-2-oxoethyl]amino]propionic acid tert-butyl ester (0.080 g), and DMF (2 mL) were added to the residue, and the resultant suspension was stirred overnight at 80°C. The reaction mixture was cooled to room temperature and then concentrated. The residue was purified by silica gel column chromatography, whereby an oily product (0.060 g) was yielded. 10% TFA-dichloromethane (3 mL) was added to the oily product, and the resultant mixture was stirred for 5 hours at room temperature. The reaction mixture was concentrated, whereby the title compound (0.044 g) was yielded.
$^1$H-NMR ($CD_3OD$) δ:
1.86-1.90(1H,m), 2.01-2.10(1H,m), 2.17-2.27(2H,m), 2.32-2.34 (2H,m), 2.83 (2H, t, J=6.7Hz), 3.23-3.28(2H,m), 3.38 (2H,t,J=6.7Hz), 3.89-3.91(1H,m), 4.08(2H,t,J=8.3Hz), 4.15(2H,s), 5.17(2H,s), 6.86(1H,d d,J=8.7,2.6Hz), 6.97(1H,d, J=2.6Hz), 7.40(1H,d,J=8.1Hz), 7.61(1H, d, J=8.1Hz), 7.72(1H, s), 8.05 (1H,d,J=8.7Hz).
MS (ESI) ;m/z:477 $(M+H)^+$.
Anal. Calcd for
$C_{25}H_{27}F_3N_2O_4 \cdot CF_3CO_2H$, $0.25H_2O$: C, 54.50; H, 4.83; N,4.71 ; F, 19.16. Found : C,54.52;H,4.75;N,4.65;F,19.47.
IR (ATR) $cm^{-1}$: 1725,1655, 14 96,142.3, 1182,1119,

[Example 73]

3-[N-[2-[5-(3-Cyclopentyl-4-trifluoromethylbenzyloxy)-2,3-dihydroindol-1-yl]-2-oxoethyl]amino]propionic acid

(1) 3-Cyclopenten-1-yl-4-trifluoromethylbenzoic acid methyl ester

[F317]

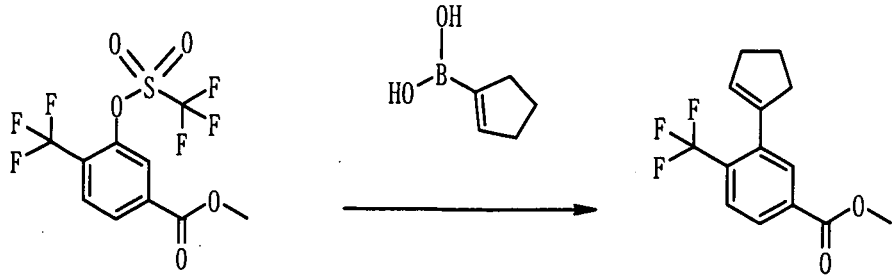

Water (2.5 mL) and toluene (5 mL) were added to 3-trifluoromethanesulfonyloxy-4-trifluoromethylbenzoic acid methyl ester (0.40 g), tetrakis(triphenylphosphine)palladium(0) (0.13 g), cyclopenten-1-ylboric acid (0.14 g), and cesium carbonate (1.9 g), and the resultant mixture was refluxed overnight under a stream of nitrogen gas. The reaction mixture was cooled to room temperature, and water was added to the mixture, followed by extraction thrice, each with diethyl ether. The extracts were combined and washed with saturated brine, followed by drying over sodium sulfate. Insoluble matter was removed by filtration, and the filtrate was concentrated. The residue was purified by silica gel column chromatography, whereby the title compound (0.23 g) was yielded.
$^1$H-NMR ($CDCl_3$) δ:
2.00-2.07(2H,m),2.51-2.55(2H,m),2.66-2.68 (2H,m), 3.94 (3H, s), 5.75-5.78(1H,m), 7.72(1H,d,J=8.1Hz),7.95(1H,s),7.98 (1H,d,J=8.1Hz).

(2) (3-Cyclopentyl-4-trifluoromethylbenzoic acid methyl ester

[F318]

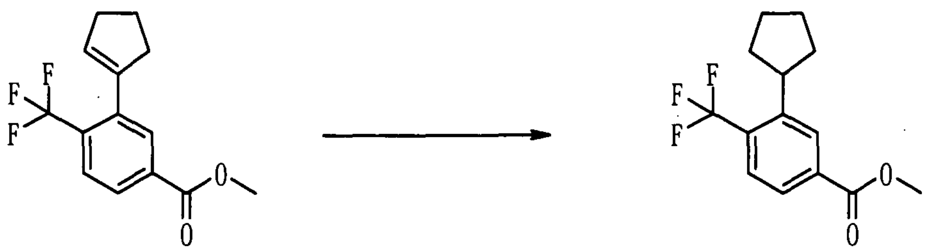

Methanol (10 mL) was added to 3-cyclopenten-1-yl-4-trifluoromethylbenzoic acid methyl ester (0.23 g) and 5% Pd/C catalyst (wet) (0.040 g), and the resultant mixture was stirred overnight under hydrogen at room temperature. The reaction mixture was filtered, and the filtrate was concentrated, whereby the title compound (0.20 g) was yielded.
$^1$H-NMR($CDCl_3$) δ:
1.60-1.78(4H,m),1.89-1.90(2H,m),2.09-2.12(2H,m),3.37-3.40(1H,m), 3.95(3H,s), 7.66(1H,d,J=8.1Hz), 7.89(1H,d, J=8.1Hz),8 .13(1H,s).

(3) (3-Cyclopentyl-4-trifluoromethylphenyl)methanol

[F319]

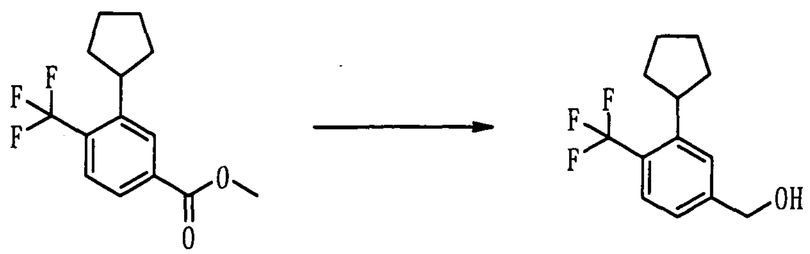

(3-Cyclopentyl-4-trifluoromethylbenzoic acid methyl ester (0.20 g) was dissolved in THF (10 mL), and lithium borohydride (0.050 g) was added to the solution, followed by reflux for 3 hours. The reaction mixture was cooled to 0°C, and 1N hydrochloric acid was added to the mixture, followed by extraction thrice, each with ethyl acetate. The extracts were combined and washed with saturated brine, followed by drying over sodium sulfate. Insoluble matter was removed by filtration, and the filtrate was concentrated. The residue was purified by silica gel column chromatography, whereby the title compound (0.10 g) was yielded.
$^1$H-NMR ($CDCl_3$) δ:
1.60-1.64(2H,m),1.67-1.78(3H,m),1.80-1.91(2H,m),2.06-2.11(2H,m),3.33-3.41(1H,m), 4.74(2H,d,J=5.6Hz), 7.24(1H,d, J=8.1Hz),7.46(1H,s),7 .58(1H,d,J=8.1Hz).

(4) 3-[N-[2-[5-(3-Cyclopentyl-4-trifluoromethylbenzyloxy)-2,3-dihydroindol-1-yl]-2-oxoethyl]amino]propionic acid

[F320]

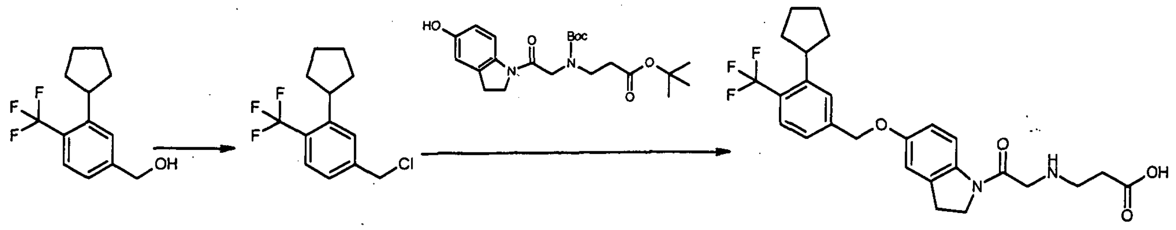

Thionyl chloride (0.15 mL) and DMF (trace) were added to a solution (5 mL) of (3-cyclopentyl-4-trifluoromethylphenyl)methanol (0.10 g) in 1,2-dichloroethane, and the resultant mixture was stirred for 1 hour at 60°C. The reaction mixture was cooled to room temperature and then concentrated. Potassium carbonate (0.17 g), 3-[N-(tert-butoxycarbonyl)-N-[2-(5-hydroxy-2,3-dihydroindol-1-yl)-2-oxoethyl]amino]propionic acid tert-butyl ester (0.17 g), and DMF (4 mL) were added to the residue, and the resultant suspension was stirred overnight at 80°C. The reaction mixture was cooled to room temperature and then concentrated. The residue was purified by silica gel column chromatography, whereby an oily product (0.23 g) was yielded. 10% TFA-dichloromethane (3 mL) was added to the oily product, and the resultant mixture was stirred for 5 hours at room temperature. The reaction mixture was concentrated, whereby the title compound (0.19 g) was yielded.
$^1$H-NMR ($CD_3OD$) δ:
1.62-1.64(2H,m),1.73-1.74(2H,m),1.86-1.89(2H,m),2.04-2.07(2H,m),2.83(2H,t,J=6.7Hz),3.24(2H,t,J=8.3Hz),3.38-3.39 (3H,m),4.07(2H,t,J=8.3Hz),4.15(2H,s),5.14(2H,s),6.84(1H,d d,J=8.8,2.6Hz),6.95(1H,d,J=2.6Hz),7.37(1H,d,J=5.1Hz), 7.60(2H, d,J=8.1Hz),8.04(1H,d,J=8.8Hz).
MS (ESI) ;m/z:491 $(M+H)^+$.
Anal. Calcd for
$C_{26}H_{29}F_3N_2O_4$. $CF_3CO_2H$, $0.25H_2O$:C,55.22 ; H, 5.05 ; N, 4.60 ; F, 18.72. Found :C,55.32;H,5.05;N,4.53;F,18.88.
IR(ATR)$cm^{-1}$:1728,1657,1495,1182,1115,1036.

[Example 74]

3-[N-[2-[5-(3-Cyclobutyl-4-trifluoromethylbenzyloxy)-2,3-dihydro-1-yl]-2-oxoethyl]amino]propionic acid

(1) 3,3,3-Trifluoro-2-hydroxy-1-phenylpropan-1-one (Nes, W.R. and Burger, Alfred. Journal of the American Chemical Society (1950), 72, 5409-13)

[F321]

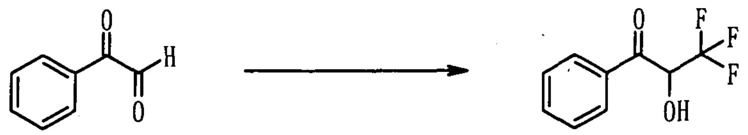

1,2-Dimethoxyethane (375 mL) was added to phenylglyoxal monohydrate (5.0 g) and trifluoromethyltrimethylsilane (11 g), and, with stirring under cooling on ice, cesium fluoride (0.57 g) was added to the resultant mixture, followed by stirring for 1 hour. The reaction temperature was elevated to room temperature, and the reaction mixture was stirred for 5 hours. The resultant mixture was concentrated, and THF (20 mL) and 6N hydrochloric acid (40 mL) were added to the residue, followed by stirring for 3 hours at room temperature. The reaction mixture was extracted thrice, each with diethyl ether. The extracts were combined and washed with saturated brine, followed by drying over sodium sulfate. Insoluble matter was removed by filtration, and the filtrate was concentrated. The residue was purified by silica gel column chromatography, whereby the title compound (1.4 g) was yielded.
$^1$H-NMR ($CDCl_3$) $\delta$ :
4.25(1H,d,J=8.3Hz),5.40-5.44(1H,m),7.55(2H,t,J=7.8Hz),7.70-7.72(1H,m),7.99(2H,d,J=7.6Hz).

(2) Acetic acid 1-benzoyl-2,2,2-trifluoroethyl ester

[F322]

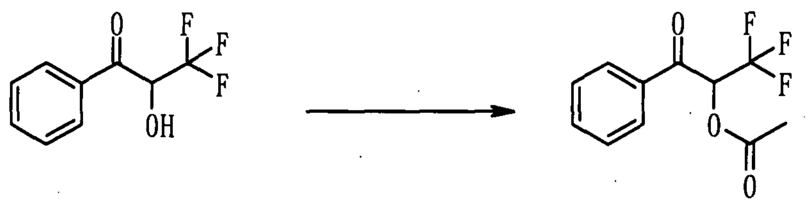

Dichloromethane (30 mL) was added to 3,3,3-trifluoro-2-hydroxy-1-phenylpropan-1-one (0.65 g), acetic anhydride (0.60 mL), and TEA (1.3 mL), and the resultant mixture was stirred overnight at room temperature. The reaction mixture was concentrated, and the residue was purified by silica gel column chromatography, whereby the title compound (0.71 g) was yielded.
$^1$H-NMR ($CDCl_3$) $\delta$ :
2.24(3H,s),6.29(1H,q,J=6.9Hz),7.52(2H,t,J=7.6Hz),7.66(1H,t,J= 7.6Hz),7.97(2H,d,J=7.6Hz).

(3) 2-Methyl-4-phenyl-5-trifluoromethyloxazole

[F323]

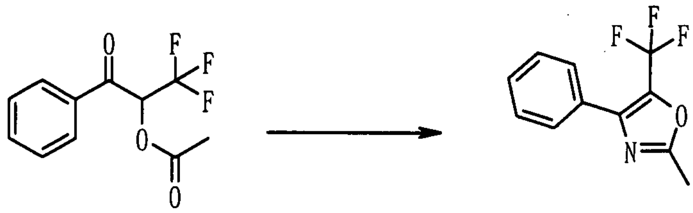

Acetic acid (5 mL) was added to acetic acid 1-benzoyl-2,2,2-trifluoroethyl ester (0.71 g) and ammonium acetate (0.67 g), and the resultant mixture was refluxed for 5 hours. The reaction temperature was cooled to room temperature, and saturated aqueous sodium bicarbonate solution was added to the reaction mixture, followed by extraction thrice, each with ethyl acetate. The extracts were combined and washed with saturated brine, followed by drying over sodium sulfate. Insoluble matter was removed by filtration, and the filtrate was concentrated. The residue was purified by silica gel column chromatography, whereby the title compound (0.25 g) was yielded.
$^1$H-NMR ($CDCl_3$) δ :
2.57(3H,s),7.41-7.47(3H,m),7.66-7.67(2H,m).

(4) 2-Bromomethyl-4-phenyl-5-trifluoromethyloxazole

[F324]

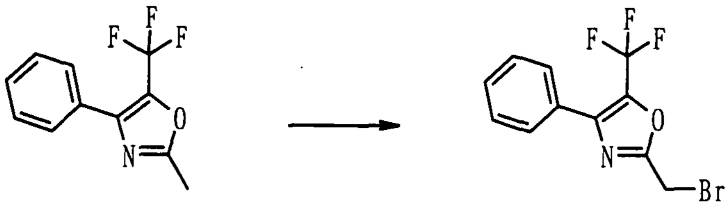

Carbon tetrachloride (10 mL) was added to 2-methyl-4-phenyl-5-trifluoromethyloxazole (0.25 g), benzoyl peroxide (0.030 g), and N-bromosuccinimide (0.20 g), and the resultant mixture was refluxed overnight. N-Bromosuccinimide (0.40 g) was further added to the reaction mixture, and the mixture was refluxed overnight. N-Bromosuccinimide (0.40 g) was further added to the resultant mixture, and the obtained mixture was refluxed for 3 days. The thus-obtained mixture was cooled to room temperature, and solvent was removed under reduced pressure. The residue was purified by silica gel column chromatography, whereby a mixture (0.14 g) of the title compound and impurities was yielded.

(5) 3-[N-[2-Oxo-2-[5-[(4-phenyl-5-trifluoromethyloxazol-2-yl)methoxy]-2,3-dihydro-1-yl]ethyl]amino]propionic acid

[F325]

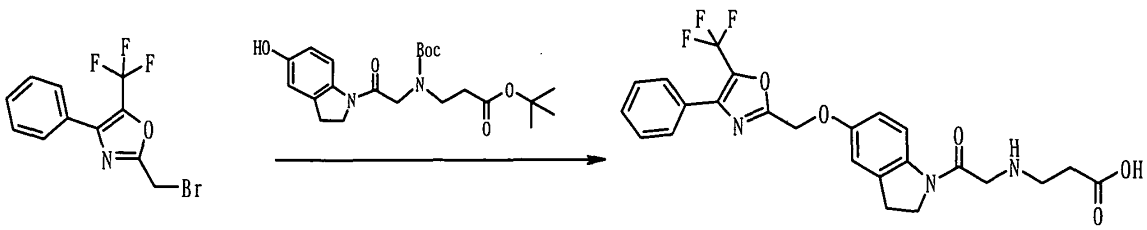

Potassium carbonate (0.12 g), 3-[N-tert-butoxycarbonyl-N-[2-(5-hydroxy-2,3-dihydroindol-1-yl)-2-oxoethyl] amino]propionic acid tert-butyl ester (0.12 g), and DMF (3 mL) were added to the above mixture (0.090 g) of 2-bromomethyl-4-phenyl-5-trifluoromethyloxazole and impurities, and the resultant suspension was stirred overnight at 80°C. The reaction mixture was cooled to room temperature and then concentrated. The residue was purified by silica gel column chromatography and then by thin-layer silica gel chromatography, whereby an oily product (0.070 g) was yielded. 10% TFA-dichloromethane (3 mL) was added to the oily product, and the resultant mixture was stirred overnight at room temperature. The reaction mixture was concentrated, whereby the title compound (0.062 g) was yielded.
$^1$H-NMR ($CD_3OD$) δ :
2.79-2.81(2H,m),3.24-3.28(2H,m),3.36-3.38(2H,m), 4.09(2H,t,J=8.3Hz), 4.14(2H,s), 5.28(2H,s), 6.93(1H,d ,J=9.1Hz), 7.05(1H,s), 7.48-7.49(3H,m), 7.67-7.68(2H,m), 8.08(1H,d,J=8.8Hz).
MS (ESI) ; m/z : 490 $(M+H)^+$.
Anal. Calcd for
$C_{24}H_{22}F_3N_3O_5 \cdot CF_3CO_2H$:C,51.75;H,3.84;N,6.96.Found:C,51.60;H,3.91; N,6.74.
IR(ATR)$cm^{-1}$:1660,1491,1188,1126,977,798.

[Example 75]

3-[N-[2-[5-(4-Cyclopropyl-5-trifluoromethylthiophen-2-ylmethoxy)-2,3-dihydroindol-1-yl]-2-oxoethyl]amino]propionic acid

(1) 4-Cyclopropylthiophene-2-carbaldehyde

[F326]

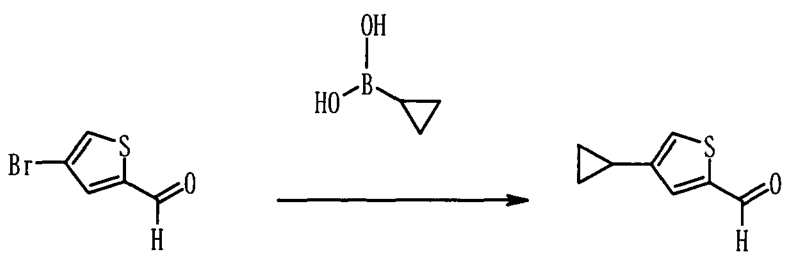

Water (25 mL) and toluene (50 mL) were added to 4-bromo-2-thiophenecarbaldehyde (2.0 g), tetrakis(triphenylphosphine)palladium(0) (1.2 g), cyclopropylboric acid (2.7 g), and cesium carbonate (17 g). The resultant mixture was stirred overnight at 80°C under a stream of nitrogen gas. The reaction mixture was cooled to room temperature, and water was added thereto, followed by extraction thrice, each with diethyl ether. The extracts were combined and washed with saturated brine, followed by drying over sodium sulfate. Insoluble matter was removed by filtration, and the filtrate was concentrated. The residue was purified by silica gel column chromatography, whereby the title compound (1.4 g) was yielded.

$^1$H-NMR ($CDCl_3$) δ :
0.66-0.68(2H,m), 0.97-0.99(2H,m), 1.94(1H,tt,J=8.4,3.9Hz), 7.31-7.31(1H,m), 7.50(1H,d,J=1.5Hz), 9.85(1H,dd, J=1.2,0.5Hz).

(2) 4-Cyclopropyl-5-iodothiophene-2-carbaldehyde

[F327]

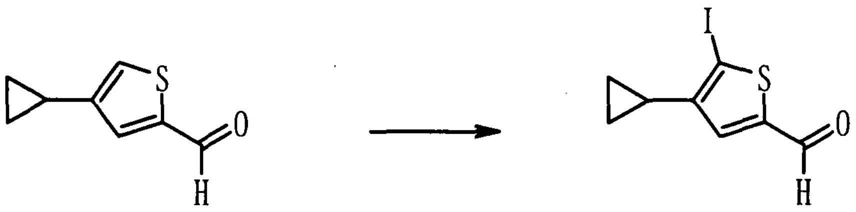

Acetic acid (3 mL) and chloroform (3 mL) were added to 4-cyclopropylthiophene-2-carbaldehyde (0.50 g) and N-iodosuccinimide (0.81 g), and the resultant mixture was stirred for 4 days at room temperature. The reaction mixture was concentrated, and the residue was purified by silica gel column chromatography, whereby the title compound (0.78 g) was yielded.
$^1$H-NMR ($CDCl_3$) δ :
0.62-0.70(2H,m),0.99-1.09(2H,m), 1.86(1H,tt,J=8.5,4.0Hz), 7.26(1H,d,J=0.5Hz), 9.71(1H , d, J=0.5Hz).

(3) 4-Cyclopropyl-5-trifluoromethylthiophene-2-carbaldehyde

[F328]

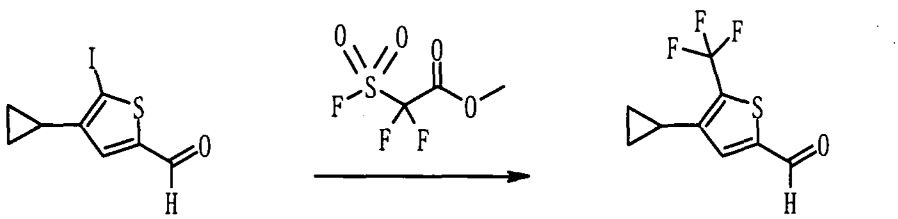

DMF (30 mL) was added to 4-cyclopropyl-5-iodothiophene-2-carbaldehyde (0.73 g), methyl 2,2-difluoro-2-(fluorosulfonyl)acetate (3.3 mL), and copper(I) iodide (0.50 g), and the resultant mixture was stirred for 4 hours at 90°C. The reaction mixture was cooled to room temperature, and saturated brine was added thereto, followed by extraction thrice, each with ethyl acetate. The extracts were combined and washed with saturated brine, followed by drying over sodium sulfate. Insoluble matter was removed by filtration, and the filtrate was concentrated. The residue was purified by silica gel column chromatography, whereby the title compound (0.57 g) was yielded.
$^1$H-NMR ($CDCl_3$) δ :
0.77-0.80(2H,m),1.10-1.15(2H,m),2.11-2.13(1H,m),7.26-7.26 (1H,m), 9.87 (1H, s).

(4) (4-Cyclopropyl-5-trifluoromethylthiophen-2-yl)methanol

[F329]

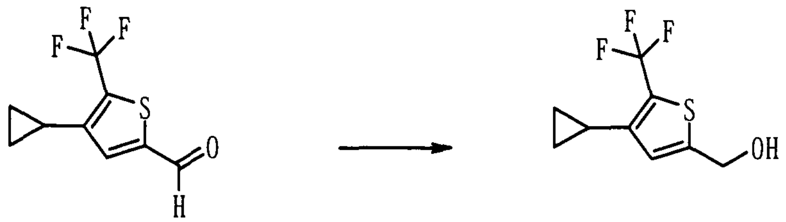

Sodium borohydride (0.12 g) was added to a solution (25 mL) of 4-cyclopropyl-5-trifluoromethylthiophene-2-carbaldehyde (0.57 g) in methanol, and the resultant mixture was stirred for 1 hour at room temperature. The reaction mixture was concentrated, and water was added thereto, followed by extraction thrice, each with ethyl acetate. The extracts were combined and washed with saturated brine, followed by drying over sodium sulfate. Insoluble matter was removed by filtration, and the filtrate was concentrated.
The residue was purified by silica gel column chromatography, whereby the title compound (0.56 g) was yielded.
$^1$H-NMR ($CDCl_3$) $\delta$:
0.70-0.72(2H,m),1.00-1.05(2H,m),1.84(1H,t,J=6.0Hz),2.09-2.09 (1H,m), 4.75-4.76 (2H,m), 6.49 (1H, s).

(5) 3-[N-[2-[5-(4-Cyclopropyl-5-trifluoromethylthiophen-2-ylmethoxy)-2,3-dihydroindol-1-yl]-2-oxoethyl]amino]propionic acid

[F330]

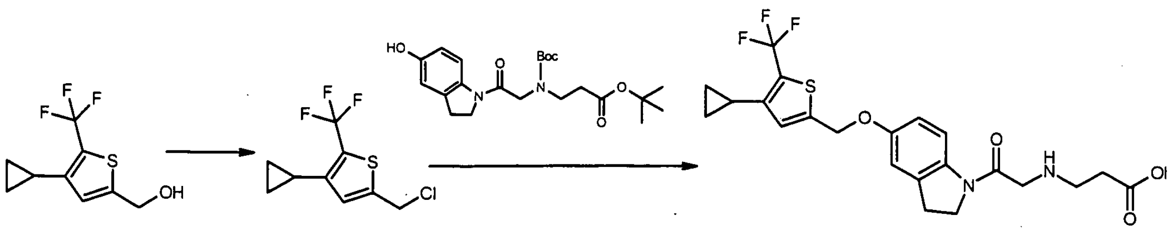

Thionyl chloride (0.13 mL) and DMF (catalytic amount) were added to a solution (5 mL) of (4-cyclopropyl-5-trifluoromethylthiophen-2-yl)methanol (0.080 g) in 1,2-dichloroethane, and the resultant mixture was stirred for 1 hour at 60°C. The reaction mixture was cooled to room temperature and then concentrated. To an aliquot (0.06 g) of the residue, potassium carbonate (0.10 g), 3-[N-tert-butoxycarbonyl-N-[2-(5-hydroxy-2,3-dihydroindol-1-yl)-2-oxoethyl]amino]propionic acid tert-butyl ester (0.10 g), and DMF (3 mL) were added, and the resultant suspension was stirred overnight at 80°C. The reaction mixture was cooled to room temperature and then concentrated. The residue was purified by silica gel column chromatography, whereby an oily product (0.14 g) was yielded. 10% TFA-dichloromethane (3 mL) was added to the oily product, and the resultant mixture was stirred overnight at room temperature. The reaction mixture was concentrated, and the residue was purified by reverse phase preparative HPLC, whereby the title compound (0.050 g) was yielded. $^1$H-NMR ($CD_3OD$) $\delta$:
0.74-0.76(2H,m),1.02-1.05(2H,m),2.05-2.08(1H,m),2.57(2H,t,J=6.5Hz),3.20-3.22(2H,m),4.06-4.08(4H,m),4.57-4.59 (2H,m),5.17(2H,s),6.73(1H,s),6.81-6.82(1H,m),6.92(1H,s),8.03(1H,d,J=8.8Hz).
MS(ESI); m/z:469(M+H)$^+$.

Anal. Calcd for $C_{22}H_{23}F_3N_2O_4S{\cdot}0.05CF_3CO_2H$, $0.5H_2O$:C, 54.93 ;H, 5.02;N, 5.80; F, 12.38; S ,6.64.Found:C,54.80;H,4.79;N,5.80;F, 12.43;S,6.53.
IR(ATR)$cm^{-1}$:1643,1491,1288,1109,1016,845.

[Example 76]

3-[N-[2-Oxo-2-[5-(4-phenyl-5-trifluoromethylthiazol-2-ylmethoxy)-2,3-dihydroindol-1-yl]ethyl]amino]propionic acid

(1) 5-Iodo-4-phenylthiazole-2-carboxylic acid ethyl ester

[F331]

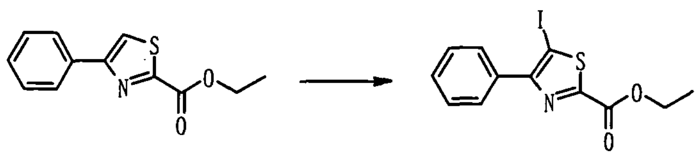

Acetic acid (3 mL) and chloroform (3 mL) were added to 4-phenylthiazole-2-carboxylic acid ethyl ester (0.50 g) and N-iodosuccinimide (0.53 g, 2.4. mmol), and the resultant mixture was stirred for 6 hours at 50°C. The reaction mixture was concentrated, and the residue was purified by silica gel column chromatography, whereby the title compound (0.60 g) was yielded.
$^1$H-NMR ($cDCl_3$) δ:
1.44(3H,t,J=7.1Hz),4.49(2H,q,J=7.1Hz),7.42-7.48(3H,m),7.86-7.87(2H,m).
MS(ESI);m/z:360$(M+H)^+$.

(2) 4-Phenyl-5-trifluoromethylthiazole-2-carboxylic acid ethyl ester

[F332]

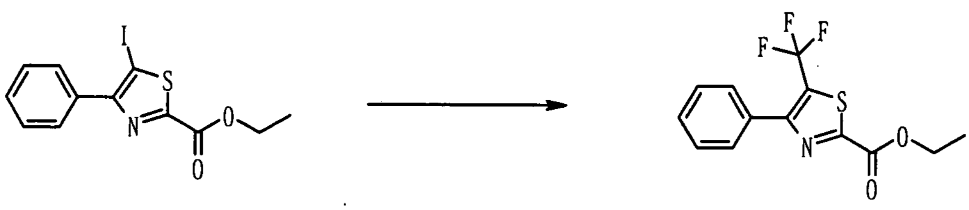

DMF (15 mL) was added to 5-iodo-4-phenylthiazole-2-carboxylic acid ethyl ester (0.60 g), 2,2-difluoro-2-(fluorosulfonyl)acetic acid methyl ester (2.1 mL), and copper(I) iodide (0.32 g), and the resultant mixture was stirred for 4 hours at 90°C. The reaction mixture was returned to room temperature, and saturated brine was added thereto, followed by extraction thrice, each with ethyl acetate. The extracts were combined and washed with saturated brine, followed by drying over sodium sulfate. Insoluble matter was removed by filtration, and the filtrate was concentrated. The residue was purified by silica gel column chromatography, whereby the title compound (0.44 g) was yielded.
$^1$H-NMR ($CDCl_3$) δ :
1.45-1.47(3H,m),4.52(2H,q,J=7.1Hz),7.46-7.47(3H,m),7.69-7.71(2H,m).
MS(ESI);m/z:302$(M+H)^+$.

(3) (4-Phenyl-5-trifluoromethylthiazol-2-yl)methanol

[F333]

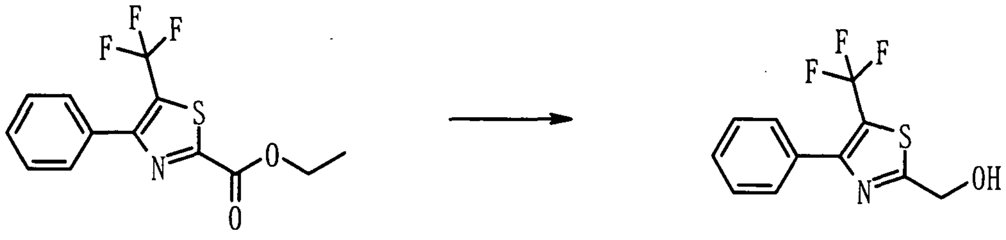

Sodium borohydride (0.16 g) was added to a solution (15 mL) of 4-phenyl-5-trifluoromethylthiazole-2-carboxylic acid ethyl ester (0.44 g) in methanol, and the resultant mixture was stirred overnight at room temperature. The reaction mixture was concentrated, and water was added thereto, followed by extraction thrice, each with ethyl acetate. The extracts were combined and washed with saturated brine, followed by drying over sodium sulfate. Insoluble matter was removed by filtration, and the filtrate was concentrated.
The residue was purified by silica gel column chromatography, whereby the title compound (0.32 g) was yielded.
MS(ESI) ;m/z:260 $(M+H)^+$.

(4) 3-[N-[2-Oxo-2-[5-(4-phenyl-5-trifluoromethylthiazol-2-ylmethoxy)-2,3-dihydroindol-1-yl]ethyl]amino]propionic acid

[F334]

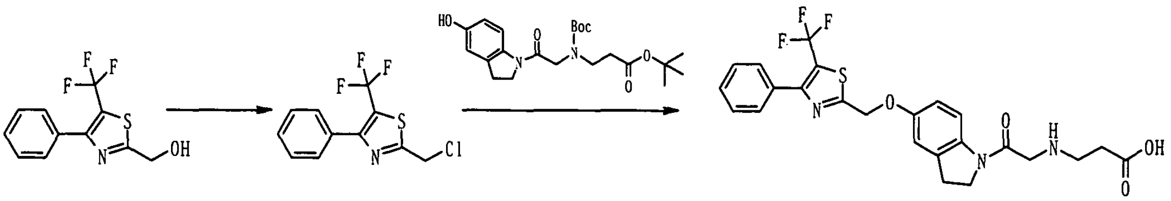

Thionyl chloride (0.17 mL, 2.3 mmol) and DMF (catalytic amount) were added to a solution (5 mL) of (4-phenyl-5-trifluoromethylthiazol-2-yl)methanol (0.12 g) in 1,2-dichloroethane, and the resultant mixture was stirred for 9 hours at room temperature. The reaction mixture was concentrated, and the residue was purified by thin-layer silica gel chromatography, whereby an oily product was yielded.
Potassium carbonate (0.12 g), 3-[N-tert-butoxycarbonyl-N-[2-(5-hydroxy-2,3-dihydroindol-1-yl)-2-oxoethyl]amino]propionic acid tert-butyl ester (0.12 g), and DMF (3 mL) were added to the above oily product, and the resultant suspension was stirred for 36 hours at room temperature. The reaction mixture was concentrated, and the residue was purified by silica gel column chromatography, whereby an oily product (0.13 g) was yielded. 10% TFA-dichloromethane (3 mL) was added to the oily product, and the resultant mixture was stirred overnight at room temperature. The reaction mixture was concentrated, and the residue was purified by reverse phase preparative HPLC, whereby the title compound (0.032 g) was yielded.
$^1H$-NMR ($CD_3OD$) δ:
2.57(2H,t,J=6.5Hz),3.26-3.27(4H,m),4.09-4.10(4H,m),5.45(2H,s), 6.94(1H,d,J=8.9Hz),7.05(1H,s),7.48(3H,t ,J=3.2Hz), 7.65(2H,d,J=3.2Hz),8.10(1H,d,J=8.9Hz).
MS(ESI) ;m/z:506$(M+H)^+$.
Anal. Calcd for
$C_{24}H_{22}F_3N_3O_4S \cdot 0.1CF_3CO_2H$, $1.5H_2O$: C, 53.44 ; H, 4.65 ; N, 7.73 ; F, 11.53 ; S, 5.90. Found:C,53.38; H,4.41; N,7.39; F,11.89; S,5.81.

IR(ATR)cm$^{-1}$:1666,1489,1344,1132,1016,704.

[Example 77]

3-[N-[2-[5-(4-Isobutyl-5-trifluoromethylthiophen-2-ylmethoxy)-2,3-dihydroindol-1-yl]-2-oxoethyl]amino]propionic acid

(1) 4-Isobutylthiophene-2-carbaldehyde

[F335]

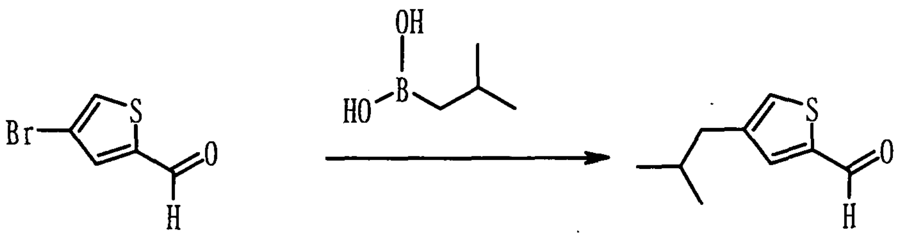

Water (25 mL) and toluene (50 mL) were added to 4-bromo-2-thiophenecarbaldehyde (2.0 g), tetrakis(triphenylphosphine)palladium(0) (1.2 g), 2-methylpropylboric acid (3.2 g), and cesium carbonate (17 g), and the resultant mixture was refluxed overnight with stirring under a stream of nitrogen gas. The reaction mixture was cooled to room temperature, and water was added thereto, followed by extraction thrice, each with diethyl ether. The extracts were combined and washed with saturated brine, followed by drying over sodium sulfate. Insoluble matter was removed through filtration, and the filtrate was concentrated. The residue was purified by silica gel column chromatography, whereby a mixture (1.4 g) containing the title compound was yielded.

(2) 5-Iodo-4-isobutylthiophene-2-carbaldehyde

[F336]

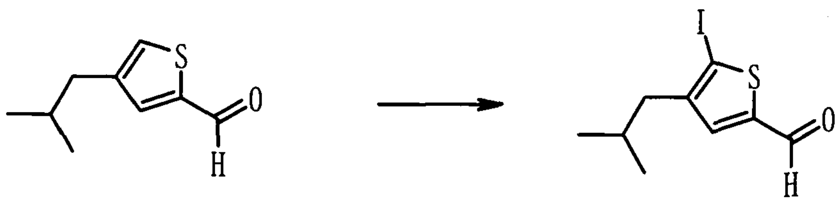

Acetic acid (3 mL) and chloroform (3 mL) were added to 4-isobutylthiophene-2-carbaldehyde (0.50 g) and N-iodosuccinimide (0.74 g), and the resultant mixture was stirred for 9 hours at 50°C. The reaction mixture was concentrated, and the residue was purified by silica gel column chromatography, whereby the title compound (0.68 g, by 2 steps) was yielded.
$^1$H-NMR ($CDCl_3$) $\delta$ :
0.96(6H,d,J=6.6Hz),1.90-1.97(1H,m),2.48(2H,d,J=7.1Hz), 7.32(1H,s),9.76(1H,d,J=0.5Hz).

(3) 4-Isobutyl-5-trifluoromethylthiophene-2-carbaldehyde

[F337]

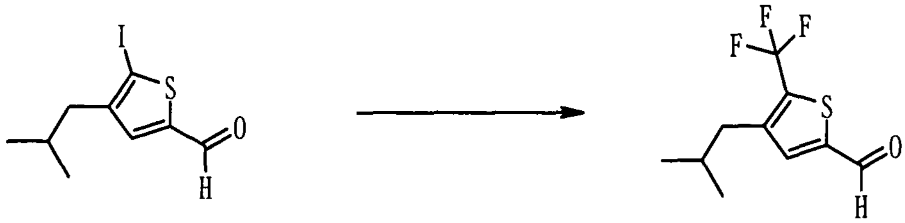

DMF (20 mL) was added to 5-iodo-4-isobutylthiophene-2-carbaldehyde (0.68 g), 2,2-difluoro-2-(fluorosulfonyl) acetic acid methyl ester (3.3 mL), and copper(I) iodide (0.44 g), and the resultant mixture was stirred for 4 hours at 80°C. The reaction mixture was cooled to room temperature, and saturated brine was added thereto, followed by extraction thrice, each with ethyl acetate. The extracts were combined and washed with saturated brine, followed by drying over sodium sulfate. Insoluble matter was removed by filtration, and the filtrate was concentrated. The residue was purified by silica gel column chromatography, whereby the title compound (0.38 g) was yielded.
$^1$H-NMR ($CDCl_3$ ) δ :
0.95(6H,d,J=6.6Hz),1.92-1.99(1H,m),2.63(2H,d,J=7.4Hz),7.58-7.58 (1H, m), 9.92 (1H,s).

(4) 4-Isobutyl-5-trifluoromethylthiophene-2-methanol

[F338]

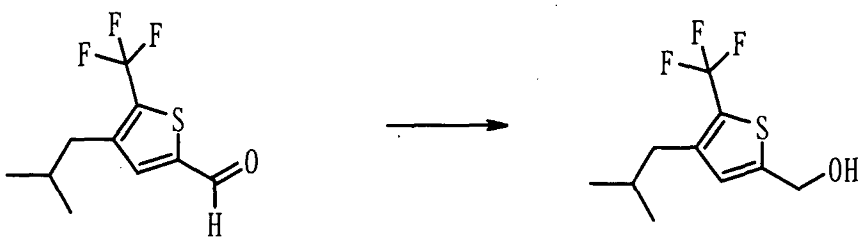

Sodium borohydride (0.070 g) was added to a solution (15 mL) of 4-isobutyl-5-trifluoromethylthiophene-2-carbaldehyde (0.38 g) in methanol, and the resultant mixture was stirred for 1 hour at room temperature. The reaction mixture was concentrated, and water was added thereto, followed by extraction thrice, each with ethyl acetate. The extracts were combined and washed with saturated brine, followed by drying over sodium sulfate. Insoluble matter was removed by filtration, and the filtrate was concentrated.
The residue was purified by silica gel column chromatography, whereby the title compound (0.36 g) was yielded.
$^1$H-NMR ($CDCl_3$) δ :
0.92(H,d,J=6.6Hz),1:84-1.94(2H,m),2.54-2.56 (2H,m), 4.80 (2H,d, J=5.9Hz), 6.82 (1H,s).

(5) 3-[N-[2-[5-(4-Isobutyl-5-trifluoromethylthiophen-2-ylmethoxy)-2,3-dihydroindol-1-yl]-2-oxoethyl]amino]propionic acid

[F339]

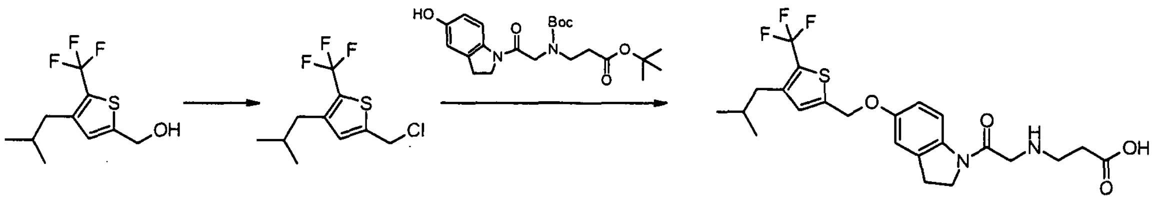

Thionyl chloride (0.090 mL) and DMF (catalytic amount) were added to a solution (5 mL) of 4-isobutyl-5-trifluoromethylthiophene-2-methanol (0.060 g) in 1,2-dichloroethane, and the resultant mixture was stirred for 1 hour at 60°C. The reaction mixture was cooled to room temperature and then concentrated. Potassium carbonate (0.10 g), 3-[N-(tert-butoxycarbonyl)-N-[2-(5-hydroxy-2,3-dihydroindol-1-yl)-2-oxoethyl]amino]propionic acid tert-butyl ester (0.10 g), and DMF (3 mL) were added to the residue, and the resultant suspension was stirred overnight at 80°C. The reaction mixture was cooled to room temperature and then concentrated. The residue was purified by silica gel column chromatography, whereby an oily product (0.13 g) was yielded. 10% TFA-dichloromethane (3 mL) was added to the oily product, and the resultant mixture was stirred overnight at room temperature. The reaction mixture was concentrated, and the residue was purified by reverse phase preparative HPLC, whereby the title compound (0.049 g) was yielded.

$^1$H-NMR ($CD_3OD$) δ:

0.91 (6H, d, J=6.6Hz), 1.91-1.96 (1H, m), 2.56-2.61 (4H, m), 3.21-3.28 (4H, m), 4.06-4.10 (4H, m), 5.23 (2H, s), 6.84 (1H, d, J=8.8Hz),6.94(1H,s),7.05(1H,s ), 8.04 (1H, d,J=8.8Hz).

MS(ESI);m/z:485(M+H)$^+$.

Anal. Calcd for

$C_{23}H_{27}F_3N_2O_4S\cdot0.1CF_3CO_2H, 0.75H_2O$:C, 54.70 ;H, 5.66 ; N, 5.50 ; F, 12.31; S ,6.29.Found: C,54.71; H,5.44; N,5.32; F,12.29; S,6.17.

IR (ATR) cm$^{-1}$: 2960, 1643, 1491, 1302, 1111, 1016.

[Example 78]

3-[N-[2-[5-(4-Cyclohexyl-5-trifluoromethylthiophen-2-ylmethoxy)-2,3-dihydroindol-1-yl]-2-oxoethyl]amino]propionic acid

(1) 4-(1-Cyclohexenyl)thiophene-2-carbaldehyde

[F340]

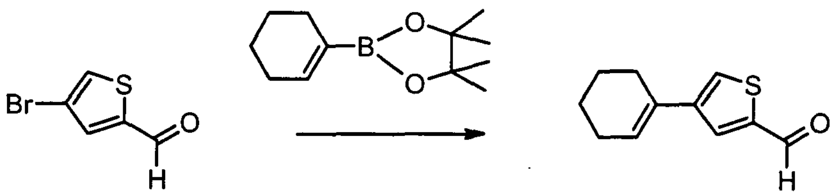

Water (15 mL) and toluene (30 mL) were added to 4-bromo-2-thiophenecarbaldehyde (0.50 g), tetrakis(triphenylphosphine)palladium(0) (0.30 g), 1-cyclohexen-1-ylboric acid pinacol ester (1.1 g), and cesium carbonate (4.3 g), and the resultant mixture was refluxed overnight with stirring under a stream of nitrogen gas. The reaction mixture was cooled to room temperature, and water was added thereto, followed by extraction thrice, each with diethyl ether. The extracts were combined and washed with saturated brine, followed by drying over sodium sulfate. Insoluble matter was removed by filtration, and the filtrate was concentrated. The residue was purified by silica gel column chromatography, whereby a mixture (0.56 g) containing the title compound was yielded.

(2) 4-Cyclohexylthiophene-2-carbaldehyde

[F341]

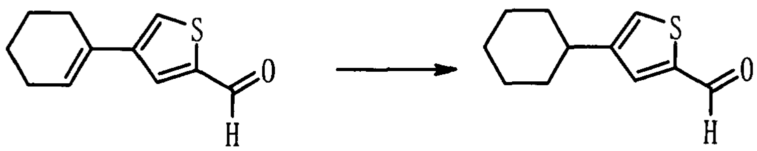

Methanol (30 mL) was added to 4-(1-cyclohexenyl)thiophene-2-carbaldehyde (0.56 g) and 5% Pd/C (0.10 g), and the resultant mixture was stirred overnight under a hydrogen atmosphere at room temperature. Nitrogen was passed through the reaction mixture, and 5% Pd/C (0.10 g) was further added thereto, followed by stirring for 3 days under a hydrogen atmosphere at room temperature. The reaction mixture was filtered, and the filtrate was concentrated. The residue was purified by thin-layer silica gel column chromatography, whereby the title compound (0.29 g) was yielded.
$^1$H-NMR($CDCl_3$)δ:
1.22-1.43 (5H, m), 1.72-1.85 (3H, m), 1.99-2.03 (2H, m), 2.61-2.64 (1H, m), 7.39 (1H, s), 7.66 (1H, d, J=1.4Hz), 9.88 (1H, d, J=1.4Hz).

(3) 4-Cyclohexyl-5-iodothiophene-2-carbaldehyde

[F342]

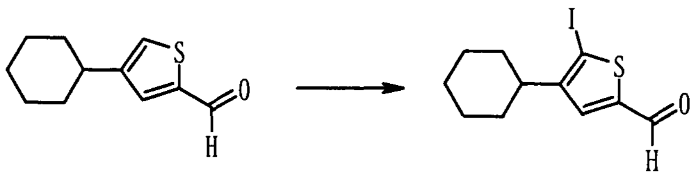

Acetic acid (5 mL) and chloroform (5 mL) were added to 4-cyclohexylthiophene-2-carbaldehyde (0.29 g) and N-iodosuccinimide (0.37 g), and the resultant mixture was stirred for 6 hours at 50°C. The reaction mixture was concentrated, and the residue was purified by silica gel column chromatography, whereby the title compound (0.37 g) was yielded.
$^1$H-NMR ($CDCl_3$) δ :
1.24-1.45(5H,m),1.77-1.79(1H,m),1.85-1.88(4H,m),2.56-2.59(1H,m),7.37(1H,s),9.76(1H,s).

(4) 4-Cyclohexyl-5-trifluoromethylthiophene-2-carbaldehyde

[F343]

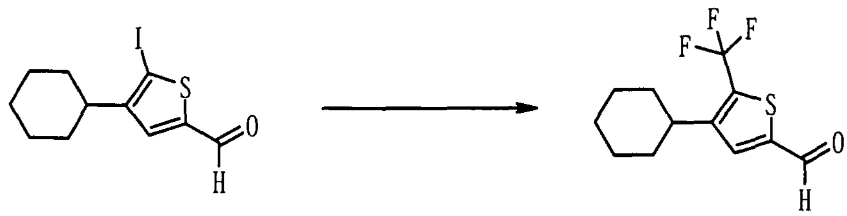

DMF (10 mL) was added to 4-cyclohexyl-5-iodo-thiophene-2-carbaldehyde (0.37 g), 2,2-difluoro-2-(fluorosulfonyl)acetic acid methyl ester (1.5 mL), and copper(I) iodide (0.22 g), and the resultant mixture was stirred at 80°C. Four hours later, the reaction mixture was cooled to room temperature, and saturated brine was added thereto, followed by extraction thrice, each with ethyl acetate. The organic layers were combined and washed with saturated brine, followed by drying over sodium sulfate anhydrate. Insoluble matter was removed by filtration, and the filtrate was concentrated. The residue was purified by silica gel column chromatography, whereby the title compound (0.28 g) was yielded.
$^1$H-NMR ($CDCl_3$) δ:
1.23-1.47(5H,m),1.76-1.91(5H,m),2.86-2.93(1H,m),7.70(1H,q,J=1.5Hz),9.92(1H,s).

(5) 4-Cyclohexyl-5-trifluoromethylthiophene-2-methanol

[F344]

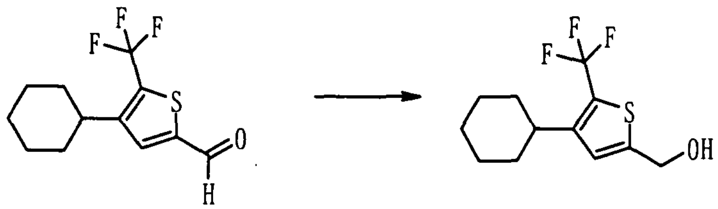

Sodium borohydride (0.050 g) was added to a solution (10 mL) of 4-cyclohexyl-5-trifluoromethylthiophene-2-carbaldehyde (0.28 g) in methanol, and the resultant mixture was stirred for 1 hour at room temperature. The reaction mixture was concentrated, and water was added thereto, followed by extraction thrice, each with ethyl acetate. The organic layers were combined and washed with saturated brine, followed by drying over sodium sulfate anhydrate. Insoluble matter was removed by filtration, and the filtrate was concentrated. The residue was purified by silica gel column chromatography, whereby the title compound (0.27 g) was yielded.
$^1$H-NMR ($CDCl_3$)δ :
1.33-1.42(4H,m),1.73-1.85(6H,m),2.82-2.88(1H,m),4.80(2H,dt,J=6.1,0.9Hz),6.94(1H,d,J=0.7Hz).

(6) 3-[N-[2-[5-(4-Cyclohexyl-5-trifluoromethylthiophen-2-ylmethoxy)-2,3-dihydroindol-1-yl]-2-oxoethyl]amino]propionic acid

[F345]

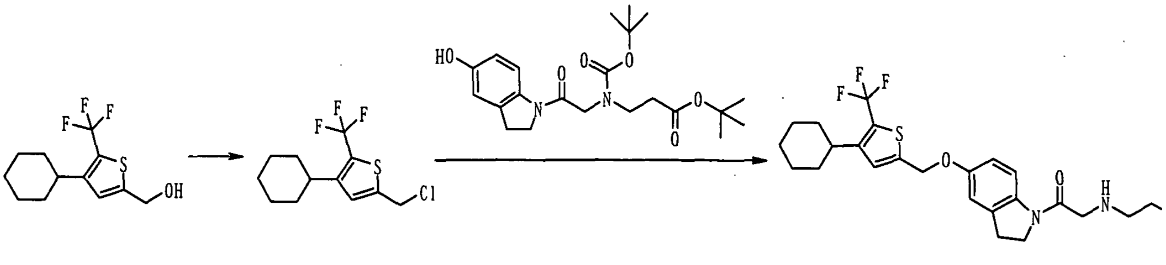

Thionyl chloride (0.090 mL) and DMF (trace) were added to a solution (5 mL) of 4-cyclohexyl-5-trifluoromethylthiophene-2-methanol (0.060 g) in 1,2-dichloroethane, and the resultant mixture was stirred for 1 hour at 60°C. The reaction mixture was cooled to room temperature and then concentrated. Potassium carbonate (0.10 g), 3-[N-(tert-butoxycarbonyl)-N-[2-(5-hydroxy-2,3-dihydroindol-1-yl)-2-oxoethyl]amino]propionic acid tert-butyl ester (0.10 g), and DMF (3 mL) were added to the residue, and the resultant suspension was stirred overnight at 80°C. The reaction mixture was cooled to room temperature and then concentrated. The residue was purified by silica gel column chromatography, whereby an oily product (0.16 g) was yielded. 10% TFA-dichloromethane (3 mL) was added to the oily product, and the resultant mixture was stirred overnight at room temperature. The reaction mixture was concentrated, and the residue was purified by reverse phase preparative HPLC, whereby the title compound (0.059 g) was yielded.
$^1$H -NMR ($CD_3OD$) δ:
1.28-1.52(5H,m),1.77-1.83(5H,m),2.61(2H,t,J=6.5Hz),2.83-2.86 (1H,m),3.22-3.26 (4H, m),4.07-4.12 (4H,m), 5.22 (2H, s), 6.84 (1H, d, J=8.8Hz), 6.94 (1H, s), 7.17 (1H,s ), 8.05 (1H, d, J=8.8Hz).
MS (ESI) ;m/z:511 $(M+H)^+$.
Anal. Calcd for
$C_{25}H_{29}F_3N_2O_4S$.0.1$CF_3CO_2H \cdot H_2O$:C,56.05;H,5.81;N, 5.19; F, 11.61; S,5.9 4.Found: C,56.14;H,5.81; N,4.97;F,11.43; S,5.94.
IR(ATR)$cm^{-1}$: 2929,1658,1491,1113,1014,841.

[Example 79]

3-[N-[2-[5-(2-Chlorobiphenyl-4-ylmethoxy)indolin-1-yl]-2-oxoethyl]amino]propionic acid hydrochloride

(1) 3-Chloro-4-trifluoromethylsulfonyloxybenzoic acid methyl ester

[F346]

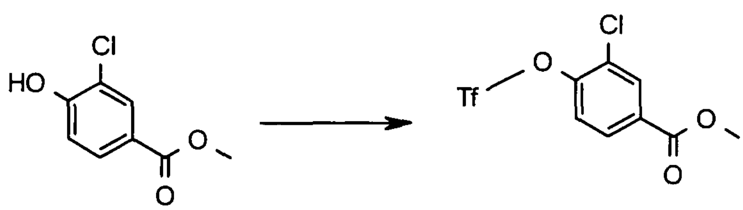

TEA (837 μL) was added to a solution (20 mL) of 3-chloro-4-hydroxybenzoic acid methyl ester (560 mg) in dichloromethane, and the resultant mixture was cooled at 0°C. Trifluoromethanesulfonic anhydride (555 μL) was added to the mixture, and the resultant mixture was stirred for 5 hours at room temperature. Saturated aqueous sodium bicarbonate solution was added to the reaction mixture, and the resultant mixture was extracted thrice, each with chloroform. The organic layer was washed with 1N hydrochloric acid and saturated brine, dried over sodium sulfate anhydrate, and then concentrated. The residue was purified by flash column chromatography (Yamazen Hi-Flash column 2L), whereby the title compound (808 mg) was yielded.
$^1$H-NMR ($CDCl_3$) δ:

3.95 (3H,s),7.44(1H,d,J=8.7Hz),8.03(1H,dd,J=2.1,8.7Hz),8.21(1H , d, J=2.1Hz).
MS (ESI)m/z:319 $(M+H)^+$.

(2) 2-Chlorobiphenyl-4-carboxylic acid methyl ester

[F347]

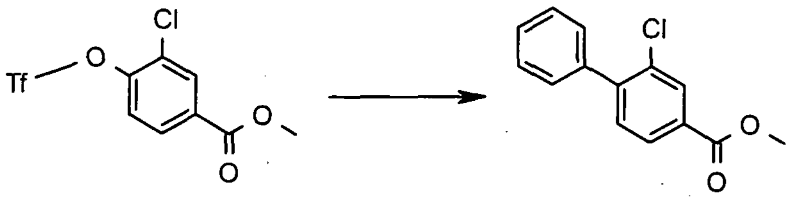

Phenylboronic acid (367 mg), potassium carbonate (1.04 g), water (4.0 mL), and tetrakis(triphenylphosphine) palladium(0) (145 mg) were added to a solution (15 mL) of 3-chloro-4-trifluoromethylsulfonyloxybenzoic acid methyl ester (800 mg) in toluene, and the resultant mixture was stirred for 3 hours under reflux. The reaction mixture was left to stand to cool to room temperature, and saturated aqueous sodium bicarbonate solution was added to the reaction mixture, followed by extraction twice, each with ethyl acetate. The extracts were combined and washed with saturated brine, followed by drying over sodium sulfate anhydrate and then concentration. The residue was purified by flash column chromatography (Yamazen Hi-Flash column 2L), whereby the title compound (552 mg) was yielded.
$^1$H-NMR ($CDCl_3$) δ:
3.95(3H,s),7.41-7.46(6H,m),7.97(1H,dd,J=1.7,8.1Hz),8.15(1H,d,J=1.7Hz).
MS(ESI)m/z:247$(M+H)^+$.

(3) 2-Chlorobiphenyl-4-carboxylic acid

[F348]

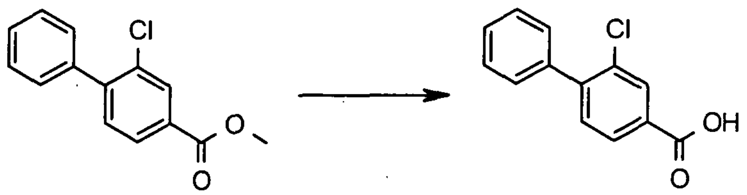

Methanol (5.0 mL) and 1N aqueous sodium hydroxide solution (5.08 mL, 5.08 mmol) were added to a solution (10 mL) of 2-chlorobiphenyl-4-carboxylic acid methyl ester (540 mg) in THF, and the resultant mixture was stirred overnight at room temperature. 1N Hydrochloric acid was added to the reaction mixture, and only the organic solvent was removed under reduced pressure. Precipitated solid was collected by filtration and dried, whereby the title compound (490 mg) was yielded.
$^1$H-NMR (DMSO-$d_6$) δ:
7.43-7.56(6H,m),8.03-7.94(2H,m).
MS (ESI) m/z : 233 $(M+H)^+$.

(4) 2-Chlorobiphenyl-4-methanol

[F349]

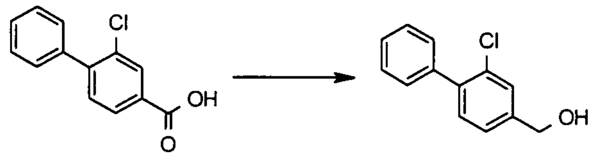

TEA (423 μL) was added to a solution (20 mL) of 2-chlorobiphenyl-4-carboxylic acid (470 mg) in THF, and the resultant mixture was cooled at 0°C. Ethyl chlorocarbonate (231 μL) was added to the reaction mixture, and the resultant mixture was stirred for 1 hour at that temperature The precipitate was removed by filtration. Sodium borohydride (459 mg) was suspended to ethanol (6.0 mL), and the suspension was cooled with ice. The filtrate obtained above was added dropwise to the suspension over 20 minutes, and the resultant mixture was stirred for 1 hour at room temperature. 1N Hydrochloric acid was added to the reaction mixture, and the resultant mixture was extracted twice, each with ethyl acetate. The extracts were combined and washed sequentially with 1N aqueous sodium hydroxide solution and saturated brine, followed by drying over sodium sulfate anhydrate. Solvent was evaporated, and the residue was purified by flash column chromatography (Yamazen Hi-Flash column L), whereby the title compound (436 mg) was yielded.
$^1$H-NMR ($CDCl_3$) δ:
1.75-1.81(1H,m), 4.73(2H,d,J=5.9Hz), 7.30-7.50(8H,m).
MS (ESI) m/z : 219 $(M+H)^+$.

(5) 2-Chloro-4-chloromethylbiphenyl

[F350]

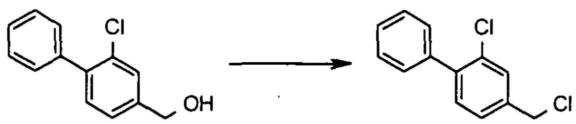

Thionyl chloride (713 μL) was added to a solution (20 mL) of 2-chlorobiphenyl-4-methanol (430 mg) in 1,2-dichloroethane, and the resultant solution was stirred for 2 hours at 50°C. The reaction mixture was left to stand to cool to room temperature and then concentrated. The residue was purified by flash column chromatography (Yamazen Hi-Flash column L), whereby the title compound (394 mg) was yielded.
$^1$H-NMR($CDCl_3$) δ:
4.59(2H,s),7.52-7.25(8H,m).

(6) 3-[N-(tert-Butoxycarbonyl)-N-[2-[5-(2-chlorobiphenyl-4-ylmethoxy)indolin-1-yl]-2-oxoethyl]amino]propionic acid tert-butyl ester

[F351]

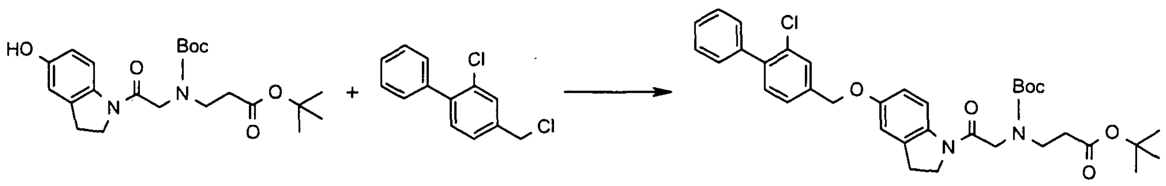

2-Chloro-4-chloromethylbiphenyl (142 mg) and potassium carbonate (104 mg) were added to a solution (5.0 mL) of 3-[N-(tert-butoxycarbonyl)-[2-(5-hydroxyindolin-1-yl)-2-oxoethyl]amino]propionic acid tert-butyl ester (210 mg) in DMF, and the resultant mixture was stirred overnight at 70°C. The reaction mixture was left to stand to cool to room temperature, saturated aqueous sodium bicarbonate solution was added to the reaction mixture, followed by extraction twice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column L), whereby the title compound (318 mg) was yielded.
$^1$H-NMR ($CDCl_3$) δ :
1.41-1.50(18H,m),2.56-2.63(2H,m),3.17-3.23(2H,m),3.56-3.62 (2H,m), 4.00-4.18(4H,m), 5.04(2H,s), 6.78-6.85(2H,m), 7.26-7.55 (8H,m), 8.11-8.16(1H,m).
MS (ESI) m/z : 621 $(M+H)^+$.

(2) 3-[N-[2-[5-(2-Chlorobiphenyl-4-ylmethoxy)indolin-1-yl]-2-oxoethyl]amino]propionic acid hydrochloride

[F352]

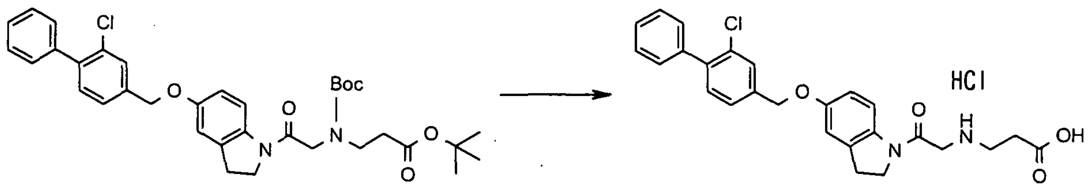

4N HCl/1,4-dioxane (5.0 mL) was added to 3-[N-(tert-butoxycarbonyl)-N-[2-[5-(2-chlorobiphenyl-4-ylmethoxy) indolin-1-yl]-2-oxoethyl]amino]propionic acid tert-butyl ester (311 mg), and the resultant mixture was stirred overnight at room temperature. Diethyl ether was added to the reaction mixture, and precipitated solid was collected by filtration. The solid was suspended in acetonitrile, collected by filtration, and dried, whereby the title compound (210 mg) was yielded.
$^1$H-NMR (DMSO-$d_6$) δ:
2.75-2.81(2H,m),3.17-3.22(4H,m),4.05-4.14(4H,m),5.16(2H,s),6.89-7.04(2H,m),7.40-7.50(7H,m), 7.63(1H,s), 7.98(1H, d,J=8.6Hz).
IR (ATR) $cm^{-1}$: 2736, 1708, 1670, 1496, 1444, 1270. 1238.
MS (ESI) m/z :465 $(M+H)^+$.
HR-MS(FAB)calcd for $C_{26}H_{26}ClN_2O_4$ $(M+H)^+$:465.1581; found 465.1562. Anal. Calcd for $C_{26}H_{25}ClN_2O_4$ -HCl: C, 62. 28; H,5. 23;Cl, 14.14 ;N, 5.59. Found: C, 62. 07;H ,5.16;Cl, 14.03;N, 5.48.

[Example 80]

3-[N-[2-[5-(4-Isobutyl-3-trifluoromethylbenzyloxy)indolin-1-yl]-2-oxoethyl]amino]propionic acid hydrochloride

(1) 4-Isobutyl-3-trifluoromethylbenzoic acid methyl ester

[F353]

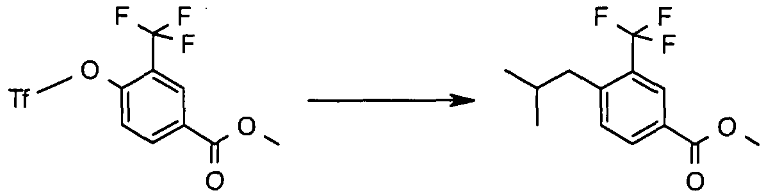

Isobutylboronic acid (1.68 g), cesium carbonate (8.93 g), water (15 mL), and tetrakis (triphenylphosphine) palladium (0) (633 mg) were added to a solution (30 mL) of 4-trifluoromethanesulfonyloxy-3-trifluoromethylbenzoic acid methyl ester (1.93 g) in toluene, and the resultant mixture was stirred overnight at reflux. The reaction mixture was left to stand to cool to room temperature and then filtered through a Celite pad. Saturated aqueous sodium bicarbonate solution was added to the filtrate, and the resultant mixture was extracted twice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column 2L), whereby the title compound (1.35 g) was yielded.
$^1$H-NMR ($CDCl_3$) δ :
0.93(6H,d,J=6.6Hz),1.93-2.03(1H,m),2.71(2H,d,J=7.4Hz),3.93(3H,s),7.40(1H,d,J=8.1Hz),8 .11(1H,dd,J=1.6,8.1Hz), 8.30(1H,d,J=1.6Hz).
MS (ESI) m/z : 261 $(M+H)^+$.

(2) (4-Isobutyl-3-trifluoromethylphenyl)methanol

[F354]

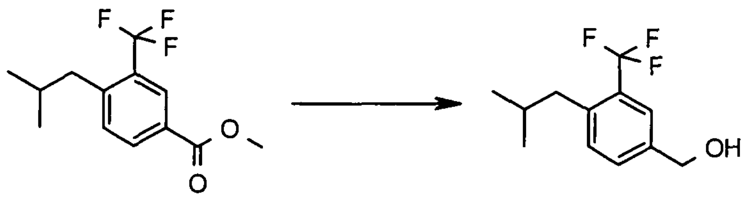

Lithium borohydride (336 mg) was added to a solution (40 mL) of 4-isobutyl-3-trifluoromethylbenzoic acid methyl ester (1.34 g) in THF, and the resultant mixture was stirred for 7 hours under reflux. The reaction mixture was left to stand to cool to room temperature, and 1N hydrochloric acid was added to the reaction mixture, followed by extraction twice, each with ethyl acetate. The extracts were combined and washed with saturated brine, followed by drying over sodium sulfate anhydrate. Insoluble matter was removed through filtration, and the filtrate was concentrated under reduced pressure. The residue was purified by flash column chromatography (Yamazen Hi-Flash column 2L), whereby the title compound (1.13 g) was yielded.
$^1$H-NMR ($CDCl_3$) δ :
0.93(6H,d,J=6.6Hz),1.74-1.78(1H,m),1.92-1.98 (1H, m), 2.65 (2H, d, J=7.1Hz), 4.72 (2H, d, J=5.6Hz), 7.31 (1H,d, J= 8.1Hz),7.46(1H,d,J=8.1Hz),7.63(1H,s).
MS (ESI) m/z : 215 $(M-OH)^+$.

(3) 4-Chloromethyl-1-isobutyl-2-trifluoromethylbenzene

[F355]

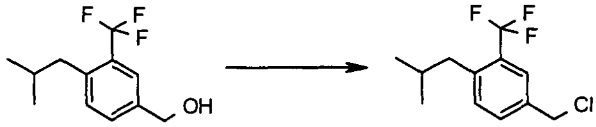

Thionyl chloride (1.75 mL) was added to a solution (50 mL) of (4-isobutyl-3-trifluoromethylphenyl)methanol (1.12 g) in 1,2-dichloroethane, and the resultant mixture was stirred for 2.5 hours at 50°C. The reaction mixture was left to stand to cool to room temperature and then concentrated under reduced pressure. The residue was purified by flash column chromatography (Yamazen Hi-Flash column 2L), whereby the title compound (1.06 g) was yielded.
$^1$H-NMR ($CDCl_3$) δ:
0.93(6H,d,J=6.6Hz),1.90-2.00(1H,m),2.65(2H,d,J=7.1Hz),4.59(2H,s),7.31(1H,d,J=7.8Hz),7 .48 (1H, dd, J=1.7,7.8Hz), 7.63 (1H, d, J=1.7Hz).

(4) 3-[N-(tert-Butoxycarbonyl)-N-[2-[5-(4-isobutyl-3-trifluoromethylbenzyloxy)indolin-1-yl]-2-oxoethyl]amino]propionic acid tert-butyl ester

[F356]

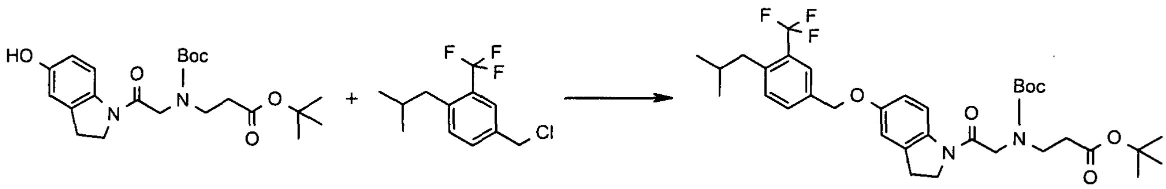

4-Chloromethyl-1-isobutyl-2-trifluoromethylbenzene (501 mg) and potassium carbonate (359 mg) were added to a solution (20 mL) of 3-[N-(tert-butoxycarbonyl)-N-[2-(5-hydroxyindolin-1-yl)-2-oxoethyl]amino]propionic acid tert-butyl ester (841 mg) in DMF, and the resultant mixture was stirred overnight at 60°C. The reaction mixture was left to stand to cool to room temperature, water was added to the reaction mixture. The resultant mixture was extracted thrice, each with ethyl acetate, and the extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column 2L), whereby the title compound (1.29 g) was yielded. $^1$H-NMR ($CDCl_3$) δ :
0.93(6H,d,J=6.6Hz),1.40-1.50(18H,m),1.92-1.99(1H,m),2.55-2.67(4H,m),3.16-3.22(2H,m),3.56-3.61(2H,m),3.99-4.18 (4H,m),5.01(2H,s),6.76-6.83(2H,m),7.32(1H,d,J=7.6Hz),7.51(1H,d,J=7.6Hz),7.67(1H,s),8 10-8.15 (1H,m).
MS (ESI)m/z:636(M+2)$^+$.

(5) 3-[N-[2-[5-(4-Isobutyl-3-trifluoromethylbenzyloxy)indolin-1-yl]-2-oxoethyl]amino]propionic acid hydrochloride

[F357]

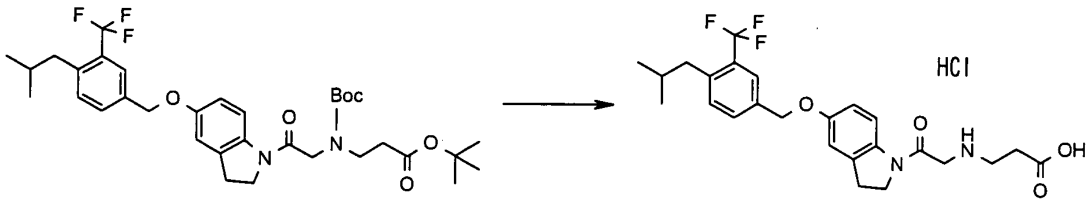

4N HCl/l,4-dioxane(20 mL) was added to 3-[N-(tert-butoxycarbonyl)-N-[2-[5-(4-isobutyl-3-trifluoromethylbenzyloxy)indolin-1-yl]-2-oxoethyl]amino]propionic acid tert-butyl ester (1.26 g), and the resultant mixture was stirred overnight at room temperature. The reaction mixture was concentrated under reduced pressure, and diethyl ether was added to the residue to form solid. Solvent was removed under reduced pressure, and the residue was recrystallized from acetonitrile, whereby the title compound (808 mg) was yielded.
$^1$H-NMR (DMSO-$d_6$) δ:
0.89(6H,d,J=6.6Hz),1.90-1.96(1H,m),2.63(2H,d,J=7.4Hz),2.77(2H,s),3.16-3.21(4H,m),4.05-4.13 (4H,m), 5.14 (2H,s), 6.88 (1H, dd, J=2.5,8.8Hz), 7.02 (1H,d, J=2.0 Hz), 7.49 (1H, d, J=8.1Hz), 7.65-7.74 (2H,m),7.97 (1H, d, J=8,8Hz),
IR(ATR)cm$^{-1}$:3332, 2958,1722,1646,1492,1319,1272,1108.
MS (ESI) m/z:479 $(M+H)^+$. HR-MS (ESI) calcd for $C_{25}H_{30}F_3N_2O_4(M+H)^+$:479.21577; found 479.21856.
Anal. Calcd for
$C_{25}H_{29}F_3N_2O_4 \cdot 4HCl \cdot 0.5H_2O$:C,57.31;H,5.96;Cl,6.77;F,10.88;N,5.35.F ound: C,57.23;H,5.93;Cl,6.85;F,10.94;N,5.24.

[Example 81]

3-[N-[2-[5-(4-Cyclopropyl-3-trifluoromethylbenzyloxy)indolin-1-yl]-2-oxoethyl]amino]propionic acid TFA salt

(1) 4-Cyclopropyl-3-trifluoromethylbenzoic acid methyl ester

[F358]

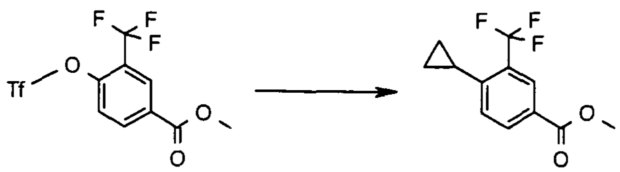

To a toluene solution (15 mL) of 4-trifluoromethanesulfonyloxy-3-trifluoromethylbenzoic acid methyl ester (1.06 g), cyclopropylboronic acid (773 mg), cesium carbonate (4.89 g), water (7.5 mL), and tetrakis(triphenylphosphine)palladium(0) (347 mg) were added, and the mixture was stirred for 3 hours at reflux. The resultant mixture was left to stand to cool to room temperature, and saturated aqueous sodium bicarbonate solution was added thereto, followed by extraction thrice, each with diethyl ether. The organic layer was washed with saturated brine and dried with sodium sulfate anhydrate. Insoluble matter was removed by filtration, and the filtrate was concentrated under reduced pressure. The residue was purified by flash column chromatography (Yamazen Hi-Flash column 2L), whereby the title compound (714 mg) was yielded.
$^1$H-NMR ($CDCl_3$) δ :
0.83-0.87(2H,m),1.11-1.16(2H,m),2.23-2.30(1H,m),3.93(3H,s),7.05(1H,d,J=8.3Hz),8.07(1H,dd,J=1.2,8.3 Hz),8.28(1H,d,J=1.2Hz).
MS(ESI) m/z:245 $(M+H)^+$.

(2) (4-cyclopropyl-3-trifluoromethylphenyl)methanol

[F359]

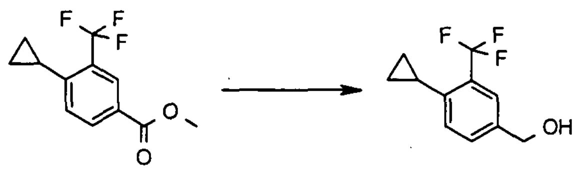

To a THF solution (20 mL) of 4-cyclopropyl-3-trifluoromethylbenzoic acid methyl ester (705 mg), lithium borohydride (189 mg) was added, and the mixture was stirred for 2.5 hours at reflux. The resultant mixture was left to stand to cool to room temperature, and 1N hydrochloric acid was added thereto, followed by extraction thrice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column L), whereby the title compound (611 mg) was yielded. $^1$H-NMR ($CDCl_3$) δ :
0.74-0.78(2H,m),1.01-1.06(2H,m),1.73(1H,t,J=5.9Hz),2.16-2.24(1H,m), 4.70(2H,d,J=5.9Hz), 7.03(1H,d,J=7.8Hz), 7.41-7.43(1H,m), 7.61-7.62(1H,m).
MS (ESI) m/z : 199 $(M-OH)^+$.

(3) 4-Chloromethyl-1-cyclopropyl-2-trifluoromethylbenzene

[F360]

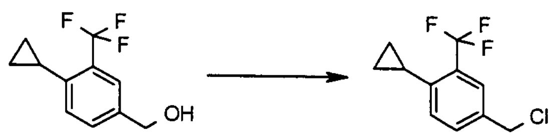

To a 1,2-dichloroethane solution (20 mL) of (4-cyclopropyl-3-trifluoromethylphenyl)methanol (600 mg), thionyl chloride (1.01 mL) was added, and the mixture was stirred for 1 hour at 50°C. The resultant mixture was left to stand to cool to room temperature and concentrated under reduced pressure. The residue was purified by flash column chromatography (Yamazen Hi-Flash column L), whereby the title compound (630 mg) was yielded.
$^1$H-NMR($CDCl_3$)δ:
0.75-0.79(2H,m),1.03-1.08(2H,m),2.17-2.23(1H,m),4.58(2H,s),7.02(1H,d,J=8.1Hz),7.45(1H,dd,J=1.7,8.1 Hz),7.62(1H, d,J=1.7Hz).

(4) 3-[N-(tert-Butoxycarbonyl)-N-[2-[5-(4-cyclopropyl-3-trifluoromethylbenzyloxy)indolin-1-yl]-2-oxoethyl]amino]propionic acid tert-butyl ester

[F361]

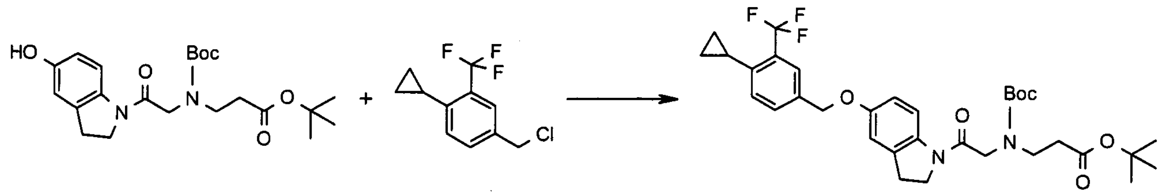

To a DMF solution (10 mL) of 3-[N-(tert-butoxycarbonyl)-N-[2-(5-hydroxyindolin-1-yl)-2-oxoethyl]amino]propionic acid tert-butyl ester (252 mg), 4-chloromethyl-1-cyclopropyl-2-trifluoromethylbenzene (125 mg) and potassium carbonate (104 mg) were added, and the mixture was stirred overnight at 60°C. The resultant mixture was left to stand to cool to room temperature, and water was added thereto. The resultant mixture was extracted thrice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column 2L), whereby the title compound (317 mg) was yielded.
$^1$H-NMR ($CDCl_3$) δ :
0.75-0.79(2H,m), 1.02-1.06(2H,m),1.40-1.50(18H,m),2.17-2.24(1H,m), 2.55-2.63(2H,m),3.15-3.22(2H,m),3.56-3.61 (2H,m), 3.99-4.18(4H,m), 5.00(2H,s),6.74-6.82(2H,m),7.04(1H,d,J=8.1Hz),7.47(1H,d,J=8.1Hz),7.66(1H,s),8 10-8.14 (1H,m).
MS (ESI) m/z : 619 $(M+H)^+$

(5) 3-[N-[2-[5-(4-Cyclopropyl-3-trifluoromethylbenzyloxy)indolin-1-yl]-2-oxoethyl]amino]propionic acid TFA salt

[F362]

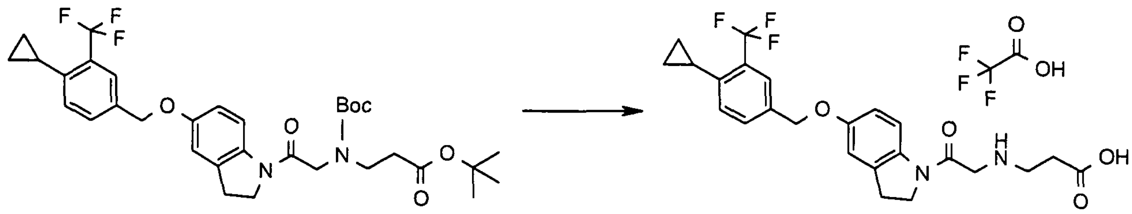

To a dichloromethane solution (8.0 mL) of 3-[N-(tert-butoxycarbonyl)-N-[2-[5-(4-cyclopropyl-3-trifluoromethylbenzyloxy)indolin-1-yl]-2-oxoethyl]amino]propionic acid tert-butyl ester (309 mg), TFA (2.0 mL) was added at 0°C, and the mixture was stirred for 1.5 hours at room temperature. TFA (1.0 mL) was further added thereto, followed by stirring for 2 hours at room temperature. The resultant mixture was concentrated under reduced pressure. The residue was suspended in diethyl ether for a while, collected by filtration, and dried, whereby the title compound (284 mg) was yielded.
$^1$H-NMR (DMSO-$d_6$) δ:
0.78-0.82(2H,m),1.02-1.07(2H,m),2.08-2.15(1H,m),2.73(2H,t,J=7.4Hz),3.16-3.22(4H,m),4.03-4.14(4H,m),5.12(2H,s), 6.87(1H,dd,J=2.7,8.8Hz),7.00(1H,d,J=2.7 Hz), 7.18 (1H, d, J=8.1Hz), 7.61 (1H, d, J=8.1Hz), 7.72 (1H, d, J=1.5Hz), 7.96 (1H,d,J=8.7Hz).
IR(ATR)$cm^{-1}$:1648,1492,1317,1255,1180,1130,1112.
MS (ESI)m/z:463 $(M+H)^+$.
HR-MS (ESI) calcd for $C_{24}H_{26}F_3N_2O_4$ $(M+H)^+$:463.18447; found 463.18301.
Anal. Calcd for $C_{24}H_{25}F_3N_2O_4 \cdot CF_3CO_2H \cdot 0.5H_2O$:C,53.34;H,4.65;F,19.47;N,4.78.Found:C,53.76;H,4.57;F,1 9.14;N,4.82.

[Example 82]

3-[N-[2-[5-(4-Isobutyl-3-methoxybenzyloxy)indolin-1-yl]-2-oxoethyl] amino] propionic acid

(1) 3-Methoxy-4-trifluoromethanesulfonyloxybenzoic acid ethyl ester (WO 95/34540)

[F363]

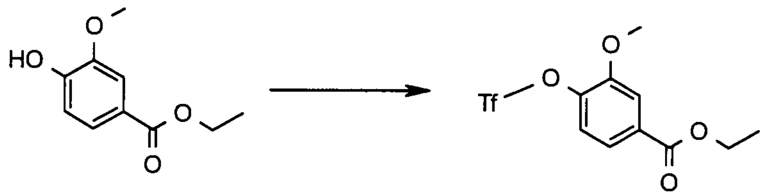

To a dichloromethane solution (50 mL) of 4-hydroxy-3-methoxybenzoic acid ethyl ester (981 mg), TEA (1.05 mL) and DMAP (61 mg) were added, and the mixture was cooled at 0°C. Thereafter, trifluoromethanesulfonic anhydride (1.01 mL) was added thereto. The resultant mixture was stirred for 3 hours at room temperature, and saturated aqueous sodium bicarbonate solution was added thereto. The thus-obtained mixture was extracted twice, each with chloroform. The organic layers were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column 2L), whereby the title compound (1.67 g) was yielded. $^1$H-NMR ($CDCl_3$) δ:
1.41 (3H,t,J=7.2Hz),3.98(3H,s), 4.40(2H,q,J=7.2Hz), 7.28(1H,d,J= 8.3Hz),7.68-7.72(2H,m).
MS(ESI)m/z:329(M+H)$^+$.

(2) 4-Isobutyl-3-methoxybenzoic acid ethyl ester

[F364]

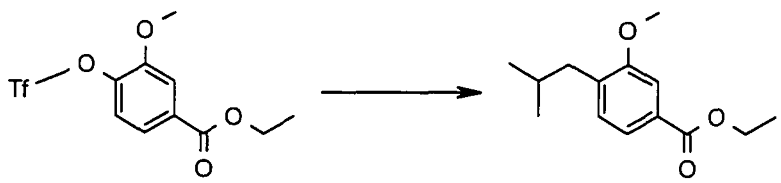

To a toluene solution (10 mL) of 3-methoxy-4-trifluoromethanesulfonyloxybenzoic acid ethyl ester (656 mg), isobutylboronic acid (612 mg), cesium carbonate (3.26 g), water (5.0 mL), and tetrakis(triphenylphosphine)palladium(0) (231 mg) were added, and the mixture was stirred overnight at reflux. The resultant mixture was left to stand to cool to room temperature, and water was added thereto, followed by extraction thrice, each with ethyl acetate. The organic layers were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column L), whereby the title compound (378 mg) was yielded.
$^1$H-NMR ($CDCl_3$) δ :
0.89(6H,d,J=6.9Hz),1.39(3H,t,J=7.1Hz),1.87-1.98 (1H, m), 2.52 (2H,d, J=7.1Hz), 3.86 (3H,s), 4.37 (2H, q, J=7.1Hz), 7 13(1H,d,J=7.8Hz),7.50(1H,d,J=1.5Hz),7.58(1H,dd,J=1.5,7.8Hz).

MS (ESI) m/z : 237 $(M+H)^+$.

(3) (4-Isobutyl-3-methoxyphenyl)methanol

[F365]

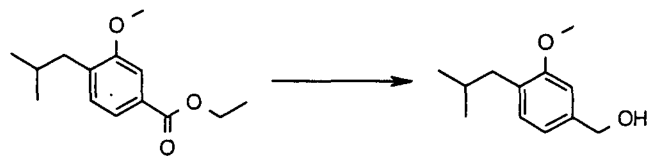

To a THF solution (20 mL) of 4-isobutyl-3-methoxybenzoic acid ethyl ester (370 mg), lithium aluminum hydride (71.1 mg) was added, and the mixture was stirred for 1 hour at reflux. The resultant mixture was left to stand to cool to room temperature and cooled at 0°C°C. Water (71 μL), 1N aqueous sodium hydroxide solution (71 μL), and water (213 μL) were sequentially added thereto, followed by drying over sodium sulfate anhydrate and concentrating under reduced pressure, whereby the title compound (320 mg) was yielded.
$^1$H-NMR ($CDCl_3$) δ:
0.89 (6H,d,J=6.6Hz),1.61(1H,t,J=5.9HZ),1.84-1.95 (1H,m),2.47(2H,d,J=7.1Hz),3.82(3H,s), 4.66(2H,d,J=5.9Hz),6 84-6.88(2H,m),7.06(1H,d,J=7.6Hz).
MS (ESI) m/z : 177 $(M-OH)^+$.

(4) 4-Chloromethyl-1-isobutyl-2-methoxybenzene

[F366]

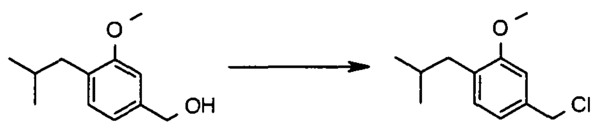

To a 1,2-dichloroethane solution (15 mL) of (4-isobutyl-3-methoxyphenyl)methanol (310 mg), thionyl chloride (579 μL) was added, and the mixture was stirred for 30 minutes at 50°C. The resultant mixture was left to stand to cool to room temperature and concentrated under reduced pressure. The residue was purified by flash column chromatography (Yamazen Hi-Flash column L), whereby the title compound (307 mg) was yielded.
$^1$H-NMR ($CDCl_3$) δ:
0.88(6H,d,J=6.6Hz), 1.84-1.94 (1H, m), 2.47 (2H, d, J=7.1Hz), 3.82 (3H,s), 4.57 (2H,s), 6.86-6.89 (2H,m),7.05(1H,d, J=7.6Hz).

(5) 3-[N-(tert-Butoxycarbonyl)-N-[2-[5-(4-isobutyl-3-methoxybenzyloxy)indolin-1-yl]-2-oxoethyl]amino]propionic acid tert-butyl ester

[F367]

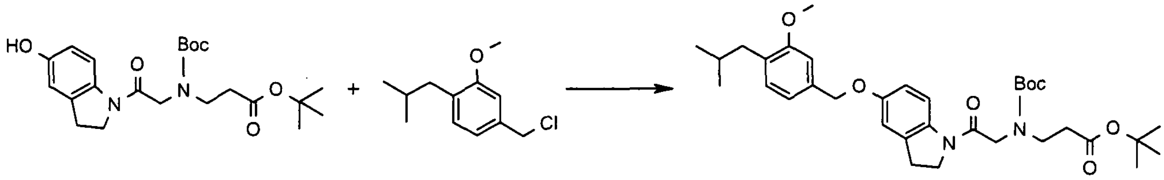

To a DMF solution (5.0 mL) of 3-[N-(tert-butoxycarbonyl)-N-[2-(5-hydroxyindolin-1-yl)-2-oxoethyl]amino]propionic acid tert-butyl ester (210 mg), 4-chloromethyl-1-isobutyl-2-methoxybenzene (117 mg) and potassium carbonate (89.8 mg) were added, and the mixture was stirred overnight at 60°C. The resultant mixture was left to stand to cool to room temperature, and water was added thereto, followed by extraction thrice, each with ethyl acetate. The organic layers were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column L), whereby the title compound (177 mg) was yielded.
$^1$H-NMR ($CDCl_3$) δ:
0.89(6H,d,J=6.6Hz),1.40-1.50(18H,m),1.87-1.94(1H,m),2.47(2H,d,J=7.1Hz),2.55-2.63(2H,m),3.15-3.21(2H,m),3.59 (2H,q,J=6.7Hz),3.81(3H,s),3.98-4.18 (4H,m), 4.98(2H,s), 6.77-6.91 (4H,m),7.08(1H,d,J=8.1Hz),8.09-8.14(1H,m).
MS (ESI) m/z : 597 $(M+H)^+$.

(6) 3-[N-[2-[5-(4-Isobutyl-3-methoxybenzyloxy)indolin-1-yl-2-oxoethyl]amino]propionic acid

[F368]

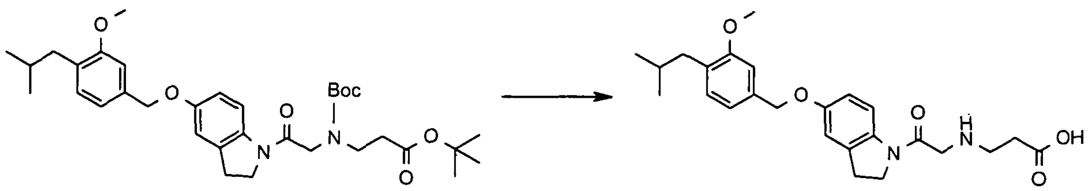

4N HCl/l,4-dioxane (10 mL) was added to 3-[N-(tert-butoxycarbonyl)-N-[2-[5-(4-isobutyl-3-methoxybenzyloxy) indolin-1-yl]-2-oxoethyl]amino]propionic acid tert-butyl ester (240 mg), and the mixture was stirred for 4 hours at room temperature. The resultant mixture was concentrated under reduced pressure. The residue was suspended in diethyl ether and filtered. The washed product was purified by ODS-flash column chromatography (Yamazen Hi-Flash column M), whereby the title compound (46 mg) was yielded.
$^1$H-NMR (DMSO-$d_6$) δ:
0.84(6H,d,J=6.6Hz),1.81-1.88(1H,m),2.42(2H,d,J=7.1Hz),2.96(2H,t,J=6.9Hz),3.14(2H,t,J= 8.3Hz),3.76-3.77(5H,m), 4.04(2H,t,J=8.3Hz), 5.01(2H,s),6.82-7.08 (5H, m),7.97 (1H, d, J=8.8Hz), (2H overlaps DMSO.).
MS (ESI) m/z : 441 $(M+H)^+$.

[Example 83]

3-[2-[5-[4-(1,1-Difluoro-2-methylpropyl)benzyloxy]indolin-1-yl]-2-oxoethylamino]propionic acid hydrochloride

(1) [4-(1,1-Difluoro-2-methylpropyl)phenyl]methanol

Starting material was synthesized in accordance with the method as described in the patent specification of WO 05/8848 (Merck & Co., Inc.)
[F369]

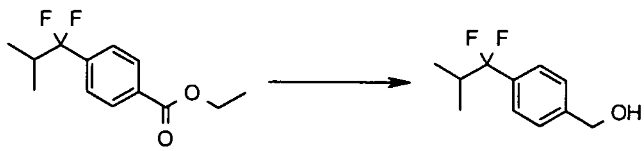

To a THF solution (25 mL) of 4-(1,1-difluoro-2-methylpropyl)benzoic acid ethyl ester (590 mg), lithium borohydride (159 mg) was added, and the mixture was stirred for 2.5 hours at reflux. The resultant mixture was left to stand to cool to room temperature, and 1N hydrochloric acid was added thereto, followed by extraction twice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column L), whereby the title compound (159 mg) was yielded. $^1$H-NMR ($CDCl_3$) δ:
0.99(6H,d,J=6.9Hz), 2.27-2.37(1H,m), 4.74(2H,s), 7.40-7.44(4H,m).

(2) 1-Chloromethyl-4-(1,1-difluoro-2-methylpropyl)benzene

[F370]

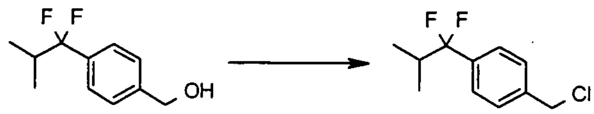

To a 1,2-dichloroethane solution (10 mL) of [4-(1,1-difluoro-2-methylpropyl)phenyl]methanol (400 mg), thionyl chloride (725 μL) was added, and the mixture was stirred for 1 hour at 50°C. The resultant mixture was left to stand to cool to room temperature and concentrated under reduced pressure. The residue was purified by flash column chromatography (Yamazen Hi-Flash column L), whereby the title compound (413 mg) was yielded. $^1$H-NMR ($CDCl_3$) δ:
0.99(6H,d,J=6.9Hz), 2.24-2.38(1H,m), 4.61(2H,s), 7.43(4H,s).

(3) 3-[N-(tert-Butoxycarbonyl) -N-[2-[5-[4-(1,1-difluoro-2-methylpropyl)benzyloxy]indolin-1-yl]-2-oxoethyl]amino]propionic acid tert-butyl ester

[F371]

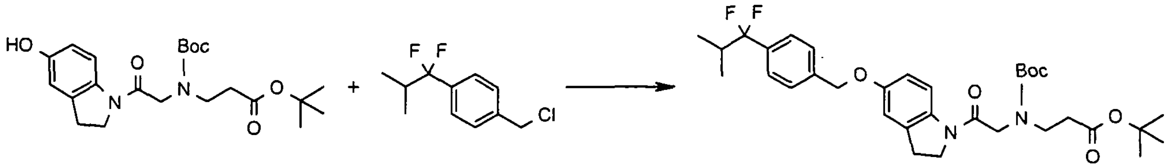

To a DMF solution (5.0 mL) of 3-[N-(tert-butoxycarbonyl)-N-[2-(5-hydroxyindolin-1-yl)-2-oxoethyl]amino]propionic acid tert-butyl ester (116 mg), 1-chloromethyl-4-(1,1-difluoro-2-methylpropyl)benzene (54.7 mg) and potassium carbonate (51.8 mg) were added, and the mixture was stirred overnight at 70°C. The resultant mixture was left to stand to cool to room temperature and concentrated under reduced pressure. The residue was purified by flash column chromatography (Yamazen Hi-Flash column 2L), whereby the title compound (169 mg) was yielded. $^1$H-NMR ($CDCl_3$) δ :
0.99(6H,d,J=6.9Hz), 1.40-1.49(18H,m), 2.25-2.39(1H,m),2.55-2.63(2H,m),3.15-3.21(2H,m),3.56-3.61(2H,m),3.99-4.18(4H,m), 5.05(2H,s), 6.76-6.83(2H,m), 7.43-7.47(4H,m), 8.10-8.15(1H,m).
MS (ESI) m/z:603 $(M+H)^+$.

(4) 3-[N-[2-[5-[4-(1,1-Difluoro-2-methylpropyl)benzyloxy]indolin-1-yl]-2-oxoethyl]amino]propionic acid hydrochloride

[F372]

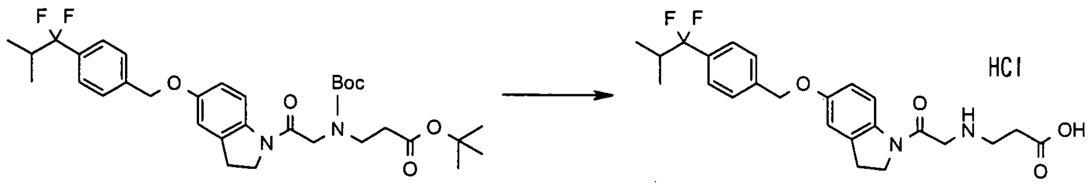

To an ethyl acetate solution (0.50 mL) of 3-[N-(tert-butoxycarbonyl)-N-[2-[5-[4-(1,1-difluoro-2-methylpropyl) benzyloxy]indolin-1-yl]-2-oxoethyl]amino]propionic acid tert-butyl ester (150 mg), 4N HCl/ethyl acetate (2.5 mL) was added, and the mixture was stirred for 3 hours at room temperature. The resultant mixture was concentrated under reduced pressure. The residue was recrystallized from acetonitrile, whereby the title compound (102 mg) was yielded. $^1$H-NMR (DMSO-$d_6$) $\delta$:
0.93(6H,d,J=6.9Hz),2.39-2.48(1H,m),2.76(2H,t,J=7.4Hz),3.16-3.21(4H,m),4.04-4.13(4H,m),5.14(2H,s),6.88(1H,dd, J=2.5,8.8Hz),7.01(1H,d,J=2.5 Hz),7.48-7.56(4H,m),7.96(1H,d,J=8.8Hz).
IR(ATR)$cm^{-1}$:2935,2601,1702,1646,1371,987,819.
MS (ESI) m/z : 447 $(M+H)^+$. HR-MS (ESI) calcd for $C_{24}H_{29}F_2N_2O_4$ $(M+H)^+$: 447.20954; found 447.21197.
Anal. Calcd for $C_{24}H_{28}F_2N_2O_4$.HCl·
$0.25H_2O$:C,59.14;H,6.10;Cl,7.27;F,7.79;N,5.75.Found: C,59.02;H, 5.96;Cl, 7.24;F,7.65;N,5.68.

[Example 84]

3-[N-[2-[5-(4-Propyl-3-trifluoromethylbenzyloxy) indolin-1-yl]-2-oxoethyl]amino]propionic acid TFA salt

(1) 4-Propyl-3-trifluoromethylbenzoic acid methyl ester

[F373]

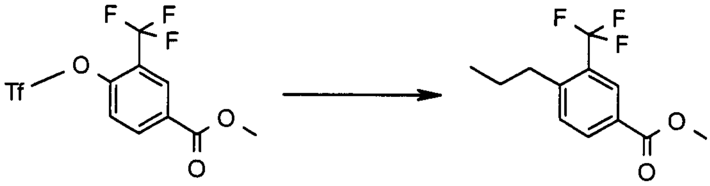

To a toluene solution (15 mL) of 4-trifluoromethanesulfonyloxy-3-trifluoromethylbenzoic acid methyl ester (1.06 g), propylboronic acid (527 mg), cesium carbonate (4.89 g), water (7.5 mL), and tetrakis(triphenylphosphine)palladium (0) (347 mg) were added, and the mixture was stirred overnight at reflux. The resultant mixture was left to stand to cool to room temperature. Saturated aqueous sodium bicarbonate solution was added thereto, followed by extraction thrice, each with diethyl ether. The extracts were combined and washed with saturated brine, followed by drying over sodium

sulfate anhydrate. Insoluble matter was removed by filtration, and the filtrate was concentrated under reduced pressure. The residue was purified by flash column chromatography (Yamazen Hi-Flash column 2L), whereby the title compound (628 mg) was yielded.
$^1$H-NMR ($CDCl_3$) δ:
1.01(3H,t,J=7.4Hz),1.63-1.72(2H,m),2.79-2.83(2H,m),3.94(3H,s),7.42(1H,d,J=8.1Hz),8.11(1H,dd,J=1.6,8.1 Hz),8.29 (1H,d,J=1.6Hz).
MS(ESI)m/z: 247(M+H)$^+$.

(2) (4-Propyl-3-trifluoromethylphenyl)methanol

[F374]

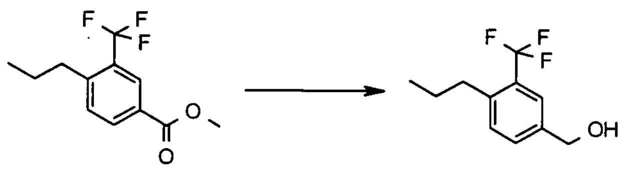

To a THF solution (20 mL) of 4-propyl-3-trifluoromethylbenzoic acid methyl ester (610 mg), lithium aluminum hydride (112 mg) was added, and the mixture was stirred for 30 minutes at reflux. The resultant mixture was left to stand to cool to room temperature. Water (115 μL), 1N aqueous sodium hydroxide solution (115 μL), and water (345 μL) were sequentially added thereto, followed by drying over sodium sulfate anhydrate. Insoluble matter was removed by filtration, and the filtrate was concentrated under reduced pressure. The residue was purified by flash column chromatography (Yamazen Hi-Flash column L), whereby the title compound (429 mg) was yielded.
$^1$H-NMR ($CDCl_3$) δ:
0.99(3H,t,J=7.2Hz), 1.60-1.75(3H,m),2.72-2.76 (2H,m),4.71(2H,d,J=5.9Hz), 7.32(1H,d,J=7.8Hz),7.46(1H,d,J= 7.8Hz), 7.61(1H,s).
MS (ESI) m/z : 201 (M-OH)$^+$.

(3) 4-Chloromethyl-1-propyl-2-trifluoromethylbenzene

[F375]

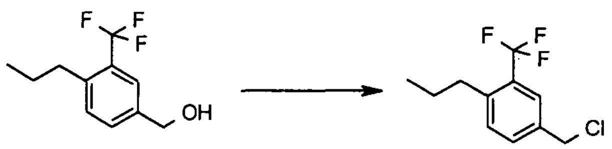

To a 1,2-dichloroethane solution (15 mL) of (4-propyl-3-trifluoromethylphenyl)methanol (420 mg), thionyl chloride (698 μL) was added, and the mixture was stirred for 2.5 hours at 50°C. The resultant mixture was left to stand to cool to room temperature and concentrated under reduced pressure, whereby the title compound (458 mg) was yielded.
$^1$H-NMR($CDCl_3$)δ:
1.00(3H,t,J=7.4Hz),1.60-1.69(2H,m),2.73-2.77(2H,m), 4.59(2H,s), 7.33(1H,d,J=8.1Hz), 7.48(1H,dd,J=1.5,8.1 Hz),7.62 (1H,d,J=1.5Hz).

(4) 3-[N-(tert-Butoxycarbonyl)-N-[2-[5-(4-propyl-3-trifluoromethylbenzyloxy)indolin-1-yl]-2-oxoethyl]amino]propionic acid tert-butyl ester

[F376]

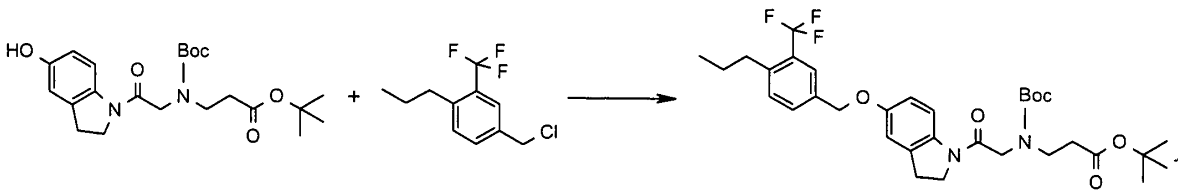

To a DMF solution (5.0 mL) of 3-[N-(tert-butoxycarbonyl)-N-[2-(5-hydroxyindolin-1-yl)-2-oxoethyl]amino]propionic acid tert-butyl ester (231 mg), 4-chloromethyl-1-propyl-2-trifluoromethylbenzene (118 mg) and potassium carbonate (173 mg) were added, and the mixture was stirred overnight at 70°C. The resultant mixture was left to stand to cool to room temperature and concentrated under reduced pressure. The residue was purified by flash column chromatography (Yamazen Hi-Flash column 2L), whereby the title compound (271 mg) was yielded. $^1$H-NMR ($CDCl_3$) δ :
1.00(3H,t,J=7.2Hz),1.40-1.50(18H,m),1.62-1.68(2H,m),2.56-2.63(2H,m),2.73-2.77(2H,m),3.16-3.22(2H,m),3.56-3.61 (2H,m),3.99-4.18(4H,m),5.01(2H,s),6.75-6.83(2H,m),7.34(1H,d,J=8.1Hz),7.51(1H,d,J=8.1Hz),7.67(1H,s),8 10-8.15 (1H,m).
MS (ESI) m/z : 621 $(M+H)^+$.

(5) 3-[N-[2-[5-(4-Propyl-3-trifluoromethylbenzyloxy)indolin-1-yl]-2-oxoethyl]amino]propionic acid TFA salt

[F377]

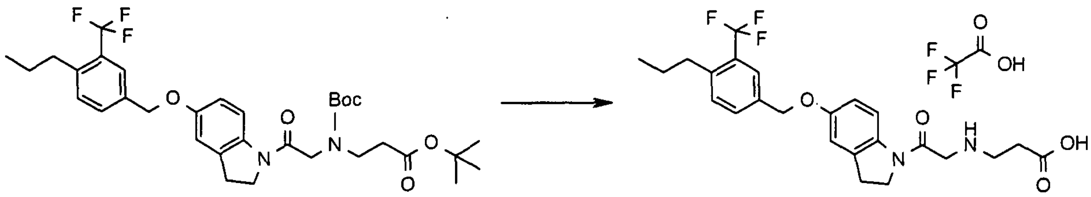

To a dichloromethane solution (8.0 mL) of 3-[N-(tert-butoxycarbonyl)-N-[2-[5-(4-propyl-3-trifluoromethylbenzyloxy)indolin-1-yl]-2-oxoethyl]amino]propionic acid tert-butyl ester (260 mg), TFA (2.0 mL) was added under cooling at 0°C, and the mixture was stirred for 2 hours at room temperature. The resultant mixture was concentrated under reduced pressure. The residue was suspended in diethyl ether for washing, filtered, and dried, whereby the title compound (201 mg) was yielded.
$^1$H-NMR (DMSO-$d_6$) δ:
0.95(3H,t,J=7.4Hz), 1.55-1.65(2H,m), 2.67-2.73(4H,m),3.15-3.20(4H,m),4.04-4.09(4H,m),5.13(2H,s),6.88(1H,dd, J=2.5,8.8Hz), 7.01(1H,d,J=2.5 Hz),7.51 (1H,d,J=8.1Hz), 7.65-7.73(2H,m),7.97(1H,d,J=8.8Hz), 10.06(1H,s).
IR(ATR)cm$^{-1}$: 2964,1724,1648,1492,1182,1135,1112.
MS(ESI)m/z:465$(M+H)^+$. HR-MS (ESI) calcd for $C_{24}H_{28}F_3N_2O_4$ $(M+H)^+$:465.20012; found 465.19736.

[Example 85]

3-[N-[2-[5-(4-Isobutyl-3-trifluoromethoxybenzyloxy)indolin-1-yl]-2-oxoethyl]amino]propionic acid TFA salt

(1) 4-Amino-3-trifluoromethoxybenzoic acid methyl ester

[F378]

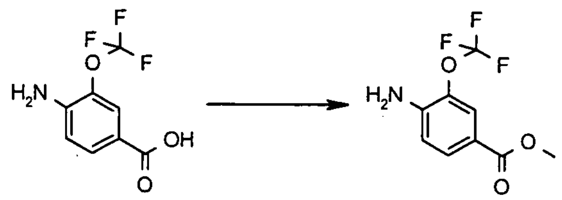

To a methanol solution (10 mL) of 4-amino-3-trifluoromethoxybenzoic acid (WO 2002/070494) (220 mg), thionyl chloride (76 μL) was added, and the mixture was stirred for 2 hours at reflux. Thionyl chloride (76 μL) was further added, followed by stirring overnight at reflux. The resultant mixture was left to stand to cool to room temperature and concentrated under reduced pressure. Saturated aqueous sodium bicarbonate solution was added to the residue, followed by extraction twice, each with ethyl acetate. The extracts were combined and washed with saturated brine, followed by drying over sodium sulfate anhydrate. Insoluble matter was removed by filtration, and the filtrate was concentrated under reduced pressure, whereby the title compound (224 mg) was yielded.
$^1$H-NMR($CDCl_3$)δ:
3.87(3H,s), 4.30(2H,s), 6.77(1H,d,J=8.6Hz), 7.78-7.84(2H,m).
MS (ESI) m/z : 236 $(M+H)^+$.

(2) 4-Bromo-3-trifluoromethoxybenzoic acid methyl ester

[F379]

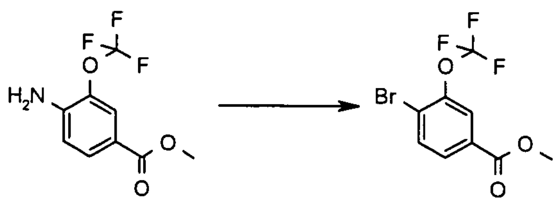

To an acetonitrile solution (10 mL) of 4-amino-3-trifluoromethoxybenzoic acid methyl ester (224 mg), copper (II) bromide (255 mg) and tert-butyl nitrite (169 μL) were added, and the mixture was stirred for 1.5 hours at 70°C. The reaction mixture was left to stand to cool to room temperature. Saturated aqueous sodium bicarbonate solution was added thereto, followed by extraction thrice, each with ethyl acetate. The extracts were combined and washed with saturated brine, followed by drying over sodium sulfate anhydrate. Insoluble matter was removed by filtration, and the filtrate was concentrated under reduced pressure, whereby the title compound (200 mg) was yielded.
$^1$H-NMR ($CDCl_3$) δ:
3.94(3H,s),7.73(1H,d,J=8.3Hz),7.84(1H,dd,J=1.7,8.3Hz),7.95-7.96(1H,m).
MS(ESI)m/z:299$(M+H)^+$.

(3) 4-Isobutyl-3-trifluoromethoxybenzoic acid methyl ester

[F380]

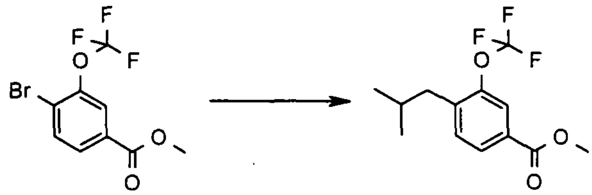

To a toluene solution (12 mL) of 4-bromo-3-trifluoromethoxybenzoic acid methyl ester (370 mg), isobutylboronic acid (378 mg), cesium carbonate (2.02 g), water (10 mL), and tetrakis(triphenylphosphine)palladium(0) (143 mg) were added, and the mixture was stirred overnight at reflux. The resultant mixture was left to stand to cool to room temperature. Saturated aqueous sodium bicarbonate solution was added thereto, followed by extraction thrice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column L), whereby the title compound (268 mg) was yielded.
$^1$H-NMR($CDCl_3$)δ:
0.92(6H,d,J=6.9Hz),1.89-1.99(1H,m),2.59(2H,d,J=7.1Hz),3.92(3H,s),7.30(1H,d,J=8.3Hz),7 .87-7.89(2H,m).
MS(ESI)m/z:277(M+H)$^+$.

(4) (4-Isobutyl-3-trifluoromethoxyphenyl)methanol

[F381]

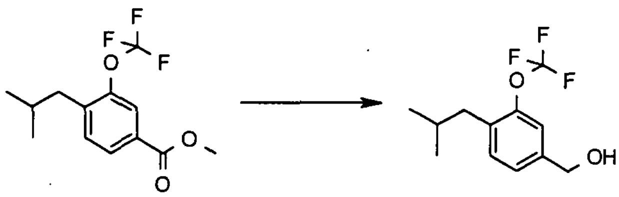

To a THF solution (10 mL) of 4-isobutyl-3-trifluoromethoxybenzoic acid methyl ester (250 mg), lithium borohydride (59.1 mg) was added, and the mixture was stirred for 1.5 hours under reflux. The resultant mixture was left to stand to cool to room temperature, and 1N hydrochloric acid was added thereto, followed by extraction thrice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column L), whereby the title compound (169 mg) was yielded.
$^1$H-NMR($CDCl_3$)δ:
0.91(6H,d,J=6.6Hz),1.73(1H,t,J=5.9Hz), 1.85-1.96(1H,m),2.53(2H,d,J=7.4Hz),4.68(2H,d,J=5.9Hz),7.20
7.23 (3H,m).
MS(ESI)m/z:231(M-OH)$^+$.

(5) 4-Chloromethyl-1-isobutyl-2-trifluoromethoxybenzene

[F382]

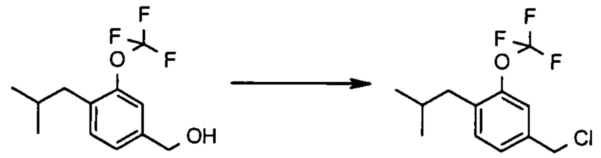

To a 1,2-dichloroethane solution (10 mL) of (4-isobutyl-3-trifluoromethoxyphenyl)methanol (160 mg), thionyl chloride (234 μL) and DMF (1 drop by means of a Pasteur pipette) were added, and the mixture was stirred for 1.5 hours at 50°C. The resultant mixture was left to stand to cool to room temperature and concentrated under reduced pressure. The residue was purified by flash column chromatography (Yamazen Hi-Flash column L), whereby the title compound (137 mg) was yielded.
$^1$H-NMR($CDCl_3$) δ:
0.91(6H,d,J=6.6Hz),1.86-1.96(1H,m),2.53(2H,d,J=7.1Hz),4.56(2H,s),7.20-7.24 (3H,m).

(6) 3-[N-(tert-Butoxycarbonyl)-N-[2-[5-(4-isobutyl-3-trifluoromethoxybenzyloxy)indolin-1-yl]-2-oxoethyl]amino]propionic acid tert-butyl ester

[F383]

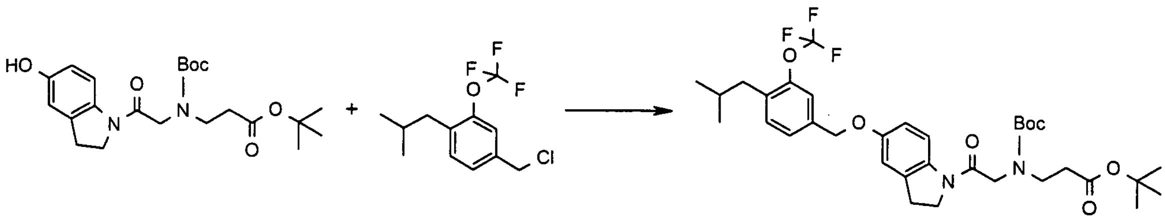

To a DMF solution (10 mL) of 3-[N-(tert-butoxycarbonyl)-N-[2-(5-hydroxyindolin-1-yl)-2-oxoethyl]amino]propionic acid tert-butyl ester (210 mg), 4-chloromethyl-1-isobutyl-2-trifluoromethoxybenzene (133 mg) and potassium carbonate (82.9 mg) were added, and the mixture was stirred overnight at 70°C. The resultant mixture was left to stand to cool to room temperature and concentrated under reduced pressure. The residue was purified by flash column chromatography (Yamazen Hi-Flash column L), whereby the title compound (216 mg) was yielded.
$^1$H-NMR ($CDCl_3$) δ :
0.91(6H,d,J=6.6Hz),1.40-1.50(18H,m),1.86-1.97(1H,m),2.49-2.63(4H,m),3.15-3.22(2H,m),3.56-3.61(2H,m),3.99-4.18 (4H,m),4.99(2H,s),6.75-6.83(2H,m),7.21-7.28(3H,m),8.10-8.14(1H,m).
MS(ESI)m/z:651(M+H)$^+$.

(7) 3-[N-[2-[5-(4-Isobutyl-3-trifluoromethoxybenzyloxy)indolin-1-yl]-2-oxoethyl]amino]propionic acid TFA salt

[F384]

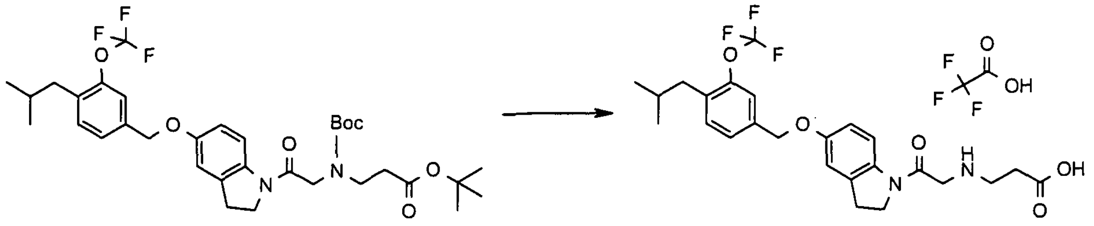

To a dichloromethane solution (2.25 mL) of 3-[N-(tert-butoxycarbonyl)-N-[2-[5-(4-isobutyl-3-trifluoromethoxybenzyloxy)indolin-1-yl)-2-oxoethyl]amino]propionic acid tert-butyl ester (200 mg), TFA (0.75 mL) was added under cooling at 0°C, and the mixture was stirred for 7 hours at room temperature. The resultant mixture was concentrated once under reduce pressure. Dichloromethane (2.25 mL) was added to the residue, and also TFA (0.75 mL) was added thereto under cooling at 0°C, followed by further stirring for 4 hours at room temperature. The resultant mixture was concentrated under reduced pressure. The residue was suspended in diethyl ether for washing, filtered, and dried, whereby the title compound (162 mg) was yielded.
$^1$H-NMR(DMSO-$d_6$) δ :
0.87(6H,d,J=6.6Hz),1.82-1.92(1H,m),2.73(2H,t,J=7.4Hz),3.16-3.22(4H,m),4.04-4.14(4H,m),5.11(2H,s),6.87(1H,dd, J=8.8,2.7Hz),7.01(1H,d,J=2.7 Hz),7.39(3H,s),7.96(1H,d,J=8.8Hz),(2H was unidentified due to overlapping of the peak thereof with that of DMSO).
IR(ATR)cm$^{-1}$:2960,1637,1496,1253,1197,1170,1137.
MS(ESI)m/z:495(M+H)$^+$.
HR-MS (ESI) calcd for $C_{25}H_{30}F_3N_2O_5$ (M+H)$^+$:495.21068; found 495.21148.
Anal. Calcd for $C_{25}H_{29}F_3N_2O_5$·
$CF_3CO_2H$: C,53.29;H,4.97;F,18.73;N,4.60. Found:C,53.31;H,4.89;F, 18.94;N,4.59.

[Example 86]

3-[N-[2-[5-(4-Cyclopropyl-3-trifluoromethoxybenzyloxy)indolin-1-yl]-2-oxoethylamino]propionic acid

(1) 4-Cyclopropyl-3-trifluoromethoxybenzoic acid methyl ester

[F385]

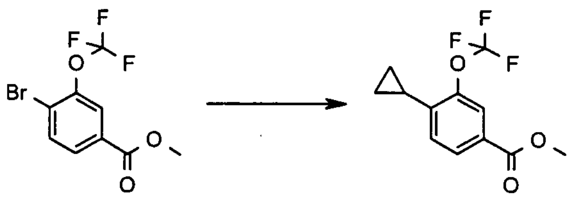

To a toluene solution (12 mL) of 4-bromo-3-trifluoromethoxybenzoic acid methyl ester (370 mg), cyclopropylboronic acid (213 mg), cesium carbonate (2.02 g), water (6.0 mL), and tetrakis(triphenylphosphine)palladium(O) (143 mg) were added, and the mixture was stirred overnight at reflux. The reaction mixture was left to stand to cool to room temperature. Aqueous sodium bicarbonate solution was added thereto, followed by extraction thrice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column L), whereby a mixture (312 mg) of the title compound and an impurity was yielded.

(2) (4-Cyclopropyl-3-trifluoromethoxyphenyl)methanol

[F386]

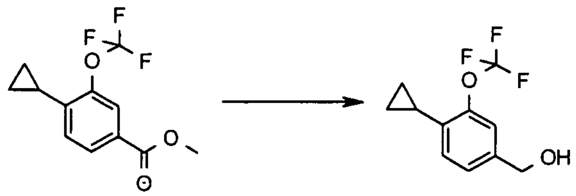

Lithium borohydride (75.3 mg) was added to a THF solution (15 mL) of impurity-containing 4-cyclopropyl-3-trifluoromethoxybenzoic acid methyl ester (300 mg), and the mixture was stirred for 1.5 hours under reflux. The reaction mixture was left to stand to cool to room temperature. 1N Hydrochloric acid was added to thereto, and the resultant mixture was extracted thrice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column L) and further purified by flash column chromatography (Yamazen Hi-Flash column 2L), whereby the title compound (71 mg) was yielded.
$^1$H-NMR($CDCl_3$)$\delta$:
0.67-0.71(2H,m),0.98-1.03(2H,m),1.69 (1H,t,J=5.9Hz),2.10-2.117(1H,m),4.67(2H,d,J=5.9Hz),6.90(1H,d,J=7.8Hz), 7.18-7.22(2H,m).
MS(ESI)m/z:215(M-OH)$^+$.

(3) 4-Chloromethyl-1-cyclopropyl-2-trifluoromethoxybenzene

[F387]

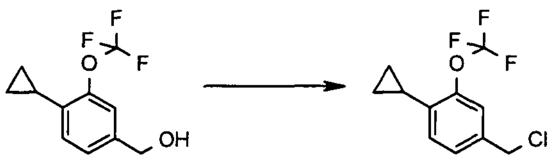

To a 1,2-dichloroethane solution (10 mL) of (4-cyclopropyl-3-trifluoromethoxyphenyl)methanol (70.0 mg), thionyl chloride (109 $\mu$L) and DMF (1 drop by means of a Pasteur pipette) were added, and the mixture was stirred for 1.5 hours at 50°C. The reaction mixture was left to stand to cool to room temperature and concentrated under reduced pressure. The residue was purified by flash column chromatography (Yamazen Hi-Flash column L), whereby the title compound (58 mg) was yielded.
$^1$H-NMR($CDCl_3$)$\delta$ :
0.68-0.72(2H,m),1.00-1.05(2H,m),2.10-2.17(1H,m),4.54(2H,s),6.89(1H,d,J=7.8Hz),7.21-7.23(2H,m).

(4) 3-[N-(tert-Butoxycarbonyl)-N-[2-[5-(4-cyclopropyl-3-trifluoromethoxybenzyloxy)indolin-1-yl]-2-oxoethyl]amino]propionic acid tert-butyl ester

[F388]

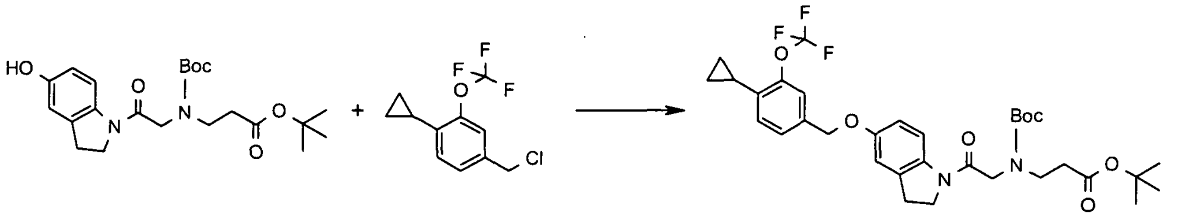

To a DMF solution (5.0 mL) of 3-[N-(tert-butoxycarbonyl)-N-[2-(5-hydroxyindolin-1-yl)-2-oxoethyl]amino]propionic acid tert-butyl ester (84.1 mg), 4-chloromethyl-1-cyclopropyl-2-trifluoromethoxybenzene (53.3 mg) and potassium carbonate (33.2 mg) were added, and the mixture was stirred overnight at 70°C. The reaction mixture was left to stand to cool to room temperature and concentrated under reduced pressure. The residue was purified by flash column chromatography (Yamazen Hi-Flash column L), and further purified by high-performance liquid chromatography (NOMURA Develosil Combi-RP-5), whereby the title compound (73 mg) was yielded.
$^1$H-NMR(CDCl$_3$)δ:
0.68-0.72(2H,m),0.99-1.04(2H,m),1.40-1.49(18H,m),2.10-2.17(1H,m),2.55-2.63(2H,m),3.15-3.21(2H,m),3.56-3.61(2H,m),3.98-4.17(4H,m),4.98(2H,s),6.74-6.81(2H,m),6.91(1H,d,J=7.8Hz),7.22-7.26(2H,m),8.09-8.14(1H,m).
MS(ESI)m/z:635(M+H)$^+$.

(5) 3-[N-[2-[5-(4-Cyclopropyl-3-trifluoromethoxybenzyloxy)indolin-1-yl]-2-oxoethyl]amino]propionic acid

[F389]

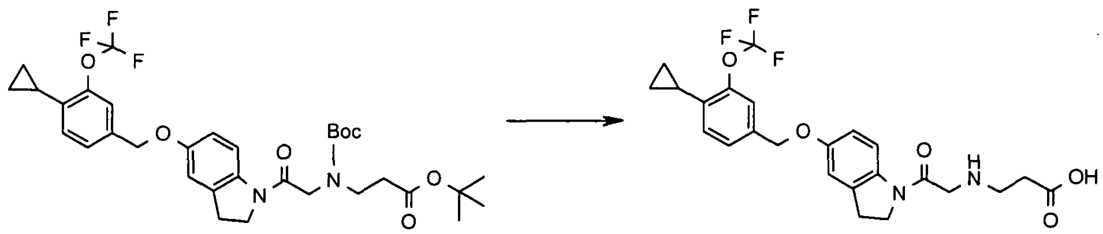

To a dichloromethane solution (1.5 mL) of 3-[N-(tert-butoxycarbonyl)-N-[2-[5-(4-cyclopropyl-3-trifluoromethoxybenzyloxy)indolin-1-yl]-2-oxoethyl]amino]propionic acid tert-butyl ester (70.0 mg), TFA (0.50 mL) was added under cooling on ice, and the mixture was stirred for 4 hours at room temperature. The reaction mixture was concentrated under reduced pressure. The residue was purified by high-performance liquid chromatography (NOMURA Develosil Combi-RP-5), whereby the title compound (35 mg) was yielded.
$^1$H-NMR(DMSO-d$_6$)δ:
0.70-0.74(2H,m),0.98-1.03(2H,m),2.03-2.09(1H,m),2.40(2H,t,J=6.7Hz),2.87(2H,t,J=6.7Hz),3.12(2H,t,J= 8.4Hz),3.62(2H,s),4.04(2H,t,J=8.4Hz),5.07(2H,s),6.81(1H,dd,J= 2.7,8.8Hz),6.94(1H,d,J=2.7Hz),7.05(1H,d,J=7.8Hz),7.34-7.37(2H,m),7.97(1H,d,J=8.8Hz).
MS(ESI)m/z:479(M+H)$^+$.

[Example 87]

3-[N-[2-[5-(4-Cyclobutyl-3-trifluoromethylbenzyloxy)indolin-1-yl]-2-oxoethyl]amino]propionic acid TFA salt

(1) 4-Cyclobutyl-3-trifluoromethylbenzoic acid methyl ester

[F390]

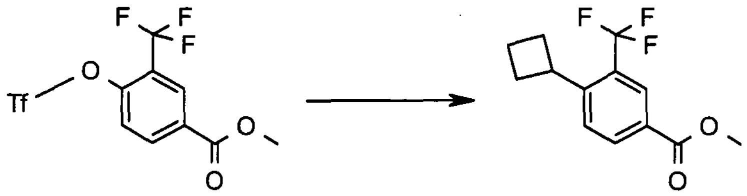

To a toluene solution (20 mL) of 4-trifluoromethanesulfonyloxy-3-trifluoromethylbenzoic acid methyl ester (1.41 g), cyclobutylboronic acid (1.20 g), cesium carbonate (6.52 g), water (10 mL), and tetrakis(triphenylphosphine)palladium (O) (462 mg) were added, and the mixture was stirred overnight at reflux. The reaction mixture was left to stand to cool to room temperature, and saturated aqueous sodium bicarbonate solution was added thereto, followed by extraction thrice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column 2L), whereby the title compound (331 mg) was yielded.
MS (ESI) m/z :258 $M^+$.

(2) (4-Cyclobutyl-3-trifluoromethylphenyl)methanol

[F391]

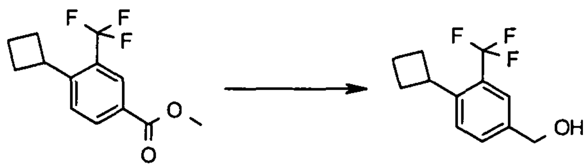

To a THF solution (20 mL) of a mixture (320 mg) of 4-cyclobutyl-3-trifluoromethylbenzoic acid methyl ester containing an impurity, lithium borohydride (81.0 mg) was added, and the mixture was stirred for 2.5 hours at reflux. The reaction mixture was left to stand to cool to room temperature, and 1N hydrochloric acid was added thereto, followed by extraction thrice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column 2L), whereby the title compound (176 mg) was yielded. $^1$H-NMR($CDCl_3$)$\delta$:
1.72-1.90(2H,m),1.96-2.08(1H,m),2.13-2.24(2H,m),2.31-2.38(2H,m),3.84-3.93(1H,m),4.72(2H,d,J=5.1Hz),7.52-7.60 (3H,m).
MS(ESI)m/z:213(M-OH)$^+$.

(3) 4-Chloromethyl-1-cyclobutyl-2-trifluoromethylbenzene

[F392]

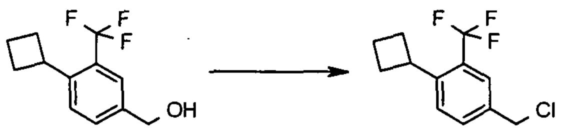

To a 1,2-dichloroethane solution (20 mL) of (4-cyclobutyl-3-trifluoromethylphenyl)methanol (170 mg), thionyl chloride (268 μL) and DMF (1 drop by means of a Pasteur pipette) were added, and the mixture was stirred for 1 hour at 50°C. The reaction mixture was left to stand to cool to room temperature and concentrated under reduced pressure. The residue was purified by flash column chromatography (Yamazen Hi-Flash column L), whereby the title compound (160 mg) was yielded.
$^1$H-NMR($CDCl_3$)δ:
1.82-1.90(1H,m),1.97-2.09(1H,m),2.14-2.24(2H,m),2.31-2.39(2H,m),3.84-3.92(1H,m),4.59(2H,s),7.54-7.60(3H,m).

(4) 3-[N-(tert-Butoxycarbonyl)-N-[2-[5-(4-cyclobutyl-3-trifluoromethylbenzyloxy)indolin-1-yl]-2-oxoethyl]amino]propionic acid tert-butyl ester

[F393]

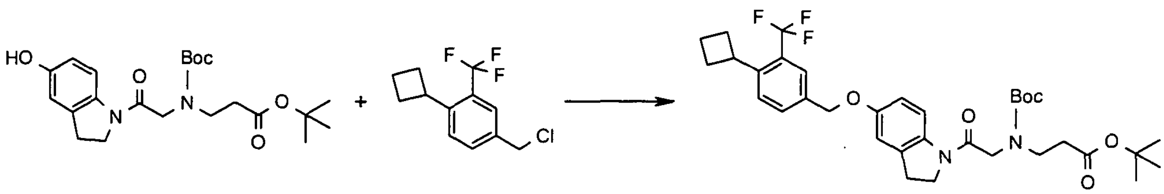

To a DMF solution (10 mL) of 3-[N-(tert-butoxycarbonyl)-N-[2-(5-hydroxyindolin-1-yl)-2-oxoethyl]amino]propionic acid tert-butyl ester (210 mg), 4-chloromethyl-1-cyclobutyl-2-trifluoromethylbenzene (149 mg) and potassium carbonate (104 mg) were added, and the mixture was stirred overnight at 70°C. The reaction mixture was left to stand to cool to room temperature, followed by filtration. The filtrate was concentrated under reduced pressure. The residue was purified by flash column chromatography (Yamazen Hi-Flash column L), whereby the title compound (330 mg) was yielded.
$^1$H-NMR($CDCl_3$)δ:
1.40-1.49(18H,m),1.81-1.90(1H,m),1.96-2.08(1H,m),2.14-2.24(2H,m),2.31-2.38(2H,m),2.55-2.63(2H,m),3.15-3.22(2H,m),3.56-3.61(2H,m),3.84-4.18(5H,m),5.02(2H,s),6.75-6.83(2H,m),7.58(2H,s),7.64(1H,s),8.10-8.14(1H,m).
MS(ESI)m/z:633(M+H)$^+$.

(5) 3-[N-[2-[5-(4-Cyclobutyl-3-trifluoromethylbenzyloxy)indolin-1-yl]-2-oxoethyl]amino]propionic acid TFA salt

[F394]

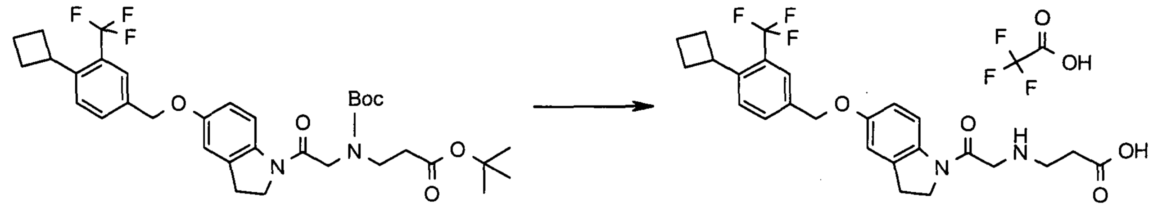

To a dichloromethane solution (4.0 mL) of 3-[N-(tert-butoxycarbonyl)-N-[2-[5-(4-cyclobutyl-3-trifluoromethylbenzyloxy)indolin-1-yl]-2-oxoethyl]amino]propionic acid tert-butyl ester (316 mg), TFA (1.0 mL) was added under cooling at 0°C, and the mixture was stirred for 9 hours at room temperature. The reaction mixture was concentrated under reduced pressure. The residue was suspended in diethyl ether for washing, filtered, and dried, whereby the title compound (259 mg) was yielded.
$^1$H-NMR(DMSO-d$_6$)δ:
1.79-2.05(2H,m),2.15-2.31(4H,m),2.72(2H,t,J=7.4Hz),3.19(4H,q,J=7.4Hz),3.75-3.83(1H,m),4.03-4.13(4H,m),5.15(2H, s),6.88(1H,dd,J=2.7,8.8Hz),7.01(1H,d,J=2.7 Hz),7.71-7.76(3H,m),7.96(1H,d,J=8.8Hz).
IR(ATR)cm$^{-1}$:2944,1722,1664,1490,1187,1133.
MS(ESI)m/z:477(M+H)$^+$.
HR-MS (ESI) calcd for $C_{25}H_{28}F_3N_2O_4$(M+H)$^+$:477.20012;found 477.19913.
Anal. Calcd for $C_{25}H_{27}F_3N_2O_4 \cdot CF_3CO_2H$:C,54.92;H,4.78;F,19.30;N,4.74.Found:C,54.96;H,4.68;F, 19.52;N,4.72.

[Example 88]

3-[N-[2-Oxo-2- [5-[(3-cyano-4-cyclohexylphenyl)methoxy]indolin-1-yl]ethyl]amino]propionic acid

(1) 3-Cyano-4-trifluoromethanesulfonyloxybenzoic acid methyl ester

[F395]

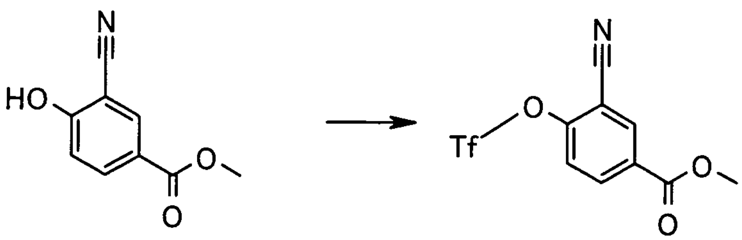

To a solution of 3-cyano-4-hydroxybenzoic acid methyl ester (870 mg) in dichloromethane (10 mL), pyridine (860 μL) and trifluoromethanesulfonic anhydride (1.13 mL) were slowly added at 0°C, and the mixture was stirred for 16 hours at room temperature. Precipitated solid was removed by filtration, and the filtrate was concentrated under reduced pressure. Water (15 mL) and ethyl acetate (20 mL) were added to the residue for phase separation, and the aqueous layer was extracted with ethyl acetate (3 x 10 mL). The extracts were combined and washed with 3% aqueous copper sulfate solution and saturated brine, followed by drying over sodium sulfate anhydrate. Solvent was removed under reduced pressure, whereby the title compound (1.34 g) was yielded.
$^1$H-NMR(CDCl$_3$) δ:
3.99(3H,s),7.59(1H,d,J=8.8Hz),8.37(1H,dd,J=8.8,2.2Hz),8.44(1H ,d,J=2.2Hz).
MS(ESI)m/z:310(M+H)$^+$.

(2) 3-Cyano-4-cyclohexylbenzoic acid methyl ester

[F396]

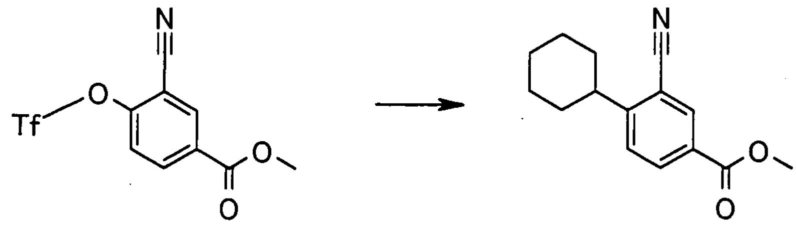

To a solution of 3-cyano-4-trifluoromethanesulfonyloxybenzoic acid methyl ester (928 mg) in THF (5.0 mL), bis (tri-tert-butylphosphine)palladium (153 mg) and 0.5M cyclohexylmethylzinc bromide/THF solution (6.60 mL) were added at room temperature under a nitrogen atmosphere, and the mixture was refluxed for 2 hours. The reaction mixture was left to stand to cool to room temperature, and saturated aqueous sodium hydrogencarbonate solution (10 mL) was added thereto, followed by stirring for 30 minutes. Precipitated solid was removed by filtration, and the filtrate was extracted with chloroform (3 x 10 mL), followed by washing with saturated brine and drying over sodium sulfate anhydrate. The filtrate was concentrated under reduced pressure, and the residue was purified by silica gel column chromatography (Biotage 40S), whereby the title compound (566 mg) was yielded.
$^1$H-NMR($CDCl_3$)δ:
1.22-1.34(1H,m),1.40-1.55(4H,m),1.58(1H,d,J=8.8Hz),1.81(1H,d,J=12.9Hz),1.90(3H,t,J =9.8Hz),3.00-3.06(1H,m),3.93 (3H,s),7.46(1H,d,J=8.3Hz),8.17(1H,dd,J=8.3,1.5 Hz),8.27(1H,d,J=1.5Hz).
MS(ESI)m/z:244(M+H)$^+$.

(3) 3-Cyano-4-cyclohexylbenzoic acid

[F397]

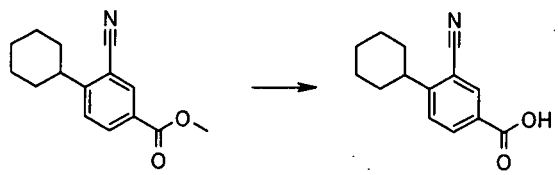

To a solution of 3-cyano-4-cyclohexylbenzoic acid methyl ester (566 mg) in THF (6.0 mL), methanol (3.0 mL) and 1N aqueous sodium hydroxide solution (3.00 mL) were added at room temperature, and the mixture was stirred for 3 days at room temperature. Water (5.0 mL) was added thereto, and the pH thereof was adjusted to 3 with 1N hydrochloric acid. Chloroform (10 mL) was added thereto for phase separation, and the aqueous layer was extracted with chloroform (3 x 7.5 mL). The extracts were combined and dried over sodium sulfate anhydrate, and solvent was removed under reduced pressure, whereby the title compound (483 mg) was yielded.
$^1$H-NMR(DMSO-$d_6$)δ:
1.23-1.31(1H,m),1.33-1.56(4H,m),1.73(1H,d,J=12.5Hz),1.82(4H,t,J=11.7Hz),2.86-2.94(1H,m),7.65(1H,d,J=8.3Hz), 8.15(1H,dd,J=8.3,1.7Hz),8.20(1H ,d,J=1.7Hz),13.36(1H,s).
MS(ESI)m/z:459(2M+H)$^+$.

(4) 3-Cyano-4-cyclohexylbenzyl alcohol

[F398]

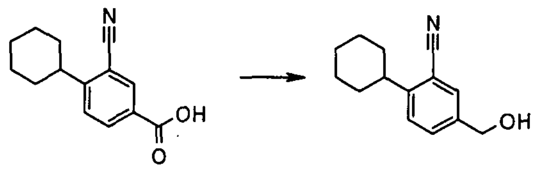

To a solution of 3-cyano-4-cyclohexylbenzoic acid (483 mg) in THF (5.0 mL), TEA (440 μL) and ethyl chloroacetate (242 μL) were slowly added at 0°C, and the mixture was stirred for 1 hour at room temperature. Precipitated matter was removed by filtration. In another flask, the filtrate containing the benzoic acid derivative was slowly added to a suspension of sodium borohydride (520 mg) in ethanol (0.5 mL) at 0°C, and the mixture was stirred for 1 hour at 0°C. 1N Hydrochloric acid was slowly added thereto to adjust the pH thereof to 4. Ethyl acetate (10 mL) was added to the resultant mixture for phase separation. The aqueous layer was extracted with ethyl acetate (3 x 5.0 mL). The extracts were combined and dried over sodium sulfate anhydrate. Insoluble matter was removed by filtration, and the filtrate was concentrated under reduced pressure. The residue was purified by silica gel column chromatography (Biotage 40S), whereby the title compound (249 mg) was yielded.

$^1$H-NMR($cDCl_3$)δ:

1.21-1.32(1H,m),1.38-1.51(4H,m),1.74-1.92(6H,m),2.94-3.00(1H,m),4.70(2H,d,J=4.9Hz),7.36(1H,d,J=8.3Hz),7.53(1H,dd,J =8.0,1.5Hz),7.61(1H,d,J=1.5Hz).

(5) 3-[N-(tert-Butoxycarbonyl)-N-[2-[5-[(3-cyano-4-cyclohexylphenyl)methoxy]indolin-1-yl]-2-oxoethyl]amino]propionic acid tert-butyl ester

[F399]

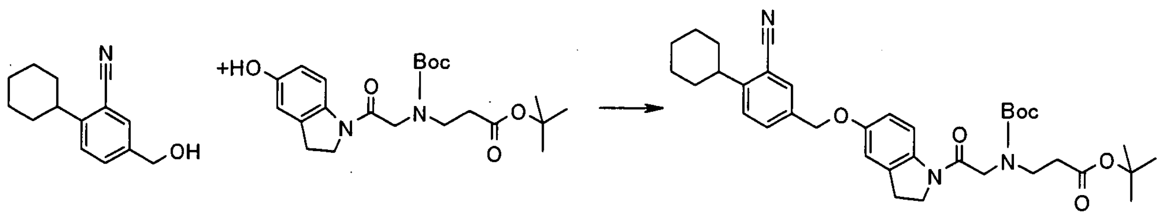

To a solution of 3-cyano-4-cyclohexylbenzyl alcohol (249 mg) in THF (5.0 mL), triphenylphosphine (364 mg), 3-[N-(tert-butoxycarbonyl)-N-[2-(5-hydroxyindolin-1-yl)-2-oxoethyl]amino]propionic acid tert-butyl ester (486 mg), and DEAD (225 μL) were added at room temperature, and the mixture was stirred for 18 hours at room temperature. The reaction mixture was concentrated under reduced pressure. The residue was purified by silica gel column chromatography (Biotage 40S), whereby the title compound (683 mg) was yielded.

$^1$H-NMR($CDCl_3$)δ:

1.38-1.51(23H,m),1.76-1.93(5H,m),2.55-2.63(2H,m),2.94-3.02(1H,m),3.16-3.24(2H,m),3.55-3.62(2H,m),3.99-4.08(2H,m),4.18(2H,s),4.99(2H,s),6.72-6.83(2H,m),7.33-7.40(1H,m),7.57(1H,d,J=7.8Hz),7.66(1H,br s),8.11(1H,d,J=8.8Hz).

MS(ESI)m/z:618(M+H)$^+$.

(6) 3-[N-[2-[5-[(3-Cyano-4-cyclohexylphenyl)methoxy]indolin-1-yl]-2-oxoethyl]amino]propionic acid

[F400]

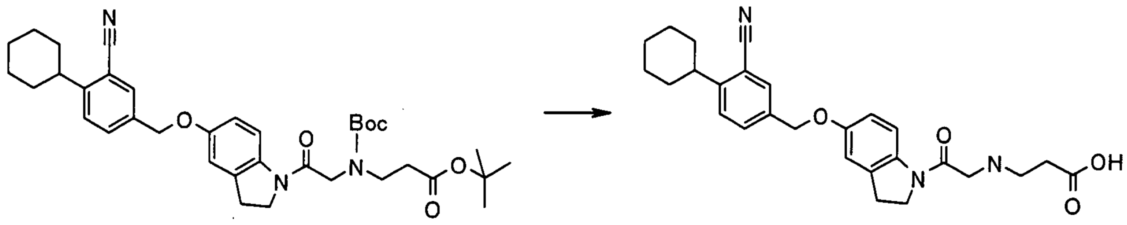

To a solution of 3-[N-(tert-butoxycarbonyl)-N-[2-[5-[(3-cyano-4-cyclohexyl-phenyl)methoxy]indolin-1-yl]-2-oxoethyl]amino]propionic acid tert-butyl ester (683 mg) in dichloromethane (12.0 mL), TFA (3.0 mL) was added at room temperature, and the mixture was stirred for 30 minutes. The reaction mixture was concentrated under reduced pressure. Dimethyl sulfoxide (6.0 mL) was added to the residue, and insoluble matter was removed. The residue was purified by high-performance liquid chromatography (NOMURA Develosil Combi-RP5), and the target fraction was concentrated and freeze-dried, whereby the title compound (357 mg) was yielded.
$^1$H-NMR(DMSO-$d_6$)δ: 1.19-1.54(5H,m),1.69-1.86(5H,m),2.41(2H,t,J=6.7Hz),2.80-2,88(1H,m),2,89(2H,t,J=6.7Hz),3,12 (2H,t,J=8,4Hz),3,65(2H,s),4 .03(2H,t,J=8,4Hz),5,07(2H,s),6.82(1H,dd,J=8.8,2.7Hz),6.95(1H, d,J=2.7Hz),7.53(1H,d, J=8.1Hz),7.70(1H,dd,J=8.3,1.5Hz),7.81(1H ,d,J=1.5Hz),7.96(1H,d,J=8.8Hz).
MS(ESI)m/z:462(M+H)$^+$.
Anal. Calcd for $C_{27}H_{31}N_3O_4$·
1.5$H_2O$,0.25$CF_3CO_2H$:C,63.88;H,6.68;N,8.13.Found:C,63.91;H,8.09; N,6.70.

[Example 89]

3-[N-[2-Oxo-2-[5-[(3-chloro-4-cyclohexylphenyl)methoxy]indolin-1-yl]ethyl]amino]propionic acid

(1) 3-Chloro-4-trifluoromethanesulfoxybenzoic acid methyl ester (EP 594022)

[F401]

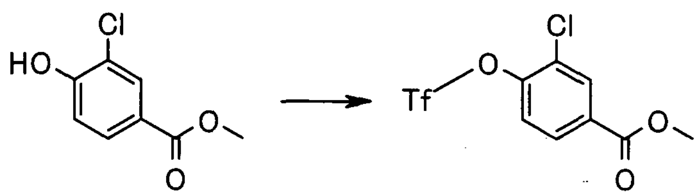

To a solution of 3-chloro-hydroxybenzoic acid methyl ester (3.44 g) in dichloromethane (50 mL), pyridine (1.94 mL) and trifluoromethanesulfonic anhydride (3.41 mL) were slowly added at 0°C, and the mixture was stirred for 16 hours at room temperature. Precipitated solid was removed by filtration, and the filtrate was concentrated under reduced pressure. Water (30 mL) and chloroform (50 mL) were added to the residue for phase separation. The aqueous layer was extracted with ethyl acetate (3 x 30 mL). The extracts were combined and washed with 3% aqueous copper sulfate solution and saturated brine, followed by drying over sodium sulfate anhydrate. Solvent was removed under reduced pressure, whereby the title compound (5.02 g) was yielded.
$^1$H-NMR($CDCl_3$)δ:
3.95(3H,s),7.44(1H,d,J=8.5Hz),8.03(1H,dd,J=8.5,2.0Hz),8.21(1H ,J=2.0Hz).
MS (ESI)m/z:319 (M+H)$^+$.

(2) 3-Chloro-4-cyclohexylbenzoic acid methyl ester (DE 2341301)

[F402]

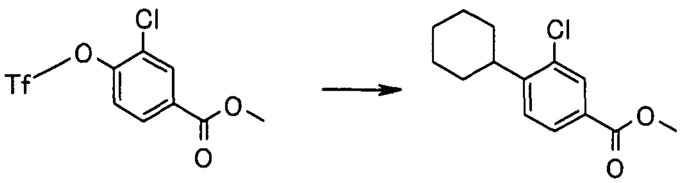

To a solution of 3-chloro-4-trifluoromethanesulfoxybenzoic acid methyl ester (1.12 g) in N,N-dimethylacetamide (10 mL), copper(I) iodide (66.7 mg), [1,1'-bis(diphenylphosphino)ferrocene]palladium dichloride-dichloromethane complex (143 mg), and a 0.5M cyclohexylzinc bromide/THF solution (8.40 mL) were added at room temperature under a nitrogen atmosphere, and the mixture was refluxed for 2 hours. The reaction mixture was left to stand to cool to room temperature, and saturated aqueous ammonium chloride solution (15 mL) was added thereto, followed by stirring for 30 minutes at room temperature. Precipitated solid was removed by filtration, and the aqueous layer of the filtrate was extracted with chloroform (3 x 10 mL). The extracts were combined and washed with saturated brine, followed by drying over sodium sulfate anhydrate and concentration under reduced pressure. The residue was purified by silica gel column chromatography (Biotage 40S), whereby the title compound (474 mg) was yielded.
$^1$H-NMR($CDCl_3$)δ: 1.25-1.33(1H,m),1.34-1.52(4H,m),1.75-1.92(5H,m),3.02-3.08(1H,m),3.90(3H,s),7.33(1H,d, J=8.3Hz),7.87(1H,dd,J=8.1,1.7 Hz),8.01(1H,d,J=1.7Hz).
MS(ESI)m/z:253(M+H)$^+$.

(3) 3-Chloro-4-cyclohexylbenzoic acid

[F403]

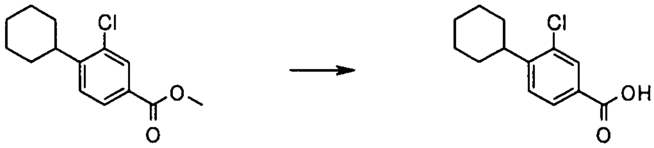

To a solution of 3-chloro-4-cyclohexylbenzoic acid methyl ester (474 mg) in THF (5.0 mL), methanol (2.5 mL) and 1N aqueous sodium hydroxide solution (2.50 mL) were added at room temperature, and the mixture was stirred for 15 hours at room temperature. Water (10 mL) was added to the reaction mixture, and the pH thereof was adjusted to 3 with 1N hydrochloric acid. Chloroform (15 mL) was added thereto for phase separation. The aqueous layer was extracted with chloroform (3 x 10 mL). The extracts were combined and dried over sodium sulfate anhydrate, and the solvent was removed under reduced pressure, whereby the title compound (213 mg) was yielded.
$^1$H-NMR(DMSO-$d_6$)δ:
1.23-1.31(1H,m),1.33-1.56(4H,m),1.73(1H,d,J=12.5Hz),1.82(4H,t,J=11.7Hz),2.86-2.92(1H,m),7.65(1H,d,J=8.3Hz), 8.15(1H,dd,J=8.3,1.7Hz),8.20(1H ,d,J=1.7Hz),13.36(1H,s).
MS (ESI)m/z:477 (2M+H)$^+$.

(4) 3-Chloro-4-cyclohexylbenzyl alcohol (DE 2341301)

[F404]

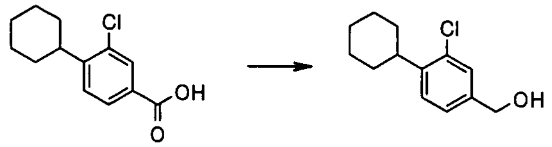

To a solution of 3-chloro-4-cyclohexylbenzoic acid (213 mg) in THF (5.0 mL), TEA (186 μL) and ethyl chloroacetate (102 μL) were slowly added at 0°C, and the mixture was stirred for 1 hour at room temperature. Precipitated matter was removed by filtration. In another flask, the filtrate containing the benzoic acid derivative was slowly added to a suspension of sodium borohydride (263 mg) in ethanol (5.0 mL) at 0°C, and the mixture was stirred for 1 hour at 0°C. 1N Hydrochloric acid was added thereto to adjust the pH thereof to 4. Water (5 mL) and ethyl acetate (10 mL) were added to thereto, and the aqueous layer was extracted with ethyl acetate (3 x 5.0 mL). The extracts were combined and dried over sodium sulfate anhydrate, and the filtrate was concentrated under reduced pressure. The residue was purified by silica gel column chromatography (Biotage 25S), whereby the title compound (148 mg) was yielded.
$^1$H-NMR($CDCl_3$)δ:
1.20-1.51(5H,m),1.65(1H,t,J=6.0Hz),1.77(1H,d,J=12.7Hz),1.82-1.90(4H,m),2.96-3.04(1H,m),4.63(2H,d,J=5.9Hz),7.21 (1H,dd,J=8.1,1.7Hz),7.25(1H d,J=8.1Hz),7.36(1H,d,J=1.7Hz).

(5) 3-[N-(tert-Butoxycarbonyl)-N-[2-[5-[(3-chloro-4-cyclohexyl-phenyl)methoxy]indolin-1-yl]-2-oxoethyl]amino]propionic acid tert-butyl ester

[F405]

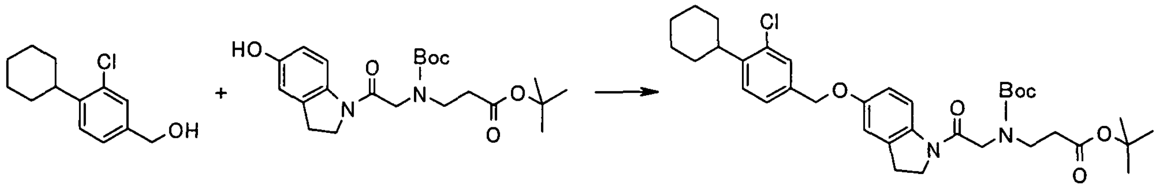

To a solution of 3-chloro-4-cyclohexylbenzyl alcohol (148 mg) in THF (3.0 mL), triphenylphosphine (225 mg), 3-[N-(tert-butoxycarbonyl)-N-[2-(5-hydroxyindolin-1-yl)-2-oxoethyl]amino]propionic acid tert-butyl ester (305 mg), and DEAD (139 μL) were added at room temperature, and the mixture was stirred for 4 hours at room temperature. The reaction mixture was concentrated under reduced pressure. The residue was purified by silica gel column chromatography (Biotage 40S), whereby the title compound (264 mg) was yielded.
$^1$H-NMR($CDCl_3$)δ: 1.23-1.60(23H,m),1.74-1.91(5H,m),2.55-2.63(2H,m),2.96-3.04(1H,m),3.15-3.22(2H,m),3.56-3.62 (2H,m),3.98-4.07(2H,m),4.09-4.18(2H,m),4.95(2H,s),6.74-6.83(2H,m),7.26(2H,s),7.41(1H,s),8.15-8.09(1H,m).

(6) 3-[N-[2-[5-[(3-Chloro-4-cyclohexylphenyl)methoxy]indolin-1-yl]-2-oxoethyl]amino]propionic acid

[F406]

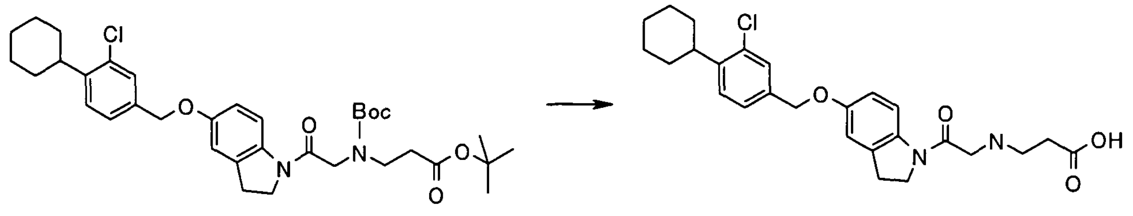

To a solution of 3-[N-(tert-butoxycarbonyl)-N-[2-[5-[(3-chloro-4-cyclohexyl-phenyl)methoxy]indolin-1-yl]-2-oxoethyl]amino]propionic acid tert-butyl ester (264 mg) in dichloromethane (1.6 mL), TFA (0.45 mL) was added at room temperature, and the mixture was stirred for 16.5 hours at room temperature. The reaction mixture was concentrated under reduced pressure, and dimethyl sulfoxide (3.0 mL) was added to the residue to remove insoluble matter. The residue was purified by high-performance liquid chromatography (NOMURA Develosil Combi-RP5), and the target fraction was concentrated and freeze-dried, whereby the title compound (120 mg) was yielded.
MS(ESI)m/z:471(M+H)$^+$.
Anal. Calcd for $C_{26}H_{31}ClN_2O_4$·
0.5H2O,0.1$CF_3CO_2$H:C,64.04;H,6.58;Cl,7.21;F,1.16;N,5.70.Found:C ,64.40;H,6.54;Cl,6.97;F,0.78;N,5.73.

[Example 90]

3-[N-[2-[5-[(4-Cyclohexyl-3-fluorophenyl)methoxy] indolin-1-yl]-2-oxoethyl]amino]propionic acid

(1) 3-Fluoro-4-hydroxybenzoic acid methyl ester

[F407]

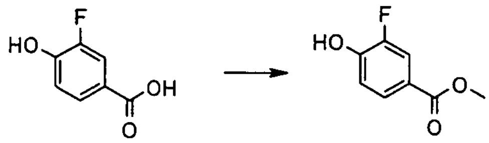

To a solution of 3-fluoro-4-hydroxybenzoic acid (1.17 g) in methanol (10 mL), thionyl chloride (602 μL) was added at room temperature, and the mixture was refluxed for 2 hours. The reaction mixture was concentrated under reduced pressure. Water (10 mL) and chloroform (10 mL) were added to the residue for phase separation. The aqueous layer was extracted with chloroform (2 x 10 mL). The organic layers were combined and dried over sodium sulfate anhydrate. The filtrate was concentrated under reduced pressure, whereby the title compound (1.19 g) was yielded.
$^1$H-NMR ($CDCl_3$)δ:
3.90(3H,s),5.85(1H,s),7.04(1H,t,J=8.3Hz),7.79-7.75(2H,m).

(2) 3-Fluoro-4-trifluoromethanesulfoxybenzoic acid methyl ester (WO 2003076397)

[F408]

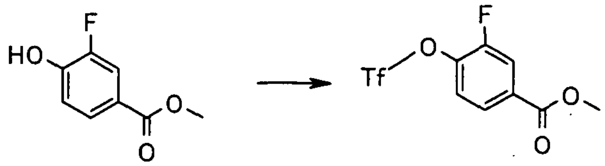

To a solution of 3-fluoro-4-hydroxybenzoic acid methyl ester (1.19 g) in dichloromethane (10 mL), pyridine (735 μL) and trifluoromethanesulfonic anhydride (1.24 mL) were slowly added at 0°C, and the mixture was stirred at room temperature for 1 hour. Precipitated solid was removed by filtration, and the filtrate was concentrated under reduced pressure. Water (20 mL) and chloroform (30 mL) were added to the residue for phase separation. The aqueous layer was extracted with ethyl acetate (3 x 20 mL). The extracts were combined and washed with 3% aqueous copper sulfate solution and saturated brine, followed by drying over sodium sulfate anhydrate. Solvent was removed under reduced pressure, whereby the title compound (1.81 g) was yielded.
$^1$H-NMR($CDCl_3$)δ:
3.95(3H,s),7.43(1H,dd,J=9.0,7.1Hz),7.94-7.91(1H,m),7.94(1H,dd,J=9.8,2.0Hz).

(3) 4-Cyclohexyl-3-fluorobenzoic acid methyl ester

[F409]

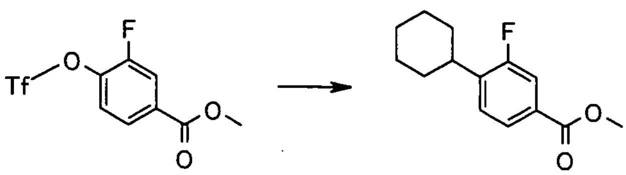

To a solution of 3-fluoro-4-trifluoromethanesulfoxybenzoic acid methyl ester (708 mg) in N,N-dimethylacetamide (10 mL), copper(I) iodide (44.6 mg), [1,1'-bis(diphenylphosphino)ferrocene]palladium chloride-dichloromethane complex (95.7 mg), and 0.5M cyclohexylzinc bromide/THF solution (5.62 mL) were added at room temperature under a nitrogen atmosphere, and the mixture was refluxed for 6.5 hours. The reaction mixture was left to stand to cool to room temperature, and saturated aqueous ammonium chloride solution (15 mL) was added thereto, followed by stirring for 30 minutes at room temperature. Precipitated solid was removed by filtration, and the aqueous layer of the filtrate was extracted with chloroform (3 x 10 mL). The extracts were combined and washed with saturated brine, followed by drying over sodium sulfate anhydrate. Insoluble matter was removed by filtration, and the filtrate was concentrated under reduced pressure. The residue was purified by silica gel column chromatography (Biotage 40S), whereby a mixture (306 mg) of the title compound and an impurity was yielded.
MS(ESI)m/z:237(M+H)$^+$.

(4) 4-Cyclohexyl-3-fluorobenzoic acid

[F410]

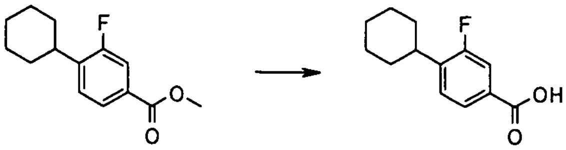

To a solution of 4-cyclohexyl-3-fluorobenzoic acid methyl ester (306 mg) in THF (4.0 mL), methanol (2.0 mL) and 1N aqueous sodium hydroxide solution (2.00 mL) were added at room temperature, and the mixture was stirred for 1 hour at room temperature. Water (5 mL) was added to the reaction mixture, and the pH thereof was adjusted to 3 with 1N hydrochloric acid. Chloroform (10 mL) was added thereto for phase separation. The aqueous layer was extracted with chloroform (3 x 10 mL). The extracts were combined and dried over sodium sulfate anhydrate. Solvent was removed under reduced pressure, and the residue was purified by silica gel column chromatography (Biotage 25M), whereby the title compound (36.6 mg) was yielded.
$^1$H-NMR ($CDCl_3$)δ: 1.21-1.34(1H,m),1.39-1.51(4H,m),1.78(1H,d,J=12.7Hz),1.82-1.91(4H,m),2.88-2.96(1H,br m),7.33 (1H,dd,J=7.6,7.6Hz),7.72(1H,dd,J=10.7,1.5Hz),7.84(1H,d d,J=7.6,1.5Hz).

(5) 4-Cyclohexyl-3-fluorobenzyl alcohol

[F411]

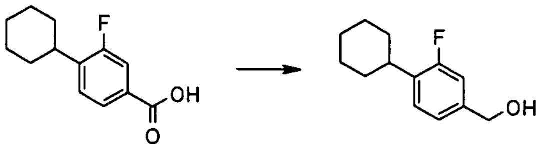

To a solution of 4-cyclohexyl-3-fluorobenzoic acid (36.6 mg) in THF (2.0 mL), TEA (34.4 μL) and ethyl chloroacetate (18.9 μL) were slowly added at 0°C, and the mixture was stirred for 1 hour at room temperature. Precipitated matter was removed by filtration. In another flask, the filtrated containing the benzoic acid derivative was slowly added to a suspension of sodium borohydride (40.6 mg) in ethanol (3.0 mL) at 0°C, and the mixture was stirred for 2 hours at 0°C. 1N Hydrochloric acid was added to adjust the pH thereof to 4. Water (3 mL) and ethyl acetate (5.0 mL) were added to the resultant mixture for phase separation, and the aqueous layer was extracted with ethyl acetate (3 x 5.0 mL). The organic layers were combined and dried over sodium sulfate anhydrate. Insoluble matter was removed by filtration, and the filtrate was concentrated under reduced pressure. The residue was purified by silica gel column chromatography (Biotage 25S), whereby the title compound (23.8 mg) was yielded.
$^1$H-NMR($CDCl_3$)δ:
1.20-1.49(5H,m),1.74-1.90(5H,m),2.80-2.88(1H,m),4.62(2H,s),7.01(1H,d,J=11.0Hz),7.05(1H,d,J=7.8Hz), 7.20(1H,dd, J=7.8,7.8Hz).

(6) 3-[N-(tert-Butoxycarbonyl)-N-[2-[5-[(4-cyclohexyl-3-fluorophenyl)methoxy]indolin-1-yl]-2-oxoethyl]amino]propionic acid tert-butyl ester

[F412]

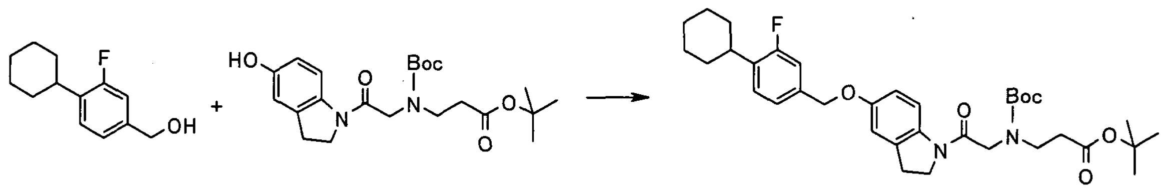

To a solution of 4-cyclohexyl-3-fluorobenzyl alcohol (23.8 mg) in THF (2.0 mL), triphenylphosphine (39.0 mg), 3-[N-(tert-butoxycarbonyl)-N-[2-(5-hydroxyindolin-1-yl)-2-oxoethyl]amino]propionic acid tert-butyl ester (52.9 mg), and DEAD (24.1 μL) were added at room temperature, and the mixture was stirred for 4 hours at room temperature, followed by concentration under reduced pressure. The residue was purified by silica gel column chromatography (Biotage 40S), and the target fraction was concentrated. The resultant residue was purified by thin-layer chromatography, whereby the title compound (40.8 mg) was yielded.
$^1$H-NMR($CDCl_3$)δ: 1.22-1.50(23H,m),1.70-1.87(5H,m),2.55-2.63(2H,m),2.81-2.88(1H,m),3.14-3.21(2H,m),3.55-3.62 (2H,m),3.97-4.06(2H,m),4.08-4.19(2H,m),4.96(2H,s),6.73-6.83(2H,m),7.07(1H,d,J=11.0Hz),7.11(1H,d,J=7.8Hz),7.22 (1H,t,J
=7.7Hz),8.10(1H,d,J=8.8Hz).

(7) 3-[N-[2-[5-[(4-Cyclohexyl-3-fluorophenyl)methoxy]indolin-1-yl]-2-oxoethyl]amino] propionic acid

[F413]

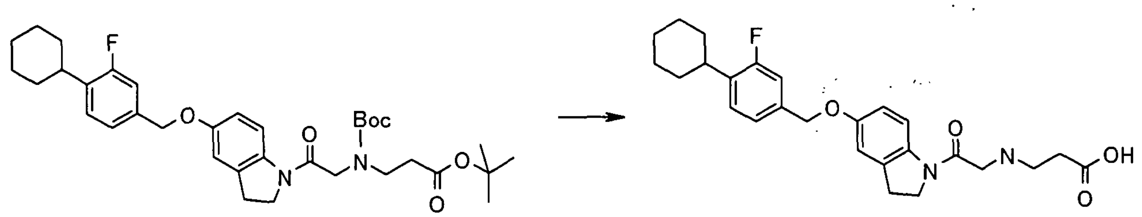

To a solution of 3-[N-(tert-butoxycarbonyl)-N-[2-[5-[(4-cyclohexyl-3-fluorophenyl)methoxy]indolin-1-yl]-2-oxoethyl]amino]propionic acid tert-butyl ester (40.8 mg) in dichloromethane (1.0 mL), TFA (1.0 mL) was added at room temperature, and the mixture was stirred for 1.5 hours at room temperature, followed by concentration under reduced pressure. Dimethyl sulfoxide (2.0 mL) was added to the residue, and insoluble matter was removed. The residue was purified by high-performance liquid chromatography (NOMURA Develosil Combi-RP5), and the target fraction was concentrated and freeze-dried, whereby the title compound (17.7 mg) was yielded.
MS(ESI)m/z:455(M+H)$^+$.
HR-MS(ESI)Calcd for $C_{26}H_{32}FN_2O_4$(M+H)$^+$:455.2346.Found:455.2333.

[Example 91]

3-[N-[2-[5-[[4-Cyclohexyl-3-(N,N-dimethylamino)phenyl]methoxy]indolin-1-yl]-2-oxoethyl]amino]propionic acid

(1) 3-Nitro-4-trifluoromethanesulfonyloxybenzoic acid methyl ester

[F414]

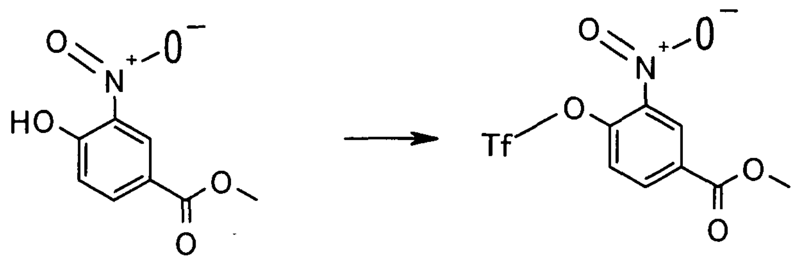

To a solution of 3-nitro-4-hydroxybenzoic acid methyl ester (2.96 g) in dichloromethane (50 mL), pyridine (1.46 mL) and trifluoromethane sulfonic anhydride (2.78 mL) were slowly added at 0°C, and the mixture was stirred for 16 hours at room temperature. Precipitated solid was removed by filtration, and the filtrate was concentrated under reduced pressure. Water (50 mL) and ethyl acetate (70 mL) were added to the residue for phase separation. The aqueous layer was extracted with ethyl acetate (3 x 30 mL). The organic layers were combined and washed with 3% aqueous copper sulfate solution and saturated brine, followed by drying over sodium sulfate anhydrate. The dried product was concentrated under reduced pressure, whereby the title compound (4.64 g) was yielded.
$^1$H-NMR($CDCl_3$)δ:
4.01(3H,s),7.57(1H,d,J=8.8Hz),8.40(1H,dd,J=8.5,2.2Hz),8.80(1H d,J=2.2Hz).

(2) 4-Cyclohexyl-3-nitrobenzoic acid methyl ester (WO 9961406)

[F415]

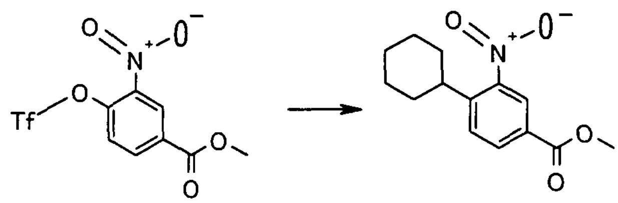

To a solution of 3-nitro-4-trifluoromethanesulfonyloxybenzoic acid methyl ester (1.65 g) in THF (30 mL), bis(tri-tert-butylphosphine)palladium (128 mg) and 0.5M (cyclohexylmethyl)zinc bromide/THF solution (13.0 mL) were added at room temperature under a nitrogen atmosphere, and the mixture was refluxed for 4 hours. The reaction mixture was left to stand to cool to room temperature. Saturated aqueous sodium hydrogencarbonate solution (30 mL) was added to the reaction mixture, followed by stirring for 30 minutes at room temperature. The precipitated solid was removed by filtration, and the filtrate was extracted with chloroform (3 x 25 mL). The extracts were combined and washed with saturated brine, followed by drying over sodium sulfate anhydrate. Insoluble matter was removed by filtration, and the filtrate was concentrated under reduced pressure. The residue was purified by silica gel column chromatography (Biotage 40M), whereby the title compound (733 mg) was yielded.
$^1$H-NMR($CDCl_3$)δ: 1.23-1.33(1H,m), 1.36-1.51(4H,m),1.76-1.94(5H,m),2.99-3.07(1H,m),3.95(3H,s),7.54(1H,d, J=8.3Hz),8.16(1H,dd,J=8.3,1.7 Hz),8.33(1H,d,J=1.7Hz).

(3) 3-Amino-4-cyclohexylbenzoic acid methyl ester (Urgess, Laurence E. Synthetic Communications (1997), 27 (12), 2181-2191)

[F416]

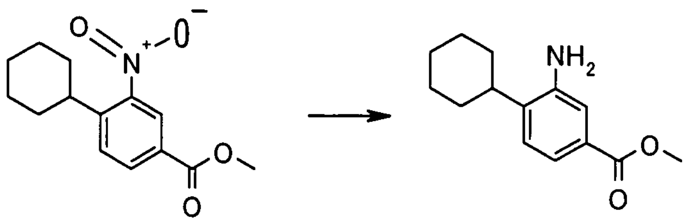

To a solution of 4-cyclohexyl-3-nitrobenzoic acid methyl ester (307 g) in methanol (20 mL), 5% Pd/C (10 mg) was added, and the mixture was stirred for 17 hours at room temperature under a hydrogen atmosphere. The reaction mixture was filtered through a Celite pad. The filtrate was concentrated under reduced pressure, whereby the title compound (270 mg) was yielded.
$^1$H-NMR($CDCl_3$)δ:
1.24-1.48(5H,m),1.76-1.93(5H,m),2.49(1H,br s),3.87(3H,s),7.17(1H,d,J=8.0Hz),7.36(1H,d,J=1.5Hz),7.44(1H,d d, J=8.0,1.5Hz).

(4) 4-Cyclohexyl-3-(N,N-dimethylamino)benzoic acid methyl ester

[F417]

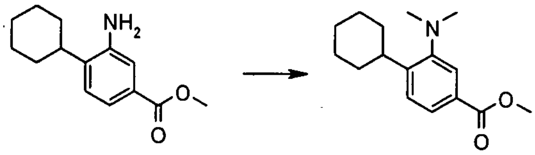

To a solution of 3-amino-4-cyclohexylbenzoic acid methyl ester (270 mg) in methanol (12 mL), 37% aqueous formaldehyde solution (548 μL) and acetic acid (600 μL) were added at room temperature, and the mixture was stirred for 3 hours at room temperature. Sodium cyanoborohydride (437 mg) was added to the reaction mixture at room temperature, followed by stirring for 3 hours. Water (15 mL) was added to the resultant mixture, and the pH thereof was adjusted to 9 with saturated aqueous sodium hydrogencarbonate solution. Chloroform (15 mL) was added to the reaction mixture, and the aqueous layer was extracted with chloroform (3 x 10 mL). The extracts were combined and dried over sodium sulfate anhydrate. Insoluble matter was removed by filtration. The filtrate was concentrated under reduced pressure, whereby the title compound (276 mg) was yielded.
$^1$H-NMR($CDCl_3$)δ: 1.24-1.51(5H,m),1.73-1.88(5H,m),2.69(6H,s),3.12-3.20(1H,m),3.89(3H,s),7.28(1H,d,J=8.0Hz),7.70 (1H,dd,J=8.0,1.7 Hz),7.76(1H,d,J=1.7Hz).
MS(ESI)m/z:262(M+H)$^+$.

(5) 4-Cyclohexyl-3-(N,N-dimethylamino)benzyl alcohol

[F418]

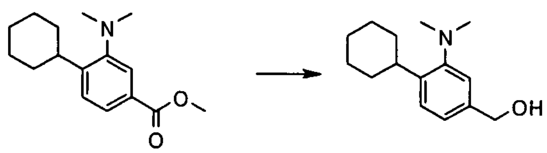

To a suspension of lithium aluminum hydride (131 mg) in THF (5.0 mL), a solution of 4-cyclohexyl-3-(N,N-dimethylamino)benzoic acid methyl ester (276 mg) in THF (2.0 mL) was added dropwise at 0°C, and the mixture was stirred for 2.5 hours at room temperature. Diethyl ether (5.0 mL) and ethyl acetate were slowly added to the reaction mixture until generation of hydrogen terminated. Subsequently, saturated aqueous sodium sulfate solution was slowly added thereto until a certain amount of precipitates were deposited on the bottom of the flask The mixture was stirred at room temperature for 15 hours. The precipitate was removed by filtration by means of a Celite pad, and the filtrate was concentrated under reduced pressure. The residue was purified by silica gel column chromatography (Biotage 25M), whereby the title compound (174 mg) was yielded.
$^1$H-NMR($CDCl_3$)δ: 1.22-1.50(5H,m),1.73-1.87(5H,m),2.68(6H,s),3.06-3.14(1H,m),4.64(2H,d,J=5.6Hz),7.04(1H,dd, J=7.8,1.7Hz),7.11(1H d,J=1.7Hz),7.22(1H,d,J=7.8Hz).

(6) 4-Cyclohexyl-3-(N,N-dimethylamino)benzyl chloride hydrochloride

[F419]

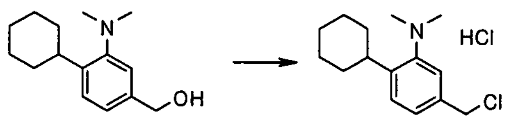

To a solution of 4-cyclohexyl-3-(N,N-dimethylamino)benzyl alcohol (174 mg) in dichloromethane (5.0 mL), thionyl chloride (109 μL) was added at room temperature, and the mixture was refluxed for 1.5 hours. The reaction mixture was left to stand to cool to room temperature and concentrated under reduced pressure, whereby the title compound (202 mg) was yielded.
$^1$H-NMR ($CDCl_3$) δ:
1.21-1.35(1H,m),1.43-1.53(2H,m),1.67-1.89(7H,m),3.27(6H,s),3.60(1H,br s),4.60(2H,s),7.52-7.44(3H,m).

(7) 3-[N-(tert-Butoxycarbonyl)-N-[2-[5-[[4-cyclohexyl-3-(N,N-dimethylamino)phenyl]methoxy]indolin-1-yl]-2-oxoethyl] amino]propionic acid tert-butyl ester

[F420]

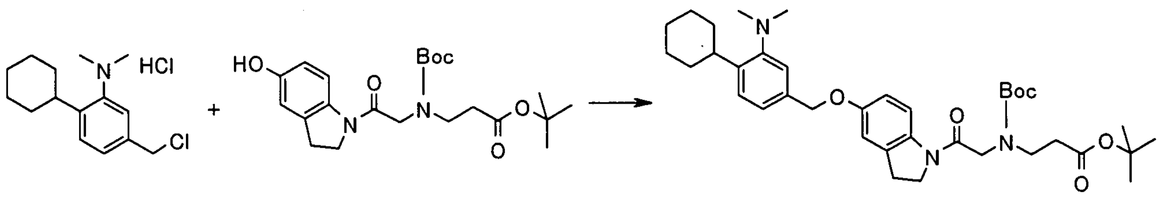

To a solution of 4-cyclohexyl-3-(N,N-dimethylamino)benzyl chloride hydrochloride (202 mg) in DMF (7.5 mL), potassium carbonate (531 mg) and 3-[N-tert-butoxycarbonyl-2-oxo-2-(5-hydroxyindolin-1-yl)ethylamino]propionic acid

tert-butyl ester (294 mg) were added at room temperature, and the mixture was stirred for 18 hours at 75°C. The reaction mixture was left to stand to cool to room temperature and concentrated under reduced pressure. Water (10 mL) and chloroform (15 mL) were added to the residue for phase separation. The aqueous layer was extracted with chloroform (2 x 10 mL). The organic layers were combined, dried over sodium sulfate anhydrate, and concentrated under reduced pressure. The residue was purified by silica gel column chromatography (Biotage 25M), whereby the title compound (345 mg) was yielded.
$^1$H-NMR($CDCl_3$)δ:
1.25-1.51(23H,m),1.73-1.87(5H,m),2.55-2.64(2H,m),2.68(6H,s),3.07-3.22(3H,m),3.56-3.62(2H,m),3.98-4.06(2H,m), 4.08-4.18(2H,m),4.94(2H,br s),6.77-6.86(2H,m),7.09(1H,d,J=7.8Hz),7.14(1H,d,J=1.7Hz),7.23(1H,d,J= 7.8Hz), 8.15-8.09(1H,m).
MS(ESI)m/z:636(M+H)$^+$.

(8) 3-[N-[2-[5-[[4-Cyclohexyl-3-(N,N-dimethylamino)phenyl]methoxy]indolin-1-yl]-2-oxoethyl]amino]propionic acid

[F421]

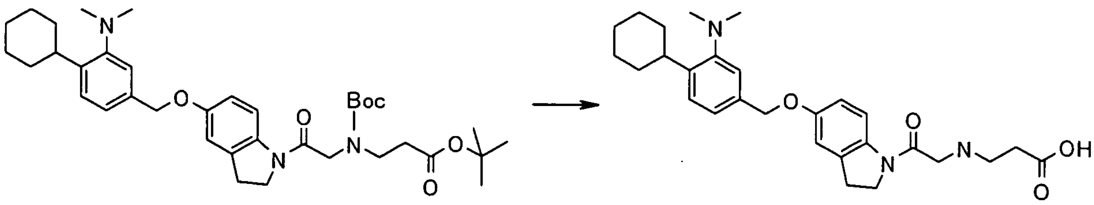

To a solution of 3-[N-(tert-butoxycarbonyl)-N-[2-[5-[[4-cyclohexyl-3-(N,N-dimethylamino)phenyl]methoxy]indolin-1-yl]-2-oxoethyl]amino]propionic acid tert-butyl ester (345 mg) in dichloromethane (1.6 mL), TFA (0.40 mL) was added at room temperature, and the mixture was stirred for 17 hours at room temperature. The reaction mixture was concentrated under reduced pressure. Dimethyl sulfoxide (4.0 mL) was added to the residue, and insoluble matter was removed. The resultant product was purified by high performance liquid chromatography (NOMURA Develosil Combi-RP5). The target fraction was concentrated, and the precipitated solid was collected by filtration. The resultant solid was washed with water and dried, whereby the title compound (116 mg) was yielded.
$^1$H-NMR(DMSO-$d_6$)δ:
1.16-1.39(5H,m),1.55-1.75(5H,m),2.33(2H,t,J=6.7Hz),2.53(6H,s),2.81(2H,t,J=6.7Hz),2 .95-3.03(1H,m),3.06(2H,t, J=8.4Hz),3.56(2H,s),3.97(2H,t,J=8.4Hz),4 90(2H,s),6.75(1H,dd,J=8.8,2.4Hz),6.87(1H,s),7.01(1H,d,J=8.1H z),7.10(1H, s),7.15(1H,d,J=8.1Hz),7.90(1H,d,J=8.8Hz).
MS(ESI)m/z:480(M+H)$^+$.
Anal. Calcd for $C_{28}H_{37}N_3O_4$·
1.0$H_2O$,0.25$CF_3CO_2H$:C,65.06;H,7.52;N,7.99.Found:C,64.93;H,7.47; N,7.91.

[Example 92]

3-[N-[2-[5-[(3-Cyano-4-isopropylphenyl)methoxy]indolin-1-yl]-2-oxoethyl]amino]propionic acid

(1) 3-Cyano-4-isopropenylbenzoic acid methyl ester

[F422]

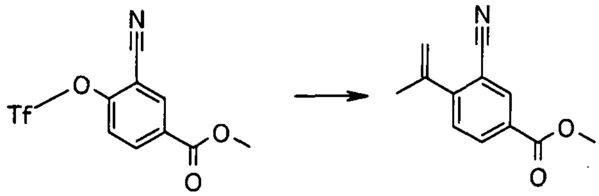

To a toluene solution of 3-cyano-4-trifluoromethanesulfonyloxybenzoic acid methyl ester (375 mg), tetrakis (triphenylphosphine)palladium (210 mg), water (2.5 mL), cesium carbonate (2.92 g), and isopropenylboric acid pinacol ester (456 μL) were added at room temperature under a nitrogen atmosphere, and the mixture was stirred for 12 hours at 90°C. The reaction mixture was left to stand to cool to room temperature and concentrated under reduced pressure. Saturated aqueous sodium hydrogencarbonate solution (10 mL) and ethyl acetate (10 mL) were added to the residue for phase separation. The aqueous layer was extracted with ethyl acetate (3 x 7.5 mL). The extracts were combined and washed with saturated brine, followed by drying over sodium sulfate anhydrate. Insoluble matter was removed by filtration, and the filtrate was concentrated under reduced pressure. The residue was purified by silica gel column chromatography (Biotage 25M), whereby the title compound (164 mg) was yielded.
$^1$H-NMR($CDCl_3$)δ:
2.21(3H,t,J=0.7Hz),3.95(3H,s),5.34(1H,d,J=1.0Hz),5.46(1H,d,J= 1.0Hz),7.46(1H,d,J=8.1Hz),8.18(1H,ddd, J=8.1,2.0,0.7Hz),8.33(1 H,br s).
MS(ESI)m/z:202(M+H)$^+$.

(2) 3-Cyano-4-isopropylbenzoic acid methyl ester

[F423]

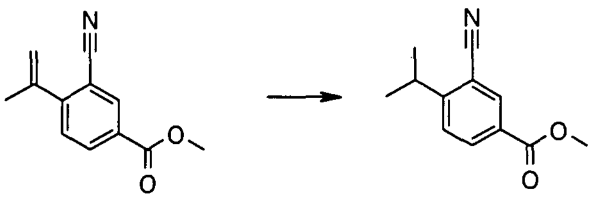

To a solution of 3-cyano-4-isopropenylbenzoic acid methyl ester (164 mg) in methanol (5.0 mL), 5% Pd/C (5 mg) was added, and the mixture was stirred for 4.5 hours at room temperature under hydrogen. The reaction mixture was filtered through a Celite pad, and the filtrate was concentrated under reduced pressure, whereby the title compound (126 mg) was yielded.
$^1$H-NMR($CDCl_3$)δ:
1.34(6H,d,J=6.8Hz),3.44(1H,dq,J=6.8,6.8Hz),3.94(3H,s),7.49(1H d,J=8.3Hz),8.19(1H,dd,J=8.3,1.7Hz),8.28(1H,d, J=1.7Hz).
MS(ESI)m/z:204(M+H)$^+$.

(3) 3-Cyano-4-isopropylbenzoic acid

[F424]

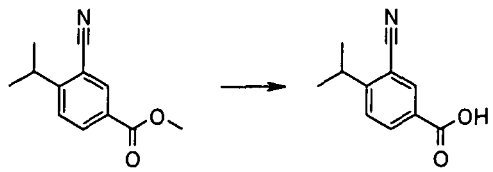

To a solution of 3-cyano-4-isopropylbenzoic acid methyl ester (126 mg) in THF (2.0 mL), methanol (1.0 mL) and 1N aqueous sodium hydroxide solution (1.00 mL) were added at room temperature, and the mixture was stirred for 3 days. Water (5.0 mL) was added to the reaction mixture, and the pH thereof was adjusted to 3 with 1N hydrochloric acid. Chloroform (10 mL) was added thereto for phase separation. The aqueous layer was extracted with chloroform (2 x 5 mL). The organic layers were combined and dried over sodium sulfate anhydrate. Solvent was removed under reduced pressure, whereby the title compound (110 mg) was yielded.
$^1$H-NMR(DMSO-$d_6$)δ:
1.36(6H,d,J=6.8Hz),3.43-3.51(1H,m),7.54(1H,d,J=8.3Hz),8.26(1H,dd,J=8.3,1.5Hz),8.36(1H ,d,J=1.5Hz).
MS (ESI) m/z : 379 (2M+H) $^+$.

(4) 3-Cyano-4-isopropylbenzyl alcohol

[F425]

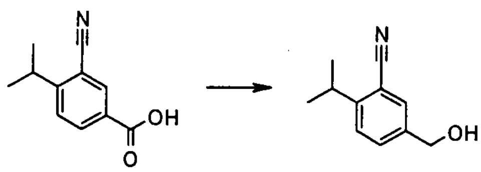

To a solution of 3-cyano-4-isopropylbenzoic acid (110 mg) in THF (3.0 mL), TEA (122 μL) and ethyl chloroacetate (66.8 μL) were slowly added at 0°C, and the mixture was stirred for 30 minutes at room temperature. Precipitated matter was removed by filtration. In another flask, the obtained filtrate was slowly added to a suspension of sodium borohydride (144 mg) in ethanol (2.0 mL) at 0°C, and the mixture was stirred for 2 hours at room temperature. 1N Hydrochloric acid was carefully added thereto to adjust the pH thereof to 4. Water (5 mL) and ethyl acetate (10 mL) were added to the reaction mixture for phase separation. The aqueous layer was extracted with ethyl acetate (3 x 5.0 mL). The extracts were combined and dried over sodium sulfate anhydrate, and the filtrate was concentrated under reduced pressure, whereby the title compound (84.9 mg) was yielded.
$^1$H-NMR($CDCl_3$)δ:
1.30(6H,d,J=6.8Hz),3.33-3.40(1H,m),4.69(2H,s),7.38(1H,d,J=8.3Hz),7.53(1H,dd,J=8.3,2.0 Hz),7.60(1H,d,J=2.0Hz).

(5) 3-Cyano-4-isopropylbenzyl chloride

[F426]

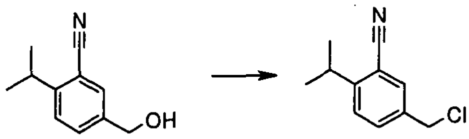

To a solution of 3-cyano-4-isopropylbenzyl alcohol (84.9 mg) in dichloromethane (2.0 mL), thionyl chloride (70.7 μL) was added at room temperature, and the mixture was refluxed for 3 hours. The reaction mixture was left to stand to cool to room temperature and concentrated under reduced pressure. The residue was purified by silica gel column chromatography (Biotage 25S), whereby the title compound (84.5 mg) was yielded.
$^1$H-NMR($CDCl_3$)δ:
1.31(6H,d,J=6.8Hz),3.35-3.41(1H,m),4.56(2H,s),7.40(1H,d,J=8.3Hz),7.57(1H,dd,J=8.3,2.1 Hz),7.63(1H,d,J=2.1Hz).
MS(ESI)m/z:193(M)$^+$.

(6) 3-[N-(tert-Butoxycarbonyl)-N-[2-[5-[(3-cyano-4-isopropylphenyl)methoxy]indolin-1-yl]-2-oxoethyl]amino]propionic acid tert-butyl ester

[F427]

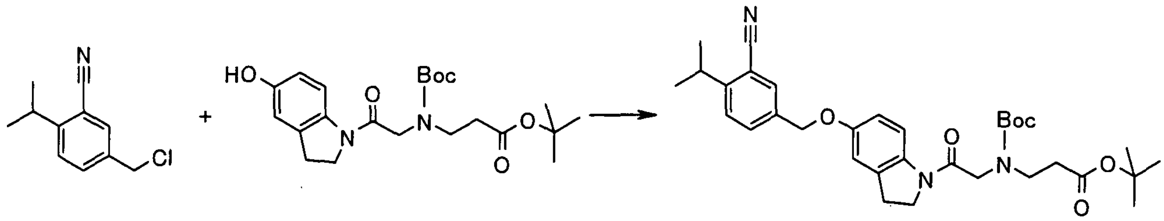

To a solution of 3-cyano-4-isopropylbenzyl chloride (84.5 mg) in DMF (2.0 mL), potassium carbonate (211 mg) and 3-[N-(tert-butoxycarbonyl)-N-[2-(5-hydroxyindolin-1-yl)-2-oxoethyl]amino]propionic acid tert-butyl ester (184 mg) were added at room temperature, and the mixture was stirred for 4.5 hours at 60°C. The reaction mixture was left to stand to cool to room temperature and concentrated under reduced pressure. Water (5 mL) and chloroform (10 mL) were added to the residue for phase separation. The aqueous layer was extracted with chloroform (2 x 10 mL). The organic layers were combined and dried over sodium sulfate anhydrate. Insoluble matter was removed by filtration, and the filtrate was concentrated under reduced pressure. The residue was purified by silica gel column chromatography (Biotage 25S), whereby the title compound (202 mg) was yielded.
$^1$H-NMR ($CDCl_3$)δ: 1.32(6H,d,J=6.8Hz),1.40-1.59(18H,m),2.55-2.63(2H,m),3.15-3.23(2H,m), 3.36-3.42(1H,m), 3.55-3.61(2H,m),3.99-4.19(4H,m),5.00(2H,s),6.73-6.84(2H,m),7.40(1H,d,J=8.0Hz),7.58(1H,dd,J=8.3,1.5Hz),7.67(1H , d,J=1.5Hz),8.12(1H,dd,J=8.8,8.8Hz).
MS(ESI)m/z:578(M+H)$^+$.

(7) 3-[N-[2-[5-[(3-Cyano-4-isopropylphenyl)methoxy]indolin-1-yl]-2-oxoethyl]amino]propionic acid

[F428]

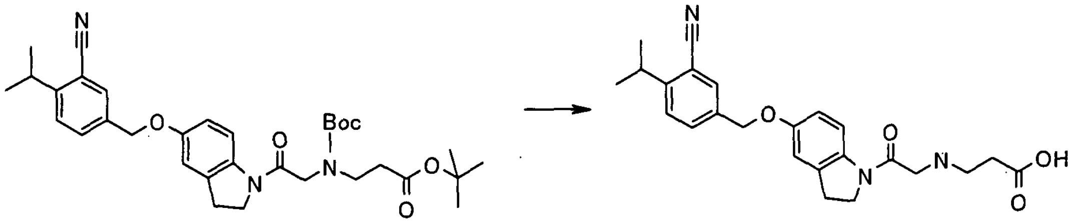

To a solution of 3-[N-(tert-butoxycarbonyl)-N-[2-[5-[(3-cyano-4-isopropylphenyl)methoxy]indolin-1-yl]-2-oxoethyl]amino]propionic acid tert-butyl ester (202 mg) in dichloromethane (2.4 mL), TFA (0.60 mL) was added at room temperature, and the mixture was stirred for 3 hours at room temperature. The reaction mixture was concentrated under reduced pressure. Dimethyl sulfoxide (6.0 mL) was added to the residue, and insoluble matter was removed. The resultant product was purified by high performance liquid chromatography (NOMURA Develosil Combi-RP5), and the target fraction was concentrated and freeze-dried, whereby the title compound (109 mg) was yielded.
$^1$H-NMR (DMSO-$d_6$) δ :
1.26 (6H,d,J=7.1Hz), 2.35(2H,t,J=6.6Hz),2.81(2H,t,J=6.6Hz),3.11 (2H, t, J=8.0Hz), 3.19-3.27 (1H,m),3.53(2H,s),4.03 (2H,t,J=8.0Hz),5.07(2H,s),6.81(1H,d d,J=8.8,2.4Hz),6.94(1H,d,J=2.4Hz),7.56(1H,d,J=8.3Hz),7.71(1H, dd, J=8.3, 2.0Hz), 7.81 (1H,d,J=2.0Hz), 7.97 (1H,d,J=8.8Hz).
MS (ESI) m/z: 421 (M)$^+$.
Anal. Calcd for $C_{24}H_{27}N_3O_4$• $0.25H_2O$, $0.25CF_3CO_2H$: C, 64.74;H, 6.15;N, 9.25. Found: C, 64.42; H,6.15 ;N,9.24.

[Example 93]

3-[N-[2- 5-[(4-Cyclohexyl-2-fluoro-phenyl)methoxy]indolin-1-yl]-2-oxoethyl]amino]propionic acid

(1) 2-Fluoro-4-hydroxybenzoic acid methyl ester (WO 9734885)

[F429]

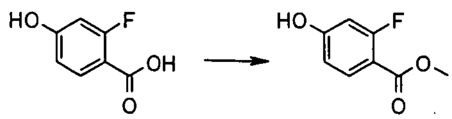

To a solution of 2-fluoro-4-hydroxybenzoic acid (5.70 g) in methanol (60 mL), thionyl chloride (2.87 mL) was slowly added at room temperature, and the mixture was refluxed for 3 hours. The reaction mixture was left to stand to cool to room temperature. The reaction mixture was concentrated under reduced pressure, whereby the title compound (3.89 g) was yielded.
$^1$H-NMR ($CDCl_3$) δ:
3.77(3H,s),6.63(1H,dd,J=13.1,2.3Hz),6.69(1H,dd,J=8.8,2.3Hz),7 .74(1H,t,J=8.8Hz),10.82(1H,br s).

(2) 2-Fluoro-4-trifluoromethanesulfonyloxybenzoic acid methyl ester

[F430]

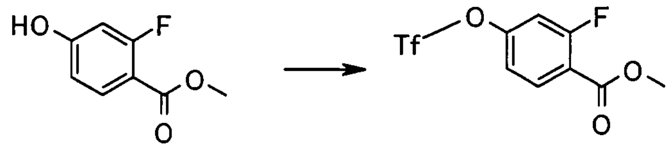

To a solution of 2-fluoro-4-hydroxybenzoic acid methyl ester (3.89 g) in dichloromethane (70 mL), pyridine (2.31 mL) and trifluoromethanesulfonic anhydride (4.23 mL) were slowly added at 0°C, and the mixture was stirred for 16 hours at room temperature. The precipitated solid was removed by filtration, and the filtrate was concentrated under reduced pressure. Water (50 mL) and chloroform (70 mL) were added to the residue for phase separation. The aqueous layer was extracted with ethyl acetate (3 x 30 mL). The organic layers were combined and washed with 3% aqueous copper sulfate solution and saturated brine, followed by drying over sodium sulfate anhydrate. Insoluble matter was removed by filtration, and the filtrate was concentrated under reduced pressure, whereby the title compound (4.75 g) was yielded.
$^1$H-NMR ($CDCl_3$) δ:
3.96(3H,s),7.14(1H,dd,J=10. 0,2. 2Hz),7.18(1H,ddd,J=8.5,2.2,1.0 Hz), 8.08(1H,t,J=8.5Hz).

(3) 4-Cyclohexyl-2-fluorobenzoic acid methyl ester

[F431]

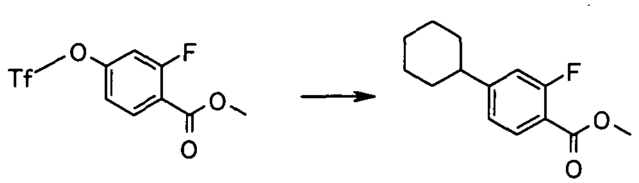

Under nitrogen, to a solution of 2-fluoro-4-trifluoromethanesulfonyloxybenzoic acid methyl ester (1.21 g) in N, N-dimethylacetamide (20 mL), copper(I) iodide (76.1 mg), [1,1'-bis(diphenylphosphino)ferrocene]palladium dichloride-dichloromethane complex (163 mg), and 0.5M cyclohexylzinc bromide/THF solution (9.6 mL) were added at room temperature, and the mixture was refluxed for 2 hours. The resultant mixture was left to stand to cool to room temperature. Saturated aqueous ammonium chloride solution (40 mL) was added thereto, and the reaction mixture was stirred for 30 minutes at room temperature. The precipitated solid was removed by filtration. The filtrate was extracted with chloroform (3 x 30 mL). The organic layers were combined and washed with saturated brine, followed by drying over sodium sulfate anhydrate. Insoluble matter was removed by filtration, and the filtrate was concentrated under reduced pressure. The residue was purified by silica gel column chromatography (Biotage 40M), whereby the title compound (713 mg) was yielded.
$^1$H-NMR ($CDCl_3$) δ:
1.19-1.45(5H,m), 1.73-1.91(5H,m), 2.54(1H,br s),3.91(3H,s),6.97(1H,d,J=12.2Hz),7.04(1H,dd,J=7.8,1.7Hz),7.8 5 (1H, t, J=7. 8Hz) .
MS (ESI) m/z : 237 $(M+H)^+$.

(4) 4-Cyclohexyl-2-fluorobenzoic acid

[F432]

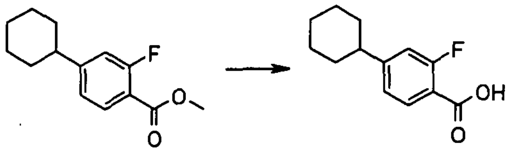

To a solution of 4-cyclohexyl-2-fluorobenzoic acid methyl ester (713 mg) in THF (8.0 mL), methanol (4.0 mL) and 1N aqueous sodium hydroxide solution (4.00 mL) were added at room temperature, and the mixture was stirred for 15 hours at room temperature. Water (20 mL) was added to the reaction mixture, and the pH thereof was adjusted to 3 with 1N hydrochroric acid. Chloroform (40 mL) was added to the pH-adjusted mixture for phase separation. The aqueous layer was extracted with chloroform (3 x 20 mL). The organic layers were combined and dried over sodium sulfate anhydrate. Solvent was removed under reduced pressure, whereby the title compound (603 mg) was yielded.
$^1$H -NMR ($CDCl_3$) δ :
1.21-1.46(5H,m),1.74-1.92(5H,m),2.52-2.60(1H,m),7.01(1H,dd,J=11.7,1.6Hz),7.08(1H,dd,J=8.2,1.6Hz),7 .94(1H,t, J=8.2Hz).
MS (ESI) m/z : 223 $(M+H)^+$.

(5) 4-Cyclohexyl-2-fluorobenzyl alcohol

[F433]

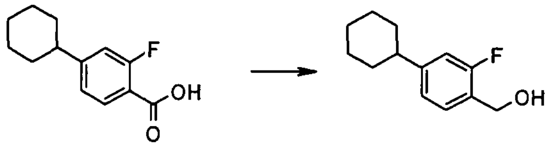

To a solution of 4-cyclohexyl-2-fluorobenzoic acid (603 mg) in THF (8.0 mL), TEA (567 μL) and ethyl chloroacetate (311 μL) were slowly added at 0°C, and the mixture was stirred for 30 minutes at room temperature. The precipitate was removed by filtration.
In another flask, to a suspension of sodium borohydride (670 mg) in ethanol (10 mL), the filtrate containing the above-obtained benzoic acid derivative was slowly added at 0°C, and the mixture was stirred for 1.5 hours at 0°C. 1N Hydrochloric acid was carefully added thereto so as to adjust the pH thereof to 4. To the reaction mixture, water (20 mL) and ethyl acetate (30 mL) were added for phase separation. The aqueous layer was extracted with ethyl acetate (3 x 20 mL). The organic layers were combined and dried over sodium sulfate anhydrate. Insoluble matter was removed by filtration, and the filtrate was concentrated under reduced pressure. The residue was purified by silica gel column chromatography (Biotage 40S), whereby the title compound (478 mg) was yielded.
$^1$H-NMR ($CDCl_3$) δ : 1.18-1.30(1H,m),1.31-1.45(4H,m),1.68-1.75(1H,m),1.75-1.89(4H,m),2.45-2.53 (1H, m), 4.71 (2H, d, J=6.1Hz), 6.90 (1H, dd,J=11.7,1.5Hz), 6.98 (1 H,dd,J=7.8,1.5Hz),7.30(1H,t,J=7.9Hz).

(6) 4-Cyclohexyl-2-fluorobenzyl chloride

[F434]

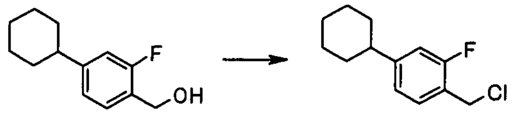

To a solution of 4-cyclohexyl-2-fluorobenzyl alcohol (478 mg) in dichloromethane (10 mL), thionyl chloride (335 μL) was added at room temperature, and the mixture was refluxed for 1.5 hours. The reaction mixture was left to stand to cool to room temperature and concentrated under reduced pressure, whereby the title compound (461 mg) was yielded.
$^1$H-NMR ($CDCl_3$) δ :
1.19-1.42(5H,m),1.75(1H,d,J=12.5Hz),1.82-1.89(4H,m),-2.50(1H,br s), 4.61(2H,s), 6.92(1H,dd,J=11.2,2. 0Hz), 6.98 (1H,dd,J=7.8,2. 0Hz ), 7.30(1H,t,J=7.8Hz).

(7) 3-[N-(tert-Butoxycarbonyl)-N-[2-[5-[(4-cyclohexyl-2-fluoro-phenyl)methoxy]indolin-1-yl]-2-oxoethyl]amino]propionic acid tert-butyl ester

[F435]

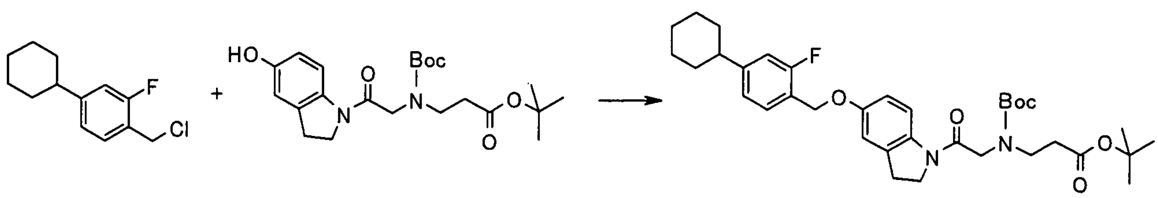

To a solution of 4-cyclohexyl-2-fluorobenzyl chloride (113 mg) in DMF (3.0 mL), potassium carbonate (242 mg) and 3-[N-(tert-butoxycarbonyl)-N-[2-(5-hydroxyindolin-1-yl)-2-oxoethyl]amino]propionic acid tert-butyl ester (210 mg) were added at room temperature, and the mixture was stirred for 4.5 hours at 60°C. The reaction mixture was left to stand to cool to room temperature and concentrated under reduced pressure. Water (5 mL) and ethyl acetate (10 mL) were added to the residue for phase separation. The aqueous layer was extracted with ethyl acetate (2 x 10 mL). The organic layers were combined and dried over sodium sulfate anhydrate, followed by concentration under reduced pressure. The residue was purified by silica gel column chromatography (Biotage 25S), whereby the title compound (265 mg) was yielded.
$^1$H-NMR ($CDCl_3$) δ: 1.23-1.58(23H,m),1.70-1.89(5H,m),2.45-2.63(3H,m),3.14-3.22(2H,m),3.56-3.61(2H,m),3.97-4.18 (4H,m),5.04(2H,s),6.76-6.85(2H,m),6.88-7.02(2H,m),7.37(1H,dd,J=7.3,7.3Hz),8.11(1H,dd,J=8.8,8.8Hz).

(8) 3-[N-[2-[5-[(4-Cyclohexyl-2-fluorophenyl)methoxy]indolin-1-yl]-2-oxoethyl]amino]propionic acid

[F436]

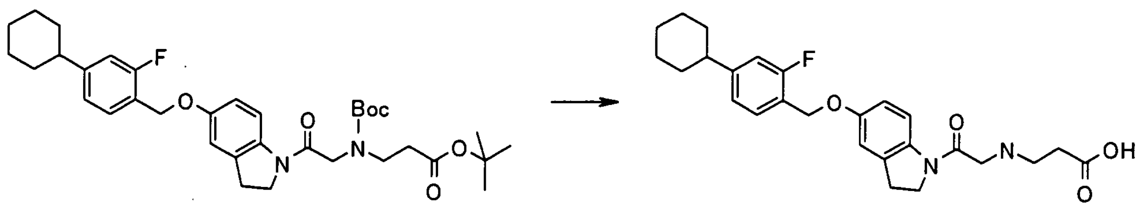

To a solution of 3-[N-(tert-butoxycarbonyl)-N-[2-[5-[(4-cyclohexyl-2-fluoro-phenyl)methoxy]indolin-1-yl]-2-oxoethyl]amino]propionic acid tert-butyl ester (265 mg) in dichloromethane (2.4 mL), TFA (0.60 mL) was added at room temperature, and the mixture was stirred for 3 hours at room temperature. The reaction mixture was concentrated under reduced pressure. Dimethyl sulfoxide (3.0 mL) was added to the residue, and insoluble matter was removed. The resultant residue was purified by high-performance liquid chromatography (NOMURA Develosil Combi-RP5), and the target fraction was concentrated. Precipitated solid was collected by filtration and washed with water, followed by drying, whereby the title compound (94.2 mg) was yielded.
MS (ESI) m/z: 455 $(M+H)^+$.
Anal. Calcd for $C_{26}H_3,FN_2O_4$•
$0.25H_2O$,:C,68.03; H,6.92; F,4.14; N,6.10. Found:C,68.15; H,6.84; F, 4.21;N,6.06.

[Example 94]

3- [N- [2- [5- (4-Ethyl-3-trifluoromethylbenzyloxy) indolin-1-yl] - 2-oxoethyl]amino]propionic acid hydrochloride

(1) 3-[N-(Benzyloxycarbonylmethyl)amino]propionic acid ethyl ester

[F437]

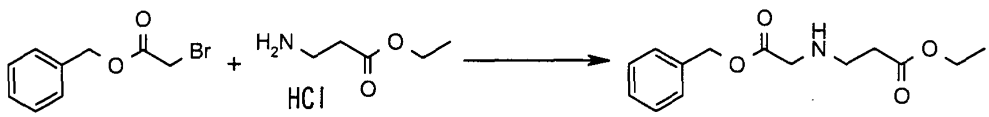

To an acetonitrile solution (650 mL) of bromoacetic acid benzyl ester (10.7 mL), β-alanine ethyl ester hydrochloride (25.0 g) and DIEA (55.3 mL) were added, and the mixture was stirred overnight at 80°C. The reaction mixture was left to stand to cool to room temperature and concentrated under reduced pressure. Saturated aqueous sodium bicarbonate solution was added to the residue, followed by extraction thrice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column 4L), whereby the title compound (16.9 g) was yielded.
$^1H$-NMR ($CDCl_3$) δ:
1.26(3H,t,J=7.2Hz),2.50(2H,t,J=6.6Hz),2.91(2H,t,J=6.6Hz),3.47 (2H,s),4.15(2H,q,J=7.2Hz),5.17(2H,s),7.32-7.37(5H, m).
MS (ESI) m/z:266 $(M+H)^+$.

(2) 3-[N-(Benzyloxycarbonylmethyl)-N-(tert-butoxycarbonyl)amino]propionic acid ethyl ester

[F438]

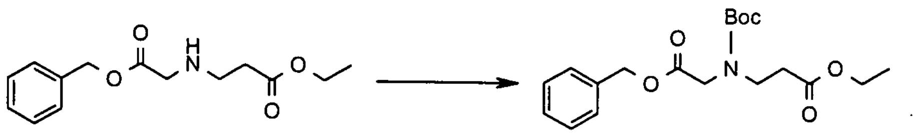

To a dichloromethane solution (200 mL) of 3-[N-(benzyloxycarbonylmethyl)amino]propionic acid ethyl ester (16.9 g), $Boc_2O$ (16.7 g) and saturated aqueous sodium bicarbonate solution (200 mL) were added, and the mixture was stirred overnight at room temperature. The reaction mixture was extracted thrice, each with chloroform. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column 5L), whereby the title compound (23.4 g) was yielded.
$^1$H-NMR ($CDCl_3$) δ : 1.22-1.28(3H,m),1.34-1.47(9H,m),2.58-2.65(2H,m),3.51-3.59(2H,m),4.01-4.15(4H,m),5.15(2H,s), 7.33-7.36(5H,m).
MS (ESI) m/z: 366 $(M+H)^+$.

(3) 3-[N-(tert-Butoxycarbonyl)-N-(carboxymethyl)amino]propionic acid ethyl ester

[F439]

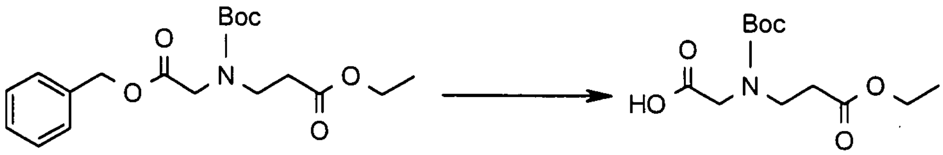

To an ethanol solution (300 mL) of 3-[N-(benzyloxycarbonylmethyl)-N-(tert-butoxycarbonyl)amino]propionic acid ethyl ester (23.3 g), 5% Pd/C (hydrous) (5.00 g) was added, and the mixture was stirred for 4 hours at room temperature under a hydrogen atmosphere. The reaction mixture was filtered, and the filtrate was concentrated under reduced pressure, whereby the title compound (16.8 g) was yielded.
$^1$H-NMR ($CDCl_3$) δ : 1.26(3H,t,J=7. 0Hz),1.42-1.48(9H,m),2.59-2.64(2H,m),3.52-3.58 (2H,m), 4.02-4.16 (4H,m).
MS (ESI) m/z: 276 $(M+H)^+$.

(4) 3-[N-[2-(5-Benzyloxyindolin-1-yl)-2-oxoethyl]-N-(tert-butoxycarbonyl)amino]propionic acid ethyl ester

[F440]

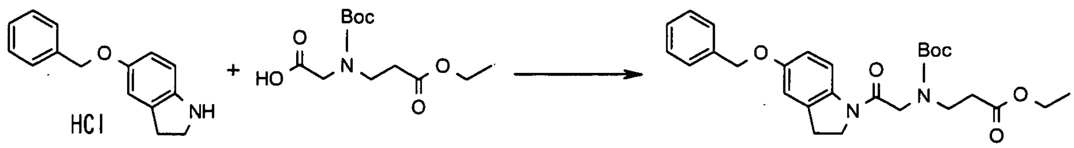

To a DMF solution (30 mL) of 5-benzyloxyindoline hydrochloride (1.54 g), 3-[N-(tert-butoxycarbonyl)-N-(carboxymethyl)amino]propionic acid ethyl ester (2.14 g), DIEA (3.00 mL), HOBt (1.03 g), and EDC•HCl (1.47 g) were added, and the mixture was stirred overnight at room temperature. Saturated aqueous sodium bicarbonate solution was added to the reaction mixture, followed by extraction thrice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column 3L), whereby the title compound (2.70 g) was yielded.
$^1$H-NMR ($CDCl_3$) δ : 1.21-1.28(3H,m),1.40-1.49(9H,m),2.64-2.71(2H,m),3.15-3.21(2H,m),3.60-3.63(2H,m),3.98-4.18 (6H,m),5.02-5.03 (2H,m), 6.76-6.83 (2H,m), 7.30-7.43 (5H,m), 8.09-8.13 (1H,m).
MS (ESI)m/z:483 $(M+H)^+$.

(5) 3-[N-(tert-Butoxycarbonyl)-N-[2-(5-hydroxyindolin-1-yl)-2-oxoethyl]amino]propionic acid ethyl ester

[F441]

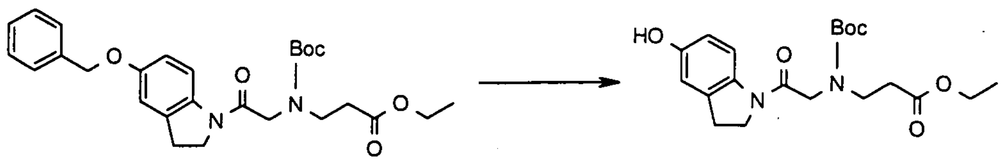

To an ethanol solution (60 mL) of 3-[N-[2-(5-benzyloxyindolin-1-yl)-2-oxoethyl]-N-(tert-butoxycarbonyl)amino] propionic acid ethyl ester (2.70 g), 5% Pd/C (hydrous) (1.00 g) was added, and the mixture was stirred for 4 hours at room temperature under hydrogen. The reaction mixture was filtered, and the filtrate was concentrated under reduced pressure. The residue was purified by flash column chromatography (Yamazen Hi-Flash column 3L), followed by concentration under reduced pressure. The residue was suspended in n-Hexane for washing, filtered, and dried, whereby the title compound (2.01 g) was yielded.
$^1$H-NMR ($CDCl_3$) δ: 1.23-1.28(3H,m),1.40-1.53(9H,m),2.65-3.12(4H,m),3.60-3.92(4H,m),4.08-4.15(4H,m),6.55-6.69 (2H,m),7.19-7.26(1H,m),7.86-8.05(1H,m).
MS (ESI) m/z : 393 $(M+H)^+$.

(6) 3-Trifluoromethyl-4-vinylbenzoic acid methyl ester

[F442]

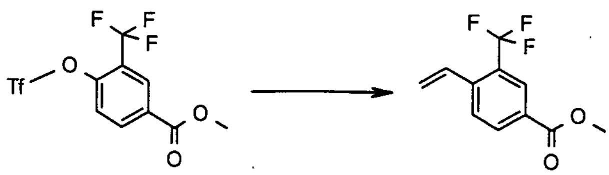

To a toluene solution (9.0 mL) of 4-trifluoromethanesulfonyloxy-3-trifluoromethylbenzoic acid methyl ester (1.06 g), 4,4,5,5-tetramethyl-2-vinyl-[1.3.2]dioxaborolane (601 mg), cesium carbonate (2.93 g), water (4.5 mL), and tetrakis (triphenylphosphine)palladium (0) (347 mg) were added, and the mixture was stirred overnight at reflux. The reaction mixture was left to stand to cool to room temperature. Saturated aqueous sodium bicarbonate solution was added thereto, followed by extraction thrice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column L), whereby the title compound (596 mg) was yielded.
$^1$H-NMR ($CDCl_3$) δ: 3.95(3H,s),5.55(1H,d,J=11.0Hz), 5.86(1H,d,J=17.4Hz),7.08-7.16(1H,m),7.74(1H,d,J=8.3Hz), 8.15-8.18(1H,m),8.31-8.31(1H,m).
MS (ESI) m/z : 231 (M+H) $^+$.

(7) 4-Ethyl-3-trifluoromethylbenzoic acid methyl ester

[F443]

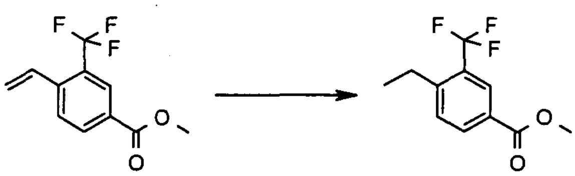

To an ethyl acetate solution (10 mL) of 3-trifluoromethyl-4-vinylbenzoic acid methyl ester (230 mg), 5% Pd/C (hydrous) (100 mg) was added, and the mixture was stirred for 1 hour at room temperature under a hydrogen atmosphere. The reaction mixture was filtered to remove the catalyst. The filtrate was concentrated under reduced pressure, whereby the title compound (239 mg) was yielded.
$^1$H-NMR ($CDCl_3$) δ:
1.28 (3H,t,J=7.5Hz),2.88(2H,q,J=7.5Hz),3.94(3H,s),7.44(1H,d,J= 8.1Hz),8.13(1H,d,J=8.1Hz),8.29(1H,s).
MS (ESI) m/z : 233 (M+H) $^+$.

(8) (4-Ethyl-3-trifluoromethylphenyl)methanol

[F444]

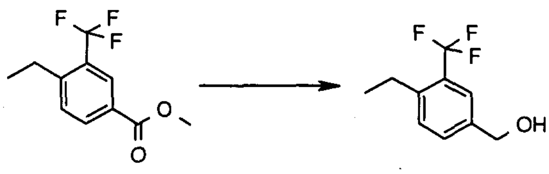

To a THF solution (10 mL) of 4-ethyl-3-trifluoromethylbenzoic acid methyl ester (232 mg), lithium borohydride (65.3 mg) was added, and the mixture was stirred for 1.5 hours under reflux. The reaction mixture was left to stand to cool to room temperature. 1N Hydrochloric acid was added thereto, and the resultant mixture was extracted thrice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column L), whereby the title compound (200 mg) was yielded.
$^1$H-NMR ($CDCl_3$) δ:
1.25(3H,t,J=7.5Hz),1.75(1H,s),2.82(2H,q,J=7. 5Hz),4.71(2H,s),7 .34(1H,d,J=8.1Hz),7.47(1H,d,J=8.1Hz),7.61(1H,s).

(9) 4-Chloromethyl-l-ethyl-2-trifluoromethylbenzene

[F445]

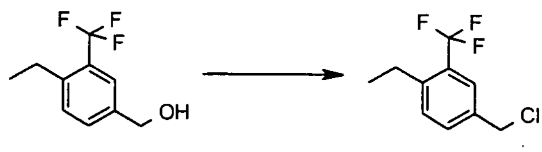

To a 1,2-dichloroethane solution (10 mL) of (4-ethyl-3-trifluoromethylphenyl)methanol (190 mg), thionyl chloride (338 μL) and DMF (1 drop by means of a Pasteur pipette) were added, and the mixture was stirred overnight at 50°C. The reaction mixture was left to stand to cool to room temperature and concentrated under reduced pressure. The residue was purified by flash column chromatography (Yamazen Hi-Flash column L), whereby the title compound (184 mg) was yielded.
$^1$H-NMR ($CDCl_3$) δ:
1.25(3H,t,J=7.5Hz),2.82(2H,q,J=7.5Hz),4.59(2H,s),7.35(1H,d,J= 7.8Hz),7.50(1H,d,J=7.8Hz),7.62(1H,s).

(10) 3-[N-(tert-Butoxycarbonyl)-N-[2-[5-(4-ethyl-3-trifluoromethylbenzyloxy)indolin-1-yl]-2-oxoethyl]amino]propionic acid ethyl ester

[F446]

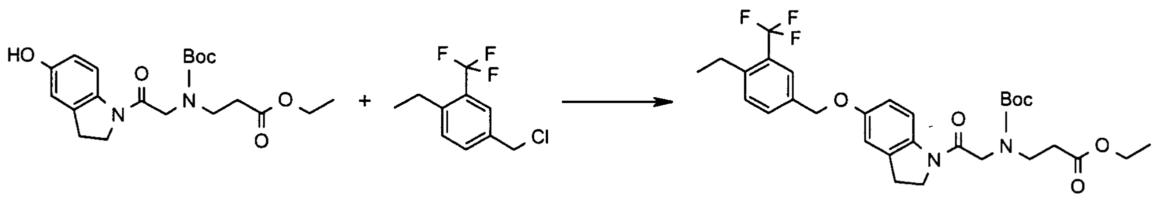

To a DMF solution (5.0 mL) of 3-[N-(tert-butoxycarbonyl)-N-[2-(5-hydroxyindolin-1-yl)-2-oxoethyl]amino]propionic acid ethyl ester (173 mg), 4-chloromethyl-l-ethyl-2-trifluoromethylbenzene (89.1 mg) and potassium carbonate (71.9 mg) were added, and the mixture was stirred overnight at 70°C. The reaction mixture was left to stand to cool to room temperature and filtered. The filtrate was concentrated under reduced pressure, and the residue was purified by flash column chromatography (Yamazen Hi-Flash column L), whereby the title compound (222 mg) was yielded.
$^1$H-NMR ($CDCl_3$) δ :
1.21-1.28(6H,m),1.40-1.49(.9H,m),2.65-2.72(2H,m),2.83(2H,q,J=7.4Hz),3.16-3.22(2H,m),3.60-3.64(2H,m),3.99-4.19 (6H,m),5.02(2H,s),6.75-6.83(2H,m),7.36(1H,d,J=8.1Hz),7.52(1H,d,J=8.1Hz),7.66(1H,s),8 .10-8.14 (1H,m) .
MS (ESI) m/z : 579 (M+H) $^+$.

(11) 3-[N-(tert-Butoxycarbonyl)-N-[2-[5-(4-ethyl-3-trifluoromethylbenzyloxy)indolin-1-yl]-2-oxoethyl]amino]propionic acid

[F447]

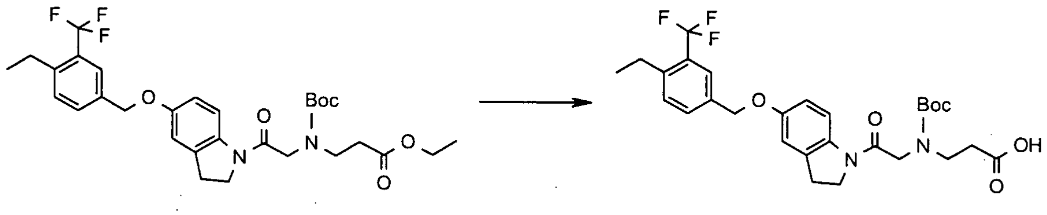

To a THF solution (5.0 mL) of 3-[N-(tert-butoxycarbonyl)-N-[2-[5-(4-ethyl-3-trifluoromethylbenzyloxy)indolin-1-yl]-2-oxoethyl]amino]propionic acid ethyl ester (210 mg), methanol (1.1 mL) and 1N aqueous sodium hydroxide (1.09 mL) were added, and the mixture was stirred at room temperature for 1 hour. 1N Hydrochloric acid was added thereto to neutralize the mixture. The reaction mixture was extracted thrice, each with chloroform. The extracts were combined and dried over sodium sulfate anhydrate. Insoluble matter was removed by filtration, and the filtrate was concentrated under the reduced pressure, whereby the title compound (195 mg) was yielded.
$^1$H-NMR ($CDCl_3$) δ:
1.26(3H,t,J=7.6Hz), 1.39-1.48(9H,m), 2.55-2.68(2H,m), 2.80-2.85(2H,m),3.20-3.24(2H,m),3.65-3.71(2H,m),4.01-4.20 (4H,m),5.03(2H,s),6.79-6.84(2H,m),7.36(1H,d,J=7.8Hz),7.52(1H,d,J=8.3Hz),7.66(1H,s),8 .13(1H,d,J=8.8Hz).
MS (ESI) m/z : 551 (M+H) $^+$.

(12) 3-[N-[2-[5-(4-Ethyl-3-trifluoromethylbenzyloxy)indolin-1-yl]-2-oxoethyl]amino]propionic acid hydrochloride

[F448]

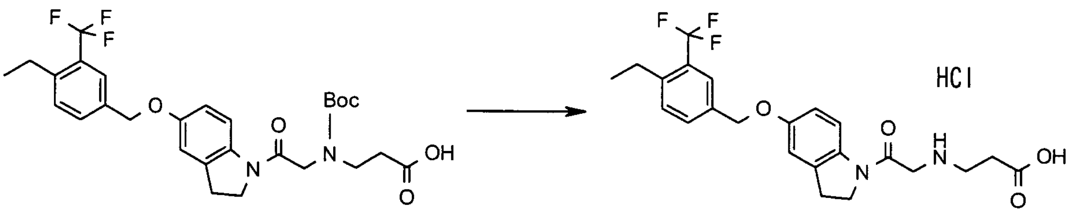

To a dichloromethane solution (1.5 mL) of 3-[N-(tert-butoxycarbonyl)-N-[2-[5-(4-ethyl-3-trifluoromethylbenzyloxy)indolin-1-yl]-2-oxoethyl]amino]propionic acid (185 mg), 4N HCl/1,4-dioxane (4.5 mL) was added, and the mixture was stirred at room temperature for 1 hour. The reaction mixture was concentrated under reduced pressure. The residue was suspended in diethyl ether for washing, collected by filtration, and dried, whereby the title compound (160 mg) was yielded.
$^1$H-NMR (DMSO-$d_6$) δ:
1.20(3H,t,J=7.5Hz),2.74-2.79(4H,m),3.16-3.21(4H,m),4.05-4.13(4H,m),5.14(2H,s),6.88(1H,dd,J=2.7,8.8Hz),7.01(1H, d,J=2.7 Hz),7.52(1H,d,J=7.8Hz),7.66-7.73(2H,m),7.96(1H,d,J=8.8Hz).
IR(ATR)cm$^{-1}$:2798,1716,1646,1492,1132,1112.
MS (ESI) m/z :451 (M+H) $^+$.
HR-MS (ESI) calcd for $C_{23}H_{26}F_3N_2O_4$ (M+H) $^+$: 451.18447; found 451.18267.
Anal. Calcd for $C_{23}H_{25}F_3N_2O_4 \cdot HCl \cdot 0.25H_2O$:C,56.21;H,5.44;Cl,7.21;F,11.60;N,5.70.Found:C,56.05;H ,5.38;Cl,7.05;F, 11.59;N,5.58.

[Example 95]

3-[N-[2-[5-(3,4-Bistrifluoromethylbenzyloxy)indolin-1-yl]-2-oxoethyl]amino]propionic acid TFA salt

(1) 4-Nitro-3-trifluoromethylbenzoic acid methyl ester

[F449]

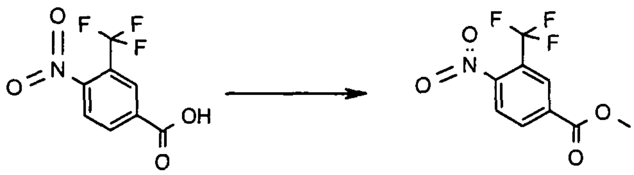

To a methanol solution (50 mL) of 4-nitro-3-trifluoromethoxybenzoic acid (1.00 g), concentrated sulfuric acid (2.0 mL) was added, and the mixture was stirred for 2.5 hours at reflux. The reaction mixture was left to stand to cool to room temperature and concentrated under reduced pressure. Saturated aqueous sodium bicarbonate solution was added to the residue. The filtrate was extracted twice, each with ethyl acetate. The extracts were combined and washed with saturated brine, followed by drying over sodium sulfate anhydrate. Insoluble matter was removed by filtration. The resultant mixture was concentrated under reduced pressure, and the residue was purified by flash column chromatography (Yamazen Hi-Flash column 2L), whereby the title compound (1.14 g) was yielded.
$^1$H-NMR ($CDCl_3$) δ:
4.02(3H,s),7.92(1H,d,J=8.3Hz),8.38(1H,dd,J=1.5,8.3Hz),8.49(1H ,d,J=1.5Hz).

(2) 4-Amino-3-trifluoromethylbenzoic acid methyl ester

[F450]

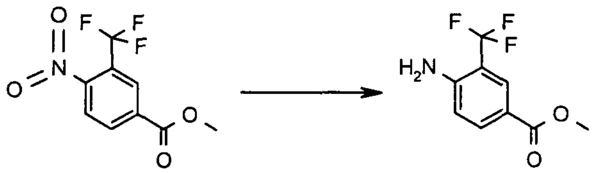

To a methanol solution (40 mL) of 4-nitro-3-trifluoromethylbenzoic acid methyl ester (1.06 g), 1N hydrochloric acid (4.25 mL) and 5% Pd/C (500 mg) were added, and the mixture was stirred for 5.5 hours at room temperature under a hydrogen atmosphere. The reaction mixture was filtered and concentrated under reduced pressure. Saturated aqueous sodium bicarbonate solution was added to the residue. The resultant mixture was extracted twice, each with ethyl acetate. The extracts were combined and washed with saturated brine, followed by drying over sodium sulfate anhydrate. Insoluble matter was removed by filtration, and the filtrate was concentrated under reduced pressure, whereby the title compound (948 mg) was yielded.
$^1$H-NMR ($CDCl_3$) δ:
3.88(3H,s),4.59(2H,s),6.73(1H,d,J=8.6Hz),7.96(1H,d,J=8.6Hz),8 .15(1H,s).
MS (ESI)m/z:220 $(M+H)^+$.

(3) 4-Bromo-3-trifluoromethylbenzoic acid methyl ester

[F451]

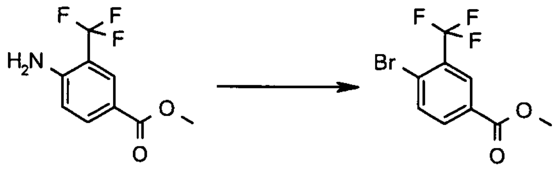

To an acetonitrile solution (40 mL) of 4-amino-3-trifluoromethylbenzoic acid methyl ester (850 mg), copper(II) bromide (1.04 g) and tert-butyl nitrite (690 μL) were added, and the mixture was stirred for 30 minutes at reflux. The reaction mixture was left to stand to cool to room temperature. Saturated aqueous sodium bicarbonate solution was added thereto, and the resultant mixture was extracted thrice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column 2L), whereby the title compound (1.09 g) was yielded.
$^1$H-NMR ($CDCl_3$) δ :
3.96(3H,s),7.81(1H,d,J=8.3Hz),8.04(1H,dd,J=2.0,8.3Hz),8.35(1H ,d,J=2.0Hz).

(4) 3,4-Bistrifluoromethylbenzoic acid methyl ester

[F452]

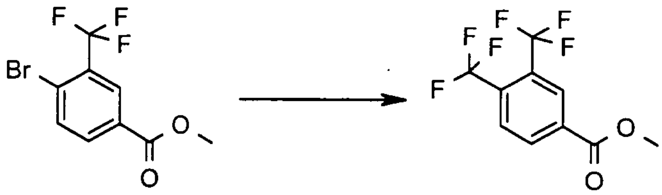

To a DMF solution (20 mL) of 4-bromo-3-trifluoromethylbenzoic acid methyl ester (1.07 g), copper(I) iodide (720 mg) and fluorosulfonyldifluoroacetic acid methyl ester (4.78 mL) were added, and the mixture was stirred overnight at 90°C. The reaction mixture was left to stand to cool to room temperature and filtered through a Celite pad. The filtrate was concentrated under reduced pressure. Saturated brine was added to the residue, followed by extraction thrice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column 2L), whereby a mixture (632 mg) of the title compound and raw materials were yielded.

(5) (3,4-Bistrifluoromethylphenyl)methanol

[F453]

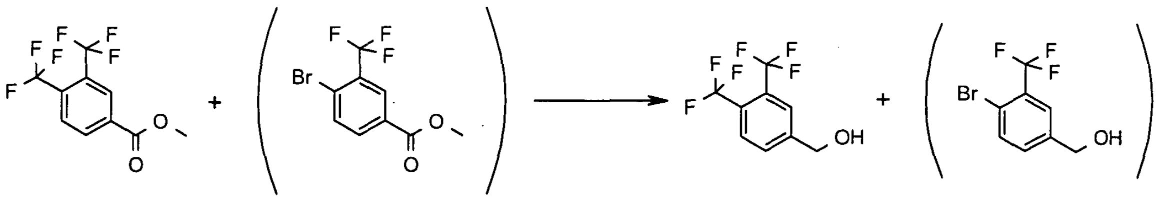

To a THF solution (20 mL) of 3,4-bistrifluoromethylbenzoic acid methyl ester (620 mg) containing impurities, lithium borohydride (149 mg) was added, and the mixture was stirred for 10 hours at reflux. The reaction mixture was left to stand to cool to room temperature. 1N Hydrochloric acid was added to the resultant mixture, followed by extraction thrice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column 2L), whereby a mixture (535 mg) of the title compound and an impurity (4-position bromo form) was yielded.
MS (ESI) m/z : 227 (M-OH) $^+$.

(6) (3,4-Bistrifluoromethylphenyl)methanol

[F454]

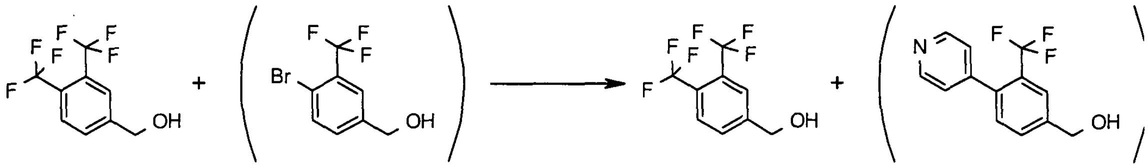

To a toluene solution (6.0 mL) of a mixture (520 mg) of (4-bromo-3-trifluoromethyl)methanol and (3,4-bistrifluoromethylphenyl)methanol, 4-pyridineboronic acid (376 mg), cesium carbonate (1.99 g), water (3.0 mL), and tetrakis (triphenylphosphine)palladium(O) (236 mg) were added, and the mixture was stirred overnight at reflux. The reaction mixture was left to stand to cool to room temperature. Saturated aqueous sodium bicarbonate solution was added thereto, followed by extraction thrice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column 2L), whereby the title compound (320 mg) was yielded.
$^1$H-NMR ($CDCl_3$) δ: 1.95(1H,t,J=5.6Hz),4.85(2H,d,J=5.6Hz),7.67-7.69(1H,m),7.84-7.86(2H,m).
MS (ESI) m/z : 227 (M-OH) $^+$.

(7) 1,2-Bistrifluoromethyl-4-chloromethylbenzene

[F455]

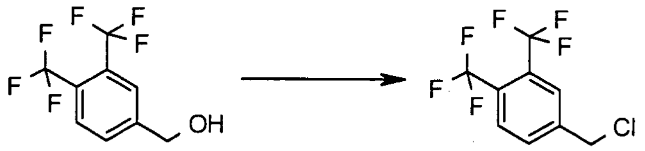

To a 1,2-dichloroethane solution (20 mL) of (3,4-bistrifluoromethylphenyl)methanol (310 mg), thionyl chloride (460 $\mu$L) and DMF (1 drop by means of a Pasteur pipette), and the mixture was stirred for 1.5 hours at 50°C. The reaction mixture was left to stand to cool to room temperature and concentrated under reduced pressure. The residue was purified by flash column chromatography (Yamazen Hi-Flash column L), whereby the title compound (206 mg) was yielded.
$^1$H-NMR ($CDCl_3$) $\delta$:
4.65(2H,s),7.71-7.75(1H,m),7.85-7.92(2H,m).

(8) 5-(3,4-Bistrifluoromethylbenzyloxy)indoline-1-carboxylic acid tert-butyl ester

[F456]

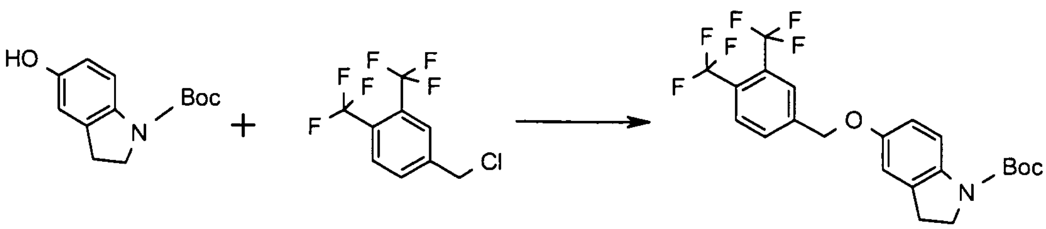

To a DMF solution (5.0 mL) of 5-hydroxyindoline-1-carboxylic acid tert-butyl ester (215 mg), 1,2-bistrifluoromethyl-4-chloromethylbenzene (200 mg) and potassium carbonate (158 mg) were added, and the mixture was stirred overnight at 70°C. The reaction mixture was left to stand to cool to room temperature and filtered. The filtrate was concentrated under reduced pressure, and the residue was purified by flash column chromatography (Yamazen Hi-Flash column 2L), whereby the title compound (299 mg) was yielded.
$^1$H-NMR ($CDCl_3$) $\delta$ : 1.55(9H,s),3.07(2H,t,J=8.7Hz),3.97(2H,s),5.12(2H,s),6.74-6.80(2H,m),7.72-7.91(4H,m).
MS (ESI)m/z:462 $(M+H)^+$.

(9) 3-[N-[2-[5-(3,4-Bistrifluoromethylbenzyloxy)indolin-1-yl]-2-oxoethyl]-N-(tert-butoxycarbonyl)amino]propionic acid ethyl ester

[F457]

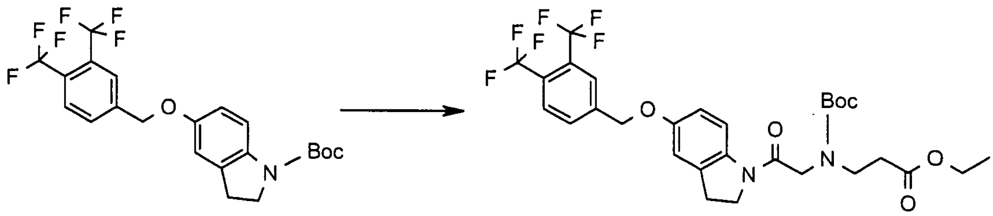

4N HCl/1,4-dioxane (5.0 mL) was added to 5-(3,4-bistrifluoromethylbenzyloxy)indoline-1-carboxylic acid tert-butyl ester (138 mg), and the mixture was stirred for 1 hour at room temperature. The reaction mixture was concentrated under reduced pressure. The residue was dissolved in DMF (10 mL), and 3-[N-(tert-butoxycarbonyl)-N-(carboxymethyl) amino]propionic acid ethyl ester (107 mg), DIEA (153 μL), HOBt (52.7 mg), and EDC-HCl (74.8 mg) were added thereto, and the mixture was stirred overnight at room temperature. Saturated aqueous sodium bicarbonate solution was added thereto, followed by extraction thrice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column L), whereby a mixture (201 mg) of the title compound and an impurity was yielded.
MS (ESI)m/z:619 $(M+H)^+$.

(10) 3-[N-[2-[5-(3,4-Bistrifluoromethylbenzyloxy)indolin-1-yl]-2-oxoethyl]-N-(tert-butoxycarbonyl)amino]propionic acid

[F458]

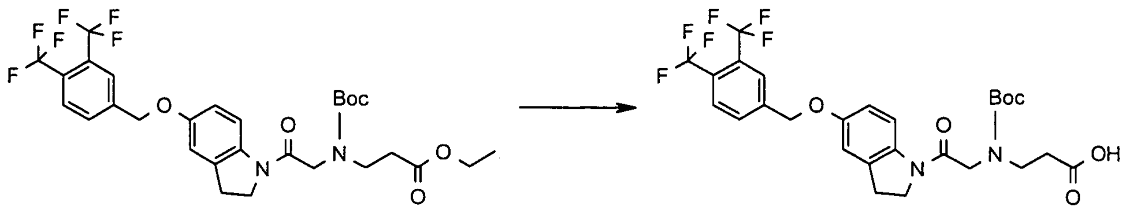

To a THF solution (5 mL) of a mixture (201 mg) of the above-mentioned impurity and 3-[N-[2-[5-(3,4-bistrifluoromethylbenzyloxy)indolin-1-yl]-2-oxoethyl]-N-tert-butoxycarbonylamino]propionic acid ethyl ester, methanol (0.90 mL), 1N aqueous sodium hydroxide solution (0.900 mL) was added, and the mixture was stirred for 1.5 hours at room temperature. 1N Hydrochloric acid was added thereto to neutralize the mixture, followed by extraction thrice, each with chloroform. The extracts were combined and washed with sodium sulfate anhydrate. Insoluble matter was removed by filtration, and the filtrate was concentrated under reduced pressure. The residue was suspended in diisopropyl ether for washing, collected by filtration, and dried, whereby the title compound (135 mg) was yielded.
$^1$H-NMR ($CDCl_3$) δ : 1.39-1.49(9H,m),2.56-2.67(2H,m),3.24(2H,t,J=8.2Hz),3.70-3.73(2H,m),4.03-4.17(4H,m),5.15(2H, s),6.79-6.85(2H,m),7.73-7.77(1H,m),7.86-7.91(2H,m),8.16(1H,d,J=8.8Hz).
MS (ESI) m/z: 591 $(M+H)^+$.

(11) 3-[2-[5-(3,4-Bistrifluoromethylbenzyloxy)indolin-1-yl]-2-oxoethylamino]propionic acid TFA salt

[F459]

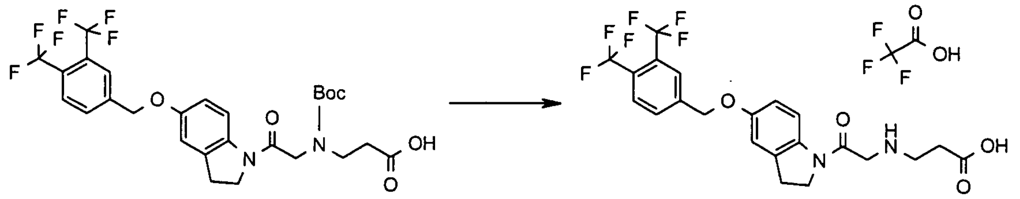

To a dichloromethane solution (4.5 mL) of 3-[N-[2-[5-(3,4-bistrifluoromethylbenzyloxy)indolin-1-yl]-2-oxoethyl]-N-(tert-butoxycarbonyl)amino]propionic acid (125 mg), TFA (0.50 mL) was added, and the mixture was stirred for 30 minutes at room temperature. TFA (0.50 mL) was further added thereto, followed by stirring for 30 minutes at room temperature. The reaction mixture was concentrated once under reduced pressure. Dichloromethane (3.0 mL) and TFA (1.5 mL) were added to the residue, followed by stirring for 1 hour at room temperature. The resultant mixture was concentrated under reduced pressure, and the residue was suspended in diethyl ether for washing, collected by filtration, and dried, whereby the title compound (119 mg) was yielded.
$^1$H-NMR (DMSO-$d_6$) δ :
2.72(2H,t,J=7.4Hz),3.17-3.22(4H,m),4.04-4.14(4H,m),5.31(2H,s),6.91(1H,dd,J=2.7,8.8Hz),7.05(1H,d,J=2.7 Hz), 7.96-7.99(2H,m),8.09-8.11(2H,m).
IR(ATR)cm$^{-1}$:1727,1660,1494,1313,1168,1126.
MS(ESI)m/z:491(M+H)$^+$.
HR-MS (ESI) calcd for $C_{22}H_{21}F_6N_2O_4$ (M+H)$^+$: 491.14055; found 491.13962.
Anal. Calcd for $C_{22}H_{20}F_6N_2O_4$.
$CF_3CO_2H$:C,47.69;H,3.50;F,28.29;N,4.63. Found:C,47.54;H,3.34;F, 28.54;N,4.57.

[Example 96]

3-[N-[2-[5-(4-Cyclohexen-1-yl-3-trifluoromethylbenzyloxy)indolin-1-yl]-2-oxoethyl]amino]propionic acid hydrochloride

(1) 4-Cyclohexen-1-yl-3-trifluoromethylbenzoic acid methyl ester

[F460]

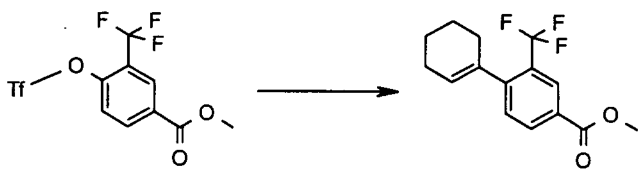

To a toluene solution (12 mL) of 4-trifluoromethanesulfonyloxy-3-trifluoromethylbenzoic acid methyl ester (1.41 g), 2-cyclohexen-1-yl-4,4,5,5-tetramethyl-[1.3.2]dioxaborolane (1.00 g), cesium carbonate (3.91 g), water (6.0 mL), and tetrakis(triphenylphosphine)palladium(0) (462 mg) were added, and the mixture was stirred for 3.5 hours at reflux. The reaction mixture was left to stand to cool to room temperature. Saturated aqueous sodium bicarbonate solution was added thereto, and the resultant mixture was extracted thrice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column 2L), and an impurity-containing fraction was purified again by flash column chromatography (Yamazen Ultrapack B). The purified products were combined, whereby the title compound (1.12 g) was yielded.
$^1$H-NMR ($CDCl_3$) δ: 1.56-2.21(8H,m),3.94(3H,s),5.60(1H,s),7.26-7.31(1H,m),8.10-8.30(2H,m).
MS (ESI) m/z : 285 (M+H) $^+$.

(2) (4-Cyclohexen-1-yl-3-trifluoromethylphenyl)methanol

[F461]

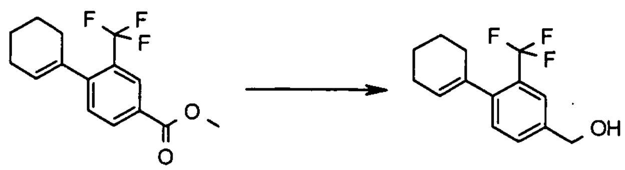

To a THF solution (10 mL) of 4-cyclohexen-1-yl-3-trifluoromethylbenzoic acid methyl ester (284 mg), lithium borohydride (65.3 mg) was added, and the mixture was stirred for 5.5 hours at reflux. The reaction mixture was left to stand to cool to room temperature, and 1N hydrochloric acid was added thereto, followed by extraction thrice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column L), whereby the title compound (235 mg) was yielded.
$^1$H-NMR ($VDCl_3$) δ :
1.64-1.78(5H,m),2.12-2.22(4H,m),4.73(2H,d,J=5.6Hz),5.57(1H,s),7.21(1H,d,J=7.8Hz),7 .46(1H,d,J=7.8Hz),7.62(1H, s).
MS (ESI)m/z:239 $(M-OH)^+$.

(3) 3-[N-(tert-Butoxycarbonyl)-N-[2-[5-(4-cyclohexen-1-yl-3-trifluoromethylbenzyloxy)indolin-1-yl]-2-oxoethyl]amino] propionic acid ethyl ester

[F462]

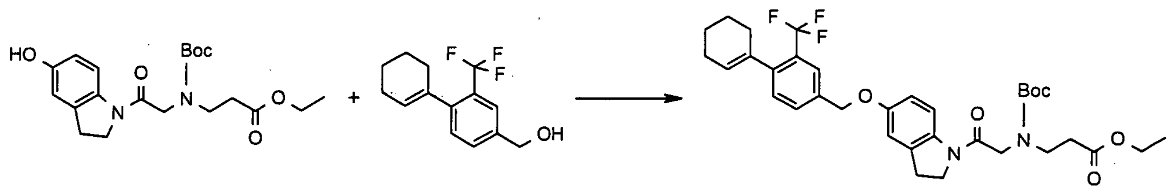

To a THF solution (10 mL) of 3-[N-(tert-butoxycarbonyl)-N-[2-(5-hydroxyindolin-1-yl)-2-oxoethyl]amino]propionic acid ethyl ester (173 mg), (4-cyclohexen-1-yl-3-trifluoromethylphenyl)methanol (225 mg), triphenylphosphine (276 mg), and DEAD (2.2 mol/L toluene solution) (479 μL) were added, and the mixture was stirred overnight at room temperature. The reaction mixture was concentrated under reduced pressure. The residue was purified by flash column chromatography (Yamazen Ultrapack B). An impurity-containing fraction was purified again by flash column chromatography (Yamazen Hi-Flash column 2L), whereby the title compound (437 mg) was yielded.
$^1$H-NMR ($CDCl_3$) δ :
1.21-1.28(3H,m),1.40-1.49(9H,m),1.64-1.78(4H,m),2.13-2.21(4H,m),2.65-2.72(2H,m),3.16-3.22(2H,m),3.60-3.64(2H, m),3.99-4.19(6H,m),5.03(2H,s),5.58(1H,s),6.76-6.84(2H,m),7.22(1H,d,J=7.6Hz),7.51(1H,d,J=7.6Hz),7.67(1H,s),8 . 10-8.14(1H,m).
MS (ESI) m/z : 631 $(M+H)^+$.

(4) 3-[N-(tert-Butoxycarbonyl)-N-[2-[5-(4-cyclohexen-1-yl-3-trifluoromethylbenzyloxy)indolin-1-yl]-2-oxoethyl] amino] propionic acid

[F463]

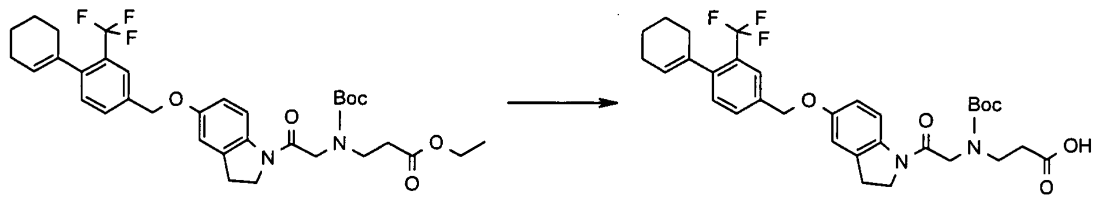

To a THF solution (10 mL) of 3-[N-(tert-butoxycarbonyl)-N-[2-[5-(4-cyclohexen-1-yl-3-trifluoromethylbenzyloxy) indolin-1-yl]-2-oxoethyl]amino]propionic acid ethyl ester (430 mg), methanol (2.05 mL) and 1N aqueous sodium hydroxide solution (2.05 mL) were added, and the mixture was stirred for 1 hour at room temperature. 1N Hydrochloric acid was added thereto to neutralize the reaction mixture, followed by extraction thrice, each with ethyl acetate. The extracts were combined and dried over sodium sulfate anhydrate. Insoluble matter was removed by filtration, and the filtrate was concentrated under reduced pressure, whereby the title compound (395 mg) was yielded.
$^1$H-NMR ($CDCl_3$) δ: 1.39-1.49(9H,m),1.64-1.78(4H,m),2.12-2.23(4H,m),2.55-2.68(2H,m),3.23(2H,t,J=8.2Hz),3.67-3.72 (3H,m),4.06-4.16(4H,m),5.04(2H,s),5.58(1H,s),6.80-6.85(2H,m),7.23(1H,d,J=7.6Hz),7.51(1H,d,J=7.6Hz),7.67(1H,s), 8 .14(1H,d,J=8.8Hz).
MS (ESI) m/z : 603 (M+H) $^+$.

(5) 3-[N-[2-[5-(4-Cyclohexen-l-yl-3-trifluoromethylbenzyloxy)indolin-1-yl]-2-oxoethyl]amino]propionic acid hydrochloride

[F464]

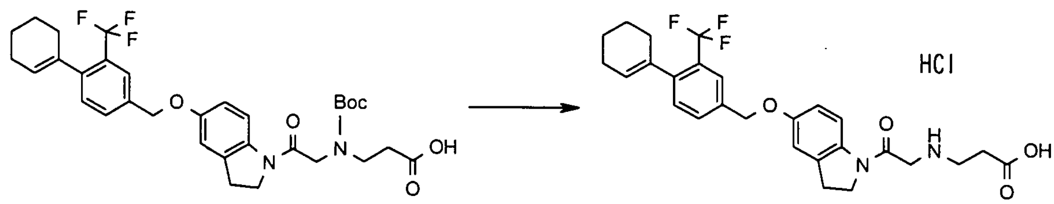

To a dichloromethane solution (2.5 mL) of 3-[N-(tert-butoxycarbonyl)-N-[2-[5-(4-cyclohexen-1-yl-3-trifluoromethylbenzyloxy)indolin-1-yl]-2-oxoethyl]amino]propionic acid (200 mg), 4N HCl/1,4-dioxane (7.5 mL) was added, and the mixture was stirred for 30 minutes at room temperature. The reaction mixture was concentrated under reduced pressure. The residue was suspended in diethyl ether for washing and collected by filtration, followed by drying, whereby the title compound (156 mg) was yielded.
$^1$H-NMR(DMSO-$d_6$)δ: 1.59-1.74(4H,m),2.11-2.18(4H,m),2.77(2H,t,J=7.5Hz),3.16-3.21(4H,m),4.05-4.13(4H,m),5.15 (2H,s),5.54(1H,s),6.89(1H,dd,J=2.5,8.8Hz),7.02 (1H,d,J=2.5Hz),7.34(1H,d,J=8.1Hz),7.66(1H,d,J=8.1Hz),7.75(1H, d, J=1.2Hz),7.97(1H,d,J=8.8Hz).
IR (ATR) $cm^{-1}$:2927, 1718, 1656, 1490, 1317, 1116.
MS (ESI) m/z : 503 (M+H) $^+$. HR-MS (ESI) calcd for $C_{27}H_{30}F_3N_2O_4$(M+H)$^+$: 503. 21577; found 503.21333.
Anal. Calcd for $C_{27}H_{29}F_3N_2O_4$·HCl· $0.5H_2O$:C,59.18;H,5.70;Cl,6.47;F,10.40;N,5.11.Found:C,59.23;H, 5.68;Cl,6.59;F, 10.32;N,5.05.

[Example 97]

3-[N-[2-[5-(4-Isopropyl-3-trifluoromethoxybenzyloxy)indolin-1-yl]-2-oxoethyl]amino]propionic acid hydrochloride

(1) 4-Isopropenyl-3-trifluoromethoxybenzoic acid methyl ester

[F465]

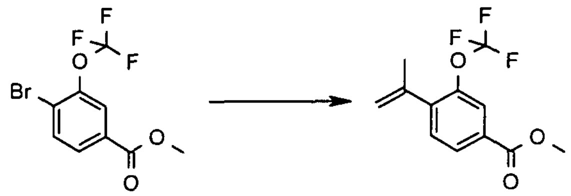

To a toluene solution (15 mL) of 4-bromo-3-trifluoromethoxybenzoic acid methyl ester (897 mg), 2-isopropenyl-4,4,5,5-tetramethyl-[1.3.2]dioxaborolane (677 μL), cesium carbonate (4.89 g), water (7.5 mL), and tetrakis(triphenylphosphine)palladium(0) (347 mg) were added, and the mixture was stirred overnight at reflux. 2-Isopropenyl-4,4,5,5-tetramethyl-[1.3.2]dioxaborolane (677 μL) and tetrakis(triphenylphosphine)palladium(0) (347 mg) were further added thereto, and the reaction mixture was stirred for 4.5 hours at reflux. The resultant mixture was left to stand to cool to room temperature and filtered. Saturated aqueous sodium bicarbonate solution was added to the filtrate, followed by extraction thrice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column 2L), whereby the title compound (765 mg) was yielded. $^1$H-NMR ($CDCl_3$) δ:
2.11(3H,t,J=0.7Hz),3.94(3H,s),5.13(1H,d,J=0.7Hz),5.30(1H,t,J= 1.2Hz),7.30-7.39(1H,m),7.90-7.93(2H,m).
MS (ESI) m/z : 261 (M+H) $^+$.

(2) 4-Isopropyl-3-trifluoromethoxybenzoic acid methyl ester

[F466]

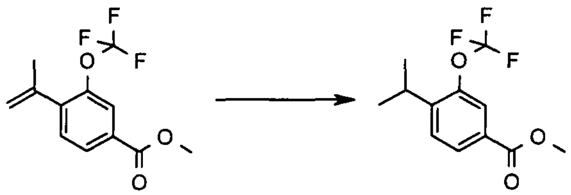

To an ethyl acetate solution (15 mL) of 4-isopropenyl-3-trifluoromethoxybenzoic acid methyl ester (755 mg), 5% Pd/C (300 mg) was added, and the mixture was stirred overnight at room temperature under a hydrogen atmosphere. The reaction mixture was filtered, and the filtrate was concentrated under reduced pressure, whereby the title compound (679 mg) was yielded.
$^1$H-NMR ($CDCl_3$) δ :
1.25(6H,d,J=6.6Hz),3.31-3.41(1H,m),3.92(3H,s),7.41(1H,d,J=8.1Hz),7.85-7.94(2H,m).
MS (ESI) m/z : 263 (M+H) $^+$.

(3) (4-Isopropyl-3-trifluoromethoxyphenyl)methanol

[F467]

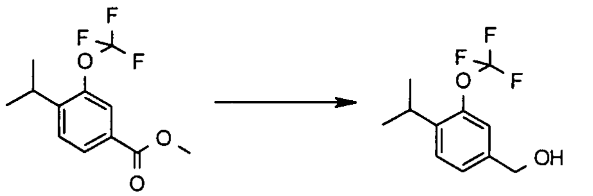

To a THF solution (30 mL) of 4-isopropyl-3-trifluoromethoxybenzoic acid methyl ester (670 mg), lithium borohydride (167 mg) was added, and the mixture was stirred for 3.5 hours at reflux. The reaction mixture was left to stand to cool to room temperature, and 1N hydrochloric acid was added thereto, followed by extraction thrice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column L), whereby the title compound (561 mg) was yielded.
$^1$H-NMR ($CDCl_3$) δ :
1.23(6H,d,J=6.9Hz),1.71(1H,t,J=5.0Hz),3.28-3.34 (1H,m) ,4.68 (2H,d,J=5.6Hz),7.22-7.34 (3H,m) .
MS(ESI)m/z:217(M-OH)$^+$.

(4) 4-Chloromethyl-1-isopropyl-2-trifluoromethoxybenzene

[F468]

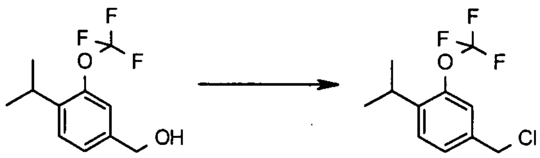

To a 1,2-dichloroethane solution (25 mL) of (4-isopropyl-3-trifluoromethoxyphenyl)methanol (550 mg), thionyl chloride (852 μL) and DMF (2 drops by means of a Pasteur pipette) were added, and the mixture was stirred for 4 hours at 50°C. The reaction mixture was left to room temperature and concentrated under reduced pressure. The residue was purified by flash column chromatography (Yamazen Hi-Flash column L), whereby the title compound (580 mg) was yielded.
$^1$H-NMR($CDCl_3$) δ:
0.93(6H,d,J=7.1Hz),2.96-3.06(1H,m),4.26(2H,s),6.93-7.04(3H,m).

(5) 3-[N-(tert-Butoxycarbonyl)-N-[2-[5-(4-isopropyl-3-trifluoromethoxybenzyloxy)indolin-1-yl]-2-oxoethyl]amino]propionic acid ethyl ester

[F469]

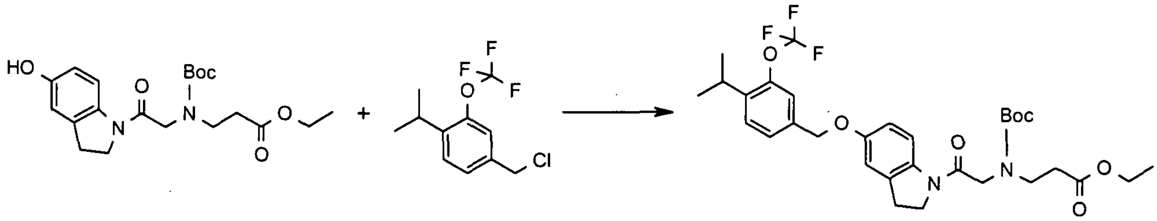

To a DMF solution (5.0 mL) of 3-[N-(tert-butoxycarbonyl)-N-[2-(5-hydroxyindolin-1-yl)-2-oxoethyl]amino]propionic acid ethyl ester (196 mg), 4-chloromethyl-1-isopropyl-2-trifluoromethoxybenzene (152 mg) and potassium carbonate (104 mg) were added, and the mixture was stirred overnight at 70°C. The reaction mixture was left to stand to cool to room temperature. Water was added thereto, and the resultant mixture was extracted thrice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column L), whereby the title compound (310 mg) was yielded.
$^1$H-NMR ($CDCl_3$) δ:
1.22-1.27(9H,m),1.40-1.49(9H,m),2.64-2.71(2H,m),3.16-3.35(3H,m),3.60-3.63(2H,m),3.98-4.18(6H,m),4.98(2H,s), 6.75-6.83(2H,m),7.26-7.35(3H,m),8.09-8.13(1H,m).

(6) 3-[N-(tert-Butoxycarbonyl)-N-[2-[5-(4-isopropyl-3-trifluoromethoxybenzyloxy)indolin-1-yl]-2-oxoethyl] amino] propionic acid

[F470]

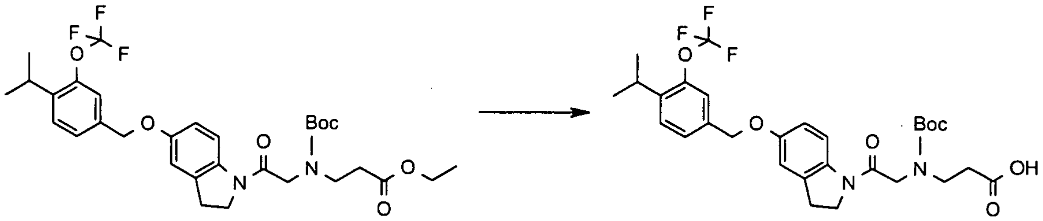

To a THF solution (10 mL) of 3-[N-(tert-butoxycarbonyl)-N-[2-[5-(4-isopropyl-3-trifluoromethoxybenzyloxy)indolin-1-yl]-2-oxoethyl]amino]propionic acid ethyl ester (300 mg), methanol (1.5 mL) and 1N aqueous sodium hydroxide solution (1.48 mL) were added, and the mixture was stirred for 3 hours at room temperature. 1N Hydrochloric acid was added thereto so as to neutralize the reaction mixture, followed by extraction thrice, each with ethyl acetate. The extracts were combined and washed with saturated brine, followed by drying over sodium sulfate anhydrate. Insoluble matter was removed by filtration, and the filtrate was concentrated under reduced pressure, whereby the title compound (286 mg) was yielded.
$^1$H-NMR ($CDCl_3$) δ: 1.23(6H,d,J=7.1Hz),1.39-1.49(9H,m),2.55-2.68(2H,m),3.20-3.37(3H,m),3.69-3.72(2H,m), 4.01-4.16(4H,m),5.00(2H,s),6.78-6.84(2H,m),7.26-7.36(3H,m),8.13(1H,d,J=8.8Hz).
MS(ESI)m/z:581(M+H)$^+$.

(7) 3-[N-[2-[5-(4-Isopropyl-3-trifluoromethoxybenzyloxy)indolin-1-yl]-2-oxoethyl]amino]propionic acid hydrochloride

[F471]

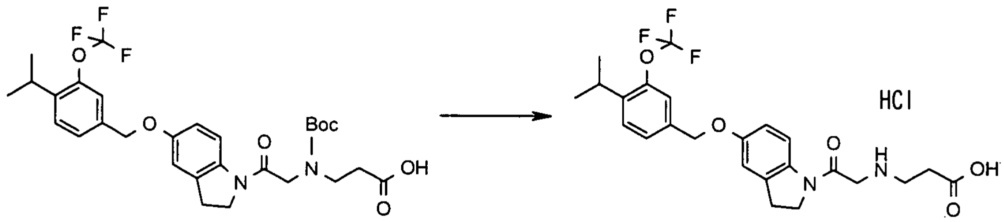

4N HCl/1,4-dioxane (10 mL) was added to 3-[N-(tert-butoxycarbonyl)-N-[2-[5-(4-isopropyl-3-trifluoromethoxybenzyloxy)indolin-1-yl]-2-oxoethyl]amino]propionic acid (280 mg), and the mixture was stirred for 1 hour at room temperature. The reaction mixture was concentrated under reduced pressure. The residue was suspended in diethyl ether for washing, collected by filtration, and dried, whereby the title compound (224 mg) was yielded.
$^1$H-NMR (DMSO-$d_6$) δ: 1.20(6H,d,J=6.9Hz),2.77(2H,t,J=7.4Hz),3.16-3.25(5H,m),4.05-4.13(4H,m),5.10(2H,s),6.88(1H, d,J=8.8Hz),7.01(1H,d,J=1.5Hz),7 .37-7.52(3H,m),7.96(1H,d,J=8.8Hz).
IR(ATR)cm$^{-1}$:2967,1720,1660,1490,1255,1211,1151.
MS (ESI) m/z :481 (M+H) $^+$.
HR-MS(ESI)calcd for $C_{24}H_{28}F_3N_2O_5$(M+H)$^+$:481.19503;found 481.19366.
Anal. Calcd for $C_{24}H_{27}F_3N_2O_5$·HCl·
0.5$H_2O$:C,54.81;H,5.56;Cl,6.74;F,10.84;N,5.33.Found:C,54.96;H, 5.47;Cl,6.74;F,10.91;N,5.19.

[Example 98]

3-[N-[2-[5-[[4-(Cyclohexyl)-2-(trifluoromethyl)phenyl]methoxy]indolin-1-yl]-2-oxoethyl]amino]propionic acid hydrochloride

(1) 4-(Trifluoromethanesulfonyloxy)-2-(trifluoromethyl)benzoic acid methyl ester

[F472]

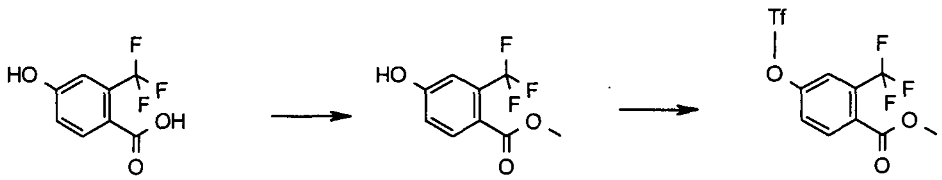

To a methanol solution (50 mL) of 4-hydroxy-2-(trifluoromethyl)benzoic acid (5.00 g), concentrated sulfuric acid (1 mL) was added at room temperature, and the mixture was stirred at 80°C. The reaction mixture was cooled to room temperature and concentrated. The residue was diluted with ethyl acetate/water and extracted with ethyl acetate. The extracts were combined and washed sequentially with saturated aqueous sodium bicarbonate solution and saturated brine, followed by drying over sodium sulfate anhydrate. Solvent was removed, and 4-hydroxy-2-(trifluoromethyl)benzoic acid methyl ester was yielded, which was subjected to a subsequent step without further purification.
To a dichloromethane solution (100 mL) of the above-mentioned 4-hydroxy-2-(trifluoromethyl)benzoic acid methyl ester, pyridine (4 mL) and trifluoromethanesulfonic acid anhydride (4.46 mL) were added at room temperature, and the mixture was stirred for 14 hours. The resultant mixture was concentrated, and the residue was diluted with ethyl acetate/water, followed by extraction with ethyl acetate. The extracts were combined and washed sequentially with 1N hydrochloric acid, saturated aqueous sodium bicarbonate solution and saturated brine, followed by drying over sodium sulfate anhydrate. Solvent was removed, and the residue was purified by silica gel flash column chromatography (Biotage 40S), whereby the title compound (5.21 g) was yielded.
$^1$H-NMR ($CDCl_3$) δ :
3.97(3H,s),7.56(1H,dd,J=8.8,2.2Hz),7.65(1H,d,J=2.5Hz),7.94(1H ,d,J=8.6Hz).

(2) 4-(Cyclohexyl)-2-(trifluoromethyl)benzoic acid

[F473]

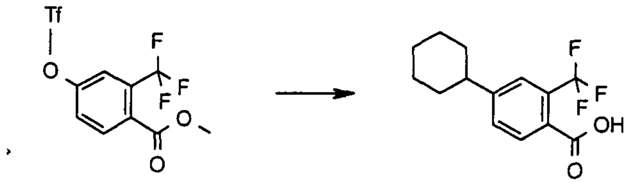

To a THF solution (80 mL) of 4-(trifluoromethanesulfonyloxy)-2-(trifluoromethyl)benzoic acid methyl ester (2.00 g) and 0.5M cyclohexylzinc bromide (45.4 mL), Pd(tert-$Bu_3P)_2$ (0.29 g) was added at room temperature under a nitrogen atmosphere, and the mixture was subjected to degasification, followed by reflux for 2 hours. The reaction mixture was cooled to room temperature, and saturated aqueous sodium bicarbonate solution was added thereto. The resultant mixture was stirred for a certain period, and filtered through a Celite pad. The filtrate was extracted with ethyl acetate. The extracts were combined and washed with saturated brine, followed by drying over sodium sulfate anhydrate. Solvent was removed under reduced pressure, and the residue was purified by silica gel flash column chromatography (Biotage 40S), whereby a mixture of 4-(cyclohexyl)-3-(trifluoromethyl)benzoic acid methyl ester and an impurity which was difficult to remove was yielded.
To a solution of the above-mentioned mixture in methanol/THF (10/20 mL), 1N aqueous sodium hydroxide solution (10 mL) was added at room temperature, and the mixture was stirred for 14 hours. The reaction mixture was concentrated, and the residue was extracted with diethyl ether. The aqueous layer was separated, and 10% hydrochloric acid was added to the aqueous layer. Precipitated solid was collected by filtration and dried under reduced pressure, whereby the title compound (1.19 g) was yielded.
$^1$H-NMR (DMSO-$d_6$) δ:
1.11-1.52(6H,m),1.68-1.90(5H,m),2.66(1H,t,J=10:9Hz),7.60(2H,t,J=7.8Hz),7.72(1H,d,J =7.8Hz).
MS(ESI)m/z:273(M+H)$^+$.

(3) 4-(Cyclohexyl)-2-(trifluoromethyl)benzyl alcohol

[F474]

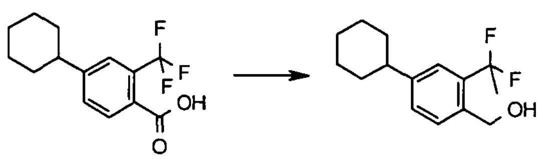

To a THF solution (50 mL) of 4-(cyclohexyl)-2-(trifluoromethyl)benzoic acid (1.39 g), 10M borane-dimethyl sulfide complex (1.76 mL) was added at room temperature, and the mixture was stirred for 18 hours. The reaction mixture was heated to 90°C, followed by stirring for 4 hours. The resultant mixture was cooled to room temperature, and 10M borane-dimethyl sulfide complex (3.62 mL) was further added thereto, followed by stirring for 15 hours at 90°C. The resultant mixture was cooled to room temperature. Saturated aqueous sodium bicarbonate solution was added thereto, followed by stirring for a certain period and extracting with ethyl acetate. The extracts were combined and washed with saturated brine, followed by drying over sodium sulfate anhydrate. Solvent was removed under reduced pressure, and the residue was purified by silica gel flash column chromatography (Biotage 40S), whereby the title compound (1.28 g) was yielded.
$^1$H-NMR ($CDCl_3$) δ:
0.96-0.10(4H,m),1.20-1.51(4H,m),1.66-1.87(2H,m),2.56(1H,s),4.84(2H,d,J=6.6Hz),7.41(1H,d,J=8.1Hz),7 .48(1H,s), 7.60(1H,d,J=7.8Hz). Assigned on the basis of a main peak.

MS (ESI)m/z:259.

(4) 4-(Cyclohexyl)-2-(trifluoromethyl)benzyl chloride

[F475]

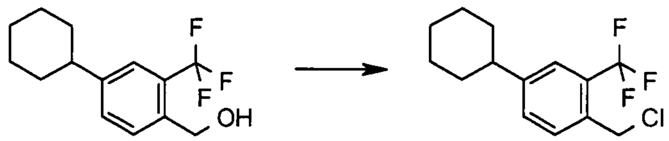

Thionyl chloride (10 mL) was added to 4-(cyclohexyl)-2-(trifluoromethyl)benzyl alcohol (1.28 g), and the mixture was stirred for 3 hours at 80°C. The reaction mixture was concentrated, and the residue was purified by silica gel flash column chromatography (Biotage 40S), whereby a mixture (1.34 g) of the title compound and an impurity which was difficult to remove was yielded.
$^1$H-NMR (CDCl) δ: 0.88-0.94(4H,m),1.16-1.48(4H,m),1.93-1.75(2H,m),2.55-2.57(1H,m),4.72(2H,s),7.40(1H,d, J=7.8Hz),7.48(1H,s),7.53(1H,d ,J=7.3Hz).

(5) 1-(tert-Butoxycarbonyl)-5-[[(4-(cyclohexyl)-2-(trifluoromethyl)phenyl)methoxy]indoline

[F476]

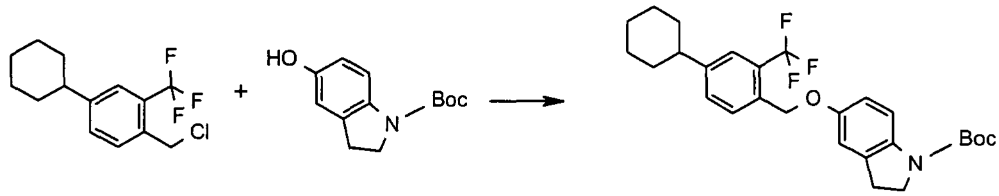

To a DMF solution (30 mL) of 4-(cyclohexyl)-2-(trifluoromethyl)benzyl chloride (1.34 g) and 1-(tert-butoxycarbonyl)indoline (1.48 g), potassium carbonate (2.68 g) was added at room temperature, and the mixture was stirred for 7 hours at 50°C. The reaction mixture was cooled to room temperature, and insoluble matter was removed by filtration. The filtrate was concentrated, and the residue was purified by silica gel flash column chromatography (Biotage 40S), whereby the title compound (1.49 g) was yielded.
$^1$H-NMR ($CDCl_3$) δ: 0.79-1.00(4H,m), 1.22-1.55(11H,m), 1.66-1.87(4H,m), 2.55-2.57(1H,m),3.05(2H,t,J=8.7Hz),3.96 (2H, s), 5.17 (2H, s), 6.76-6.78 (2H, m), 7.38 (1H, d, J=7.8Hz), 7.51-7.54 (1H, m), 7.62 (1H, d, J=8.1Hz), 7.71 (1H, dd, J=5.8,3.3Hz).
MS (ESI) m/z :476 (M+H) $^+$.

(6) 5-[[4-(Cyclohexyl)-2-(trifluoromethyl)phenyl]methoxy]indoline hydrochloride

[F477]

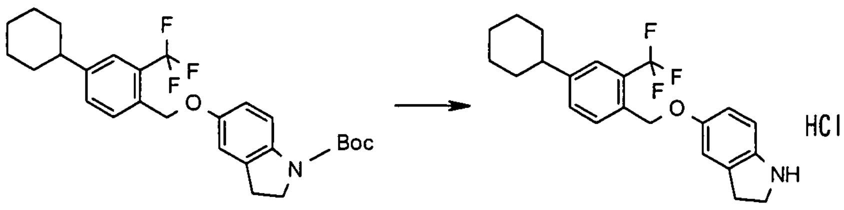

1-(tert-Butoxycarbonyl)-5-[[4-(cyclohexyl)-2-(trifluoromethyl)phenyl]methoxy]indoline was dissolved in 4N HCl/1,4-dioxane (20 mL), and the solution was stirred for 14 hours. Diethyl ether was added to the residue, and precipitated solid was collected by filtration, followed by drying under reduced pressure, whereby the title compound (1.2 g) was yielded.
$^1$H-NMR (DMSO-$d_6$) δ:
1.06-1.47 (4H, m), 1.61-1.87 (6H, m), 2.55-2.73 (1H, m), 3.16 (2H, t, J=7.8Hz), 3.57-3.83 (2H, m), 5.17 (2H, s), 6.96 (5H, dd, J=8.7,2.6Hz),7.12(1H,d,J=2.5 Hz), 7.34(1H,d,J=8.8Hz), 7.58(2H,t,J=7.0Hz), 7.65(1H,d,J=7.8Hz), 10.89(1H,s).
MS(ESI)m/z:376(M+H)$^+$.

(7) 3-[N-(tert-Butoxycarbonyl)-N-[2-[5-[[4-(cyclohexyl)-2-(trifluoromethyl)phenyl]methoxy]indolin-1-yl]-2-oxoethyl]amino]propionic acid tert-butyl ester

[F478]

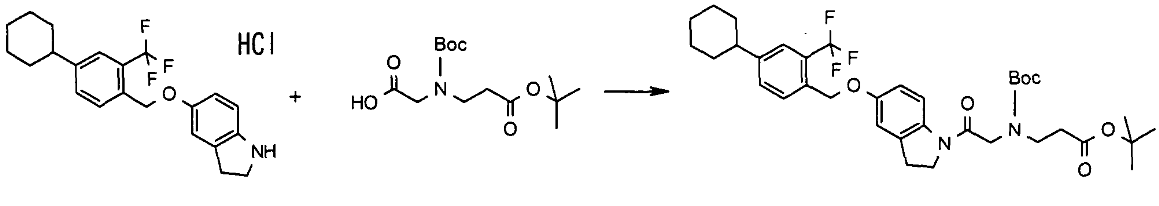

To a DMF solution (5 mL) of 5-[[4-(cyclohexyl)-2-(trifluoromethyl)phenyl]methoxy]indoline hydrochloride (0.21 g), [N-(tert-butoxycarbonyl)-N-(tert-butoxycarbonylethyl)amino]acetic acid (0.15 g), EDC·HC1 (0.14 g), and HOBt (0.10 g), TEA (0.35 mL) was added at room temperature, followed by stirring for 20 hours. The reaction mixture was concentrated, and the residue was purified by silica gel flash column chromatography (Biotage 25S), whereby the title compound (220 mg) was yielded.
$^1$H-NMR ($CDCl_3$) δ : 0.82-0.98(2H,m),1.16-1.53(20H,m),1.66-2.08(5H,m),2.45-2.65(3H,m),3.05-3.27(2H,m),3.58(2H,t, J=6.9Hz), 3.92-4.25 (4H, m), 4.82-5.02 (1H, m), 5.17 (2H, s), 6.63-6.91 (2H, m), 7.39 (1H, d, J=7.8Hz), 7.53 (1H, t, J=6.1Hz), 7.58-7.67(1H,m),8.22-8.03(1H,m).

(8) 3-[N-[2-[5-[[4-(Cyclohexyl)-2-(trifluoromethyl)phenyl]methoxy]indolin-1-yl]-2-oxoethyl]amino]propionic acid hydrochloride

[F479]

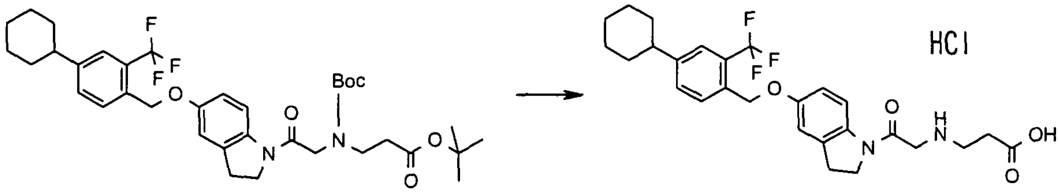


4N HCl/1,4-dioxane (10 mL) was added to 3-[N-(tert-butoxycarbonyl)-N-[2-[5-[[4-(cyclohexyl)-2-(trifluoromethyl)phenyl]methoxy]indolin-1-yl]-2-oxoethyl]amino]propionic acid tert-butyl ester (0.22 g) at room temperature, and the mixture was stirred for 23 hours, followed by concentration under reduced pressure. Diethyl ether was added to the residue. Precipitated solid was collected by filtration and dried, whereby the title compound (0.16 g) was yielded.
$^1$H-NMR (DMSO-$d_6$) δ : 1.16-1.52(3H,m) ,1.55-1.89(6H,m) ,2.42-2.79(4H,m) ,3.23-3.13(4H,m),4.05(2H,t, J=8.3Hz), 4.14(2H,s),5.13(2H,s),6.85(1H,d d,J=8.8,2.7Hz) ,6.98(1H,s) ,7.57(1H,d,J=7.8Hz) ,7.59(1H,s) ,7.64( 1H,d,J=7.8Hz), 7.96(1H,d,J=8.8Hz).
IR(ATR) cm-
1:2927,2852,2723,2598,2465,2418,2258,2166,2024,1709,1649.
MS (ESI) m/z : 505 (M+H)$^+$.
HR-MS (AqTOF) Calcd for $C_{27}H_{32}F_3N_2O_4$: 505.2314. Found: 505.2290.
Anal. Calcd for $C_{27}H_{31}F_3N_2O_4$· HCl:C,59.94;H,5.96;Cl,6.55;F,10.54;N,5.18.Found:C,59.56;H,5.9 2;Cl,6.90;F,10.39;N, 4.97.90;F,10.39;N,4.97.

[Example 99]

3-[2-Oxo-2-[5-(2-trifluoromethoxybiphenyl-4-ylmethoxy)indolin-1-yl]ethylamino]propionic acid hydrochloride

(1) 2-Trifluoromethoxybiphenyl-4-carboxylic acid methyl ester

[F480]

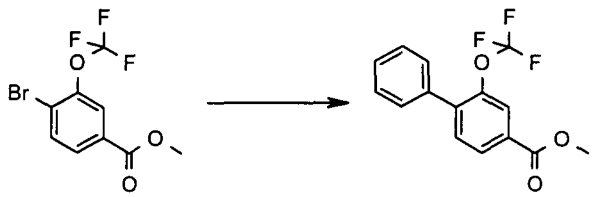

To a toluene solution (4.0 mL) of 4-bromo-3-trifluoromethoxybenzoic acid methyl ester (195 mg), phenylboronic acid (119 mg), cesium carbonate (637 mg), water (2.0 mL), and tetrakis(triphenylphosphine)palladium(0) (75 mg) were added, and the mixture was stirred for 2.5 hours at reflux. Tetrakis(triphenylphosphine)palladium(0) (75.4 mg) was further added thereto, followed by stirring overnight at reflux. The reaction mixture was cooled to room temperature and filtered through a Celite pad. Saturated aqueous sodium bicarbonate solution was added to the filtrate, followed by extraction twice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column L), whereby the title compound (177 mg) was yielded.
$^1$H-NMR($CDCl_3$)δ:
3.96(3H,s) ,7.41-7.53(6H,m) ,8.01-8.04(2H,m).
MS (ESI) m/z : 297 (M+H)$^+$.

(2) 2-Trifluoromethoxybiphenyl-4-methanol

[F481]

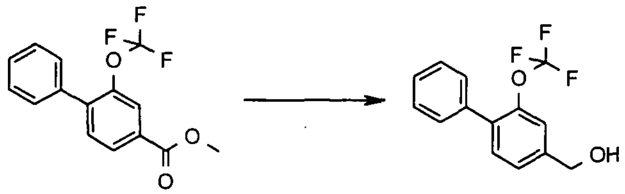

To a THF solution (20 mL) of 2-trifluoromethoxybiphenyl-4-carboxylic acid methyl ester (172 mg), lithium borohydride (37.9 mg) was added, and the mixture was stirred overnight at reflux. The reaction mixture was left to stand to cool to room temperature, and 1N hydrochloric acid was added thereto, followed by extraction twice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column L), whereby the title compound (148 mg) was yielded.
$^1$H-NMR ($CDCl_3$) $\delta$ :
1.81(1H,t,J=5.6Hz) ,4.77(2H,d,J=5.6Hz) ,7.35-7.47(8H,m).
MS (ESI) m/z : 251 $(M-OH)^+$.

(3) 4-Chloromethyl-2-trifluoromethoxybiphenyl

[F482]

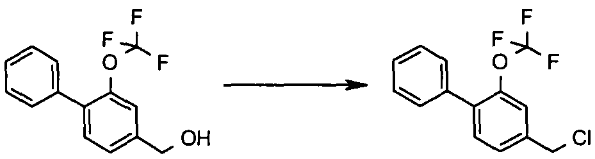

To a 1,2-dichloroethane solution (5.0 mL) of 2-trifluoromethoxybiphenyl-4-methanol, thionyl chloride (189 μL) and DMF (1 drop by means of a Pasteur pipette) were added, and the mixture was stirred for 1.5 hours at 50°C. The reaction mixture was left to stand to cool to room temperature and concentrated under reduced pressure. The residue was purified by flash column chromatography (Yamazen Hi-Flash column L), whereby the title compound (147 mg) was yielded.
$^1$H-NMR ($CDCl_3$) $\delta$ :
4.63(2H,s) ,7.38-7.46(8H,m).

(4) 1-(tert-Butoxycarbonyl)-5-(2-trifluoromethoxybiphenyl-4-ylmethoxy)indoline

[F483]

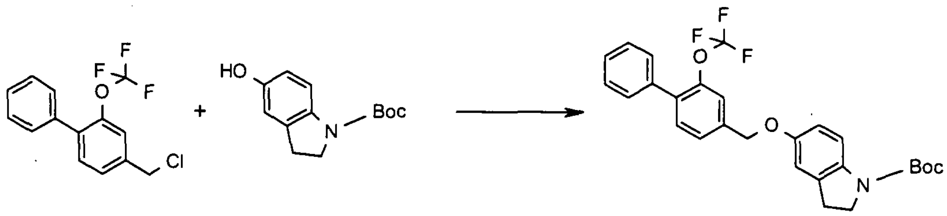

To a DMF solution (5.0 mL) of 1-(tert-butoxycarbonyl)-5-hydroxyindoline (140 mg), 4-chloromethyl-2-trifluoromethoxybiphenyl (115 mg) and potassium carbonate (87.7 mg) were added, and the mixture was stirred overnight at 70°C. The reaction mixture was left to stand to cool to room temperature, and saturated aqueous sodium bicarbonate solution was added thereto, followed by extraction twice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column L), whereby the title compound (192 mg) was yielded.
$^1$H-NMR ($CDCl_3$) δ : 1.55(9H,s) ,3.07(2H,t,J=8.7Hz) ,3.97(2H,s) ,5.07(2H,s) ,6.78-6.83(2H,m),7.35-7.47(8H,m),7.76 (1H,s).

(5) 5-(2-Trifluoromethoxybiphenyl-4-ylmethoxy)indoline hydrochloride

[F484]

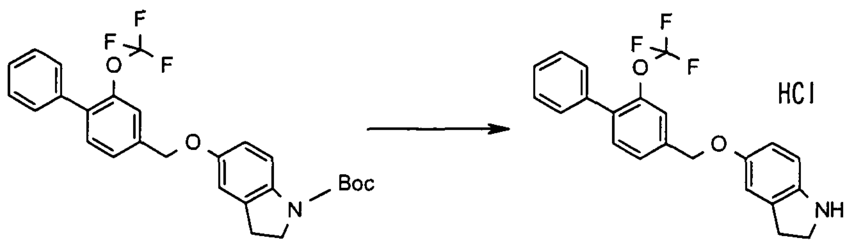

4N HCl/1,4-dioxane (10 mL) was added to 1-(tert-butoxycarbonyl)-5-(2-trifluoromethoxybiphenyl-4-ylmethoxy) indoline (185 mg), followed by stirring for 1.5 hours. The reaction mixture was concentrated under reduce pressure, whereby the title compound (173 mg) was yielded.
$^1$H-NMR (DMSO-$d_6$) δ:
3.18(2H,t,J=7.7Hz) ,3.72(3H,t,J=7.7Hz) ,5.26(2H,s) ,7.04(1H,dd,J =2.5,8.8Hz) ,7.18(1H,d,J=2.5Hz) ,7.36-7.57(9H,m).
MS (ESI) m/z : 386 $(M+H)^+$.

(6) 3-[N-[2-Oxo-2-[5-(2-trifluoromethoxybiphenyl-4-ylmethoxy)indolin-1-yl]ethyl]amino]propionic acid tert-butyl ester

[F485]

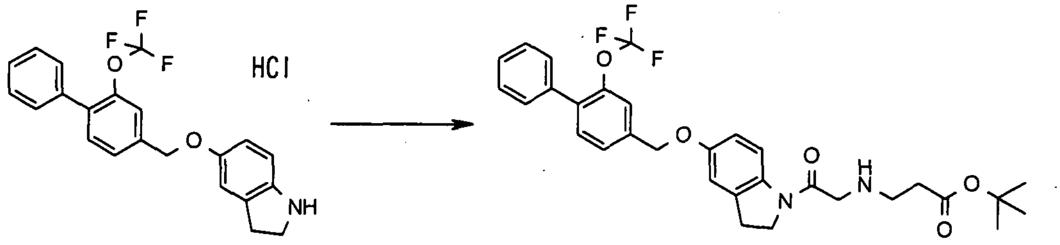

To a dichloromethane solution (10 mL) of 5-(4-phenoxymethylbenzyloxy)indoline hydrochloride (155 mg), DIEA (187 μL) and chloroacetyl chloride (44 μL) were added, and the mixture was stirred for 1 hour at room temperature. Saturated aqueous sodium bicarbonate solution was added to the reaction mixture, followed by extraction twice, each with chloroform. The extracts were combined and washed sequentially with saturated aqueous ammonium chloride solution and saturated brine, followed by drying over sodium sulfate anhydrate. Insoluble matter was removed by filtration, and the filtrate was concentrated under reduced pressure. The residue was dissolved in acetonitrile (20 mL). DIEA (624 μL) and β-alanine tert-butyl ester hydrochloride (200 mg) were added to the acetonitrile solution, followed by stirring overnight at 70°C. The mixture was left to stand to cool to room temperature and concentrated under reduced pressure. The residue was purified by flash column chromatography (Yamazen Hi-Flash column L), whereby the title compound (128 mg) was yielded.
$^1$H-NMR ($CDCl_3$) δ :
1.46(9H,s) ,2.47(2H,t,J=6.7Hz) ,2.93(2H,t,J=6.7Hz) ,3.20(2H,t,J= 8.3Hz) ,3.50(2H,s) ,4.02(2H,t,J=8.3Hz) ,5.08(2H,s), 6.81-6.85(2H,m) ,7.38-7.48(1H,m) ,8.17(1H,d,J=8.6Hz).
MS (ESI) m/z : 571 $(M+H)^+$.

(7) 3-[N-[2-Oxo-2-[5-(2-trifluoromethoxybiphenyl-4-ylmethoxy)indolin-1-yl]ethyl]amino]propionic acid hydrochloride

[F486]

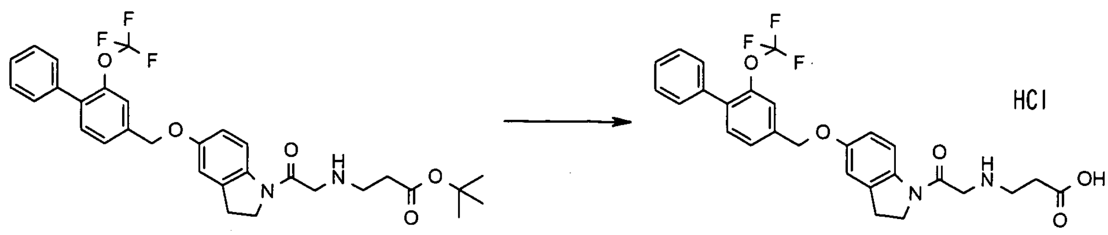

4N HCl/1,4-dioxane (10 mL) was added to 3-[N-[2-oxo-2-[5-(2-trifluoromethoxybiphenyl-4-ylmethoxy)indolin-1-yl]ethyl]amino]propionic acid tert-butyl ester (120 mg), and the mixture was stirred overnight. The reaction mixture was concentrated under reduced pressure. The residue was recrystallized from acetonitrile, whereby the title compound (69 mg) was yielded.
$^1$H-NMR (DMSO-$d_6$) δ:
2.74-2.78(2H,m),3.17-3.22(4H,m),4.05-4.14 (4H,m) ,5.20 (2H,s), 6.91 (1H, dd, J=2.6,8.7Hz), 7.04 (1H, d, J=2.6 Hz), 7.41-7.56(8H,m), 7.98(1H,d,J=8.7Hz).
IR(ATR)$cm^{-1}$:2715,1643,1490,1249,1211,1145.
MS(ESI)m/z:515$(M+H)^+$.
HR-MS (FAB) calcd for $C_{27}H_{26}F_3N_2O_5$ $(M+H)^+$: 515.1794. found 515.1832.
Anal. Calcd for $C_{27}H_{25}F_3N_2O_5 \cdot HCl \cdot$
$0.5H_2O$:C,57.91;H, 4.86;Cl,6.33;F,10.18;N,5.00.Found:C,58.16;H, 4.69;Cl,6.51;F,10.34;N,5.02.

[Example 100]

3-[N-[2-[5-(5-Cyano-1-phenyl-1H-pyrazol-3-ylmethoxy)indolin-1-yl]-2-oxoethyl]amino]propionic acid hydrochloride

(1) 5-Hydroxymethyl-2-phenyl-2H-pyrazole-3-carbonitrile

[F487]

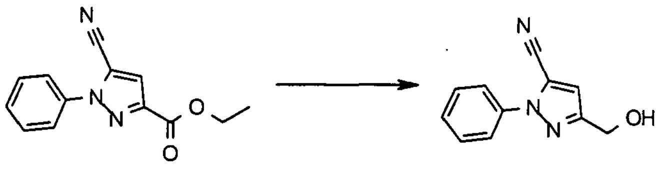

Starting materials were synthesized according to the reported procedure (Heterocycles, Vol. 27, No. 12, 2857-2862, 1988).
To a THF solution (40 mL) of 5-cyano-1-phenyl-1H-pyrazole-3-carboxylic acid ethyl ester (1.00 g), lithium borohydride (271 mg) was added, and the mixture was stirred for 40 minutes at reflux. The reaction mixture was left to stand to cool to room temperature. Saturated aqueous sodium bicarbonate solution was added thereto, followed by extraction twice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column 2L), whereby the title compound (713 mg) was yielded.
$^1$H-NMR ($CDCl_3$) $\delta$:
2.09(1H,t,J=6.0Hz), 4.80(2H,d,J=6.0Hz), 7.03(1H,s), 7.44-7.55(3H,m),7.67-7.70(2H,m).
MS(ESI)m/z:200(M+H)$^+$.

(2) 1-(tert-Butoxycarbonyl)-5-(5-cyano-1-phenyl-1H-pyrazol-3-ylmethoxy)indoline

[F488]

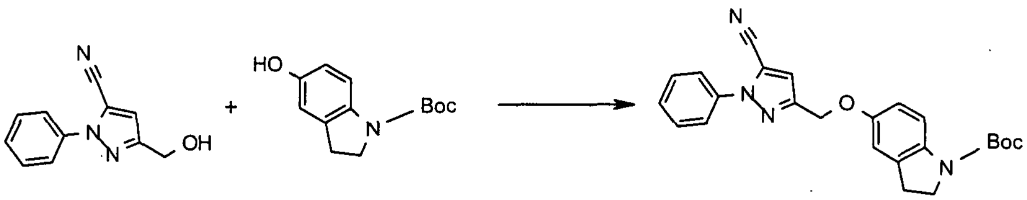

To a THF solution (5.0 mL) of 1-(tert-butoxycarbonyl)-5-hydroxyindoline (235 mg), 5-hydroxymethyl-2-phenyl-2H-pyrazole-3-carbonitrile (299 mg), triphenylphosphine (393 mg) and DEAD (2.2M toluene solution) (682 $\mu$L) were added, and the mixture was stirred overnight. The reaction mixture was concentrated under reduced pressure. The residue was purified by flash column chromatography (Yamazen Hi-Flash column L), whereby the title compound (284 mg) was yielded.
$^1$H-NMR ($CDCl_3$)$\delta$:
1.57-1.58(9H,m) ,3.06(2H,t,J=8.7Hz) ,3.97(2H,s) ,5.14(2H,s) ,6.78-6.82 (2H,m) ,7.10 (1H,s) ,7.45-7.56 (3H,m) , 7.69-7.76 (3H,m).
MS (ESI) m/z : 417 (M+H)$^+$.

(3) 5-(5-Cyano-1-phenyl-1H-pyrazol-3-ylmethoxy)indoline hydrochloride

[F489]

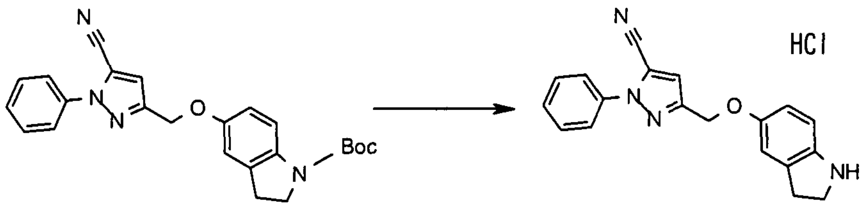

4N HCl/1,4-dioxane (5.0 mL) was added to 1-(tert-butoxycarbonyl)-5-(5-cyano-1-phenyl-1H-pyrazol-3-ylmethoxy)indoline (275 mg), and the mixture was stirred for 1 hour. The reaction mixture was concentrated under reduced presssure, whereby the title compound (239 mg) was yielded.
$^1$H-NMR (DMSO-$d_6$) δ:
3.18(2H,t,J=7.7Hz) ,3.71(2H,t,J=7.7Hz) ,5.23(2H,s) ,7.04(1H,dd,J =2.5,8.7Hz) ,7.18(1H,d,J=2.5Hz) ,7.36(1H,d, J=8.7Hz) ,7.55-7.66(4H,m),7.73-7.76(2H,m).
MS(ESI)m/z:317(M+H)$^+$.

(4) 3-[N-(tert-Butoxycarbonyl)-N-[2-[5-(5-cyano-1-phenyl-1H-pyrazol-3-ylmethoxy)indolin-1-yl]-2-oxoethyl]amino]propionic acid tert-butyl ester

[F490]

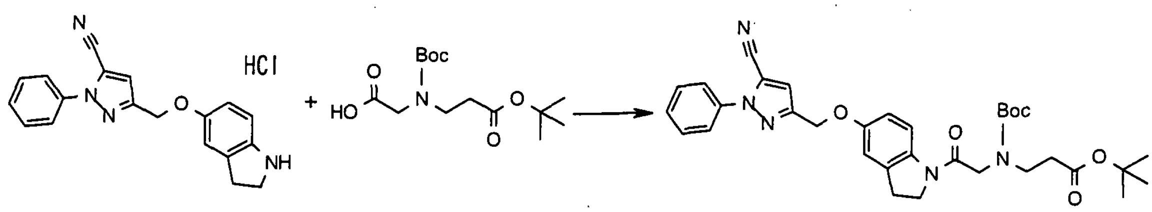

To a DMF solution (10 mL) of 5-(5-cyano-1-phenyl-1H-pyrazol-3-ylmethoxy)indoline (225 mg), 3-[N-(tert-butoxycarbonyl)-N-(carboxymethyl)amino]propionic acid tert-butyl ester (203 mg), DIEA (542 μL), HOBt (103 mg), and EDC·HCl (148 mg) were added, and the mixture was stirred overnight at room temperature. Saturated aqueous sodium bicarbonate solution was added to the reaction mixture, followed by extraction twice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column L), whereby the title compound (344 mg) was yielded.
$^1$H-NMR ($CDCl_3$) δ :
1.40-1.49(18H,m) ,2.55-2.62(2H,m) ,3.16-3.22(2H,m) ,3.56-3.61(2H,m) ,3.99-4.17(4H,m) ,5.14(2H,s),6.79-6.84 (2H, m) ,7.09(1H,s) ,7.45-7.55 (3H,m), 7.70 (2H,d,J=7.6Hz) ,8.11-8.15(1H,m).
MS (ESI) m/z : 602 (M+H)$^+$.

(5) 3-[N-[2-[5-(5-Cyano-1-phenyl-1H-pyrazol-3-ylmethoxy)indolin-1-yl]-2-oxoethyl]amino]propionic acid hydrochloride

[F491]

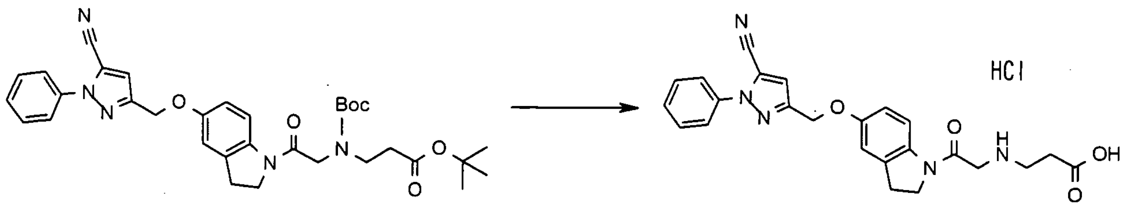


4N HCl/1,4-dioxa (10 mL) was added to 3-[N-(tert-butoxycarbonyl)-N-[2-[5-(5-cyano-1-phenyl-1H-pyrazol-3-ylmethoxy)indolin-1-yl]-2-oxoethyl]amino]propionic acid tert-butyl ester (330 mg), and the mixture was stirred overnight. The reaction mixture was concentrated under reduced pressure. The residue was suspended in acetonitrile for washing, collected by filtration, and dried, whereby the title compound (203 mg) was yielded.
$^1$H-NMR (DMSO-$d_6$) δ:
2.76-2.80(2H,m),3.17-3.21(4H,m),4.06-4.14(4H,m) ,5.17(2H,s) ,6.92(1H,dd,J=2.6,8.9Hz) ,7.05(1H,s) ,7.55 -7.75(6H, m) ,7.98(1H,d,J=8.6Hz).
IR(ATR)cm$^{-1}$:2566,2235,1725,1664,1488,1373.
MS(ESI)m/z:446(M+H)$^+$.
HR-MS(FAB)calcd for $C_{24}H_{24}N_5O_4$ (M+H)$^+$:446. 1828. found 446.1830.
Anal. Calcd for $C_{24}H_{23}N_5O_4$· 1.0HCl:C,59.81;H,5.02;Cl, 7.36;N,14.53.Found:C,59.63 ;H,4.94; Cl ,7.26;N,14.54.

[Example 101]

3-[N-[2-Oxo-2-[5-(3-trifluoromethylbiphenyl-4-ylmethoxy)indolin-1-yl]ethyl]amino]propionic acid hydrochloride

(1) 4-Nitro-2-trifluoromethylbenzoic acid methyl ester

[F492]

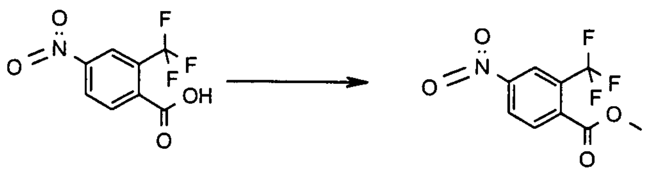

To a methanol solution (40 mL) of 4-nitro-2-trifluoromethylbenzoic acid (1.00 g), thionyl chloride (599 μL) was added. The mixture was stirred overnight at reflux, and thionyl chloride (898 μL) was added thereto, followed by stirring for 5 hours at reflux. The mixture was left to stand to cool to room temperature, the reaction mixture was concentrated under reduced pressure. Saturated sodium bicarbonate solution was added to the residue, followed by extraction twice, each with ethyl acetate. The extracts were combined and washed with saturated brine, followed by drying over sodium sulfate anhydrate. Insoluble matter was filtered off, and the filtrate was concentrated under reduced pressure, whereby the title compound (824 mg) was yielded.
$^1$H-NMR($CDCl_3$) δ:
4.00(3H,s) ,7.98(1H,d,J=8.3Hz), 8.47(1H,dd,J=2.2,8.3Hz) ,8.61(1H ,d,J=2.2Hz).
MS(ESI)m/z: No molecular ion peak was observed.

(2) 4-Amino-2-trifluoromethylbenzoic acid methyl ester (WO2006/076706)

[F493]

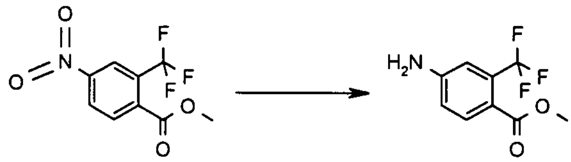

To a methanol solution (30 mL) of 4-nitro-2-trifluoromethylbenzoic acid methyl ester (820 mg), 5%Pd/C (hydrated) (400 mg) was added, and the mixture was stirred for 6 hours at room temperature under a hydrogen atmosphere. The reaction mixture was filtered, and the filtrate was concentrated under reduced pressure. The resultant residue was purified by flash column chromatography (Yamazen Hi-Flash column L), whereby the title compound (730 mg) was yielded.
$^1$H-NMR ( $CDCl_3$) δ:
3.87(3H,s) ,4.18(2H,s) ,6.76(1H,dd,J=2.3,8.5Hz) ,6.98(1H,d,J=2.3 Hz) ,7.76 (1H, d, J=8.5Hz).
MS (ESI) m/z :220 $(M+H)^+$.

(3) 4-Bromo-2-trifluoromethylbenzoic acid methyl ester

[F494]

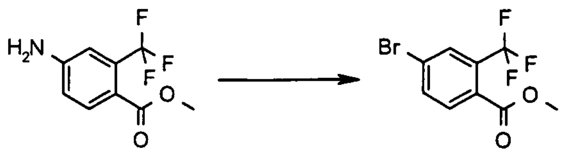

To an acetonitrile solution (15 mL) of 4-amino-2-trifluoromethylbenzoic acid methyl ester (328 mg), copper(II) bromide (402 mg), and tert-butyl nitrite (267 μL) were added, and the mixture was stirred for 1.5 hours at reflux. The mixture was left to stand to cool to room temperature, and saturated aqueous sodium bicarbonate solution was added to the reaction mixture. The resultant solution was extracted thrice, each with ethyl acetate. The extracts were combined and washed with saturated brine, followed by drying over sodium sulfate anhydrate. Insoluble matter was filtered off, and the filtrate was concentrated under reduced pressure. The residue was purified by flash column chromatography (Yamazen Hi-Flash column L), whereby the title compound (359 mg) was yielded.
$^1$H-NMR ($CDCl_3$) δ :
3.94(3H,m) ,7.68-7.77(2H,m) ,7.90(1H,d,J=1.5Hz).
MS (ESI) m/z : 283 $(M+H)^+$.

(4) 3-Trifluoromethylbiphenyl-4-carboxylic acid methyl ester

[F495]

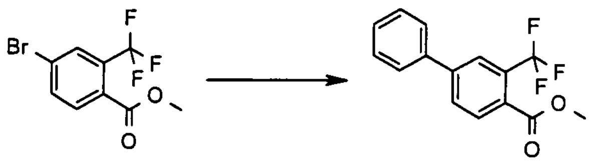

Phenylboronic acid (302 mg), cesium carbonate (2.01 g), water (4.0 mL), and tetrakis(triphenylphosphine) palladium(0) (143 mg) were added to a toluene solution (8.0 mL) of 4-bromo-2-trifluoromethylbenzoic acid methyl ester (350 mg). The mixture was refluxed overnight with stirring. The mixture was left to stand to cool to room temperature, and water was added to the mixture, followed by extraction twice, each with ethyl acetate. The organic layer was washed with saturated brine, and then dried over sodium sulfate anhydrate. After filtration and concentration under reduced pressure, the residue was purified by flash column chromatography (Yamazen Hi-Flash column L), whereby the title compound (331 mg) was yielded.
$^1$H-NMR ($CDCl_3$) δ:
3.96(3H,s) ,7.41-7.63(5H,m) ,7.80-7.96(3H,m).
MS(ESI)m/z: No molecular ion peak was observed.

(5) 3-Trifluoromethylbiphenyl-4-methanol

[F496]

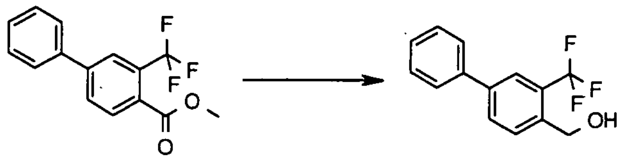

To a THF solution (20 mL) of 2-trifluoromethoxybiphenyl-4-carboxylic acid methyl ester (325 mg), lithium borohydride (75.8 mg) was added, and the mixture was stirred overnight at reflux. The mixture was left to stand to cool to room temperature, and 1N hydrochloric acid was added to the reaction mixture, followed by extraction twice, each with ethyl acetate. The extracts were combined and washed with saturated brine, and then dried over sodium sulfate anhydrate, and insoluble matter was filtered off. The filtrate was concentrated under reduced pressure. The residue was purified by flash column chromatography (Yamazen Hi-Flash column L), whereby the title compound (282 mg) was yielded.
$^1$H-NMR ($CDCl_3$) δ:
1.90(1H,s) ,4.93(2H,s) ,7.37-7.49(3H,m) ,7.58-7.61(2H,m) ,7.79(2H,m) ,7.86(1H,s).
MS (ESI) m/z : 235 (M-OH)$^+$.

(6) 5-(3-Trifluoromethylbiphenyl-4-ylmethoxy)indoline-1-carboxylic acid tert-butyl ester

[F497]

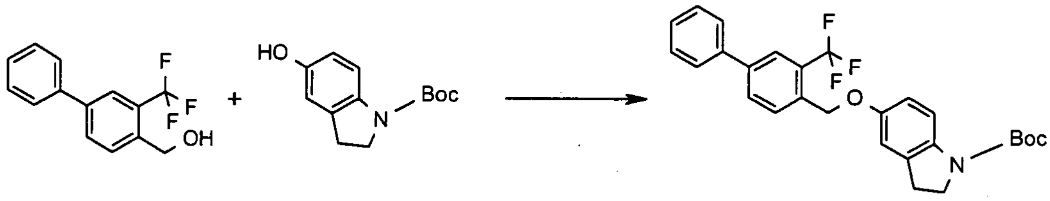

3-Trifluoromethylbiphenyl-4-methanol (275 mg), triphenylphosphine (429 mg), and DEAD (2.2M toluene solution (743 μL) were added to a THF solution (10 mL) of 5-hydroxyindoline-1-carboxylic acid tert-butyl ester (385 mg), and the mixture was stirred overnight at room temperature. The reaction mixture was concentrated under reduced pressure. The residue was purified by flash column chromatography (Yamazen Hi-Flash column L), whereby the title compound (413 mg) was yielded.
$^1$H-NMR ($CDCl_3$)δ:
1.55 (9H,s) ,3.07(2H,t,J=8.7Hz) ,3.97(2H,s) ,5.07(2H,s) ,6.78-6.83(2H,m) ,7.35-7.47(8H,m) ,7.76(1H,s).
MS(ESI)m/z:469 $M^+$.

(7) 5-(3-Trifluoromethylbiphenyl-4-ylmethoxy)indoline hydrochloride

[F498]

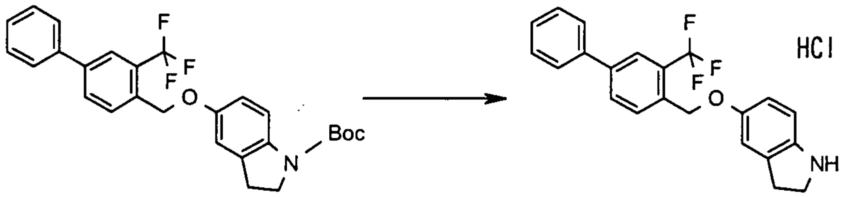

To 5-(3-trifluoromethylbiphenyl-4-ylmethoxy)indoline-1-carboxylic acid tert-butyl ester (400 mg), 4N HCl/1,4-dioxane (10 mL) was added, and the mixture was stirred for 2 hours. The reaction mixture was concentrated under reduced pressure. The residue was suspended in diethyl ether for washing, collected by filtration, and dried, whereby the title compound (376 mg) was yielded.
$^1$H-NMR (DMSO-$d_6$) δ:
3.19(2H,t,J=7.8Hz) ,3.72(2H,t,J=7.8Hz) ,5.30(2H,s) ,7.02(1H,dd,J =2.5,8.6Hz) ,7.17(1H,d,J=2.5Hz) ,7.36-7.54(4H,m) , 7.75-7.86(3H,m),8.01-8.04(2H,m).
MS (ESI) m/z : 370 $(M+H)^+$.

(8) 3-[N-(tert-Butoxycarbonyl)-N-[2-oxo-2-[5-(3-trifluoromethylbiphenyl-4-ylmethoxy)indolin-1-yl]ethyl]amino] propionic acid tert-butyl ester

[F499]

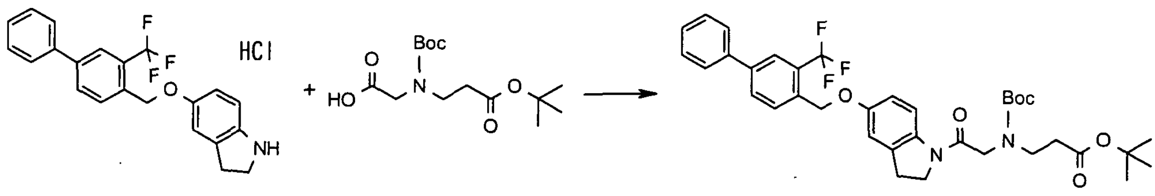

To a DMF solution (10 mL) of 5-(3-trifluoromethylbiphenyl-4-ylmethoxy)indoline hydrochloride (353 mg), 3-[(N-tert-butoxycarbonyl)-N-(carboxymethyl)amino]propionic acid tert-butyl ester (317 mg), DIEA (740 μl), HOBt (141 mg), and EDC·HCl (200 mg) were added, and the mixture was stirred overnight at room temperature. Saturated sodium bicarbonate solution was added to the reaction mixture, and the solution was extracted twice, each with ethyl acetate. The extracts were combined and washed with saturated brine, and the mixture was dried over sodium sulfate anhydrate. Insoluble matter was filtered off, and the filtrate was concentrated under reduced pressure. The residue was purified by flash column chromatography (Yamazen Hi-Flash column L), and purified again by flash column chromatography (Yamazen Hi-Flash column 2L), whereby the title compound (420 mg) was yielded.
$^1$H-NMR ($CDCl_3$)δ:
1.40-1.50(18H,m),2.55-2.63(2H,m),3.16-3.23(2H,m),3.56-3.61(2H,m),3.99-4.18(4H,m),5.27(2H,s),6.78-6.84(2H,m),7.38-7.52(3H,m),7.59-7.61(2H,m),7.75-7.81(2H,m),7.90(1H,s),8.11-8.16(1H,m).
MS(ESI)m/z:655(M+H)$^+$.

(9) 3-[N-[2-Oxo-2-[5-(3-trifluoromethylbiphenyl-4-ylmethoxy) indolin-1-yl] ethyl] amino] propionic acid hydrochloride

[F500]

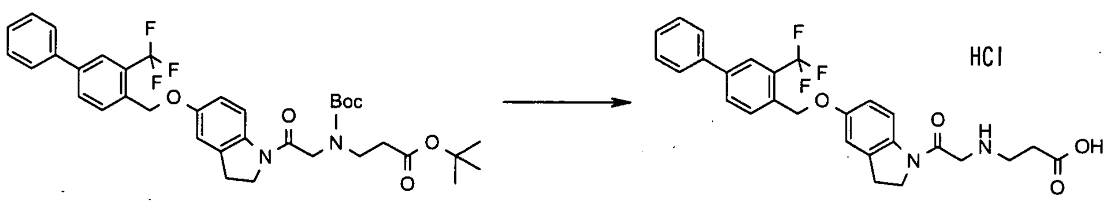

To 3-[N-(tert-butoxycarbonyl)-N-[2-oxo-2-[5-(3-trifluoromethylbiphenyl-4-ylmethoxy)indolin-1-yl]ethyl]amino] propionic acid tert-butyl ester (410 mg), 4N HCl/1,4-dioxane (10 mL) was added, and the mixture was stirred overnight at room temperature. Diethyl ether was added to the reaction mixture, and the precipitated solid matter was collected by filtration, followed by drying, whereby the title compound (314 mg) was yielded.
$^1$H-NMR (DMSO-$d_6$) δ:
2.78(2H,t,J=7.5Hz),3.20(4H,t,J=7.6Hz),4.06-4.15(4H,m),5.25(2H,s),6.90(1H,dd,J=2.7,8.8Hz),7.03(1H,d,J=2.7 Hz), 7.43-7.54(3H,m),7.75-7.86(3H,m),7.99-8.02(3H,m).
IR(ATR)cm$^{-1}$:2632,1702,1654,1494,1164,1112,1043.
MS (ESI) m/z : 499 (M+H)$^+$.
HR-MS (ESI) calcd for $C_{27}H_{26}F_3N_2O_4$ (M+H)$^+$:499.18447. found 499.18050.
Anal. Calcd for $C_{27}H_{25}F_3N_2O_4$·
1.0HCl:C,60.02;H,4.90;Cl,6.63;F,10.65;N,5.24.Found:C,60.39;H, 4.92;Cl,6.70;F,10.66;N,4.94.

[Example 102]

3-[N-[2-Oxo-2-[5-(4-isobutyl-2-trifluoromethylbenzyloxy) indolin-1-yl]ethyl]amino]propionic acid hydrochloride

(1) 4-Isobutyl-2-trifluoromethylbenzoic acid methyl ester

[F501]

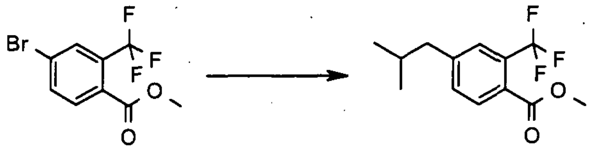

To a toluene solution (8.0 mL) of 4-bromo-2-trifluoromethylbenzoic acid methyl ester (377 mg), isobutylboronic acid (407 mg), cesium carbonate (2.17g), water (4.0 mL), and tetrakis(triphenylphosphine)palladium(0) (154 mg) were added, and the mixture was stirred overnight at reflux. The mixture was left to stand to cool to room temperature. Water was added to the reaction mixture, and the mixture was twice extracted with ethyl acetate. The extracts were combined and washed with saturated brine, and then dried over sodium sulfate anhydrate. Insoluble matter was filtered off, and the filtrate was concentrated under reduced pressure. The residue was purified by flash column chromatography (Yamazen Hi-Flash column L), whereby the title compound (325 mg) was yielded.
$^1$H-NMR ($CDCl_3$) δ:
0.91 (6H, d, J=6.6Hz), 1.85-1.96 (1H, m), 2.57 (2H, d, J=7.1Hz), 3.93 (3H, s), 7.37 (1H, d, J=7.8Hz), 7 .51 (1H, s), 7.72 (1H, d, J=7.8Hz).

(2) (4-Isobutyl-2-trifluoromethylphenyl)methanol

[F502]

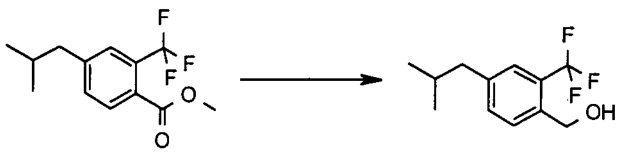

To a THF solution (20 mL) of 4-isobutyl-2-trifluoromethylbenzoic acid methyl ester (320 mg), lithium borohydride (80.3 mg) was added, and the mixture was stirred overnight at reflux. The reaction mixture was left to stand to cool to room temperature, and to the reaction mixture, 1N hydrochloric acid was added. The resultant mixture was extracted twice, each with ethyl acetate. The extracts were combined and washed with saturated brine, followed by drying over sodium sulfate anhydrate. Insoluble matter was filtered off, and the filtrate was concentrated under reduced pressure. The resultant residue was purified by flash column chromatography (Yamazen Hi-Flash column L), whereby the title compound (254 mg) was yielded.
$^1$H-NMR($CDCl_3$) δ :
0.91(6H,d,J=6.6Hz),1.80-1.93(2H,m),2.52(2H,d,J=7.1Hz),4.84(2H,d,J=6.1Hz),7.33-7.36(2H,m),7.59(1H,d,J=7.8Hz).
MS(ESI)m/z:215(M-OH)$^+$.

(3) 5-(4-Isobutyl-2-trifluoromethylbenzyloxy)indoline-1-carboxylic acid tert-butyl ester

[F503]

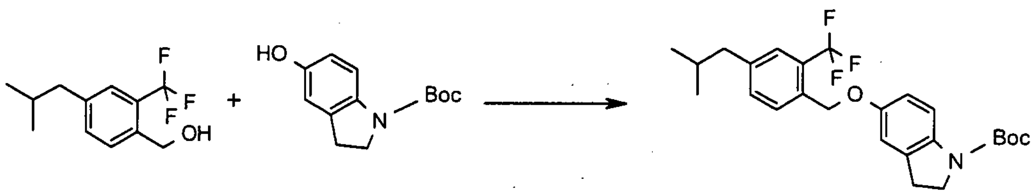

To a THF solution (10 mL) of 5-hydroxyindoline-1-carboxylic acid tert-butyl ester (372 mg), (4-isobutyl-2-trifluoromethylbiphenyl)methanol (245 mg), triphenylphosphine (415 mg) and DEAD (2.2 M toluene solution) (719 μL) were added, and the mixture was stirred overnight at room temperature. The reaction mixture was concentrated under reduced pressure. The obtained residue was purified by flash column chromatography (Yamazen Hi-Flash column L), whereby the title compound (298 mg) was yielded.
$^1$H-NMR ($CDCl_3$) δ:
0.91 (6H, d, J=6.6Hz), 1.55 (9H, s), 1.83-1.93 (1H, m), 2.52 (2H, d, J=7.4Hz), 3.06 (2H, t, J=8.6Hz), 3.96 (2H, s), 5 . 18 (2H, s), 6.75-6.80(2H,m),7.32(1H,d,J=7.8Hz),7.45(1H,s),7.61-7.75(2H,m).
MS(ESI)m/z:449 $M^+$.

(4) 5-(4-Isobutyl-2-trifluromethylbenzyloxy)indoline hydrochloride

[F504]

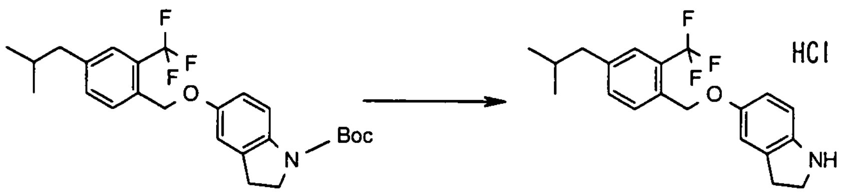

To 5-(4-isobutyl-2-trifluoromethylbenzyloxy)indoline-1-carboxylic acid tert-butyl ester (290 mg), 4N HCl/1,4-dioxane (10 ml) was added, and the reaction mixture was stirred for 2 hours. The reaction mixture was concentrated under reduced pressure. The residue was suspended in diethyl ether for washing, whereby the title compound (246 mg) was yielded.
$^1$H-NMR (DMSO-$d_6$) δ:
0.87(6H,d,J=6.6Hz) ,1.82-1.92(1H,m) ,2.56(2H,d,J=7.1Hz) ,3.18(2H,t,J=7.7Hz) ,3.71(2H,t,J= 7.7Hz) ,5.20(2H,s) ,6.99 (1H,dd,J=2.6,8.7Hz) ,7.13 (1H, d,J=2.6Hz), 7.36(1H,d,J=8.7Hz) ,7.52(1H,d,J=7.8Hz) ,7.58(1H,s) ,7.66(1H,d,J= 8.1Hz).
MS (ESI) m/z : 350 $(M+H)^+$.

(5) 3-[N-(tert-Butoxycarbonyl)-N-[2-oxo-2-[5-(4-isobutyl-2-trifluoromethylbenzyloxy)indolin-1-yl]ethyl]amino]propionic acid tert-butyl ester

[F505]

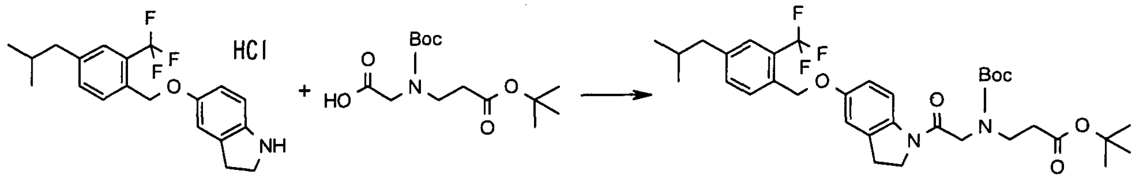

To a DMF solution (10 mL) of 5-(4-isobutyl-2-trifluoromethylbenzyloxy)indoline hydrochloride (240 mg), 3-[N-(tert-butoxycarbonyl)-N-(carboxymethyl)amino]propionic acid tert-butyl ester (226 mg), DIEA (529 μL), HOBt (101 mg), and EDC·HCl (143 mg) were added, and the mixture was stirred overnight at room temperature. Saturated aqueous sodium bicarbonate solution was added to the reaction mixture, and the resultant mixture was extracted thrice, each with ethyl acetate. The extracts were combined and washed with saturated brine, followed by drying over sodium sulfate anhydrate. Insoluble matter was filtered off, and the filtrate was concentrated under reduced pressure. The obtained residue was purified by flash column chromatography (Yamazen Hi-Flash column chromatography L) and was purified again by flash column chromatography (Yamazen Hi-Flash column chromatography 2L), whereby the title compound (375 mg) was yielded.
$^1$H-NMR ($CDCl_3$) δ:
0.91(6H,d,J=6.6Hz),1.40-1.49(18H,m),1.83-1.93(1H,m),2.52-2.63(4H,m),3.16-3.22(2H,m),3.56-3.61(2H,m),3.99-4.17 (4H,m),5.19(2H,s),6.76-6.82(2H,m),7.32-7.62(3H,m),8.09-8.14(1H,m).
MS(ESI)m/z:635 $(M+H)^+$.

(6) 3-[N-[2-oxo-2-[5-(4-isobutyl-2-trifluoromethylbenzyloxy)indolin-1-yl]ethyl]amino]propionic acid hydrochloride

[F506]

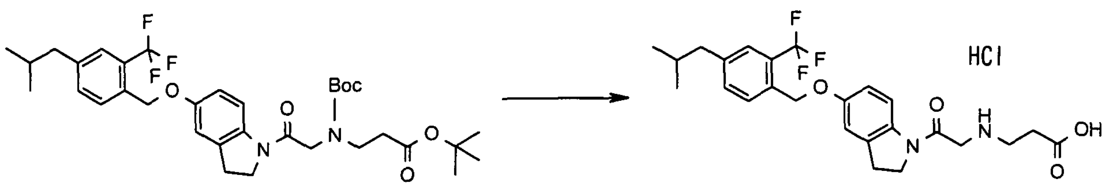

To 3-[N-(tert-butoxycarbonyl)-N-[2-oxo-2-[5-(4-isobutyl-2-trifluoromethylbenzyloxy)indolin-1-yl]ethyl]amino] propionic acid tert-butyl ester (375 mg), 4N HCl/1,4-dioxane (10 mL) was added, and the reaction mixture was stirred overnight. Diethyl ether was added to the reaction mixture, and the precipitated solid was filtered off and dried, whereby the title compound (267 mg) was yielded.
$^1$H-NMR (DMSO-$d_6$) δ:
0.87(6H,d,J=6.6Hz),1.84-1.91(1H,m),2.56(2H,d,J=7.4Hz),2.79(2H,t,J=7.4Hz),3.17-3.22(4H,m),4.06-4.15(4H,m),5.16 (2H,s),6.86(1H,dd,J=2.3,8.7Hz),7.00(1H,d,J=2.3 Hz),7.50-7.67(3H,m),7.98(1H,d,J=8.6Hz).
IR(ATR)$cm^{-1}$:2867,1706,1646,1494,1118.
MS(ESI)m/z:479$(M+H)^+$.
HR-MS (ESI) calcd for $C_{25}H_{30}F_3N_2O_4$ $(M+H)^+$: 479.21577. found 479.21404.

[Example 103]

3-[N-[2-[5-(1-Isobutyl-3-trifluoromethyl-1H-pyrazol-4-ylmethoxy)indolin-1-yl]-2-oxoethyl]amino]propionic acid TFA salt

(1) 1-Isobutyl-3-trifluoromethyl-1H-pyrazole-4-carboxylic acid ethyl ester

[F507]

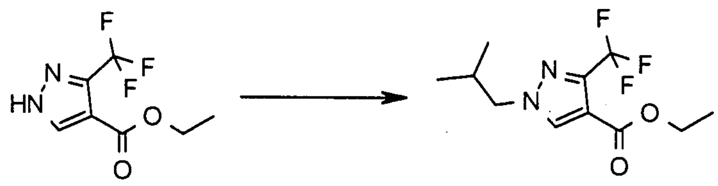

To a DMF solution (15 mL) of 3-trifluoromethyl-1H-pyrazole-4-carboxylic acid ethyl ester (624 mg), 55% sodium hydride (196 mg) was added, and the mixture was stirred for 1 hour at 80°C. Isobutyl iodide (518 μL) was thereto, and the reaction mixture was stirred overnight at the same temperature. This mixture was left to stand to cool to room temperature. Water was added to the reaction mixture, and the resultant mixture was extracted thrice, each with ethyl acetate. The extracts were combined and washed with saturated brine, followed by drying over sodium sulfate anhydrate. After filtration and concentration under reduced pressure, the residue was purified by flash column chromatography (Yamazen Hi-Flash column L), whereby the title compound (604 mg) was yielded as a colorless oily material.
$^1$H-NMR ( $CDCl_3$) δ :
0.94(6H,d,J=6.6Hz),1.35(3H,t,J=7.1Hz),2.20-2.30(1H,m),3.95(2H,d,J=7.1Hz),4.32(2H,q,J=7.1Hz),7.94(1H,s).
MS(ESI)m/z:265(M+H)$^+$.

(2) 1-Isobutyl-3-trifluoromethyl-1H-pyrazole-4-methanol

[F508]

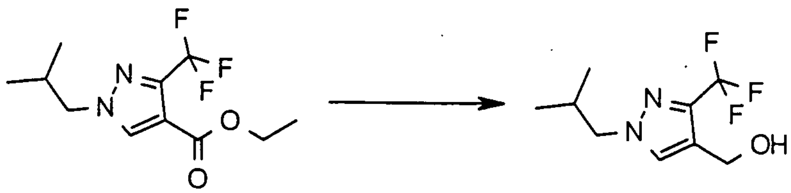

To a THF solution (15 mL) of 1-isobutyl-3-trifluoromethyl-1H-pyrazole-4-carboxylic acid ethyl ester (600 mg), lithium aluminum hydride (103 mg) was added. The mixture was stirred for 20 minutes at reflux. The reaction mixture was left to stand to cool to room temperature and cooled at 0°C. To the reaction mixture, water (105 μL), 1N aqueous sodium hydroxide solution (105 μL), and water (315 μL) were sequentially added, and the resultant mixture was dried over sodium sulfate anhydrate. Insoluble matter was filtered off, and the filtrate was concentrated under reduced pressure, whereby the title compound (523 mg) was yielded.
$^1$H-NMR ($CDCl_3$) δ :
0.92(6H,d,J=6.6Hz),1.73(1H,t,J=5.9Hz),2.17-2.27 (1H,m) , 3.92 (2H, d,J=7.1Hz) , 4.68 (2H, d, J=5. 9Hz) , 7.45 (1H, s).
MS (ESI) m/z : 223 (M+H) $^+$.

(3) 5-(1-Isobutyl-3-trifluoromethyl-1H-pyrazol-4-ylmethoxy)indoline-1-carboxylic acid tert-butyl ester

[F509]

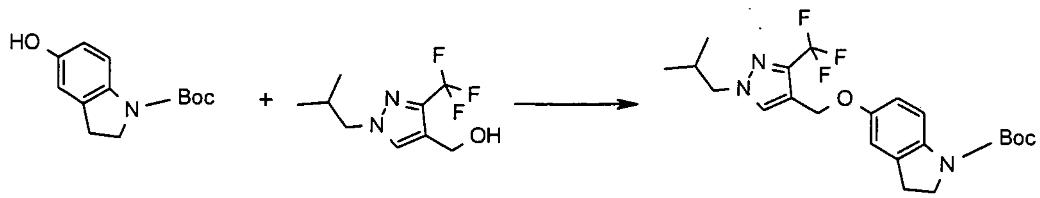

To a THF solution (20 mL) of 5-hydroxyindoline-1-carboxylic acid tert-butyl ester (778 mg), 1-isobutyl-3-trifluoromethyl-1H-pyrazole-4-methanol (490 mg), triphenylphosphine (868 mg), and DEAD (2.2M toluene solution) (1.50 mL) were added, and the mixture was stirred overnight at room temperature. Saturated aqueous sodium bicarbonate solution was added to the reaction mixture, and the resultant mixture was extracted thrice, each with ethyl acetate. The extracts were combined and washed with saturated brine, followed by drying over sodium sulfate anhydrate. Insoluble matter was filtered off, and the filtrate was concentrated under reduced pressure. The residue was purified by flash column chromatography (Yamazen Hi-Flash column 2L), whereby the title compound (564 mg) was yielded.
$^1$H-NMR ($CDCl_3$)$\delta$:
0.92(6H,d,J=6.6Hz),1.55(9H,s),2.16-2.27(1H,m),3.05(2H,t,J=8.6Hz),3.91-3.97(4H,m),4.99(2H,s),6.73-6.77(2H,m), 7.46(1H,s),7.75(1H,s).
MS (ESI) m/z: 440 $(M+H)^+$.

(4) 5-(1-Isobutyl-3-trifluoromethyl-1H-pyrazol-4-ylmethoxy)indoline

[F510]

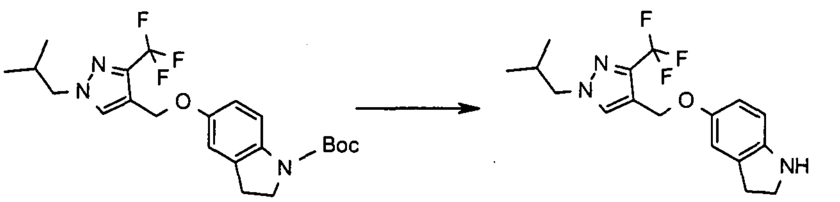

To 5-(1-isobutyl-3-trifluoromethyl-1H-pyrazol-4-ylmethoxy)indoline-1-carboxylic acid tert-butyl ester (555 mg), 4N HCl/1,4-dioxa (15 mL) was added, and the mixture was stirred at room temperature for 1.5 hours. The reaction mixture was concentrated under reduced pressure. Saturated aqueous sodium bicarbonate solution was added to the residue, and the resultant mixture was extracted thrice, each with chloroform. The extract were combined and washed with saturated brine, followed by drying over sodium sulfate anhydrate. Insoluble matter was filtered off, and the filtrate was concentrated under reduced pressure. The residue was purified by flash column chromatography (Yamazen Hi-Flash column L), whereby the title compound (359 mg) was yielded.
$^1$H-NMR ($CDCl_3$)$\delta$:
0.91(6H,d,J=6.6Hz),2.16-2.26(1H,m),3.00(2H,t,J=8.3Hz),3.54(2H,t,J=8.3Hz),3.91(2H,d,J= 7.4Hz),4.95(2H,s), 6.56-6.65(2H,m),6.79-6.80(1H,m),7.46(1H,s).
MS(ESI)m/z:339 $M^+$.

(5) 3-[N-(tert-Butoxycarbonyl)-N-[2-[5-(1-isobutyl-3-trifluoromethyl-1H-pyrazol-4-ylmethoxy)indolin-1-yl]-2-oxoethyl]amino]propionic acid tert-butyl ester

[F511]

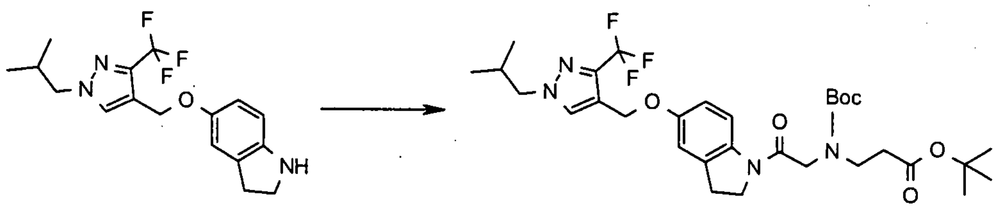

To a DMF solution (10 mL) of 5-(1-isobutyl-3-trifluoromethyl-1H-pyrazol-4-ylmethoxy)indoline (170 mg), 3-[N-(tert-butoxycarbonyl)-N-(carboxymethyl)amino]propionic acid tert-butyl ester (182 mg), DIEA (255 μL), HOBt (81.1 mg), and EDC·HCl (115 mg) were added, and the mixture was stirred overnight at room temperature. Saturated aqueous sodium bicarbonate solution was added to the reaction mixture, followed by extraction thrice, each with ethyl acetate. The extracts were combined and washed with saturated brine, followed by drying over sodium sulfate anhydrate. Insoluble matter was filtered off, and the filtrate was concentrated under reduced pressure. The residue was purified by flash column chromatography (Yamazen Hi-Flash column L), whereby the title compound was yielded (278 mg). $^1$H-NMR ($CDCl_3$) δ :
0.91(6H,d,J=6.6Hz),1.40-1.50(18H,m),2.18-2.25(1H,m),2.55-2.63(2H,m),3.15-3.22(2H,m),3.56-3.61(2H,m),3.91-4.18(6H,m),5.00(2H,s),6.74-6.80(2H,m),7.46-7.48(1H,m),8.10-8.15(1H,m).
MS(ESI)m/z:625(M+H)$^+$.

(6) 3-[N-[2-[5-(1-Isobutyl-3-trifluoromethyl-1H-pyrazol-4-ylmethoxy)indolin-1-yl]-2-oxoethyl]amino]propionic acid TFA salt

[F512]

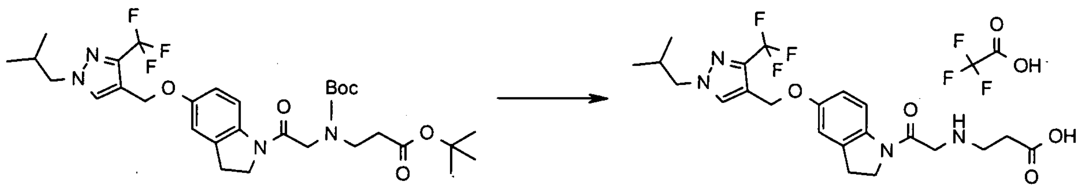

To a dichloromethane solution (10 mL) of 3-[N-(tert-butoxycarbonyl)-N-[2-[5-(1-isobutyl-3-trifluoromethyl-1H-pyrazol-4-ylmethoxy)indolin-1-yl]-2-oxoethyl]amino]propionic acid tert-butyl ester (270 mg), TFA (5.0 mL) was added, and the mixture was stirred for 2 hours at room temperature. The reaction mixture was concentrated under reduced pressure, whereby the title compound (151 mg) was yielded. $^1$H-NMR (DMSO- $d_6$) δ:
0.84(6H,d,J=6.6Hz),2.05-2.16(1H,m),2.73(2H,t,J=7.4Hz),3.16-3.23(4H,m),3.99-4.15 (6H,m) , 4.98 (2H,s) , 6.85 (1H, dd, J=2.5,8.8Hz) , 6.97 (1H,d, J=2.5 Hz), 7.96 (1H, d,J=8.8Hz),8.09 (1H,s) ,8.98 (1H,s).
MS (ESI) m/z : 469 (M+H)$^+$.

[Example 104]

2-[N-[3-Oxo-3-[5-[(4-phenyl-5-trifluoromethyl-2-thienyl)methoxy]indolin-1-yl]propyl]amino]acetic acid

(1) 1-[3-(N-tert-Butoxycarbonylamino)propionyl]-5-[(4-phenyl-5-trifluoromethyl-2-thienyl)methoxy]indoline

[F513]

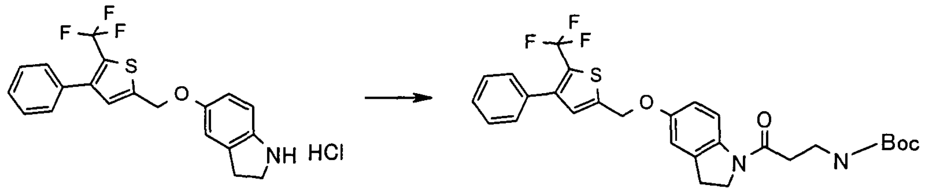

To a solution of 3-(N-tert-butoxycarbonylamino)propionic acid (114 mg) in DMF (5.0 mL), DIEA (316 μL), HOBt (105 mg), and EDC·HCl (149 mg) were added at room temperature. The mixture was stirred for 10 minutes, and 5-[(4-phenyl-5-trifluoromethyl-2-thienyl)methoxy]indoline hydrochloride (247 mg) was added thereto at room temperature. The reaction mixture was stirred for 14 hours, and subsequently concentrated under reduced pressure. Ethyl acetate (10.0 mL) and water (7.5 mL) were added to the concentrate for phase separation, followed by extraction with ethyl acetate (3 x 5.0 mL). The organic layers were combined, and solvent was removed therefrom under reduced pressure. The residue was purified by silica gel chromatography (Biotage 25M), whereby the title compound (324 mg) was yielded.
$^1$H-NMR ($CDCl_3$)δ:
1.43(9H,s),2.61(2H,t,J=5.4Hz),3.18(2H,t,J=8.4Hz),3.51(2H,dt,J =5.4,5.4Hz),4.03(2H,t,J=8.4Hz),5.20(2H,s),6.82(1H, dd,J=8.5,2. 4Hz),6.84(1H,br s),7.06(1H,s),7.42-7.39(5H,m),8.15(1H,d,J=8.5Hz).
MS(ESI) m/z : 547 $(M+H)^+$.

(2) 1-(3-Aminopropionyl)-5-[(4-phenyl-5-trifluoromethyl-2-thienyl)methoxy]indoline hydrochloride

[F514]

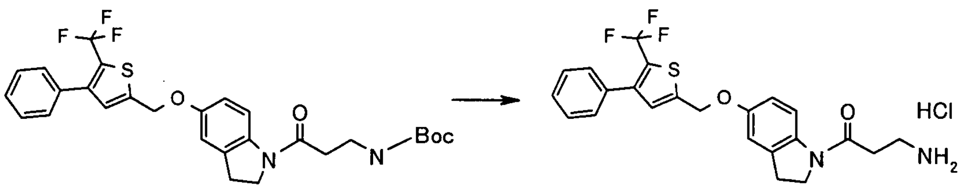

To a solution of 1-[3-(N-tert-butoxycarbonylamino)propionyl]-5-[(4-phenyl-5-trifluoromethyl-2-thienyl)methoxy] indoline (324 mg) in dioxane (0.5 mL), 4N HCl/1,4-dioxane (2.0 mL) was added at room temperature. The reaction mixture was stirred for 13 hours, and subsequently, concentrated under reduced pressure. Diethyl ether (5.0 mL) was added to the residue, and the precipitated solid was collected by filtration and dried under reduced pressure, whereby the title compound (203 mg) was yielded.
MS (ESI)m/z:447$(M+H)^+$.

(3) 2-[N-[3-Oxo-3-[5-[(4-phenyl-5-trifluoromethyl-2-thienyl)methoxy]indolin-1-yl]propyl]amino]acetic acid tert-butyl ester

[F515]

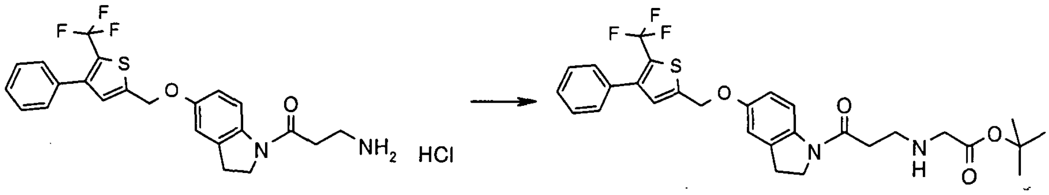

To a suspension of 1-(3-aminopropionyl)-5-[(4-phenyl-5-trifluoromethyl-2-thienyl)methoxy]indoline hydrochloride (203 mg) in acetonitrile (3.5 mL), tert-butyl bromoacetate (68.4 μL) and DIEA (293 μL) were added at room temperature. The mixture was stirred for 7 hours at 80°C, and subsequently concentrated under reduced pressure. A 20% methanol/chloroform mixture (5.0 mL) and saturated aqueous sodium hydrogen carbonate solution (5.0 mL) were added to the reaction mixture for phase separation. The resultant mixture was extracted with a 20% methanol/chloroform mixture (3 x 5.0 mL). The extracts were combined, and the resultant extract was concentrated under reduced pressure. The residue was purified by silica gel chromatography (Biotage 25M), whereby the title compound (129 mg) was yielded.
$^1$H-NMR ($CDCl_3$) δ:
1.47(9H,s),2.63(2H,t,J=6.3Hz),3.00(2H,t,J=6.3Hz),3.18(2H,t,J= 8.4Hz),3.35(2H,s),4.06(2H,t,J=8.4Hz),5.19(2H,s),6.80 (1H,dd,J= 8.8,2.2Hz),6.83(1H,brs),7.06(1H,s),7.38-7.43(5H,m) ,8.17(1H,d,J=8.8Hz).
MS (ESI) m/z : 561 $(M+H)^+$.

(4) 2-[N-[3-Oxo-3-[5-[(4-phenyl-5-trifluoromethyl-2-thienyl)methoxy]indolin-1-yl]propyl]amino]acetic acid

[F516]

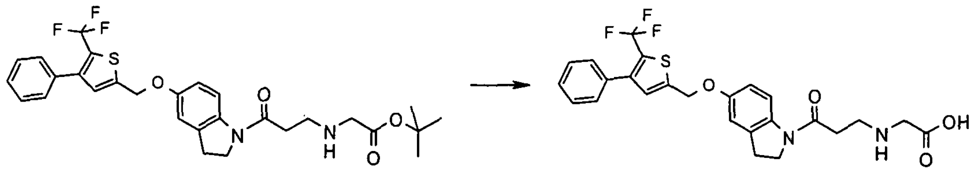

To a solution of 2-[N-[3-oxo-3-[5-[(4-phenyl-5-trifluoromethyl-2-thienyl)methoxy]indolin-1-yl]propyl]amino]acetic acid tert-butyl ester (129 mg) in 1,4-dioxane (1.0 mL), 4N HCl/1,4-dioxane solution (2.0 mL) was added at room temperature. The reaction mixture was stirred for 13 hours, and subsequently concentrated under reduced pressure. Ethyl acetate (5.0 mL) was added to the residue, and the precipitated solid was collected by filtration and dried under reduced pressure, whereby the title compound (78.9 mg) was yielded.
$^1$H-NMR (DMSO-$d_6$) δ:
2.86-2.92(2H,br m) ,3.16(2H,t,J=8.3Hz) ,3.26(2H,t,J=6.8Hz) ,3.92(2H,s) ,4.06(2H,t ,J=8.3Hz) ,5.35(2H,s) ,6.87(1H,dd, J=8.8,2.2Hz) ,7.01(1H,d,J=2.2H z),7.36(1H,s),7.50-7.41(5H,m),7.99(1H,d,J=8.8Hz).
MS (ESI) m/z : 505 $(M+H)^+$.
Anal. Calcd for $C_{25}H_{23}F_3N_2O_4S \cdot$ 1.0HCl:C,55.50;H,4.47;Cl,6.55;F,10.54;N,5.18;S,5.93.Found:C,5 5.53;H,4.23;Cl,6.35; F,10.31;N,5.22;S,5.98.

[Example 105]

3-[N-[2-Oxo-2-[7-[[4-phenyl-5-(trifluoromethyl)-2-thienyl]methoxy]-2,3-dihydro-4H-1,4-benzoxazin-4-yl]ethyl]amino] propionic acid TFA salt

(1) 2-Amino-5-(benzyloxy)phenol

Reference: Eur. J. Med. Chem. 37 (2002), 461-468
[F517]

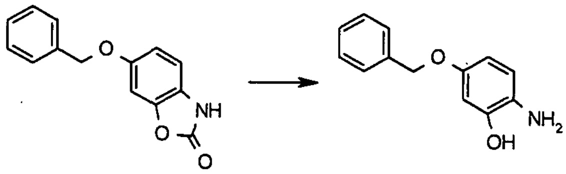

To a solution of 6-(benzyloxy)-1,3-benzoxazol-2(3H)-one (commercially available) (5.00 g) in methanol (70 mL), aqueous sodium hydroxide (12.4 g) solution (70 mL) was added at room temperature, and the mixture was refluxed for 24 hours. The reaction mixture was left to stand to cool to room temperature. 6N Hydrochloric acid (60 mL) was added to the reaction mixture to acidify the mixture. The resultant mixture was filtered, and saturated aqueous sodium hydrogencarbonate solution was added to the filtrate to alkalize the filtrate. The resultant solid was collected by filtration, followed by washing with water and drying (in vacuo at room temperature for 10 hours). The formed solid was reprecipitated with ethyl acetate-hexane, collected by filtration, and dried, whereby the title compound (3.00 g) was yielded.
$^1$H-NMR (DMSO-$d_6$) δ:
4.10(2H,br s) ,4.90(2H,s) ,6.24(1H,dd,J=8.4,2.8Hz) ,6.36(1H,d,J=2.8Hz) ,6.48 (1H,d,J=8.4Hz) ,7.37-7.41(5H,m) ,9.00(1H,br s).
MS(ESI)m/z:216(M+H)$^+$

(2) 4-(Benzyloxy)-2-hydroxyphenylcarbamic acid tert-butyl ester

Reference: Tetrahedron, 56, (2000), 605-614
[F518]

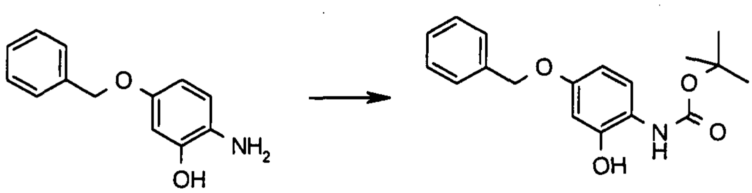

To a solution of 2-amino-5-(benzyloxy)phenol (3.00 g) in THF (30 mL), $Boc_2O$ (3.64 g) was added at room temperature, and the mixture was stirred for 3 hours at room temperature. The reaction mixture was concentrated under reduced pressure. The residue was purified by silica gel flash column chromatography (Biotage 40M), whereby the title compound (2.68 g) was yielded.
$^1$H-NMR ( $CDCl_3$) δ :
1.53(9H,s) ,5.03(2H,s) ,6.45(1H,br s) ,6.50(1H,dd,J=8.8,2.8Hz) ,6.65(1H,d,J=2.8Hz) ,6.83(1H,d,J=8.8 Hz), 7.29-7.45 (5H,m) ,8.43 (1H,br s).
MS (ESI) m/z : 316 (M+H)$^+$.

(3) 7-(Benzyloxy)-2,3-dihydro-4H-1,4-benzoxazine-4-carboxylic acid tert-butyl ester

Reference: Tetrahedron, 56, (2000), 605-614
[F519]

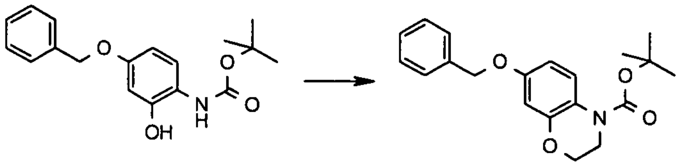

To a solution of 4-(benzyloxy)-2-hydroxyphenylcarbamic acid tert-butyl ester (1.50 g) in acetone (100 mL), dibromoethane (3.28 mL) and potassium carbonate (13.2 g) were added at room temperature, and the mixture was refluxed for 16 hours at room temperature. The reaction mixture was left to stand to cool to room temperature, and the mixture was concentrated under reduced pressure. The residue was purified by silica gel flash column chromatography (Biotage 40M), whereby the title compound (1.26 g) was yielded.
$^1$H-NMR ($CDCl_3$) δ:
1.54 (9H,s) ,3.83 (2H,t,J=4.4Hz) ,4.24(2H,t,J=4.4Hz) ,5.02(2H,s),6 .51(1H, d,J=2.9Hz) ,6.55 (1H, dd,J=9.0,2.9Hz) , 7.29-7.45(5H,m),7.64(1H,br s).
MS (ESI) m/z : 342 $(M+H)^+$.

(4) 7-Hydroxy-2,3-dihydro-4H-1,4-benzoxazine-4-carboxylic acid tert-butyl ester

[F520]

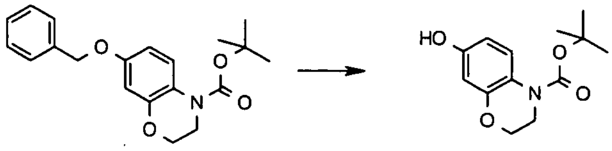

To a solution of 7-(benzyloxy)-2,3-dihydro-4H-1,4-benzoxazine-4-carboxylic acid tert-butyl ester (1.25 g) in a mixture of methanol (10 mL) and THF (10 mL), 5% Pd/C (500 mg) was added, and the mixture was stirred for 2 hours under a hydrogen atmosphere at room temperature. The reaction mixture was filtered, and the filtrate was concentrated under reduced pressure. The residue was purified by silica gel flash column chromatography (Biotage 40M), whereby the title compound (856 mg) was yielded. $^1$H-NMR ($CDCl_3$) δ:
1.53(9H,s) ,3.82(2H,t,J=4.5Hz) ,4.22(2H,t,J=4.5Hz) ,4.65(1H,s) ,6 .35-6.40(2H,m) ,7.59(1H,br s).
MS (ESI) m/z : 252 $(M+H)^+$.

(5) 7-[[4-Phenyl-5-(trifluoromethyl)-2-thienyl]methoxy]-2,3-dihydro-4H-1,4-benzoxazine-4-carboxylic acid tert-butyl ester

[F521]

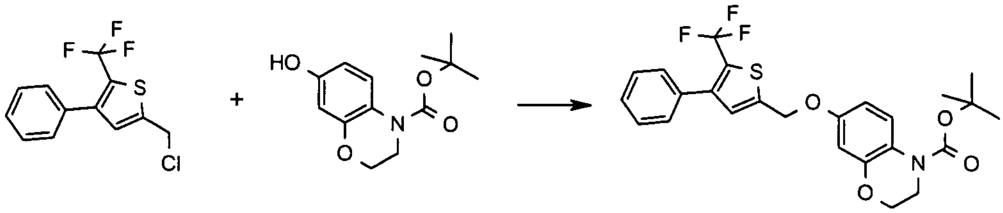

To a solution of 5-(chloromethyl)-3-phenyl-2-(trifluoromethyl)thiophene (198 mg) and 7-hydroxy-2,3-dihydro-4H-1,4-benzoxazine-4-carboxylic acid tert-butyl ester (180 mg) in DMF (4.0 mL), potassium carbonate (148 mg) was added at room temperature, and the mixture was stirred for 16 hours at 50°C. The reaction mixture was cooled to room temperature, and the precipitated matter was removed by filtration. Water (40 mL) and saturated aqueous ammonium chloride solution (40 mL) were added to the filtrate, followed by extraction with ethyl acetate (2 x 30 mL). The extracts were combined and washed with saturated brine (30 mL), followed by drying over sodium sulfate anhydrate. Solvent was removed under reduced pressure. The resultant residue was purified by silica gel flash column chromatography (Biotage 25M), whereby the title compound (313 mg) was yielded.
$^1$H-NMR ($CDCl_3$) δ :
1.53(9H,s) ,3.83(2H,t,J=4.4Hz) ,4.24(2H,t,J=4.4Hz) ,5.17(2H,s) ,6 .51(1H,d,J=2.8Hz) ,6.54(1H,dd,J=9.0,2.8Hz) ,7.06 (1H,s) ,7.37-7.45(5H,m),7.69(1H,br s).
MS (ESI) m/z : 514 $(M+Na)^+$.

(6) 7-[[4-Phenyl-5-(trifluoromethyl)-2-thienyl]methoxy]-3,4-dihydro-2H-1,4-benzoxazine hydrochloride

[F522]

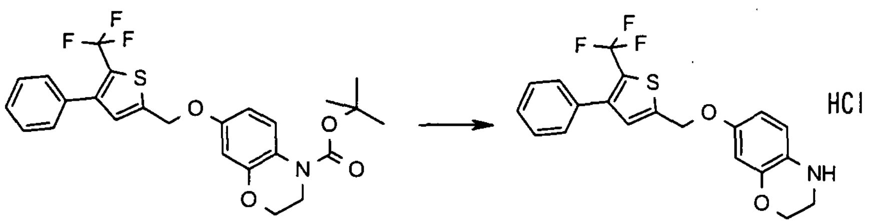

To 7-[[4-phenyl-5-(trifluoromethyl)-2-thienyl]methoxy]-2,3-dihydro-4H-1,4-benzoxazine-4-carboxylic acid tert-butyl ester (310 mg), 4N HCl/1,4-dioxane (5.0 mL) was added at room temperature, and the mixture was stirred for 2 hours at room temperature. The reaction mixture was concentrated under reduced pressure, and the resultant residue was slurried with hexane. The slurry was filtered, and the collected solid was dried (in vacuo at 40°C for 2 hours), whereby the title compound (268 mg) was yielded.
$^1$H-NMR ($CDCl_3$) δ:
3.62-3.70(2H,m),4.44-4.56(2H,m) ,5.19(2H,s) ,6.59(1H,d,J=2.7Hz) ,6.66(1H,dd,J=8.8,2.7 Hz) ,7.08(1H,s) ,7.38-7.45 (5H,m) ,7.52(1H,d,J=8.8Hz).
No NH was observed.
MS(ESI)m/z:391 $M^+$.

(7) 3-[N-(tert-Butoxycarbonyl)-N-[2-oxo-2-[7-[[4-phenyl-5-(trifluoromethyl)-2-thienyl]methoxy]-2,3-dihydro-4H-1,4-benzoxazin-4-yl]ethyl]amino]propionic acid tert-butyl ester

[F523]

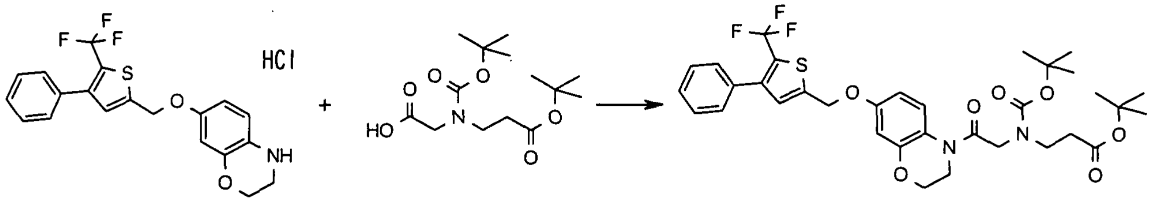

7-[[4-Phenyl-5-(trifluoromethyl)-2-thienyl]methoxy]-3,4-dihydro-2H-1,4-benzoxazine hydrochloride (140 mg) was dissolved in DMF (5.0 mL). To the resultant solution, 2-[N-(tert-butoxycarbonyl)-N-[3-(tert-butoxy)-3-oxopropyl]amino]acetic acid (110 mg), EDC·HCl (81.5 mg), HOBt (57.9 mg), and DIEA (0.171 mL) were added at room temperature, followed by stirring for 12 days. The reaction mixture was concentrated under reduced pressure, and ethyl acetate (30 mL), saturated aqueous sodium hydrogen carbonate solution (40 mL), and water (40 mL) were added to the concentrate for phase separation. The aqueous layer was extracted with ethyl acetate (20 mL). The resultant extracts were combined and dried over sodium sulfate anhydrate. Solvent was removed under reduced pressure. The residue was purified by silica gel flash column chromatography (Biotage 25M), whereby the title compound (180 mg) was yielded. $^1$H-NMR ($CDCl_3$) δ :
1.30-1.60(18H,m),2.49-2.61(2H,m),3.48-3.60(2H,m),3.89(2H,br s),4.21-4.32(4H,m),5.18(1/2 of 2H,s),5.19(1/2 of 2H,s), 6.50-6.60(2H,m),7.08(1H,s),7.10(1/2 of 1H,br s),7.37-7.46(SH,m),7.50(1/2 of 1H,br s).
MS(ESI)m/z:677(M+H)$^+$.

(8) 3-[N-[2-Oxo-2-[7-[[4-phenyl-5-(trifluoromethyl)-2-thienyl]methoxy]-2,3-dihydro-4H-1,4-benzoxazin-4-yl]ethyl]amino] propionic acid TFA salt

[F524]

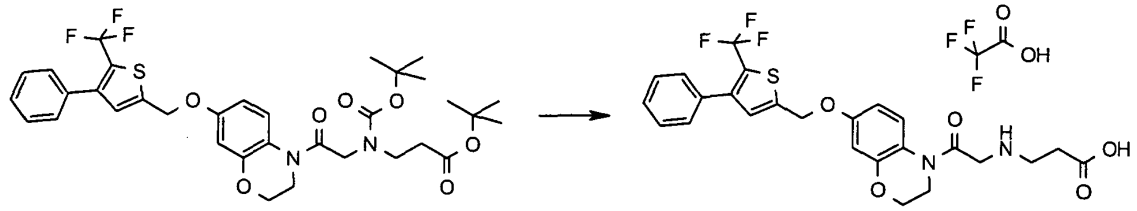

To a solution of 3-[N-(tert-butoxycarbonyl)-N-[2-oxo-2-[7-[[4-phenyl-5-(trifluoromethyl)-2-thienyl]methoxy]-2,3-dihydro-4H-1,4-benzoxazin-4-yl]ethyl]amino]propionic acid tert-butyl ester (47.0 mg) in dichloromethane (4.0 mL), TFA (1.0 mL) was added at room temperature, and the mixture was stirred for 4 hours at room temperature. The reaction mixture was concentrated under reduced pressure, and triturated with diethyl ether and hexane. The resultant solid was collected by filtration and dried (in vacuo at room temperature for 1 hour), whereby the title compound (153 mg) was yielded.
$^1$H-NMR (DMSO-$d_6$) δ:
2.70(2H,br s),3.17(2H,br s),3.76(2/3 of 2H,br s),3.89(1/3 of 2H,br s),4.27(4H,br s),5.37(2H,s),6.60-6.70(2H,m),7.29(1/3 of
1H,br s) ,7.29(1H,s) ,7.40=7.50(5H,m) ,8.02(2/3 of 1H,br s). Neither COOH nor NH was observed.
IR (ATR) $cm^{-1}$:
3157,2978,1726,1680,1508,1381,1288,1174,1119,1014,791,766,721 ,696.
MS (ESI) m/z : 521 (M+H)$^+$.
HR-MS (ESI) Calcd for
$C_{25}H_{24}F_3N_2O_5S$(M+H)$^+$: 521.13580.Found: 521.13115.
Anal. Calcd for $C_{25}H_{23}F_3N_2O_5S$·
1.0TFA: C,51.11;H,3.81;F,17.96;N,4.41;S,5.05.Found:C,50.83;H,3 .73;F,18.06;N,4.30;S,4.90.

[Example 106]

3-[N-[2-[5-(4-Cyclohexyl-3-trifluoromethylbenzyloxy)indolin-1-yl]-2-oxoethyl]-N-methylamino]propionic acid

[F525]

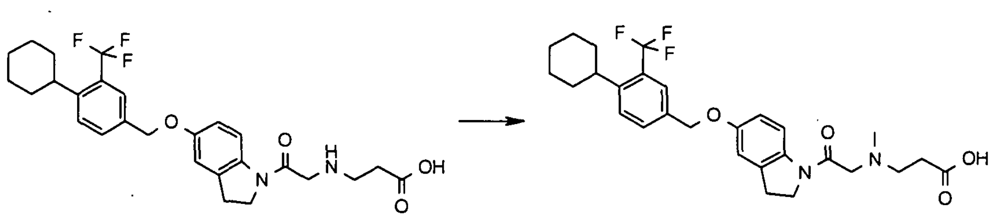

To a methanol solution (2 mL) of 3-[N-[2-[5-(4-cyclohexyl-3-trifluoromethylbenzyloxy)indolin-1-yl]-2-oxoethyl] amino]propionic acid(0.20 g), 37% formalin solution (94 μL), and acetic acid (0.10 mL), sodium cyanoborohydride (75 mg) was added at room temperature, and the mixture was stirred for 3.5 hours. Saturated aqueous sodium bicarbonate solution was added to the reaction mixture to adjust pH to 8, followed by extraction with a mixture of chloroform/methanol (10/1, v/v). The extracts were combined and washed with saturated brine, and dried over sodium sulfate anhydrate. Solvent was removed, and the residue was purified by thin-layer chromatography, followed by addition of diethyl ether/hexane. The precipitated solid was collected by filtration and dried, whereby the title compound (114 mg) was yielded.
$^1$H-NMR (DMSO-$d_6$) δ:
1.23-1.89(12H,m) ,2.22-2.87(10H,m) ,3.03-3.16(1H,m) ,4.02-4.26(1H,m),5.02-5.21(2H,m),6.74-7.04(2H,m),7.57 7.74 (3H,m) , 7.81-8.00 (1H,m).
IR(ATR)cm$^{-1}$:2925,2854,1637,1589,1489,1408,1379.
MS (ESI) m/z : 519 $(M+H)^+$.
HR-MS (AqTOF) Calcd for $C_{28}H_{34}F_3N_2O_4$: 519.2471. Found: 519.2409.

[Example 107]

3-[N-[2-[5-(4-Cyclohexyl-3-trifluoromethylbenzyloxy)indolin-1-yl]-2-oxoethyl]-N-ethylamino]propionic acid

[F526]

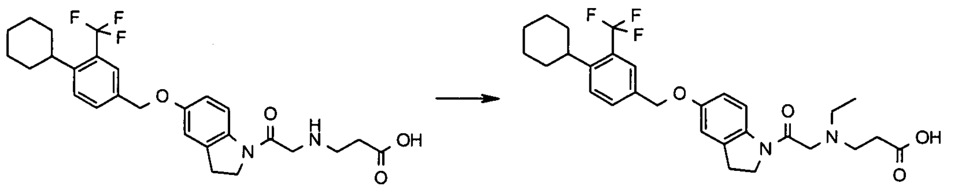

To a methanol solution (2 mL) of 3-[N-[2-[5-(4-cyclohexyl-3-trifluoromethylbenzyloxy)indolin-1-yl]-2-oxoethyl] amino]propionic acid (0.20 g), formaldehyde (67 μL), and acetic acid (0.10 mL), sodium cyanoborohydride (75 mg, 1.2 mmol) was added at room temperature, and the mixture was stirred for 3 days. Saturated aqueous sodium bicarbonate solution was added to the reaction mixture to adjust pH thereof to 8, followed by extraction with a mixture of chloroform/methanol (10/1, v/v). The extracts were combined and washed with saturated brine, and dried over sodium sulfate anhydrate. Solvent was removed therefrom, and the residue was purified by reverse phase chromatography (GILSON NOMURA DEVELOSIL COMBI-RP5). Diethyl ether/hexane was added to the purified product, and the precipitated solid was collected by filtration and dried, whereby the title compound (21 mg) was yielded.
$^1$H-NMR (DMSO-$d_6$) δ:
0.90-1.06(3H,m),1.14-1.59(6H,m),1.61-1.86(4H,m) ,2.36(2H,t,J=7.1Hz) ,2.58-2.70(2H,m) ,2.74-2.86(3H,m),3.01-3.14 (2H,m) ,3.41(2H,s) ,4.12(2H,t,J=8.3Hz) ,5.09(2H,s) ,6.80(1H,d d,J=8.8,2.7Hz) ,6.93(1H,s),7.61-7.69(3H,m) ,7.96(1H, d,J=8.8Hz) ,8.14(10H,s).
MS (ESI) m/z : 533 $(M+H)^+$.
HR-MS(AqTOF)Calcd for $C_{29}H_{36}F_3N_2O_4$ $(M+H)^+$: 533.2627. Found: 533.2699.

[Example 108]

3-[N-[2-[5-(4-Cyclopentyl-3-trifluoromethylbenzyloxy)indolin-1-yl]-2-oxoethyl]-N-methyl]amino]propionic acid

[F527]

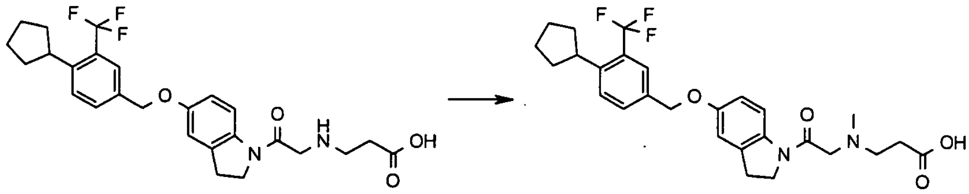

To a methanol solution (5 mL) of 3-[N-[2-[5-(4-cyclopentyl-3-trifluoromethylbenzyloxy)indolin-1-yl]-2-oxoethyl] amino]propionic acid (0.15 g), 37% formalin solution (0.12 mL), and acetic acid (0.50 mL), sodium cyanoborohydride (80 mg) was added at room temperature, and the mixture was stirred for 1 day. Saturated aqueous sodium bicarbonate solution was added to the reaction mixture to adjust pH thereof to 8, followed by extraction with a mixture of chloroform/ methanol (10/1, v/v). The extracts were combined and washed with saturated brine, followed by drying over sodium sulfate anhydrate. Solvent was removed, and the residue was purified by thin-layer chromatography. Diethyl ether/ hexane was added to the purified product, and the precipitated solid was collected by filtration and dried, whereby the title compound (17 mg) was yielded.
$^1$H-NMR (DMSO-$d_6$) δ:
0.77-0.88(4H,m),1.11-1.35(4H,m),1.54-1.72(2H,m),1.74-1.91(2H,m),1.92-2.04(1H,m),2.29(3H,s),2.38(2H,t,J=7.0Hz), 2.74(2H,t,J=7.0Hz),3 .08(2H,t,J=8.2Hz),4.05-4.18(2H,m),5.11(2H,s),6.80(1H,d,J=8.8Hz),6.93(1H,s),7.54-7.74 (2H, m), 7.96 (1H, d, J=8.8Hz) .
MS (ESI) m/z : 505 $(M+H)^+$.

[Example 109]

3-[N-[2-[5-[(3-Cyano-4-cyclohexylphenyl)methoxy]indolin-1-yl]-2-oxoethyl]-N-methylamino]propionic acid

[F528]

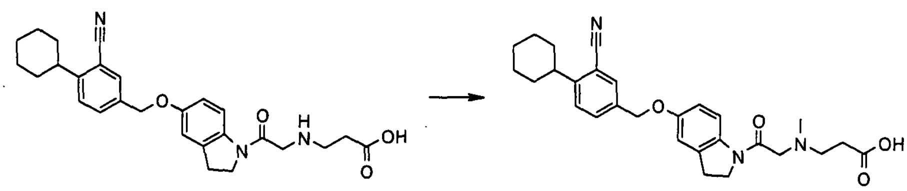

To a solution of 3-[N-[2-[5-[(3-cyano-4-cyclohexylphenyl)methoxy]indolin-1-yl]-2-oxoethyl]amino]propionic acid (94.6 mg) in methanol (2.0 mL), 37% aqueous formaldehyde solution (48.5 μL) and acetic acid (100 μL) were added at room temperature, and the mixture was stirred for 1 hour at room temperature. Sodium cyanoborohydride (38.6 mg) was added to the reaction mixture at room temperature, followed by stirring for 3 hours. Water (3.0 mL) was added to the reaction mixture, and the pH of the resultant mixture was adjusted to 7 with saturated aqueous sodium hydrogen carbonate solution. A 20% methanol/chloroform mixture (5.0 mL) was added to the the reaction mixture, and the aqueous layer was extracted with a 20% methanol/chloroform mixture (3 x 3.0 mL). The extracts were combined and dried over sodium sulfate anhydrate. Insoluble matter was removed by filtration, and the filtrate was concentrated under reduced pressure, whereby the title compound (39.9 mg) was yielded.

$^1$H-NMR (DMSO-$d_6$) δ:
1.21-1.54(5H,m),1.69-1.85(5H,m),2.29(3H,s),2.38(2H,t,J=6.8Hz),2.74(2H,t,J=6.8H.z),2 .80-2.88(1H,m),3.08(2H,t, J=8.5Hz), 3.32 (2H, s), 4.10 (2H, t, J=8.5Hz), 5 . 06 (2H, s), 6.80 (1H, s), 6.93 (1H, s), 7.53 (1H, d, J=8.0Hz), 7.70 (1H, dd , J=8.0,1.2Hz),7.80(1H,s),7.96(1H,d,J=8.5Hz).
MS (ESI) m/z : 476 $(M+H)^+$.
Anal. Calcd for $C_{28}H_{33}N_3O_4$·
1.5$H_2$O:C,66.91;H,7.22;N,8.36.Found:C,67.09;H,7.02;N,8.15.

[Example 110]

3-[N-[2-[5-(4-Isobutyl-3-trifluoromethylbenzyloxy) indolin-1-yl]-2-oxoethyl]-N-methylamino]propionic acid

(1) 3-[N-(Benzyloxycarbonylmethyl)amino]propionic acid ethyl ester

[F529]

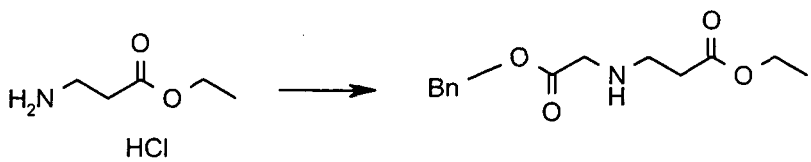

To acetonitrile solution (150 mL) of β-alanine ethyl ester hydrochloride (5.0 g) and DIEA (4.5 mL, 26 mmol), bromoacetic acid benzyl ester (2.1 mL, 13 mmol) was added at room temperature. The mixture was heated to 80°C and stirred for 14 hours. The reaction mixture was concentrated, and the residue was purified by silica gel flash column chromatography (Biotage 40M), whereby the title compound (2.3 g) was yielded.
$^1$H-NMR ($CDCl_3$ ) δ :
1.26(3H,t,J=7.1Hz),2.50(2H,t,J=6.6Hz),2.91(2H,t,J=6.5Hz),3.47 (2H,s),4.10-4.17(2H,m),5.17(2H,s),7.31-7.39(5H,m).
MS(ESI)m/z:266$(M+H)^+$.

(2) 3-(N-Carboxymethyl-N-methylamino)propionic acid ethyl ester

[F530]

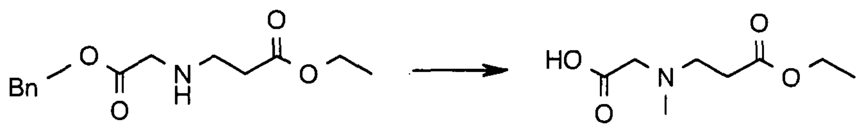

A suspension of 3-[N-(benzyloxycarbonylmethyl)amino]propionic acid ethyl ester (2.4 g), 37% aqueous formalin solution (2 mL), and 10% palladium hydroxide/C (1.2 g) in ethanol (50 mL) was stirred for 1 day under a hydrogen atmosphere at room temperature. The catalyst was removed by filtration, and the filtrate was concentrated, whereby the title compound (1.5 g) was yielded.
$^1$H-NMR ($CDCl_3$ ) δ :
1.19-1.33(3H,m),2.53(3H,s),2.54-2.70(2H,m),2.74-3.07 (2H,m), 3.29 (2H, s), 4.17 (2H, q, J=7.2Hz) .
MS (ESI) m/z : 190 $(M+H)^+$.

(3) 5-(4-Isobutyl-3-trifluoromethylbenzyloxy)indoline-1-carboxylic acid tert-butyl ester

[F531]

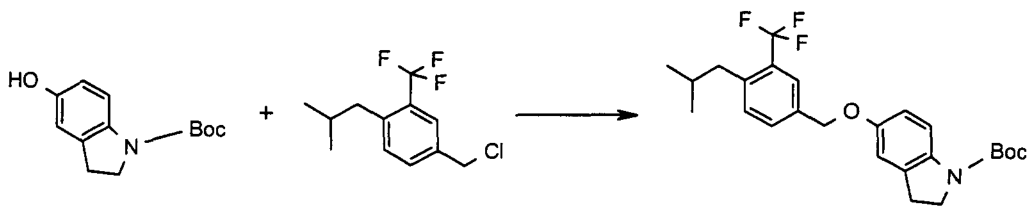

To a DMF solution (5.0 mL) of 5-hydroxyindoline-1-carboxylic acid tert-butyl ester (118 mg), 4-chloromethyl-1-isobutyl-2-trifluoromethylbenzene (150 mg) and potassium carbonate (104 mg) were added, and the mixture was stirred overnight at 70°C. The reaction mixture was left to stand to cool to room temperature and filtered. The filtrate was concentrated under reduced pressure, and the residue was purified by flash column chromatography (Yamazen Hi-Flash column L), whereby the title compound (212 mg) was yielded.
$^1$H-NMR ($CDCl_3$ ) δ :
0.93(6H,d,J=6.6Hz), 1.55(9H,s),1.91-2.01(1H,m),2.66(2H,d,J=7.4Hz),3.06(2H,t,J=8.7Hz),3.97(2H,s),5 .01(2H,s), 6.75-6.80(2H,m),7.32(1H,d,J=8.1Hz),7.51(1H,d,J=7.8Hz),7.68-7.76(2H,m).
MS (ESI) m/z :450 $(M+H)^+$.

(4) 3-[N-[2-[5-(4-Isobutyl-3-trifluoromethylbenzyloxy)indolin-1-yl]-2-oxoethyl]-N-methylamino]propionic acid ethyl ester

[F532]

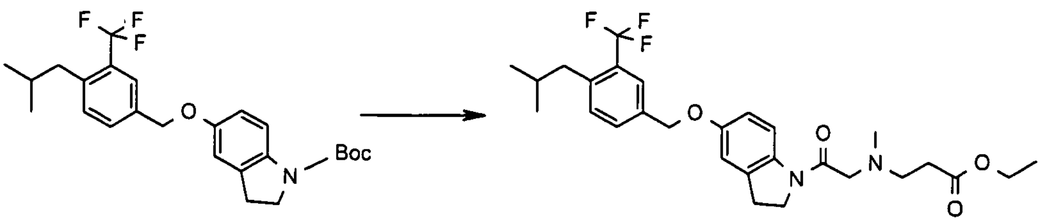

To 5-(4-isobutyl-3-trifluoromethylbenzyloxy)indoline-1-carboxylic acid tert-butyl ester (200 mg), 4N HCl/1,4-dioxane (10 mL) was added, and the mixture was stirred for 1 hour at room temperature. The reaction mixture was concentrated under reduced pressure. The residue was dissolved in DMF (10 mL), and to the resultant solution, 3-(N-carboxylmethyl-N-methylamino)propionic acid ethyl ester (92.6 mg), DIEA (227 μL), HOBt (78.2 mg, 0.578 mmol), and EDC-HCl (111 mg, 0.578 mmol) were added, followed by stirring overnight at room temperature. Saturated aqueous sodium bicarbonate solution was added to the reaction mixture. The resultant mixture was extracted thrice, each with ethyl acetate. The extracts were combined and washed with saturated brine, followed by drying over sodium sulfate anhydrate. Insoluble matter was removed by filtration, and the filtrate was concentrated under redcued pressure. The residue was purified by NH-silica gel flash column chromatography (Yamazen Hi-Flash column L), and further purified through flash column chromatography (Yamazen Hi-Flash column L), whereby the title compound (62 mg) was yielded.
$^1$H-NMR ($CDCl_3$) δ :
0.93(6H,d,J=6.6Hz), 1.23(3H,t,J=7.1Hz),1.91-2.01(1H,m),2.39(3H,s),2.51(2H,t,J=7.0Hz),2.66(2H,d,J=7.1Hz),2 .88 (2H,t,J=7.0Hz),3.15(2H,t,J=8.3Hz),3.30(2H,s),4.07-4.17 (4H,m), 5.02 (2H, s), 6.78-6.83(2H,m),7.32(1H,d,J=8.1Hz),

7.51(1H,d,J=8.1Hz),7.67(1H,s),8 .15(1H,d,J=8.8Hz).
MS (ESI) m/z : 521 $(M+H)^+$.

(5) 3-[N-[2-[5-(4-Isobutyl-3-trifluoromethylbenzyloxy)indolin-1-yl]-2-oxoethyl]-N-methylamino]propionic acid

[F533]

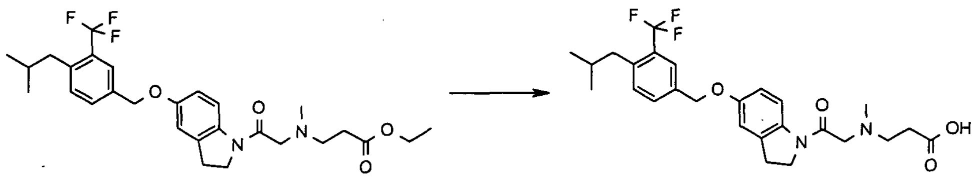

To a THF solution (3.0 mL) of 3-[N-[2-[5-(4-isobutyl-3-trifluoromethylbenzyloxy)indolin-1-yl]-2-oxoethyl]-N-methylamino]propionic acid ethyl ester (60.0 mg), methanol (0.35 mL) and 1N aqueous sodium hydroxide solution (0.350 mL) were added, and the mixture was stirred for 1.5 hours at room temperature. 1N Hydrochloric acid was added to the reaction mixture to neutralize the mixture. The neutralized mixture was diluted with water, and subsequently, the diluted mixture was extracted five times, each with a mixture of 17% methanol/chloroform. The extracts were combined and dried over sodium sulfate anhydrate. Insoluble matter was removed by filtration, and the filtrate was concentrated under reduced pressure. The residue was purified by flash column chromatography (Yamazen Hi-Flash column L), whereby the title compound (53 mg) was yielded.
$^1$H-NMR (DMSO-$d_6$ ) δ:
0.89(6H,d,J=6.6Hz),1.89-2.05(3H,m),2.19(3H,s),2.57-2.63(4H,m),3.07-3.18(4H,m),4.18(2H,t,J=8.5Hz),5.11(2H,s),6.80(1H,d,J=8.8Hz),6 .94(1H,s),7.48(1H,d,J=7.8Hz),7.66(1H,d,J=8.1Hz),7.73(1H,s),7. 97(1H,d,J=8.8Hz).
MS(ESI)m/z:493 $(M+H)^+$.

[Example 111]

3-[N-[2-[5-(4-Isopropyl-3-trifluoromethylbenzyloxy)indolin-1-yl]-2-oxoethyl]-N-methylamino]propionic acid

(1) 5-(4-Isopropyl-3-trifluoromethyl-benzyloxy)indoline-1-carboxylic acid tert-butyl ester

[F534]

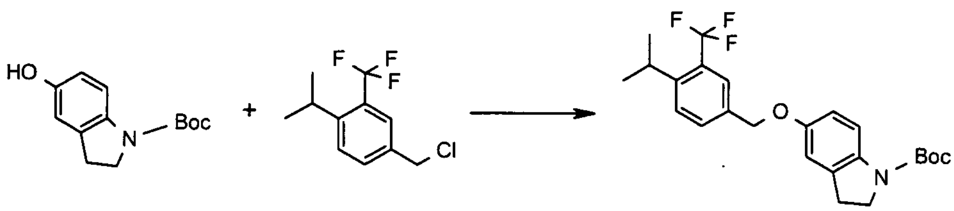

To a DMF solution (5.0 mL) of 5-hydroxyindoline-1-carboxylic acid tert-butyl ester (118 mg), 4-chloromethyl-1-isopropyl-2-trifluoromethylbenzene (142 mg) and potassium carbonate (104 mg) were added, and the mixture was stirred overnight at 70°C. The reaction mixture was left to stand to cool to room temperature and filtered. The filtrate was concentrated under reduced pressure. The residue was purified by flash column chromatography (Yamazen Hi-Flash column L), whereby the title compound (189 mg) was yielded.
$^1$H-NMR ($CDCl_3$) δ :
1.27(6H,d,J=6.9Hz),1.55(9H,s),3.06(2H,t,J=8.7Hz),3.32-3.39(1H,m),3.97(2H,s),5.00(2H,s),6.75-6.81(2H,m),7.35-7.76

(3H, m) .
MS (ESI)m/z:436 (M+H)+.

(2) 3-[N-[2-[5-(4-Isopropyl-3-trifluoromethylbenzyloxy)indolin-1-yl]-2-oxoethyl]-N-methylamino]propionic acid ethyl ester

[F535]

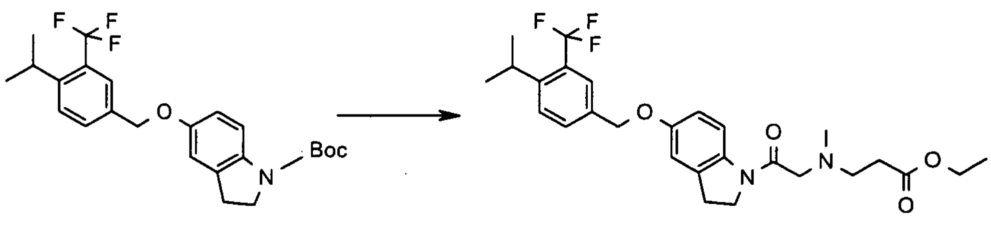

To 5-(4-isopropyl-3-trifluoromethylbenzyloxy)indoline-1-carboxylic acic tert-butyl ester (180 mg), 4N HCl/1,4-dioxane (10 mL) was added, and the mixture was stirred for 1 hour at room temperature. The reaction mixture was concentrated under reduced pressure. The residue was dissolved in DMF (10 mL), and 3-(N-carboxymethyl-N-methylamino)propionic acid ethyl ester (86.0 mg), DIEA (211 μL), HOBt (72.7 mg), and EDC-HCl (103 mg) were added to the solution. The obtained mixture was stirred overnight at room temperature. Saturated aqueous sodium bicarbonate solution was added to the reaction mixture, followed by extraction thrice, each with ethyl acetate. The extracts were combined and washed with saturated brine, followed by drying over sodium sulfate anhydrate. Insoluble matter was removed by filtration, and the filtrate was concentrated under reduced pressure. The residue was purified by NH-silica gel flash column chromatography (Yamazen Hi-Flash column L), and purified again by flash column chromatography (Yamazen Hi-Flash column L), whereby the title compound (91 mg) was yielded.
$^1$H-NMR ($CDCl_3$) δ:
1.21-1.28(9H,m),2.39(3H,s),2.51(2H,t,J=77.0Hz),2.88(2H,t,J=7.0Hz),3 .15(2H,t,J=8.3Hz),3.30-3.39(3H,m),4.04-4.17 (4H,m), 5.02 (2H, s), 6.78-6.83 (2H,m), 7.48-7.65 (3H, m), 8.15 (1H, d, J=8.6Hz).
MS (ESI) m/z : 507 (M+H)+.

(3) 3-[N-[2-[5-(4-Isopropyl-3-trifluoromethylbenzyloxy)indolin-1-yl]-2-oxoethyl]-N-methylamino]propionic acid

[F536]

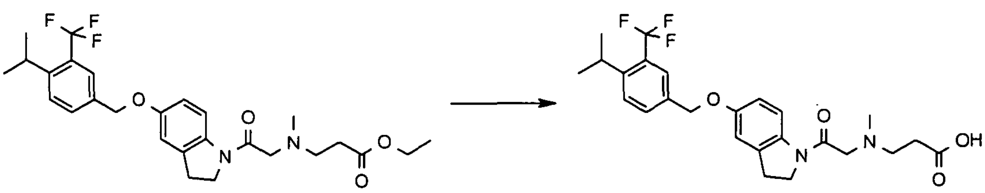

To a THF solution (5.0 mL) of 3- [N- [2- [5- (4-isopropyl-3-trifluoromethylbenzyloxy)indolin-1-yl]-2-oxoethyl]-N-methylamino]propionic acid ethyl ester (90.0 mg), methanol (0.53 mL) and 1N aqueous sodium hydroxide solution (0.530 mL) were added, and the mixture was stirred for 1.5 hours at room temperature. 1N Hydrochloric acid was added to the reaction mixture for neutralization, and the mixture was diluted with water. The resultant mixture was extracted eight times with 17% methanol/chloroform solution. The extracts were combined and dried over sodium sulfate anhydrate. Insoluble matter was removed by filtration, and the filtrate was concentrated under reduced pressure. The residue was

purified by flash column chromatography (Yamazen Hi-Flash column L), whereby the title compound (77 mg) was yielded.
$^1$H-NMR (DMSO-$d_6$) δ :
1.24(6H,d,J=6.6Hz),2.11-2.21(5H,m),2.60-2.64(2H,m),3.07-3.26(5H,m),4.16(2H,t,J=8.3Hz),5.10(2H,s),6.80(1H,d,J=8.8Hz),6 .93(1H,s),7.65-7.70(3H,m),7.97(1H,d,J=8.8Hz).
MS (ESI) m/z :479 (M+H)$^+$.

[Example 112]

2-Hydroxy-3-[N-[2-oxo-2-[5-[(4-phenyl-5-trifluoromethyl-2-thienyl)methoxy]indolin-1-yl]ethyl]amino]propionic acid

(1) 3-Amino-2-hydroxypropionic acid methyl ester hydrochloride

[F537]

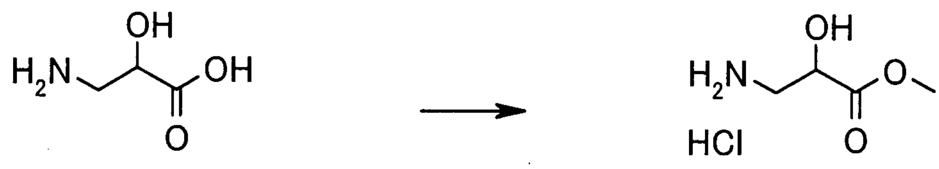

To a solution of (DL)-3-amino-2-hydroxypropionic acid (1.05 g) in methanol (60 mL), thionyl chloride (1.31 mL) was slowly added at room temperature, and the mixture was refluxed for 3 hours. The reaction mixture was concentrated under reduced pressure, whereby the title compound (1.50 g) was yielded.
$^1$H-NMR (DMSO-$d_6$) δ:
2.88(1H,br s), 3.06(1H,br s),3.66(3H,s),4.30-4.36(1H,m),8.19(1H,br s),8.25(2H,br s).

(2) 2-Hydroxy-3-[N-[2-oxo-2-[5-[(4-phenyl-5-trifluoromethyl-2-thienyl)methoxy]indolin-1-yl]ethyl]amino]propionic acid methyl ester

[F538]

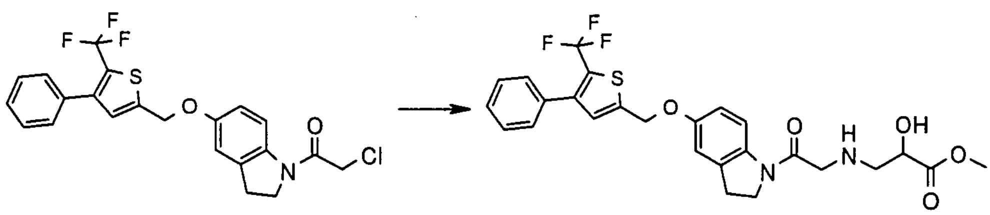

To a suspension of 1-(2-chloroacetyl)-5-[(4-phenyl-5-trifluoromethyl-2-thienyl)methoxy]indoline (346 mg) in acetonitrile (20 mL), 3-amino-2-hydroxypropionic acid methyl ester hydrochloride (298 mg) and DIEA (801 μL) were added at room temperature, and the mixture was stirred for 1 hour at 85°C. The reaction mixture was concentrated under reduced pressure. Saturated aqueous sodium hydrogencarbonate solution (15 mL) was added to the resultant mixture, followed by extraction with a 20% methanol/chloroform mixture (3 × 10 mL). The extracts were combined, and solvent was removed under reduced pressure. The residue was purified by silica gel column chromatography (Biotage 25M), whereby the title compound (199 mg) was yielded.
$^1$H-NMR ($CDCl_3$) δ:

2.06(1H,br s),3.07(2H,ddd,J=22.9,12.7,4.8Hz),3.20(2H,t,J=8.3Hz),3.55(2H, d,J=4.8Hz), 3.79 (3H,s), 3.99 (2H,t,J=8.3Hz),4.30 (1H, dd, J=5.7, 4.0 Hz), 5.20 (2H, s), 6.82 (1H, dd, J=8.5, 2.7Hz), 6.84 (1H,br s), 7.06(1H,s),7.39-7.43(5H,m),8.16(1H,d, J=8.5Hz).
MS (ESI) m/z : 535 $(M+H)^+$.

(3) 2-Hydroxy-3-[N-[2-oxo-2-[5-[(4-phenyl-5-trifluoromethyl-2-thienyl)methoxy]indolin-1-yl]ethyl]amino]propionic acid

[F539]

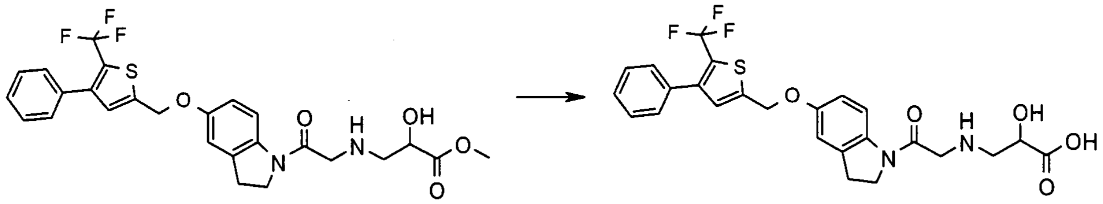

To a solution of 2-hydroxy-3-[N-[2-oxo-2-[5-[(4-phenyl-5-trifluoromethyl-2-thienyl)methoxy]indolin-1-yl]ethyl] amino]propionic acid methyl ester (199 mg) in THF (2.0 mL), methanol (1.0 mL) and 1N aqueous sodium hydroxide solution (1.0 mL) were added at room temperature, and the mixture was stirred for 4.5 hours at room temperature. Water (2 mL) was added to the reaction mixture, and the pH of the mixture was adjusted to 4 with 1N hydrochloric acid. A 20% methanol/chloroform mixture (5 mL) was added to the resultant mixture for phase separation. Further, the aqueous layer was extracted with a 20% methanol/chloroform mixture (2 × 5 mL). The extracts were combined, and solvent was removed under reduced pressure. Diethyl ether was added to the residue, and the precipitated solid was collected by filtration, whereby the title compound (125 mg%) was yielded.
$^1$H-NMR (DMSO-$d_6$) δ:
2.90(2H,t,J=6.6Hz),3.15(2H,t,J=8.4Hz),3.81(2H,s),3.92(1H,t,J= 6.6Hz),4.03(2H,t,J=8.4Hz),5.35(2H,s),6.89(1H,dd,J=8.5,2.4Hz), 7.00(1H,d,J=2.4Hz),7.36(1H,br s),7.41-7.51(5H,m), 7.99(1H,d,J=8.5Hz).
MS (ESI)m/z:521 $(M+H)^+$.
Anal. Calcd for $C_{25}H_{23}F_3N_2O_5S\cdot$
$0.5H_2O$:C,56.71;H,4.57;F,10.76;N,5.29;5,6.06.Found:C,56.87;H,4 .42;F,10.90;N,5.38;5,6.11.

[Example 113]

3-[N-Ethyl-N-[2-oxo-2-[5-[(4-phenyl-5-trifluoromethyl-2-thienyl)methoxy]indolin-1-yl]ethyl]amino]propionic acid

(1) 1-[2-(N-Ethylamino)acetyl]-5-[(4-phenyl-5-trifluoromethyl-2-thienyl)methoxy]indoline

[F540]

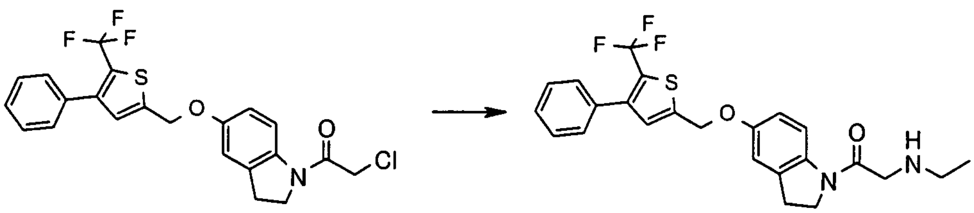

To a solution of 1-(2-chloroacetyl)-5-[(4-phenyl-5-trifluoromethyl-2-thienyl)methoxy]indoline (181 mg) in acetonitrile (5.0 mL), DIEA (348 μL) and 2N ethylamine/THF solution (0.5 mL) were added at room temperature, and the mixture was stirred for 2 hour at 90°C. The reaction mixture was concentrated under reduced pressure, and water (2.0 mL) was added to the residue, followed by extraction with a 10% methanol/chloroform mixture (4 × 3.0 mL). The extracts were combined and dried over sodium sulfate anhydrate. Insoluble matter was removed by filtration, and the filtrate was concentrated under reduced pressure. The residue was purified by silica gel column chromatography (Biotage 25M), whereby the title compound (45.4 mg) was yielded.
$^1$H-NMR ($CDCl_3$) δ :
1.17(3H,t,J=7.1Hz),2.71(2H,q,J=7.1Hz),3.20(2H,t,J=8.3Hz),3.49 (2H,s),4.02(2H,t,J=8.3Hz), 5.20(2H,s), 6.82(1H,d, J=9.0Hz),6.85( 1H,br s), 7.06(1H,s), 7.38-7.43(5H,m), 8.17(1H,d,J=9.0Hz).
MS (ESI) m/z :461 $(M+H)^+$.

(2) 3-[N-Ethyl-N-[2-oxo-2-[5-[(4-phenyl-5-trifluoromethyl-2-thienyl)methoxy]indolin-1-yl]ethyl]amino]propionic acid ethyl ester

[F541]

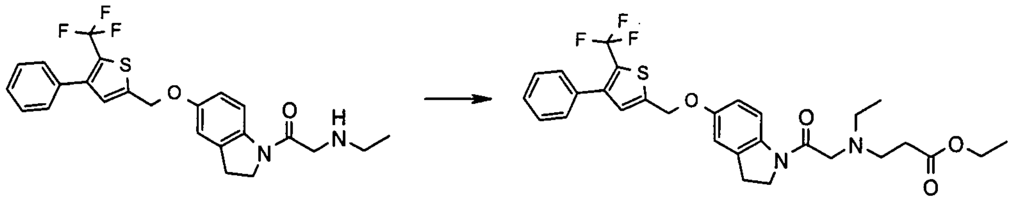

To a solution of 1- [2-(N-ethylamino)acetyl]-5-[(4-phenyl-5-trifluoromethyl-2-thienyl)methoxy]indoline (45.4 mg) in THF (0.3 mL), 1-butyl-3-methylimidazolium hexafluorophosphate (2.0 mL) and acrylic acid ethyl ester (26.7 μL) were added at room temperature, and the mixture was stirred for 4 days at 60°C. The reaction mixture was concentrated under reduced pressure, and to the resultant mixture, a 10% methanol/chloroform mixture (5.0 mL), saturated aqueous sodium hydrogencarbonate solution (2.0 mL), and water (5.0 mL) were added for phase separation. Further, the aqueous layer was extracted with a 10% methanol/chloroform mixture (3 × 3.0 mL). The extracts were combined and concentrated under reduced pressure. The residue was purified by silica gel column chromatography (Biotage 25M), whereby the title compound (46.3 mg) was yielded.
$^1$H-NMR ($CDCl_3$) δ :
1.07(3H,t,J=7.1Hz), 1.21(3H,t,J=7.2Hz),2.49(2H,t,J=7.0Hz),2.71 (2H,q,J=7.1Hz),2.98(2H,t,J=7.1Hz),3.15(2H,t,J=8.3Hz),3.39(2H, s),4.08(2H,q,J=7.2Hz),4.16(2H,t,J=8.3Hz),5.19(2H, s),6.80(1H,d d,J=8.8,2.2Hz),6.84(1H,br s),7.06(1H,s),7.42-7.37(5H,m),8.16(1H,d,J=8.8Hz).
MS (ESI) m/z : 561 $(M+H)^+$.

(3) 3-[N-Ethyl-N-[2-oxo-2-[5-[(4-phenyl-5-trifluoromethyl-2-thienyl)methoxy]indolin-1-yl]ethyl]amino]propionic acid

[F542]

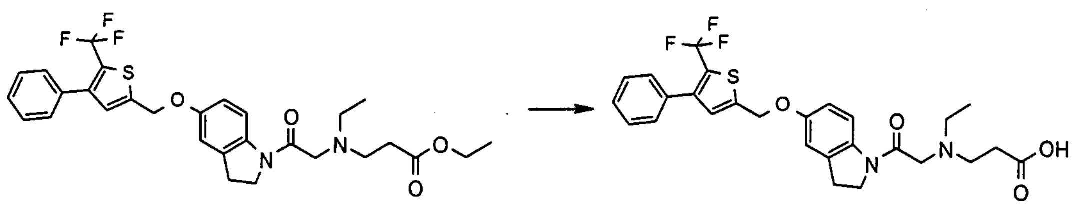

To a solution of 3-[N-ethyl-N-[2-oxo-2-[5-[(4-phenyl-5-trifluoromethyl-2-thienyl)methoxy]indolin-1-yl]ethyl]amino]propionic acid ethyl ester (46.3 mg) in THF (1.0 mL), methanol (0.5 mL) and 1N aqueous sodium hydroxide solution (0.5 mL) were added at room temperature, and the mixture was stirred for 1.5 hours at room temperature. Water (2 mL) was added to the reaction mixture, and the pH of the mixture was adjusted to 4 with 1N hydrochloric acid. A 20% methanol/chloroform mixture (5 mL) was added to the resultant mixture for phase separation. The aqueous layer was extracted with a 10% methanol/chloroform mixture (2 × 5 mL), and the extracts were combined and dried over sodium sulfate anhydrate, followed by concentration under reduced pressure. Dimethyl sulfoxide (2 mL) was added to the residue, and insoluble matter was removed. The filtrate was purified by high performance liquid chromatography (NOMURA Develosil Combi-RP5), and the target fraction was concentrated, whereby the title compound (30.5 mg) was yielded.
MS (ESI) m/z : 533 $(M+H)^+$.
Anal. Calcd for $C_{27}H_{27}F_3N_2O_4S$.
$1.5H_2O$:C,57.95;H,5.40;F,10.18;N,5.01;S,5.73.Found:C,57.86;H,5 .19;F,9.95;N,4.95;S,5.69.

[Example 114]

3-[N-Methyl-N-[2-oxo-2-[5-[(4-phenyl-5-trifluoromethyl-2-thienyl)methoxy]indolin-1-yl]ethyl]amino]acetic acid

(1) 3-[N-Methyl-N-[2-oxo-2-[5-[(4-phenyl-5-trifluoromethyl-2-thienyl)methoxy]indolin-1-yl]ethyl]amino]acetic acid ethyl ester

[F543]

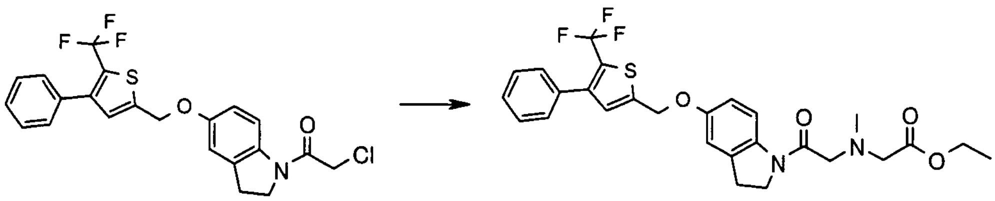

To a solution of 1-(2-chloroacetyl)-5-[(4-phenyl-5-trifluoromethyl-2-thienyl)methoxy]indoline (226 mg) in acetonitrile (10 mL), DIEA (279 μL) and N-methylglycine ethyl ester hydrochloride (115 mg) were added at room temperature, and the mixture was stirred for 1 hour at 80°C. The reaction mixture was concentrated under reduced pressure, and to the residue, water (5.0 mL), saturated aqueous sodium hydrogencaronate solution (3.0 mL), and a 10% methanol/chloroform mixture (10 mL) were added for phase separation. The aqueous layer was extracted with a 10% methanol/chloroform mixture (3 × 5.0 mL), and the extracts were combined and dried over sodium sulfate anhydrate. Insoluble matter was removed through filtration, and the filtrate was concentrated under reduced pressure. The residue was purified by silica gel column chromatography (Biotage 25M), whereby the title compound (218 mg) was yielded.
$^1$H-NMR ($CDCl_3$ ) δ :
1.27(3H,t,J=7.2Hz),2.57(2H,s),2.96(2H,s),3.18(2H,t,J=8.3Hz),3 .54(3H,d,J=10.7Hz),4.16(2H,t,J=8.3Hz),4.17(2H,q, J=7.1Hz),5.20 (2H,s),6.81(1H,dd,J=8.5,2.4Hz),6.84(1H,br s),7.06(1H,s),7.43-7.39(5H,m),8.17(1H,d,J=8.5Hz).
MS (ESI) m/z : 533 $(M+H)^+$.

(2) 3-[N-Methyl-N-[2-oxo-2-[5-[(4-phenyl-5-trifluoromethyl-2-thienyl)methoxy]indolin-1-yl]ethyl]amino]acetic acid

[F544]

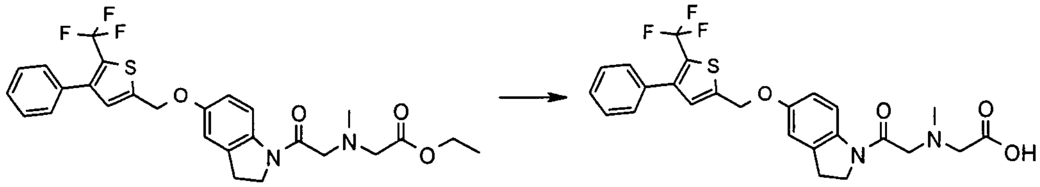

To a solution of 3-[N-methyl-N-[2-oxo-2-[5-[(4-phenyl-5-trifluoromethyl-2-thienyl)methoxy]indolin-1-yl]ethyl] amino]acetic acid ethyl ester (218 mg) in THF (1.0 mL), methanol (0.5 mL) and 1N aqueous sodium hydroxide solution (0.5 mL) were added at room temperature, and the mixture was stirred for 15 hours at room temperature. Water (2 mL) was added to the reaction mixture, and the pH of the mixture was adjusted to 4 with 1N hydrochloric acid. A 20% methanol/chloroform mixture (5 mL) was added to the resultant mixture for phase separation. The aqueous layer was extracted with a 20% methanol/chloroform mixture (2 $\times$ 5 mL). The extracts were combined and dried over sodium sulfate anhydrate, followed by removing solvent under reduced pressure. The residue was purified by silica gel column chromatography (Biotage 25S), whereby the title compound (141 mg) was yielded.
$^1$H-NMR(DMSO-$d_6$) $\delta$:
2.27(2H,br s),2.90-3.15(2H,m),3.32(3H,br s),3.40-3.51(3H,br m), 4.07 (2H,br s), 5.33 (2H, s), 6.86 (1H,br s),6.98(1H, s), 7.33(1H,s), 7.38-7.48(5H,m), 8.03(1H,d,J=8.5Hz).
MS(ESI)m/z:505(M+H)$^+$.

[Example 115]

3-[N-Isopropyl-N-[2-oxo-2-[5-[(4-phenyl-5-trifluoromethyl-2-thienyl)methoxy]indolin-1-yl]ethyl]amino]propionic acid

(1) 2-(N-Isopropylamino)acetyl-5-[(4-phenyl-5-trifluoromethyl-2-thienyl)methoxy]indoline

[F545]

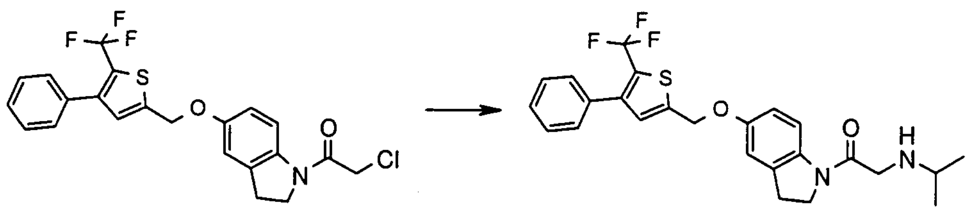

To a solution of 1-(2-chloroacetyl)-5-[(4-phenyl-5-trifluoromethyl-2-thienyl)methoxy]indoline (262 mg) in acetonitrile (5.0 mL), DIEA (606 μL) and isopropylamine (148 μL) were added at room temperature, and the mixture was stirred 2 hours at 85°C. The reaction mixture was concentrated under reduced pressure, and water (2.0 mL), saturated aqueous sodium hydrogencarbonate (1.5 mL), and a 10% methanol/chloroform mixture (3.0 mL) were added to the residue for phase separation. The aqueous layer was extracted with a 10% methanol/chloroform mixture (3 $\times$ 3.0 mL). The extracts were combind and dried over sodium sulfate anhydrate, and solvent was removed by filtration. The residue was purified by silica gel column chromatography (Biotage 25M), whereby the title compound (173 mg, 63%) was yielded.
$^1$H-NMR ($CDCl_3$) $\delta$ :
1.11(6H,d,J=6.1Hz),2.85(1H,dq,J=6.1,6.1Hz),3.20(2H,t,J=8.4Hz) ,3.49(2H,s),4.03(2H,t,J=8.4Hz), 5.20(2H,s),6.82(1H, dd,J=8.5,2. 0Hz), 6.84(1H,br s), 7.06(1H,s),7.43-7.38 (5H, m), 8.17 (1H, d, J=8.5Hz).
MS (ESI) m/z : 475 (M+H)$^+$.

(2) 3-[N-Isopropyl-N-[2-oxo-2-[5-[(4-phenyl-5-trifluoromethyl-2-thienyl)methoxy]indolin-1-yl]ethyl]amino]propionic acid ethyl ester

[F546]

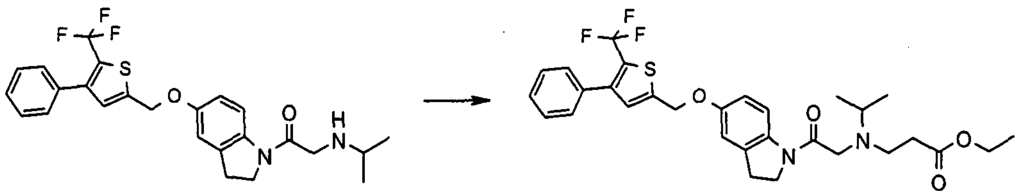

To a solution of 1- [2-(N-isopropylamino)acetyl]-5-[(4-phenyl-5-trifluoromethyl-2-thienyl)methoxy]indoline (173 mg) in THF (0.5 mL), 1-butyl-3-methylimidazolium hexafluorophosphate (0.75 mL) and acrylic acid ethyl ester (0.750 mL) were added at room temperature, and the mixture was stirred for 4 days at 60°C. The reaction mixture was concentrated under reduced pressure, and a 10% methanol/chloroform mixture (5.0 mL), saturated aqueous sodium hydrogencarbonate solution (2.0 mL), and water (5.0 mL) were added thereto for phase separation. The aqueous layer was extracted with a 10% methanol/chloroform mixture (3 × 3.0 mL). The extracts were combined and dried under reduced pressure. The residue was concentrated under reduced pressure, and purified by silica gel column chromatography (Biotage 25M), whereby the title compound (160 mg) was yielded.
$^1$H-NMR ($CDCl_3$) δ :
1.05(6H,d,J=6.6Hz),1.19(3H,t,J=7.2Hz),2.46(2H,t,J=7.0Hz),2.89 (2H,t,J=7.0Hz),3.05(1H,dq,J=6.6,6.6Hz),3.15(2H,t,J=8.4Hz),3.3 8(2H,s),4.05(2H,q,J=7.2Hz),4.23(2H,t,J=8.4Hz),5.20(2H,s),6.81 (1H,dd,J=8.8,2.2Hz),6.85(1H,d,J=2.2Hz),7.06(1H,s),7.43-7.38(5H,m),8.17(1H,d,J=8.8Hz).

(3) 3-[N-Isopropyl-N-[2-oxo-2-[5-[(4-phenyl-5-trifluoromethyl-2-thienyl)methoxy]indolin-1-yl]ethyl]amino]propionic acid

[F547]

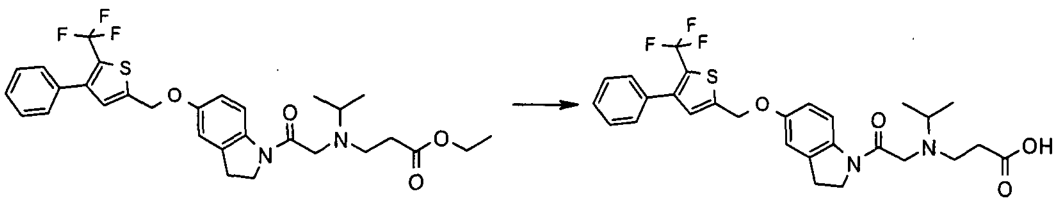

To a solution of 3-[N-isopropyl-N-[2-oxo-2-[5-[(4-phenyl-5-trifluoromethyl-2-thienyl)methoxy]indolin-1-yl]ethyl] amino]propionic acid ethyl ester (160 mg) in THF (2.0 mL), methanol (1.0 mL) and 1N aqueous sodium hydroxide solution (1.0 mL) were added at room temperature, and the mixture was stirred for 15 hours at room temeprature. Water (2 mL) was added to the reaction mixture, and the pH of the resultant mixture was adjusted to 4 with 1N hydrochloric acid, followed by addition of a 20% methanol/chloroform mixture (5 mL) for phase separation. The aqueous layer was extracted with a 10% methanol/chloroform mixture (2 × 5 mL). The extracts were combined and dried over sodium sulfate anhydrate, followed by removing solvent under reduced pressure. The residue was purified by silica gel column chromatography (Biotage 25S), whereby the title compound (130 mg) was yielded.
MS(ESI)m/z:547(M+H)$^+$.

[Example 116]

1-[2-Oxo-2-[5-[(4-phenyl-5-trifluoromethyl-2-thienyl)methoxy]indolin-1-yl]ethyl]piperidine-4-carboxylic acid

(1) 1-[2-Oxo-2-[5-[(4-phenyl-5-trifluoromethyl-2-thienyl)methoxy]indolin-1-yl]ethyl]piperidine-4-carboxylic acid ethyl ester

[F548]

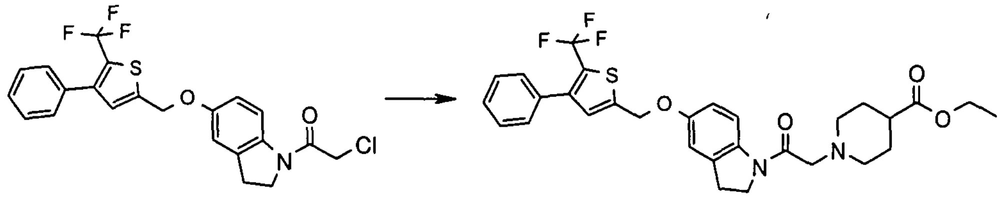

To a solution of 1-(2-chloroacetyl)-5-[(4-phenyl-5-trifluoromethyl-2-thienyl)methoxy]indoline (226 mg) in acetonitrile (3.0 mL), DIEA (235 μL) and 4-piperidine-carboxylic acid ethyl ester (100 mg) were added at room temperature, and the mixture was stirred for 1 hour at 80°C. The reaction mixture was concentrated under reduced pressure, and, to the residue, water (5.0 mL), saturated aqueous sodium bicarbonate solution (3.0 mL), and a 10% methanol/chloroform mixture (10 mL) were added for phase separation. The aqueous layer was extracted with a 10% methanol/chloroform mixture (3 × 5.0 mL). The extracts were combined and dried over sodium sulfate anhydrate. Insoluble matter was removed by filtration, and the filtrate was concentrated under reduced pressure. The residue was purified by silica gel column chromatography (Biotage 25M), whereby the title compound (145 mg) was yielded.
$^1$H-NMR ($CDCl_3$) δ:
1.25(3H,t,J=7.2Hz),1.76-1.88(2H,m),1.89-1.97(2H,m),2.24-2.37(3H,m),2.97(2H,d,J=10.7Hz),3.17(2H,t,J=8.3Hz),3.26 (2H,s), 4.14(2H,q,J=7.2Hz),4.20(2H,t,J=8.4Hz),5.20(2H,s),6.81(1H,dd,J =8.5,2.2Hz),6.85(1H,br s),7.06(1H,s), 7.39-7.43 (5H,m),8.17 (1H,d,J=8.5Hz).
MS(ESI)m/z:573(M+H)$^+$.

(2) 1-[2-Oxo-2-[5-[(4-phenyl-5-trifluoromethyl-2-thienyl)methoxy]indolin-1-yl]ethyl]piperidine-4-carboxylic acid

[F549]

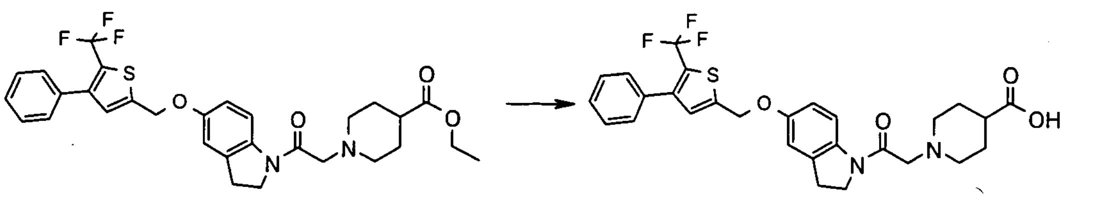

To a solution of 1-[2-oxo-2-[5-[(4-phenyl-5-trifluoromethyl-2-thienyl)methoxy]indolin-1-yl]ethyl]piperidine-4-carboxylic acid ethyl ester (145 mg) in THF (2.0 mL), methanol (1.0 mL) and 1N aqueous sodium hydroxide solution (1.0 mL) were added at room temperature, and the mixture was stirred for 15 hours at room temperature. Water (2 mL) was added to the reaction mixture, and the pH of the resultant mixture was adjusted to 4 with 1N hydrochloric acid, followed by addition of a 20% methanol/chloroform mixture (5 mL) for phase separation. The aqueous layer was extracted with a 20% methanol/chloroform mixture (2 × 5 mL).
The extracts were combined and dried over sodium sulfate anhydrate, followed by concentration under reduced pressure.

The residue was purified by silica gel column chromatography (Biotage 25S), and the target fraction was concentrated under reduced pressure. Diethyl ether (1.0 mL) and hexane (2.0 mL) were added to the residue, and the precipitated solid was collected by filtration and dried, whereby the title compound (64.5 mg) was yielded.
$^1$H-NMR (DMSO-$d_6$) δ:
1.53(2H,dtd,J=11.0,11.0,3.4Hz),1.75(2H,dd,J=13.4,2.7Hz),2.03-2.16 (3H,m), 2.81 (2H, d, J=11.0Hz), 3.10 (2H, t, J=8.4Hz), 3.18 (2H, s), 4.16(2H,t,J=8.4Hz),5.34(2H,s),6.84(1H,dd,J=8.8,2.2Hz),6.97(1H ,d,J=2.2Hz),7.35(1H,s), 7.50-7.41(5H,m),7.98(1H,d,J=8.8Hz).
MS (ESI) m/z : 545 $(M+H)^+$.
Anal. Calcd for $C_{28}H_{27}F_3N_2O_4S\cdot 1.75H_2O$,0.1HCl:C,58.01;H,5.32;Cl,0.61;F,9.83;N,4.83;S,5.53.Fo und:C,57.93;H,5.16; Cl,0.83;F,9.66;N,4.80;5,5.30.

[Example 117]

3-[N-Cyclopropyl-N-[2-oxo-2-[5-[(4-phenyl-5-trifluoromethyl-2-thienyl)methoxy]indolin-1-yl]ethyl]amino]propionic acid

(1) 1-[2-(N-Cyclopropylamino)acetyl]-5-[(4-phenyl-5-trifluoromethyl-2-thienyl)methoxy]indoline

[F550]

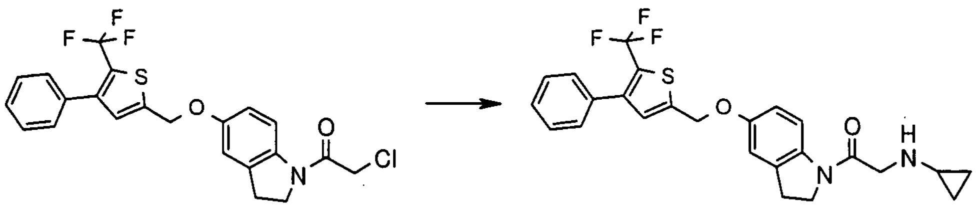

To a solution of 1-(2-chloroacetyl)-5-[(4-phenyl-5-trifluoromethyl-2-thienyl)methoxy]indoline (262 mg) in acetonitrile (5.0 mL), DIEA (606 μL) and cyclopropylamine (148 μL) were added at room temperature, and the mixture was stirred for 2 hours at 85°C. The reaction mixture was concentrated under reduced pressure. To the residue, water (2.0 mL), saturated aqueous sodium hydrogencarbonate solution (1.5 mL), and a 10% methanol/chloroform mixture (3.0 mL) were added for phase separation. The aqueous layer was extracted with a 10% methanol/chloroform mixture (3 × 3.0 mL). The extracts were combined and dried with sodium sulfate anhydrate. Insoluble matter was removed by filtration, and the filtrate was concentrated under reduced pressure. The residue was purified by silica gel column chromatography (Biotage 25M), whereby the title compound (121 mg) was yielded.
$^1$H-NMR ($CDCl_3$) δ:
0.38-0.48(4H,m),2.26-2.32(1H,m),3.20(2H,t,J=8.4Hz),3.55(2H,s),4.04(2H,t,J=8.4Hz),5

- 20(2H,s),6.82(1H,dd,J=8.5,2.0Hz),6.85(1H,br s),7.06(1H,s),7.43-7.39(5H,m),8.18(1H,d,J=8.5Hz).
  MS (ESI) m/z :473 $(M+H)^+$.

(2) 3-[N-Cyclopropyl-N-[2-oxo-2-[5-[(4-phenyl-5-trifluoromethyl-2-thienyl)methoxy]indolin-1-yl]ethyl]amino]propionic acid ethyl ester

[F551]

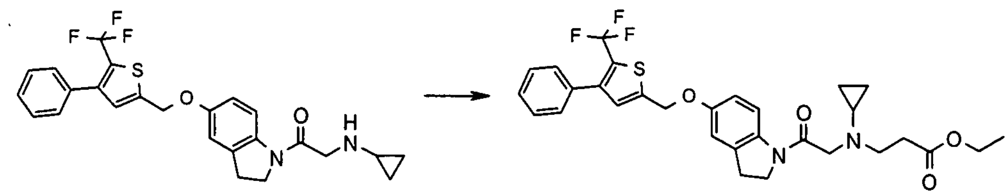

To a solution of 1-[2-(N-cyclopropylamino)acetyl]-5-[(4-phenyl-5-trifluoromethyl-2-thienyl)methoxy]indoline (121 mg) in THF (0.5 mL), 1-butyl-3-methylimidazolium hexafluorophosphate (0.75 mL) and acrylic acid ethyl ester (0.750 mL) were added at room temperature, and the mixture was stirred for 4 days at 60°C. The reaction mixture was concentrated under reduced pressure, and a 10% methanol/chloroform mixture (5.0 mL), saturated aqueous sodium hydrogencarbonate solution (2.0 mL), and water (5.0 mL) were added thereto for phase separation. The aqueous layer was extracted with a 10% methanol/chloroform mixture (3 × 3.0 mL), and the extracts were combined and concentrated under reduced pressure. The residue was purified by silica gel column chromatography (Biotage 25M), whereby the title compound (84.8 mg) was yielded.

$^1$H-NMR ($CDCl_3$) δ :

0.40-0.52(4H,m),1.22(3H,t,J=7.1Hz),2.19-2.25(1H,br m),2.56(2H,t,J=7.1Hz),3.14(2H,t,J=8.0Hz),3.18(2H,t,J=7.3Hz), 3 .52(2H,s),4.09(4H,q,J=7.1Hz),5.19(2H,s),6.81(1H,dd,J=8.8,2.4H z),6.84(1H,br s),7.06(1H,s),7.43-7.37(5H,m),8.17 (1H,d,J=8.8Hz).

MS (ESI)m/z:573 (M+H)$^+$.

(3) 3-[N-Cyclopropyl-N-[2-oxo-2-[5-[(4-phenyl-5-trifluoromethyl-2-thienyl)methoxy]indolin-1-yl] ethyl] amino] propionic acid

[F552]

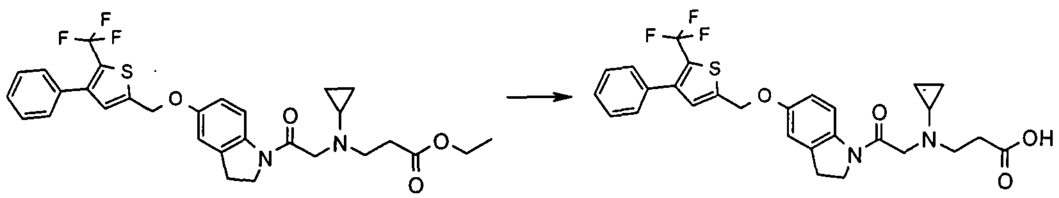

To a solution of 3-[N-cyclopropyl-N-[2-oxo-2-[5-[(4-phenyl-5-trifluoromethyl-2-thienyl)methoxy]indolin-1-yl] ethyl]amino]propionic acid ethyl ester (84.8 mg) in THF (1.4 mL), methanol (0.7 mL) and 1N aqueous sodium hydroxide solution (0.7 mL) were added at room temperature, and the mixture was stirred for 15 hours at room temperature. Water (2 mL) was added to the reaction mixture, and the pH of the resultant mixture was adjusted to 4 with 1N hydrochloric acid. A 20% methanol/chloroform mixture (5 mL) was added to the pH-adjusted mixture for phase separation. The aqueous layer was extracted with a 20% methanol/chloroform mixture (2 × 5 mL), and the extracts were combined and dried over sodium sulfate anhydrate, followed by concentration under reduced pressure. The residue was purified by silica gel column chromatography (Biotage 25S), whereby the title compound (58.8 mg) was yielded.

MS (ESI) m/z : 545 (M+H)$^+$.

[Example 118]

1-[2-Oxo-2-[5-[(4-phenyl-5-trifluoromethyl-2-thienyl)methoxy]indolin-1-yl]ethyl]piperidine-(3S)-carboxylic acid

(1) 1-(tert-Butoxycarbonyl)piperidine-(3S)-carboxylic acid methyl ester

[F553]

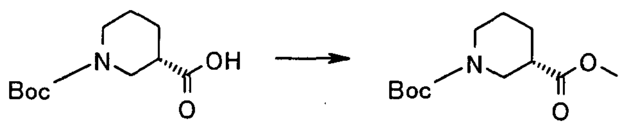

To a solution of 1-(tert-butoxycarbonylpiperidine)-(3S)-carboxylic acid (2.75 g) in DMF (20 mL), potassium carbonate (2.49 g) and methyl iodide (1.12 mL) were added at room temperature, and the mixture was stirred for 3 hours at room temperature. The reaction mixture was concentrated under reduced pressure, and to the resultant residue, water (15.0 mL) and ethyl acetate (20 mL) were added for phase separation. The aqueous layer was extracted with ethyl acetate (3 × 10.0 mL), and the extracts were combined and dried with sodium sulfate anhydrate. Insoluble matter was removed by filtration, and the filtrate was concentrated under reduced pressure. The residue was purified by silica gel column chromatography (Biotage 40M), whereby the title compound (2.88 g) was yielded.
$^1$H-NMR ($CDCl_3$) δ:
1.46 (9H, s), 1.56-1.74 (3H, m), 2.00-2.08 (1H, m), 2.45 (1H, tdd, J=10.2,3.9,3.9Hz), 2.81 (1H, ddd, J=11.5,3 . 2,13.7Hz), 2.88-3.10(1H,br m),3.69(3H,s),3.91(1H,d,J=13.7Hz),4.29-4.00(1H,br m).

(2) (3S)-Piperidinecarboxylic acid methyl ester

[F554]

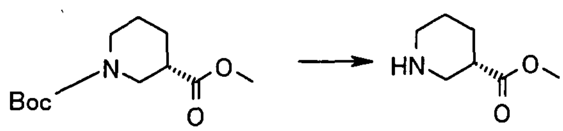

To 1-(tert-butoxycarbonyl)piperidine-(3S)-carboxylic acid methyl ester (2.88 g), 4N HCl/1,4-dioxane (5.0 mL) was added at room temperature, and the mixture was stirred for 16 hours. The resultant mixture was concentrated under reduced pressure. To the residue, water (15.0 mL) was added, and the pH of the resultant mixture was adjusted to 9 with saturated aqueous sodium hydrogencarbonate solution. The resultant mixture was extracted with a 10% methanol/chloroform mixture (3 × 15.0 mL), and the extracts were combined and dried over sodium sulfate anhydrate, and further dried under reduced pressure, whereby the title compound (577 mg) was yielded.
$^1$H-NMR ($CDCl_3$) δ:
1.40-1.52(1H,m),1.63-1.72(2H,m),1.95-2.02(1H,m),2.46(1H,tt,J=9.8,3.9Hz),2.64(1H,ddd,J=9.8,12.4,3.2 Hz),2.82(1H,dd,J=12.4,9.3Hz),2.93(1H,dt,J=12.4,3.9Hz),3.16(1H ,dd,J=12.4,3.2Hz),3.68(3H,s).

(3) 1-[2-Oxo-2-[5-[(4-phenyl-5-trifluoromethyl-2-thienyl)methoxy]indolin-1-yl]ethyl]piperidine-(3S)-carboxylic acid methyl ester

[F555]

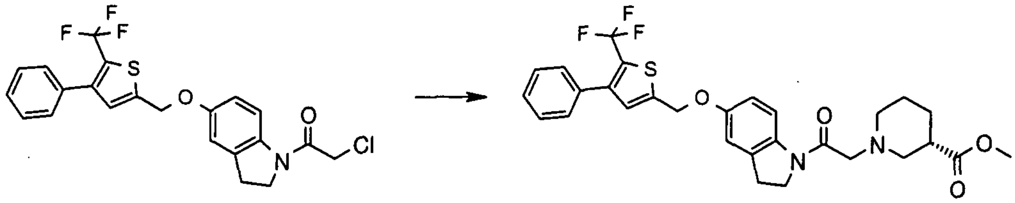

To a solution of 1-(2-chloroacetyl)-5-[(4-phenyl-5-trifluoromethyl-2-thienyl)methoxy]indoline (360 mg) in acetonitrile (3.0 mL), DIEA (347 μL) and (3S)-piperidinecarboxylic acid methyl ester (143 mg) were added at room temperature, and the mixture was stirred for 30 minutes at 80°C. The reaction mixture was concentrated under reduce pressure, and to the resultant residue, water (5.0 mL), saturated aqueous sodium hydrogencarbonate solution (3.0 mL), and a 10% methanol/chloroform mixture (10 mL) were added for phase separation. The aqueous layer was extracted with a 10% methanol/chloroform mixture (3 × 5.0 mL), and the extracts were combined and dried over sodium sulfate anhydrate, followed by concentration under reduced pressure. The residue was purified by silica gel column chromatography (Biotage 25M), whereby the title compound (322 mg) was yielded.
$^1$H-NMR ($CDCl_3$) δ:
1.47-1.69(2H,m),1.70-1.78(1H,m),1.89-1.97(1H,br m),2.25(1H,td,J=9.8,2.4Hz),2.44(1H,t,J=9.8Hz),2.65(1H,ddd,J=9 . 8,13.7,3.9Hz),2.79-2.86(1H,m),3.03-3.08 (1H,m), 3.17 (2H, t, J=8.3Hz), 3.25 (2H, s), 3.64 (3H, s), 4.18 (2H, d t, J=3.9,8.3Hz),5.20(2H,s),6.82(1H,d,J=8.8Hz),6.85(1H,br s),7.06(1H,s),7.43-7.39(5H,m),8.18(1H,d,J=8.8Hz).
MS(ESI)m/z:559(M+H)$^+$.

(4) 1- [2-Oxo-2-[5-[(4-phenyl-5-trifluoromethyl-2-thienyl)methoxy]indolin-1-yl]ethyl]piperidine-(3S)-carboxylic acid

[F556]

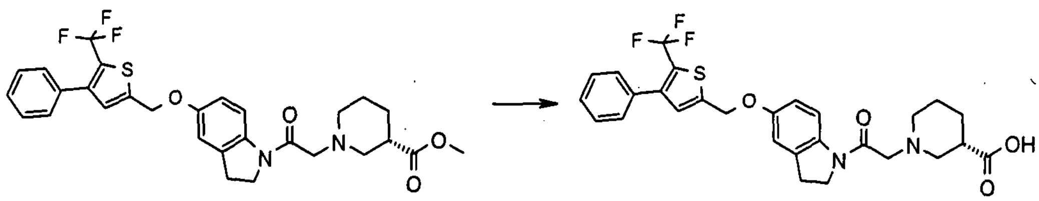

To a solution of 1-[2-oxo-2-[5-[(4-phenyl-5-trifluoromethyl-2-thienyl)methoxy]indolin-1-yl]ethyl]piperidine-(3S)-carboxylic acid methyl ester (322 mg) in THF (2.0 mL), methanol (1.0 mL) and 1N aqueous sodium hydroxide solution (1.0 mL) were added at room temperature, and the mixture was stirred for 15 hours at room temperature. Water (2 mL) was added to the reaction mixture, and the pH of the resultant mixture was adjusted to 4 with 1N hydrochloric acid, followed by adding thereto a 20% methanol/chloroform mixture (5 mL) for phase separation. The aqueous layer was extracted with a 20% methanol/chloroform mixture (2 × 5 mL), and a combined extract was dried over sodium sulfate anhydrate, followed by concentration under reduced pressure. The residue was purified by silica gel column chromatography (Biotage 25S), whereby the title compound (195 mg) was yielded.
$^1$H-NMR (DMSO-$d_6$) δ:
1.26-1.47(2H,br m),1.60(1H,br s),1.75(1H,br s),2.15(1H,br s),2.32(2H,br s),2.65(1H,br s),2.88(1H,br s),3.08(2H,t, J=7.8Hz),3.21(2H,br s),4.14(2H,t,J=7.8Hz),5.33(2H,s),6.84(1H,d,J=8.5Hz),6.96(1H,s ),7.34(1H,s),7.49-7.40(5H,m), 7.98(1H,d,J=8.5Hz).
MS (ESI) m/z : 545 (M+H)$^+$.

[Example 119]

1-[2-Oxo-2-[5-[(4-phenyl-5-trifluoromethyl-2-thienyl)methoxy]indolin-1-yl]ethyl]piperidine-(3R)-carboxylic acid

(1) 1-(tert-Butoxycarbonyl)piperidine-(3R)-carboxylic acid methyl ester

[F557]

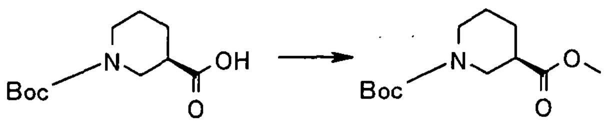

To a solution of 1-(tert-butoxycarbonyl)piperidine-(3R)-carboxylic acid (2.75 g) in DMF (20 mL), potassium carbonate (2.49 g) and methyl iodide (1.12 mL) were added at room temperature, and the mixture was stirred for 3 hours at room temperature. The resultant mixture was concentrated under reduced pressure. To the residue, water (15.0 mL) and ethyl acetate (20 mL) were added for phase separation. The aqueous layer was extracted with ethyl acetate (3 × 10.0 mL), and the extracts were combined and dried over sodium sulfate anhydrate. Insoluble matter was removed by filtration, and the filtrate was concentrated under reduced pressure. The residue was purified by silica gel column chromatography (Biotage 40M), whereby the title compound (2.28 g) was yielded.
$^1$H-NMR ($CDCl_3$) δ :
1.46(9H,s),1.56-1.75(3H,m),2.00-2.07(1H,m),2.45(1H,tdd,J=10.5,3.9,3.9Hz),2.78-2.85(1H,m),2.98(1H,br s),3.69(3H, s),3.91(1H,d,J=13.2Hz),4.11(1H,br s).

(2) (3R)-Piperidinecarboxylic acid methyl ester

[F558]

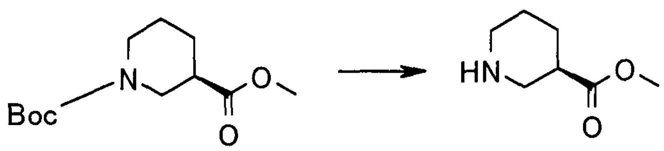

To 1-(tert-butoxycarbonyl)piperidine-(3R)-carboxylic acid methyl ester (2.28 g), 4N HCl/1,4-dioxane (5.0 mL) was added at room temperature, and the mixture was stirred for 16 hours. The resultant mixture was concentrated under reduced pressure. To the residue, water (15.0 mL) was added, and the pH of the.resultant mixture was adjusted to 9 with saturated aqueous sodium hydrogencarbonate solution. The resultant mixture was extracted with a 10% methanol/ chloroform mixture (3 × 15.0 mL), and the extracts were combined and dried over sodium sulfate anhydrate. Insoluble matter was removed by filtration, the filtrate was concentrated under reduced pressure, whereby the title compound (384 mg) was yielded.
$^1$H-NMR ($CDCl_3$) δ:
1.46-1.53(1H,m),1.62-1.72(2H,m),1.96-2.03(1H,br m),2.48(1H,tt,J=9.3,3.9Hz),2.65(1H,ddd,J=10.7,12.7,3.2Hz),2.8 2 (1H,dd,J=12.4,9.3Hz),2.95(1H,dt,J=12.7,3.7Hz),3.18(1H,dd,J=1 2.4,3.2Hz),3.68(3H, s).

(3) 1- [2-Oxo-2- [5- [(4-phenyl-5-trifluoromethyl-2-thienyl)methoxy]indolin-1-yl]ethyl]piperidine-(3R)-carboxylic acid methyl ester

[F559]

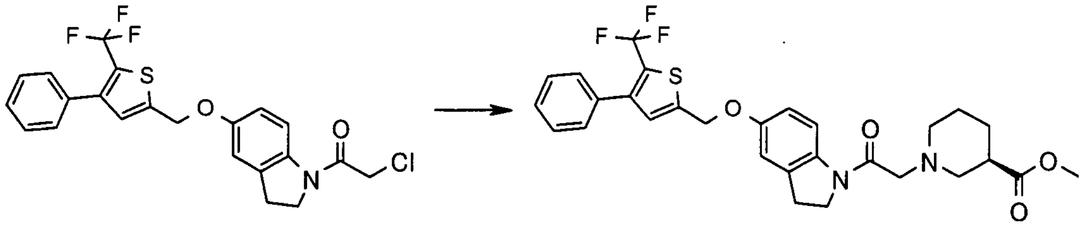

To a solution of 1-(2-chloroacetyl)-5-[(4-phenyl-5-trifluoromethyl-2-thienyl)methoxy]indoline (360 mg) in acetonitrile (3.0 mL), DIEA (347 μL) and (3R)-piperidinecarboxylic acid methyl ester (143 mg) were added at room temperature. The reaction mixture was stirred for 30 minutes at 80°C, and concentrated under reduced pressure. To the resultant residue, water (5.0 mL), saturated aqueous sodium hydrogencarbonate solution (3.0 mL), and a 10% methanol/chloroform mixture (10 mL) were added for phase separation. The aqueous layer was extracted with a 10% methanol/chloroform mixture (3 × 5.0 mL), and the extracts were combined and dried over sodium sulfate anhydrate. Insoluble matter was removed by filtration, and the filtrate was concentrated under reduced pressure. The residue was purified by silica gel column chromatography (Biotage 25M), whereby the title compound (323 mg) was yielded.
$^1$H-NMR ($CDCl_3$) δ:
1.50-1.68(2H,m),1.74(1H,dt,J=13.4,3.9Hz),1.88-1.96(1H,m),2.25(1H,dt,J=2.0,10.0Hz),2.44(1H,t,J=10.0Hz),2.62-2.69 (1H,m),2.80-2.86(1H,m),3.03-3.08(1H,m),3.17(2H,t,J=8.4Hz),3.25(2H,s),3.64(3H,s),4.18(2H,d t,J=3.4,8.4Hz),5.20(2H, s),6.81(1H,dd,J=8.5,2.4Hz),6.85(1H,br s),7.06(1H,s),7.39-7.43(5H,m),8.18(1H,d,J=8.5Hz).
MS (ESI) m/z : 559 $(M+H)^+$.

(4) 1-[2-Oxo-2-[5-[(4-phenyl-5-trifluoromethyl-2-thienyl)methoxy]indolin-1-yl]ethyl]piperidine-(3R)-carboxylic acid

[F560]

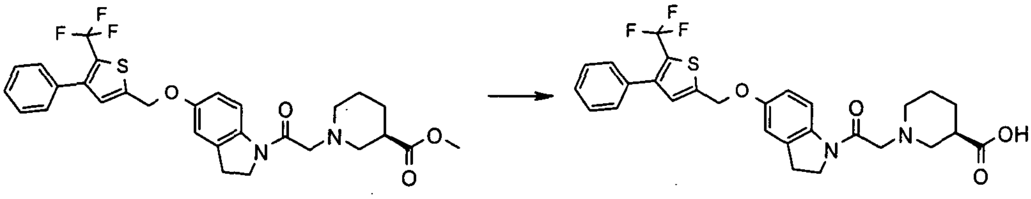

To a solution of 1-[2-oxo-2-[5-[(4-phenyl-5-trifluoromethyl-2-thienyl)methoxy]indolin-1-yl]ethyl]piperidine-(3R)-carboxylic acid methyl ester (323 mg) in THF (2.0 mL), methanol (1.0 mL) and 1N sodium hydroxide solution (1.0 mL) were added at room temperature, and the mixture was stirred for 15 hours at room temperature. Water (2 mL) was added to the reaction mixture, and the pH of the resultant mixture was adjusted to 4 with 1N hydrochloric acid, followed by addition of a 20% methanol/chloroform mixture (5 mL) for phase separation. The aqueous layer was extracted with a 20% methanol/chloroform mixture (2 × 5 mL), and the extracts were combined and dried with sodium sulfate anhydrate, followed by concentration under reduced pressure. The residue was purified by silica gel column chromatography (Biotage 25S), whereby the title compound (155 mg) was yielded.
$^1$H-NMR (DMSO-$d_6$) δ:
1.25-1.48(2H,br m),1.60(1H,br s),1.75(1H,br s),2.10-2.38(3H,br m),2.65(1H,br s),2.88(1H,br s),3.09(2H,t,J=7.3Hz),3.22 (2H,br s),4.14(2H,t,J=7.3Hz),5.33(2H,s),6.84(1H,d,J=8.5Hz),6.96(1H,s ),7.34(1H,s),7.49-7.39(5H,m),7.98(1H,d, J=8.5Hz).
MS (ESI) m/z : 545 $(M+H)^+$.

[Example 120]

1-[2-Oxo-2-[5-[(4-phenyl-5-trifluoromethyl-2-thienyl)methoxy]indolin-1-yl]ethyl]pyrrolidine-3-carboxylic acid

(1) 3-Pyrrolidinecarboxylic acid ethyl ester hydrochloride

[F561]

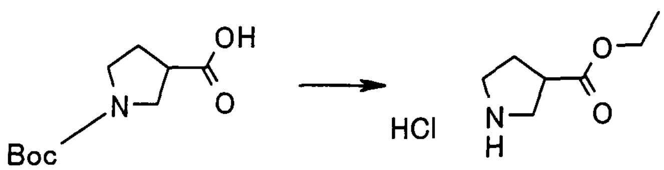

To a solution of 1-(tert-butoxycarbonyl)-3-pyrrolidinecarboxylic acid ethyl ester (1.61 g) in ethanol (20 mL), thionyl chloride (1.09 mL) was slowly added at room temperature. The reaction mixture was refluxed for 2 hours, and the resultant mixture was concentrated under reduce pressure, whereby the title compound (1.30 g) was yielded.
$^1$H-NMR (DMSO-$d_6$) δ :
1.19 (3H, t, J=7.2Hz), 1.95-2.05 (2H, m), 2.15 (2H, dtt, J=14.2,7.1,7.1Hz), 3.19-3.29 (2H, m), 3.38-3.31 (1H, m), 4.10 (2H, q, J=7.2Hz).

(2) 1-[2-Oxo-2-[5-[(4-phenyl-5-trifluoromethyl-2-thienyl)methoxy]indolin-1-yl]ethyl]pyrrolidine-3-carboxylic acid ethyl ester

[F562]

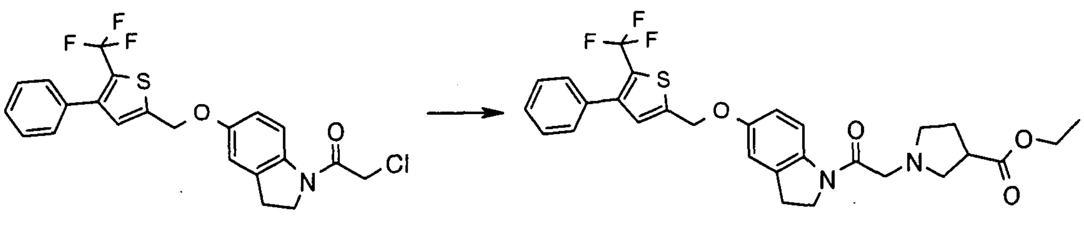

To a solution of 1-(2-chloroacetyl)-5-[(4-phenyl-5-trifluoromethyl-2-thienyl)methoxy]indoline (350 mg) in acetonitrile (8.0 mL), DIEA (539 μL) and 3-pyrrolidinecarboxylic acid ethyl ester hydrochloride (209 mg) were added at room temperature, followed by stirring for 1 hour at 80°C. The reaction mixture was concentrated under reduced pressure. To the residue, water (5.0 mL), saturated aqueous sodium hydrogencarbonate solution (3.0 mL), and a 10% methanol/chloroform mixture (10 mL) were added for phase separation. The aqueous layer was extracted with a 10% methanol/chloroform mixture (3 × 5.0 mL), and the extracts were combined and dried over sodium sulfate anhydrate. Insoluble matter was removed by filtration, and the filtrate was concentrated under reduced pressure. The residue was purified by silica gel column chromatography (Biotage 40M), whereby the title compound (313 mg) was yielded.
$^1$H-NMR ($CDCl_3$) δ:
1.25 (3H, t, J=7.2Hz), 2.13 (2H, dt, J=8.5,7.1Hz), 2.66 (1H, dt, J=7.6,7 . 6Hz), 2.79 (1H, dd, J=5.9,8.3Hz), 2.90 (1H, dt, J=8.5,7.1Hz),3.04-3.14(2H,m),3.17(2H,t,J=7.8Hz),3.40(2H,d,J=1.7Hz),4.14(4H,q,J= 7.2Hz),5.20(2H,s),6.81(1H,d, J=8.8Hz),6.84(1H,s),7.06(1H,s),7. 44-7.39(5H,m),8.17(1H,d,J=8.8Hz).
MS(ESI)m/z:559(M+H)$^+$.

(3) 1-[2-Oxo-2-[5-[(4-phenyl-5-trifluoromethyl-2-thienyl)methoxy]indolin-1-yl]ethyl]pyrrolidine-3-carboxylic acid

[F563]

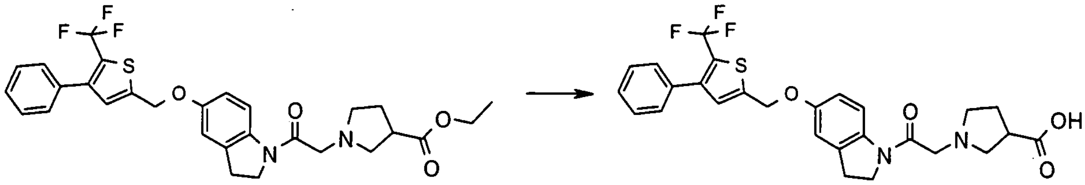

To a solution of 1-[2-oxo-2-[5-[(4-phenyl-5-trifluoromethyl-2-thienyl)methoxy]indolin-1-yl]ethyl]pyrrolidine-3-carboxylic acid ethyl ester (313 mg) in THF (3.0 mL), methanol (1.5 mL) and 1N aqueous sodium hydroxide solution (1.5 mL) were added at room temperature, and the mixture was stirred for 15 hours at room temperature. Water (5.0 mL) was added to the reaction mixture, and the pH of the resultant mixture was adjusted to 4 with 1N hydrochloric acid, followed by adding thereto a 20% methanol/chloroform mixture (10 mL) for phase separation. The aqueous layer was extracted with a 20% methanol/chloroform mixture (3 $\times$ 5 mL). The extracts were combined and dried over sodium sulfate anhydrate. The resultant mixture was concentrated under reduced pressure. Diethyl ether (5.0 mL) was added to the residue, and the precipitated solid was collected by filtration and dried, whereby the title compound (183 mg) was yielded.
$^1$H-NMR (DMSO-$d_6$) $\delta$:
1.94(2H,br s),2.66(2H,br s) , 3.09 (1H,br s) , 3.26-3.58 (6H,br m),4.08(2H,br s),5.32(2H,s),6.86(1H,d,J=8.5Hz),6.96(1H, br s),7.34(1H,s),7.43-7.45(5H,m),7.97(1H,d,J=8.5Hz).
MS (ESI)m/z:531 $(M+H)^+$.

[Example 121]

1-[2-Oxo-2-[5-[(4-phenyl-5-trifluoromethyl-2-thienyl)methoxy]indolin-1-yl]ethyl]pyrrolidine-(3R)-carboxylic acid

(1) (3R)-Pyrrolidinecarboxylic acid methyl ester hydrochloride

[F564]

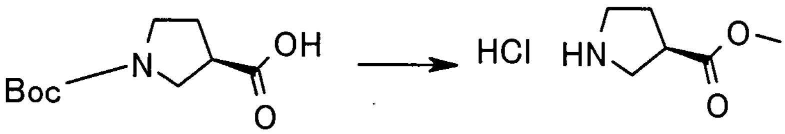

To a solution of 1-(tert-butoxycarbonyl)pyrrolidine-(3R)-carboxylic acid (1.00 g) in methanol (10 mL), thionyl chloride (712 $\mu$L) was slowly added at room temperature, and the mixture was refluxed for 2 hours. The reaction mixture was left to stand to cool to room temperature and concentrated under reduced pressure, whereby the title compound (617 mg) was yielded.
$^1$H-NMR (DMSO-$d_6$) $\delta$:
1.96-2.04(1H,m),2.11-2.19(1H,m),3.12-3.20(4H,m),3.23-3.36 (4H,m), 9.37 (1H,br s).

(2) 1- [2-Oxo-2- [5- [(4-phenyl-5-trifluoromethyl-2-thienyl)methoxy] indolin-1-yl] ethyl]pyrrolidine- (3R) - carboxylic acid 2-oxo-2-[5-[(4-phenyl-5-trifluoromethyl-2-thienyl)methoxy]indolin-1-yl]ethyl ester

[F565]

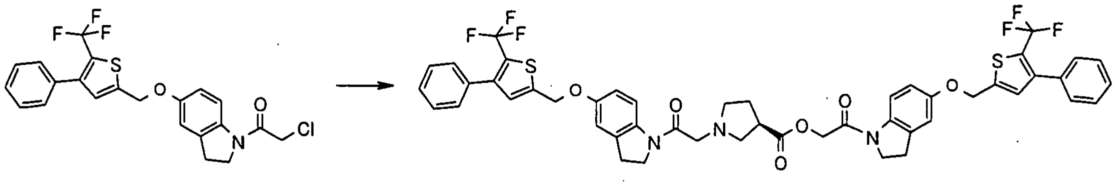

To a suspension of 1-(2-chloroacetyl)-5-[(4-phenyl-5-trifluoromethyl-2-thienyl)methoxy]indoline (407 mg) in acetonitrile (5 mL), 3-(R)-pyrrolidinecarboxylic acid methyl ester hydrochloride (164 mg) and DIEA (784 μL) were added at room temperature, and the mixture was stirred for 2 hours at 85°C. The reaction mixture was left to stand to cool to room temperature and concentrated under reduced pressure. To the concentrated mixture, a 10% methanol/chloroform mixture (10 mL) and saturated aqueous sodium hydrogencarbonate solution (10 mL) were added for phase separation. The resultant mixture was extracted with a 10% methanol/chloroform mixture (3 × 10 mL), and the extracts were combined and concentrated under reduced pressure. The residue was purified by silica gel column chromatography (Biotage 40S), whereby the title compound (244 mg) was yielded.
$^1$H-NMR ($CDCl_3$) δ:
2.17-2.33(2H,m),2.72(1H,dt,J=8.0,7.6Hz),2.90-3.00(2H,m),3.14-3.32 (6H,m), 3.42 (2H, s), 4.04 (2H, t,J=8.3Hz), 4.15 (2H, t, J=8.4Hz), 4 .76(2H,s),5.19(4H,s),6.78-6.86(4H,m),7.06(2H,br s),7.39-7.43(10H,m),8.10(1H,d,J=8.8Hz),8.18(1H, d,J=8.8Hz).
MS (ESI) m/z : 946 $(M+H)^+$.

(3) 1- [2-Oxo-2- [5- [(4-phenyl-5-trifluoromethyl-2-thienyl)methoxy] indolin-1-yl]ethyl]pyrrolidine- (3R) - carboxylic acid

[F566]

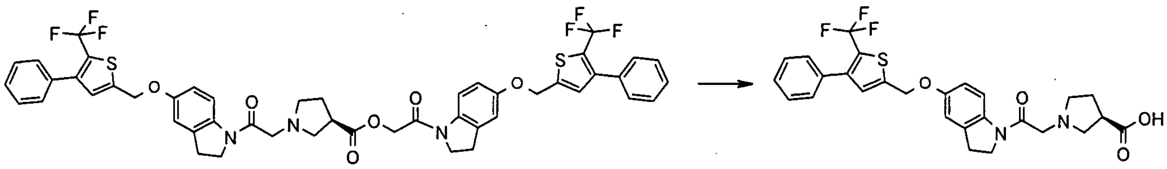

To a solution of 1- [2-oxo-2- [5- [(4-phenyl-5-trifluoromethyl-2-thienyl)methoxy]indolin-1-yl] ethyl] pyrrolidine-(3R) -carboxylic acid 2-oxo-2- [5- [ (4-phenyl-5-trifluoromethyl-2-thienyl)methoxy]indolin-1-yl] ethyl ester (244 mg) in THF (2.0 mL), methanol (1.0 mL) and 1N aqueous sodium hydroxide solution (1.00 mL) were added at room temperature, and the mixture was stirred for 7 hours at room temperature. To the reaction mixture, water (2 mL) and diethyl ether (5 mL) were added, and the mixture was stirred for 30 minutes at room temperature. The resultant mixture was partitioned, and the pH of the aqueous layer was adjusted to 4 with 1N hydrochloric acid. The aqueous layer was extracted with a 20% methanol/chloroform mixture (3 × 5 mL). The extracts were combined and dried over sodium sulfate anhydrate, followed by concentration under reduced pressure. Dimethyl sulfoxide (3 mL) was added to the residue, and insoluble matter was removed. The filtrate was purified by high-performance liquid chromatography (NOMURA Develosil Combi-RP5), and the target fraction was freeze-dried, whereby the title compound (67.5 mg) was yielded.
$^1$H-NMR (DMSO-$d_6$) δ:
1.89(2H,dt,J=7.1,7.1Hz),2.58(2H,dt,J=7.9,7.9Hz),2.69(1H,dd,J= 8.0,6.3Hz),2.80(1H,t,J=8.5Hz),2.86(1H,dt, J=7.1,7.1Hz),3.04(2H ,t,J=8.0Hz),3.31(2H,s),4.05(2H,t,J=8.5Hz),5.28(2H,s),6.79(1H, dd,J=8.8,2.0Hz),6.91(1H,s),7.30(1H,s),7.44-7.35(5H, m),7.92(1H,d,J=8.8Hz).
MS (ESI) m/z : 531 $(M+H)^+$.

Anal. Calcd for $C_{27}H_{25}F_3N_2O_4S$.
$1.0H_2O$:C,59.12;H,4.96;F,10.39;N,5.11;S,5.85.Found:C,58.96;H,4 .71;F,10.20;N,4.98;5,5.70.

[Example 122]

1-[2-[5-(4-Isopropyl-3-trifluoromethylbenzyloxy)indolin-1-yl]-2-oxoethyl]piperidine-(3R)-carboxylic acid

(1) (3R)-Ethoxycarbonylpiperidine-1-acetic acid benzyl ester

[F567]

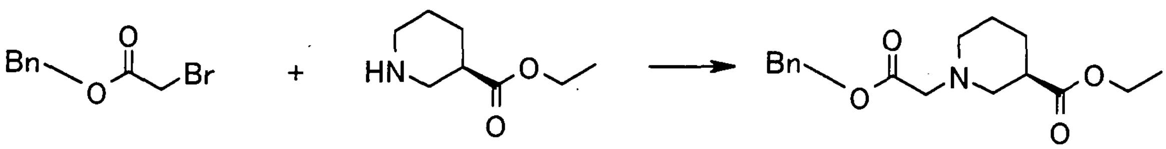

To a solution of bromoacetic acid benzyl ester (2.38 mL) in acetonitrile (30 mL), DIEA (3.92 mL) and 3-(R)-piperidinecaroboxylic acid ethyl ester (2.36 g) were added at room temperature, and the mixture was stirred for 2 hours at 80°C. The reaction mixture was left to stand to cool to room temperature and concentrated under reduced pressure. To the residue, water (20 mL) and a 10% methanol/chloroform mixture (30 mL) were added for phase separation. The aqueous layer was extracted with a 10% methanol/chloroform mixture (2 × 15 mL), and the extracts were combined and dried over sodium sulfate anhydrate. Solvent was removed under reduced pressure, whereby the title compound (4.18 g) was yielded.
$^1$H-NMR ($CDCl_3$) δ :
1.24(3H,dt,J=1.0,7.1Hz),1.35-1.47(1H,m),1.58-1.77(2H,m),1.97(1H,dd,J=12.4,3.2Hz),2.20(1H,dt,J=3.2,11.0Hz), 2.36 (1H,t,J=11.0Hz) ,2.59-2.67(1H,m),2.85(1H,d,J=11.0Hz),3.09(1H,dd,J=11.0,2.9Hz),3.29( 2H,s),4.12(2H,q,J=7.2Hz), 5.16(2H,s),7.32-7.36(5H,m).

(2) (3R)-Ethoxycarbonylpiperidine-1-acetic acid

[F568]

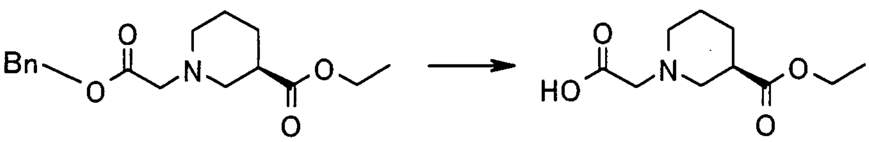

(3R)-Ethoxycarbonylpiperidine-1-acetic acid benzyl ester (4.18 g) was dissolved in methanol (70 mL), and 5% Pd/C (20 mg) was added to the solution. The mixture was refluxed for 5 hours under a hydrogen atmosphere at room temperature. The reaction mixture was filtrated through a Celite pad, and the filtrate was concentrated under reduced pressure, whereby the title compound (2.69 g) was yielded.
$^1$H -NMR ($CDCl_3$ ) δ :
1.26 (3H, t, J=7.1Hz), 1.50-1.60 (1H, m), 1.89 (1H, dt, J=14.6,3.4Hz), 2.01-2.10 (1H, m), 2.11.- 2.20 (1H, m), 2.69 (1H, dt, J=2.2,11.7Hz),2.85(1H,t,J=11.6Hz),3.06-3.14(1H,m),3.54(2H,s),3.58-3.65(1H,m),3.74(1H,d,J=12.4Hz),4.15(2H,q, J=7.1Hz).

(3) 1-[2-(5-Benzyloxyindolin-1-yl)-2-oxoethyl]piperidine-(3R)-carboxylic acid ethyl ester

[F569]

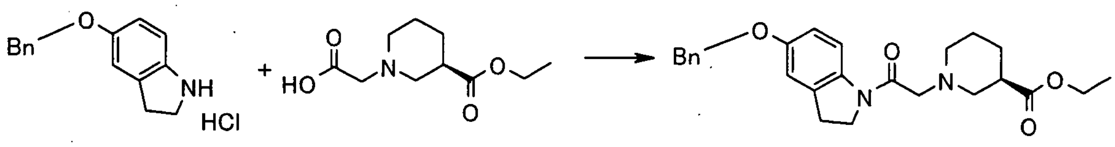

To a solution of (3R)-ethoxycarbonylpiperidine-1-acetic acid (646 mg) in DMF (10 mL), DIEA (1.57 mL), HOBt (527 mg), and EDC·HCl (748 mg) were added at room temperature, and the mixture was stirred for 10 minutes. 5-Benzyloxyindoline hydrochloride (785 mg) was added to the mixture at room temperature. The reaction mixture was stirred for 3 hours at room temperature and concentrated under reduced pressure. To the residue, water (30 mL) and chloroform (30 mL) were added for phase separation. The aqueous layer was extracted with chloroform (3 × 20 mL). The extracts were combined and dried over sodium sulfate anhydrate and then concentrated under reduced pressure. The residue was purified by silica gel column chromatography (Biotage 40S), whereby the title compound (980 mg) was yielded.
$^1$H-NMR ($CDCl_3$) δ:
1.21(3H,t,J=7.8Hz),1.60-1.77(3H,m),1.87-2.02(1H,m),2.15-2.47(2H,m),2.59-2.67(1H,m),2.79-2.87(1H,m),3.01-3.10 (1H,m),3.14(2H,t,J=8.5Hz),3.25(2H,s),4.11(2H,q,J=7.8Hz),4 .16(1H,dt,J=9.0,7.1Hz),5.04(2H,s),6.79-6.84(2H,m), 7.44-7.29(6H,m),8.14(1H,d,J=8.8Hz).
MS (ESI)m/z:423 $(M+H)^+$.

(4) 1-[2-(5-Hydroxyindolin-1-yl)-2-oxoethyl]piperidine-(3R)-carboxylic acid ethyl ester

[F570]

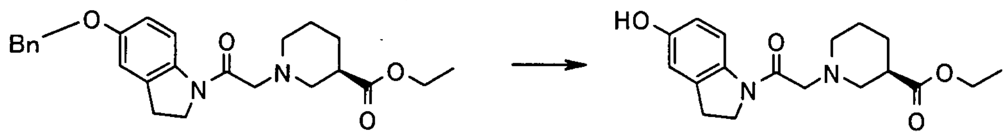

To a solution of 1-[2-(5-benzyloxyindolin-1-yl)-2-oxoethyl]piperidine-(3R)-carboxylic acid ethyl ester (980 mg) in methanol (30 mL), 5% Pd/C (15 mg) was added, and the mixture was stirred for 5 hours under a hydrogen atmosphere at room temperature. The reaction mixture was filtered through a Celite pad, and the filtrate was concentrated under reduced pressure. The residue was purified by silica gel column chromatography (Biotage 25M), whereby the title compound (469 mg) was yielded.
$^1$H-NMR ($CDCl_3$) δ:
1.21 (3H, t, J=7.1Hz), 1.45-1.68 (2H, m), 1.69-1.77 (1H, m), 1.87-1.94 (1H, m), 2.24 (1H, dt, J=3.2,10.5Hz), 2.43 (1H, t, J=10.3Hz),2.60-2.66(1H,m),2.79-2.85(1H,m),3.03(1H,dd,J=11.2,2.7Hz),3.09(2H,t,J=8.3Hz),3.24(2 H,d,J=2.7Hz),4.11 (2H,q,J=7.3Hz),4.14(2H,t,J=8.5Hz),6.72-6.68(2H,m),8.07(1H,d,J=8.5Hz).

(5) 1-[2-[5-(4-Isopropyl-3-trifluoromethylbenzyloxy)indolin-1-yl]-2-oxoethyl]piperidine-(3R)-carboxylic acid ethyl ester

[F571]

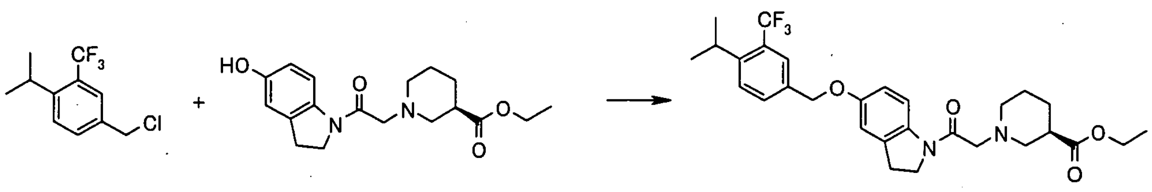

To a solution of 4-isopropyl-3-(trifluoromethyl)benzyl chloride (166 mg) in DMF (5.0 mL), potassium carbonate (290 mg) and 1-[2-(5-hydroxyindolin-1-yl)-2-oxoethyl]piperidine-(3R)-carboxylic acid ethyl ester (233 mg) were added at room temperature, and the mixture was stirred for 4 hours at 60°C. The reaction mixture was left to stand to cool to room temperature, and concentrated under reduced pressure. To the residue, water (15 mL) and a 10% methanol/chloroform mixture (30 mL) were added for phase separation. The aqueous layer was extracted with a 10% methanol/chloroform mixture (2 x 15 mL). The extracts were combined and dried over sodium sulfate anhydrate, followed by was concentration under reduced pressure. The residue was purified by silica gel column chromatography (Biotage 40S), whereby the title compound (324 mg) was yielded.
$^1$H-NMR ($CDCl_3$) δ :
1.21(3H,t,J=7.2Hz), 1.26(6H,d,J=6.6Hz), 1.46-1.57(1H,m), 1.60-1.67(1H,m), 1.72-1.76(1H,m), 1.89-1.93(1H,m), 2.21-2.29(1H,m), 2.45(1H,t,J=10.7Hz), 2.60-2.66(1H,m), 2.78-2.86(1H,m), 3.03(1H,d,J=8.8Hz), 3.15(2H,t,J=8.4Hz), 3.25(2H,d,J= 3.9Hz), 3.36(1H,dq,J=6.6,6.6Hz), 4.10(2H,t,J=7.1Hz), 4.17(2H,q,J =7.3Hz), 5.02(2H,s), 6.79(1H,dd, J=8.8,2.4Hz), 6.83(1H,br s), 7.48(1H,d,J=8.0Hz), 7.56(1H,d,J=8.0Hz), 7.65(1H,s), 8.15(1H,d , J=8.8Hz).
MS(ESI)m/z:533(M+H)+.

(6) 1-[2-[5-(4-Isopropyl-3-trifluoromethylbenzyloxy)indolin-1-yl]-2-oxoethyl]piperidine-(3R)-carboxylic acid

[F572]

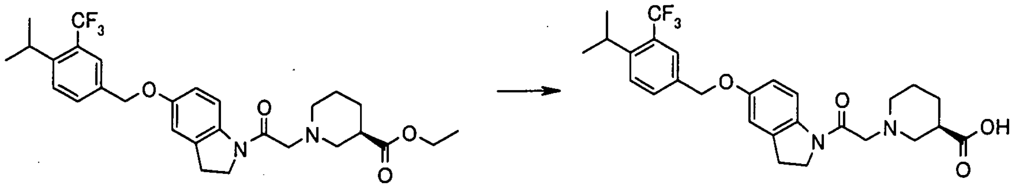

To a solution of 1- [2- [5- (4-isopropyl-3-trifluoromethylbenzyloxy)indolin-1-yl]-2-oxoethyl]piperidine-(3R)-carboxylic acid ethyl ester (324 mg) in THF (3.0 mL), methanol (1.5 mL) and 1N aqueous sodium hydroxide solution (1.50 mL) were added at room temperature. The reaction mixture was stirred for 2 hours at room temperature. Water (5 mL) was added to the mixture, and the pH of the resultant mixture was adjusted to 4 with 1N hydrochloric acid. A 10% methanol/chloroform mixture (30 mL) was added to the mixture for phase separation. The aqueous layer was extracted with a 10% methanol/chloroform mixture (2 x 15 mL). The extracts were combined and dried over sodium sulfate anhydrate, and solvent was removed under reduced pressure to concentrate the mixture. To the residue, dimethyl sulfoxide (4.0 mL) was added, and insoluble matter was removed. The residue was purified by high-performance liquid chromatography (NOMURA Develosil Combi-RP5), and the target fraction was freeze-dried, whereby the title compound (149 mg) was yielded.
$^1$H-NMR (DMSO-$d_6$) δ :
1.23(6H,d,J=6.8Hz), 1.31-1.52(2H,m), 1.58-1.66(1H,m), 1.72-1.80(1H,m), 2.21(1H,t,J=9.8Hz), 2.29-2.37(1H,m), 2.40-2.46(1H,m), 2.63-2.70(1H,m), 2.89(1H,d,J=8.5Hz), 3.08(2H,t,J=8.4Hz), 3.18-3.22(1H,m), 3.24(1H,d,J=5.4Hz), 3.30(2H,br s), 4.14(2H,t,J=8.4Hz), 5.10(2H,s), 6.80(1H,dd,J=8.8,2.OHz), 6.93 (1H,br s), 7.70-7.64(3H,m), 7.95(1H,d, J=8.8Hz).
MS (ESI) m/z : 505 $(M+H)^+$.
Anal. Calcd for $C_{27}H_{31}F_3N_2O_4$.

0.75$H_2O$:C,62.60;H,6.32;F,11.00;N,5.41.Found:C,62.66;H,6.18;F, 10.81;N,5.38.

[Example 123]

3-[N-[(1S)-Methyl-2-oxo-2-[5-[(4-phenyl-5-trifluoromethyl-2-thienyl) methoxy] indolin-1-yl] ethyl] amino] propionic acid

(1) 1-[(2S)-[N-[(tert-Butoxycarbonyl)amino]propionyl]-5-[(4-phenyl-5-trifluoromethyl-2-thienyl)methoxy]indoline

[F573]

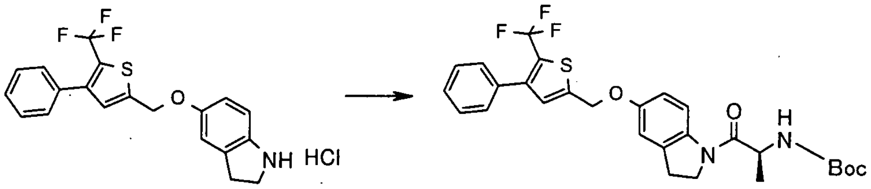

To a solution of N-(tert-butoxycarbonyl)-D-alanine (189 mg) in DMF (5.0 mL), DIEA (523 μL), HOBt (176 mg), and EDC•HCl (249 mg) were added at room temperature, and the reaction mixture was stirred for 10 minutes. To the mixture, 5-[(4-phenyl-5-trifluoromethyl-2-thienyl)methoxy]indoline hydrochloride (412 mg) was added, and the mixture was stirred for 14 hours. The reaction mixture was concentrated under reduced pressure, and to the concentrated mixture, ethyl acetate (10.0 mL) and water (7.5 mL) were added for phase separation. The mixture was extracted with ethyl acetate (3 x 5.0 mL). The extracts were combined and concentrated under reduced pressure. The residue was purified by silica gel column chromatography (Biotage 25M), whereby the title compound (559 mg) was yielded. $^1$H-NMR ($CDCl_3$) δ:
1.40(3H,d,J=6.8Hz), 1.44(9H,s), 3.17-3.25(2H,br m), 4.04-4.14(1H,m), 4.30(1H,dt,J=8.0,9.8Hz), 4.58(1H,dq, J=7.3,7.1Hz), 5. 20(2H,s), 5.44(1H,d,J=8.0Hz), 6.82(1H,d,J=8.8Hz), 6.85(1H,s), 7.0 6(1H,s), 7.43-7.38(5H,m), 8.15(1H, d,J=8.8Hz).
MS(ESI)m/z:547(M+H)$^+$.

(2) 1-[(2S)-Aminopropionyl]-5-[(4-phenyl-5-trifluoromethyl-2-thienyl)methoxy]indoline hydrochloride

[F574]

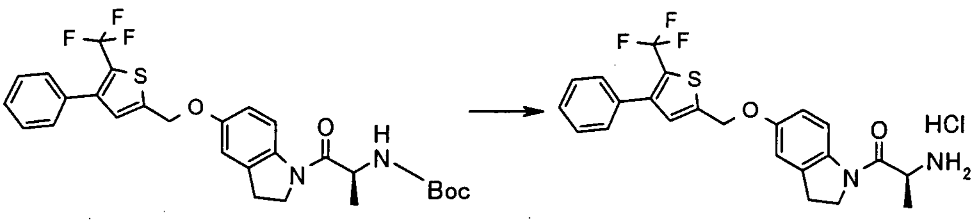

To a solution of 1-[(2S)-N-(tert-butoxycarbonyl)amino]propionyl]-5-[(4-phenyl-5-trifluoromethyl-2-thienyl)methoxy]indoline (324 mg) in dioxane (0.5 mL), 4N HC1/1,4-dioxane solution (1.0 mL) was added at room temperature, and the mixture was stirred for 13 hours. The reaction mixture was concentrated under reduced pressure. Diethyl ether (5.0 mL) was added to the residue, and the precipitated solid was collected by filtration and dried, whereby the title compound (284 mg) was yielded.
$^1$H-NMR (DMSO-$d_6$) δ:
1.43(3H,d,J=6.8Hz), 3.18(2H,t,J=8.3Hz), 4.08-4.27(3H,m), 5.37(2H,s), 6.92(1H,dd,J=8.8,2.4Hz), 7.04(1H,d,J=2.4 Hz),

7.37(1H,s), 7.41-7.51(5H,m), 8.01(1H,d,J=8.8Hz), 8.45-8.29(2H,br m).
MS(ESI)m/z:447(M+H)+.

(3) 3-[N-[(1S)-Methyl-2-oxo-2-[5-[(4-phenyl-5-trifluoromethyl-2-thienyl)methoxy]indolin-1-yl]ethyl]amino]propionic acid tert-butyl ester

[F575]

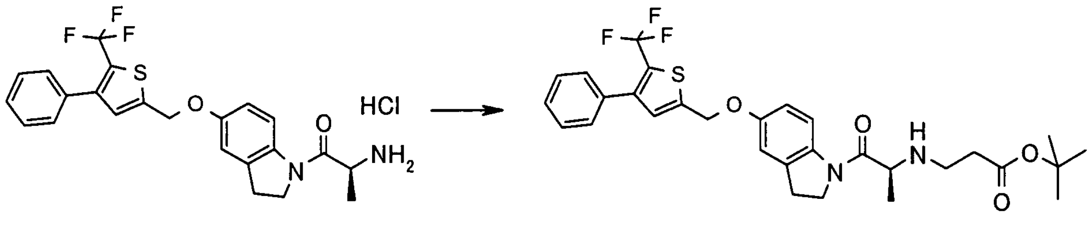

To a solution of 1-[(2S)-aminopropionyl]-5-[(4-phenyl-5-trifluoromethyl-2-thienyl)methoxy]indoline hydrochloride (236 mg) in THF (1.0 mL), 1-butyl-3-methylimidazolium hexafluorophosphate (2.0 mL) and tert-butyl acrylate (116 μL) were added at room temperature, and the mixture was stirred for 24 hours at 60°C. tert-Butyl acrylate (600 μL) was added to the mixture, and the mixture was stirred for 2 days at 60°C. The reaction mixture was concentrated under reduced pressure, and chloroform (10.0 mL) and water (7.5 mL) were added to the mixture for phase separation. The aqueous layer was extracted with chloroform (3 x 5.0 mL). The extracts were combined and concentrated under reduced pressure. The residue was purified by silica gel column chromatography (Biotage 25M), whereby the title compound (220 mg) was yielded. $^1$H-NMR($CDCl_3$)δ:
1.31(3H,d,J=6.8Hz), 1.44(9H,s), 2.41(2H,t,J=7.6Hz), 2.70(1H,dt,J =11.7,7.3Hz), 2.87(1H,dt,J=11.7,7.3Hz), 3.21(2H,t, J=8.5Hz), 3.53 (1H,q,J=6.8Hz), 4.06(1H,dt,J=7.3,9.8Hz), 4.22(1H,dt,J=8.5,9.8Hz ), 5.21(2H,s), 6.82(1H,dd, J=8.8,2.4Hz), 6.85(1H,br s), 7.06(1H,s), 7.43-7.38(5H,m), 8.22(1H,d,J=8.8Hz).

(4) 3-[N-[(1S)-Methyl-2-oxo-2-[5-[(4-phenyl-5-trifluoromethyl-2-thienyl)methoxy]indolin-1-yl] ethyl] amino] propionic acid

[F576]

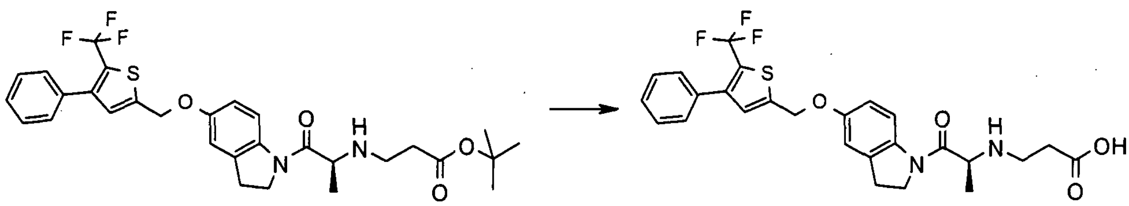

To a solution of 3-[N-[(1S)-methyl-2-oxo-2-[5-[(4-phenyl-5-trifluoromethyl-2-thienyl)methoxylindolin-1-yl]ethyl] amino]propionic acid tert-butyl ester (220 mg) in 1,4-dioxane (0.3 mL), 4N HCl/1,4-dioxane solution (1.5 mL) was added at room temperature, and the mixture was stirred for 13 hours. The reaction mixture was concentrated under reduced pressure. Water (3.0 mL) was added to the residue, and the pH of the residue was adjusted to 7 with saturated aqueous sodium hydrogencarbonate solution. The pH-adjusted mixture was extracted with a 20% methanol/chloroform mixture (4 x 3.0 mL). The extracts were combined and dried over sodium sulfate anhydrate. Insoluble matter was removed by filtration, and the filtrate was concentrated under reduced pressure. The residue was purified by silica gel column chromatography (Biotage 25M), whereby the title compound (61.8 mg) was yielded.
$^1$H-NMR (DMSO-$d_6$)δ:
1.17(3H,d,J=5.6Hz),2.26(2H,t,J=6.8Hz),2.56-2.73(2H,m), 3.13(2H,t,J=7.8Hz), 3.59(1H,d,J=5.4Hz), 4.06(1H,dt,J

=8.5,8.0Hz), 4.25(1H,dt,J=8.5,8.5Hz), 5.34(2H,s), 6.86(1H,d,J=8. 3Hz), 6.98(1H,s), 7.35(1H,s), 7.40-7.49(5H,m), 8.06 (1H,d,J=8.3Hz).
MS (ESI) m/z : 519 $(M+H)^+$.
Anal. Calcd for $C_{26}H_{25}F_3N_2O_4S\cdot$ $1.75H_2O$:C,56.77;H,5.22;F,10.36;N,5.09;5,5.83.Found:C,56.76;H, 5.07;F,10.16;N, 5.09;5,5.66.

[Example 124]

3-[N-[1,1-Dimethyl-2-oxo-2-[5-[(4-phenyl-5-trifluoromethyl-2-thienyl)methoxy]indolin-1-yl]ethyl]amino]propionic acid

(1) 1-[2-[N-(tert-Butoxycarbonyl)amino]-2-methylpropionyl]-5-[(4-phenyl-5-trifluoromethyl-2-thienyl)methoxy]indoline

[F577]

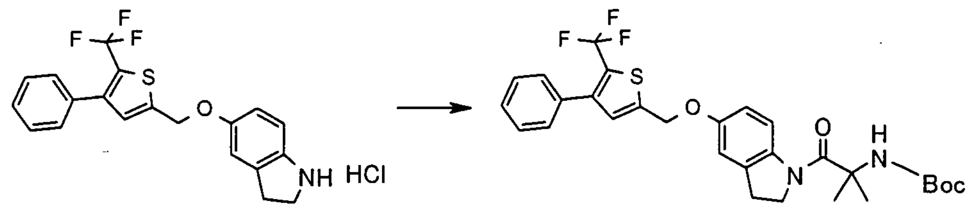

To a solution of N-(tert-butoxycarbonyl)-2,2-dimethylglycine (203 mg) in DMF (5.0 mL), DIEA (523 μL), HOBt (176 mg), and EDC·HCl (249 mg) were added at room temperature, and the mixture was stirred for 10 minutes. To the mixture, 5-[(4-phenyl-5-trifluoromethyl-2-thienyl)methoxy]indoline hydrochloride (412 mg) was added at room temperature, and the mixture was stirred for 14 hours. The reaction mixture was concentrated under reduced pressure, and to the concentrated mixture, chloroform (10.0 mL) and water (7.5 mL) were added for phase separation. The mixture was extracted with chloroform (3 x 5.0 mL). The extracts were combined and concentrated under reduced pressure. The residue was purified by silica gel column chromatography (Biotage 25M), whereby the title compound (561 mg) was yielded.
$^1H$-NMR($CDCl_3$)δ :
1.28-1.45(9H,br m),1.58(3H,br s), 1.65(3H,s), 3.09(2H,t,J=8.0Hz), 4.21(2H,t,J=8.0Hz), 5.20(2H,s ), 6.82(1H,dd, J=8.5,2.2Hz), 6.85(1H,br s), 7.06(1H,s), 7.39-7.43(5H,m), 8.02(1H,s), 8.27-8.19(1H,br m).
MS (ESI) m/z : 560 $(M)^+$.

(2) 1-(2-Amino-2-methylpropionyl)-5-[(4-phenyl-5-trifluoromethyl-2-thienyl)methoxy]indoline

[F578]

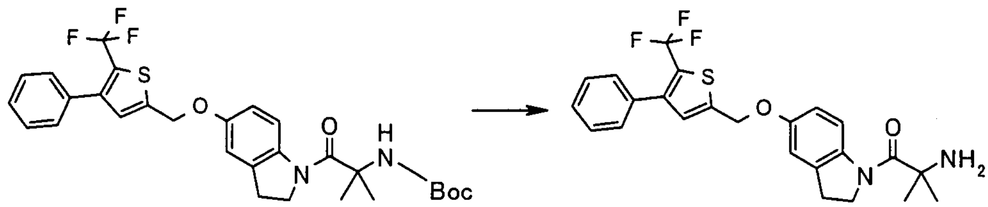

To a solution of 1-[2-(tert-butoxycarbonylamino)-2-methyl-propionyl]-5-[(4-phenyl-5-trifluoromethyl-2-thienyl) methoxy]indoline (561 mg) in 1,4-dioxane (0.5 mL), 4N HCl/1,4-dioxa (1.5 mL) was added at room temperature, and the mixture was stirred for 1 hour. The reaction mixture was concentrated under reduced pressure. To the residue, water

(2.0 mL), a 10% methanol/chloroform mixture (3.0 mL), and saturated aqueous sodium hydrogencarbonate were added for phase separation. The aqueous layer was extracted with a 10% methanol/chloroform mixture (3 x 3.0 mL). The extracts were combined and dried over sodium sulfate anhydrate. Insoluble matter was removed by filtration, and the filtrate was concentrated under reduced pressure, whereby the title compound (318 mg) was yielded.
$^1$H-NMR ($CDCl_3$) δ :
1.49(6H,s), 3.11(2H,t,J=8.1Hz), 4.48(2H,t,J=8.1Hz), 5.20(2H,s), 6 .81(1H,dd,J=9.0,2.2Hz), 6.85(1H,br s), 7.06(1H,s), 7.38-7.43(5H,m), 8.18(1H,d,J=9.0Hz).
MS(ESI)m/z:461(M+H)$^+$.

(3) 3-[N-[1,1-Dimethyl-2-oxo-2-[5-[(4 -phenyl-5-trifluoromethyl-2-thienyl)methoxy]indolin-1-yl]ethyl]amino]propionic acid tert-butyl ester

[F579]

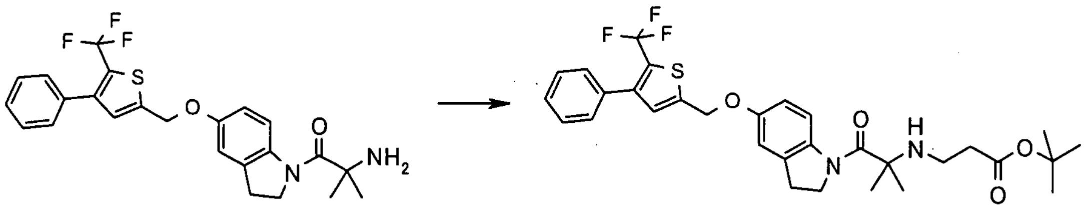

To a solution of 1-(2-amino-2-methylpropionyl)-5-[(4-phenyT-5-trifluoromethyl-2-thienyl)methoxy]indoline (318 mg) in THF (1.0 mL), 1-butyl-3-methylimidazolium hexafluorophosphate (2.0 mL) and tert-butyl acrylate (1.50 mL) were added at room temperature. The reaction mixture was stirred for 24 hours at 60°C, and tert-butyl acrylate (3.0 mL) was added to the mixture. The mixture was stirred for 4 days at 60°C. The reaction mixture was concentrated under reduced pressure, and chloroform (10.0 mL) and water (7.5 mL) were added to the mixture for phase separation. The aqueous layer was extracted with chloroform (3 x 5.0 mL). The extracts were combined and concentrated under reduced pressure. The residue was purified by silica gel column chromatography (Biotage 25M), whereby the title compound (40.6 mg) was yielded.
MS(ESI)m/z:589(M+H)$^+$.

(4) 3-[1,1-Dimethyl-2-oxo-2-[5-[(4-phenyl-5-trifluoromethyl-2-thienyl)methoxy]indolin-1-yl]ethylamino]propionic acid

[F580]

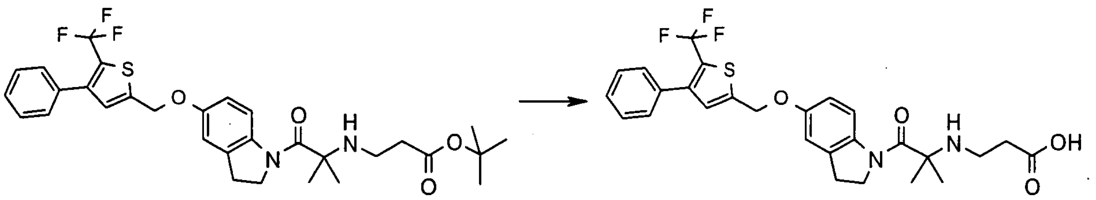

To a solution of 3-[N-[1,1-dimethyl-2-oxo-2-[5-[(4-phenyl-5-trifluoromethyl-2-thienyl)methoxy]indolin-1-yl]ethyl] amino]propionic acid tert-butyl ester (40.6 mg) in 1,4-dioxane (0.5 mL), 4N HC1/1,4-dioxane solution (1.0 mL) was added at room temperature, and the mixture was stirred for 1 hour. The reaction mixture was concentrated under reduced pressure, and to the residue, water (2.0 mL), a 20% methanol/chloroform mixture (3.0 mL), and saturated aqueous sodium hydrogencarbonate solution were added, to adjust the pH of the mixture to 7. The mixture was extracted with a 20% methanol/chloroform mixture (3 x 3.0 mL). The extracts were combined and dried over sodium sulfate anhydrate. Insoluble matter was removed by filtration, and the filtrate was concentrated under reduced pressure. The residue was purified by silica gel column chromatography (Biotage 25S), whereby the title compound (19.9 mg) was yielded.

MS(ESI)m/z:S33(M+H)+.

[Example 125]

2-Fluoro-3-[N-[2-oxo-2-[5-[(4-phenyl-5-trifluoromethyl-2-thienyl)methoxy]-2,3-dihydro-1H-indol-1-yl] ethyl] amino] propionic acid

(1) 3-(Dibenzylamino)-2-fluoropropanoic acid ethyl ester cf.) Tetrahedron Lett., 2003, 44, 2375

[F581]

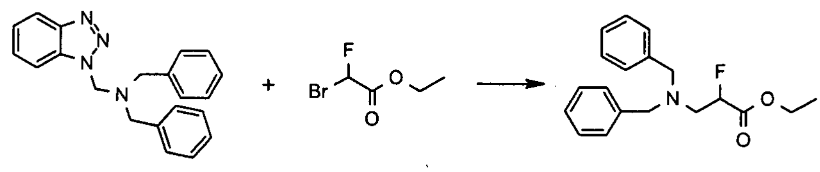

THF (10 mL) and trimethylsilane chloride (1.06 mL) were added to zinc (1.10 g) at room temperature, and the mixture was stirred for 10 minutes at room temperature. 2-Bromo-2-fluoroacetic acid ethyl ester (1.00 g) was added thereto at room temperature, and the resultant mixture was stirred for 10 minutes at room temperature. A solution of 1H-1,2,3-benztriazol-1-yl-N,N-dibenzylmethanamine (Tetrahedron Lett., 2003, 44, 2375) (2.74 g) in THF (15 mL) was added thereto at room temperature, followed by stirring for 18 hours at room temperature. Saturated aqueous sodium hydrogencarbonate solution (50 mL) was added to the reaction mixture, and the resultant mixture was filtered through a Celite pad. Ethyl acetate (50 mL) was added to the mixture for phase separation. The organic layer was dried over sodium sulfate anhydrate, and solvent was removed under reduced pressure. The residue was purified by silica gel flash column chromatography (Biotage 40M), whereby the title compound (1.15 g) was yielded.
$^1$H-NMR ($CDCl_3$)δ:
1.21(3H,t,J=7.1Hz), 2.92-3.12(2H,m), 3.55(2H,d,J=13.6Hz), 3.83(2H,d,J=13.6Hz), 4.07-4.29(2H,m), 4.96-5.13(1H,m), 7.21-7.36(10H,m).
MS (ESI) m/z : 316 (M+H)+.

(2) 3-Amino-2-fluoropropanoic acid ethyl ester trifluoroacetic acid salt

[F582]

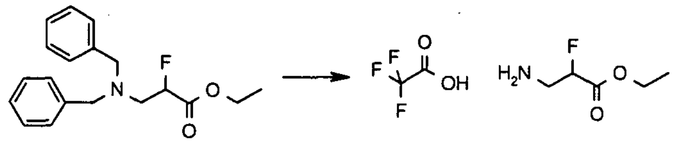

To a solution of 3-(dibenzylamino)-2-fluoropropanoic acid ethyl ester (1.15 g) in ethanol (4.0 mL), palladium hydroxide (120 mg) and trifluoroacetic acid (0.337 mL) were added at room temperature, and the resultant mixture was hydrogenated with stirring for 18 hours. The reaction mixture was filtered, and the filtrate was concentrated under reduced pressure, whereby the title compound (850 mg) was yielded. $^1$H-NMR(DMSO-d6)δ:
1.24(3H,t,J=7.1Hz), 3.25-3.48(2H,m), 4.21(2H,q,J=7.1Hz), 5.30-5.47(1H,m), 8.26(3H,br s). The peak at 8.26 was also attributed to TFA.
MS (ESI) m/z : 135 M+.

(3) 2-Fluoro-3-[N-[2-oxo-2-[5-[(4-phenyl-5-trifluoromethyl-2-thienyl)methoxy]-2,3-dihydro-1H-indol-1-yl]ethyl]amino] propionic acid ethyl ester

[F583]

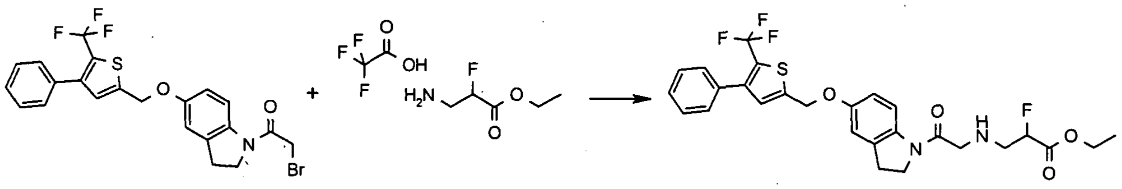

To a solution of 3-amino-2-fluoropropanoic acid ethyl ester trifluoroacetic acid salt (125 mg) in acetonitrile (3.0 mL), 2-bromo-1-[5-[[4-phenyl-5-(trifluoromethyl)-2-thienyl]methoxy]-2,3-dihydro-1H-indol-1-yl]-1-ethanone (100 mg) and DIEA (0.175 mL) were added at room temperature, and the mixture was stirred for 15 hours at 80°C. The mixture was left to stand to cool to room temperature and concentrated under reduced pressure. The residue was purified by silica gel flash column chromatography (Biotage 25M), whereby the title compound (60.6 mg) was yielded.
$^1$H-NMR($CDCl_3$)δ:
1.32(3H,t,J=7.1Hz), 3.12-3.32(4H,m), 3.56(2H,s), 4.01(2H,t,J=8.5Hz), 4.28(2H,q,J=7.1Hz), 4 .96-5.12(1H,m), 5.20(2H, s), 6.82(1H,dd,J=8.6,2.6Hz), 6.85(1H,s), 7.06 (1H,s), 7.37-7.44(5H,m), 8.16(1H,d,J=8.6Hz). No NH was observed.
MS (ESI) m/z : 551 $(M+H)^+$.

(4) 2-Fluoro-3-[N-[2-oxo-2-[5-[(4-phenyl-5-trifluoromethyl-2-thienyl)methoxy]-2,3-dihydro-1H-indol-1-yl] ethyl] amino] propionic acid

[F584]

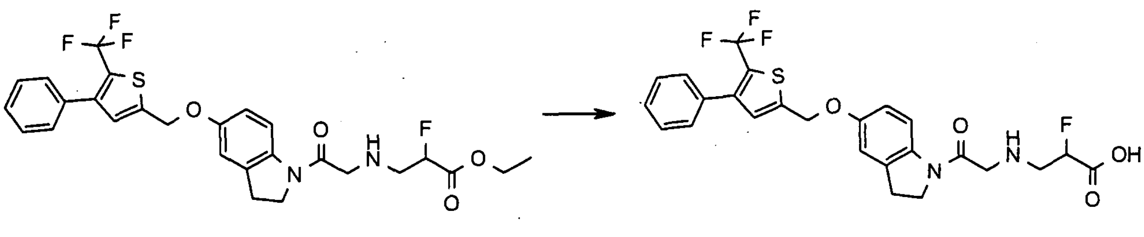

To a solution of 2-fluoro-3- [N- [2-oxo-2- [5- [(4-phenyl-5-trifluoromethyl-2-thienyl)methoxy]-2,3-dihydro-1H-indol-1-yl]ethyl]amino]propionic acid ethyl ester (60.0 mg) in THF (2.0 mL), methanol (1.0 mL), water (0.50 mL), and 1N aqueous sodium hydroxide solution (0.545 mL) were added at room temperature, and the mixture was stirred for 3 hours at room temperature. 1N Hydrochloric acid (0.545 mL) and water (10 mL) were added to the reaction mixture, and organic solvent was removed under reduced pressure. The resultant solid was collected by filtration and dried, whereby the title compound (53.0 mg) was yielded. $^1$H-NMR(DMSO-d6)δ:
3.00-3.20(4H,m), 3.65(2H,s), 4.02(2H,t,J=8.4Hz), 4.90-5.07(1H,m), 5.35(2H,s), 6.87(1H,dd,J=8.8,2.7Hz), 6.99(1H,s), 7.36 (1H,s), 7.40-7.51(5H,m), 7.99(1H,d,J=8.8Hz). Neither $CO_2H$ nor NH was observed.
IR (ATR) $cm^-$
$^1$:3055,1674,1491,1371,1290,1265,1242,1115,1093,1014,808,762,6 98.
MS (ESI) m/z : 523 $(M+H)^+$.
HR-MS(ESI)Calcd for $C_{25}H_{23}F_4N_2O_4S(M+H)^+$:523.15654.Found:523.15758 .
Anal. Calcd for $C_{25}H_{22}F_4N_2O_4S$:C,57.47;H,4.24;F,14.54;N,5.36;S,6.14.Found:C,57.6 3;H,4.37;F,14.06;N,4.98;5,5.95.

[Example 126]

3- [N- [2- [4-Methyl-5- [[4-phenyl-5- (trifluoromethyl) -2-thienyl]methoxy]-2,3-dihydro-1H-indol-1-yl]-2-oxoethyl] amino] propionic acid

(1) 5-Methoxy-4-methyl-1H-indole-1-carboxylic acid tert-butyl ester

[F585]

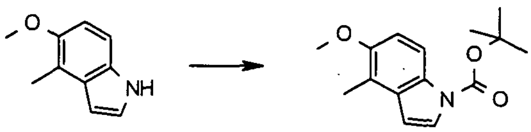

To a solution of 5-methoxy-4-methyl-1H-indole-1-carboxylic acid (commercially available) (6.99 g) in THF (50 mL), DMAP (265 mg) and $Boc_2O$ (9.95 g) were added at room temperature, and the reaction mixture was stirred for 17 hours at room temperature and concentrated under reduced pressure. The residue was purified by silica gel flash column chromatography (Biotage 40M), whereby the title compound (11.3 g) was yielded. $^1$H-NMR($CDCl_3$)δ:
1.66(9H,s), 2.38(3H,s), 3.87(3H,s), 6.55(1H,d,J=3.5Hz), 6.92(1H,d , J=8.5Hz), 7.56(1H,br d,J=3.5Hz), 7.90(1H,br d, J=8.5Hz).
MS (ESI) m/z : 262 $(M+H)^+$

(2) 5-Methoxy-4-methyl-1-indolinecarboxylic acid tert-butyl ester

[F586]

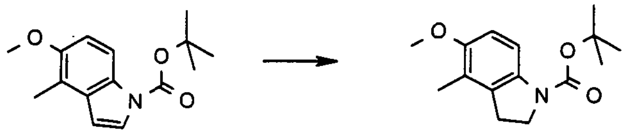

To a solution of 5-methoxy-4-methyl-1H-indole-1-carboxylic acid tert-butyl ester (12.7 g) in THF (50 mL), ethanol (200 mL) and 5% Pd/C (6.35 g) were added at room temperature. The reaction mixture was stirred for 15 hours under a hydrogen atmosphere at room temperature and subjected to filtration. The filtrate was concentrated under reduced pressure. The concentrated product was slurried with water (300 mL), and the slurry was filtered. The collected solid was dried (in vacuo at 50°C for 4 hours), whereby the title compound (12.4 g) was yielded. $^1$H-NMR ($CDCl_3$)δ:
1.54(9H,s),2.10(3H,s),2.98(2H;t,J=8.5Hz),3.79(3H,s),3.97(2H,b r s), 6.64(1H,d,J=8.8Hz), 7.22(1/2 of 1H,br s),7.61(1/2 of 1H, br s).
MS (ESI) m/z : 264 $(M+H)^+$.

(3) 5-Hydroxy-4-methyl-1-indolinecarboxylic acid tert-butyl ester (Padwa, Albert. *et al.*, J. Org. Chem., (1999), 64 (10), 3595-3607.)

[F587]

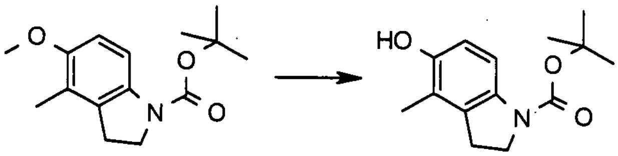

To a solution of 5-methoxy-4-methyl-1-indolinecarboxylic acid tert-butyl ester (1.00 g) in dichloromethane (35 mL), boron tribromide (1M dichloromethane solution) (8.30 mL) was added at -78°C. The reaction mixture was allowed to warm to room temperature and stirred for 17 hours. Saturated aqueous sodium hydrogencarbonate solution (200 mL), water (50 mL), and dichloromethane (100 mL) were added to the reaction mixture, and the resultant mixture was stirred for 10 minutes. $Boc_2O$ (870 mg) was added thereto, and the reaction mixture was stirred for 1 hour at room temperature for phase separation. The aqueous layer was extracted with dichloromethane (100 mL). The extracts were combined and dried over sodium sulfate anhydrate. Solvent was removed under reduced pressure, and the residue was purified by silica gel flash column chromatography (Biotage 40M). The obtained solid was slurried with hexane, then subjected to filtration, and the collected solid was dried, whereby the title compound (580 mg) was yielded.
$^1$H-NMR ($CDCl_3$)δ :
1.56(9H,s), 2.12(3H,s), 2.98(2H,t,J=8.7Hz), 3.97(2H,br s), 4.50(1H,s), 6.58(1H,d,J=8.5Hz), 7.14(1/2 of 1H,br s),7.55 (1/2 of 1H,br s).
MS(ESI)m/z:250(M+H)$^+$.

(4) 4-Methyl-5-[[4-phenyl-5-(trifluoromethyl)-2-thienyl]methoxy]-1-indolinecarboxylic acid tert-butyl ester

[F588]

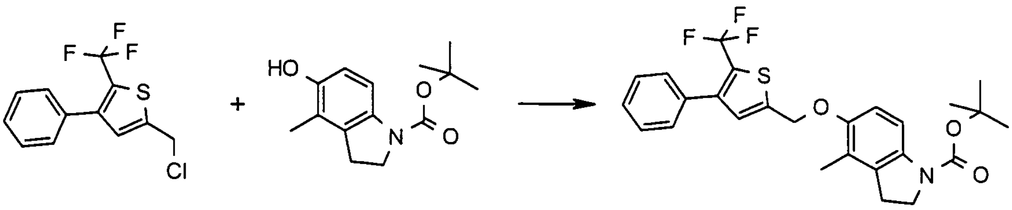

To a solution of 5-(chloromethyl)-3-phenyl-2-(trifluoromethyl)thiophene (333 mg) and 5-hydroxy-4-methyl-1-indolinecarboxylic acid tert-butyl ester (300 mg) in dichloromethane (5.0 mL), potassium carbonate (250 mg) was added at room temperature, and the mixture was stirred for 16 hours at 50°C. The resultant mixture was cooled to room temperature. The precipitated matter was removed by filtration, and water (40 mL) and saturated aqueous ammonium chloride solution (40 mL) were added to the filtrate, then subjected to extraction with ethyl acetate (2 x 30 mL). The extracts were combined and washed with saturated brine (30 mL), followed by drying over sodium sulfate anhydrate. Solvent was removed under reduced pressure, and the residue was purified by silica gel flash column chromatography (Biotage 40M), whereby the title compound (547 mg) was yielded.
$^1$H-NMR($CDCl_3$)δ:
1.55(9H,s),2.16(3H,s),3.00(2H,t,J=8.5Hz),3.98(2H,br s), 5.19(2H,s), 6.73(1H,d,J=8.8Hz), 7.04(1H,s), 7.24(1/2 of 1H,br s), 7.35-7.45(5H,m), 7.63(1/2 of 1H,br s).
MS (ESI)m/z:512 (M+Na)$^+$.

(5) 4-Methyl-5-[[4-phenyl-5-(trifluoromethyl)-2-thienyl]methoxy]-1-indoline hydrochloride

[F589]

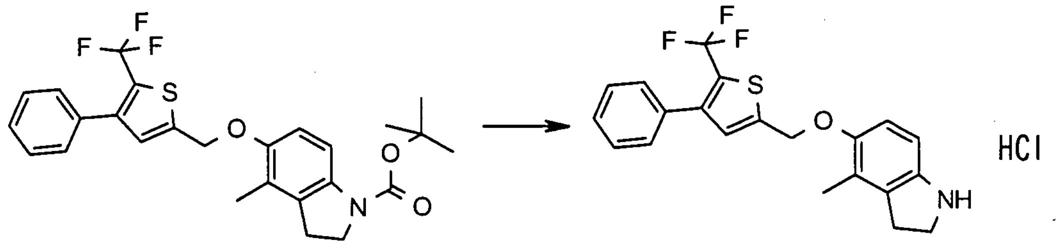

To 4-methyl-5-[[4-phenyl-5-(trifluoromethyl)-2-thienyl]methoxy]-1-indolinecarboxylic acid tert-butyl ester (547 mg), 4N HC1/1,4-dioxane (5.0 mL) was added at room temperature, and the mixture was stirred for 2 hours. The resultant mixture was concentrated under reduced pressure and slurried with hexane. The slurry was filtered, and the collected solid was dried, whereby the title compound (433 mg) was yielded. $^1$H-NMR(DMSO-d6)δ:
2.16(3H,s), 3.13(2H,t,J=7.7Hz), 3.70(2H,t,J=7.7Hz), 5.44(2H,s), 7 .09(1H,d,J=8.8Hz), 7.23(1H,d,J=8.8Hz), 7.38(1H,s), 7.40-7.52(SH,m),11.00(2H,br s). The peak at 11.00 was also attributed to HCl.
MS (ESI) m/z : 390 (M+H)$^+$.

(6) 3-[N-(tert-Butoxycarbonyl)-N-[2-[4-methyl-5-[[4-phenyl-5-(trifluoromethyl)-2-thienyl]methoxy]-2,3-dihydro-1H-indol-1-yl]-2-oxoethyl]amino]propanoic acid tert-butyl ester

[F590]

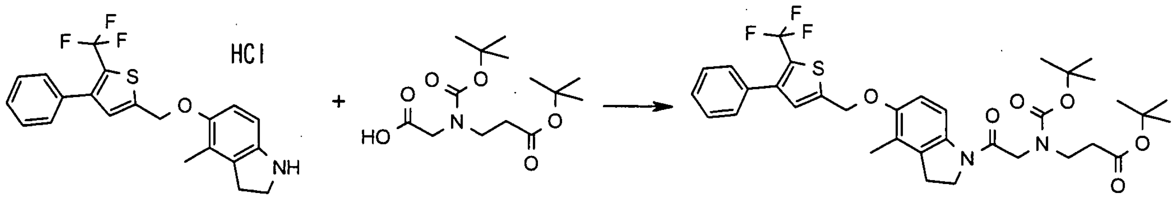

To a suspension of 4-methyl-5-[[4-phenyl-5-(trifluoromethyl)-2-thienyl]methoxy]-1-indoline hydrochloride (130 mg) and 2-[N-(tert-butoxycarbonyl)-N-[3-(tert-butoxy)-3-oxopropyl]amino]acetic acid (111 mg) in dichloromethane (3.0 mL), EDC·HCl (87.7 mg), HOBt (61.8 mg), and TEA (0.213 mL) were added at room temperature, and the mixture was stirred for 18 hours. The resultant mixture was concentrated under reduced pressure. Ethyl acetate (20 mL), saturated aqueous sodium hydrogencarbonate solution (40 mL), and water (40 mL) were added thereto for phase separation. The aqueous layer was extracted with ethyl acetate (20 mL). The extracts were combined and dried over sodium sulfate anhydrate, and solvent was removed under reduced pressure. The residue was purified by silica gel flash column chromatography (Biotage 25M), whereby the title compound (178 mg) was yielded.
$^1$H-NMR ($CDCl_3$)δ:
1.35-1.62(18H,m),2.17(2/3 of 3H,s),2.19(1/3 of 3H,s),2.54-2.63(2H,m), 3.08-3.18(2H,m), 3.54-3.62(2H,m), 4.00-4.20 (4H,m),5.20(2/3 of 2H,s),5.21(1/3 of 2H,s),6.70-6.78(1H,m), 7.05(1H,s), 7.36-7.46(5H,m), 7.95-8.05(1H,m).
MS (ESI) m/z : 675 (M+H)$^+$.

(7) 3-[N-[2-[4-Methyl-5-[[4-phenyl-5-(trifluoromethyl) -2-thienyl]methoxy]-2,3-dihydro-1H-indol-1-yl]-2-oxoethyl] amino] propionic acid

[F591]

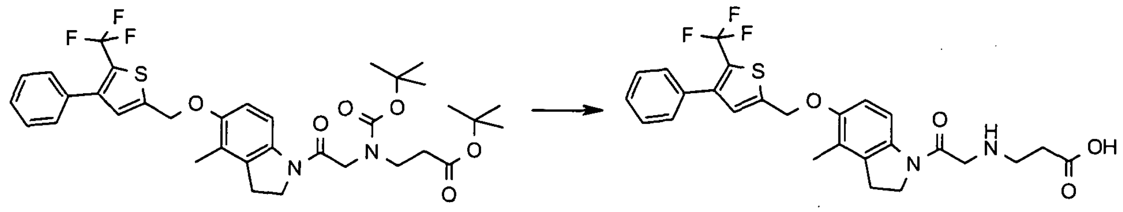

To 3-[N-(tert-butoxycarbonyl)-N-[2-[4-methyl-5-[[4-phenyl-5-(trifluoromethyl)-2-thienyl]methoxy]-2,3-dihydro-1H-indol-l-yl]-2-oxoethyl]amino]propanoic acid tert-butyl ester (176 mg), 4N HCl/1,4-dioxane (5.0 mL) was added at room temperature, and the mixture was stirred for 16 hours. The reaction mixture was concentrated under reduced pressure. Diethyl ether was added to the residue to form a slurry. The slurry was filtered, and the collected solid was purified by reverse phase high-performance liquid chromatography (column: Nomura Chemistry; Develosil Combi-RP-5 (10 cm)). The target fraction was freeze-dried, whereby the title compound (60.5 mg) was yielded.
$^1$H-NMR(DMSO-d6)δ:
2.11(3H,s), 2.37(2H,t,J=6.7Hz), 2.84(2H,t,J=6.7Hz), 3.07(2H,t,J= 8.1Hz), 3.59(2H,s), 4.05(2H,t,J=8.1Hz), 5.35(2H,s), 6.91(1H,d,J=8 .7Hz), 7.35(1H,s), 7.40-7.50(5H,m), 7.86(1H,d,J=8.7Hz). Neither $CO_2$ nor NH was observed.
IR (ATR) $cm^-$
$^1$:2951,1641,1597,1377,1288,1257,1173,1117,1099,1014,766,698.
MS (ESI) m/z : 519 $(M+H)^+$.
HR-MS(ESI)Calcd for
$C_{26}H_{26}F_3N_2O_4S$ $(M+H)^+$: 519.15654.Found: 519.15631.
Anal. Calcd for $C_{26}H_{25}F_3N_2O_4S$·
1.0H20:C,58.20;H,5.07;F,10.62;N,5.22;S,5.98.Found:C,58.07;H,4 .82;F,10.48;N,5.13;5,5.92.

[Example 127]

(R)-3-[N-(tert-Butoxycarbonyl)-N-methylamino]-4-oxo-4-[5-[(4-phenyl-5-trifluoromethyl-2-thienyl)methoxy]indolin-1-yl] butyric acid

(1) (R)-3-[N-(tert-Butoxycarbonyl)-N-methylamino]-4-oxo-4-[5-[(4-phenyl-5-trifluoromethyl-2-thienyl)methoxy]indolin-1-yl]butyric acid methyl ester

[F592]

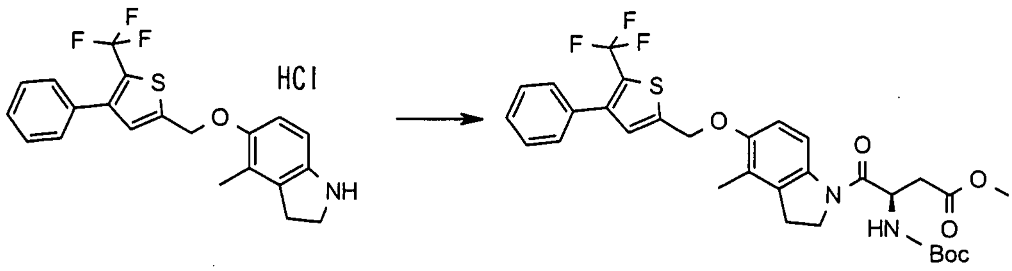

To a DMF solution (5 mL) of 1-(tert-butoxycarbonyl)-5-[(4-phenyl-5-trifluoromethyl-2-thienyl)methoxy]indoline hydrochloride (0.13 mg), Boc-D-Me-Asp (OMe)-OH (78 mg), EDC·HCl (86 mg), and HOAt (61 mg), DIEA (0.26 mL) was added at room temperature, and the mixture was stirred for 18 hours. The reaction mixture was concentrated, and the residue was purified by silica gel flash column chromatography (Biotage 25S), whereby the title compound (80 mg) was yielded.
$^1$H-NMR ($CDCl_3$)δ:
1.48(9H,s), 2.18(3H,s), 2.48-2.60(1H,m), 2.70-2.85(3H,m), 3.00-3.26(3H,m),3.57-3.75(3H,m),3.96-4.35(2H,m), 5.10-5.19 and 5.45-5.58(total 1H,each m,amide isomers), 5.21(2H,s), 6.75(1H,d,J=8.8Hz), 7.06(1H,s), 7.45-7.33 (5H, m), 8.00(1H,d,J=8.6Hz).
MS(ESI)m/z:633(M+H).

(2) (3R)-[N-(tert-Butoxycarbonyl)-N-methylamino]-4-oxo-4-[5-[(4-phenyl-5-trifluoromethyl-2-thienyl)methoxy]indolin-1-yl]butyric acid

[F593]

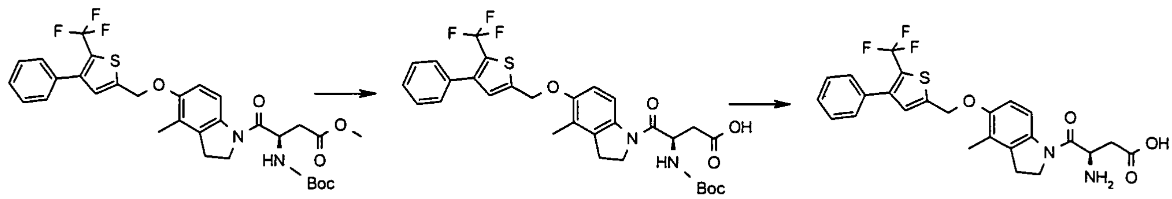

To a solution of (3R)-[N-(tert-butoxycarbonyl)-N-methylamino]-4-oxo-4-[5-[(4-phenyl-5-trifluoromethyl-2-thienyl)methoxy]indolin-1-yl]butyric acid methyl ester (80 mg) in methanol/THF (1 mL/2 mL), 1N aqueous sodium hydroxide solution (0.26 mL) was added at room temperature, and the mixture was stirred for 14 hours. The reaction mixture was concentrated, and 1N hydrochloric acid (0.13 mL) was added thereto, followed by extraction with a mixture of chloroform/methanol (10/1, v/v). The extracts were combined and washed with saturated brine, and dried over sodium sulfate anhydrate. Solvent was removed, whereby (R)-3-[(N-tert-butoxycarbonyl-N-methyl)amino]-4-oxo-4-[(4-phenyl-5-trifluoromethyl-2-thienyl)methoxy]indolin-1-yl]butyric acid methyl ester was yielded, which was subjected to a subsequent step without further purification.
To a dichloromethane solution (10 mL) of the above-obtained (R)-3-[(N-tert-butoxycarbonyl-N-methyl)amino]-4-oxo-4-[(4-phenyl-5-trifluoromethyl-2-thienyl)methoxy]indolin-1-yl]butyric acid methyl ester, TFA (0.5 mL) was added at room temperature, and the mixture was stirred for 2 days. The reaction mixture was concentrated, and 1N aqueous sodium hydroxide solution was added to the residue to adjust the pH thereof to 7. The resultant mixture was extracted with a chloroform/methanol solution (10/1, v/v), and the extract was concentrated. The residue was purified by thin layer chromatography and freeze-dried with dioxane, whereby the title compound (48 mg) was yielded.

$^1H$-NMR ($CDCl_3$) δ:
2.17(3H,s), 2.43-2.67(5H,m), 3.03-3.23(2H,m), 3.75-3.92(1H,m), 4.01-4.30(2H,m), 5.20(2H,s), 6.74(1H,s), 7.05(1H,s), 7.32-7.44(5H,m), 8.01(1H,s) IR(ATR)cm-1:2929,2848,1647,1593,1473,1377,1317,1288.
MS (ESI) m/z : 519 (M+H).
HR-MS(AqTOF)Calcd for $C_{26}H_{26}F_3N_2O_4S$: 519.1567.Found: 519.1574.

[Example 128]

(3R)-4-[5-[(4-Cyclohexyl-3-trifluoromethylphenyl)methoxy]-4-methyl]indolin-1-yl]-3-(N-methylamino)-4-oxobutyric acid

(1) (3R)-[N-(tert-Butoxycarbonyl)-N-methylamino]-4-[5-(4-cyclohexyl-3-trifluoromethylphenyl)methoxy-4-methyl]-4-methylindolin-1-yl]-4-oxobutyric acid methyl ester

[F594]

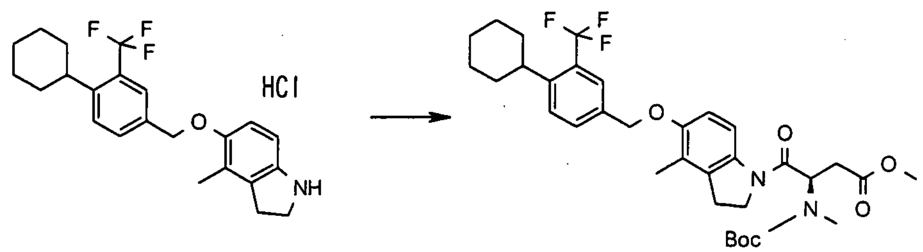

To a DMF solution (5 mL) of 5-[(4-cyclohexyl-3-trifluoromethylphenyl)methoxy]-4-methylindoline hydrochloride (187 mg, 0.44 mmol), Boc-D-Me-Asp (OMe)-OH (0.11 g), EDC·HCl (0.13 g), and HOAt (90 mg), DIEA (0.38 mL) was added at room temperature, and the mixture was stirred for 14 hours. The reaction mixture was concentrated, and the residue was purified by silica gel flash column chromatography (Biotage 25S), whereby the title compound (211 mg) was yielded.
$^1H$-NMR ($CDCl_3$)δ:
1.11-1.54(12H,m),1.67-1.93(3H,m),2.16(3H,s),2.42-2.61(1H,m), 2.70-3.25(9H,m), 3.59-3.75(5H,m), 3.94-4.36(2H,m), 5.03(2H,s),5.11-5.57(1H,m), 6.72(1H,d,J=8.8Hz), 7.46(1H,d,J=8.1Hz), 7.55(1H,d,J= 7.8Hz), 7.67(1H,s), 7.98(1H,d, J=8.3Hz).

(2) (3R)-4-[5-[(4-Cyclohexyl-3-trifluoromethylphenyl)methoxy]-4-methylindolin-1-yl]-3-(N-methylamino)-4-oxobutyric acid

[F595]

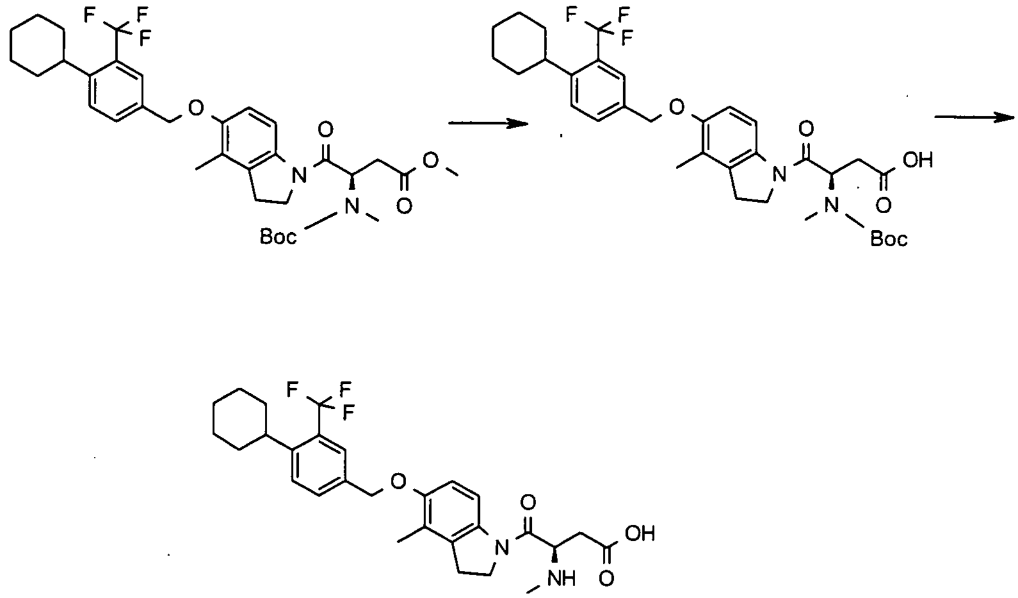

To a solution of (3R)-[N-(tert-butoxycarbonyl)-N-methylamino]-4-[5-[(4-cyclohexyl-3-trifluoromethylphenyl) methoxy]-4-methylindolin-1-yl]-4-oxobutyric acid methyl ester (0.21 g) in methanol/THF (1 mL/2 mL), 1N aqueous sodium hydroxide solution (1.0 mL) was added at room temperature, and the mixture was stirred for 14 hours. The reaction mixture was concentrated, and 1N hydrochloric acid was added thereto to adjust the pH to 2. The acidified mixture was subjected to extraction with chloroform/methanol solution (10/1, v/v). The extracts were combined, washed with saturated brine, and dried over sodium sulfate anhydrate. Solvent was removed, whereby (R)-3-[(N-tert-butoxycarbonyl-N-methyl) amino] -4-oxo-4- [5- (4-cyclohexyl-3-trifluoromethylphenyl)methoxy-4-methyl]-4-methylindolin-l-yl]butyric acid was yielded. This product was employed in a subsequent step without further purification.

To a dichloromethane solution (10 mL) of (3R)-[N-(tert-butoxycarbonyl)-N-methylamino]-4-[5-[(4-cyclohexyl-3-trifluoromethylphenyl)methoxy]-4-methylindolin-1-yl]-4-oxobutyric acid, TFA (1 mL) was added at room temperature, and the mixture was stirred for 5 hours. The reaction mixture was concentrated, and 1N aqueous sodium hydroxide solution and 1N hydrochloric acid were added to the residue to adjust the pH to 6, followed by extraction with chloroform/methanol solution (10/1, v/v). The extract was concentrated, and the residue was purified by thin layer chromatography and solidified with n-hexane. The solid was collected by filtration and dried, whereby the title compound (48 mg) was yielded. $^1$H-NMR (DMSO-$d_6$) δ:

1.23-1.88(11H,m),2.09(3H,s),2.43-2.57(6H,m),2.71-2.88(1H,m), 3.00-3.13(2H,m), 3.78-3.87(1H,m), 4.31-4.01(2H,m), 5.11(2H,s), 6.83(1H,d,J=8.8Hz), 7.63(1H,d,J=8.1Hz), 7 .68(1H,d,J=8.3Hz), 7.71(1H,s), 7.88(1H,d,J=8.8Hz).

MS (ESI) m/z : 519 $(M+H)^+$.

IR(ATR)cm$^{-1}$:2925,2854,1647,1593,1477,1375,1315,1255.

Anal. Calcd for $C_{28}H_{33}F_3N_2O_4 \cdot 0.05HCl \cdot 2H_2O$:C,60.24;H,6.70;Cl,0.64;F,10.21;N,5.02.Found:C,60.42;H,6. 40;C1,0.65; F,10.06;N,4.83.

[Example 129]

4-[5-[(4-Cyclopentyl-3-trifluoromethylphenyl)methoxy]-4-methylindolin-1-yl]-(3R)-(N-methylamino)-4-oxobutyric acid

(1) (3R)-[N-(tert-Butoxycarbonyl)-N-methylamino]-4-[5-[(4-cyclopentyl-3-trifluoromethylphenyl)methoxy]-4-methylindolin-1-yl]-4-oxobutyric acid methyl ester

[F596]

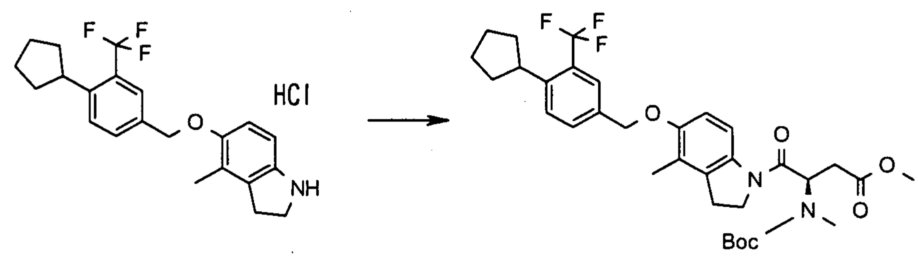

To a DMF solution (5 mL) of 5-[(4-cyclopentyl-3-trifluoromethylphenyl)methoxy]-4-methylindoline hydrochloride (0.26 g), Boc-D-Me-Asp(OMe)-OH (0.17 g), EDC·HCl (0.18 g), and HOAt (0.13 g), DIEA (0.56 mL) was added at room temperature, and the mixture was stirred for 14 hours. The reaction mixture was concentrated, and the residue was purified by silica gel flash column chromatography (Biotage 25S), whereby the title compound (174 mg) was yielded.
$^1$H-NMR($CDCl_3$)δ:
1.43-2.01(14H,m), 2.04-2.33(5H,m), 2.41-2.59(1H,m), 2.69-2.84(3H,m), 2.96-3.22(3H,m), 3.30-3.43(1H,m), 3.48-3.84 (4H,m),3.91-4.36(2H,m),5.02(2H,s),5.08-5.57(1H,m), 6.72(1H,d,J=8.6Hz), 7.47(1H,d,J=8.1Hz), 7.55(1H,d,J= 8.6Hz), 7.66(1H,s), 7.98(1H,d,J=8.6Hz).

(2) 4-[5-[(4-Cyclopentyl-3-trifluoromethylphenyl)methoxy]-4-methylindolin-1-yl]-(3R)-(N-methylamino)-4-oxobutyric acid

[F597]

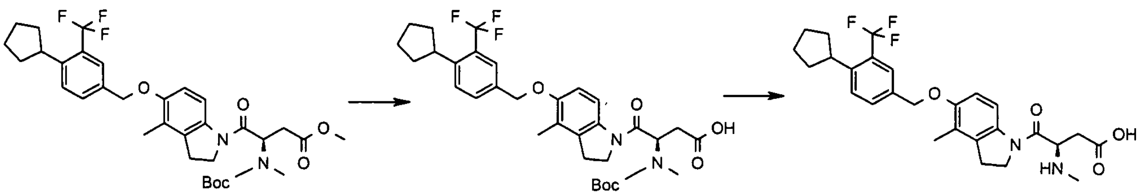

To a solutionof (3R)-[N-(tert-butoxycarbonyl)-N-methylamino]-4-[5-[(4-cyclopentyl-3-trifluoromethylphenyl) methoxy]-4-methylindolin-1-yl]-4-oxobutyric acid methyl ester (0.17 g) in methanol/THF (1/2 mL), 1N aqueous sodium hydroxide solution (1.0 mL) was added at room temperature, and the mixture was stirred for 14 hours. The resultant mixture was concentrated, and 1N hydrochloric acid was added thereto. The precipitated solid was collected by filtration and dried, whereby (3R)-[N-(tert-butoxycarbonyl)-N-methylamino]-4-[5-[(4-cyclopentyl-3-trifluoromethylphenyl)methoxy]-4-methylindolin-1-yl]-4-oxobutyric acid, which was employed in a subsequent step without further purfication.
To a dichloromethane solution (10 mL) of the above-obtained (3R)-[N-(tert-butoxycarbonyl)-N-methylamino]-4-[5-[(4-cyclopentyl-3-trifluoromethylphenyl)methoxy]-4-methylindolin-l-yl]-4-oxobutyric acid, TFA (0.5 mL) was added at room temperature, and the mixture was stirred for 7 hours. The reaction mixture was concentrated, and 1N aqueous sodium hydroxide solution and 1N hydrochloric acid were added thereto, whereby the pH thereof was adjusted to 6, followed by extraction with a chloroform/methanol solution (10/1, v/v). The extract was concentrated, and the residue was purified by thin layer chromatography. The purified residue was solidified with n-hexane, and the solid was collected by filtration and dried, whereby the title compound (66 mg) was yielded. $^1$H-NMR (DMSO-$d_6$)δ:
1.49-1.70(5H,m),1.73-2.06(5H,m),2.09(3H,s),2.42-2.63(5H,m), 3.01-3.13(2H,m), 3.17-3.31(1H,m), 3.79-3.88(1H,m), 4.06-4.35(2H,m), 5.11(2H,s), 6.83(1H,d,J=8.8Hz), 7.58-7.74(3H,m), 7.87(1H,d,J=8.8Hz).
MS (ESI) m/z : 505 (M+H).
IR(ATR)$cm^{-1}$:2954,2871,1651,1593,1475,1431,1375.
Anal. Calcd for $C_{27}H_{31}F_3N_2O_4 \cdot 2H_2O$:C,59,99;H,6.53;F,10.54;N,5.18.Found:C,60.32;H,6.22;;F,10 .28;N,4.97.

[Example 130]

3-[N-[2-[5-[4-(Cyclopentyl)-3-(trifluoromethyl)benzyloxy]-4-methylindolin-1-yl]-2-oxoethyl]amino]propionic acid

(1) 1-(tert-Butoxycarbonyl)-5-[4-(cyclopentyl)-3-(trifluoromethyl)benzyloxy]-4-methylindoline

[F598]

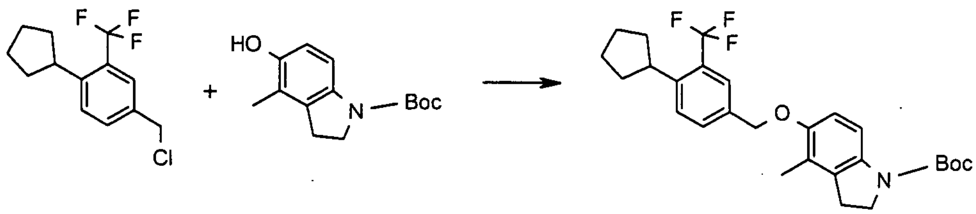

To a DMF solution (20 mL) of 4-(cyclopentyl)-3-(trifluoromethyl)benzyl chloride (0.50 g) and 1-(tert-butoxycarbonyl)-4-methyl-5-hydroxyindoline (0.47 g), potassium carbonate (0.78 g) was added at room temperature, and the mixture was heated to 70°C and stirred for 14 hours. The reaction mixture was cooled to room temperature, and insoluble matter was removed by filtration. The filtrate was concentrated, and the solid was recrystallized from ethyl acetate/ hexane, whereby the title compound (0.61 g) was yielded. $^{1}$H-NMR($CDCl_3$)δ: 1.43-1.93(15H,m),2.02-2.21(5H,m),2.95-3.09(2H,m),3.31-3.44(1H,m), 3.87-4.08(2H,m), 5.01(2H,s), 6.70(1H,d, J=8.3Hz), 7.19-7.33(1,m), 7.39-7.61(2H,m), 7.66(1H,s).

(2) 3-[N-(tert-Butoxycarbonyl)-N-[2-[5-[4-(cyclopentyl)-3-(trifluoromethyl)benzyloxy]-4-methylindolin-1-yl]-2-oxoethyl] amino]propionic acid tert-butyl ester

[F599]

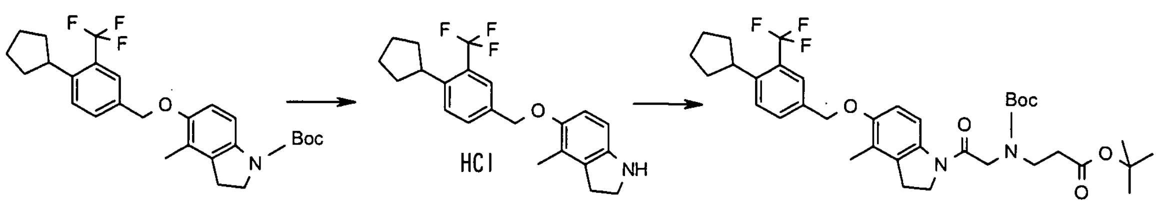

1-(tert-Butoxycarbonyl)-5-[4-(cyclopentyl)-3-(trifluoromethyl)benzyloxy]-4-methylindoline (0.61 g) was dissolved in 4N HC1/1,4-dioxane (20 mL) at room temperature, and the solution was stirred for 15 hours. The reaction mixture was concentrated, to thereby obtain 5-[(4-cyclopentyl-3-trifluoromethylphenyl)methoxy]-4-methylindoline hydrochloride (0.53 g). This product was employed in a subsequent step without further purification.
MS(ESI)m/z:376(M+H)$^{+}$.
To a DMF slution (5 mL) of the above-mentioned 5-[(4-cyclopentyl-3-trifluoromethylphenyl)methoxy]-4-methylindoline

hydrochloride (0.26 g), 3-[N-(tert-butoxycarbonyl)-N-(3-carboxymethyl)amino]propionic acid tert-butyl ester (0.19 g), EDC·HCl (0.18 g), and HOAt (0.13 g), DIEA (0.56 mL) was added at room temperature, and the mixture was stirred for 18 hours. The reaction mixture was concentrated, and the residue was purified by silica gel flash column chromatography (Biotage 25S), whereby the title compound (0.20 g) was yielded.
$^1$H-NMR ($CDCl_3$)δ:
0.87-1.91(22H,m),1.98-2.28(6H,m),2.47-2.66(3H,m),2.98-3.16(2H,m),3.33-3.42(1H,m),3.52-3.66(2H,m),3. 4.24(4H, m), 5.01(2H,s), 6.63-6.78(1H,m), 7.43-7.59(1H,m), 7.67(1H,s), 8.05-7.91(2H,m).
MS (ESI) m/z : 661 (M+H)$^+$.

(3) 3-[N-[2-[5-(4-Cyclopentyl-3-trifluoromethylbenzyloxy)-4-methylindolin-1-yl]-2-oxoethyl]amino]propionic acid

[F600]

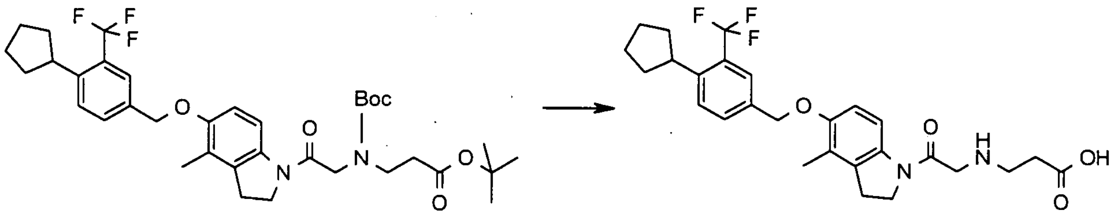

To a dichloromethane solution (10 mL) of 3-[N-(tert-butoxycarbonyl)-N-[2-[5-[4-(cyclopentyl)-3-(trifluoromethyl) benzyloxy]-4-methylindolin-1-yl]-2-oxoethyl]amino]propionic acid tert-butyl ester (0.20 g), TFA (0.5 mL) was added at room temperature, and the mixture was stirred for 20 hours. TFA (0.5 mL) was further added to the reaction mixture, and the resultant mixture was stirred for 4 hours and concentrated. To this concentrated mixture, 1N aqueous sodium hydroxide solution was added to adjust the pH thereof to 7. The pH-adjusted mixture was extracted with a chloroform/ methanol (10/1, v/v) mixture. The extracts were combined and washed with saturated brine, followed by drying over sodium sulfate anhydrate. Solvent was removed, and the solid was collected by filtration and washed with hexane, whereby the title compound (0.20 g) was yielded.
$^1$H-NMR (DMSO-$d_6$)δ:
1.50-1.73(4H,m),1.73-2.00(4H,m), 2.11(3H,s), 2.67(2H,t,J=7.0Hz), 3.04-3.18(4H,m),3.17-3.40(2H,m),4.00-4.11(4H,m), 5.13(2H,s), 6.88(1H,d,J=8.8Hz), 7.61-7.73(3H,m), 7.82(1H,d,J=8.8Hz).
IR(ATR)cm-1:3055,2925,2852,2224,1732-,1631,1604.
MS (ESI) m/z : 505 (M+H)$^+$.

[Example 131]

4-[5-(4-Cyclopropyl-3-trifluoromethylbenzyloxy)-4-methyl-2,3-dihydro-1H-indol-1-yl]-(3R)-(methylamino)-4-oxobutyric acid

(1) 5-(4-Cyclopropyl-3-trifluoromethylbenzyloxy)-4-methyl-2,3-dihydro-1H-indole-1-carboxylic acid tert-butyl ester

[F601]

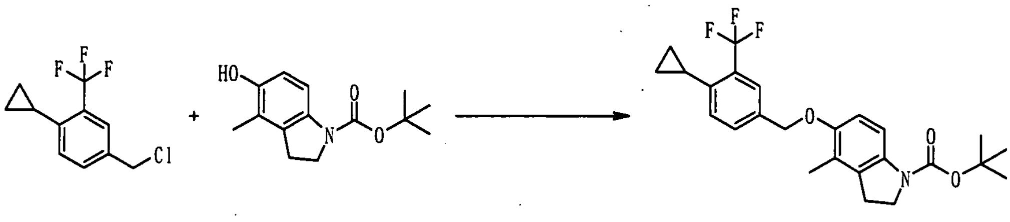

5-Hydroxy-4-methyl-2,3-dihydro-1H-indole-1-carboxylic acid tert-butyl ester (0.69 mmol), potassium carbonate (0.29 g), 4-chloromethyl-1-cyclopropyl-2-trifluoromethylbenzene (0.16 g), and dichloromethane (7 mL) were mixed, to thereby form a suspension. The reaction mixture was stirred overnight at 80°C, and the resultant mixture was cooled to room temperature and concentrated. The residue was purified by silica gel column chromatography, whereby the title compound (0.21 g) was yielded.
$^1$H-NMR ($CDCl_3$)δ:
0.75-0.79(2H,m),1.03-1.05(2H,m),1.54(9H,s),2.14(3H,s),2.18-2.21(1H,m), 2.99(2H,t,J=8.5Hz), 3.96-3.99(2H,m), 5.00 (2H,s), 6.68(1H,d,J=8.3Hz), 7.04(1H,d,J=8.1Hz), 7 .48(1H,d,J=8.3Hz), 7.53-7.66(1H,m), 7.68(1H,s).

(2) 5-(Cyclopropyl-3-trifluoromethylbenzyloxy)-4-methyl-2,3-dihydro-1H-indole hydrochloride

[F602]

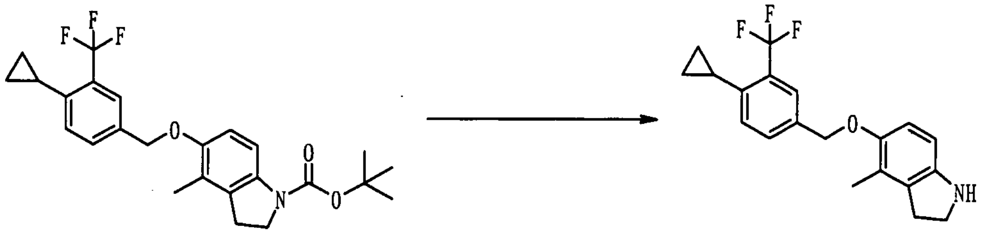

To 5-(4-cyclopropyl-3-trifluoromethylbenzyloxy)-4-methyl-2,3-dihydro-1H-indole-1-carboxylic acid tert-butyl ester (0.21 g), 4N HCl/1,4-dioxane (5 mL) was added, and the mixture was stirred for 4 hours at room temperature. The reaction mixture was concentrated, whereby the target hydrochloride (0.20 g) was yielded.

(3) (3R)-[N-(tert-Butoxycarbonyl)-N-methylamino]-4-[5-(4-cyclopropyl-3-trifluoromethylbenzyloxy)-4-methyl-2,3-dihydro-1H-indol-1-yl]-4-oxobutyric acid methyl ester

[F603]

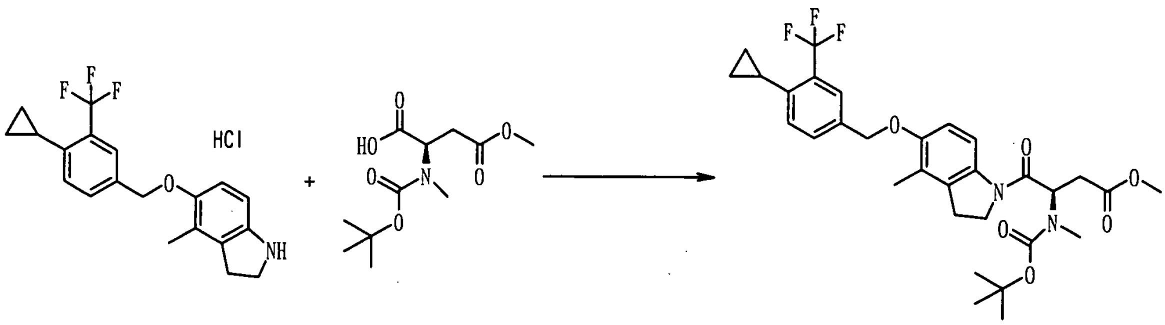

Dichloromethane (5 mL) was added to a mixture of 5-(cyclopropyl-3-trifluoromethylbenzyloxy)-4-methyl-2,3-dihydro-1H-indole hydrochloride (0.20 g), HOAt (0.10 g), EDC·HCl (0.15 g), DIEA (0.44 mL), and (2R)-[N-(tert-butoxycarbonyl)-N-methylamino]succinic acid 4-methyl ester (0.13 g), and the resultant mixture was stirred overnight, followed by concentration. Saturated aqueous sodium bicarbonate solution was added thereto, and the resultant mixture was extracted thrice, each with ethyl acetate. The extracts were combined and washed with saturated brine, followed by drying over sodium sulfate. Insoluble matter was removed by filtration, and the filtrate was concentrated. The residue was purified by silica gel column chromatography, whereby the title compound (0.23 g) was yielded.
$^1$H-NMR($CDCl_3$)δ :
0.75-0.80(2H,m),1.02-1.07(2H,m),1.47(9H,s),2.16-2.23(4H,m), 2.52-2.56(1H,m), 2.76-2.79(3H,m), 3.07-3.18(3H,m),3.66-3.73(4H,m),4.11-4.20(2H,m), 5.03(2H,s), 6.71(1H,d,J=8.6Hz), 7.05(1H,d,J=8.1Hz), 7 .47-7.48(1H,m), 7.68(1H,s), 7.97(1H,d,J=8.8Hz).
MS (ESI) ;m/z:591 $(M+H)^+$.

(4) (3R) - [N- (tert-Butoxycarbonyl) -N-methylamino] -4- [5- (4-cyclopropyl-3-trifluoromethylbenzyloxy)-4-methyl-2,3-dihydro-1H-indol-1-yl]-4-oxobutyric acid

[F604]

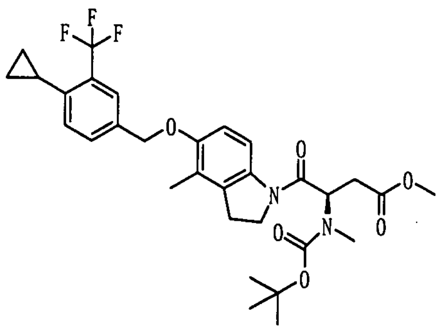

THF (3 mL) and water (3 mL) were added to (3R)-[N-(tert-butoxycarbonyl)-N-methylamino]-4-[5-(4-cyclopropyl-3-trifluoromethylbenzyloxy)-4-methyl-2,3-dihydro-1H-indol-1-yl]-4-oxobutyric acid methyl ester (0.23 g), and lithium hydroxide (0.030 g), and the mixture was stirred overnight at room temperature. The reaction mixture was concentrated, and water was added to the residue. The resultant product was extracted thrice, each with a chloroform-methanol mixture (methanol/chloroform = 10%). The extracts were combined and washed with saturated brine, followed by drying over sodium sulfate. Insoluble matter was removed by filtration, and the filtrate was concentrated. The residue was purified by silica gel column chromatography, whereby the title compound (0.20 g) was yielded. $^1$H-NMR ($CDCl_3$)$\delta$ :
0.72-0.80(2H,m),0.97-1.07(2H,m),1.33-1.47(9H,m),2.04-2.23(4H,m), 2.55-2.58(1H,m), 2.73-2.78(3H,m), 3.02-3.21(3H, m),3.99-4.24(2H,m),4.98-5.01(2H,m),5.46-5.47(1H,m), 6.70-6.73(1H,m), 7.04-7.05(1H,m), 7.48(1H,d,J=8.3Hz), 7.68 (1H,s), 7.97(1H,d,J=8.6Hz).
MS (ESI) ; m/z : 577 $(M+H)^+$.

(5) 4-[5-(4-Cyclopropyl-3-trifluoromethylbenzyloxy)-4-methyl-2,3-dihydro-1H-indol-1-yl]-(3R)-(methylamino)-4-oxobutyric acid

[F605]

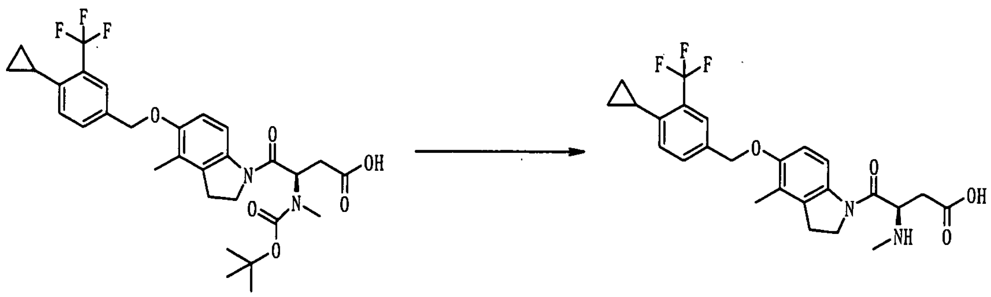

To (3R)-[N-(tert-butoxycarbonyl)-N-methylamino]-4-[5-(4-cyclopropyl-3-trifluoromethylbenzyloxy)-4-methyl-2,3-dihydro-1H-indol-1-yl]-4-oxobutyric acid (0.20 g), 10% TFA/dichloromethane solution (3 mL) was added, and the mixture was stirred overnight at room temperature. The reaction mixture was concentrated, whereby the title compound

(0.14 g) was yielded.
$^1$H-NMR($CD_3OD$)δ :
0.78-0.79(2H,m),1.03-1.05(2H,m),2.17(4H,s),2.75(3H,s),2.85-2.92(1H,m), 3.09-3.22(3H,m), 4.20-4.33(2H,m), 4.57(1H, dd,J=8.0,4.6Hz), 5.09(2H,s), 6.83(1H,d,J=8.9 Hz), 7.13(1H,d,J=7.9Hz), 7.56(1H,d,J=7.9Hz), 7.70(1H,s), 7.95(1H, d, J=8.9Hz).
MS (ESI) ; m/z:477(M+H)$^+$.
Anal. Calcd for
$C_{25}H_{27}F_3N_2O_4 \cdot CF_3CO_2H$,0.25$H_2O$:C,54.50;H,4.83;N,4.71;F,19.16.Found:C,54.44;H, 4.70;N,4.66;F,19.48.
IR(ATR)cm$^{-1}$:1655,1479,1182,1120,823,717.

[Example 132]

(3R)-(N-Methylamino)-4-oxo-4-[5-[(1-phenyl-5-trifluoromethyl-1H-pyrazol-3-yl)methoxy]-4-methyl-2,3-dihydro-1H-indol-1-yl] butyric acid

(1) 4-Methyl-5-[(1-phenyl-5-trifluoromethyl-1H-pyrazol-3-yl)methoxy]indoline-1-carboxylic acid tert-butyl ester

[F606]

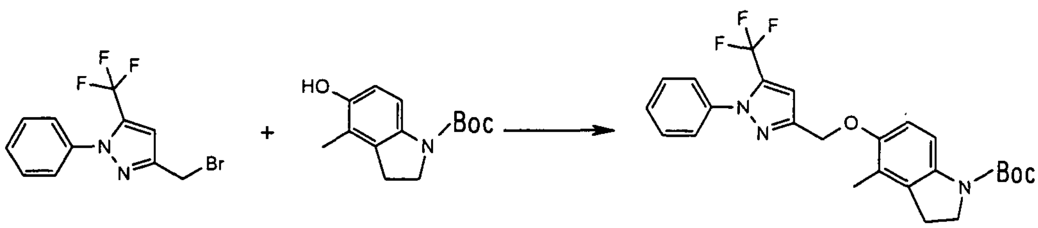

To a DMF solution (60 mL) of 3-bromomethyl-1-phenyl-5-trifluoromethyl-1H-pyrazole (100 mg), potassium carbonate (181 mg) and 5-hydroxy-4-methylindoline-1-carboxylic acid tert-butyl ester (90 mg) were added, and the mixture was stirred for 3 days at 70°C. The reaction mixture was left to stand to cool to room temperature and diluted with water.
The aqueous layer was extracted with ethyl acetate. The extracts were combined and washed with saturated brine, followed by drying over magnesium sulfate anhydrate. Solvent was removed under reduced pressure. The residue was purified by silica gel column flash chromatography, whereby the title compound (81 mg) was yielded.
$^1$H-NMR ($CDCl_3$)δ :
1.26(9H,s), 2.16(3H,s), 3.00(2H,t,J=8.5Hz), 3.96-3.98(2H,m), 5.11(2H,s), 6.78(1H,d,J=8.5Hz), 6.90(1H,s), 7.46-7.57 (6H,m).

(2) 4-Methyl-5-[(1-phenyl-5-trifluoromethyl-1H-pyrazol-3-yl)methoxy]indoline

[F607]

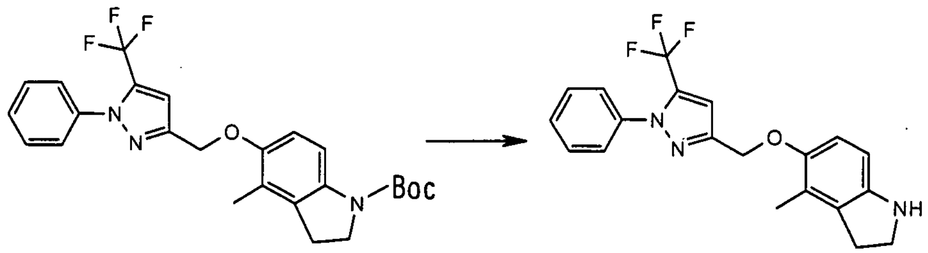

4-Methyl-5-[(1-phenyl-5-trifluoromethyl-1H-pyrazol-3-yl)methoxy]indoline-1-carboxylic acid tert-butyl ester (79 mg) was dissolved in 4N HCl/dioxane (4.0 mL), and the solution was stirred for 2 hours at room temperature. After evaporation of solvent, the resultant mixture was diluted with saturated aqueous sodium hydrogencarbonate solution. The aqueous layer was extracted with chloroform. The extracts were combined and washed with saturated brine, followed by drying over magnesium sulfate anhydrate. The mixture was subjected to filtration, and the filtrate was concentrated, whereby a crude product of the title compound (64 mg) was yielded.
MS(ESI)m/z:374(M+H)$^+$.

(3) (3R)-[N-(tert-Butoxycarbonyl)-N-methylamino]-4-oxo-4-[5-[(1-phenyl-5-trifluoromethyl-1H-pyrazol-3-yl)methoxy]-4-methyl-2,3-dihydro-1H-indol-1-yl]butyric acid methyl ester

[F608]

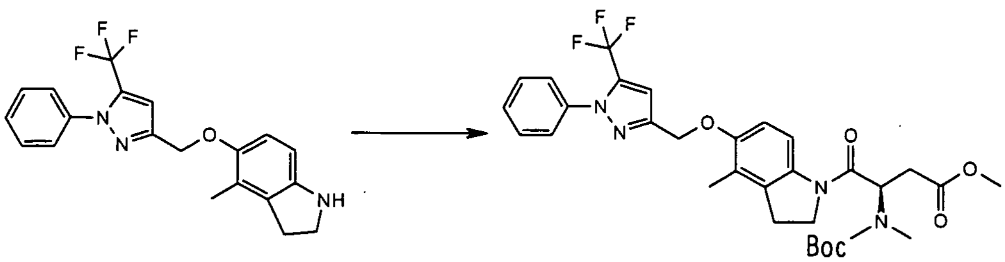

To a DMF solution (5.0 mL) of 5-[(1-phenyl-5-trifluoromethyl-1H-pyrazol-3-yl)methoxy]-4-methyl-indoline (crude product) (62 mg), N-(tert-butoxycarbonyl)-N-methyl-D-aspartic acid 4-methyl ester (52 mg), EDC·HCl (38 mg), HOBt (27 mg), and DIEA (85 μL) were added, and the mixture was stirred overnight at room temperature. Saturated aqueous sodium hydrogencarbonate solution was added to the reaction mixture. The resultant mixture was extracted with ethyl acetate. The extracts were combined and washed with saturated brine, followed by drying over magnesium sulfate anhydrate. The dried product was filtered, and the filtrate was concentrated. The residue was purified by silica gel column flash chromatography, whereby the title compound (51 mg) was yielded.

(4) (3R)-[N-(tert-Butoxycarbonyl)-N-methylamino]-4-oxo-4-[5-[(1-phenyl-5-trifluoromethyl-1H-pyrazol-3-yl)methoxy]-4-methyl-2,3-dihydro-1H-indol-1-yl]butyric acid

[F609]

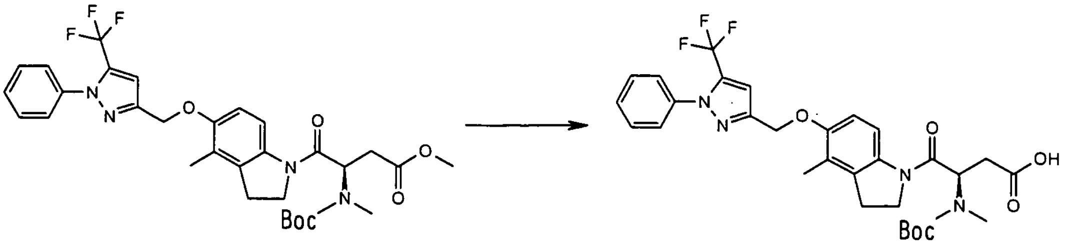

(3R)-[N-(tert-butoxycarbonyl)-N-methylamino]-4-oxo-4-[5-[(1-phenyl-5-trifluoromethyl-1H-pyrazol-3-yl)methoxy]-4-methyl-2,3-dihydro-1H-indol-1-yl]butyric acid methyl ester (51 mg) was dissolved in a 50% methanol/THF solvent (4.0 mL), and 1N aqueous sodium hydroxide solution (2.0 mL) was added thereto. The mixture was stirred overnight at room temperature. 1N Hydrochloric acid was added to the reaction mixture, to thereby adjust the pH thereof to about 6. Water was added to the pH-adjusted mixture, and the aqueous layer was extracted with a 10% methanol/chloroform solvent. The extracts were combined and washed with saturated brine, followed by drying over magnesium sulfate anhydrate and filtration. The filtrate was concentrated, and the residue was purified by preparative thin-layer silica gel chromatography, whereby the title compound (42 mg) was yielded. $^1$H-NMR ($CDCl_3$)$\delta$:
1.48(9H,s), 2.18(3H,s), 2.60-2.62(1H,m), 2.78-2.81(3H,m), 3.14-3.16(3H,m),4.08-4.19(2H,m),5.03-5.50(1H,m), 5.14 (2H,s), 6.82(1H,d,J=8.8Hz), 6.89(1H,s), 7.49(5H,s
), 7.99(1H,d,J=8.8Hz).

(5) (3R)-(N-Methylamino)-4-oxo-4-[5-[(1-phenyl-5-trifluoromethyl-1H-pyrazol-3-yl)methoxy]-4-methyl-2,3-dihydro-1H-indol-1-yl]butyric acid

[F610]

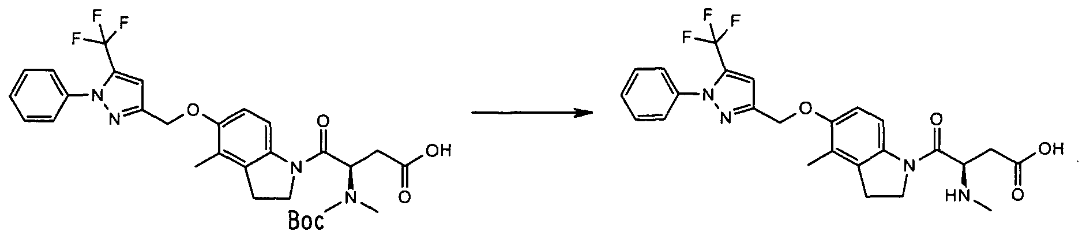

(3R)-[N-(tert-Butoxycarbonyl)-N-methylamino]-4-oxo-4-[5-[(1-phenyl-5-trifluoromethyl-1H-pyrazol-3-yl)methoxy]-4-methyl-2,3-dihydro-1H-indol-1-yl]butyric acid (41 mg) was dissolved in a 10% TFA/dichloromethane solution (5.0 mL), and the solution was stirred for 1 hour at room temperature. Solvent of the resultant mixture was evaporated, and the residue was purified by preparative reverse phase HPLC, whereby the title compound (28 mg) was yielded.
$^1$H-NMR ($CD_3OD$)$\delta$:
2.17(3H,s), 2.55(1H,dd,J=16.9,10.2Hz), 2.72(3H,s), 2.78(1H,dd,J= 16.9,3.6Hz), 3.19(2H,t,J=8.9Hz), 4.18(1H,q, J=8.9Hz), 4.30(1H,q,J =8.9Hz), 4.41(1H,dd,J=10.2,3.6Hz), 5.15(2H,s), 6.91(1H,d,J=8.9Hz ), 7.04(1H,s), 7.49-7.51(2H, m), 7.55-7.56(3H,m), 7.97(1H,d,J=8.9Hz).
IR (ATR) $cm^{-1}$:1649,1597,1473,1392,1261,1225,1182,1124,1099,1084,823,690.
MS (ESI) m/z : 503 $(M+H)^+$.
HR-MS(ESI) :Calcd for $C_{25}H_{26}F_3N_4O_4$ $(M+H)^+$:503.19061.Found:503.18967.
Anal. Calcd for $C_{25}H_{25}F_3N_4O_4 \cdot H_2O$:C,57.69;H,5.23;F,10.95;N,10.76.Found:C,57.81;H,5.10;F,10. 74;N,10.50.

[Example 133]

4-[5-(4-Cyclobutyl-3-trifluoromethylbenzyloxy)-4-methylindolin-1-yl]-(3R)-(N-methylamino)-4-oxobutyric acid TFA salt

(1) 5-(4-Cyclobutyl-3-trifluoromethylbenzyloxy)-4-methylindoline-1-carboxylic acid tert-butyl ester

[F611]

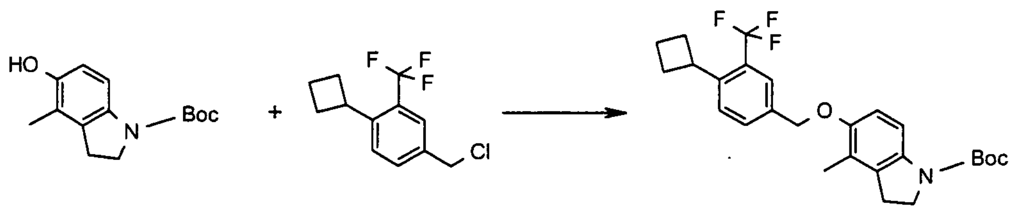

To a dichloromethane solution (5.0 mL) of 5-hydroxy-4-methylindoline-1-carboxylic acid tert-butyl ester (125 mg), 4-chloromethyl-l-cyclobutyl-2-trifluoromethylbenzene (162 mg) and potassium carbonate (104 mg) were added, and the mixture was stirred overnight at 70°C and left to stand to cool to room temperature. Saturated aqueous sodium bicarbonate solution was added to the reaction mixture. The resultant mixture was extracted thrice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column L), whereby the title compound (195 mg) was yielded.
$^1$H-NMR($CDCl_3$)δ:
1.55(9H,s),1.82-2.39(9H,m),2.99(2H,t,J=8.5Hz),3.85-3.98(3H,m), 5.02(2H,s), 6.69(1H,d,J=8.5Hz), 7.58-7.66(4H,m).

(2) (3R)-[N-(tert-Butoxycarbonyl)-N-methylamino]-4-[5-(4-cyclobutyl-3-trifluoromethylbenzyloxy)-4-methylindolin-1-yl]-4-oxobutyric acid methyl ester

[F612]

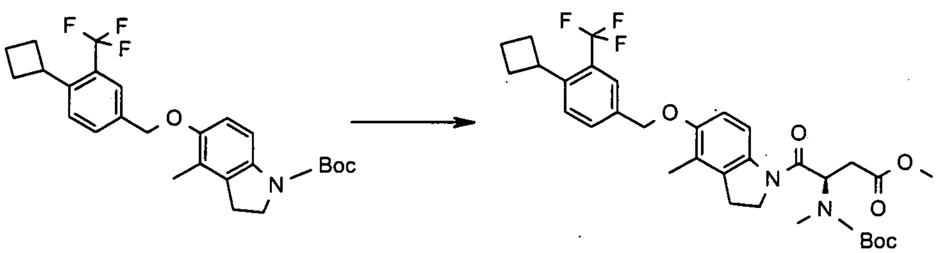

4N HCl/1,4-dioxa (10 mL) was added to 5-(4-cyclobutyl-3-trifluoromethylbenzyloxy)-4-methylindoline-1-carboxylic acid tert-butyl ester (190 mg), and the mixture was stirred for 3.5 hours at room temperature. The reaction mixture was concentrated under reduced pressure. The residue was dissolved in dichloromethane (10 mL), and (R)-2-[N-(tert-butoxycarbonyl)-N-methylamino]succinic acid 4-methyl ester (136 mg), DIEA (350 μL), HOBt (83.5 mg), and EDC-HCl (118 mg) were added to the solution. The resultant mixture was stirred overnight at room temperature, and saturated aqueous sodium bicarbonate solution was added thereto. The resultant mixture was extracted thrice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column L), whereby the title compound (235 mg) was

yielded.
$^{1}$H-NMR ($CDCl_3$)δ :
1.43-1.52(9H,m), 1.83-2.39(9H,m), 2.51-2.57(1H,m), 2.77-3.18(6H,m),3.66-4.27(6H,m),5.04(2H,s),5.16-5.52(1H,m), 6.72(1H,d,J=8.8Hz), 7.59(2H,s), 7.66(1H,s), 7.97(1H,d , J=8.8Hz).

(3) (3R)-[N-(tert-Butoxycarbonyl)-N-methylamino]-4-[5-(4-cyclobutyl-3-trifluoromethylbenzyloxy)-4-methylindolin-1-yl]-4-oxobutyric acid

[F613]

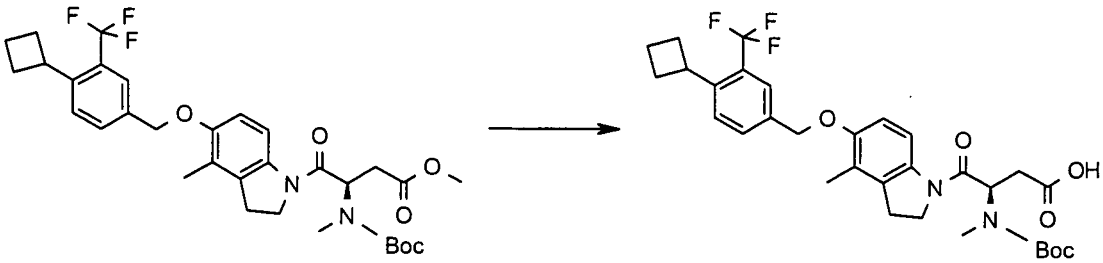

To a THF solution (5.0 mL) of (3R)-[N-(tert-butoxycarbonyl) -N-methylamino] -4- [5- (4-cyclobutyl-3-trifluoromethylbenzyloxy)-4-methylindolin-1-yl]-4-oxobutyric acid methyl ester (230 mg), methanol (1.14 mL) and 1N aqueous sodium hydroxide solution (1.14 mL) were added, and the mixture was stirred for 1 hour at room temperature. 1N Hydrochloric acid was added to the reaction mixture, and the resultant mixture was extracted thrice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column L), whereby the title compound (206 mg) was yielded. $^{1}$H-NMR ($CDCl_3$)δ:
1.47(9H,s), 1.82-2.38(9H,m), 2.55-2.60(1H,m), 2.77-2.80(3H,m),3.06-3.24(3H,m),3.85-4.23(3H,m),5.04-5.07(2H,m), 5.45-5.48(1H,m),6.72(1H,d,J=8.8Hz),7.58-7.66(3H,m), 7.97(1H,d,J=8.8Hz).

(4) 4-[5-(4-Cyclobutyl-3-trifluoromethylbenzyloxy)-4-methylindolin-1-yl]-(3R)-(N-methylamino)-4-oxobutyric acid TFA salt

[F614]

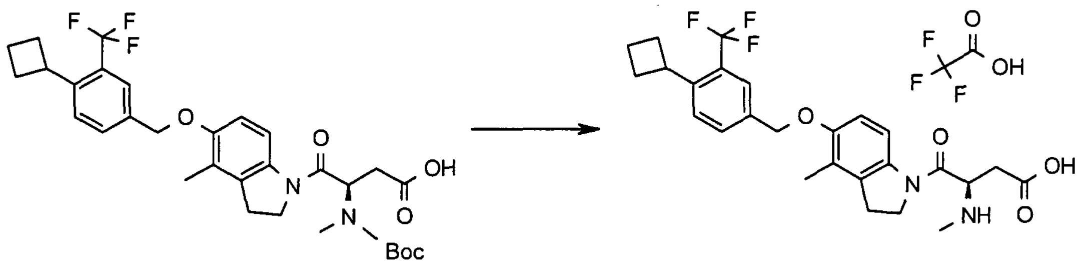

TFA (5.0 mL) was added to a dichloromethane solution (5.0 mL) of (3R)-[N-(tert-butoxycarbonyl)-N-methylamino]-4-[5-(4-cyclobutyl-3-trifluoromethylbenzyloxy)-4-methylindolin-1-yl]-4-oxobutyric acid (200 mg), and the mixture

was stirred for 1.5 hours at room temperature. The reaction mixture was concentrated under reduced pressure. A mixture of diethyl ether and diisopropyl ether was added to the residue, and the precipitated matter was collected by filtration and dried, whereby the title compound (147 mg) was yielded.
$^1$H-NMR (DMSO-$d_6$) δ:
1.80-1.87(1H,m),1.94-2.31(8H,m), 2.49(3H,s), 2.63(1H,dd,J=7.3,17.1Hz), 2.89(1H,dd,J=5 .6,17.1Hz), 3.11(2H,t, J=8.2Hz), 3.75-3.83(1H,m), 4.13-4.35(3H,m), 5.16(2H,s), 6.88(1H,d,J=9.0Hz), 7.75(3H,t,J=7.9Hz), 7 .88(1H,d, J=9.0Hz).
IR(ATR)cm$^{-1}$:2944,1652,1475,1315,1255,1197,1162,1116,1056.
MS (ESI) m/z : 491 $(M+H)^+$.
HR-MS(ESI)calcd for $C_{26}H_{30}F_3N_2O_4$ $(M+H)^+$:491 .21577; found 491.21224.

[Example 134]

4-[5-(4-Isopropyl-3-trifluoromethoxybenzyloxy)-4-methylindolin-1-yl]-(3R)-(N-methylamino)-4-oxobutyric acid TFA salt

(1) 5-(4-Isopropyl-3-trifluoromethoxybenzyloxy)-4-methylindoline-1-carboxylic acid tert-butyl ester

[F615]

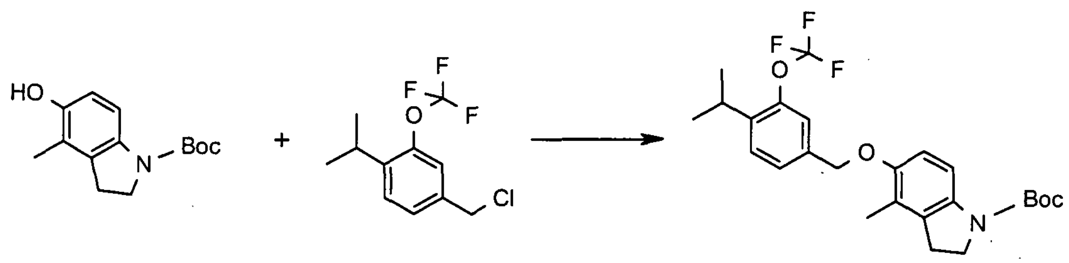

To a dichloromethane solution (5.0 mL) of 5-hydroxy-4-methylindoline-1-carboxylic acid tert-butyl ester (125 mg), 4-chloromethyl-1-isopropyl-2-trifluoromethoxybenzene (152 mg) and potassium carbonate (104 mg) were added, and the mixture was stirred overnight at 70°C. The reation mixture was left to stand to cool to room temperature, and saturated aqueous sodium bicarbonate solution was added thereto. The resultant mixture was extracted thrice, each with ethyl acetate. The extracts were combined and washed with saturated brine, followed by drying over sodium sulfate anhydrate and filtration. The filtrate was concentrated under reduced pressure. The residue was purified by flash column chromatography (Yamazen Hi-Flash column L), whereby the title compound (196 mg) was yielded. $^1$H-NMR ($CDCl_3$) δ : 1.24(6H,d,J=6.8Hz), 1.55(9H,s), 2.15(3H,s), 3.00(2H,t,J=8.5Hz),3 .27-3.37(1H,m), 3.98(2H,s),4.99(2H,s), 6.70(1H,d, J=8.5Hz), 7.30-7.61 (4H,m) .

(2) (3R)-[N-(tert-Butoxycarbonyl)-N-methylamino]-4-[5-(4-isopropyl-3-trifluoromethoxybenzyloxy)-4-methylindolin-1-yl]-4-oxobutyric acid methyl ester

[F616]

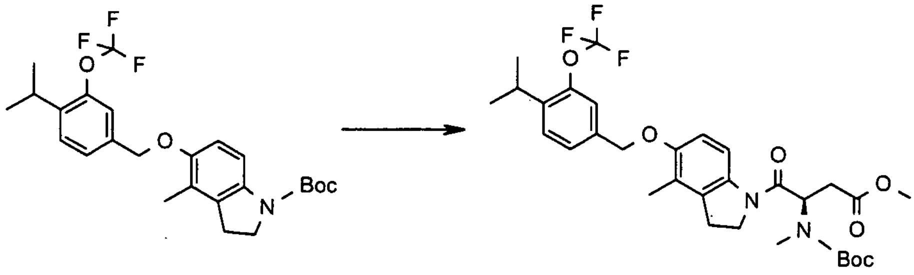

4N HCl/1,4-dioxane (10 mL) was added to 5-(4-isopropyl-3-trifluoromethoxybenzyloxy)-4-methylindoline-1-carboxylic acid tert-butyl ester (190 mg), and the mixture was stirred for 3.5 hours at room temperature. The reaction mixture was concentrated under reduced pressure. The residue was dissolved in dichloromethane (10 mL), and (2R)-[N-(tert-butoxycarbonyl)-N-methylamino]succinic acid 4-methyl ester (135 mg), DIEA (347 μL), HOBt (82.7 mg), and EDC·HCl (117 mg) were added to the solution. The resultant mixture was stirred overnight at room temperature. Saturated aqueous sodium bicarbonate solution was added to the reaction mixture, and the resultant mixture was extracted thrice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column L), whereby the title compound (248 mg) was yielded.

$^1$H-NMR ($CDCl_3$) δ :

1.24(6H,d,J=7.1Hz), 1.43-1.53(9H,m), 2.17(3H,s), 2.51-2.57(1H,m), 2.77-3.35(7H,m), 3.66-3.74(3H,m), 4.03-4.31(2H,m), 5.01(2H,s),5.14-5.52(1H,m), 6.72(1H,d,J=8.8Hz), 7.29-7.35(3H,m), 7.97(1H,d,J=8.8Hz).

(3) (3R)-[N-(tert-Butoxycarbonyl)-N-methylamino]-4-[5-(4-isopropyl-3-trifluoromethoxybenzyloxy)-4-methylindolin-1-yl]-4-oxobutyric acid

[F617]

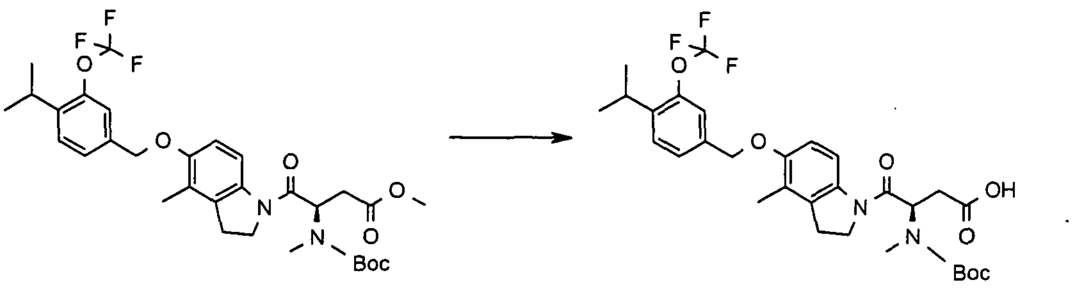

To a THF solution (5.0 mL) of (3R)-[N-(tert-butoxycarbonyl)-N-methylamino]-4-[5-(4-isopropyl-3-trifluoromethoxybenzyloxy)-4-methylindolin-1-yl]-4-oxobutyric acid methyl ester (240 mg), methanol (1.18 mL) and 1N aqueous sodium hydroxide solution (1.18 mL) were added, and the mixture was stirred for 1.5 hours at room temperature. 1N Hydrochloric acid was added to the reaction mixture, and the resultant mixture was extracted thrice, each with ethyl acetate. The extracts were combined and washed with saturated brine, followed by drying over sodium sulfate anhydrate. Insoluble matter was removed by filtration, and the filtrate was concentrated under reduced pressure. The residue was purified by flash column chromatography (Yamazen Hi-Flash column 2L), whereby the title compound (209 mg) was

yielded.
$^1$H-NMR ($CDCl_3$) δ :
1.24(6H,d,J=7.1Hz), 1.47(9H,s), 2.17(3H,s), 2.55-2.60(1H,m), 2.77-2.80(3H,m), 3.06-3.35(4H,m), 3.99-4.27 (2H,m), 5.01 (2H, s), 5.45-5.49(1H,m), 6.72(1H,d,J=8.8Hz), 7.30-7.35(3H,m), 7.98(1H,d,J=8.8Hz).

(4) 4-[5-(4-Isopropyl-3-trifluoromethoxybenzyloxy)-4-methylindolin-1-yl]-(3R)-(N-methylamino)-4-oxobutyric acid TFA salt

[F618]

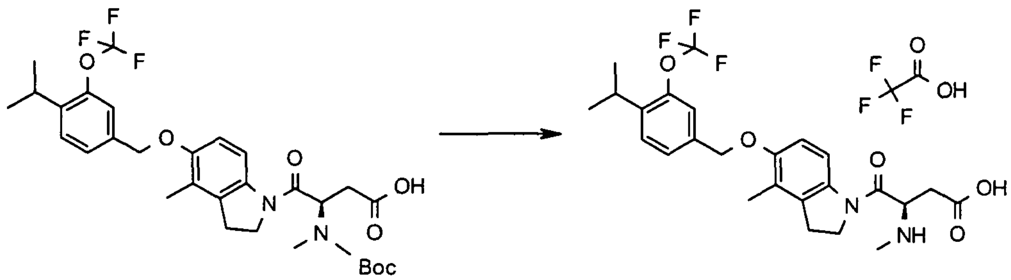

TFA (5.0 mL) was added to a dichloromathane solution (5.0 mL) of (3R)-[N-(tert-butoxycarbonyl)-N-methylamino]-4-[5-(4-isopropyl-3-trifluoromethoxybenzyloxy)-4-methylindolin-1-yl]-4-oxobutyric acid (200 mg), and the mixture was stirred for 1.5 hours at room temperature. The reaction mixture was concentrated under reduced pressure, and a mixture of diethyl ether and diisopropyl ether was added to the residue. The precipitated matter was collected by filtration and dried, whereby the title compound (97 mg) was yielded.
$^1$H-NMR (DMSO-$d_6$) δ:
1.19(6H,d,J=6.8Hz), 2.11(3H,s), 2.50(3H,s), 2.66(1H,dd,J=7.1,17. 1Hz),2.92(1H,dd,J=5.6,17.3Hz), 3.10(2H,t,J=8.2Hz), 3.17-3.24(1H,m), 4.11-4.18(1H,m), 4.27-4.35(2H,m), 5.10(2H,s), 6.87(1H,d,J=8.8Hz), 7.37-7.51(3H,m), 7.87(1H,d, J=8.8Hz).
IR(ATR)$cm^{-1}$:2967,1654,1477,1245,1201,1151,1087.
MS (ESI) m/z :495 $(M+H)^+$. HR-MS(ESI)calcd for $C_{25}H_{30}F_3N_2O_5$ $(M+H)^+$:495.21068; found 495.20701.

[Example 135]

4-[5-[[4-Cyclohexyl-3-(N,N-dimethylamino)phenyl]methoxy]-4-methylindolin-1-yl]-(3R)-(N-methylamino)-4-oxobutyric acid

(1) 1-(tert-Butoxycarbonyl)-5-[[4-cyclohexyl-3-(N,N-dimethylamino)phenyl]methoxy]-4-methylindoline

[F619]

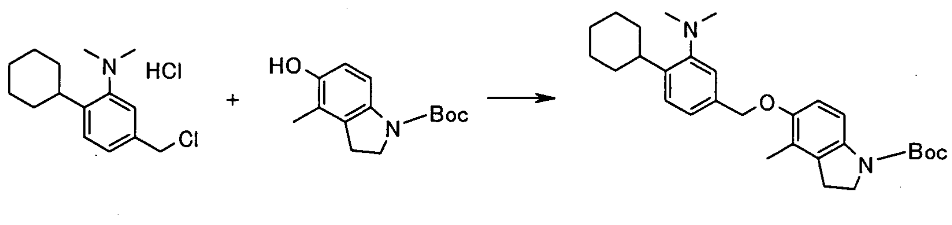

To a solution of 4-cyclohexyl-3-(N,N-dimethylamino)benzyl chloride hydrochloride (518 mg) in dichloromethane (20 mL), potassium carbonate (1.24 g) and 1-(tert-butoxycarbonyl)-5-hydroxy-4-methylindoline (448 mg) were added at

room temperature. The reaction mixture was stirred for 6 hours at 60°C and then left to stand to cool to room temperature, followed by concentration under reduced pressure. Water (20 mL) and chloroform (30 mL) were added to the residue for phase separation. The aqueous layer was extracted with chloroform (2 x 20 mL). The extracts were combined and dried over sodium sulfate anhydrate, and solvent was removed under reduced pressure. The residue was purified by silica gel column chromatography (Biotage 40S), whereby the title compound (397 mg) was yielded.
$^1$H-NMR ($CDCl_3$) δ :
1.24-1.48(5H,m),1.55(9H,s),1.74-1.86(5H,m),2.16(3H,s),2.68(6H,s),2.99(2H,t,J=8.7Hz),3.07-3.15 (1H,m), 3.97 (2H,br s),4.96(2H,s),6.74(1H,d,J=8.5Hz), 7.11(1H,dd,J=7.8,1.7Hz), 7.17 (1H,br s), 7.23(1H,d,J=7.8Hz), 7.61(1H,br s).

(2) 5-[[4-Cyclohexyl-3-(N,N-dimethylamino)phenyl]methoxy]-4-methylindoline hydrochloride

[F620]

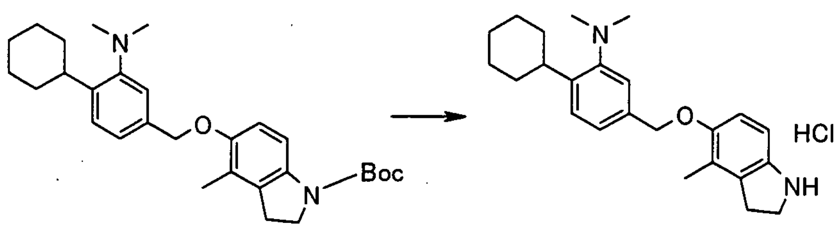

To a solution of 1-(tert-butoxycarbonyl)-5-[[4-cyclohexyl-3-(N,N-dimethylamino)phenyl]methoxy]-4-methylindoline (397 mg) in 1,4-dioxane (2.0 mL), 4N HCl/1,4-dioxane (2.0 mL) was added at room temperature, and the mixture was stirred for 3 hours and concentrated under reduced pressure. Diethyl ether (2.0 mL) was added to the residue, and the precipitated solid was collected by filtration, whereby the title compound (401 mg) was yielded.
$^1$H-NMR (DMSO-$d_6$) δ:
1.21-1.54(5H,m),1.67-1.79(5H,m),2.49(3H,br s),3.00-3.25(7H,m), 3.13(3H,t,J=7.8Hz), 3.70(2H,t,J=7.8Hz), 5.13(2H,s), 7 .05(1H,d,J=8.8Hz), 7.22(1H,d,J=8.3Hz), 7.48(2H,br s), 7.78(1H,br s) .
MS (ESI) m/z : 365 $(M+H)^+$.

(3) 4-[5-[[4-Cyclohexyl-3-(N,N-dimethylamino)phenyl]methoxy] - 4-methylindolin-1-yl]-4-oxo-(3R)-[N-(tert-butoxycarbonyl)-N-methylamino]butyric acid methyl ester

[F621]

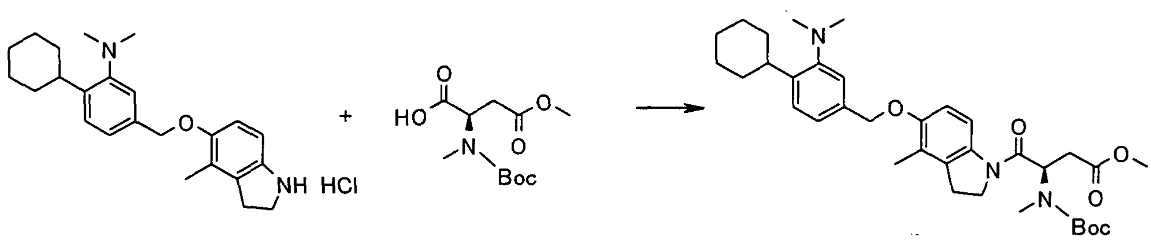

To a solution of (2R)-[N-(tert-butoxycarbonyl)-N-methylamino]succinic acid 4-methyl ester (112 mg) in dichloromethane (3.0 mL), DIEA (372 μL), HOBt (75.0 mg), and EDC·HCl (106 mg) were added at room temperature, and the mixture was stirred for 10 minutes. 5-[[4-Cyclohexyl-3-(N,N-dimethylamino)phenyl]methoxy]-4-methylindoline hydrochloride (200 mg) was added thereto at room temperature, and the reaction mixture was stirred for 3 hours at room temperature and concentrated under reduced pressure. Water (5 mL) and chloroform (10 mL) were added thereto for phase separation. The aqueous layer was extracted with chloroform (2 x 10 mL). The organic layers were combined

and dried over sodium sulfate anhydrate, and solvent was removed under reduced pressure. The residue was purified by silica gel column chromatography (Biotage 25M), whereby the title compound (243 mg) was yielded. $^{1}H$-NMR ($CDCl_3$) δ :
1.27-1.50(17H,m), 1.83-1.87(5H,m), 2.18(3H,s), 2.68(6H,s), 2.77-2.81(3H,m),3.04-3.22(3H,m),3.65-3.74(4H,m), 4.01-4.32(2H,m), 4.98(2H,s), 6.76(1H,d,J=8.8Hz), 7.11(1H,d,J=7.8Hz),7 .17(1H,s), 7.23(1H,d,J=7.8Hz), 7.98(1H,d, J=8.8Hz).
MS (ESI) m/z : 608 $(M+H)^+$.

(4) (3R)-[N-(tert-Butoxycarbonyl)-N-methylamino]-4-[5-[[4-cyclohexyl-3-(N,N-dimethylamino)phenyl]methoxy]-4-methylindolin-1-yl]-4-oxobutyric acid

[F622]

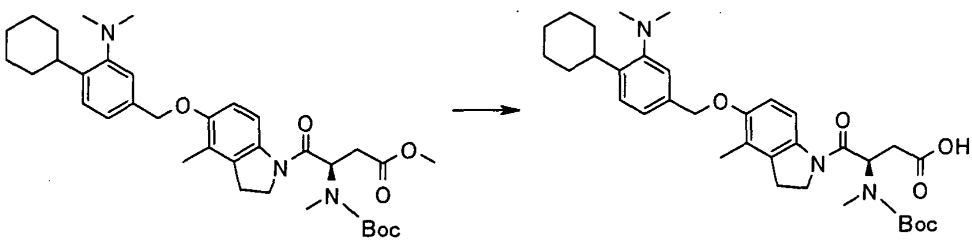

To a solution of (3R)-[N-(tert-butoxycarbonyl)-N-methylamino]-4-[5-[[4-cyclohexyl-3-(N,N-dimethylamino)phenyl]methoxy]-4-methylindolin-1-yl]-4-oxobutyric acid methyl ester (243 mg) in THF (2.0 mL), methanol (1.0 mL) and 1N aqueous sodium hydroxide solution (1.00 mL) were added at room temperature, and the mixture was stirred for 3 days at room temperature. Water (5 mL) was added to the reaction mixture, and the pH thereof was adjusted to 4 with 1N hydrochloric acid. A 20% methanol/chloroform mixture (10 mL) was added thereto for phase separation. The resultant mixture was extracted with a 20% methanol/chloroform mixture (2 x 10 mL). The extracts were combined and dried over sodium sulfate anhydrate, and solvent was removed under reduced pressure, whereby the title compound (248 mg) was yielded. $^{1}H$-NMR (DMSO-$d_6$) δ:
1.27-1.49(17H,m), 1.74-1.87(5H,m), 2.17(3H,s), 2.55-2.94(8H,m),3.04-3.25(4H,m),3.98-4.27(2H,m),4.99(2H,s),6.76 (1H,d,J=8.5Hz),7.14-7.25(3H,m),7.98(1H,d,J=8.5Hz).
MS (ESI) m/z : 594 $(M+H)^+$.

(5) 4-[5-[[4-Cyclohexyl-3-(N,N-dimethylamino)phenyl]methoxy]-4-methylindolin-1-yl]-(3R)-(N-methylamino)-4-oxobutyric acid

[F623]

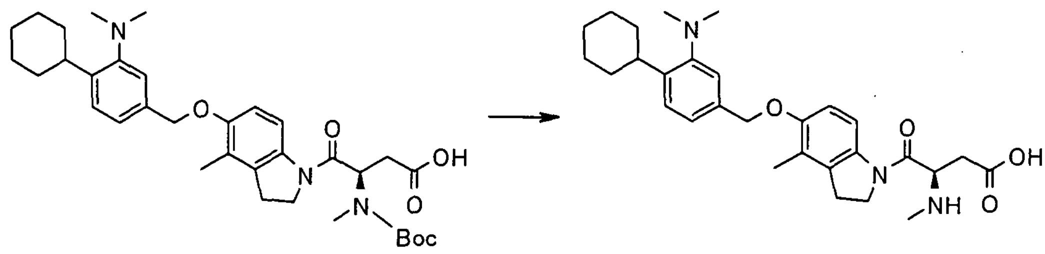

To a solution of of (3R)-[N-(tert-butoxycarbonyl)-N-methylamino]-4-[5-[[4-cyclohexyl-3-(N,N-dimethylamino) phenyl]methoxy]-4-methylindolin-1-yl]-4-oxobutyric acid (247 mg) in dichloromethane (2.4 mL), TFA (0.60 mL) was added at room temperature, and the mixture was stirred for 3 hours. The reaction mixture was concentrated under reduced pressure. Dimethyl sulfoxide (5.0 mL) was added to the residue, and insoluble matter was removed. The resultant product was purified by high-performance liquid chromatography (NOMURA Develosil Combi-RP5), and the target fraction was concentrated and freeze-dried, whereby the title compound (91.4 mg) was yielded.
$^1$H-NMR (DMSO-$d_6$) δ:
1.17-1.39(5H,m),1.56-1.75(5H,m), 2.03(3H,s), 2.23(3H,s), 2.24(1H,dd,J=16.2,7.9Hz), 2.5 1-2.57(1H,m), 2.54(6H,s), 3.01(2H,t,J=8.4Hz), 3.23(1H,br s), 3.80(1H,t,J=7.1Hz), 4.07(1H,dt,J=9.8,8.5Hz), 4.23(1H,dt,J=8. 1,10.0Hz),4.92(2H,s), 6.78(1H,d,J=8.8Hz),7.02(1H,d,J=7.8Hz),7. 13(1H,s), 7.15(1H,d,J=8.1Hz), 7.81(1H,d,J=8.8Hz).
MS (ESI) m/z :494 $(M+H)^+$.
Anal. Calcd for $C_{29}H_{39}N_3O_4 \cdot$ 1.75$H_2O$:C,66.32;H,8.16;N,8.00.Found:C,66.28;H,7.88;N,7.86.

[Example 136]

(3S)-(N-Methylamino)-4-[7-methyl-5-[(4-phenyl-5-trifluoromethyl-2-thienyl)methoxy]-1-indolinyl]-4-oxobutyric acid hydrochloride

(1) 1-(tert-Butoxycarbonyl)-7-methyl-1H-indole

[F624]

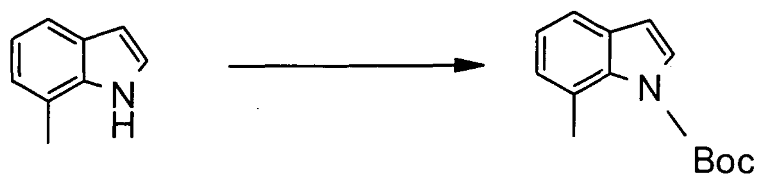

7-Methyl-1H-indole (1.00 g) was dissolved in THF (10 mL), and $Boc_2O$ (2.50 g) and DMAP (90.0 mg) were added to the solution, followed by stirring for 20 hours. The reaction mixture was concentrated, and the residue was purified by silica gel flash column chromatography (Yamazen Hi-Flash column 4L), whereby the title compound (1.76 g) was yielded.
$^1$H-NMR ($CDCl_3$) δ :
1.63(9H,s),2.64(3H,s), 6.53(1H,d,J=3.7Hz), 7.17-7.08(2H,m),7.38(1H,d,J=7.3Hz), 7.51(1H,d,J=3.7Hz).

(2) 1-(tert-Butoxycarbonyl)-7-methyl-indoline

[F625]

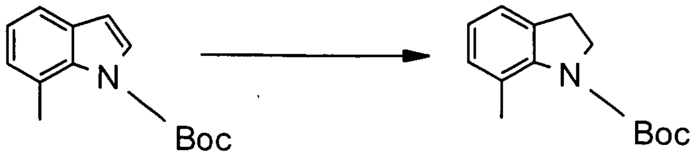

1-(tert-Butoxycarbonyl)-7-methyl-1H-indole (2.50 g) was dissolved in ethanol (100 mL), and 5% Pd/C (2.00 g) was added to the solution. The resultant mixture was subjected to catalytic hydrogenation with stirring under normal pressure for 1 day. Nitrogen was caused to pass through the reaction vessel, and the catalyst was removed by filtration. The filtrate was concentrated, and the residue was purified by silica gel flash column chromatography (Yamazen Hi-Flash column 3L), whereby the title compound (1.71 g) was yielded.
$^1$H-NMR ($CDCl_3$) $\delta$ :
1.53(9H,s), 2.30(3H,s), 2.96(2H,t,J=7.7Hz), 4.06(2H,t,J=7.3Hz), 7 .04-6.93 (3H,m).
MS (ESI) m/z : 256 $(M+Na)^+$.

(3) 7-Methylindoline

[F626]

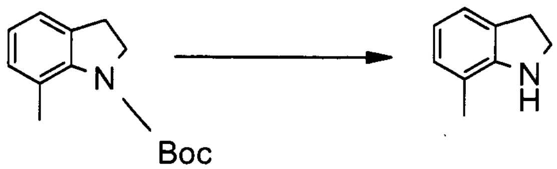

To 1-(tert-butoxycarbonyl)-7-methylindoline (1.71 g), 4N HCl/dioxane (10 mL) was added, and the mixture was stirred for 18 hours. The reaction mixture was concentrated, and saturated aqueous sodium hydrogencarbonate solution was added to the residue. The resultant mixture was extracted with dichloromethane (2 x 100 mL). The extracts were combined and dried over sodium sulfate anhydrate, and solvent was removed, whereby the title compound (1.00 g) was yielded.
$^1$H-NMR ($CDCl_3$) $\delta$:
2.13(3H,s), 3.05(2H,t,J=8.1Hz), 3.54-3.59(3H,m), 6.65(1H,t,J=7.3Hz), 6.86(1H,d,J=7.3Hz), 6.98(1H,d,J= 7.6Hz).
MS (ESI) m/z : 134 $(M+H)^+$.

(4) 5-Hydroxy-7-methyl-1H-indole (WO 9523141)

[F627]

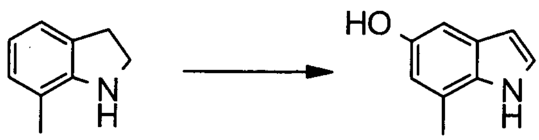

To a solution of 7-methylindoline (500 mg) in acetone (25 mL), a 0.1M phosphate buffer solution (pH 7.0) (100 mL) and $(KSO_3)_2NO$ (2.22 g) were sequentially added with stirring. The resultant mixture was stirred for 1 hour and extracted with ethyl acetate (500 mL). The extract was washed with saturated brine (2 x 200 mL), followed by drying over sodium sulfate anhydrate. Solvent was evaporated, and the residue was purified by silica gel column chromatography (Yamazen Hi-Flash column 2L), whereby the title compound (276 mg) was yielded.
$^1H$-NMR (DMSO-$d_6$) δ:
2.36(3H,d,J=0.5Hz), 6.20(1H,t,J=2.3Hz), 6.41-6.39(1H,m), 6.65(1H,d,J=1.7Hz), 7.17(1H,t,J=2.7Hz), 8.44(1H,s),1 0.68 (1H,s).
MS(ESI)m/z:148$(M+H)^+$.

(5) 5-Benzyloxy-7-methyl-1H-indole (Buchheit, K. -H., et al. Bioorganic & Medicinal Chemistry Letters (1995), 5 (21), 2495-500)

[F628]

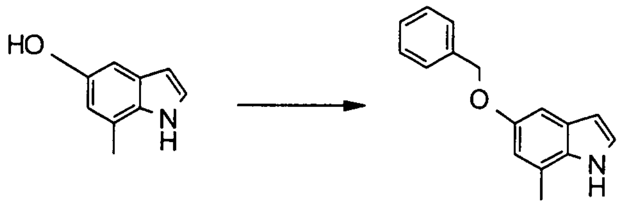

5-Hydroxy-7-methyl-1H-indole (640 mg), benzyl bromide (776 μL), potassium carbonate (902 mg), and DMF (10 mL) were mixed together, and the mixture was stirred for 18 hours at room temperature. The reaction mixture was diluted with ethyl acetate (200 mL) and washed with saturated brine (2 x 100 mL), followed by drying over sodium sulfate anhydrate. Solvent was evaporated, and the residue was purified by silica gel column chromatography (Yamazen Hi-Flash column 3L), whereby the title compound (763 mg) was yielded.
$^1H$ -NMR ($CDCl_3$) δ :
2.46(3H,s), 5.09(2H,s), 6.48(1H,dd,J=3.2,2.0Hz), 6.77-6.79(1H,m), 7.01-7.06(1H,m), 7.18(1H,t,J=2.9Hz), 7.28-7.49(5H, m), 7.96(1H,br s).
MS (ESI) m/z : 238 $(M+H)^+$.

(6) 5-Benzyloxy-1-(tert-butoxycarbonyl)-7-methyl-1H-indole

[F629]

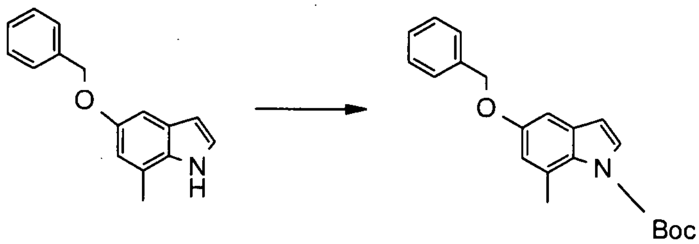

5-Benzyloxy-7-methyl-1H-indole (763 mg) was dissolved in THF (10 mL), and $Boc_2O$ (1.05 g) and DMAP (39.3 mg) were added to the solution under stirring, followed by further stirring for 14 hours. The reaction mixture was concentrated, and the residue was purified by silica gel column chromatography (Yamazen Hi-Flash column 2L), whereby the title compound (1.08 g) was yielded.

$^1$H-NMR ($CDCl_3$) δ:
1.62(9H,s), 2.61(3H,s), 5.09(2H,s), 6.43(1H,d,J=4.2Hz), 6.82(1H,d ,J=2.4Hz), 6.91(1H,d,J=2.7Hz), 7.50-7.28(6H,m).
MS (ESI) m/z : 338 $(M+H)^+$.

(7) 1-(tert-Butoxycarbonyl)-5-hydroxy-7-methylindoline

[F630]

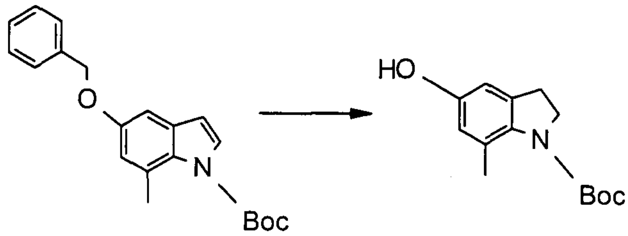

5-Benzyloxy-1-(tert-butoxycarbonyl)-7-methyl-1H-indole (1.18 g) was dissolved in ethanol (20 mL), and 5% Pd/C (1 g) was added to the solution. The resultant mixture was subjected to catalytic hydrogenation for 20 hours with stirring. Nitrogen was caused to pass through the reaction vessel. The catalyst was removed by filtration, and the filtrate was concentrated. The residue was purified by silica gel column chromatography (Yamazen Hi-Flash column 2L), whereby the title compound (613 mg) was yielded.
$^1$H-NMR ($CDCl_3$) δ :
1.52(9H,s), 2.23(3H,s), 2.89(2H,t,J=7.8Hz), 4.04(2H,t,J=7.8Hz), 5 .08(1H,s), 6.44(1H,d,J=2.0Hz), 6.52(1H,d,J=2.0Hz).
MS (ESI) m/z : 272 $(M+Na)^+$.

(8) 1-(tert-Butoxycarbonyl)-5-[(4-phenyl-5-trifluoromethyl-2-thienyl)methoxy]-7-methylindoline

[F631]

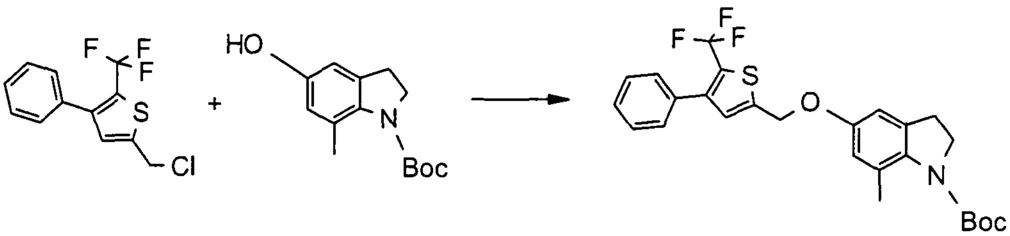

1-(tert-Butoxycarbonyl)-5-hydroxy-7-methylindoline (613 mg), 4-phenyl-5-trifluoromethyl-2-thienylmethyl chloride (1.02 g), potassium carbonate (510 mg), and DMF (10 mL) were mixed together, and the mixture was stirred for 20 hours at 85°C. The reaction mixture was left to stand to cool to room temperature and diluted with ethyl acetate (200 mL). The resultant mixture was dried over sodium sulfate anhydrate. Solvent was removed, and the residue was purified by silica gel column chromatography (Yamazen Hi-Flash column 2L), whereby the title compound (1.07 g) was yielded.
$^1$H-NMR ($CDCl_3$) δ:
1.52(9H,s), 2.29(3H,s), 2.94(2H,t,J=7.6Hz), 4.06(2H,t,J=8.1Hz),5 .17(2H,s), 6.62-6.65(1H,m), 6.69-6.71(1H,m), 7.06-7.04(1H,m), 7.35-7.44(5H,m).

(9) 7-Methyl-5-[(4-phenyl-5-trifluoromethyl-2-ethynyl)methoxy]indoline

[F632]

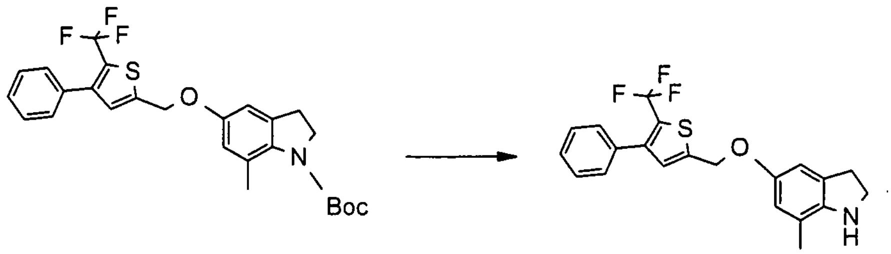

To 1-(tert-butoxycarbonyl)-5-[(4-phenyl-5-trifluoromethyl-2-ethynyl)methoxy]-7-methylindoline (1.07 g), 4N HCl/dioxane (10 mL) was added, and the mixture was stirred for 3 hours. The reaction mixture was concentrated, and saturated aqueous sodium bicarbonate solution (50 mL) was added to the residue for alkalization. The formed alkaline product was extracted with ethyl acetate (200 mL). The extract was dried over sodium sulfate anhydrate, and solvent was evaporated, whereby the title compound (800 mg) was yielded.
$^1$H-NMR ($CDCl_3$) $\delta$:
2.14(3H,s), 3.04(2H,t,J=8.4Hz), 3.57(3H,t,J=8.8Hz), 5.13(2H,s), 6 .55-6.57(1H,m), 6.69-6.72(1H,m), 7.02-7.05(1H,m), 7.35-7.44(5H,m).
MS(ESI)m/z:390(M+H)$^+$.

(10) (3R)-[N-(tert-Butoxycarbonyl)-N-methylamino]-4-[7-methyl-5-[(4-phenyl-5-trifluoromethyl-2-thienyl)methoxy]-1-indolinyl]-4-oxobutyric acid methyl ester

[F633]

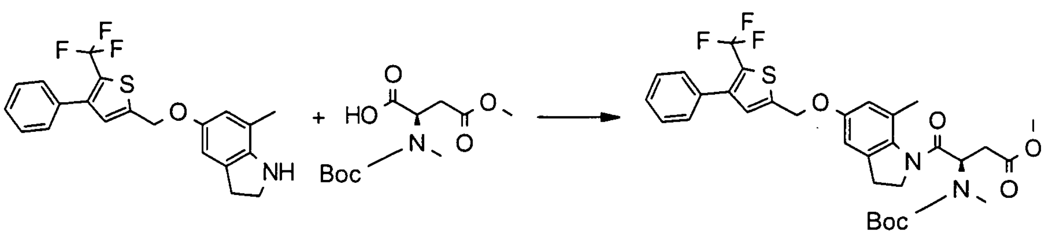

7-Methyl-5-[(4-phenyl-5-trifluoromethyl-2-thienyl)methoxy]indoline (166 mg), Boc-D-Me-Asp(OMe)-OH (111 mg), EDC·HCl (123 mg), HOBt (86 mg), TEA (59 μL), and DMF (10 mL) were mixed together, and the mixture was stirred for 19 hours. The reaction mixture was extracted with ethyl acetate (200 mL), and the extract was washed with saturated brine (2 x 100 mL), followed by drying over sodium sulfate anhydrate. Solvent was evaporated, and the residue was purified by silica gel flash column chromatography (Yamazen Hi-Flash column 2L), whereby the title compound (98 mg) was yielded. $^1$H-NMR ($CDCl_3$) $\delta$:
1.45-1.54(9H,m), 2.21(3H,s), 2.54(1H,dd,J=15.5,5.7Hz), 2.80-2.94(4H,m), 3.08-3.24(2H,m), 3.64-3.71(3H,m), 3.83-4.00(1H,m), 4.20-4.35(1H,m), 5.18(2H,s), 5.42-5.69(1H,m), 6.66(1H,s), 6.73(1H,s), 7.07-7.04(1H,m), 7.44-7.37(5H,m). MS(ESI)m/z:655(M+Na)+.

(11) (3R)-(N-Methylamino)-4-[7-methyl-5-[(4-phenyl-5-trifluoromethyl-2-thienyl)methoxy]-1-indolinyl]-4-oxobutyric acid hydrochloride

[F634]

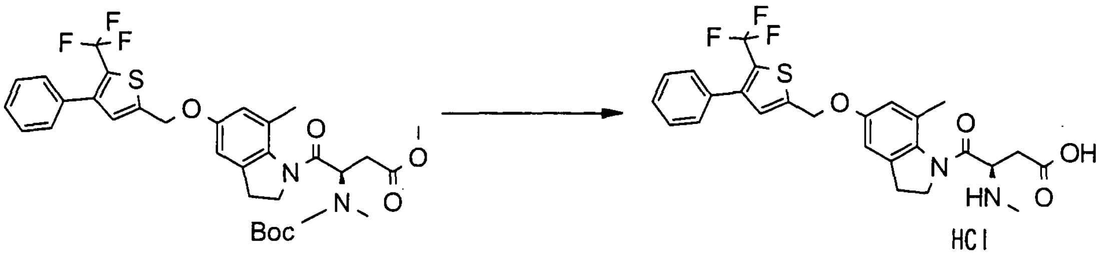

To a of (3R)-[N-(tert-butoxycarbonyl)-N-methylamino]-4-[7-methyl-5-[(4-phenyl-5-trifluoromethyl-2-thienyl) methoxy]-1-indolinyl]-4-oxobutyric acid methyl ester (90 mg) in THF (5 mL), 0.25N aqueous sodium hydroxide solution was added, and the mixture was stirred for 18 hours. The reaction mixture was neutralized with 1N hydrochloric acid, followed by extraction with 20% methanol/chloroform (2 x 100 mL). The extracts were combined and dried over sodium sulfate anhydrate, and solvent was evaporated. 4N HCl/1,4-dioxane (10 mL) was added to the residue, followed by stirring for 3 hours. The resultant mixture was concentrated, and the solid was collected by filtration and dried, whereby the title compound (20 mg) was yielded.
$^1$H-NMR (DMSO-$d_6$) δ:
2.04(1.5H,s), 2.24(1.5H,s), 2.40-2.43(2H,m), 2.80-3.09(5H,m), 3.53(1H,t,J=8.3Hz), 3.90-4.01(1H,m),4.21-4.30(0.5H, m), 4.54-4.60(0.5H,m), 5.28(2H,s), 6.66-6.68(0.5H,m), 6.73-6.76(0.5H,m), 6.80-6.85(1H,m),7.28-7.30(1H,m), 7.43-7.33 (5H,m). No proton peak was observed for either $NH_2Cl$ or $CO_2H$.
MS (ESI)m/z:519 (M+H)+.

[Example 137]

3-[N-[2-[4-Methoxy-5-[[4-phenyl-5-(trifluoromethyl)-2-thienyl]methoxy]-2,3-dihydro-1H-indol-1-yl]-2-oxoethyl] amino] propionic acid

(1) 2,3-Bis(benzyloxy)benzaldehyde

[F635]

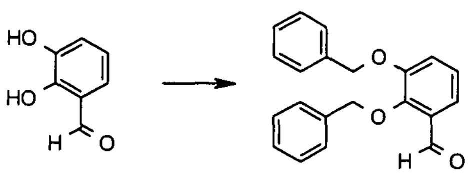

To a solution of 2,3-dihydroxybenzaldehyde (commercially available) (10.0 g) in acetonitrile (200 mL), potassium

carbonate (40.0 g) and benzyl bromide (25.8 mL) were added at room temperature, and the mixture was stirred for 4 hours at 90°C. The reaction mixture was left to stand to cool to room temperature, and insoluble matter was removed by filtration. The filtrate was concentrated under reduced pressure, and dichloromethane-hexane was added to the concentrate to form solid. The solid was collected by filtration and dried, whereby the title compound (20.9 g) was yielded.
$^1$H-NMR ($CDCl_3$) δ:
5.19(2H,s),5.20(2H,s), 7.11(1H,t,J=7.9Hz), 7.25-7.50(12H,m), 10.26(1H,s).
MS (ESI) m/z : 319 $(M+H)^+$.

(2) 2,3-Bis(benzyloxy)-6-nitrobenzaldehyde and 2,3-bis(benzyloxy)-5-nitrobenzaldehyde

[F636]

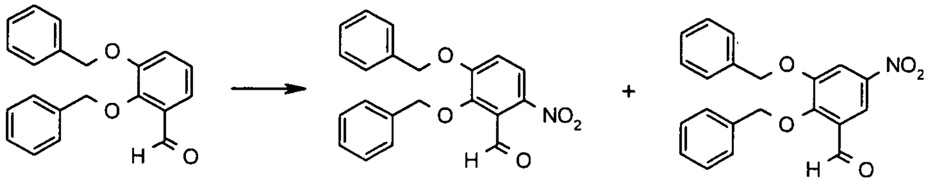

To a solution of 2,3-bis(benzyloxy)benzaldehyde (20.9 g) in glacial acetic acid (400 mL), fuming nitric acid (95 mL) was added at 0°C. The reaction mixture was stirred for 4 hours at room temperature and pourred onto ice (1 kg). The precipitated matter was collected by filtration and dried (uder reduced pressure at 50°C for 15 hours) to obtain a solid. The solid was purified by silica gel flash column chromatography (Biotage 65i x 2), whereby 2,3-bis(benzyloxy)-6-nitrobenzaldehyde (6.93 g) and 2,3-bis(benzyloxy)-5-nitrobenzaldehyde (11.3 g) were yielded.
6-Nitro form:
$^1$H-NMR ($CDCl_3$) δ:
5.08(2H,s), 5.26(2H,s), 7.13(1H,d,J=9.2Hz), 7.29-7.35(5H,m),7.38-7.46(5H,m), 7.93(1H,d,J=9.2Hz), 10.21(1H,s).
MS (ESI)m/z:364 $(M+H)^+$.
5-Nitro form:
$^1$H-NMR ($CDCl_3$) δ:
5.28 (2H, s), 5.35 (2H, s), 7.25-7.53(10H,m), 8.08(1H,d,J=2.7Hz), 8.28(1H,d,J=2.7Hz), 10.19(1H,s).
MS (ESI)m/z:386 $(M+Na)^+$.

(3) Acetic acid 6-(benzyloxy)-2-formyl-3-nitrophenyl ester

[F637]

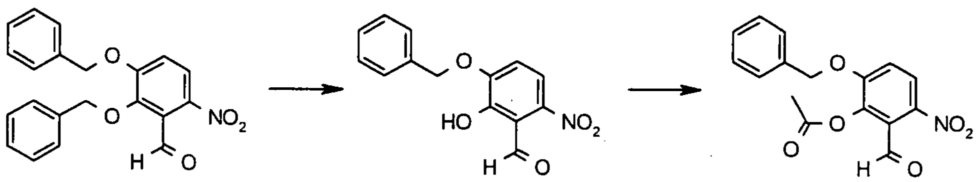

To 2,3-bis(benzyloxy)-6-nitrobenzaldehyde (6.90 g), benzene (340 mL) and diethyl ether (54 mL) were added, and the mixture was heated to 75°C to form a solution. To the solution, magnesium bromide-diethyl ether complex (5.40 g) was slowly added, and the mixture was stirred for 15 hours at 75°C. The reaction mixture was left to stand to cool to room temperature and poured into 2N hydrochloric acid (200 mL) and ice (200 g) under stirring. Diethyl ether (200 mL) was added to the resultant mixture for phase separation. The aqueous layer was extracted with diethyl ether (200 mL). The extract was dried over sodium sulfate anhydrate, and solvent was removed under reduced pressure. The residue was purified by silica gel flash column chromatography (Biotage 40M) to thereby obtain 3-(benzyloxy)-2-hydroxy-6-nitrobenzaldehyde (5.90 g). The aldehyde was dissolved in acetic anhydride (35 mL), and sodium acetate (350 mg) was

added to the mixture at room temperature, followed by stirring for 1 hour at 60°C. The resultant mixture was left to stand to cool to room temperature and poured into ice (400 g), followed by stirring for 20 hours. The solid was collected by filtration and washed with water, followed by drying (in vacuo at 50°C for 2 days), whereby the title compound (5.20 g) was yielded.
$^1$H-NMR ($CDCl_3$) δ :
2.29 (3H, s), 5.24 (2H, s), 7.13 (1H, d, J=9.3Hz), 7.33-7.45(5H,m),8.11(1H,d,J=9.3Hz),10.23(1H,s).
MS (ESI)m/z:316 $(M+H)^+$.

(4) 3-(Benzyloxy)-2-hydroxy-6-nitrobenzaldehyde and 6-(benzyloxy)-2-(dimethoxymethyl)-3-nitrophenol

[F638]

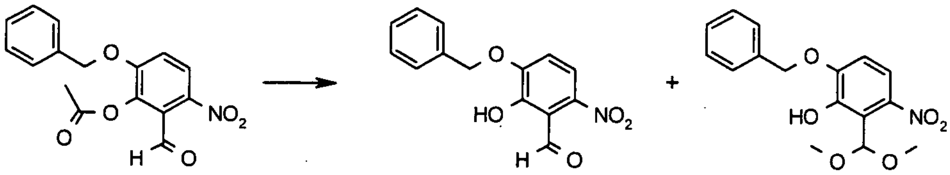

To a solution of acetic acid 6-(benzyloxy)-2-formyl-3-nitrophenyl ester (2.61 g) in THF (30 mL), methanol (15 mL) and 1N aqueous sodium hydroxide solution (25.0 mL) were added at room temperature, and the mixture was stirred for 1 hour at room temperature. 1N Hydrochloric acid (25.0 mL) and ethyl acetate (100 mL) were added to the reaction mixture for phase separation. The aqueous layer was extracted with ethyl acetate (50 mL). The organic layers were combined and dried over sodium sulfate anhydrate, and organic solvent was removed under reduced pressure. The resultant concentrate was purified by silica gel flash column chromatography (Biotage 40M), whereby 3-(benzyloxy)-2-hydroxy-6-nitrobenzaldehyde (680 mg) and, as a high-polar substance, 6-(benzyloxy)-2-(dimethoxymethyl)-3-nitrophenol (1.49 g) were yielded.
Aldehyde:
$^1$H-NMR ($CDCl_3$) δ:
5.28(2H,s),7.06(1H,d,J=8.9Hz),7.33-7.46(5H,m), 7.67(1H,d,J=8.9Hz), 10.49(1H,d,J=2.4Hz), 12.57(1H,s).
MS(ESI)m/z:272 $(M-H)^+$.
Acetal:
$^1$H-NMR ($CDCl_3$) δ :
3.51(6H,s), 5.22(2H,s), 6.13(1H,s), 6.88(1H,d,J=8.8Hz), 7.30-7.48(6H,m), 9.21(1H,s).
MS (ESI)m/z:288 $(M-OMe (31))^+$.

(5) 3-(Benzyloxy)-2-hydroxy-6-nitrobenzaldehyde

[F639]

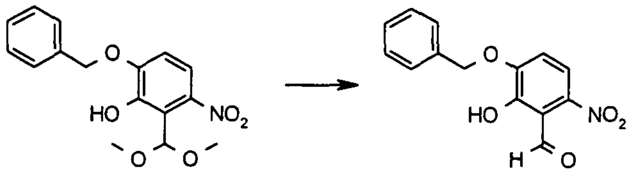

To a solution of 6-(benzyloxy)-2-(dimethoxymethyl)-3-nitrophenol (1.49 g) in 2-propanol (10 mL), concentrated hydrochloric acid (0.60 mL) was added at room temperature, and the mixture was stirred for 1 hour at 80°C and left to stand to cool to room temperature. The solid was collected by filtration and dried (in vacuo at 40°C for 1 hour), whereby the title compound (1.24 g) was yilded.

(6) 3-(Benzyloxy)-2-methoxy-6-nitrobenzaldehyde

[F640]

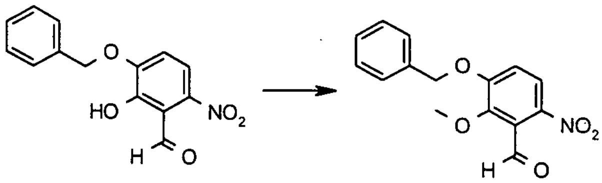

To a solution of 3-(benzyloxy)-2-hydroxy-6-nitrobenzaldehyde (1.93 g) in dichloromethane (30 mL), potassium carbonate (2.93 g) and methyl iodide (0.660 mL) were added at room temperature, and the mixture was stirred for 4 days at room temperature. Water (400 mL) was added to the reaction mixture. The solid was collected by filtration and dried, whereby the title compound (1.99 g) was yielded.
$^1$H-NMR ($CDCl_3$) δ:
3.93(3H,s),5.25(2H,s), 7.09(1H,d,J=9.0Hz),7.33-7.50(5H,m), 7.91(1H,d,J=9.0Hz), 10.37(1H,s).
MS(ESI)m/z:288 $(M+H)^+$.

(7) 1-(Benzyloxy)-2-methoxy-4-nitro-3-[(E)-2-nitroethenyl]benzene

[F641]

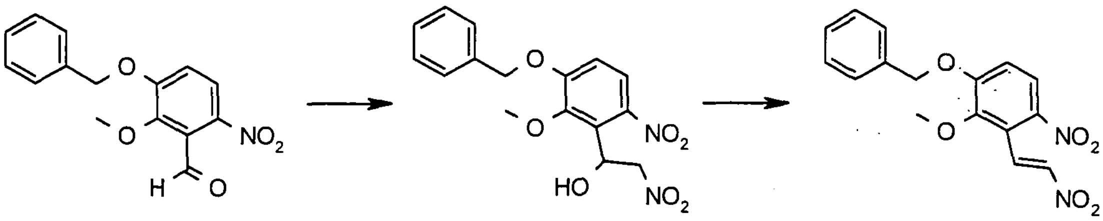

To a solution of 3-(benzyloxy)-2-methoxy-6-nitrobenzaldehyde (1.99 g) in N-methylmorpholine (35 mL), potassium fluoride (173 mg), and crown ether (55.0 mg), nitromethane (1.88 mL) was added at 10°C. The reaction mixture was stirred for 2 days at 5°C and poured into water (200 mL), followed by extraction with chloroform (2 x 200 mL). The extracts were combined and dried over sodium sulfate anhydrate, followed by concentration under reduced pressure. The concentrate was purified by silica gel flash column chromatography (Biotage 40M), whereby 1-[3-(benzyloxy)-2-methoxy-6-nitrophenyl]-2-nitro-1-ethanol (2.08 g) was obtained. This compound was dissolved in acetic anhydride (18 mL), and sodium acetate (400 mg) was added thereto at room temperature, followed by stirring for 1 hour at 60°C. The reaction mixture was left to stand to cool to room temperature and poured into ice water (200 g). The solid was collected by filtration and dried (in vacuo at 40°C for 2 hours), whereby the title compound (1.88 g) was yielded.
$^1$H-NMR ($CDCl_3$) δ :
3.89(3H,s), 5.25(2H,s), 7.09(1H,d,J=9.2Hz), 7.36-7.45(5H,m), 7.71(1H,d,J=13.6Hz), 7.92(1H,d,J=9.2Hz), 8.22(1H,d,J =13.6Hz).

(8) 5-(Benzyloxy)-4-methoxy-1H-indole (EP 343574)

[F642]

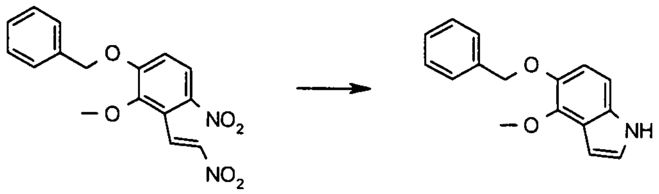

To a solution of 1-(benzyloxy)-2-methoxy-4-nitro-3-[(E)-2-nitroethenyl]benzene (1.88 g) in toluene (60 mL), silica gel (14.0 g), reduced iron (5.40 g), and glacial acetic acid (30 mL) were added, and the mixture was stirred for 10 minutes at 90°C. The reaction mixture was left to stand to cool to room temperature and filtered with ethyl acetate. The filtrate was concentrated under reduced pressure. To the residue, chloroform (100 mL), water (30 mL), and saturated aqueous sodium hydrogencarbonate solution (100 mL) were added, and the resultant mixture was filtered. The filtrate was partitioned, and the aqueous layer was extracted with chloroform (50 mL). The organic layers were combined and dried over sodium sulfate anhydrate, and solvent was removed under reduced pressure. The concentrate was purified by silica gel flash column chromatography (Biotage 40M), whereby the title compound (1.25 g) was yielded.
$^1$H-NMR ($CDCl_3$) δ:
4.08(3H,s), 5.13(2H,s), 6.64-6.68(1H,m), 6.93(1H,d,J=8.8Hz), 7.01(1H,d,J=8.8Hz), 7.15(1H,t,J= 2.8Hz),7.25-7.52(5H, m), 8.06(1H,br s).
MS (ESI) m/z : 254 $(M+H)^+$.

(9) 5-(Benzyloxy)-4-methoxy-1H-indole-1-carboxylic acid tert-butyl ester

[F643]

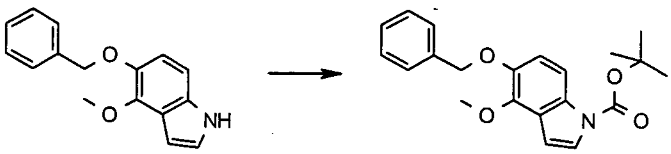

To a solution of 5-(benzyloxy)-4-methoxy-1H-indole (1.24 g) in THF (10 mL), DMAP (30.0 mg) and $Boc_2O$ (1.12 g) were added at room temperature, and the mixture was stirred for 15 hours at room temperature. The reaction mixture was concentrated under reduced pressure. The residue was purified by silica gel flash column chromatography (Biotage 40M), whereby the title compound (1.71 g) was yielded.
$^1$H-NMR ($CDCl_3$) δ:
1.65(9H,s), 4.02(3H,s), 5.16(2H,s), 6.67(1H,d,J=3.7Hz), 7.00(1H,d ,J=8.9Hz), 7.28-7.50(5H,m), 7.51(1H,d,J=3.7Hz), 7.75(1H,br d,J=8.9Hz).
MS (ESI) m/z : 354 $(M+H)^+$.

(10) 5-Hydroxy-4-methoxy-1H-indolinecarboxylic acid tert-butyl ester

[F644]

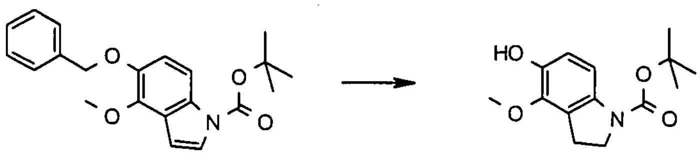

To a solution of 5-(benzyloxy)-4-methoxy-1H-indole-1-carboxylic acid tert-butyl ester (1.70 g) in ethanol (40 mL), 5% Pd/C (1.30 g) was added at room temperature, and the mixture was stirred for 2 days at room temperature under a hydrogen atmosphere. The resultant mixture was filtered, and the filtrate was concentrated under reduced pressure. The concentrated matter was slurried with a small amount of hexane. The slurry was filtered, and the collected solid was dried, whereby the title compound (986 mg) was yielded.
$^1$H-NMR ($CDCl_3$) $\delta$ :
1.56(9H,s), 3.15(2H,t,J=8.7Hz), 3.87(3H,s), 3.99(2H,br t,J=7.7Hz), 5.32(1H,s), 6.75(1H,d,J=8.5Hz), 7.09(1/2 of 1H,br s), 7.48 (1/2 of 1H,br s) .
MS (ESI)m/z:266(M+H)$^+$.

(11) 4-Methoxy-5-[[4-phenyl-5-(trifluoromethyl)-2-thienyl]methoxy]-1-indolinecarboxylic acid tert-butyl ester

[F645]

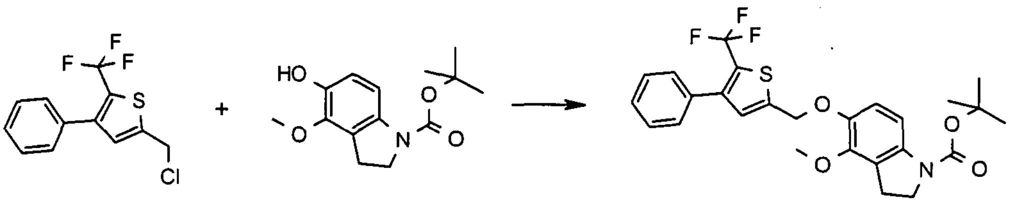

To a solution of 5-(chloromethyl)-3-phenyl-2-(trifluoromethyl)thiophene (333 mg) and 5-hydroxy-4-methoxy-1H-indolinecarboxylic acid tert-butyl ester (318 mg) in dichloromethane (5.0 mL), potassium carbonate (250 mg) was added at room temperature, and the mixture was stirred for 16 hours at 50°C. The reaction mixture was cooled to room temperature. Precipitated matter was removed by filtration, and water (40 mL) and saturated aqueous ammonium chloride solution (40 mL) were added to the filtrate. The resultant mixture was extracted with ethyl acetate (2 x 30 mL). The extracts were combined and washed with saturated brine (30 mL), followed by drying over sodium sulfate anhydrate. Solvent was removed under reduced pressure. The residue was purified by silica gel flash column chromatography (Biotage 40M), whereby the title compound (552 mg) was yielded.
$^1$H-NMR ($CDCl_3$) $\delta$:
1.55(9H,s),3.09(2H,t,J=8.5Hz),3.91(3H,s),3.99(2H,br s), 5.21(2H,s), 6.81(1H,d,J=8.8Hz), 7.05(1H,d,J=1.0Hz), 7.06(1/2 of 1H,br s), 7.35-7.45(5H,m), 7.48(1/2 of 1H,br s).
MS(ESI)m/z:506 (M+H)$^+$.

(12) 4-Methoxy-5-[[4-phenyl-5-(trifluoromethyl)-2-thienyl]methoxy]indoline hydrochloride

[F646]

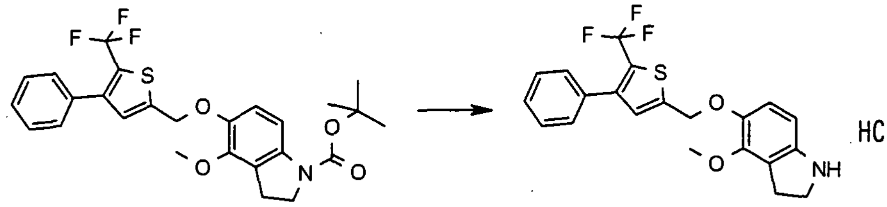

To 4-methoxy-5-[[4-phenyl-5-(trifluoromethyl)-2-thienyl]methoxy]-1-indolinecarboxylic acid tert-butyl ester (550 mg), 4N HCl/1,4-dioxane (5.0 mL) was added at room temperature, and the mixture was stirred for 2 hours. The reaction mixture was concentrated under reduced pressure. Hexane was added to the resultant product to form a slurry. The slurry was filtered, and the collected solid was dried, whereby the title compound (449 mg) was yielded.
$^1$H-NMR (DMSO-$d_6$) δ:
3.19(2H,t,J=7.8Hz), 3.68(2H,t,J=7.8Hz), 3.86(3H,s), 5.43(2H,s),7 .05(1H,d,J=8.5Hz), 7.16(1H,d,J=8.5Hz), 7.38(1H,d, J=1.5Hz), 7.40-7.51(5H,m), 10.87(1H,br s).
MS(ESI)m/z:406 $(M+H)^+$.

(13) 3-[N-(tert-Butoxycarbonyl)-N-[2-[4-methoxy-5-[[4-phenyl-5-(trifluoromethyl)-2-thienyl]methoxy]-2,3-dihydro-1H-indol-1-yl]-2-oxoethyl]amino]propionic acid tert-butyl ester

[F647]

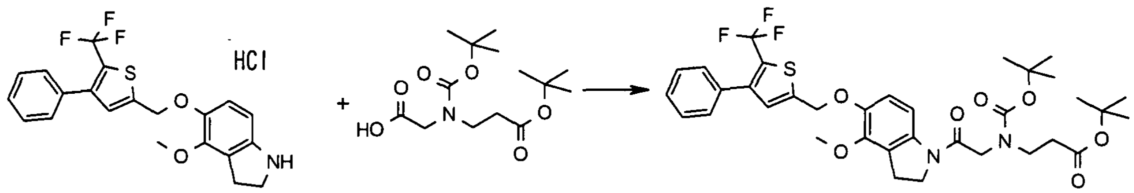

To a dichloromethane (3.0 mL) suspension of 4-methoxy-5-[[4-phenyl-5-(trifluoromethyl)-2-thienyl]methoxy] indoline hydrochloride (135 mg) and 2-[N-(tert-butoxycarbonyl)-N-[3-(tert-butoxy)-3-oxopropyl]amino]acetic acid (111 mg), EDC·HCl (87.7 mg), HOBt (61.8 mg), and TEA (0.213 mL, 1.53 mmol) were added at room temperature, and the mixture was stirred for 18 hours. The reaction mixture was concentrated under reduced pressure. To the concentrate, ethyl acetate (20 mL), saturated aqueous sodium hydrogencarbonate solution (40 mL), and water (40 mL) were added for phase separation. The aqueous layer was extracted with ethyl acetate (20 mL). The extracts were combined and dried over sodium sulfate anhydrate. Solvent was removed under reduced pressure. The residue was purified by silica gel flash column chromatography (Biotage 25M), whereby the title compound (183 mg) was yielded. $^1$H-NMR ($CDCl_3$) δ : 1.38-1.61(18H,m),2.54-2.63(2H,m),3.18-3.26(2H,m),3.54-3.62(2H,m),3.91(2/3 of 3H,s),3.93(1/3 of 3H,s),4.00-4.20(4H, m),5.22(2/3 of 2H,s),5.23(1/3 of 2H,s),6.79-6.86(1H,m), 7.06(1H,s), 7.35-7.45(5H,m), 7.84-7.90(1H,m).
MS (ESI) m/z : 691 $(M+H)^+$.

(14) 3-[N-[2-[4-Methoxy-5-[[4-phenyl-5-(trifluoromethyl)-2-thienyl]methoxy]-2,3-dihydro-1H-indol-1-yl]-2-oxoethyl] amino] propionic acid

[F648]

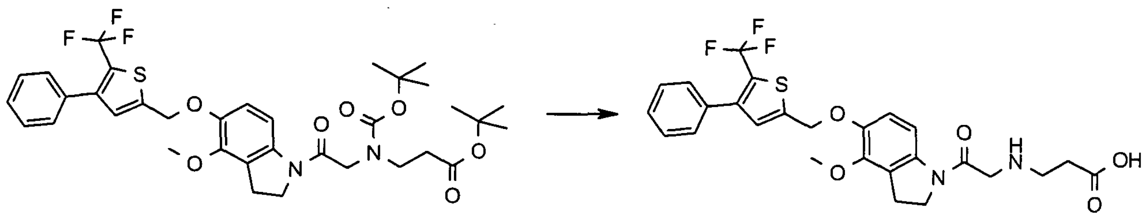

To 3-[N-(tert-butoxycarbonyl)-N-[2-[4-methoxy-5-[[4-phenyl-5-(trifluoromethyl)-2-thienyl]methoxy]-2,3-dihydro-1H-indol-1-yl]-2-oxoethyl]amino]propionic acid tert-butyl ester (180 mg), 4N HCl/1,4-dioxane (5.0 mL) was added at room temperature, and the mixture was stirred for 16 hours. The reaction mixture was concentrated under reduced pressure, and diethyl ether was added thereto to form a slurry. The solid was obtained by filtration and purified by reverse phase high performance liquid chromatography (column: Nomura Chemistry Develosil Combi-RP-5, 10 cm). The target fraction was freeze-dried, whereby the title compound (22.3 mg) was yielded.
$^1$H-NMR(DMSO-d6) δ:
2.37(2H,t,J=6.6Hz), 2.83(2H,t,J=6.6Hz), 3.13(2H,t,J=8.3Hz), 3.57 (2H,s), 3.81(3H,s), 4.05(2H,t,J=8.3Hz), 5.36(2H,s), 7.00(1H,d,J=8 .7Hz), 7.37(1H,s), 7.40-7.52(5H,m), 7.74(1H,d,J=8.7Hz). Neither $CO_2H$ nor NH was observed.
MS (ESI) m/z : 535 $(M+H)^+$.
HR-MS(ESI)Calcd for $C_{26}H_{26}F_3N_2O_5S$ $(M+H)^+$: 535. 15145. Found: 535. 14835.

[Example 138]

3-[N-[2-[4-Fluoro-5-[(4-phenyl-5-trifluoromethyl-2-thienyl)methoxy]-1-indolinyl]-2-oxoethyl]amino]propionic acid

(1) 4-Benzyloxy-3-fluoro-1-nitrobenzene

[F649]

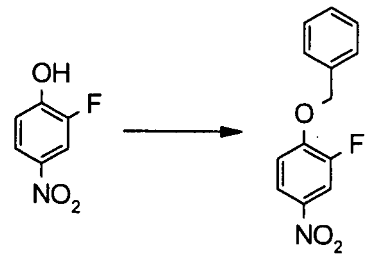

2-Fluoro-4-nitrophenol (5.00 g), benzyl bromide (3.97 mL), potassium carbonate (6.60 g), and DMF (50 mL) were mixed together, and the mixture was stirred for 20 hours at 80°C. The reaction mixture was left to stand to cool to room temperature, and water (200 mL) was added thereto. Precipitated matter was collected by filtration and dried, whereby the title compound (7.75 g) was yielded.
$^1$H-NMR $(CDCl_3)$ δ :
5.25(2H,s), 7.10-7.04(1H,m), 7.46-7.34(5H,m), 8.04-7.97(2H,m).
MS(ESI)m/z:248$(M+H)^+$.

(2) 3-Benzyloxy-2-fluoro-6-nitrophenylacetonitrile and 5-benzyloxy-4-fluoro-2-nitrophenylacetonitrile (WO 2000047212)

[F650]

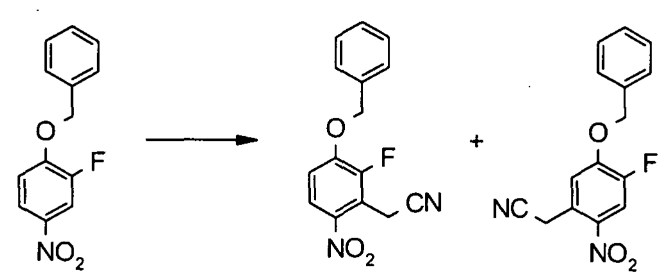

4-Benzyloxy-3-fluoro-1-nitrobenzene (3.00 g) and 4-chlorophenoxyacetonitrile (2.28 g) were dissolved in DMF (50 mL). A solution of potassium tert-butoxide (3.00 g) in DMF (10 mL) was added thereto under stirring at -10°C, and the mixture was stirred for 30 minutes at the same temperature. Ice-cooled 1N hydrochloric acid (100 mL) was added to the reaction mixture. The resultant mixture was extracted with ethyl acetate (200 mL), and the extract was washed with saturated brine (2 x 100 mL), followed by drying over sodium sulfate anhydrate. Solvent was evaporated, and the residue was purified by silica gel column chromatography (Yamazen Hi-Flash column 2L), whereby 3-benzyloxy-2-fluoro-6-nitrophenylacetonitrile (high-polar fraction, 1.83 g) and 5-benzyloxy-4-fluoro-2-nitrophenylacetonitrile (low-polar fraction, 1.09 g) were yielded. $^1$H-NMR ($CDCl_3$) δ :
4.16(2H,d,J=2.0Hz), 5.27(2H,s), 7.10(1H,t,J=8.8Hz), 7.45-7.35(5H,m), 8.07(1H,dd,J=9.4,1.8Hz).

(3) 5-Benzyloxy-4-fluoro-1H-indole

[F651]

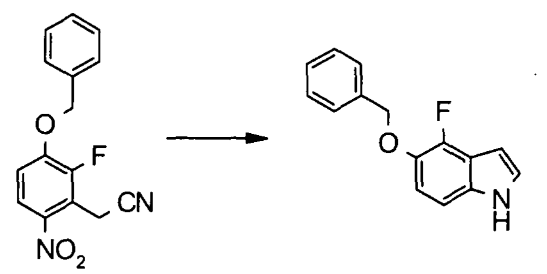

3-Benzyloxy-2-fluoro-6-nitrophenylacetonitrile (500 mg) was dissolved in a solvent mixture of 95% ethanol (10 mL) and THF (2 mL), and $PtO_2$ (50 mg) was added to the solution. The resultant mixture was subjected to catalytic hydrogenation for 15 hours at 10 atm under stirring. Nitrogen was caused to pass through the mixture vessel, and the catalyst was removed by filtration, followed by concentration of the filtrate. The residue was purified by silica gel column chromatography (Yamazen Hi-Flash column 2L), whereby the title compound (150 mg) was yielded.
$^1$H-NMR ($CDCl_3$) δ :
5.15(2H,s), 6.60-6.63(1H,m), 6.94(1H,dd,J=8.5,7.6Hz), 7.02(1H,d,J=8.5Hz),7.15-7.17(1H,m), 7.28-7.50(5H,m), 8.10 (1H,br s).
MS (ESI) m/z : 242 $(M+H)^+$.

(4) 5-Benzyloxy-1-(tert-butoxycarbonyl)-4-fluoro-1H-indole

[F652]

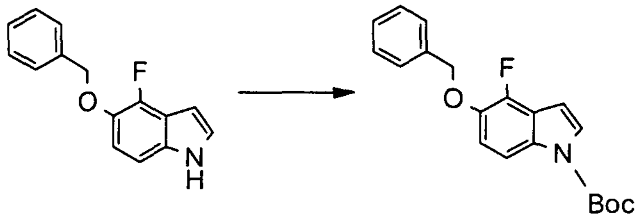

5-Benzyloxy-4-fluoro-1H-indole (244 mg) was dissolved in dichloromethane (10 mL), and $Boc_2O$ (331 mg) and DMAP (12.4 mg) were added thereto, followed by stirring for 20 hours. The resultant mixture was concentrated. The residue was purified by silica gel column chromatography (Yamazen Hi-Flash column 2L), whereby the title compound (226 mg) was yield. $^1$H-NMR ($CDCl_3$) $\delta$ :
1.65(9H,s), 5.18(2H,s), 6.64(1H,d,J=3.7Hz), 7.01(1H,t,J=8.4Hz), 7 .29-7.40(3H,m),7.48-7.44(2H,m), 7.53(1H,d, J=3.4Hz), 7.77(1H,d,J=7.8Hz).

(5) 1-(tert-Butoxycarbonyl)-4-fluoro-5-hydroxyindoline

[F653]

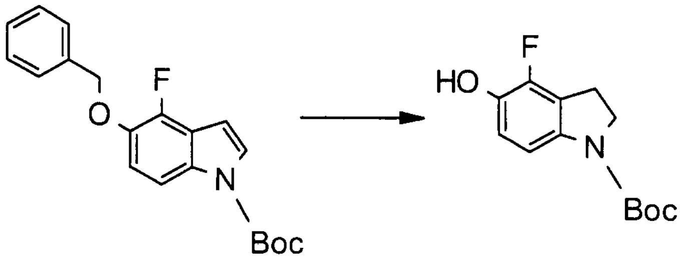

5-Benzyloxy-1-(tert-butoxycarbonyl)-4-fluoro-1H-indole (225 mg) was dissolved in ethanol (200 mL), and 5% Pd/C was added thereto, followed by catalytic hydrogenation for 15 hours with stirring. Nitrogen was caused to pass through the reaction vessel, and the catalyst was removed by filtration, followed by concentration of the filtrate. The residue was purified by silica gel column chromatography (Yamazen Hi-Flash column 2L), whereby the title compound (168 mg) was yielded.
$^1$H-NMR ($CDCl_3$) $\delta$ :
1.54(9H,s),3.10(2H,t,J=8.7Hz),3.96-4.06(2H,m), 4.72(1H,d,J=3.4Hz), 6.79(1H,t,J=8.8Hz), 7.49(1H,brs).
MS (ESI) m/z :254 $(M+H)^+$.

(6) 1-(tert-Butoxycarbonyl)-4-fluoro-5-[(4-phenyl-5-trifluoromethyl-2-thienyl)methoxy]indoline

[F654]

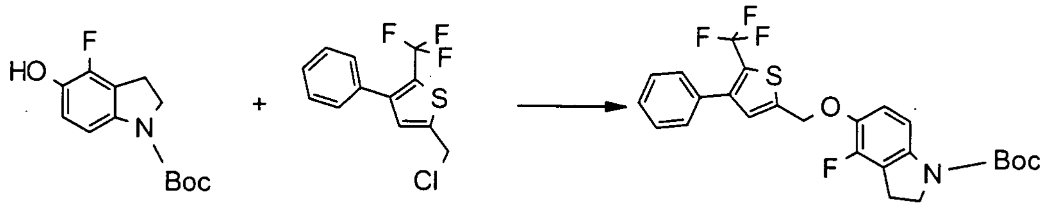

1-(tert-Butoxycarbonyl)-4-fluoro-5-hydroxyindoline (74 mg), 4-phenyl-5-trifluoromethyl-2-thienylmethyl chloride (97 mg), potassium carbonate (61 mg), and DMF (5 mL) were mixed together, and the mixture was stirred for 5 hours at 80°C. The reaction mixture was left to stand to cool to room temperature, followed by extraction with ethyl acetate (200 mL). The extract was washed with saturated brine (2 x 100 mL) and dried over sodium sulfate anhydrate. Solvent was evaporated, and the residue was purified by silica gel column chromatography (Yamazen Hi-Flash column L), whereby the title compound (143 mg) was yielded. $^1$H-NMR ($CDCl_3$) δ :
1.55(9H,s), 3.11(2H,t,J=8.5Hz), 4.01(2H,t,J=8.1Hz), 5.22(2H,s), 6 .85(1H,t,J=8.5Hz), 7.07-7.04(1H,m), 7.63-7.33(6H, m).
MS (ESI)m/z:394 $(M-99)^+$.

(7) 3-[N-(tert-Butoxycarbonyl)-N-[2-[4-fluoro-5-[(4-phenyl-5-trifluoromethyl-2-thienyl)methoxy]-1-indolinyl]-2-oxoethyl] amino]propionic acid tert-butyl ester

[F655]

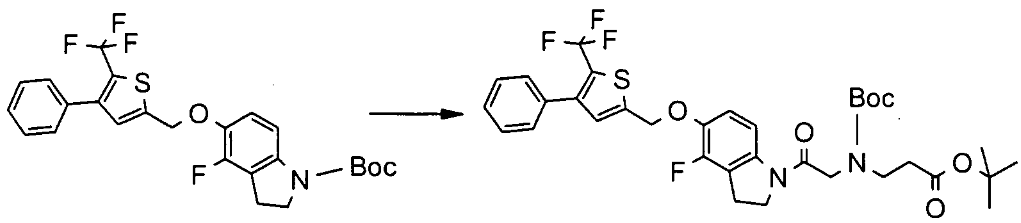

4N HCl/1,4-dioxane (10 mL) was added to 1-(tert-butoxycarbonyl)-4-fluoro-5-[(4-phenyl-5-trifluoromethyl-2-thienyl)methoxy]indoline (143 mg), and the mixture was stirred for 3 hours, followed by concentration. 2-[N-(tert-Butoxycarbonyl)-N-(tert-butoxycarbonylethyl)amino]acetic acid (88 mg), EDC·HCl (83 mg), HOBt (59 mg), TEA (202 μL), and DMF (10 mL) were added thereto, and the mixture was stirred for 14 hours. The reaction mixture was extracted with ethyl acetate (200 mL). The filtrate was washed with saturated brine (2 x 100 mL), followed by drying over sodium sulfate anhydrate. Solvent was removed, and the residue was purified by silica gel column chromatography (Yamazen Hi-Flash column L), whereby the title compound (196 mg) was yielded.
$^1$H-NMR($CDCl_3$) δ:
1.38-1.50(18H,m), 2.49-2.64(2H,m), 3.21-3.30(2H,m), 3.46-3.65(2H,m), 3.70-3.77(2H,m), 3.99-4.30(2H,m), 5.24(2H,s), 6.87(1H,dd,J=18.1,9.5Hz), 7.07(1H,s), 7.3 9-7.43(5H,m), 7.93-7.86(1H,m).
MS(ESI)m/z:679 $(M+H)^+$.

(8) 3-[N-[2-[4-Fluoro-5-[(4-phenyl-5-trifluoromethyl-2-thienyl)methoxy]-1-indolinyl]-2-oxoethyl]amino]propionic acid hydrochloride

[F656]

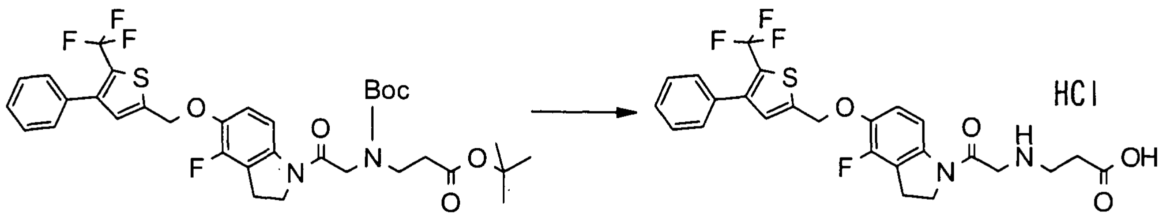

4N HCl/1,4-dioxane (10 mL) was added to 3-[N-(tert-butoxycarbonyl)-N-[2-[4-fluoro-5-[(4-phenyl-5-trifluoromethyl-2-thienyl)methoxy]-1-indolinyl]-2-oxoethyl]amino]propionic acid tert-butyl ester (196 mg), and the mixture was stirred for 1 day, followed by concentration. The concentrate was purified by reverse phase HPLC, and the target fraction was freeze-dried, whereby the title compound (119 mg) was yielded.
$^1$H-NMR (DMSO-$d_6$) δ :
2.35(2H,t,J=6.6Hz), 2.82(2H,t,J=6.7Hz), 3.14-3.25(2H,m), 3.56(2H,s), 4.11(2H,t,J=8.4Hz), 5.42(2H,s), 7.15(1H,t , J=8.7Hz), 7.39(1H,s), 7.42-7.52(5H,m), 7.80(1H,d,J=8.5Hz), 8.31(1H,br s). No proton of $CO_2H$ was observed.
MS(ESI)m/z:523(M+H)$^+$.
Anal. Calcd for $C_{25}H_{22}F_4N_2O_4S\cdot$
$0.75H_2O$:C,56.02;H,4.42;F,14.18;N,5.23;5,5.98.Found:C,55.96;H, 4.15;F,13.84;N,5.11;S,5.88.

[Example 139]

3-[N-[2-[6-Fluoro-5-[(4-phenyl-5-trifluoromethyl-2-thienyl)methoxy]-1-indoline]-2-oxoethyl]amino]propionic acid hydrochloride

(1) 5-Benzyloxy-6-fluoro-1H-indole (EP 505322)

[F657]

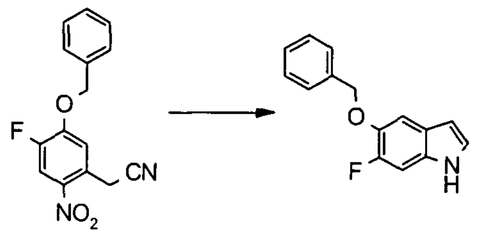

5-Benzyloxy-4-fluoro-2-nitrophenylacetonitrile (1.83 g) was dissolved in 95% ethanol (30 mL), and $PtO_2$ (500 mg) was added thereto, followed by catalytic hydrogenation for 15 hours with stirring at 10 atm. Nitrogen was caused to pass through the reaction vessel. The catalyst was removed by filtration, and the filtrate was concentrated. The residue was purified by silica gel column chromatography (Yamazen Hi-Flash column 2L), whereby the title compound (140 mg) was yielded.
$^1$H-NMR($CDCl_3$)δ :
5.15(2H,s), 6.43-6.45(1H,m), 7.11-7.17(2H,m), 7.21(1H,d,J=8.3Hz), 7.28-7.41(3H,m),7.46-7.51 (2H,m), 8.04 (1H,br s) .
MS (ESI) m/z : 242 (M+H)$^+$.

(2) 5-Benzyloxy-1-(tert-butoxycarbonyl)-6-fluoro-1H-indole

[F658]

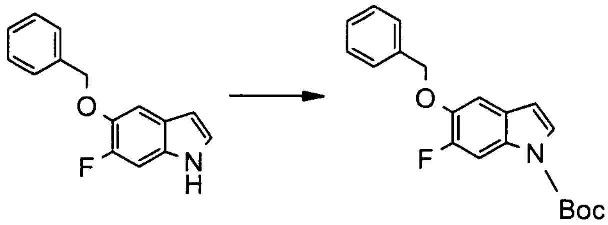

5-Benzyloxy-6-fluoro-1H-indole (140 mg) was dissolved in dichloromethane (10 mL), and $Boc_2O$ (189 mg) and DMAP (7.09 mg) were added to the solution. The mixture was stirred for 20 hours and then concentrated. The residue was purified by silica gel column chromatography (Yamazen Hi-Flash column 2L), whereby the title compound (226 mg) was yielded.
$^1$H-NMR ($CDCl_3$) δ :
1.66(9H,s), 5.15(2H,s), 6.44(1H,d,J=3.7Hz), 7.10(1H,d,J=8.0Hz), 7 28-7.40(3H,m), 7.44-7.48(2H,m), 7.52(1H,m), 7.96-7.86(1H,m).
MS (ESI) m/z : 342 $(M+H)^+$.

(3) 1-(tert-Butoxycarbonyl)-6-fluoro-5-hydroxyindoline

[F659]

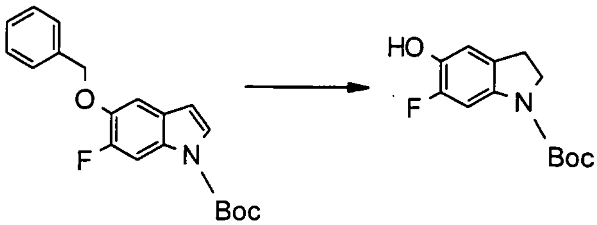

5-Benzyloxy-1-(tert-butoxycarbonyl)-6-fluoro-1H-indole (185 mg) was dissolved in ethanol (15 mL), and 5% Pd/C (185 mg) was added thereto, followed by catalytic hydrogenation for 15 hours with stirring. Nitrogen was caused to pass through the reaction vessel. The catalyst was removed by filtration, and the filtrate was concentrated. The residue was purified by silica gel column chromatography (Yamazen Hi-Flash column 2L), whereby the title compound (137 mg) was yielded. $^1$H -NMR ($CDCl_3$) δ :
1.54(9H,s), 3.01(2H,t,J=8.3Hz), 3.89-4.01(2H,m), 4.79-4.83(1H,m), 6.78(1H,d,J=8.5Hz), 7.-71-7.57(1H,m).
MS (ESI) m/z : 254 $(M+H)^+$.

(4) 1-(tert-Butoxycarbonyl)-6-fluoro-5-[(4-phenyl-5-trifluoromethyl-2-thienyl)methoxy]indoline

[F660]

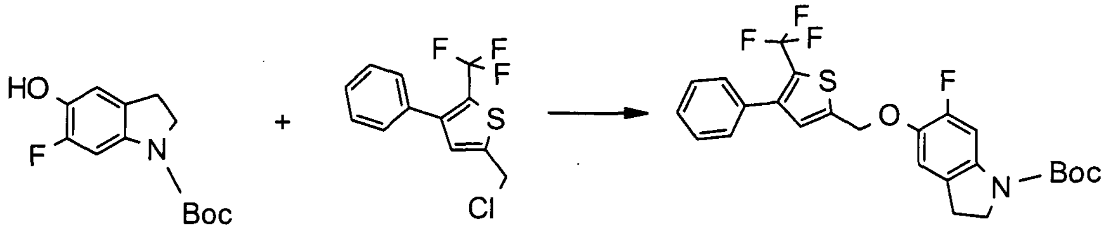

1-(tert-Butoxycarbonyl)-6-fluoro-5-hydroxyindoline (114 mg), 4-phenyl-5-trifluoromethyl-2-thienylmethyl chloride (149 mg), potassium carbonate (93 mg), and DMF (10 mL) were mixed together, and the mixture was stirred for 5 hours at 80°C. The reaction mixture was left to stand to cool to room temperature and diluted with ethyl acetate (200 mL). The diluted mixture was washed with saturated brine (2 x 100 mL), followed by drying over sodium sulfate anhydrate. Solvent was evaporated, and the residue was purified by silica gel column chromatography (Yamazen Hi-Flash column L), whereby the title compound (228 mg) was yield.
$^1$H-NMR ($CDCl_3$) δ:
1.55(9H,s),3.03(2H,t,J=8.7Hz),3.93-4.03(2H,m), 5.21(2H,s), 6.85(1H,d,J=8.1Hz), 7.05(1H,s), 7.43-7.36(6H,m).
MS(ESI)m/z:494(M+H)$^+$.

(5) 3-[N-(tert-Butoxycarbonyl)-N-[2-[6-fluoro-5-[(4-phenyl-5-trifluoromethyl-2-thienyl)methoxy]-1-indolinyl]-2-oxoethyl] amino]propionic acid tert-butyl ester

[F661]

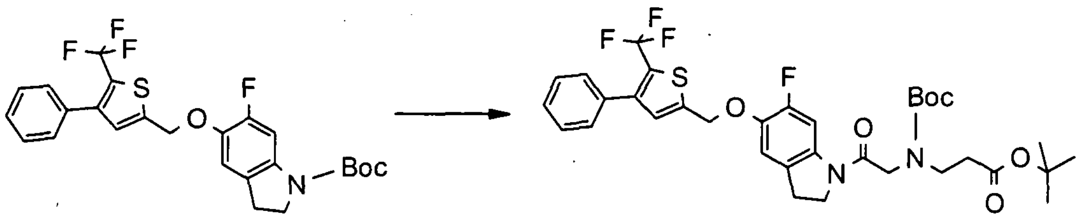

4N HCl/1,4-dioxane (10 mL) was added to 1-(tert-butoxycarbonyl)-6-fluoro-5-[(4-phenyl-5-trifluoromethyl-2-thienyl)methoxy]indoline (228 mg), and the mixture was stirred for 3 hours. The reaction mixture was concentrated, and 2-[N-(tert-butoxycarbonyl)-N-(tert-butoxycarbonylethyl)amino]acetic acid (140 mg), EDC·HCl (133 mg), HOBt (94 mg), TEA (234 μL), and DMF (10 mL) were added thereto. The resultant mixture was stirred for 14 hours. The reaction mixture was extracted with ethyl acetate (200 mL), and the extract was washed with saturated brine (2 x 100 mL), followed by drying over sodium sulfate anhydrate. Solvent was evaporated, and the residue was purified by silica gel column chromatography (Yamazen Hi-Flash column L), whereby the title compound (280 mg) was yielded. $^1$H-NMR ($CDCl_3$) δ : 1.38-1.51(18H,m), 2.54-2.63(2H,m), 3.12-3.21(2H,m), 3.54-3.79(4H,m),4.00-4.19(2H,m), 5.23(2H,s), 6.88(1H,t, J=8.4Hz), 7.05(1H,s), 7.38-7.43(5H,m), 8.10-8.01(1H,m).
MS(ESI)m/z:679 (M+H)$^+$.

(6) 3-[N-(tert-Butoxycarbonyl)-N-[2-[6-fluoro-5-[(4-phenyl-5-trifluoromethyl-2-thienyl)methoxy]-1-indolinyl]-2-oxoethyl] amino]propionic acid hydrochloride

[F662]

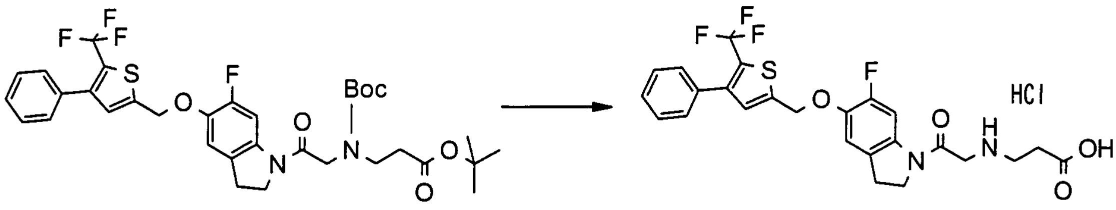

4N HCl/1,4-dioxane (10 mL) was added to 3-[1-(tert-butoxycarbonyl)-6-fluoro-5-[(4-phenyl-5-trifluoromethyl-2-thienyl)methoxy]-1-indolinylcarbonylmethylamino]propionic acid tert-butyl ester (280 mg), and the mixture was stirred for 1 day. The reaction mixture was concentrated, and chloroform/hexane was added to the concentrate to form solid. The solid was collected by filtration and dried, whereby the title compound (155 mg) was yielded.
$^1$H-NMR (DMSO-$d_6$) δ:
2.77(2H,t,J=7.4Hz),3.16-3.25(4H,m), 4.11(2H,t,J=8.4Hz), 4.16(2H,s), 5.45(2H,s), 7.36(1H,d ,J=8.5Hz), 7.41-7.38(1H, m), 7.52-7.42(5H,m), 7.88(1H,d,J=12.2Hz).
No proton of either COOH or $NH_2Cl$ was observed.
MS(ESI)m/z:523(M+H)$^+$.
Anal. Calcd for $C_{25}H_{22}F_4N_2O_4S \cdot 0.75H_2O \cdot$
HCl:C,52.45;H,4.31;F,13.27;N,4.89;S,5.60.Found:C,52.40;H,4.27 ;F,12.90;N,4.89;5,5.38.

[Example 140]

4-[5-(4-Cyclohexyl-3-trifluoromethylbenzyloxy)-4-methyl-1-indolinyl]-(3S)-(N-methylamino)-4-oxobutyric acid hydrochloride

(1) (3S)-(N-tert-Butoxycarbonyl-N-methylamino)-4-[5-(4-cyclohexyl-3-trifluoromethylbenzyloxy)-4-methyl-1-indolinyl]-4-oxobutyric acid tert-butyl ester

[F663]

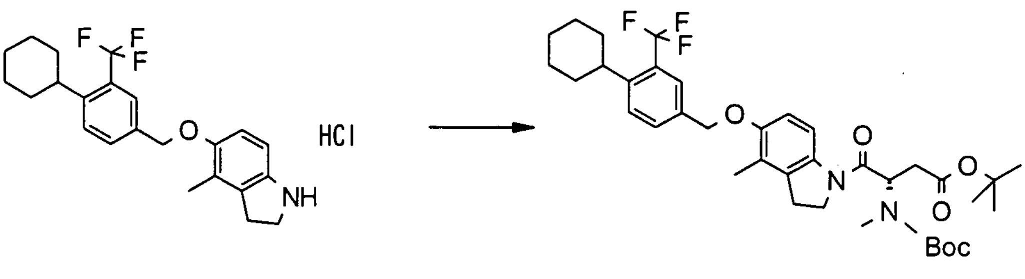

5-(4-Cyclohexyl-3-trifluoromethylbenzyloxy)-4-methyl-1-indoline hydrochloride (250 mg), Boc-Me-Asp(Ot-Bu)-OH (153 mg), EDC·HCl (169 mg), HOBt (119 mg), TEA (409 μL), and DMF (10 mL) were mixed together, and the mixture was stirred for 22 hours. The reaction mixture was extracted with ethyl acetate (200 mL), and the extract was washed with saturated brine (2 x 100 mL), followed by drying over sodium sulfate anhydrate. Solvent was evaoprated, and the residue was purified by silica gel flash column chromatography (Yamazen Hi-Flash column 2L), whereby the title compound (310 mg) was yielded.

(2) 4-[5-(4-Cyclohexyl-3-trifluoromethylbenzyloxy)-4-methyl-1-indolinyl]-(3S)-(N-methylamino)-4-oxobutyric acid hydrochloride

[F664]

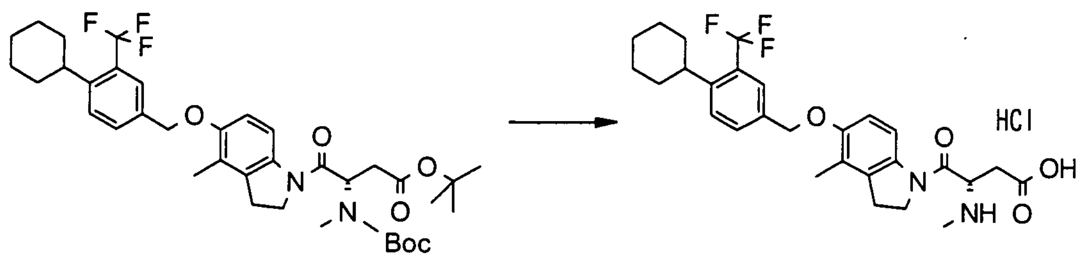

4N HCl/1,4-dioxane (10 mL) was added to (3S)-(N-tert-butoxycarbonyl-N-methylamino)-4-[5-(4-cyclohexyl-3-trifluoromethylbenzyloxy)-4-methyl-1-indolinyl]-4-oxobutyric acid tert-butyl ester (310 mg), and the mixture was stirred for 1 day. The reaction mixture was concentrated. The solid was dried, whereby the title compound (255 mg) was yielded.
$^1$H-NMR (DMSO-$d_6$) δ:
1.12-1.78(10H,m), 2.05(3H,s), 2.49(3H,s), 2.70-3.08(5H,m), 4.07-4.16(1H,m), 4.26-4.35(1H,m), 4.40(1H,t,J=6.2Hz), 5.07(2H,s), 6.82(1H,d,J=8.8Hz), 7 .66-7.55(3H,m), 7.81(1H,d,J=8.8Hz). No proton peak was observed for $NH_2Cl$ and $CO_2H$.
MS(ESI)m/z:519(M+H)$^+$.
Anal. Calcd for $C_{28}H_{33}F_3N_2O_4$·0.5$H_2O$· HCl:C,59.62;H,6.25;Cl,6.29;F,10.10;N,4.97.Found:C,59.29;H,6.3 7;Cl,6.45;F,1.10;N,4.92.

Test method

1. In vitro evaluation of test substances:

(1) Cloning of HA-Gqi5 DNA

Genomic DNA was extracted from CHO cells expressing HA-Gqi5 (purchased from Molecular Devices) by means of a DNeasy Tissue Kit (product of QIAGEN). PCR was performed with the extract as a template by use of KOD plus DNA polymerase (product of TOYOBO). A target PCR product was purified and subjected to blunting kination (BKL Kit, product of Takara Bio), and the treated product was ligated with pUC118/HincII-BAP (product of Takara Bio). The ligation mixture was integrated into *E. Coli* DH5α (product of TOYOBO), and a positive clone was selected through PCR, whereby a plasmid into which HA-Gqi5 DNA had been integrated was produced.

(2) Establishing a HA-Gqi5 expression plasmid

An HA-Gqi5 gene integrated into pUC118 was digested by use of restriction enzymes, and the obtained fragment was purified. The fragment was ligated with an expression plasmid pcDNA3.1 Hygro(+) (product of Invitrogen). The ligation mixture was integrated into *E. Coli* DH5α, and a positive clone was selected, whereby a HA-Gqi5 expression plasmid was produced.

(3) Production of CHO cells expressing HA-Gqi5

The HA-Gqi5 expression plasmid, which had been produced in (2) above, was integrated into CHO-K1 cells by use of a Fugene 6 reagent (product of Roche Diagnostics K.K.), and cells of interest were selected by use of hygromycin. A CHO cell expressing HA-Gqi5 was selected through western blotting employing an anti-HA antibody, and the selected cells were cloned twice.

(4) Cloning of human S1P1 (EDG-1)

By use of a human S1P1 (EDG-1) cDNA clone (open biosystems, cDNA collection #4071217) as a template, human S1P1 (EDG-1) DNA was produced through PCR. This PCR product was integrated into pUC118. By means of a site-directed mutagenesis kit (product of STRATAGENE), a plasmid into which DNA having a target sequence (The Journal of Biological Chemistry Vol. 265, No. 16, 9308-9313, 1990) had been integrated was produced.

(5) Establishing a human S1P1 (EDG-1) expression plasmid

A human S1P1 (EDG-1) gene (The Journal of Biological Chemistry Vol. 265, No. 16, 9308-9313, 1990) integrated into pUC118 was digested by use of restriction enzymes, and the cut fragment was purified. The fragment was ligated with an expression plasmid pcDNA3.1/mycHisA (product of Invitrogen). The ligation mixture was integrated into *E. Coli* DH5α, and a positive clone was selected, whereby a S1P1 (EDG-1) expression plasmid was produced.

(6) Production of CHO cells expressing human S1P1 (EDG-1)

A S1P1 (EDG-1)expression plasmid was integrated into the CHO cells expressing HA-Gqi5, which had been produced in (3) above, by use of a Fugene 6 reagent (product of Roche Diagnostics K.K.). Cells of interest were selected by use of G418, and a cell exhibiting elevation of intracellular calcium level when stimulated with S1P was selected. The selected cells were cloned twice.

(7) Intracellular calcium flux assay

CHO cells expressing Gqi5 protein, to which human S1P1 (EDG-1) had been introduced and which had been produced in (6) above, were seeded on a 96-well black plate with a transparent bottom surface ($2.5 \times 10^4$ cell/well) and cultured overnight. The culture was washed once with a serum-free medium, and an assay buffer(calcium assay kit, product of Molecular Devices, 100 μL) containing 2.5mM probenicid and 0.25% fatty acid-free BSA was added to the washed cells. The mixture was allowed to react at 37°C for one hour under 5% $CO_2$. Separately, each test compound was diluted so as to attain a concentration five times the final test concentration. The diluted liquid (25 μL) was added to each well, and change in intracellular calcium concentration was measured by means of a FLEXstation II (product of Molecular Devices). The change was determined as the difference between the minimum intracellular calcium level and the peak (maximum) value thereof. A sigmoid curve was obtained from the measurements, and an $EC_{50}$ value was calculated from the curve so as to evaluate agonist activity against an S1P1 (EDG-1) receptor.

2. In vivo evaluation of test substances (decrease in peripheral blood lymphocyte count of mice after administration of each compound):

There has already been reported that lymphocytes in peripheral blood of mice decrease after administration of an S1P receptor agonist [SCIENCE, 296, 346-349 (2002)]. According to the evaluation method disclosed in the document, test compounds were evaluated. As shown in Table 1, each test substance was dissolved or suspended in MC (methylcellulose) solution, and the resultant liquid was perorally administered to each mouse at a dose of 0.2 mL/20 g-body weight.

[Table 1]

| Dose | Amount of compound | MC solution | Concentration |
|---|---|---|---|
| 3 mg/kg | 0.6 mg | 2 mL | 0.3 mg/mL |
| 10 mg/kg | 2 mg | 2 mL | 1 mg/mL |

Four hours after peroral administration, blood (0.5 mL) was collected from each mouse through the posterior vena cava under anesthesia with ether by use of EDTA as an anti-coagulant. The number of lymphocytes in peripheral blood was determined by means of a general hematological tester ADVIA 120 (Bayer Medical). The pharmacological action of each test drug was evaluated as T/C (%) ratio, which is a ratio of average peripheral blood lymphocyte count of a test drug-administered group to that of the solvent-administered group (control group).
T/C (%) calculation:

T/C (%) = (average peripheral blood lymphocyte count of a test drug-administered group)/(average peripheral blood lymphocyte count of the solvent-administered group)×100

Test results

According to the aforementioned test method, compounds of Examples were tested, and the results are shown in Table 2.

[Table 2]

| Compound | S1P1 $EC_{50}$ (nM) | T/C(%) (3 mg/kg) | T/C (%) (10 mg/kg) |
|---|---|---|---|
| Example 10 | 1.3 | 21.6 | 16.3 |
| Example 14 | 4.4 | 17.0 | 11.1 |
| Example 29 | 2.4 | 15.5 | 13.9 |
| Example 30 | 2.9 | 21.7 | 11.1 |
| Example 31 | 6.3 | 30.8 | 19.8 |

3. In vivo evaluation of test substances (decrease in peripheral blood lymphocyte count of rats after administration of each compound):

The effect of administration of an S1P receptor agonist on decrease in peripheral blood lymphocyte counts of rats (female Lewis) was evaluated. As shown in Table 3, each test substance was dissolved or suspended in 0.5% MC (methylcellulose) solution, and the resultant liquid was perorally administered to each rat at a dose of 1 mL/200 g-body weight.

[Table 3]

| Dose | Amount of compound | 0.5% MC solution | Concentration |
|---|---|---|---|
| 3 mg/kg | 3 mg | 5 mL | 0.6 mg/mL |
| 10 mg/kg | 10 mg | 5 mL | 2 mg/mL |

Four hours after peroral administration, blood (0.5 mL) was collected from each rat through the cervical vein under anesthesia with ether by use of EDTA as an anti-coagulant. The number of lymphocytes in peripheral blood and blood cell fractions were determined by means of a general hematological tester ADVIA 120 (Bayer Medical). The action of each test drug on reducing peripheral blood lymphocyte count was evaluated as T/C (%) ratio at the time of blood collection, which is a ratio of average peripheral blood lymphocyte count of a test drug-administered group to that of the solvent-administered group (control group).

T/C (%) calculation:

T/C (%) = (average peripheral blood lymphocyte count of a test drug-administered group)/(average peripheral blood lymphocyte count of the solvent-administered group)×100

Test results

According to the aforementioned test method, compounds of Examples were tested, and the results are shown in Table 4. Similar to the case of four hours after peroral administration, an excellent action of reducing lymphocytes in peripheral blood was also attained 24 hours after peroral administration.

[Table 4]

| Compound | S1P1 $EC_{50}$ (nM) | T/C(%) (3 mg/kg) | T/C(%) (10 mg/kg) |
|---|---|---|---|
| Example 43 | 8.7 | 34.9 | 25.7 |
| Example 97 | 4.7 | 22.2 | 17.9 |
| Example 119 | 6.2 | 28.7 | 20.7 |
| Example 129 | 4.5 | 18.2 | 17.7 |

As described above, the compound according to the present invention was found to have an agonist activity against an S1P1 receptor and to effectively reduce lymphocytes in peripheral blood through oral administration.

## Claims

**1.** A compound represented by general formula (I):

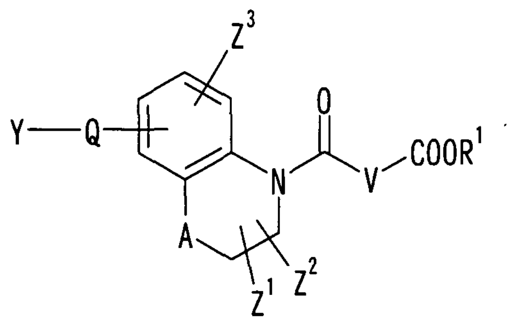

(I)

[wherein A represents a single bond, -O-, or $-CH_2-$;
$R^1$ represents a hydrogen atom or a $C_1$-$C_6$ alkyl group;
V represents any one group selected from the following (1) to (3) :

(1) $-G^1-$ (wherein $G^1$ represents an optionally substituted straight-chain alkylene group having 1 to 5 carbon atoms),
(2) $-G^2-N(R^2)-G^3-$ (wherein $G^2$ represents a single bond or an optionally substituted straight-chain alkylene group having 1 to 3 carbon atoms, and $G^3$ represents an optionally substituted straight-chain alkylene group having 1 to 4 carbon atoms, and $R^2$ represents a hydrogen atom, a $C_1$-$C_6$ alkyl group, or a 3- to 8-membered cycloalkyl group), and
(3) the following group:

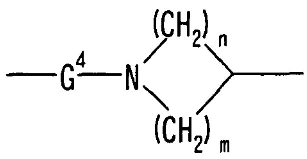

(wherein $G^4$ represents a single bond or an optionally substituted straight-chain alkylene group having 1 to 2 carbon atoms, and each of m and n independently represents 1, 2, or 3) ;
each of $Z^1$ and $Z^2$ independently represents a hydrogen atom or a $C_1$-$C_6$ alkyl group;
$Z^3$ represents a hydrogen atom, a halogen atom, a cyano group, a $C_1$-$C_6$ alkyl group, a halogeno-$C_1$-$C_6$ alkyl group, an alkoxy group, or a halogeno-$C_1$-$C_6$ alkoxy group;
Q represents a single bond, -O-, $-CH_2-$, $-CH_2-CH_2-$, $-CH_2-CH_2-CH_2-$, $-CH_2-CH_2-CH_2-CH_2-$, $-CH_2-CH_2-CH_2-CH_2-CH_2-$, $-CH_2-O-$, - $CH_2-CH_2-O-$, $-CH_2-O-CH_2-$, $-CH_2-CH_2-O-CH_2-$, $-CH_2-CH_2-CH_2-O-$, $-CH_2-CH_2-CH_2-O-CH_2-$, $-CH_2-CH_2-O-CH_2-CH_2-$, $-CH_2-CH_2-CH_2-CH_2-O-$, - CH=CH-, or $-CH=CH-CH_2-O-$ (wherein these groups may each have 1 or 2 $C_1$-$C_6$ alkyl groups or halogen atoms as substituents);and
Y represents the following group:

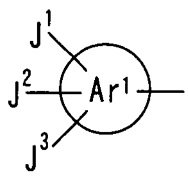
or
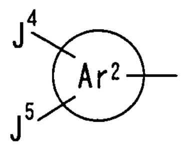

(wherein each of $Ar^1$ and $Ar^2$ independently represents a benzene ring or a 5- or 6-membered aromatic heterocycle; each of $J^1$, $J^2$, $J^3$ , $J^4$, and $J^5$ independently represents a hydrogen atom, a halogen atom, a hydroxyl group, a nitro group, a cyano group, a $C_1$-$C_6$ alkyl group, a halogeno-$C_1$-$C_6$ alkyl group, a $C_1$-$C_6$ alkoxy-$C_1$-$C_6$ alkyl group, a $C_1$-$C_6$ alkoxy group, a halogeno-$C_1$-$C_6$ alkoxy group, an amino group, a mono-$C_1$-$C_6$ alkylamino group, a di-$C_1$-$C_6$ alkylamino group, a carboxyl group, a $C_1$-$C_6$ alkoxycarbonyl group, a carbamoyl group, a mono-$C_1$-$C_6$ alkylcarbamoyl group, a di-$C_1$-$C_6$ alkylcarbamoyl group, a sulfamoyl group, a mono-$C_1$-$C_6$ alkylsulfamoyl group, a di-$C_1$-$C_6$ alkylsulfamoyl group, an optionally substituted phenyl group, an optionally substituted benzyl group, an optionally substituted phenethyl group, an optionally substituted styryl group, an optionally substituted phenoxy group, an optionally substituted benzyloxy group, an optionally substituted phenoxymethyl group, an optionally substituted 3- to 8-membered cycloalkyl group, an optionally substituted 3- to 8-membered cycloalkenyl group, an optionally substituted 3- to 8-membered cycloalkylmethyl group, an optionally substituted 3- to 8-membered cycloalkyloxy group, an optionally substituted 3- to 8-membered cycloalkylmethoxy group, an optionally substituted 5- or 6-membered aromatic heterocyclic group, an optionally substituted 4- to 6-membered saturated heterocyclic group, or an optionally substituted 5- or 6-membered heteroaryloxy group)], a saltthereof, or a solvate thereof.

**2.** A compound according to claim 1, a salt thereof, or a solvate thereof, wherein A in the general formula (I) is a single bond.

**3.** A compound according to claim 1, a salt thereof, or a solvate thereof, wherein A in the general formula (I) is -O-.

**4.** A compound according to any one of claims 1 to 3, a salt thereof, or a solvate thereof, wherein Y in the general formula (I) is the following group:

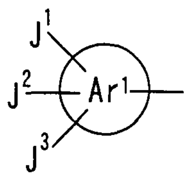

(wherein $J^1$, $J^2$, and $J^3$ have the same meanings as defined in claim 1), and $Ar^1$ is a benzene ring, a furan ring, a thiophene ring, an imidazole ring, a pyrazole ring, a pyridine ring, or a pyridazine ring.

**5.** A compound according to claim 4, a salt thereof, or a solvate thereof, wherein each of $J^1$, $J^2$ , and $J^3$ is independently a hydrogen atom, a halogen atom, a nitro group, a cyano group, a $C_1$-$C_6$ alkyl group, a halogeno-$C_1$-$C_6$ alkyl group, a $C_1$-$C_6$ alkoxy-$C_1$-$C_6$ alkyl group, a $C_1$-$C_6$ alkoxy group, a halogeno-$C_1$-$C_6$ alkoxy group, a di-$C_1$-$C_6$ alkylamino group, an optionally substituted phenyl group, an optionally substituted phenethyl group, an optionally substituted phenoxy group, an optionally substituted benzyloxy group, an optionally substituted 3- to 8-membered cycloalkyl group, an optionally substituted 3- to 8-membered cycloalkenyl group, an optionally substituted 3- to 8-membered cycloalkylmethyl group, an optionally substituted 3- to 8-membered cycloalkylmethoxy group, or an optionally substituted 5- or 6-membered aromatic heterocyclic group.

**6.** A compound according to any one of claims 1 to 3, a salt thereof, or a solvate thereof, wherein Y in the general formula (I) is the following group:

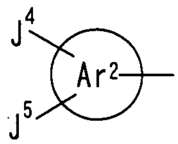

(wherein $J^4$ and $J^5$ have the same meanings as defined in claim 1), and $Ar^2$ is an oxazole ring, a thiazole ring, or a triazole ring.

**7.** A compound according to claim 6, a salt thereof, or a solvate thereof, wherein each of $J^4$ and $J^5$ is independently a hydrogen atom, a halogen atom, a nitro group, a cyano group, a $C_1$-$C_6$ alkyl group, a halogeno-$C_1$-$C_6$ alkyl group, a $C_1$-$C_6$ alkoxy-$C_1$-$C_6$ alkyl group, a $C_1$-$C_6$ alkoxy group, a halogeno-$C_1$-$C_6$ alkoxy group, an optionally substituted phenyl group, or an optionally substituted 3- to 8-membered cycloalkyl group.

**8.** A compound according to any one of claims 1 to 7, a salt thereof, or a solvate thereof, wherein V in the general formula (I) is -$G^1$- (wherein $G^1$ has the same meaning as defined in claim 1).

**9.** A compound according to claim 8, a salt thereof, or a solvate thereof, wherein $G^1$ is $-CH_2-CH_2-$ which may have as a substituent 1 or 2 groups selected from the group consisting of a halogen atom, a hydroxyl group, a $C_1$-$C_6$ alkyl group, an amino group, a mono-$C_1$-$C_6$ alkylamino group, and a di-$C_1$-$C_6$ alkylamino group.

**10.** A compound according to claim 9, a salt thereof, or a solvate thereof, wherein $G^1$ is $-CH_2-CH_2-$ which has a mono-$C_1$-$C_6$ alkylamino group as a substituent.

**11.** A compound according to any one of claims 1 to 7, a salt thereof, or a solvate thereof, wherein V in the general formula (I) is a $-G^2-N(R^2)-G^3-$ (wherein $G^2$, $G^3$, and $R^2$ have the same meanings as defined in claim 1).

**12.** A compound according to claim 11, a salt thereof, or a solvate thereof, wherein $G^2$ is $-CH_2-$ which may have as a substituent 1 or 2 groups selected from the group consisting of a halogen atom, a hydroxyl group, a $C_1$-$C_6$ alkyl group, and an oxo group, $G^3$ is $-CH_2-CH_2-$ which may have as a substituent 1 or 2 groups selected from the group consisting of a halogen atom, a hydroxyl group, a $C_1$-$C_6$ alkyl group, and an oxo group, and $R^2$ is a hydrogen atom or a $C_1$-$C_6$ alkyl group.

**13.** A compound according to any one of claims 1 to 7, a salt thereof, or a solvate thereof, wherein V in the general formula (I) is the following group:

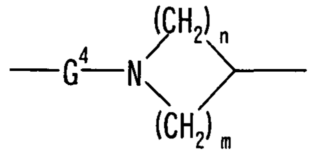

(wherein $G^4$, m, and n have the same meanings as defined in claim 1) .

**14.** A compound according to claim 13, a salt thereof, or a solvate thereof, wherein $G^4$ is $-CH_2-$, m is 1, and n is 1, 2, or 3.

**15.** A compound according to any one of claims 1 to 14, a salt thereof, or a solvate thereof, wherein Q in the general formula (I) is $-CH_2-O-$ which may have as a substituent 1 or 2 $C_1$-$C_6$ alkyl groups or halogen atoms.

**16.** A compound according to any one of claims 1 to 15, a salt thereof, or a solvate thereof, wherein each of $Z^1$ and $Z^2$ in the general formula (I) is a hydrogen atom.

**17.** A compound according to any one of claims 1 to 16, a salt thereof, or a solvate thereof, wherein $Z^3$ in the general formula (I) is a hydrogen atom.

**18.** A compound according to any one of claims 1 to 16, a salt thereof, or a solvate thereof, wherein $Z^3$ in the general formula (I) is a methyl group.

**19.** A medicament containing, as an effective ingredient, a compound according to any one of claims 1 to 18, a salt thereof, or a solvate thereof.

**20.** An S1P receptor agonist containing, as an effective ingredient, a compound according to any one of claims 1 to 18, a salt thereof, or a solvate thereof.

**21.** An S1P1 receptor agonist containing, as an effective ingredient, a compound according to any one of claims 1 to 18, a salt thereof, or a solvate thereof.

**22.** An immunosuppressor containing, as an effective ingredient, a compound according to any one of claims 1 to 18, a salt thereof, or a solvate thereof.

**23.** A therapeutic and/or preventive agent for rejection upon transplantation, an autoimmune disease, and/or an allergic disease, containing, as an effective ingredient, a compound according to any one of claims 1 to 18, a salt thereof, or a solvate thereof.

**24.** A medicament containing a compound according to any one of claims 1 to 18, a salt thereof, or a solvate thereof, in combination with one or more selected from an immunosuppressor, an antibody useful for immunosuppression, a rejection-treating agent, an antibiotic, and a steroidal agent.

**25.** Use of a compound according to any one of claims 1 to 18, a salt thereof, or a solvate thereof, for production of a medicament.

**26.** Use of a compound according to any one of claims 1 to 18, a salt thereof, or a solvate thereof, for production of an S1P receptor agonist.

**27.** Use of a compound according to any one of claims 1 to 18, a salt thereof, or a solvate thereof, for production of an S1P1 receptor agonist.

**28.** Use of a compound according to any one of claims 1 to 18, a salt thereof, or a solvate thereof, for production of an immunosupressor.

**29.** Use of a compound according to any one of claims 1 to 18, a salt thereof, or a solvate thereof, for production of a therapeutic and/or preventive agent for rejection upon transplantation, an autoimmune disease, and/or an allergic disease.

**30.** Use of a compound according to any one of claims 1 to 18, a salt thereof, or a solvate thereof, for production of a medicament in combination with one or more selected from an immunosuppressor, an antibody useful for immunosuppression, a rejection-treating agent, an antibiotic, and a steroidal agent.

**31.** A method for preventing and/or treating an S1P receptor-relating disease, **characterized by** administering an effective amount of a compound according to any one of claims 1 to 18, a salt thereof, or a solvate thereof.

**32.** A method for preventing and/or treating an S1P1 receptor-relating disease, **characterized by** administering an effective amount of a compound according to any one of claims 1 to 18, a salt thereof, or a solvate thereof.

**33.** A method for suppressing an immune system **characterized by** administering an effective amount of a compound according to any one of claims 1 to 18, a salt thereof, or a solvate thereof.

**34.** A method for treating and/or preventing a rejection upon transplantation, an autoimmune disease, and/or an allergic disease, **characterized by** administering an effective amount of a compound according to any one of claims 1 to 18, a salt thereof, or a solvate thereof.

**35.** A method for treating and/or preventing a rejection upon transplantation, an autoimmune disease, and/or an allergic disease, **characterized by** administering a compound according to any one of claims 1 to 18, a salt thereof, or a solvate thereof, in combination with one or more selected from an immunosuppressor, an antibody useful for immunosuppression, a rejection-treating agent, an antibiotic, and a steroidal agent.

**INTERNATIONAL SEARCH REPORT**

International application No.
PCT/JP2007/059624

A. CLASSIFICATION OF SUBJECT MATTER
*C07D209/08*(2006.01)i, *A61K31/404*(2006.01)i, *A61K31/4155*(2006.01)i, *A61K31/4709*(2006.01)i, *A61K31/538*(2006.01)i, *A61K45/00*(2006.01)i, *A61P37/06*(2006.01)i, *A61P37/08*(2006.01)i, *A61P43/00*(2006.01)i,
According to International Patent Classification (IPC) or to both national classification and IPC

B. FIELDS SEARCHED

Minimum documentation searched (classification system followed by classification symbols)
C07D209/08, A61K31/404, A61K31/4155, A61K31/4709, A61K31/538, A61K45/00, A61P37/06, A61P37/08, A61P43/00, C07D403/12, C07D409/06, C07D409/12, C07D409/14, C07D413/12

Documentation searched other than minimum documentation to the extent that such documents are included in the fields searched

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | JP 2006-508965 A (SMITHKLINE BEECHAM CORP.), 16 March, 2006 (16.03.06), Particularly, abstract; Claims 1, 5, 14; description, page 38, Par. No. [0128], chemical formura 14; page 42, tables 1 to 4, 'example No.28'<br>& WO 2004/043379 A2 & EP 1581514 A2<br>& US 2006/241149 A1 & AU 2003290661 A1 | 1-2,4,8-12, 16-17,19 |
| X | VALLAT, J. N. et al., "Dérivés indoliniques apparentés aux cardénolides", European Journal of Medicinal Chemistry, (1981), Vol.16, No.5, pp.409-414, particularly, page 411, Tableau 1, 17a, 17c, 17f, 17g, compounds | 1-2,4,8-9, 16-17 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13 August, 2007 (13.08.07) | 21 August, 2007 (21.08.07) |
| Name and mailing address of the ISA/<br>Japanese Patent Office<br>Facsimile No. | Authorized officer<br><br>Telephone No. |

Form PCT/ISA/210 (second sheet) (April 2005)

**INTERNATIONAL SEARCH REPORT**

International application No.
PCT/JP2007/059624

C (Continuation). DOCUMENTS CONSIDERED TO BE RELEVANT

| Category* | Citation of document, with indication, where appropriate, of the relevant passages | Relevant to claim No. |
|---|---|---|
| A | WO 2005/020882 A2 (ONO PHARMACEUTICAL CO., LTD.), 10 March, 2005 (10.03.05), Full text; particularly, abstract; Claims 1, 4 to 5, 23, 27 to 34; CAS RN: 847585-53-9, 847585-89-1, compounds (cited in the description of the present application as "patent document 3") & US 2007/167425 A1 & EP 1661881 A2 & AU 2004268455 A1 & BR PI0413923 A & CA 2537093 A1 & CN 1874991 A & KR 20060119919 A & MX PA06002310 A & NO 20061372 A | 1-30 |
| P,A | WO 2006/064757 A1 (ONO PHARMACEUTICAL CO., LTD.), 22 June, 2006 (22.06.06), Full text; particularly, abstract; Claims 1 to 2, 5, 20 to 26; CAS RN:891859-06-6, 891858-48-3, compounds & AU 2005314938 A1 | 1-30 |

Form PCT/ISA/210 (continuation of second sheet) (April 2005)

**INTERNATIONAL SEARCH REPORT**

International application No.
PCT/JP2007/059624

Continuation of A. CLASSIFICATION OF SUBJECT MATTER
(International Patent Classification (IPC))

*C07D403/12*(2006.01)i, *C07D409/06*(2006.01)i, *C07D409/12*(2006.01)i, *C07D409/14*(2006.01)i, *C07D413/12*(2006.01)i

(According to International Patent Classification (IPC) or to both national classification and IPC)

Form PCT/ISA/210 (extra sheet) (April 2005)

**INTERNATIONAL SEARCH REPORT**

International application No.
PCT/JP2007/059624

**Box No. II Observations where certain claims were found unsearchable (Continuation of item 2 of first sheet)**

This international search report has not been established in respect of certain claims under Article 17(2)(a) for the following reasons:

1. [x] Claims Nos.: 31-35
because they relate to subject matter not required to be searched by this Authority, namely:
Claims 31 to 35 are relevant to "methods for treatment of the human body by therapy" (PCT Rule 39.1(iv)).

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the
[ ] The additional search fees were accompanied by the applicant's protest and, where applicable, payment of a protest fee..
[ ] The additional search fees were accompanied by the applicant's protest but the applicable protest fee was not paid within the time limit specified in the invitation.
[ ] No protest accompanied the payment of additional search fees.

Form PCT/ISA/210 (continuation of first sheet (2)) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 94008943 A
- WO 2005000833 A
- WO 2005020882 A
- WO 2004058149 A
- WO 9523141 A
- EP 1428514 A, Francois, C.
- WO 2004026837 A, Selsam, B. L.
- WO 9940088 A, Illig, C. R.
- WO 9119708 A, Matsuo, M.
- JP 51093999 A, Kotone, A.
- US 6362188 B, Timothy, G.
- WO 9711069 A, Oku, T.
- WO 9325535 A
- GB 2149402 A
- EP 401166 A
- WO 058848 A
- WO 2002070494 A


### Non-patent literature cited in the description


- *Science,* 2002, vol. 296, 346-349
- *Journal of the American Society of Nephrology,* 2002, vol. 13 (4), 1073-1083
- **T. W. GREEN ; P. G. WUTS.** Protective Groups in Organic Synthesis. John Wiley & Sons, Inc,
- **G. H. et al.** *Org. Lett.,* 2003, vol. 5, 1899-1902
- **MICHAEL, E. F. et al.** *J. Med. Chem.,* 1979, vol. 22, 63-69
- **JOSEPH, G. C. et al.** *J. Heterocycl. Chem.,* 1990, vol. 27, 2093-2095
- **TROXLER, F. et al.** *Helv. Chim. Acta.,* 1968, vol. 51, 1203-1213
- **PANKAJ, D. REGE et al.** *Org. Lett.,* 2006, vol. 8, 3117-3120
- **STARK, L. M. et al.** *J. Org. Chem.,* 2000, vol. 65, 3227-3230
- **KAMIYA, S. et al.** *Chem. Pharm. Bull.,* 2001, vol. 49, 563-571
- **IGARASHI, S. et al.** *Chem. Pharm. Bull.,* 2000, vol. 48, 1689-1697
- **BUON L. et al.** *Tetrahedron,* 2000, vol. 56, 605-614
- **IAKOVOU, K. et al.** *Eur. J. Med. Chem.,* 1999, vol. 34, 903-917
- **ILAS, J. et al.** *Tetrahedron,* 2005, vol. 61, 7325-7348
- **ATKINS, R. L. et al.** *J. Org. Chem.,* 1978, vol. 43 (10), 1975-1980
- **MICHAEL, H. et al.** *J. Chem. Soc., Perkin Trans. 1: Organic and Bio-Organic Chemistry,* 1972, 1933-1999
- Jikken Kagaku Koza. Chemical Society of Japan, Maruzen Co., Ltd, vol. 26
- Jikken Kagaku Koza. Chemical Society of Japan, Maruzen Co., Ltd, vol. 19
- *Organic Synthesis I: Hydrocarbon-Halogen Compound,* 438-446465-470
- **PIERRE, M. et al.** *Tetrahedron Letters,* 1985, vol. 26 (33), 3947-3950
- **GATTUSO, M. et al.** *Atti della Societa Peloritana di Science Fische, Matematiche Naturali,* 1968, vol. 14 (4), 371-380
- **VICENTINI, C. B. et al.** *Heterocycles,* 2000, vol. 53 (6), 1285-1292
- **TENSMEYER, L. G. et al.** *J. Org. Chem.,* 1966, vol. 31, 1878-1883
- **PADWA, A. et al.** *J. Org. Chem.,* 1982, vol. 47, 786-791
- **CAPUANO, L. et al.** *Liebigs Annalen der Chemie,* 1985, vol. 12, 2305-2312
- **RAFFERTY, M. F. et al.** *J. Med. Chem.,* 1982, vol. 25, 1204-1208
- **WRIGHT, S. W. et al.** *J. Org. Chem.,* 1994, vol. 59, 6095-6097
- **LISELOTTE, O. et al.** *Synlett,* 2001, 1893-1896
- **FLETCHER, S. R. et al.** *Bioorg. Med. Chem. Lett.,* 1992, vol. 2, 627-630
- **ANA, B. B. et al.** *Tetrahedron Lett.,* 2005, vol. 46, 7769-7771
- *Organic Synthesis I: Hydrocarbon-Halogen Compound,* 427, 429
- **GUPTA, A. K. et al.** *Synlett,* 2004, vol. 12, 2227-2229
- **CASALNUOVO, A. L. et al.** *J. Am. Chem. Soc.,* 1990, vol. 112, 4324-4330
- **SCHLOSSER, M. et al.** *Eur. J. Org. Chem.,* 2002, 2913-2920
- **LYGA, J. W. et al.** *Journal of Heterocyclic Chemistry,* 1990, vol. 27 (4), 9191-921

- **SHRIDHAR, D. R. et al.** *Indian Journal of Chemistry, Section B: Organic Chemistry Including Medicinal Chemistry,* 1983, vol. 22B (12), 1187-1190
- **KAMIYA, S. et al.** *Chem. Pharm. Bull.,* 2001, vol. 49 (5), 563-571
- **BAILEY, P. S. et al.** *Org. Synth.,* 1973, vol. 5, 489
- **GRONOWITZ, S. et al.** *Heterocylces,* 1981, vol. 15 (2), 947-959
- **HEGEDUS, L. S. et al.** *Comp. Org. Syn.,* 1991, vol. 4, 552-559
- **GAUNT, M. J. et al.** *Chem. Commun.,* 2001, vol. 18, 1844-1845
- **K. C. NICOLAOU et al.** *Angew. Chem. Int. Ed.,* 2005, vol. 44, 4442-4489
- **THOMPSON, L. S. A. et al.** *Synthesis,* 1994, vol. 2, 107-108
- **MIWA, K. et al.** *Synlett,* 1994, vol. 2, 107-108
- **COREY, E. et al.** *Tetrahedron Lett.,* 1972, 3769-3772
- **PRASAD, G. et al.** *J. Org. Chem.,* 1991, vol. 56, 7188-7190
- **SUZUKI, S. et al.** *Can. J. Chem.,* 1994, vol. 72, 357-361
- **WANG, W. et al.** *Tetrahedron,* 2002, vol. 58, 3101-3110
- **LEBOUVIER, N. et al.** *Tetrahedron Lett.,* 2006, vol. 47, 6479-6483
- **MURATA, M. et al.** *Synlett,* 2006, 1867-1870
- **MOLEELE, S. et al.** *Tetrahedron,* 2006, vol. 62, 2831-2844
- **T. W. GREEN ; P. G. WUTS.** Protective Groups in Organic Synthesis. John Wiley & Sons, Inc, 1991
- **BODANSZKY, M. ; KLAUSNER, Y. S. ; ONDETTI, A.** Peptide Synthesis. Wiley-interscience publication, 1976
- **PETTIT, G.** Synthetic Peptide. Elsevier Scientific Publication, 1976
- Jikken Kagaku Koza, 4th Edition, Vol. 22, Organic Synthesis IV. Maruzen Co., Ltd, 1991
- **ZHAO, H. et al.** *Bioorg. Med. Chem. Lett.,* 2002, vol. 12, 3105-3109
- **JIANG, X. -H. et al.** *Tetrahedron,* 2005, vol. 61, 1281-1288
- **NAM, J. et al.** *Tetrahedron Lett.,* 2003, vol. 44, 7727-7730
- **HAYASHI, K. et al.** *J. Med. Chem.,* 1989, vol. 32, 289-297
- **BERMUDEZ, J. et al.** *J. Med. Chem.,* 1990, vol. 33, 1932-1935
- **RAJASHEKHAR, B. et al.** *J. Org. Chem.,* 1985, vol. 50, 5480-5484
- **KUBO, Y. et al.** *J. Org. Chem.,* 1985, vol. 50, 5485-5487
- **SUN, W. S. et al.** *J. Med. Chem.,* 2003, vol. 46, 5619-5627
- **LHERBET, C. et al.** *Bioorg. Med. Chem. Lett.,* 2003, vol. 13, 997-1000
- **SHULTES, C. M. et al.** *Bioorg. Med. Chem. Lett.,* 2004, vol. 14, 4347-4351
- *Eur. J. Med. Chem.,* 1999, vol. 34, 903-917
- *Heterocycles,* 1988, vol. 27 (12), 2857-2862
- *Eur. J. Med. Chem.,* 2002, vol. 37, 461-468
- *Tetrahedron,* 2000, vol. 56, 605-614
- *Tetrahedron Lett.,* 2003, vol. 44, 2375
- *The Journal of Biological Chemistry,* 1990, vol. 265 (16), 9308-9313
- *SCIENCE,* 2002, vol. 296, 346-349